# EUROPEAN PATENT APPLICATION

(11) **EP 4 049 680 A1**
(43) Date of publication of application: **31.08.2022**
(21) Application number: 20880018.5
(22) Date of filing: 23.10.2020
(51) Int. Cl.: A61K 45/00, A61P 1/02, A61P 1/16, A61P 7/00, A61P 9/00, A61P 11/00, A61P 11/02, A61P 11/04, A61P 13/02, A61P 15/00, A61P 19/02, A61P 27/02, A61P 27/16, A61P 29/00, A61P 31/04, A61P 31/10, A61P 31/12, A61P 37/08, A61P 43/00, C07F 5/02, A61P 17/00

(54) **NOVEL SUBSTITUTED CONDENSED RING COMPOUND**

(30) Priority: 25.10.2019 JP 2019194753
(71) Applicant: Sumitomo Pharma Co., Ltd., Osaka 541-0045 (JP)
(72) Inventor: KOIKE, Sachiko, Osaka-shi, Osaka 554-0022 (JP); IKUMA, Yohei, Osaka-shi, Osaka 554-0022 (JP); FUKAYA, Takayuki, Osaka-shi, Osaka 554-0022 (JP); UESUGI, Shunichiro, Osaka-shi, Osaka 554-0022 (JP)
(74) Representative: Vossius & Partner Patentanwälte Rechtsanwälte mbB
(86) International application number: PCT/JP2020/039897
(87) International publication number: WO 2021/079984

(57) **Abstract**

The present invention provides a novel compound having an excellent β-lactamase inhibitory effect. The present invention provides: a compound which has an excellent β-lactamase inhibitory effect, and is represented by formula (1a), (1b) or (11); or a pharmaceutically acceptable salt thereof. This compound provides a prophylactic or therapeutic agent effective for bacterial infections when used in combination with β-lactam-based drugs or used as a single agent. The present invention also provides a prophylactic or therapeutic agent effective for treating various diseases, by being used in combination with β-lactam-based drugs.

## Description

### [Technical Field]

The present invention relates to a substituted condensed compound that is useful as a medicament or a pharmaceutically acceptable salt thereof. More specifically, the present invention relates to a pharmaceutical composition comprising a novel substituted condensed ring compound or a pharmaceutically acceptable salt thereof, or a medicament comprising the substituted condensed ring compound or a pharmaceutically acceptable salt thereof (therapeutic agent, prophylactic agent, etc.) .

### [Background Art]

Since the discovery of penicillin, antimicrobial agents have taken an important role in the treatment of infections.

In particular, β-lactam agents (e.g., penicillin antimicrobial agents, cephalosporin antimicrobial agents, and carbapenem antimicrobial agents) are agents that are most commonly used in the treatment of bacterial infections in view of their potent sterilizing capacity and high degree of safety. However, with increased use of β-lactam agents, emergence and prevalence of pathogenic bacteria that have acquired resistance to β-lactam agents have become a global problem. Examples of the mechanism of acquiring resistance of such pathogens include production of β-lactamase, structural change in the target molecule of a β-lactam agent, reduced drug permeation into microbial cells, elevated drug discharge, and the like. In particular, production of β-lactamase, which degrades and inactivates β-lactam agents, is one of the most influential in the maintenance of efficacy of β-lactam agents. Various bacteria are involved in the evolution of β-lactamase that antagonizes the efficacy of various β-lactam agents, β-lactamases can be classified into 4 classes based on their amino acid sequences, i.e., Ambler classes A, B, C, and D. Since class A, C, and D enzymes have a serine residue at the center of enzymatic activity, they are known as serine-p-lactamases. Since class B enzymes do not have a serine residue at the center of enzymatic activity but have zinc metal ion (Zn²⁺), they are known as metallo-p-lactamases (zinc-p-lactamases).

It has been already confirmed that concomitant use of an agent for inhibiting β-lactamase and a β-lactam agent is effective for solving the problem of resistance acquisition due to production of β-lactamase. It is known that commercially available agents for inhibiting β-lactamase, clavulanic acid, sulbactam, and tazobactam primarily inhibit class A β-lactamases excluding KPC (Klebsiella pneumoniae Carbapenemase), and avibactam inhibits class A β-lactamases (including KPC), class C β-lactamases, and some class D β-lactamases including OXA-48 (Non Patent Literature 1). However, these existing agents for inhibiting β-lactamase cannot effectively and broadly inhibit all β-lactamases produced by various bacteria. For example, such inhibiting agents do not exert an effect on class B metallo-β lactamases. Recently, β-lactamases called ESBLs (Extended Spectrum β-Lactamases) that can degrade more substrates (β-lactam agent) compared to conventional β-lactamases were isolated, which have led to a problem as a new resistant bacteria, especially as a causative bacteria for hospital-acquired infections in the US and Europe. In addition, emergence and prevalence of metallo-β-lactamas producing bacteria is becoming a problem in Japan. In view of such a circumstance, it is very important to address β-lactamase producing bacteria including ESBLs and metallo-β-lactamase for the prophylaxis of hospital-acquired infections. Furthermore, pathogenic bacteria evolve quickly, such that emergence of new β-lactamase resistant bacteria is very likely. Accordingly, as a solution to such problems or as a safeguard against such issues to be addressed, there is a demand for the development of a novel agent for inhibiting β-lactamase that has a different structure from existing agents for inhibiting β-lactamse, whereby a broader β-lactamase inhibitory action or metallo-β-lactamase inhibitory action is expected.

Recently, boronic acid derivatives with β-lactamase inhibitory action have been reported in Patent Literatures 1 to 13 and the like. Patent Literature 10 discloses a structure formed by fusing a saturated cycloalkyl ring or the like to a ring comprising boronic acid. These Patent Literatures do not disclose a structure related to the novel substituted condensed ring compounds encompassed by the present invention, i.e., a boronic acid compound group having a non-aryl heterocycle (preferably a nitrogen-containing non-aryl heterocycle, and more preferably an azetidine ring) structure having a specific substitution mode on a side chain at a specific position (wherein the specific substitution mode is, for example, a mode having a specific substitution on the non-aryl heterocycle via an alkylene group or a mode having a specific substituent on the non-aryl heterocycle via an oxo substituent (-C(=O)-, -S(=O)-, -S(=O)₂-, or the like) and a structure formed by fusing a cyclopropane ring to a ring comprising boronic acid as a combination.

### [Citation List]

### [Patent Literature]

[PTL 1] WO 2014/107535
[PTL 2] WO 2014/107536
[PTL 3] WO 2015/179308
[PTL 4] WO 2016/003929
[PTL 5] WO 2016/149393
[PTL 6] WO 2014/089365
[PTL 7] WO 2014/110442
[PTL 8] WO 2014/151958
[PTL 9] WO 2015/191907
[PTL 10] WO 2018/005662
[PTL 11] WO 2018/199291
[PTL 12] WO 2019/009369
[PTL 13] WO 2019/009370

### [Non Patent Literature]

[NPL 1] Buynak. JD. Expert Opinion on Therapeutic Patents, 2013, 23(11), 1469-1481.

### [Summary of Invention]

### [Solution to Problem]

The present invention provides a novel compound having excellent β-lactamase inhibitory action and provides a prophylactic or therapeutic agent that is useful for a bacterial infection, alone or in concomitant use with a β-lactam agent. Specifically, the present invention provides a prophylactic or therapeutic agent that is useful for therapy, by concomitant use with a β-lactam agent, of a disease such as sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, deep skin infection, lymphangitis/lymphadenitis, secondary infection of trauma, burn injury, surgical wound, or the like, urinary tract infection, genital infection, eye infection, or odontogenic infection.

More specifically, the inventors completed the present invention by finding that a compound represented by formula (1a), (1b), or (11) described below or a pharmaceutically acceptable salt thereof (also referred to as the "compound of the invention" hereinafter) has excellent β-lactamase inhibitory action.

Specifically, the present invention is the following.

### [Item 1]

A compound represented by formula (1a) or (1b): 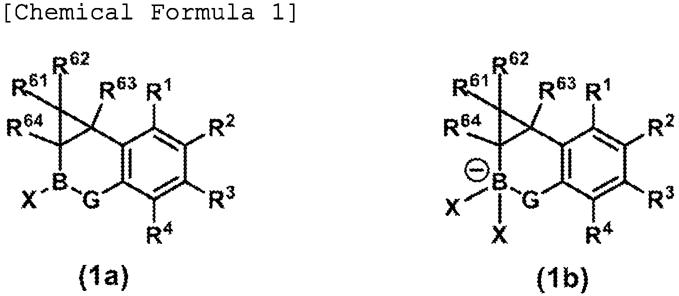 or a pharmaceutically acceptable salt thereof
wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 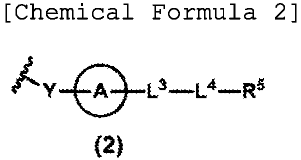 wherein
Y is an oxygen atom, a sulfur atom, or -NR^{j}-, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, optionally substituted -NH-, optionally substituted -NH-SO₂-, -S(=O)-, or -S(=O)₂-,
L⁴ is a single bond, an optionally substituted hydrocarbylene group, an optionally substituted heterohydrocarbylene group, or - C(=N-OR^{h1})-,
R^{h1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
   (wherein each substituent from 2) to 6) is optionally substituted),
R⁵ is a hydrogen atom, a hydroxyl group, an optionally substituted hydrocarbyl group, an optionally substituted heterohydrocarbyl group, optionally substituted -NHOH, a sulfo group (sulfonic acid group), optionally substituted -SO₂H, optionally substituted -SO₂-NH₂, optionally substituted - S(=O)(=NH)H, optionally substituted -NH-SO₂-H, optionally substituted -NH-SO₂-NH₂, optionally substituted -N=S(=O)H₂, or optionally substituted -NH₂,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
   1) -C(=O)R⁸,
   2) -SO₂-L⁶-R⁸, (wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene group),
   3) -NR^{a4}R^{b3},
   4) -B(OR^{m1})_{2,}
   5) -PO(OR^{m1})(OR^{m2}),
   6) optionally substituted heteroaryl,
   7) an optionally substituted non-aryl heterocycle, or
   8) a bioisostere of one of 1) to 7),
   (wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

### [Item 2]

A compound represented by formula (1a) or (1b): 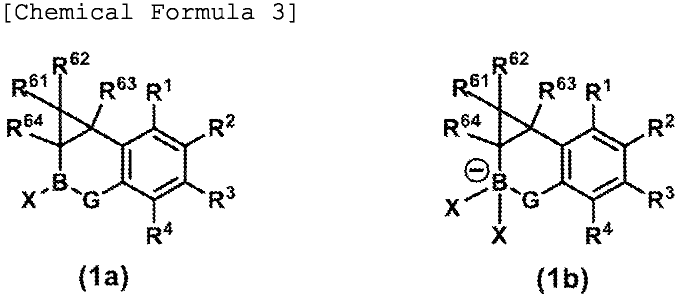 or a pharmaceutically acceptable salt thereof
wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 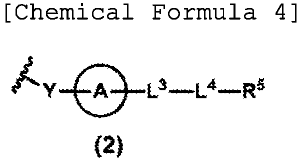 wherein
Y is an oxygen atom, a sulfur atom, or -NR^{j}-, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, -S(=O)-, or -S(=O)₂-,
L⁴ is a single bond, an optionally substituted hydrocarbylene group, or -C(=N-OR^{h1})-,
R^{h1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
   (wherein each substituent from 2) to 6) is optionally substituted),
R⁵ is a hydrogen atom, a hydroxyl group, an optionally substituted hydrocarbyl group, or an optionally substituted heterohydrocarbyl group,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
   1) -C(=O)R⁸,
   2) -SO₂-L⁶-R⁸,
      (wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene,
   3) -NR^{a4}R^{b3},
   4) -B(OR^{m1})₂,
   5) -PO(OR^{m1}) (OR^{m2}),
   6) optionally substituted heteroaryl,
   7) an optionally substituted non-aryl heterocycle, or
   8) a bioisostere of one of 1) to 7),
   (wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

### [Item 3]

The compound or the pharmaceutically acceptable salt thereof according to item 1 or 2, represented by formula (1a) or (1b): 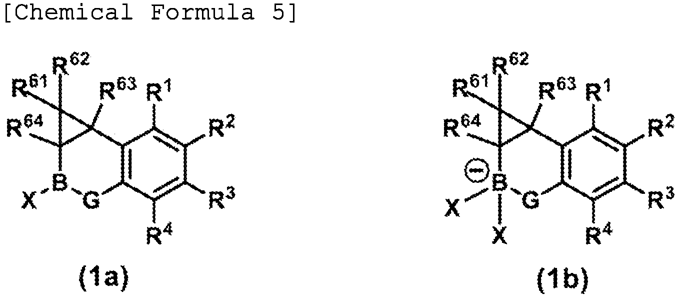 wherein
G is an oxygen atom, a sulfur atom, or -NR^{a1}-,
X is a hydroxyl group, an optionally substituted C₁₋₆ alkoxy group, or -NR^{a2}R^{b1},
R^{a1}, R^{a2}, and R^{b1} are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group, or
   15) -OR^{c1},
      (wherein each substituent from 2) to 14) is optionally substituted),
wherein R^{a2} and R^{b1} together may form an optionally substituted 4- to 10-membered nitrogen-containing non-aryl heterocycle,
R^{c1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
L¹ is a single bond, an oxygen atom, a sulfur atom, -SO-, - SO₂-, -NR^{d1}-, -NR^{d1}C(=O)-, or -NR^{d1}SO₂-,
L² is a single bond or an optionally substituted C₁₋₆ alkylene group,
Z is
   1) a hydrogen atom,
   2) a hydroxyl group,
   3) a cyano group,
   4) a carboxyl group,
   5) a C₃₋₁₀ alicyclic group,
   6) C₆₋₁₀ aryl,
   7) 5- or 6-membered heteroaryl,
   8) a 4- to 10-membered non-aryl heterocycle,
   9) a C₁₋₆ alkoxy group,
   10) a C₃₋₁₀ alicyclic oxy group,
   11) a C₆₋₁₀ aryloxy group,
   12) a 5- or 6-membered heteroaryloxy group,
   13) a 4- to 10-membered non-aryl heterocyclyl oxy group,
   14) a C₁₋₆ alkylthio group,
   15) a C₃₋₁₀ alicyclic thio group,
   16) a C₆₋₁₀ arylthio group,
   17) a 5- or 6-membered heteroarylthio group,
   18) a 4- to 10-membered non-aryl heterocyclyl thio group,
      (wherein each substituent from 5) to 18) is optionally substituted),
   19) -SO₂-NR^{e1}R^{f1},
   20) -NR^{e1}-C(=O)OR^{f1},
   21) -NR^{g1}-C(=O)NR^{e1}R^{f1},
   22) -NR^{e1}-C(=S)R^{f1},
   23) -NR^{e1}-C(=S)OR^{f1},
   24) -NR^{g1}-C(=S)NR^{e1}R^{f1},
   25) -NR^{g1}-CR^{e1}(=NR^{f1}),
   26) -NR^{g1}-CR^{e1}(=N-OR^{f1}),
   27) -NR^{h1}-C(=NR⁹¹)NR^{e1}R^{f1},
   28) -NR^{h1}-C(=N-OR^{g1})NR^{e1}R^{f1},
   29) -NRⁱ¹-C(=NR^{h1})NR^{g1}-NR^{e1}R^{f1},
   30) -NRⁱ¹-C(=N-OR^{h1})NR^{g1}-NR^{e1}R^{f1},
   31) -NR^{e1}-SO₂-R^{f1},
   32) -NR^{g1}-SO₂-NR^{e1}R^{f1},
   33) -C(=O)OR^{e1},
   34) -C(=S)OR^{e1},
   35) -C(=S)NR^{e1}R^{f1},
   36) -C(=S)NR^{e1}OR^{f1},
   37) -C(=S)NR^{g1}-NR^{e1}R^{f1},
   38) -C(=NR^{e1})R^{f1},
   39) -C(=N-OR^{e1})R^{f1},
   40) -C(=NR^{h1})NR^{g1}-NR^{e1}R^{f1},
   41) -C(=N-OR^{h1})NR^{g1}-NR^{e1}R^{f1},
   42) -NR^{e1}R^{f1},
   43) NR^{g1}-NR^{e1}R^{f1},
   44) -NR^{e1}OR^{f1},
   45) -NR^{e1}-C(=O)R^{f1},
   46) -C(=O)NR^{e1}R^{f1},
   47) -C(=O)NR^{e1}OR^{f1},
   48) -C(=O)NR^{g1}-NR^{e1}R^{f1},
   49) -C(=O)R^{e1},
   50) -C(=NR^{g1})NR^{e1}R^{f1}, or
   51) -C(=N-OR^{h1})NR^{e1}R^{f1},
one of R¹, R², and R³ is a group represented by formula (2) : 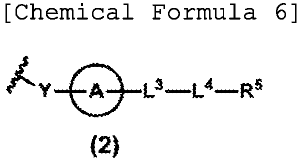 wherein
Y is an oxygen atom, a sulfur atom, or -NR^{j}-,
ring A is an optionally substituted 4- to 20-membered non-aryl heterocycle,
L³ is
   1) an oxygen atom,
   2) a sulfur atom,
   3) -NR^{d2}-,
   4) -NR^{d2}C(=O)-,
   5) -NR^{d2}SO₂-,
   6) a C₁₋₆ alkylene group,
   7) a C₃₋₁₀ cycloalkylene group,
   8) a 4- to 10-membered non-aryl heterocyclylene group,
      (wherein each substituent from 6) to 8) is optionally substituted),
   9) -C(=O)-,
   10) -S(=O)-, or
   11) -S(=O)₂-,
L⁴ is
   1) a single bond,
   2) a C₁₋₆ alkylene group,
   3) a C₃₋₁₀ cycloalkylene group,
   4) a C₆₋₁₀ arylene group,
   5) a 5- or 6-membered heteroarylene group,
   6) a 4- to 10-membered non-aryl heterocyclylene group,
      (wherein each substituent from 2) to 6) is optionally substituted), or
   7) -C(=N-OR^{h1})-,
R⁵ is
   1) a hydrogen atom,
   2) a C₁₋₆ alkyl group,
   3) a C₃₋₁₀ alicyclic group,
   4) a 4- to 10-membered non-aryl heterocycle,
   5) C₆₋₁₀ aryl,
   6) 5- to 10-membered heteroaryl,
   7) a C₁₋₆ alkylthio group,
      (wherein each substituent from 2) to 7) is optionally substituted, and if two substituents further substituted with the substituent of 1), 2), or 3) are each substituted on adjacent atoms within a ring, the two substituents together may further form a condensed ring structure),
   8) -NR^{e1}OH,
   9) a carboxyl group (-C(=O)OH)
   10) a carboxylic acid isostere (wherein the carboxylic acid isostere comprises an ester group-C(=O)OR^{20a}),
   11) a sulfo group (sulfonic acid group),
   12) -SO₂R^{e1},
   13) -SO₂-NR^{e1}R^{f1},
   14) -S(=O)(=NR^{f1})R^{e1},
   15) -NR^{e1}-C(=O)R^{f1},
   16) -NR^{e1}-C(=O)OR^{f1},
   17) -NR^{g1}-C(=O)NR^{e1}R^{f1},
   18) -NR^{e1}-SO₂-R^{f1},
   19) -NR^{g1}-SO₂-NR^{e1}R^{f1},
   20) -N=S(=O)R^{e1}R^{f1},
   21) -C(=O)NR⁵⁰R⁵¹, or
   22) -NR^{e1}R^{f1} (wherein if R⁵ is the substituent of 22), -L³-L⁴-R⁵ is not -(CH₂)₁₋₄NR^{e1}R^{f1} (wherein R^{e1} and R^{f1} are a hydrogen atom, optionally substituted C₁₋₄ alkyl, an optionally substituted C₃₋₇ alicyclic group, an optionally substituted 4- to 10-membered non-aryl heterocyclic group, optionally substituted C₆₋₁₀ aryl, or optionally substituted 5- to 10-membered heteroaryl)),
R20a is
   1) a C₁₋₆ alkyl group,
   2) a C₃₋₁₀ alicyclic group,
   3) C₆₋₁₀ aryl,
   4) 5- or 6-membered heteroaryl, or
   5) a 4- to 10-membered non-aryl heterocycle,
      (wherein each substituent from 1) to 5) is optionally substituted),
R⁵⁰ represents
   1) a hydrogen atom,
   2) a C₁₋₆ alkyl group,
   3) a hydroxyl group,
   4) a C₁₋₆ alkoxy group,
   5) a C₃₋₆ cycloalkoxy group,
   6) a C₃₋₆ alicyclic group,
   7) a 4- to 6-membered non-aryl heterocycle,
   8) C₆₋₁₀ aryl,
   9) 5- to 10-membered heteroaryl
   10) 4- to 6-membered non-aryl heterocyclyl oxy,
   11) C₆₋₁₀ aryloxy,
   12) 5- to 10-membered heteroaryloxy,
   13) a C₁₋₆ alkylsulfonyl group,
   14) a C₃₋₆ cycloalkoxysulfonyl group, or
   15) a 4- to 6-membered non-aryl heterocyclyl sulfonyl group, (wherein each substituent of 2) and 4) to 15) is optionally substituted),
R⁵¹ represents
   1) a hydrogen atom,
   2) a C₁₋₆ alkyl group,
   3) a C₃₋₆ alicyclic group,
   4) a 4- to 6-membered non-aryl heterocycle,
   5) C₆₋₁₀ aryl, or
   6) 5- to 10-membered heteroaryl,
      (wherein each substituent from 2) to 6) is optionally substituted), or
R⁵⁰ and R⁵¹ together may form optionally substituted 4- to 7-membered nitrogen-containing non-aryl heterocycle,
the remaining two (without the structure of formula (2) among R¹, R², and R³) are the same or different, each independently a hydrogen atom, a halogen atom, an optionally substituted C₁₋₆ alkyl group, an optionally substituted C₁₋₆ alkoxy group, an optionally substituted C₁₋₆ alkylthio group, optionally substituted 5- or 6-membered heteroaryl, or -NR^{a3}R^{b2},
R^{d1}, R^{d2}, R^{e1}, R^{f1}, R^{g1}, R^{h1}, Rⁱ¹, and R^{j} are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
a combination of R^{e1} and R^{f1}, when attached to the same nitrogen atom, together may form an optionally substituted 4- to 10-membered nitrogen-containing non-aryl heterocycle,
R⁴ is
   1) -C(=O)R⁸,
   2) -SO₂-L⁶-R⁸,
      (wherein R⁸ in 1) and 2) is -NR^{a5}R^{b4}, -NR^{a5}-L⁷-B(OR^{m1})₂, -OR^{m1}, or an optionally substituted C₁₋₆ alkyl group, and L⁶ is a single bond or -NR^{a6}-),
   3) -NR^{a4}R^{b3},
   4) -B(OR^{m1})₂,
   5) -PO(OR^{m1})(OR^{m2}),
   6) optionally substituted 5-membered heteroaryl,
   7) an optionally substituted 5-membered non-aryl heterocycle, or
   8) a bioisostere of one of 1) to 7),
      (wherein the formulas of 2), 4), 5), and 6) include a carboxylic acid isostere, and 8) may include them in duplicates),
R^{a3}, R^{a4}, R^{a5}, R^{a6}, R^{b2}, R^{b3}, and R^{b4} are the same or different, each independently having the same definition as R^{a1}, R^{a2}, and R^{b1}, wherein a combination of R^{a3} and R^{b2}, R^{a4} and R^{b3}, or R^{a5} and R^{b4}, when attached to the same nitrogen atom, together may form an optionally substituted 4- to 10-membered nitrogen-containing non-aryl heterocycle,
R^{m1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
wherein if R^{m1} is attached to a boron atom via an oxygen atom, two R^{m1}, as C₂₋₄ alkylene, together with the boron atom and two oxygen atoms, may form a 5- to 7-membered non-aryl heterocycle (wherein an alkylene moiety is optionally substituted in the non-aryl heterocycle),
R^{m2} is a hydrogen atom, an optionally substituted C₁₋₆ alkyl group, or an optionally substituted C₃₋₁₀ alicyclic group,
L⁷ is an optionally substituted C₁₋₃ alkylene group,
R⁶¹, R⁶², and R⁶³ are each independently a hydrogen atom, halogen, or an optionally substituted C₁₋₆ alkyl group, and
R⁶⁴ is a hydrogen atom, halogen, an optionally substituted C₁₋₆ alkyl group, or -L¹-L²-Z.

### [Item 4]

The compound or the pharmaceutically acceptable salt thereof according to any one of items 1 to 3, wherein L³ is
1) a C₁₋₆ alkylene group,
2) a C₃₋₁₀ cycloalkylene group, or
3) a 4- to 10-membered non-aryl heterocyclylene group,
   (wherein each substituent from 1) to 3) is optionally substituted), and

L⁴ is a single bond or an optionally substituted C₁₋₅ alkylene group.

### [Item 5]

The compound or the pharmaceutically acceptable salt thereof according to any one of items 1 to 4, wherein
L³ is an optionally substituted C₁₋₄ alkylene group, and
L⁴ is a single bond or an optionally substituted C₁₋₃ alkylene group.

### [Item 6]

The compound or the pharmaceutically acceptable salt thereof according to any one of items 1 to 5, wherein
L³ is -(CR³⁰R³¹)ₙ₁-,
R³⁰ and R³¹ are each independently, or if there are multiple instances of each of them, all of them independently represent
   1) a hydrogen atom,
   2) -NR^{a7}R^{a8},
   3) a C₁₋₄ alkyl group,
   4) C₆₋₁₀ aryl,
   5) 5- to 10-membered heteroaryl,
   6) a C₃₋₆ alicyclic group,
   7) a 4- to 10-membered non-aryl heterocycle,
      (wherein each substituent from 3) to 7) is optionally substituted), or
   8) -OR^{c2}, or
R³⁰ and R³¹, together with the carbon atom to which they are attached, form a C₃₋₆ alicyclic group or a 4- to 6-membered non-aryl heterocycle,
R^{a7} and R^{a8} are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group, or
   15) -OR^{c3},
      (wherein each substituent from 2) to 14) is optionally substituted),
wherein R^{a7} and R^{a8} together may form an optionally substituted 4- to 10-membered nitrogen-containing non-aryl heterocycle,
R^{c2} and R^{c3} are each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
n1 is an integer 1, 2, 3, or 4,
L⁴ is -(CR⁴⁰R⁴¹)ₙ₂-,
R⁴⁰ and R⁴¹ each independently, or if there are multiple instances of each of them, all of them independently represent
   1) a hydrogen atom,
   2) -NR^{a9}R^{a10},
   3) a C₁₋₄ alkyl group,
   4) C₆₋₁₀ aryl,
   5) 5- to 10-membered heteroaryl,
   6) a C₃₋₆ alicyclic group
   7) a 4- to 10-membered non-aryl heterocycle,
      (wherein each substituent from 3) to 7) is optionally substituted), or
   8) -OR^{c4}, or
R⁴⁰ and R⁴¹, together with the carbon atom to which they are attached, form a C₃₋₆ alicyclic group or a 4- to 6-membered non-aryl heterocycle,
R^{a9} and R^{a10} are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group,
      (wherein each substituent from 2) to 14) is optionally substituted), or
   15) -OR^{c5},
wherein R^{a9} and R^{a10} together may form an optionally substituted 4- to 10-membered nitrogen-containing non-aryl heterocycle,
R^{c4} and R^{c5} are each independently defined the same as R^{c2} and R^{c3}, and
n2 is an integer 0 (i.e., when L⁴ is a single bond), 1, 2, or 3.

### [Item 7]

The compound or the pharmaceutically acceptable salt thereof according to any one of items 1 to 6, wherein
-L³-L⁴- is an optionally substituted C₁₋₂ alkylene group.

### [Item 8]

The compound or the pharmaceutically acceptable salt thereof according to any one of items 1 to 7, wherein -L³-L⁴- is a C₁₋₂ alkylene group optionally substituted with a C₁₋₃ alkyl group, an amino group, or a hydroxymethyl group, or a plurality of the same or different groups thereamong (wherein two C₁₋₃ alkyl groups, when attached to the same carbon atom, together with the carbon atom to which they are attached, may form a C₃₋₆ alicyclic group).

### [Item 9]

The compound or the pharmaceutically acceptable salt thereof according to any one of items 1 to 8, wherein
R⁵ is
   1) a C₃₋₁₀ alicyclic group,
   2) C₆₋₁₀ aryl,
   3) 5- to 10-membered heteroaryl,
   4) a C₁₋₆ alkylthio group,
      (wherein each substituent from 1) to 4) is optionally substituted, and if two substituents further substituted with the substituent of 2) or 3) are each substituted on adjacent atoms within a ring, the two substituents together may further form a condensed ring structure),
   5) -NR^{e1}OH,
   6) -C(=O)NR⁵⁰R⁵¹,
   7) -SO₂-NR^{e1}R^{f1},
   8) -NR^{e1}-SO₂-R^{f1},
   9) -C(=O)OR²⁰, or
   10) -NR^{e1}R^{f1} (wherein if R⁵ is the substituent of 10), L³ and/or L⁴ is a C₁₋₆ alkylene group that is necessarily substituted with one or more groups other than a hydrogen atom (wherein L³ or L⁴, together with said substituent, may form a C₃₋₁₀ alicyclic group or a 4- to 10-membered non-aryl heterocycle)), and
R²⁰ is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted).

### [Item 10]

The compound or the pharmaceutically acceptable salt thereof according to any one of items 1 to 9, wherein
R⁵ is
1) C₆₋₁₀ aryl,
2) 5- to 10-membered heteroaryl,
3) -C(=O)NR⁵⁰R⁵¹,
4) -C(=O)OR²⁰, or
5) -NR^{e1}R^{f1} (wherein if R⁵ is the substituent of 5), L³ and/or L⁴ is a C₁₋₆ alkylene group that is necessarily substituted with one or more groups other than a hydrogen atom, and together with said substituent forms at least one C₃₋₁₀ alicyclic group or 4- to 10-membered non-aryl heterocycle)
   (wherein each substituent from 1) to 2) is optionally substituted, and any two groups thereof, when each is attached to adjacent atoms within a ring, together may further form a condensed ring structure).

### [Item 11]

The compound or the pharmaceutically acceptable salt thereof according to any one of items 1 to 10, wherein Z-L²-L¹ is an optionally substituted C₁₋₆ alkylthio group.

### [Item 12]

The compound or the pharmaceutically acceptable salt thereof according to any one of items 1 to 11, wherein R⁶¹, R⁶², R⁶³, and R⁶⁴ are each independently a hydrogen atom, a fluorine atom, or a C₁₋₃ alkyl group optionally substituted with a fluorine atom.

### [Item 13]

The compound or the pharmaceutically acceptable salt thereof according to any one of items 1 to 12, wherein R⁶¹, R⁶², R⁶³, and R⁶⁴ are each independently a hydrogen atom or a fluorine atom.

### [Item 14]

The compound or the pharmaceutically acceptable salt thereof according to any one of items 1 to 13, wherein G is an oxygen atom.

### [Item 15]

The compound or the pharmaceutically acceptable salt thereof according to any one of items 1 to 14, wherein X is a hydroxyl group or an optionally substituted C₁₋₆ alkoxy group.

### [Item 16]

The compound or the pharmaceutically acceptable salt thereof according to any one of items 1 to 15, wherein X is a hydroxyl group.

### [Item 17]

The compound or the pharmaceutically acceptable salt thereof according to any one of items 1 to 16, wherein the compounds of formulas (1a) and (1b) are represented by formulas (3a) and (3b), respectively: 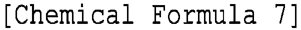 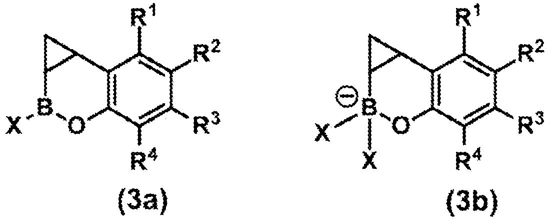 wherein X, R¹, R², and R³ are defined the same as any one of items 1 to 16, and
R⁴ is selected from the group consisting of
1) -C(=O)OR^{m1} (wherein R^{m1} is a hydrogen atom, a C₁₋₆ alkyl group, a C₃₋₁₀ alicyclic group, C₆₋₁₀ aryl, 5- or 6-membered heteroaryl, or a 4- to 10-membered non-aryl heterocycle, wherein the C₁₋₆ alkyl group, the C₃₋₁₀ alicyclic group, the C₆₋₁₀ aryl, the 5- or 6-membered heteroaryl, and the 4- to 10-membered non-aryl heterocycle are each optionally substituted), and
2) a bioisostere of 1).

### [Item 18]

The compound or the pharmaceutically acceptable salt thereof according to any one of items 1 to 17, wherein R⁴ is 1) -C(=O)OH (i.e., a carboxyl group), or 2) a carboxylic acid isostere.

### [Item 19]

The compound or the pharmaceutically acceptable salt thereof according to any one of items 1 to 18, wherein the compounds of formulas (1a) and (1b) or the compounds of formulas (3a) and (3b) are represented by formulas (4a) and (4b), respectively: 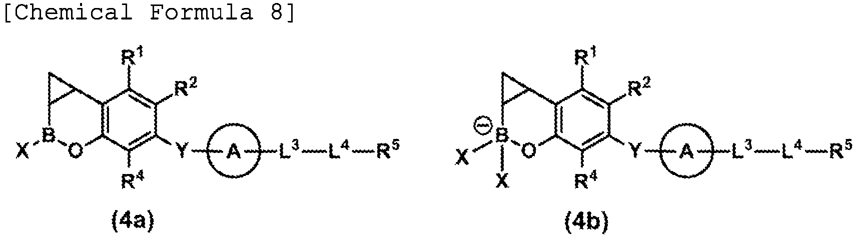 wherein X, R⁴, Y, ring A, L³, L⁴, and R⁵ are defined the same as any one of items 1 to 18, and
R¹ and R² are the same or different, each independently a hydrogen atom, a halogen atom, a C₁₋₆ alkyl group, a C₁₋₆ alkoxy group, or a C₁₋₆ alkylthio group (wherein the C₁₋₆ alkyl group, C₁₋₆ alkoxy group, and C₁₋₆ alkylthio group are optionally substituted).

### [Item 20]

The compound or the pharmaceutically acceptable salt thereof according to any one of items 1 to 19, wherein ring A is an optionally substituted 4- to 10-membered non-aryl heterocycle.

### [Item 21]

The compound or the pharmaceutically acceptable salt thereof according to any one of items 1 to 20, wherein ring A is an optionally substituted 4- to 7-membered non-aryl heterocycle.

### [Item 22]

The compound or the pharmaceutically acceptable salt thereof according to any one of items 1 to 21, wherein Y is an oxygen atom or a sulfur atom.

### [Item 23]

The compound or the pharmaceutically acceptable salt thereof according to any one of items 1 to 22, wherein Y is an oxygen atom.

### [Item 24]

The compound or the pharmaceutically acceptable salt thereof according to any one of items 1 to 23, wherein the compounds of formulas (1a) and (1b), the compounds of formulas (3a) and (3b), or the compounds of formulas (4a) and (4b) are represented by formulas (5a) and (5b), respectively: 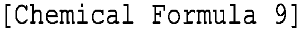 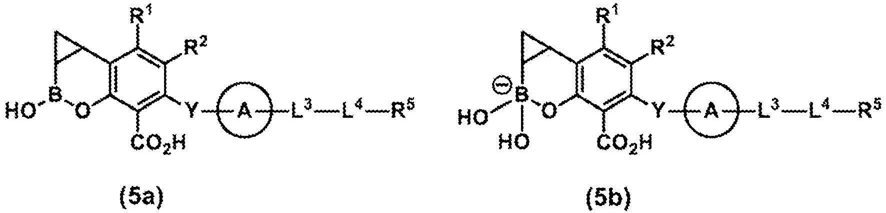 wherein R¹, R², Y, L³, L⁴, and R⁵ are defined the same as any one of items 1 to 23, and ring A is an optionally substituted 4- to 6-membered nitrogen-containing non-aryl heterocycle.

### [Item 25]

The compound or the pharmaceutically acceptable salt thereof according to any one of items 1 to 3 and 11 to 24, wherein L³ is - C(=O)- or -S(=O)₂-.

### [Item 26]

The compound or the pharmaceutically acceptable salt thereof according to any one of items 1 to 3 and 11 to 25, wherein L³ is - C(=O)-.

### [Item 27]

The compound or the pharmaceutically acceptable salt thereof according to any one of items 1 to 3 and 11 to 26, wherein L⁴ is a single bond, -C (=N-OR^{h1}) -, or an optionally substituted C₁₋₆ alkylene group, wherein R^{h1} is an optionally substituted C₁₋₆ alkyl group.

### [Item 28]

The compound or the pharmaceutically acceptable salt thereof according to any one of items 1 to 27, wherein R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
   (wherein each substituent from 3) to 5) is optionally substituted).

### [Item 29]

The compound or the pharmaceutically acceptable salt thereof according to any one of items 1 to 28, wherein R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
   (wherein each substituent from 3) to 5) is optionally substituted with a halogen atom).

### [Item 30]

The compound or the pharmaceutically acceptable salt thereof according to any one of items 1 to 29, wherein R¹ and R² are the same or different, each independently selected from the group consisting of
1) a hydrogen atom,
2) a halogen atom, and
3) a C₁₋₆ alkyl group optionally substituted with a halogen atom.

### [Item 31]

The compound or the pharmaceutically acceptable salt thereof according to any one of items 1 to 30, wherein R¹ and R² are the same or different, each independently selected from the group consisting of
1) a hydrogen atom,
2) a fluorine atom, and
3) a C₁₋₃ alkyl group optionally substituted with a fluorine atom.

### [Item 32]

The compound or the pharmaceutically acceptable salt thereof according to any one of items 1 to 31, wherein R¹ and R² are both hydrogen atoms.

### [Item 33]

The compound or the pharmaceutically acceptable salt thereof according to any one of items 1 to 32, wherein the compounds of formulas (1a) and (1b), the compounds of formulas (3a) and (3b), the compounds of formulas (4a) and (4b), or the compounds of formulas (5a) and (5b) are represented by formulas (6a) and (6b), respectively: 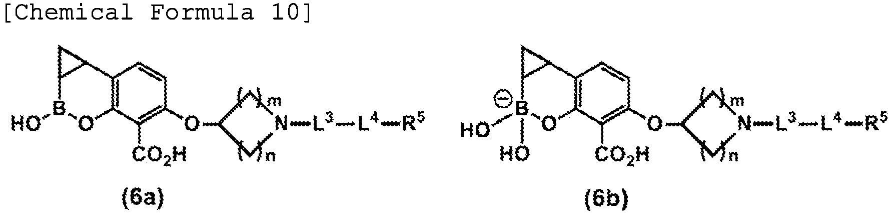 wherein
L³, L⁴, and R⁵ are defined the same as any one of items 1 to 32,
m is an integer 1, 2, or 3,
n is an integer 1, 2, or 3, and
m + n is 2, 3, or 4.

### [Item 34]

The compound or the pharmaceutically acceptable salt thereof according to item 33, wherein the compounds of formulas (6a) and (6b) are enantiomers represented by
formulas (7a) and (7b): 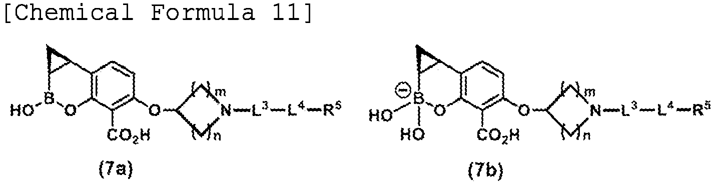 or
formulas (8a) and (8b): 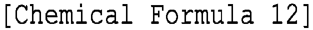 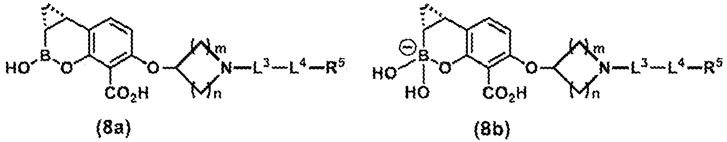 , respectively wherein
L³, L⁴, and R⁵ are defined the same as any one of items 1 to 33, and
m and n are defined the same as item 33.

### [Item 35]

The compound or the pharmaceutically acceptable salt thereof according to any one of items 1 to 34, wherein m is 1 or 2, n is 1 or 2, and m + n is 2 or 3.

### [Item 36]

The compound or the pharmaceutically acceptable salt thereof according to any one of items 1 to 35, wherein m is 1, and n is 1.

### [Item 37]

The compound or the pharmaceutically acceptable salt thereof according to any one of items 1 to 6, 9 to 24, and 28 to 36, wherein
L³ is -(CR³⁰R³¹)ₙ₁- or 4- to 10-membered non-aryl heterocyclylene,
R³⁰ and R³¹ each independently, or if there are multiple instances of each of them, all of them independently represent
   1) a hydrogen atom,
   2) an optionally substituted C₁₋₄ alkyl group, or
   3) optionally substituted C₆₋₁₀ aryl,
n1 is 1, 2, or 3,
L⁴ is a single bond or -(CR⁴⁰R⁴¹)ₙ₂-,
R⁴⁰ and R⁴¹ each independently, or if there are multiple instances of each of them, all of them independently represent
   1) a hydrogen atom,
   2) -NR^{a9}R^{a10},
   3) an optionally substituted C₁₋₄ alkyl group, or
   4) -OR^{c4} or
R⁴⁰ and R⁴¹, together with the carbon atom to which they are attached, form a C₃₋₆ alicyclic group or a 4- to 6-membered non-aryl heterocycle,
R^{a9}, R^{a10}, and R^{c4} are the same or different, each independently
   1) a hydrogen atom,
   2) an optionally substituted C₁₋₆ alkyl group, or
   3) an optionally substituted C₃₋₁₀ alicyclic group,
      wherein R^{a9} and R^{a10} together may form an optionally substituted 4-to 10-membered nitrogen-containing non-aryl heterocycle, and
n2 is 0, 1, or 2.

### [Item 38]

The compound or the pharmaceutically acceptable salt thereof according to item 37, wherein n1 is 1 or 2, and n2 is 0 or 1.

### [Item 39]

The compound or the pharmaceutically acceptable salt thereof according to any one of items 1 to 6, 9 to 24, and 28 to 38, wherein
L³ is -CR³⁰R³¹- or 4- to 10-membered non-aryl heterocyclylene,
R³⁰ and R³¹ each independently represent,
   1) a hydrogen atom,
   2) a C₁₋₄ alkyl group (wherein the group is optionally substituted with halogen, -NR^{a11}R^{a12}, or -OR^{c6}), or
   3) C₆ aryl (wherein the group is optionally substituted with halogen, -NR^{a13}R^{a14}, -OR^{c7}, or a C₁₋₃ alkyl group (wherein the group is optionally substituted with halogen, -NR^{a15}R^{a16}, or -OR^{c8})),
L⁴ is a single bond or -(CR⁴⁰R⁴¹)ₙ₂-,
R⁴⁰ and R⁴¹ each independently represent,
   1) a hydrogen atom,
   2) -NR^{a9}R^{a10},
   3) a C₁₋₄ alkyl group (wherein the group is optionally substituted with halogen, -NR^{a17}R^{a18}, or -OR^{c9}), or
   4) -OR^{c4}, or
R⁴⁰ and R⁴¹, together with the carbon atom to which they are attached, form a C₃₋₆ alicyclic group,
R^{a9}, R^{a10}, R^{a11}, R^{a1}**²,** R^{a1}**³,** R^{a14}, R^{a1}**⁵,** R^{a1}**⁶,** R^{a1}**⁷,** R^{a18}, R^{c4}, R^{c6}, R^{c7}, R^{c8}, and R^{c9} are the same or different, each independently representing
   1) a hydrogen atom, or
   2) a C₁₋₄ alkyl group (wherein the group is optionally substituted with halogen, -NR^{a19}R^{a20}, or -OR^{c10}),
R^{a19}, R^{a20}, and R^{c10} are the same or different, each independently representing
   1) a hydrogen atom, or
   2) a C₁₋₄ alkyl group (wherein the group is optionally substituted with halogen),
wherein each combination of R^{a9} and R^{a10}, R^{a11} and R^{a12}, R^{a13} and R^{a14}, R^{a15} and R^{a16}, R^{a17} and R^{a18}, or R^{a19} and R^{a20} together may form an optionally substituted 4- to 7-membered nitrogen-containing non-aryl heterocycle, and
n2 is 0 or 1.

### [Item 40]

The compound or the pharmaceutically acceptable salt thereof according to any one of items 1 to 6, 9 to 24, and 28 to 39, wherein
L³ is -CH₂-, -CH(CH₂NH₂)-, or -CH(CH₂OH)-, and
L⁴ is a single bond, -CH₂-, -CH(NH₂)-, -CMe(NH₂)-, -CEt(NH₂)-, -C(iso-Pr) (NH₂)-, -CH(CH₂NH₂)-, -CH(OH)-, -CH(CH₂OH) -, -C(CH₂OH)₂-, or

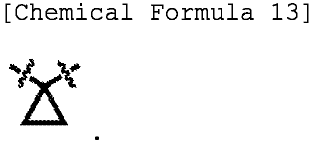

### [Item 41]

The compound or the pharmaceutically acceptable salt thereof according to any one of items 1 to 6, 9 to 24, and 28 to 39, wherein L³ is 4- to 10-membered non-aryl heterocyclylene.

### [Item 42]

The compound or the pharmaceutically acceptable salt thereof according to any one of items 1 to 6, 9 to 24, 28 to 39, and 41, wherein L³ is 5-membered non-aryl heterocyclylene.

### [Item 43]

The compound or the pharmaceutically acceptable salt thereof according to any one of items 1 to 6, 9 to 24, 28 to 39, and 41 to 42, wherein L³ is 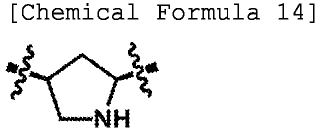 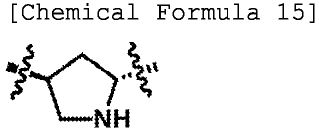 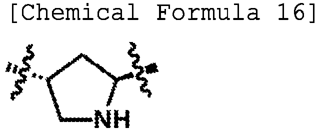 or

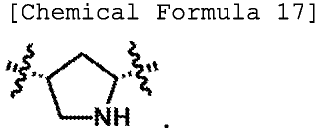

### [Item 44]

The compound or the pharmaceutically acceptable salt thereof according to any one of items 1 to 6, 9 to 24, 28 to 39, and 41 to 43, wherein L⁴ is a single bond.

### [Item 45]

The compound or the pharmaceutically acceptable salt thereof according to any one of items 1 to 24 and 28 to 40, wherein R⁵ is
1) C₆₋₁₀ aryl, or
2) 5- to 10-membered heteroaryl,
   (wherein each substituent from 1) to 2) is optionally substituted, and if two substituents further substituted with the substituent of 1) or 2) are each substituted on adjacent atoms within a ring, the two substituents together may further form a condensed ring structure).

### [Item 46]

The compound or the pharmaceutically acceptable salt thereof according to any one of items 1 to 24 and 28 to 45, wherein
R⁵ is C₆ aryl (i.e., phenyl) or 5-membered, 6-membered, 9-membered, or 10-membered heteroaryl,
each group of R⁵ is optionally substituted with each of R^{6a} or R^{6b} at all chemically substitutable positions on a carbon atom or a nitrogen atom within a ring thereof,
wherein R^{6a}, which are substituents on the carbon atom, if there are multiple instances on the same ring, are all independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e2}R^{f2},
   13) -NR^{g2}-CR^{e2}(=NR^{f2}),
   14) -NR^{g2}-CR^{e2}(=N-OR^{f2}),
   15) -NR^{h2}-C(=NR^{g2})NR^{e2}R^{f2},
   16) -NR^{h2}-C(=N-OR^{g2})NR^{e2}R^{f2},
   17) -NRⁱ²-C(=NR^{h2})NR^{g2}-NR^{e2}R^{f2},
   18) -NRⁱ²-C(=N-OR^{h2})NR^{g2}-NR^{e2}R^{f2},
   19) -C(=NR^{e2})R^{f2},
   20) -C(=N-OR^{e2})R^{f2},
   21) -C(=NR^{h2})-NR^{e2}R^{f2},
   22) -C(=NR^{h2})NR^{g2}-NR^{e2}R^{f2},
   23) -C(=N-OR^{h2})NR^{g2}-NR^{e2}R^{f2},
   24) -NR^{e2}R^{f2},
   25) -NR^{g2}-NR^{e2}R^{f2},
   26) -NR^{e2}OR^{f2},
   27) -NR^{e2}-C(=O)R^{f2},
   28) -C(=O)NR^{e2}R^{f2},
   29) -C(=O)NR^{e2}OR^{f2},
   30) -C(=O)NR^{g2}-NR^{e2}R^{f2},
   31) -C(=O)R^{e2},
   32) -C(=O)OR^{e2}, and
   33) -C(=N-OR^{h2})NR^{e2}R^{f2},
R^{6b}, which are substituents on the nitrogen atom, if there are multiple instances on the same ring, are all independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C(=NR^{e2})R^{f2},
   6) -C(=N-OR^{e2})R^{f2},
   7) -SO₂-NR^{e2}R^{f2},
   8) -C(=NR^{h2})-NR^{e2}R^{f2},
   9) -C(=NR^{h2})NR^{g2}-NR^{e2}R^{f2},
   10) -C(=N-OR^{h2})NR^{h2}-NR^{e2}R^{f2},
   11) -C(=O)NR^{e2}R^{f2},
   12) -C(=O)NR^{e2}OR^{f2},
   13) -C(=O)NR^{g2}-NR^{e2}R^{f2},
   14) -C(=O)R^{e2}, and
   15) -C(=N-OR^{h2})NR^{e2}R^{f2}, or
if a combination of two R^{6a} or R^{6a} and R^{6b} are each substituted on adjacent atoms within a ring, the two substituents together may form an optionally substituted 5- to 6-membered heteroaryl ring or an optionally substituted 5- to 7-membered non-aryl heterocycle, which further fuses to an attachment moiety between the adjacent atoms within the ring,
R^{e2}, R^{f2}, R^{g2}, R^{h2}, and Rⁱ² are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted), and
a combination of R^{e2} and R^{f2}, when attached to the same nitrogen atom, together may form an optionally substituted 4- to 10-membered nitrogen-containing non-aryl heterocycle.

### [Item 47]

The compound or the pharmaceutically acceptable salt thereof according to any one of items 1 to 24 and 28 to 46, wherein
R⁵ is C₆ aryl or 5-membered, 6-membered, 9-membered, or 10-membered heteroaryl selected from the group consisting of 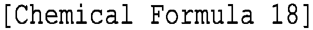 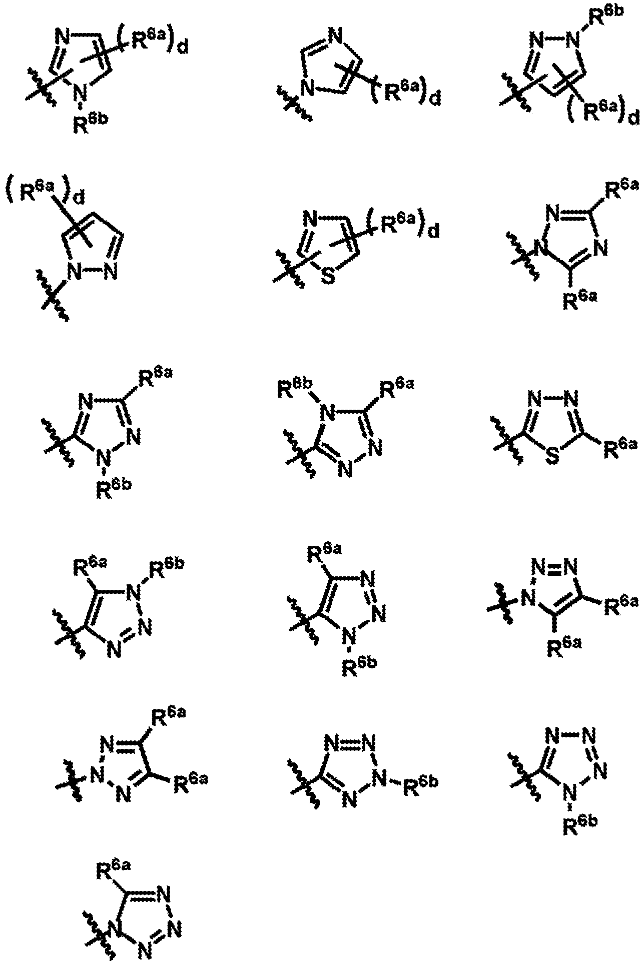 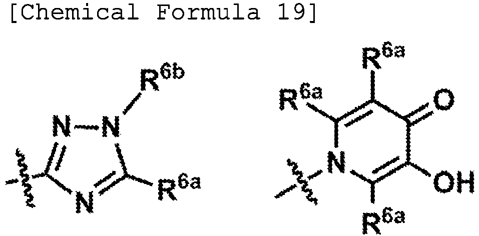 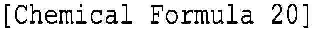 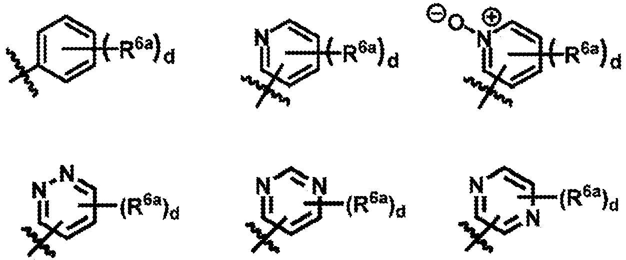 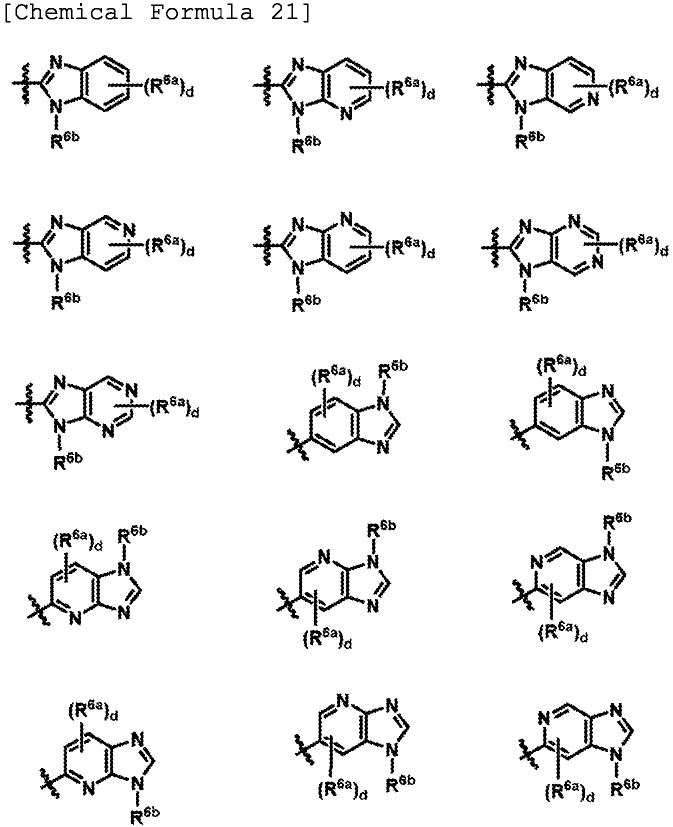 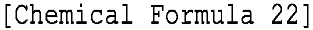 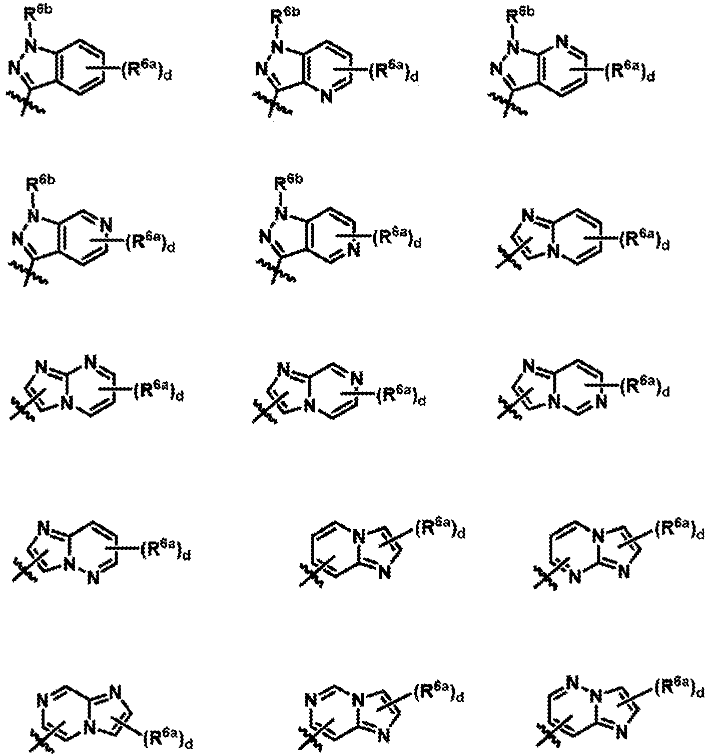 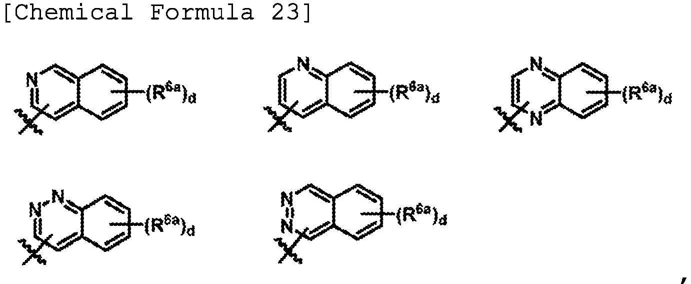
d is the number of chemically substitutable positions on a ring of each group by R^{6a}, and
each R^{6a} and each R^{6b} are defined the same as item 46.

### [Item 48]

The compound or the pharmaceutically acceptable salt thereof according to item 46 or 47, wherein
R^{6a}, if there are multiple instances on the same ring, are all independently selected from the group consisting of
   1) a hydrogen atom,
   2) a hydroxyl group,
   3) halogen,
   4) a C₁₋₄ alkyl group
      (wherein the alkyl group is optionally substituted with NR^{e2}R^{f2}, - C(=O)OR^{f2}, -C(=O)NR^{e2}R^{f2}, -C(=O)NR^{e2}(OR^{f2}), or a hydroxyl group),
   5) a C₁₋₄ alkoxy group
   6) -NR^{e2}R^{f2},
   7) -C(=O)NR^{e2}R^{f2}, and
   8) -C(=O)OR^{e2}, and
each of R^{6b}, if there are multiple instances on the same ring, are all independently selected from the group consisting of
   1) a hydrogen atom,
   2) a hydroxyl group, and
   3) a C₁₋₄ alkyl group
      (wherein the alkyl group is optionally substituted with NR^{e2}R^{f2}, - C(=O)OR^{f2}, -C(=O)NR^{e2}R^{f2}, -C(=O)NR^{e2}(OR^{f2}), or a hydroxyl group).

### [Item 49]

The compound or the pharmaceutically acceptable salt thereof according to any one of items 1 to 24 and 28 to 48, wherein R^{e2} and R^{f2} are the same or different, each independently a hydrogen atom, an optionally substituted C₁₋₆ alkyl group, or an optionally substituted C₃₋₁₀ alicyclic group.

### [Item 50]

The compound or the pharmaceutically acceptable salt thereof according to any one of items 1 to 24 and 28 to 49, wherein R^{e2} and R^{f2} are the same or different, each independently a hydrogen atom or an optionally substituted C₁₋₆ alkyl group.

### [Item 51]

The compound or the pharmaceutically acceptable salt thereof according to any one of items 1 to 24 and 28 to 50, wherein R^{e2} and R^{f2} are hydrogen atoms.

### [Item 52]

The compound or the pharmaceutically acceptable salt thereof according to any one of items 46 to 51, wherein R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

### [Item 53]

The compound or the pharmaceutically acceptable salt thereof according to any one of items 1 to 24 and 28 to 44, wherein R⁵ is -C(=O)NR⁵⁰R⁵¹.

### [Item 54]

The compound or the pharmaceutically acceptable salt thereof according to any one of items 1 to 24, 28 to 40, and 53, wherein
R⁵⁰ represents
   1) a hydrogen atom,
   2) an optionally substituted C₁₋₄ alkyl group,
   3) a hydroxyl group,
   4) an optionally substituted C₁₋₄ alkoxy group, or
   5) an optionally substituted C₁₋₆ alkylsulfonyl group,
R⁵¹ represents
   1) a hydrogen atom, or
   2) an optionally substituted C₁₋₄ alkyl group, or
R⁵⁰ and R⁵¹ together may form a 4- to 6-membered nitrogen-containing non-aryl heterocycle.

### [Item 55]

The compound or the pharmaceutically acceptable salt thereof according to any one of items 1 to 24, 28 to 40, and 53 to 54, wherein
R⁵⁰ represents
   1) a hydrogen atom,
   2) a C₁₋₄ alkyl group (wherein the group is optionally substituted with halogen, a C₃₋₆ alicyclic group, -NR^{a21}R^{a22}, or -OR^{c11}),
   3) a hydroxyl group,
   4) a C₁₋₄ alkoxy group (wherein the group is optionally substituted with halogen, a C₃₋₆ alicyclic group, -NR^{a23}R^{a24}, or -OR^{c12}), or
   5) a C₁₋₄ alkylsulfonyl group (wherein the group is optionally substituted with halogen, a C₃₋₆ alicyclic group, -NR^{a21}R^{a22}, or - OR^{c11}),
R^{a21}, R^{a22}, R^{a23}, R^{a24}, R^{c11}, and R^{c12} are the same or different, each independently representing
   1) a hydrogen atom, or
   2) a C₁₋₄ alkyl group (wherein the group is optionally substituted with halogen),
wherein each combination of R^{a21} and R^{a22} or R^{a23} and R^{a24} together may form an optionally substituted 4- to 7-membered nitrogen-containing non-aryl heterocycle, and
R⁵¹ is a hydrogen atom or C₁₋₄ alkyl (wherein the group is optionally substituted with halogen).

### [Item 56]

The compound or the pharmaceutically acceptable salt thereof according to any one of items 1 to 24, 28 to 40, and 53 to 55, wherein
R⁵⁰ represents
   1) a hydrogen atom,
   2) a C₁₋₄ alkyl group (wherein the group is optionally substituted with a cyclopropyl group, -NH₂, -NHMe, -C(=O)NH₂, -C(=O)NHMe, - C(=O)NMe₂, or a -hydroxyl group),
   3) a hydroxyl group,
   4) a C₁₋₄ alkoxy group (wherein the group is optionally substituted with a cyclopropyl group, -NH₂, -NHMe, or a -hydroxyl group), or
   5) a C₁₋₄ alkylsulfonyl group, and
R⁵¹ is a hydrogen atom.

### [Item 57]

The compound or the pharmaceutically acceptable salt thereof according to any one of items 1 to 24 and 28 to 40, wherein R⁵ is -C(=O)OR²⁰.

### [Item 58]

The compound or the pharmaceutically acceptable salt thereof according to item 57, wherein R²⁰ is
1) a hydrogen atom, or
2) an optionally substituted C₁₋₄ alkyl group.

### [Item 59]

The compound or the pharmaceutically acceptable salt thereof according to item 56 or 58, wherein
L³ is -CH₂-, and
L⁴ is a single bond, -CH(NH₂)-, or -CMe(NH₂)-.

### [Item 60]

The compound or the pharmaceutically acceptable salt thereof according to item 59, wherein L⁴ is 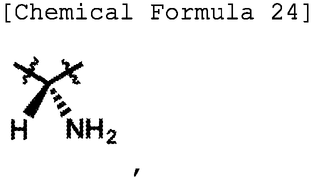 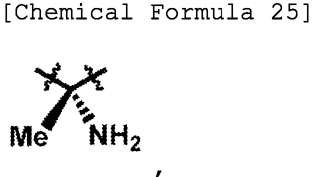 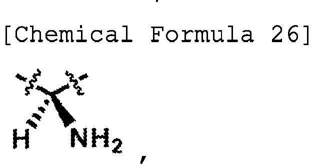 or

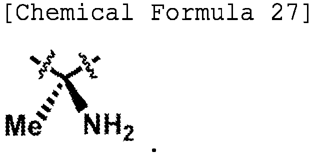

### [Item 61]

The compound or the pharmaceutically acceptable salt thereof according to item 59 or 60, wherein L⁴ is 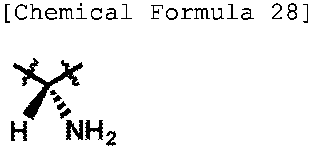 or

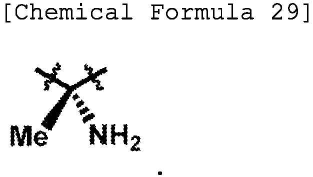

### [Item 62]

The compound or the pharmaceutically acceptable salt thereof according to any one of items 57 to 61, wherein R²⁰ is a hydrogen atom.

### [Item 63]

The compound or the pharmaceutically acceptable salt thereof according to any one of items 1 to 24 and 28 to 40, wherein
R⁵ is -NR^{e1}R^{f1},
L³ is -CH₂-,
L⁴ is -CR⁴⁰R⁴¹-, and
R⁴⁰ and R⁴¹ are each independently a C₁₋₄ alkyl group substituted with a hydroxyl group, or together with the carbon atom to which they are attached, form a C₃₋₆ alicyclic group.

### [Item 64]

The compound or the pharmaceutically acceptable salt thereof according to item 63, wherein
R^{e1} and R^{f1} are the same or different, each independently
   1) a hydrogen atom, or
   2) an optionally substituted C₁₋₃ alkyl group, and
L⁴ is

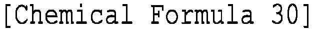

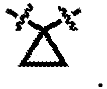

### [Item 65]

The compound or the pharmaceutically acceptable salt thereof according to item 63, wherein
R^{e1} and R^{f1} are the same or different, each independently
   1) a hydrogen atom, or
   2) an optionally substituted C₁₋₃ alkyl group, and
L⁴ is a C₁₋₄ alkylene group substituted with a hydroxyl group.

### [Item 66]

The compound or the pharmaceutically acceptable salt thereof according to any one of items 1 to 3 and 11 to 36, wherein L⁴ is a single bond, or a C₁₋₆ alkylene group optionally substituted with - NR²¹R²² or =NOR²³, wherein R²¹, R²², and R²³ are each independently a hydrogen atom, an optionally substituted C₁₋₆ alkyl group, or an optionally substituted 4- to 10-membered non-aryl heterocyclyl carbonyl group.

### [Item 67]

The compound or the pharmaceutically acceptable salt thereof according to any one of items 1 to 3, 11 to 36, and 66, wherein L⁴ is a single bond, -CH₂-, -CH(NH₂)-, -CMe(NH₂)-, -CH(NHMe)-, - CD(NH₂)-(wherein D represents a heavy hydrogen atom), -CH₂CH₂-, or -CH(NH₂)-CH₂-, wherein if an amino group is present in L⁴, carbon that attaches to the amino group attaches to L³.

### [Item 68]

The compound or the pharmaceutically acceptable salt thereof according to item 67, wherein
L³ is -C(=O)-, and
L⁴ is a single bond, -CH₂-, -CH(NH₂)-, -CMe(NH₂)-, or - CH(NH₂)-CH₂-.

### [Item 69]

The compound or the pharmaceutically acceptable salt thereof according to item 68, wherein L⁴ is one of the following isomeric structures: 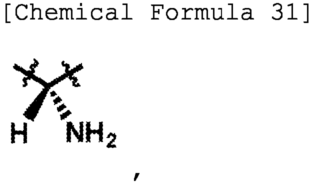 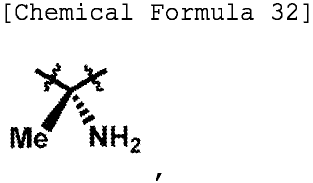 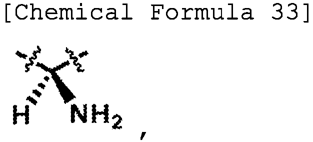 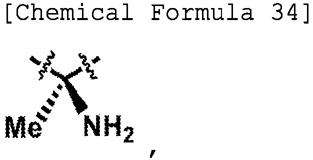 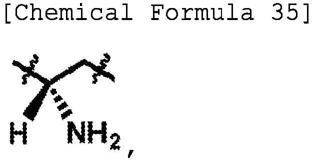 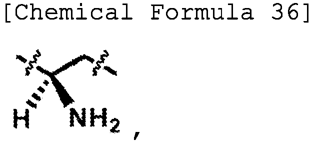 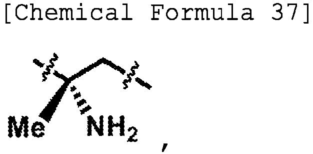 or

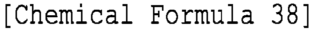

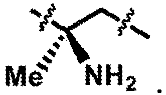

### [Item 70]

The compound or the pharmaceutically acceptable salt thereof according to item 68 or 69, wherein L⁴ is 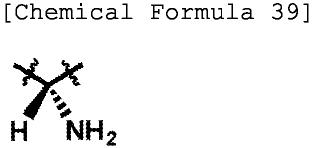 or

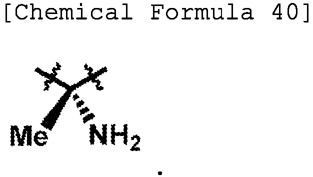

### [Item 71]

The compound or the pharmaceutically acceptable salt thereof according to item 68 or 69, wherein L⁴ is 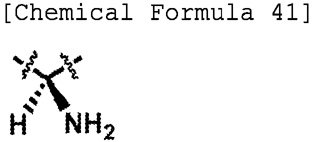 or

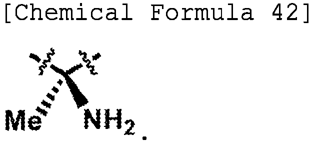

### [Item 72]

The compound or the pharmaceutically acceptable salt thereof according to item 68 or 69, wherein L⁴ is 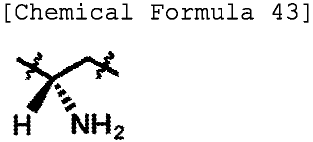 or

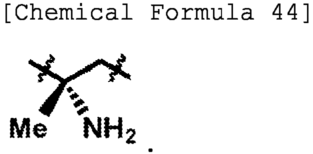

### [Item 73]

The compound or the pharmaceutically acceptable salt thereof according to item 68 or 69, wherein L⁴ is 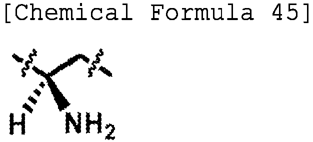 or

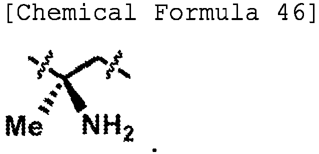

### [Item 74]

The compound or the pharmaceutically acceptable salt thereof according to any one of items 1 to 3, 11 to 36, and 66 to 73, wherein R⁵ is a hydrogen atom, an optionally substituted C₁₋₆ alkyl group, an optionally substituted C₃₋₁₀ alicyclic group, an optionally substituted 4- to 10-membered non-aryl heterocycle, optionally substituted C₆₋₁₀ aryl, optionally substituted 5- or 6-membered heteroaryl, an optionally substituted C₁₋₆ alkylthio group, or -NR^{e1}OH, wherein R^{e1} is a hydrogen atom or an optionally substituted C₁₋₆ alkyl group.

### [Item 75]

The compound or the pharmaceutically acceptable salt thereof according to any one of items 1 to 3, 11 to 36, and 66 to 74, wherein R⁵ is optionally substituted 5- or 6-membered heteroaryl or optionally substituted C₆₋₁₀ aryl.

### [Item 76]

The compound or the pharmaceutically acceptable salt thereof according to any one of items 1 to 3, 11 to 36, and 66 to 75, wherein R⁵ is optionally substituted 5- or 6-membered heteroaryl.

### [Item 77]

The compound or the pharmaceutically acceptable salt thereof according to any one of items 1 to 3, 11 to 36, and 66 to 76, wherein R⁵ is an optionally substituted C₄₋₁₀ alicyclic group or an optionally substituted 4- to 10-membered non-aryl heterocycle.

### [Item 78]

The compound or the pharmaceutically acceptable salt thereof according to any one of items 1 to 3, 11 to 36, and 66 to 74, wherein L⁴ is a single bond and R⁵ is -NR^{e1}OH, wherein R^{e1} is a hydrogen atom or an optionally substituted C₁₋₆ alkyl group.

### [Item 79]

The compound or the pharmaceutically acceptable salt thereof according to any one of items 1 to 3 and 11 to 36, wherein
L⁴ is
   1) - (CH₂)ₚ-CR¹⁰(NHR¹¹) -,
   2) -(CH₂)_{q}-CR¹²R¹³-, or
   3) - (CH₂)ₚ-CR¹⁰ (NHR¹¹) - (CH₂)_{q}-CR¹²R¹³- (wherein p and q are independently 0 or 1),
R¹⁰ is
   1) a hydrogen atom,
   2) a carboxyl group, or
   3) -C(=O)NR^{10a}R^{10b},
R¹¹ is
   1) a hydrogen atom,
   2) -C (=O) R^{11a}, or
   3) an optionally substituted 5- or 6-membered non-aryl heterocyclyl carbonyl group,
      wherein if R¹⁰ is -C (=O) NR^{10a}R^{10b}, R^{10b} and R¹¹ together may form - CH₂CH₂-,
R¹² is
   1) a hydrogen atom, or
   2) an optionally substituted C₁₋₄ alkyl group,
R¹³ is
   1) a hydrogen atom,
   2) a hydroxyl group
   3) an optionally substituted C₁₋₄ alkyl group
   4) a sulfanyl group,
   5) a carboxyl group,
   6) an optionally substituted C₁₋₄ alkylthio group,
   7) -NR^{13a}R^{13b},
   8) -NR^{13a}-C (=O) R^{13b},
   9) an optionally substituted 5- or 6-membered non-aryl heterocyclyl carbonylamino group,
   10) -NR^{13a}-C (=O) NR^{13b}R^{13c},
   11) -C(=O)NR^{13a}R^{13b},
   12) -C(=O)NR^{13a}OR^{13b},
   13) -S (=O) ₂-R^{13a},
   14) -S(=O) ₂-NR^{13a}R^{13b},
   15) -C (=O) NR^{13a}-S (=O) ₂-R^{13b}, or
   16) -C (=O) NR^{13a}-S(=O) ₂NR^{13b}R^{13c}, and
R^{10a}, R^{10b}, R^{11a}, R^{13a}, R^{13b}, and R^{13c} are each independently a hydrogen atom or an optionally substituted C₁₋₄ alkyl group.

### [Item 80]

The compound or the pharmaceutically acceptable salt thereof according to item 79, wherein R⁵ is a hydrogen atom or an optionally substituted C₁₋₄ alkyl group.

### [Item 81]

The compound or the pharmaceutically acceptable salt thereof according to any one of items 1 to 3, 11 to 36, and 66 to 76, wherein
R⁵ is selected from the group consisting of 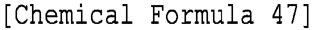 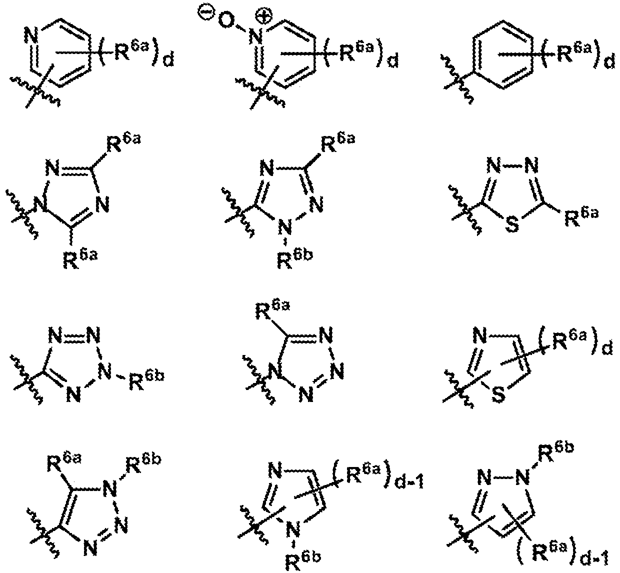 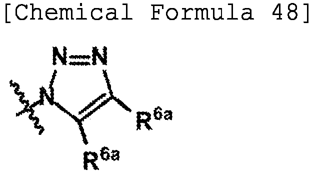 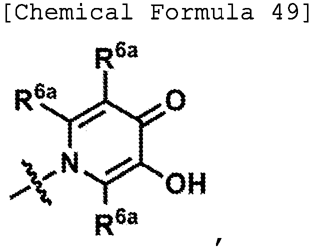
subscript d is the number of substitutable positions on a ring of R⁵,
each R^{6a} is independently selected from the group consisting of
   1) a hydrogen atom,
   2) a hydroxyl group,
   3) a cyano group,
   4) a nitro group,
   5) halogen,
   6) a C₁₋₄ alkyl group,
   7) a C₃₋₁₀ alicyclic group,
   8) a C₁₋₄ alkoxy group,
   9) a C₃₋₁₀ alicyclic oxy group,
   10) a C₆₋₁₀ aryloxy group,
   11) a 5- or 6-membered heteroaryloxy group,
   12) a 4- to 10-membered non-aryl heterocyclyl oxy group,
      (wherein each substituent from 6) to 12) is optionally substituted),
   13) -SO₂-NR^{e2}R^{f2},
   14) -NR^{g2}-CR^{e2} (=NR^{f2}) ,
   15) -NR^{g2}-CR^{e2}(=N-OR^{f2}),
   16) -NR^{h2}-C(=NR^{g2})NR^{e2}R^{f2},
   17) -NR^{h2}-C(=N-OR^{g2})NR^{e2}R^{f2},
   18) -NRⁱ²-C(=NR^{h2})NR^{g2}-NR^{e2}R^{f2},
   19) -NRⁱ²-C(=N-OR^{h2})NR^{g2}NR^{e2}R^{f2},
   20) -C (=NR^{e2})R^{f2},
   21) -C(=N-OR^{e2})R^{f2},
   22) -C(=NR^{h2})-NR^{e2}R^{f2},
   23) -C(=NR^{h2})NR^{g2}NR^{e2}R^{f2},
   24) -C(=N-OR^{h2})NR^{g2}NR^{e2}R^{f2},
   25) -NR^{e2}R^{f2},
   26) -NR^{g2}-NR^{e2}R^{f2},
   27) -NR^{e2}OR^{f2},
   28) -NR^{e2}-C(=O) R^{f2},
   29) -C(=O) NR^{e2}R^{f2},
   30) -C(=O) NR^{e2}OR^{f2},
   31) -C(=O) NR^{g2}-NR^{e2}R^{f2},
   32) -C(=O) R^{e2},
   33) -C (=O) OR^{e2}, and
   34) -C(=N-OR^{h2})NR^{e2}R^{f2}, and
each R^{6b} is independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C (=NR^{e2})R^{f2},
   6) -C(=N-OR^{e2})R^{f2},
   7) -SO₂-NR^{e2}R^{f2},
   8) -C (=NR^{h2})-NR^{e2}R^{f2},
   9) -C (=NR^{h2})NR^{g2}NR^{e2}R^{f2},
   10) -C(=N-OR^{h2})NR^{g2}-NR^{e2}R^{f2},
   11) -C(=O) NR^{e2}R^{f2},
   12) -C(=O)NR^{e2}OR^{f2},
   13) -C (=O) NR^{g2}-NR^{e2}R^{f2},
   14) -C (=O) R^{e2}, and
   15) -C(=N-OR^{h2})NR^{e2}R^{f2}.

### [Item 82]

The compound or the pharmaceutically acceptable salt thereof according to any one of items 1 to 3, 11 to 36, 66 to 76, and 81, wherein
R⁵ is 5- or 6-membered aryl or heteroaryl selected from the group consisting of 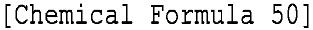 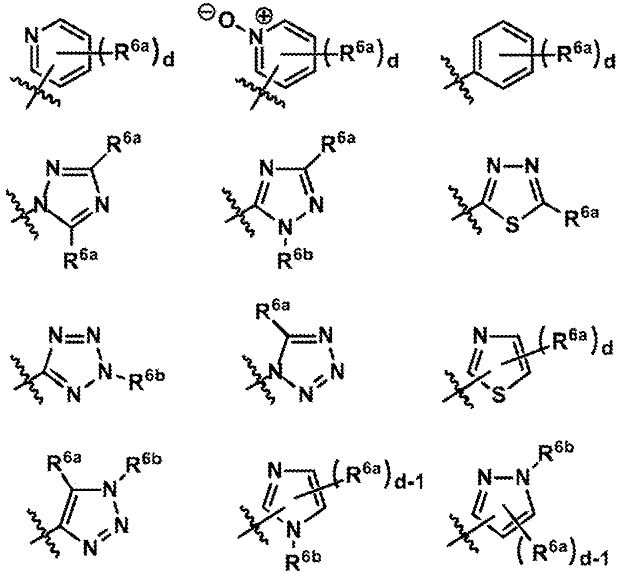 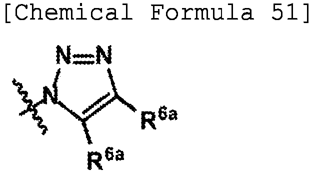 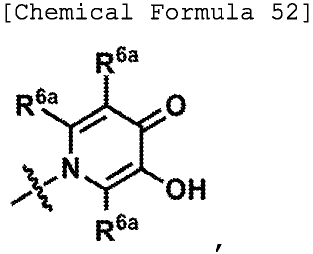
subscript d is the number of substitutable positions on a ring of R⁵,
each R^{6a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e2}R^{f2}, a 5- or 6-membered non-aryl heterocycle, -C(=O)OR^{f2}, or a hydroxyl group),
   5) a C₁₋₄ alkoxy group
   6) -NR^{e2}R^{f2}, and
   7) -C(=O)OR^{e2}, and
each R^{6b} is independently selected from the group consisting of
   1) a hydrogen atom,
   2) a hydroxyl group, and
   3) a C₁₋₄ alkyl group
      (wherein the alkyl group is optionally substituted with NR^{e2}R^{f2}, - C (=O) NR^{e2}R^{f2}, -C(=O)OR^{f2}, or a hydroxyl group).

### [Item 83]

The compound or the pharmaceutically acceptable salt thereof according to any one of items 1 to 3, 11 to 36, 66 to 76, and 81 to 82, wherein R⁵ is

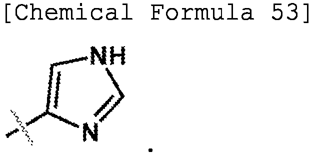

### [Item 84]

The compound or the pharmaceutically acceptable salt thereof according to any one of items 1 to 3, 11 to 36, 66 to 76, and 81 to 82, wherein R^{e2} and R^{f2} are the same or different, each independently a hydrogen atom, an optionally substituted C₁₋₆ alkyl group, or an optionally substituted C₃₋₁₀ alicyclic group.

### [Item 85]

The compound or the pharmaceutically acceptable salt thereof according to any one of items 1 to 3, 11 to 36, 66 to 76, 81 to 82, and 84, wherein R^{e2} and R^{f2} are the same or different, each independently a hydrogen atom or an optionally substituted C₁₋₆ alkyl group.

### [Item 86]

The compound or the pharmaceutically acceptable salt thereof according to any one of items 1 to 3, 11 to 36, 66 to 76, 81 to 82, and 84 to 85, wherein R^{e2} and R^{f2} are hydrogen atoms.

### [Item 87]

The compound or the pharmaceutically acceptable salt thereof according to any one of items 81 to 82 and 84 to 85, wherein R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

### [Item 88]

The compound or the pharmaceutically acceptable salt thereof according to any one of items 1 to 3, 11 to 36, 66 to 74, and 77, wherein
R⁵ is a 4- to 6-membered non-aryl heterocycle selected from the group consisting of 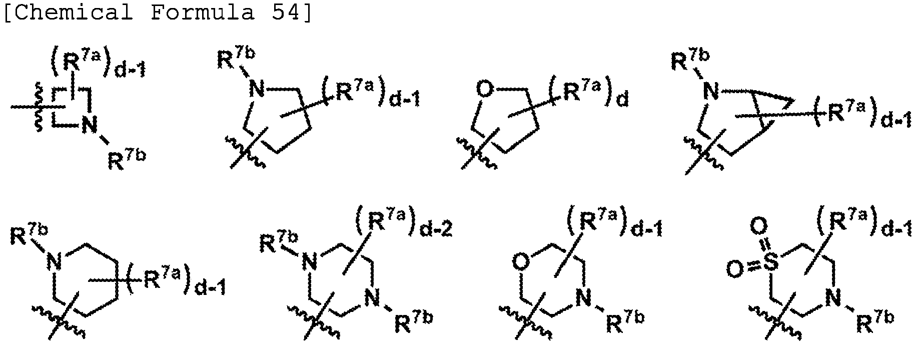 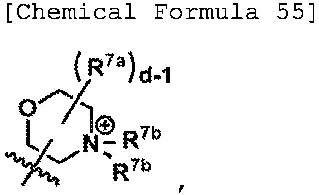
subscript d is the number of substitutable positions on a ring of R⁵,
each R^{7a} is independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e3}R^{f3},
   13) -NR^{g2}-CR^{e3}(=NR^{f3}),
   14) -NR^{g2}-CR^{e3}(=N-OR^{f3}),
   15) -NR^{h2}-C(=NR^{g2})NR^{e3}R^{f3},
   16) -NR^{h2}-C(=N-OR^{g2})NR^{e3}R^{f3},
   17) -NRⁱ²-C(=NR^{h2})NR^{g2}-NR^{e3}R^{f3},
   18) -NRⁱ²-C(=N-OR^{h2})NR^{g2}-NR^{e3}R^{f3},
   19) -C(=NR^{e3})R^{f3},
   20) -C(=N-OR^{e3})R^{f3},
   21) -C(=NR^{h2})-NR^{e3}R^{f3},
   22) -C(=NR^{h2})NR^{g2}-NR^{e3}R^{f3},
   23) -C(=N-OR^{h2})NR^{g2}-NR^{e3}R^{f3},
   24) -NR^{e3}R^{f3},
   25) -NR^{g2}-NR^{e3}R^{f3},
   26) -NR^{e3}OR^{f3},
   27) -NR^{e3}-C(=O) R^{f3},
   28) -C(=O) NR^{e3}R^{f3},
   29) -C(=O)NR^{e3}OR^{f3},
   30) -C(=O) NR^{g2}-NR^{e3}R^{f3},
   31) -C(=O)R^{e3},
   32) -C(=O)OR^{e3}, and
   33) -C (=N-OR^{h2})NR^{e3}R^{f3},
each R^{7b} is independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C (=NR^{e3})R^{f3},
   6) -C(=N-OR^{e3})R^{f3},
   7) -SO₂-NR^{e3}R^{f3},
   8) -C(=NR^{h2})-NR^{e3}R^{f3},
   9) -C(=NR^{h2})NR^{g2}-NR^{e3}R^{f3},
   10) -C (=N-OR^{h2})NR^{g2}-NR^{e3}R^{f3},
   11) -C(=O) NR^{e3}R^{f3},
   12) -C(=O)NR^{e3}OR^{f3},
   13) -C(=O) NR^{g2}-NR^{e3}R^{f3},
   14) -C (=O) R^{e3}, and
   15) -C(=N-OR^{h2})NR^{e3}R^{f3}, and
R^{e3}, R^{f3}, R^{g2}, R^{h2}, and Rⁱ² are defined the same as R^{e2}, R^{f2}, R^{g2}, R^{h2}, and Rⁱ² according to item 3.

### [Item 89]

The compound or the pharmaceutically acceptable salt thereof according to any one of items 1 to 3, 11 to 36, 66 to 74, 77, and 88, wherein
R⁵ is a 4- to 6-membered non-aryl heterocycle selected from the group consisting of 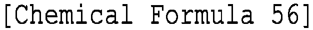 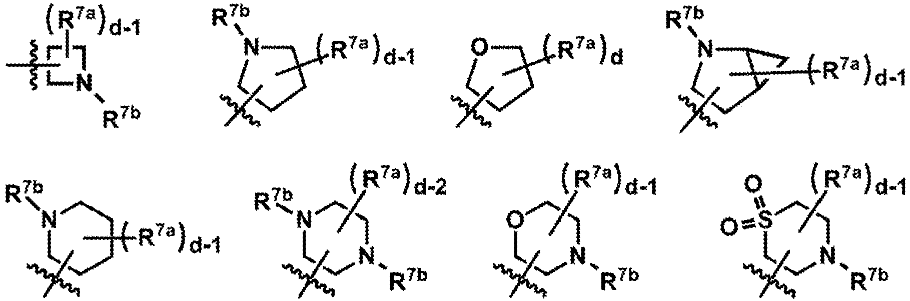 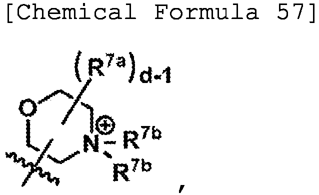
subscript d is the number of substitutable positions on a ring of R⁵,
each R^{7a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e3}R^{f3}, a 5- or 6-membered non-aryl heterocycle, -C(=O)OR^{f3}, or a hydroxyl group),
   5) a C₁₋₄ alkoxy group
   6) -NR^{e3}R^{f3},
   7) -C(=O) OR^{e3},
   8) C₆₋₁₀ aryl, and
   9) -C(=O) NR^{e3}R^{f3},
each R^{7b} is independently selected from the group consisting of
   1) a hydrogen atom,
   2) a hydroxyl group, and
   3) a C₁₋₄ alkyl group
      (wherein the alkyl group is optionally substituted with NR^{e3}R^{f3}, - C(=O)OR^{f3}, or a hydroxyl group), and
R^{e3} and R^{f3} are defined the same as R^{e2} and R^{f2} according to any of items 84 to 86.

### [Item 90]

The compound or the pharmaceutically acceptable salt thereof according to any one of items 1 to 3, 11 to 36, 66 to 74, and 77, wherein
R⁵ is C₄₋₆ cycloalkyl selected from the group consisting of 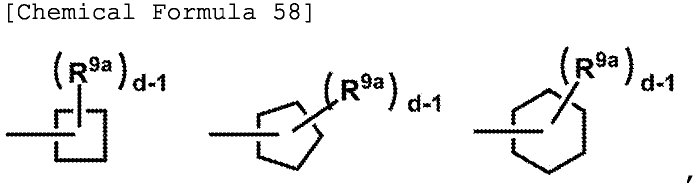
subscript d is the number of substitutable positions on a ring of R⁵,
each R^{9a} is independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e3}R^{f3},
   13) -NR^{g2}-CR^{e3}(=NR^{f3}) ,
   14) -NR^{g2}-CR^{e3}(=N-OR^{f3}) ,
   15) -NR^{h2}-C(=NR^{g2})NR^{e3}R^{f3},
   16) -NR^{h2}-C(=N-OR^{g2})NR^{e3}R^{f3},
   17) -NRⁱ²-C(=NR^{h2})NR^{g2}-NR^{e3}R^{f3},
   18) -NRⁱ²-C(=N-OR^{h2})NR^{g2}-NR^{e3}R^{f3},
   19) -C (=NR^{e3})R^{f3},
   20) -C(=N-OR^{e3})R^{f3},
   21) -C (=NR^{h2})-NR^{e3}R^{f3},
   22) -C(=NR^{h2})NR^{g2}-NR^{e3}R^{f3},
   23) -C (=N-OR^{h2})NR^{g2}-NR^{e3}R^{f3},
   24) -NR^{e3}R^{f3},
   25) -NR^{g2}-NR^{e3}R^{f3},
   26) -NR^{e3}OR^{f3},
   27) -NR^{e3}-C(=O) R^{f3},
   28) -C(=O) NR^{e3}R^{f3},
   29) -C(=O)NR^{e3}OR^{f3},
   30) -C (=O) NR^{g2}-NR^{e3}R^{f3},
   31) -C(=O)R^{e3},
   32) -C (=O) OR^{e3}, and
   33) -C(=N-OR^{h2})NR^{e3}R^{f3}, and
R^{e3}, R^{f3}, R^{g2}, R^{h2}, and Rⁱ² are defined the same as R^{e2} and R^{f2} according to item 3.

### [Item 91]

The compound or the pharmaceutically acceptable salt thereof according to any one of items 1 to 3, 11 to 36, 66 to 74, 77, and 90, wherein
R⁵ is C₄₋₆ cycloalkyl selected from the group consisting of 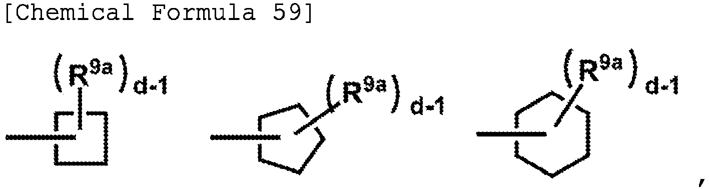
subscript d is the number of substitutable positions on a ring of R⁵,
each R^{9a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e3}R^{f3}, a 5- or 6-membered non-aryl heterocycle, -C(=O)OR^{f3}, or a hydroxyl group),
   5) a C₁₋₄ alkoxy group
   6) -NR^{e3}R^{f3},
   7) -C(=O) OR^{e3},
   8) C₆₋₁₀ aryl, and
   9) -C (=O) NR^{e3}R^{f3}, and
R^{e3} and R^{f3} are defined the same as R^{e2} and R^{f2} according to any of items 75 to 77.

### [Item 92]

The compound or the pharmaceutically acceptable salt thereof according to any one of items 1 to 3, 11 to 36, and 79 to 80, wherein
L⁴ is -CH(NH₂)-CHR¹³-, wherein carbon that attaches to the NH₂ attaches to L³,
R⁵ is a hydrogen atom, and
R¹³ is
   1) -NH-C (=O) CH₃,
   2) -NH-C (=O)NH₂,
   3) -NH-C(=O)CH(NH₂)-CH₂C(=O)NH₂,
   4) -NH-C (=O) CH₂-NH₂,
   5) -NH-C (=O) CH (NH₂)-CH₂OH, or
   6) a pyrrolidin-2-ylcarbonylamino group.

### [Item 93]

The compound or the pharmaceutically acceptable salt thereof according to any one of items 1 to 3, 11 to 36, and 79 to 80, wherein L⁴ is -CH (NH₂)-CR¹²R¹³-, wherein carbon that attaches to the NH₂ attaches to L³,
R⁵ is a hydrogen atom or methyl,
R¹² is a hydrogen atom or methyl, and
R¹³ is a benzylthio group or a sulfanyl group.

### [Item 94]

The compound or the pharmaceutically acceptable salt thereof according to any one of items 1 to 3, 11 to 36, and 79 to 80, wherein
L⁴ is -CH(NH₂)-(CH₂)_{q}-CHR¹³-, wherein q is 0 or 1, and carbon that attaches to the NH₂ attaches to L³,
R⁵ is a hydrogen atom, and
R¹³ is
   1) a carboxyl group,
   2) -C(=O) NH₂,
   3) -C(=O)NH(CH₃),
   4) -C(=O)N(CH₃)₂,
   5) -C(=O)NH-(CH₂)₂-OH,
   6) -C(=O) NH- (CH₂)₂-NH₂,
   7) -C(=O) NH-S (=O) ₂-CH₃,
   8) -C(=O)NHOH,
   9) -S (=O) ₂-NH₂,
   10) -S(=O)₂-CH₃, or
   11) a hydroxyl group.

### [Item 95]

The compound or the pharmaceutically acceptable salt thereof according to any one of items 1 to 3, 11 to 36, and 79 to 80, wherein
L⁴ is -CH(NHR¹¹)-CH₂-, wherein carbon that attaches to the NHR¹¹ attaches to L³,
R⁵ is hydrogen, and
R¹¹ is
   1) -C(=O) CH(NH₂)-CH₂C(=O) NH₂,
   2) -C(=O) CH₂-NH₂,
   3) -C(=O) CH(CH₃)-NH₂,
   4) -C(=O) CH(NH₂)-CH₂OH, or
   5) pyrrolidin-2-ylcarbonyl.

### [Item 96]

The compound or the pharmaceutically acceptable salt thereof according to any one of items 1 to 3, 11 to 36, and 79 to 80, wherein
L⁴ is -CH (NHR¹¹) -CH (COOH) -, wherein carbon that attaches to the NHR¹¹ attaches to L³,
R⁵ is hydrogen, and
R¹¹ is
   1) -C(=O) CH(NH₂)-CH₂C(=O) NH₂,
   2) -C(=O) CH₂-NH₂,
   3) -C(=O) CH(CH₃)-NH₂,
   4) -C(=O) CH(NH₂)-CH₂OH, or
   5) pyrrolidin-2-ylcarbonyl.

### [Item 97]

The compound or the pharmaceutically acceptable salt thereof according to any one of items 1 to 3, 11 to 36, and 79 to 80, wherein
L⁴ is -CHR¹³- or -CH₂-CHR¹³-
R⁵ is hydrogen, and
R¹³ is -C(=O)NH₂ or -C(=O)NHOH.

### [Item 98]

The compound or the pharmaceutically acceptable salt thereof according to any one of items 1 to 3, 11 to 36, and 79 to 80, wherein
L⁴ is -CH₂CR¹⁰ (NH₂)-, wherein the CH₂ group attaches to L³,
R⁵ is hydrogen, and
R¹⁰ is a carboxy group or -C(=O)NH₂.

### [Item 99]

The compound or the pharmaceutically acceptable salt thereof according to any one of items 1 to 3, 11 to 36, and 79 to 80, wherein
L⁴ is - (CH₂)ₚ-CR¹⁰ (NHR¹¹) - (CH₂)_{q}-CHR¹³- or -CHR¹³-(CH₂)_{q}-CR¹⁰(NHR¹¹) - (CH₂)ₚ-, wherein q is 0 or 1,
R⁵ is hydrogen,
   (1) if L⁴ is -CHR^{1L}(CH₂)_{q}-CR¹⁰(NHR^{ll})_(CH₂)ₚ-, carbon of the -CHR¹³- group attaches to L³,
      p is 0,
      R¹⁰ is a hydrogen atom, a carboxyl group, or -C(=O)NHR^{10b},
      R¹¹ is a hydrogen atom,
      R^{10b} is a hydrogen atom,
         wherein if R¹⁰ is -C (=O) NHR^{10b}, R^{10b} and R¹¹ together may form - CH₂CH₂-, and
      R¹³ is a hydrogen atom,
   (2) if L⁴ is -(CH₂)ₚ-CR¹⁰(NHR¹¹)-(CH₂)_{q}-CHR¹³-, carbon of the -(CH₂)ₚ- group attaches to L³,
      p is 1,
      R¹⁰ and R¹¹ are both hydrogen atoms,
      R¹³ is a carboxyl group or -C (=O) NR^{13a}R^{13b}, and
      R^{13a} and R^{13b} are each independently a hydrogen atom or an optionally substituted C₁₋₄ alkyl group.

### [Item 100]

The compound or the pharmaceutically acceptable salt thereof according to any one of items 1 to 3, 11 to 36, and 79 to 80, wherein
L⁴ is -CR¹²(NH₂)-,
R¹² is a hydrogen atom or a methyl group, and
R⁵ is a C₁₋₄ alkyl group optionally substituted with a hydroxyl group.

### [Item 101]

The compound or the pharmaceutically acceptable salt thereof according to item 1 or 2, selected from the group consisting of the compounds represented by the following names or structural formulas:
5-({1-[amino(1H-imidazol-4-yl)acetyl]azetidin-3-yl}oxy)-2-hydroxy-1,1a,2,7b-tetrahydrocyclopropa[c] [1,2]benzoxaborinine-4-carboxylic acid 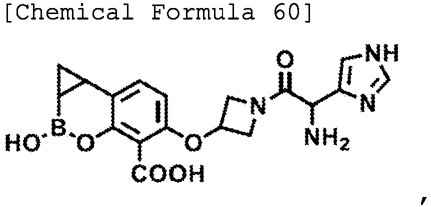
9-[1-[2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 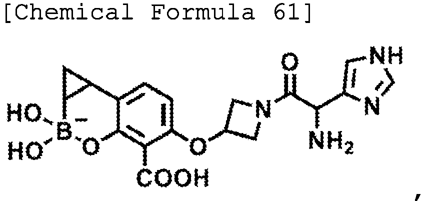
(1aS,7bR)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 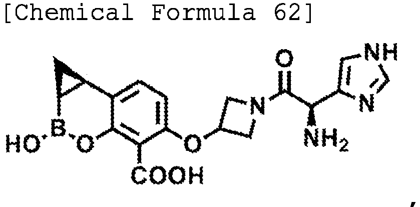
(2R,4S)-9-[1-[(2R)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 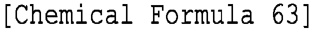 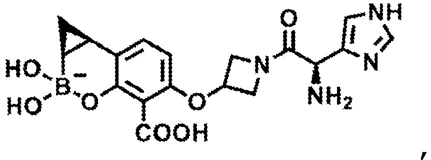
(1aS,7bR)-5-({1-[(2S)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 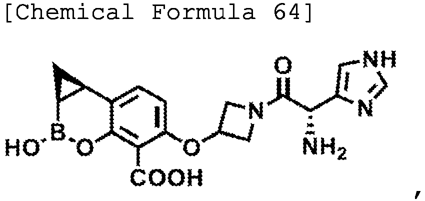
(2R,4S)-9-[1-[(2S)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 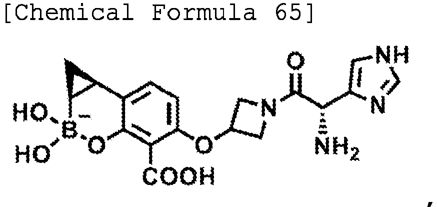
(1aR,7bS)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 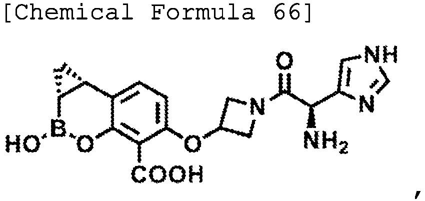
(2S,4R)-9-[1-[(2R)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 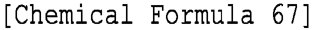 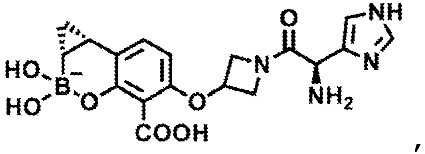
(1aR,7bS)-5-({1-[(2S)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 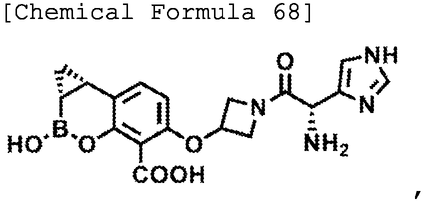
(2S,4R)-9-[1-[(2S)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 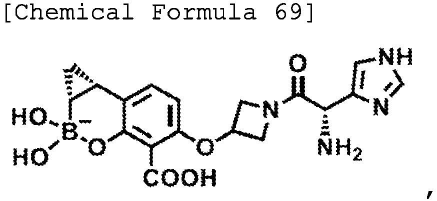
5-({1-(2-amino-2-carboxypropyl)azetidin-3-yl}oxy)-2-hydroxy-1,1a,2,7b-tetrahydrocyclopropa[c] [1,2]benzoxaborinine-4-carboxylic acid 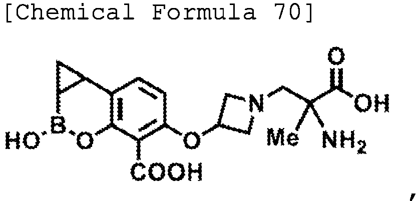
9-[1-(propyl 2-amino-2-carboxylate)azetidin-3-yl]oxy-5,5-dihydroxy-6-oxa-5-boranuidatricyclo[5.4.0.0^{2,4}]undeca-1(7),8,10-triene-8-carboxylate 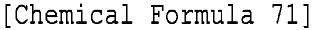 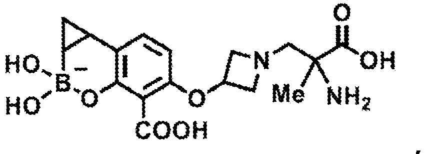
(1aS,7bR)-5-({1-[(2R)-2-amino-2-carboxypropyl]azetidin-3-yl}oxy)-2-hydroxy-1,la,2,7b-tetrahydrocyclopropa[c] [1,2]benzoxaborinine-4-carboxylic acid 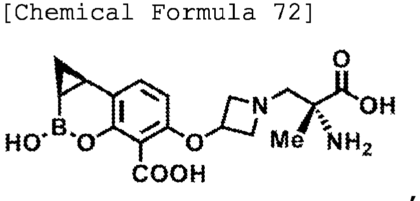
(2R,4S)-9-[1-[propyl (2R)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 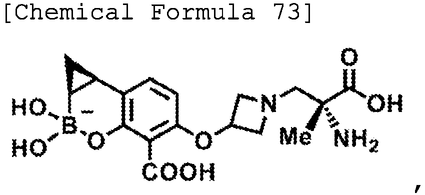
(1aR,7bS)-5-({1-[(2R)-2-amino-2-carboxypropyl]azetidin-3-yl}oxy)-2-hydroxy-1,la,2,7b-tetrahydrocyclopropa[c] [1,2]benzoxaborinine-4-carboxylic acid 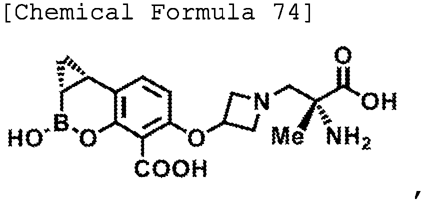
(2S,4R)-9-[1-[propyl (2R)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 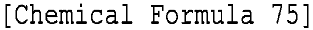 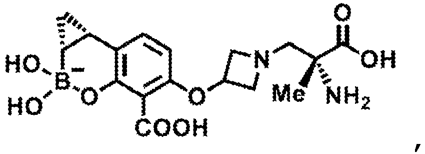
5-({1-[2-amino-2-(1H-imidazol-4-yl)propanoyl]azetidin-3-yl}oxy)-2-hydroxy-1,1a,2,7b-tetrahydrocyclopropa[c] [1,2]benzoxaborinine-4-carboxylic acid 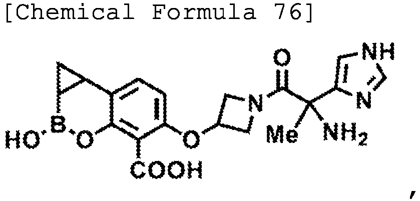
9-[1-[2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 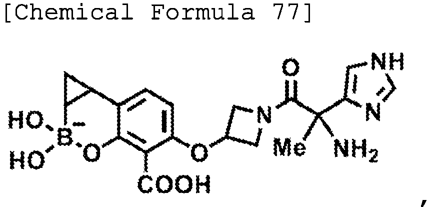
(1aS,7bR)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 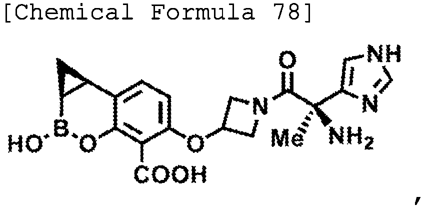
(2R,4S)-9-[1-[(2R)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 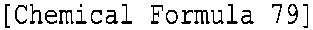 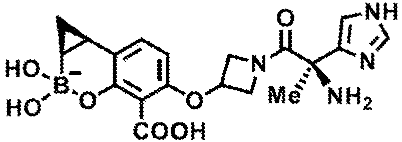 ,
(1aS,7bR)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 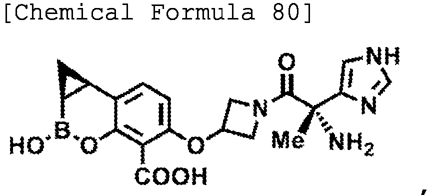
(2R,4S)-9-[1-[(2S)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 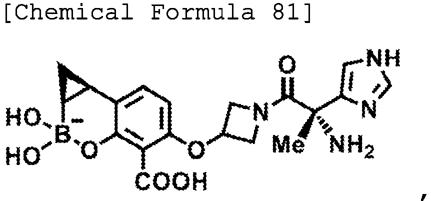
(1aR,7bS)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 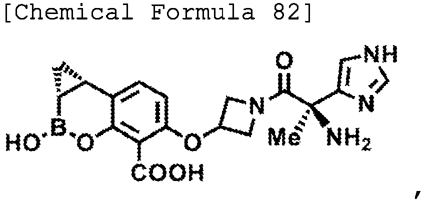
(2S,4R)-9-[1-[(2R)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 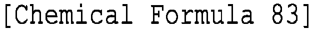 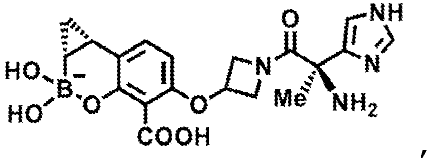
(1aR,7bS)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 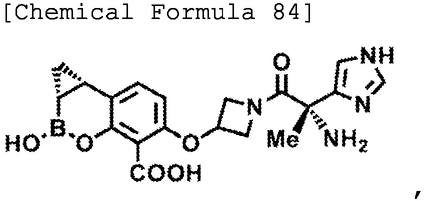 and
(2S,4R)-9-[1-[(2S)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid

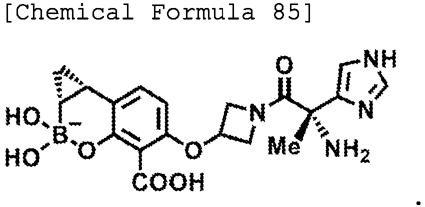

### [Item 102]

The compound or the pharmaceutically acceptable salt thereof according to item 1 or 2, selected from the group consisting of the compounds represented by the following names or structural formulas:
2-hydroxy-5-({1-[(1H-imidazol-4-yl)acetyl]azetidin-3-yl}oxy)-1,1a,2,7b-tetrahydrocyclopropa[c] [1,2]benzoxaborinine-4-carboxylic acid 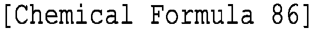 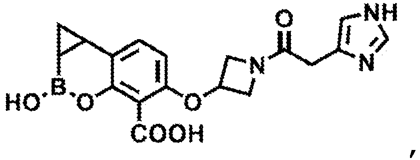
5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid 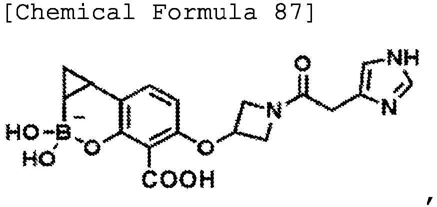
(1aS,7bR)-2-hydroxy-5-({1-[(1H-imidazol-4-yl)acetyl]azetidin-3-yl}oxy)-1,1a,2,7b-tetrahydrocyclopropa[c] [1,2]benzoxaborinine-4-carboxylic acid 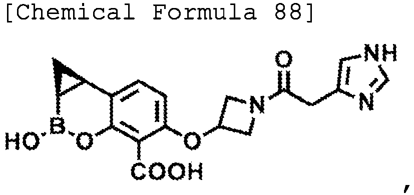
(2R,4S)-5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid 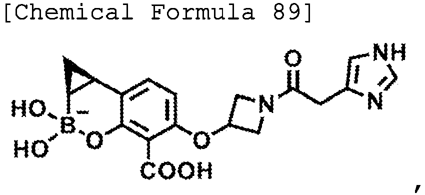
(1aR,7bS)-2-hydroxy-5-({1-[(1H-imidazol-4-yl)acetyl]azetidin-3-yl}oxy)-1,1a,2,7b-tetrahydrocyclopropa[c] [1,2]benzoxaborinine-4-carboxylic acid 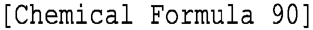 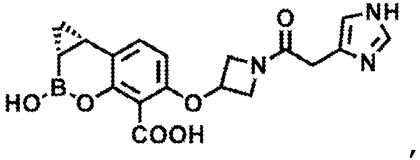
(2S,4R)-5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid 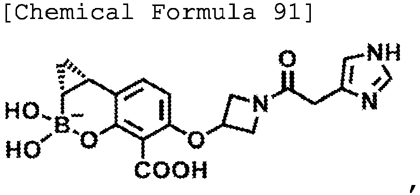
2-hydroxy-5-[(1-serylazetidin-3-yl)oxy]-1,1a,2,7b-tetrahydrocyclopropa[c][1,2]benzoxaborinine-4-carboxylic acid 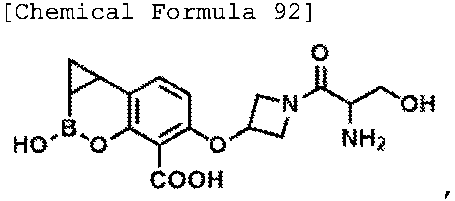
5,5-dihydroxy-9-(1-serylazetidin-3-yl)oxy-6-oxa-5-boranuidatricyclo[5.4.0.0^{2,4}]undeca-1(11),7,9-triene-8-carboxylic acid 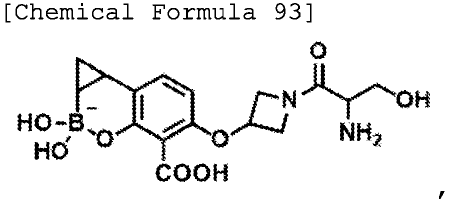
(laS,7bR)-2-hydroxy-5-[(1-D-serylazetidin-3-yl)oxy]-1,1a,2,7b-tetrahydrocyclopropa[c][1,2]benzoxaborinine-4-carboxylic acid 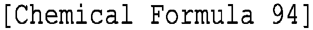 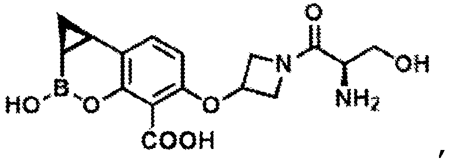
(2R,4S)-5,5-dihydroxy-9-(1-D-serylazetidin-3-yl)oxy-6-oxa-5-boranuidatricyclo[5.4.0.0^{2,4}]undeca-1(11),7,9-triene-8-carboxylic acid 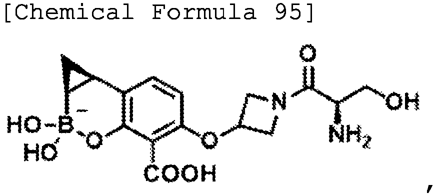
(1aS,7bR)-2-hydroxy-5-[(1-L-serylazetidin-3-yl)oxy]-1,1a,2,7b-tetrahydrocyclopropa[c][1,2]benzoxaborinine-4-carboxylic acid 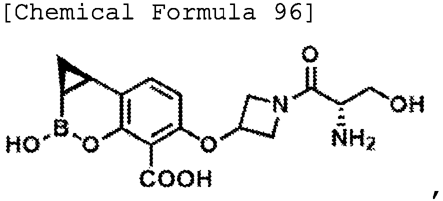
(2R,4S)-5,5-dihydroxy-9-(1-L-serylazetidin-3-yl)oxy-6-oxa-5-boranuidatricyclo[5.4.0.0^{2,4}]undeca-1(11),7,9-triene-8-carboxylic acid 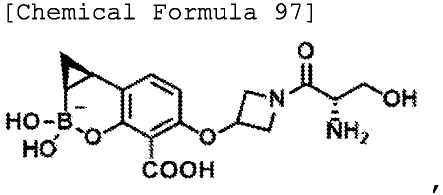
(1aR,7bS)-2-hydroxy-5-[(1-D-serylazetidin-3-yl)oxy]-1,1a,2,7b-tetrahydrocyclopropa[c][1,2]benzoxaborinine-4-carboxylic acid 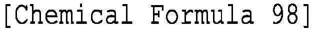 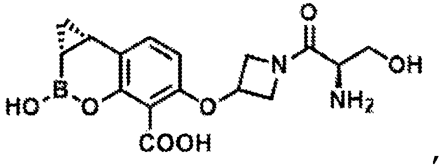
(2S,4R)-5,5-dihydroxy-9-(1-D-serylazetidin-3-yl)oxy-6-oxa-5-boranuidatricyclo[5.4.0.0^{2,4}]undeca-1(11),7,9-triene-8-carboxylic acid 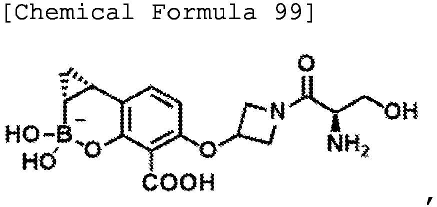
(1aR,7bS)-2-hydroxy-5-[(1-L-serylazetidin-3-yl)oxy]-1,1a,2,7b-tetrahydrocyclopropa[c][1,2]benzoxaborinine-4-carboxylic acid 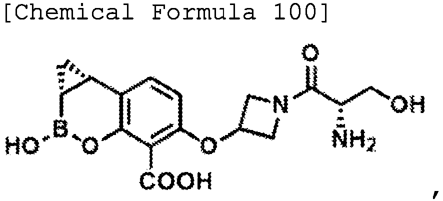
(2S,4R)-5,5-dihydroxy-9-(1-L-serylazetidin-3-yl)oxy-6-oxa-5-boranuidatricyclo[5.4.0.0^{2,4}]undeca-1(11),7,9-triene-8-carboxylic acid 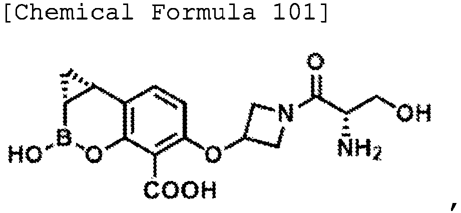
2-hydroxy-5-({1-[4-hydroxy-prolyl]azetidin-3-yl}oxy)-1,1a,2,7b-tetrahydrocyclopropa[c][1,2]benzoxaborinine-4-carboxylic acid 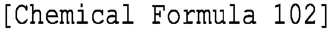 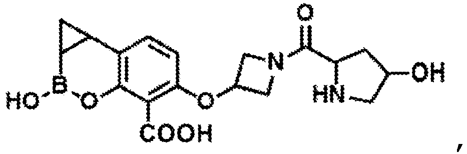
5,5-dihydroxy-9-{1-[4-hydroxy-prolyl]azetidin-3-yl}oxy-6-oxa-5-boranuidatricyclo[5.4.0.0^{2,4}]undeca-1(11),7,9-triene-8-carboxylic acid 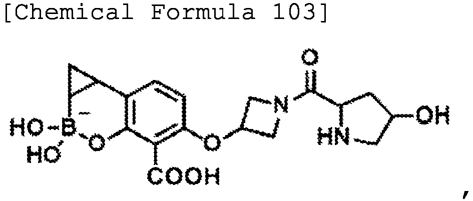
(1aS,7bR)-2-hydroxy-5-({1-[(4R)-4-hydroxy-L-prolyl]azetidin-3-yl}oxy)-1,1a,2,7b-tetrahydrocyclopropa[c] [1,2]benzoxaborinine-4-carboxylic acid 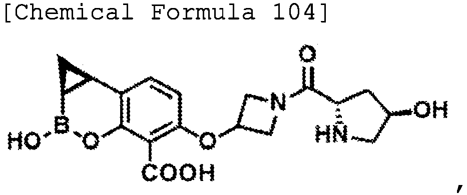
(2R,4S)-5,5-dihydroxy-9-{1-[(4R)-4-hydroxy-L-prolyl]azetidin-3-yl}oxy-6-oxa-5-boranuidatricyclo[5.4.0.0^{2,4}]undeca-1(11),7,9-triene-8-carboxylic acid 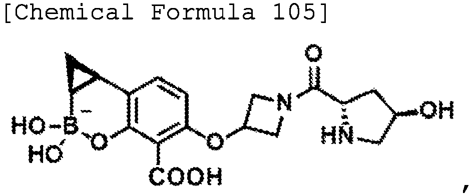
(1aS,7bR)-2-hydroxy-5-({1-[(4S)-4-hydroxy-L-prolyl]azetidin-3-yl}oxy)-1,1a,2,7b-tetrahydrocyclopropa[c] [1,2]benzoxaborinine-4-carboxylic acid 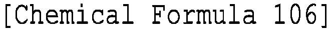 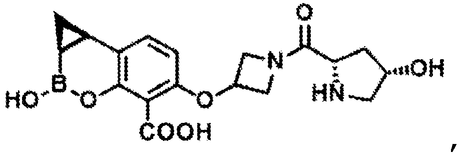
(2R,4S)-5,5-dihydroxy-9-{1-[(4S)-4-hydroxy-L-prolyl]azetidin-3-yl}oxy-6-oxa-5-boranuidatricyclo[5.4.0.0^{2,4}]undeca-1(11),7,9-triene-8-carboxylic acid 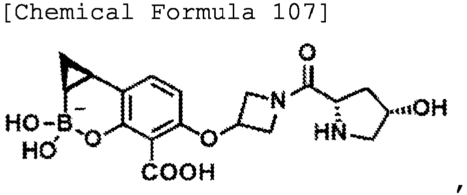
(1aS,7bR)-2-hydroxy-5-({1-[(4R)-4-hydroxy-D-prolyl]azetidin-3-yl}oxy)-1,1a,2,7b-tetrahydrocyclopropa[c] [1,2]benzoxaborinine-4-carboxylic acid 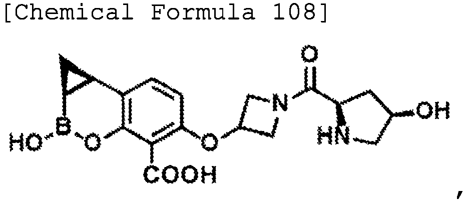
(2R,4S)-5,5-dihydroxy-9-{1-[(4R)-4-hydroxy-D-prolyl]azetidin-3-yl}oxy-6-oxa-5-boranuidatricyclo[5.4.0.0^{2,4}]undeca-1(11),7,9-triene-8-carboxylic acid 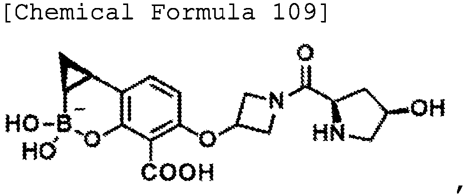
(1aS,7bR)-2-hydroxy-5-({1-[(4S)-4-hydroxy-D-prolyl]azetidin-3-yl}oxy)-1,1a,2,7b-tetrahydrocyclopropa[c] [1,2]benzoxaborinine-4-carboxylic acid 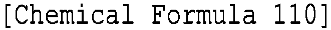 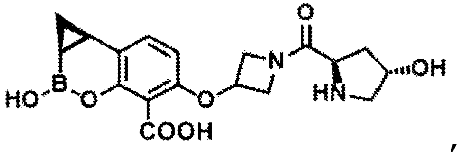
(2R,4S)-5,5-dihydroxy-9-{1-[(4S)-4-hydroxy-D-prolyl]azetidin-3-yl}oxy-6-oxa-5-boranuidatricyclo[5.4.0.0^{2,4}]undeca-1(11),7,9-triene-8-carboxylic acid 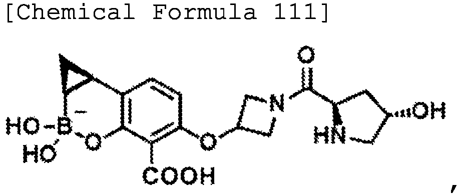
(1aR,7bS)-2-hydroxy-5-({1-[(4R)-4-hydroxy-L-prolyl]azetidin-3-yl}oxy)-1,1a,2,7b-tetrahydrocyclopropa[c] [1,2]benzoxaborinine-4-carboxylic acid 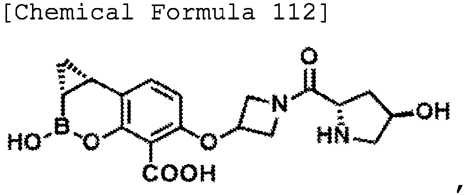
(2S,4R)-5,5-dihydroxy-9-{1-[(4R)-4-hydroxy-L-prolyl]azetidin-3-yl}oxy-6-oxa-5-boranuidatricyclo[5.4.0.0^{2,4}]undeca-1(11),7,9-triene-8-carboxylic acid 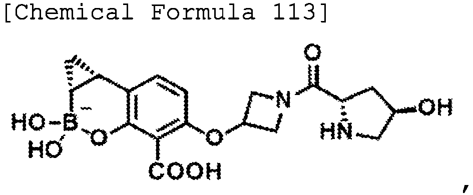
(1aR,7bS)-2-hydroxy-5-({1-[(4S)-4-hydroxy-L-prolyl]azetidin-3-yl}oxy)-1,1a,2,7b-tetrahydrocyclopropa[c] [1,2]benzoxaborinine-4-carboxylic acid 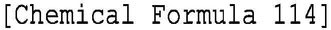 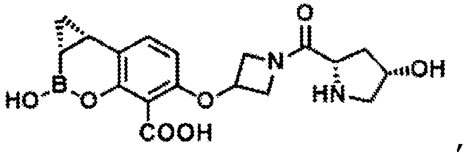
(2S,4R)-5,5-dihydroxy-9-{1-[(4S)-4-hydroxy-L-prolyl]azetidin-3-yl}oxy-6-oxa-5-boranuidatricyclo[5.4.0.0^{2,4}]undeca-1(11),7,9-triene-8-carboxylic acid 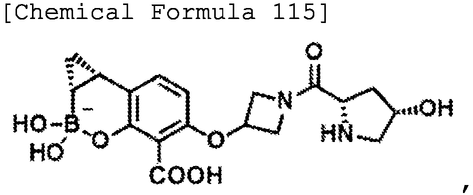
(1aS,7bR)-2-hydroxy-5-({1-[(4R)-4-hydroxy-D-prolyl]azetidin-3-yl}oxy)-1,1a,2,7b-tetrahydrocyclopropa[c] [1,2]benzoxaborinine-4-carboxylic acid 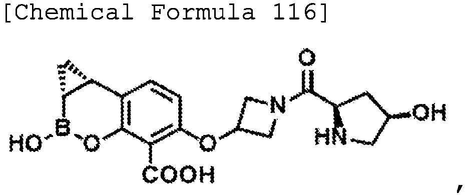
(2S,4R)-5,5-dihydroxy-9-{1-[(4R)-4-hydroxy-D-prolyl]azetidin-3-yl}oxy-6-oxa-5-boranuidatricyclo[5.4.0.0^{2,4}]undeca-1(11),7,9-triene-8-carboxylic acid 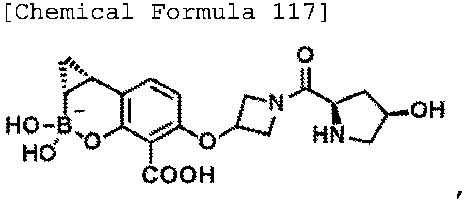
(1aR,7bS)-2-hydroxy-5-({1-[(4S)-4-hydroxy-D-prolyl]azetidin-3-yl}oxy)-1,1a,2,7b-tetrahydrocyclopropa[c] [1,2]benzoxaborinine-4-carboxylic acid 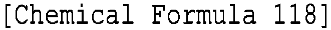 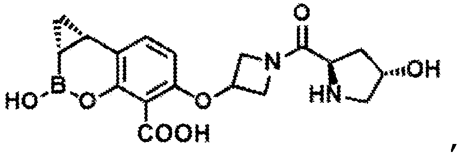
(2S,4R)-5,5-dihydroxy-9-{1-[(4S)-4-hydroxy-D-prolyl]azetidin-3-yl}oxy-6-oxa-5-boranuidatricyclo[5.4.0.0^{2,4}]undeca-1(11),7,9-triene-8-carboxylic acid 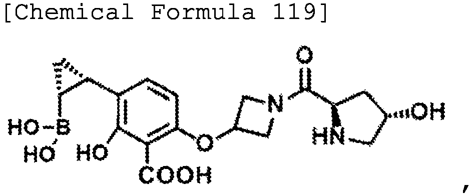
2-hydroxy-5-{[1-(2-methyl-seryl)azetidin-3-yl]oxy}-1,1a,2,7b-tetrahydrocyclopropa[c][1,2]benzoxaborinine-4-carboxylic acid 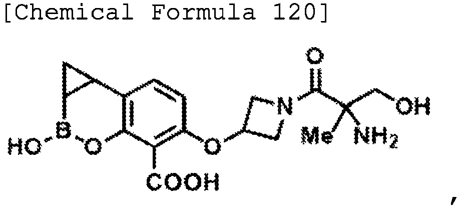
5,5-dihydroxy-9-[1-(2-methyl-seryl)azetidin-3-yl]oxy-6-oxa-5-boranuidatricyclo[5.4.0.0^{2,4}]undeca-1(11),7,9-triene-8-carboxylic acid 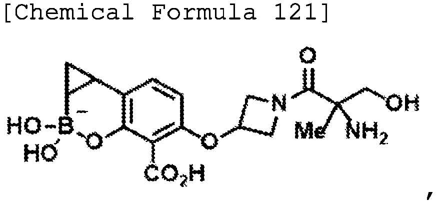
(1aS,7bR)-2-hydroxy-5-{[1-(2-methyl-D-seryl)azetidin-3-yl]oxy}-1,1a,2,7b-tetrahydrocyclopropa[c] [1,2]benzoxaborinine-4-carboxylic acid 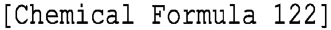 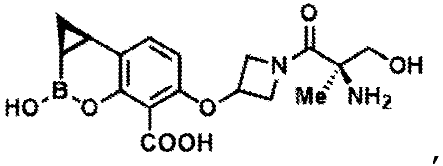
(2R,4S)-5,5-dihydroxy-9-[1-(2-methyl-D-seryl)azetidin-3-yl]oxy-6-oxa-5-boranuidatricyclo[5.4.0.0^{2,4}]undeca-1(11),7,9-triene-8-carboxylic acid 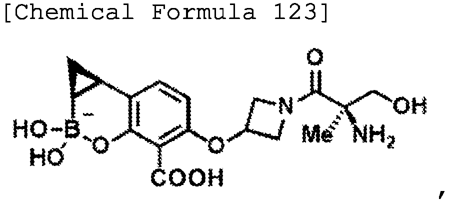
(1aS,7bR)-2-hydroxy-5-{[1-(2-methyl-L-seryl)azetidin-3-yl]oxy}-1,1a,2,7b-tetrahydrocyclopropa[c] [1,2]benzoxaborinine-4-carboxylic acid 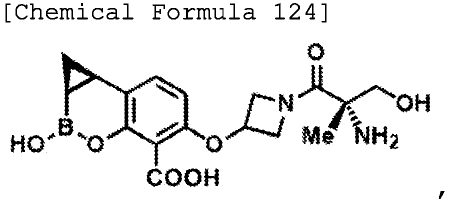
(2R,4S)-5,5-dihydroxy-9-[1-(2-methyl-L-seryl)azetidin-3-yl]oxy-6-oxa-5-boranuidatricyclo[5.4.0.0^{2,4}]undeca-1(11),7,9-triene-8-carboxylic acid 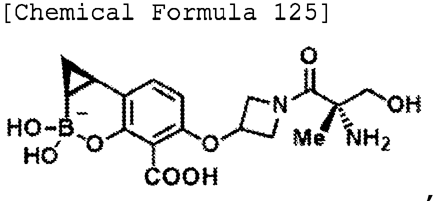
(1aR,7bS)-2-hydroxy-5-{[1-(2-methyl-D-seryl)azetidin-3-yl]oxy}-1,1a,2,7b-tetrahydrocyclopropa[c] [1,2]benzoxaborinine-4-carboxylic acid 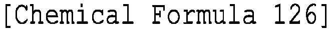 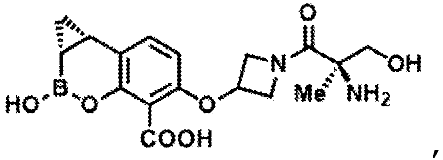
(2S,4R)-5,5-dihydroxy-9-[1-(2-methyl-D-seryl)azetidin-3-yl]oxy-6-oxa-5-boranuidatricyclo[5.4.0.0^{2,4}]undeca-1(11),7,9-triene-8-carboxylic acid 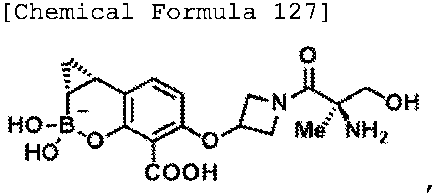
(1aR,7bS)-2-hydroxy-5-{[1-(2-methyl-L-seryl)azetidin-3-yl]oxy}-1,1a,2,7b-tetrahydrocyclopropa[c] [1,2]benzoxaborinine-4-carboxylic acid 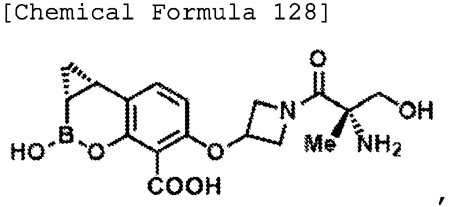
(2S,4R)-5,5-dihydroxy-9-[1-(2-methyl-L-seryl)azetidin-3-yl]oxy-6-oxa-5-boranuidatricyclo[5.4.0.0^{2,4}]undeca-1(11),7,9-triene-8-carboxylic acid 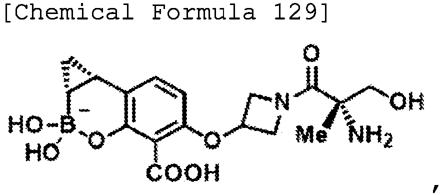
2-hydroxy-5-[(1-1[morpholin-2-yl]acetyl}azetidin-3-yl)oxy]-1,1a,2,7b-tetrahydrocyclopropa[c] [1,2]benzoxaborinine-4-carboxylic acid 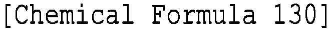 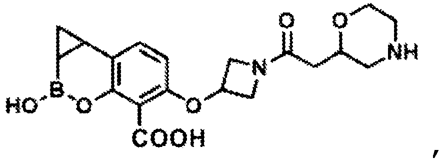
5,5-dihydroxy-9-(1-{[morpholin-2-yl]acetyl}azetidin-3-yl)oxy-5-boranuidatricyclo[5.4.0.0^{2,4}]undeca-1(11),7,9-triene-8-carboxylic acid 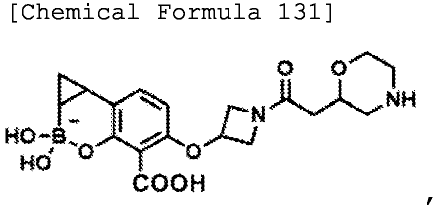
(1aS,7bR)-2-hydroxy-5-[(1-{[(2R)-morpholin-2-yl]acetyl}azetidin-3-yl)oxy]-1,1a,2,7b-tetrahydrocyclopropa[c] [1,2]benzoxaborinine-4-carboxylic acid 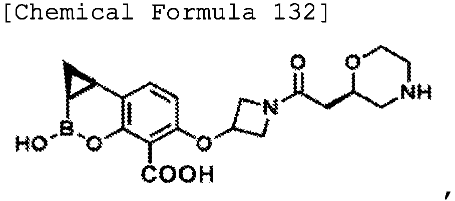
(2R,4S)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid 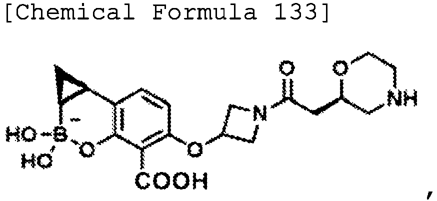
(1aS,7bR)-2-hydroxy-5-[(1-{[(2S)-morpholin-2-yl]acetyl}azetidin-3-yl)oxy]-1,1a,2,7b-tetrahydrocyclopropa[c] [1,2]benzoxaborinine-4-carboxylic acid 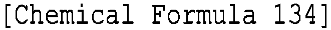 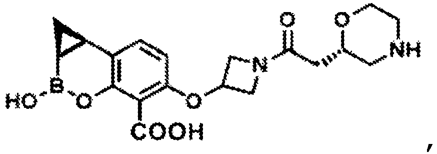
(2S,4R)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid 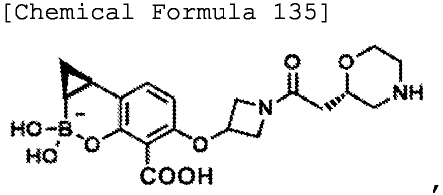
(1aR,7bS)-2-hydroxy-5-[(1-{[(2R)-morpholin-2-yl]acetyl}azetidin-3-yl)oxy]-1,1a,2,7b-tetrahydrocyclopropa[c] [1,2]benzoxaborinine-4-carboxylic acid 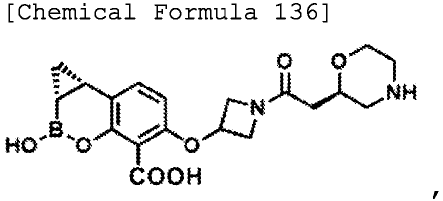
(2S,4R)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid 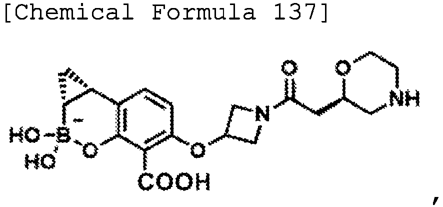
(1aR,7bS)-2-hydroxy-5-[(1-{[(2S)-morpholin-2-yl]acetyl}azetidin-3-yl)oxy]-1,1a,2,7b-tetrahydrocyclopropa[c] [1,2]benzoxaborinine-4-carboxylic acid 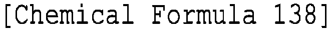 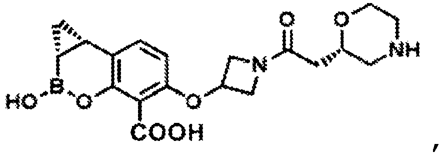
(2S,4R)-5,5-dihydroxy-9-(1-{[(2S)-morpholin-2-yl]acetyl}azetidin-3-yl)oxy-5-boranuidatricyclo[5.4.0.0^{2,4}]undeca-1(11),7,9-triene-8-carboxylic acid 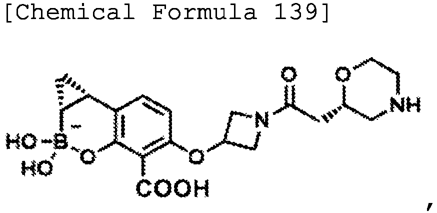
(1aS,7bR)-5-({1-[(2S)-2-amino-2-carboxypropyl]azetidin-3-yl}oxy)-2-hydroxy-1,la,2,7b-tetrahydrocyclopropa[c] [1,2]benzoxaborinine-4-carboxylic acid 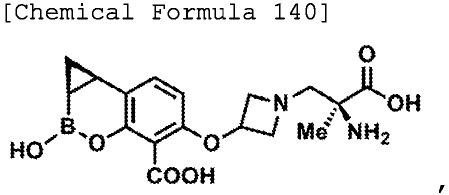
(2R,4S)-9-[1-[propyl (2S)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 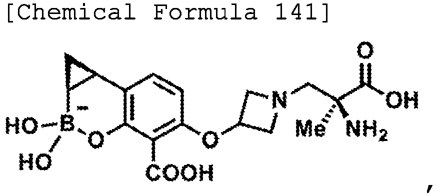
(1aR,7bS)-5-({1-[(2S)-2-amino-2-carboxypropyl]azetidin-3-yl}oxy)-2-hydroxy-1,la,2,7b-tetrahydrocyclopropa[c] [1,2]benzoxaborinine-4-carboxylic acid 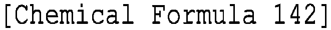 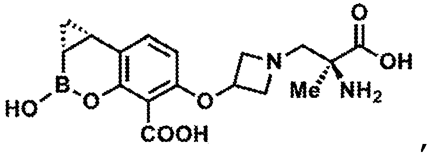
(2S,4R)-9-[1-[propyl (2S)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 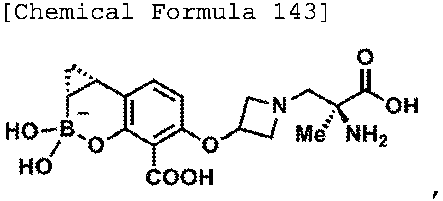
5-({1-[2-amino-2-carboxypropyl]azetidin-3-yl}oxy)-2-hydroxy-1,1a,2,7b-tetrahydrocyclopropa[c] [1,2]benzoxaborinine-4-carboxylic acid 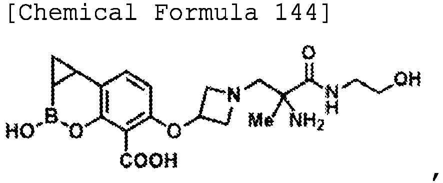
9-(1-{2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 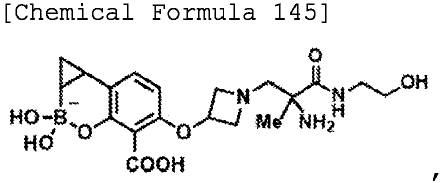
(1aS,7bR)-5-[(1-{ (2R)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 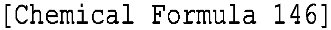 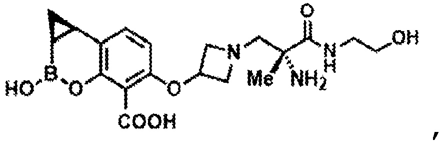
(2R,4S)-9-(1-{(2R)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 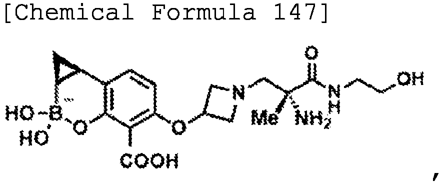
(1aS,7bR)-5-[(1-{ (2S)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 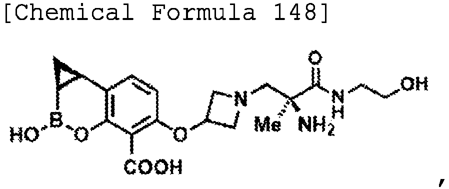
(2R,4S)-9-(1-{(2S)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 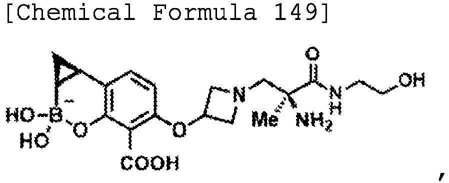
(1aR,7bS)-5-[(1-{ (2R)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 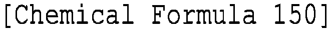 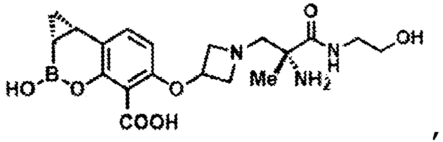
(2S,4R)-9-(1-{(2R)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 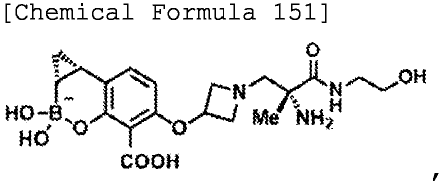
(1aR,7bS)-5-[(1-{ (2S)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 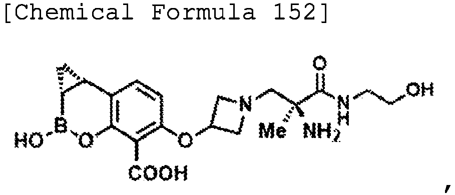
(2S,4R)-9-(1-{ (2S)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 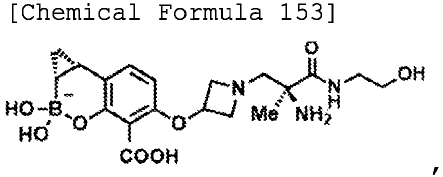
5-[(1-{2-amino-3-[(2-amino-2-oxoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 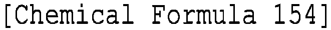 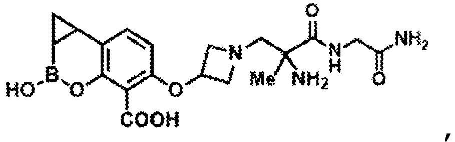
9-(1-{2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 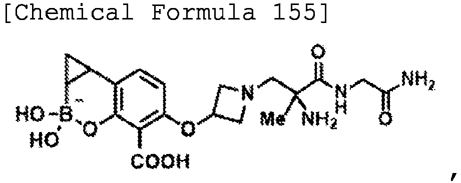
(1aS,7bR)-5-[(1-{ (2R)-2-amino-3-[(2-amino-2-oxoethyl)amino]-2-methyl-3-oxopropyl}azetidin-3-yl)oxy]-2-hydroxy-1,1a,2 ,7b-tetrahydrocyclopropa[c][1,2]benzoxaborinine-4-carboxylic acid 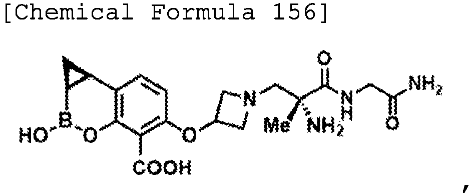
(2R,4S)-9-(1-{(2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 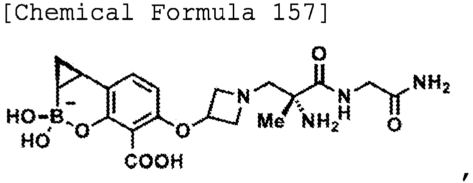
(1aS,7bR)-5-[(1-{ (2S)-2-amino-3-[(2-amino-2-oxoethyl)amino]-2-methyl-3-oxopropyl}azetidin-3-yl)oxy]-2-hydroxy-1,1a,2 ,7b-tetrahydrocyclopropa[c][1,2]benzoxaborinine-4-carboxylic acid 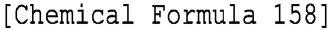 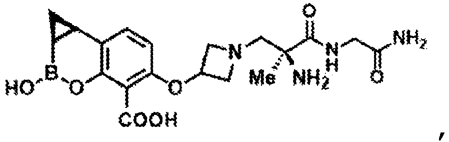
(2R,4S)-9-(1-{(2S)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 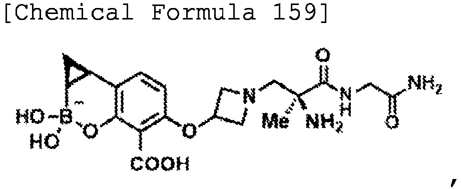
(1aR,7bS)-5-[(1-{ (2R)-2-amino-3-[(2-amino-2-oxoethyl)amino]-2-methyl-3-oxopropyl}azetidin-3-yl)oxy]-2-hydroxy-1,1a,2 ,7b-tetrahydrocyclopropa[c][1,2]benzoxaborinine-4-carboxylic acid 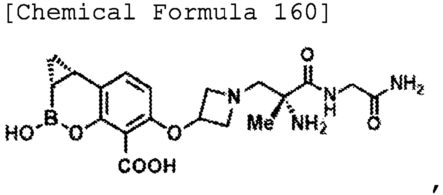
(2S,4R)-9-(1-{ (2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 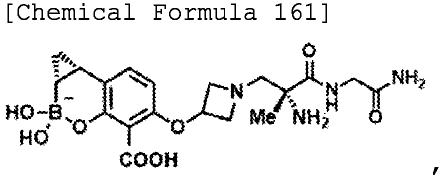
(1aR,7bS)-5-[(1-{ (2S)-2-amino-3-[(2-amino-2-oxoethyl)amino]-2-methyl-3-oxopropyl}azetidin-3-yl)oxy]-2-hydroxy-1,1a,2 ,7b-tetrahydrocyclopropa[c][1,2]benzoxaborinine-4-carboxylic acid 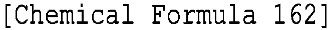 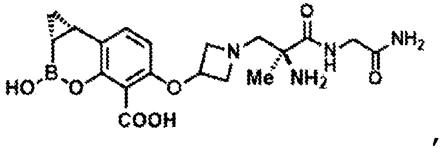
(2S,4R)-9-(1-{ (2S)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 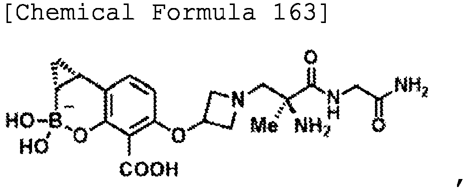
5-({1-[5-carbamoylpyrrolidin-3-yl]azetidin-3-yl}oxy)-2-hydroxy-1,1a,2,7b-tetrahydrocyclopropa[c] [1,2]benzoxaborinine-4-carboxylic acid 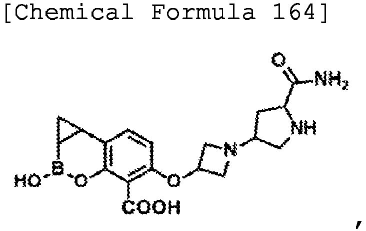
9-{1-[5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 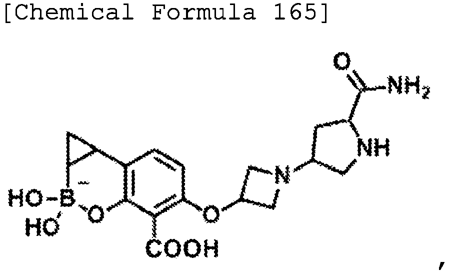
(1aS,7bR)-5-({1-[(3S,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 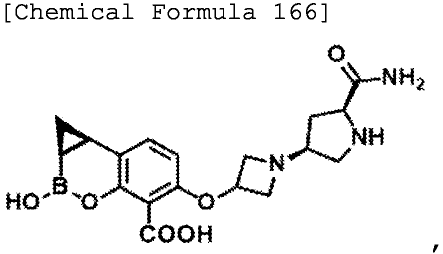
(2R,4S)-9-{1-[(3S,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 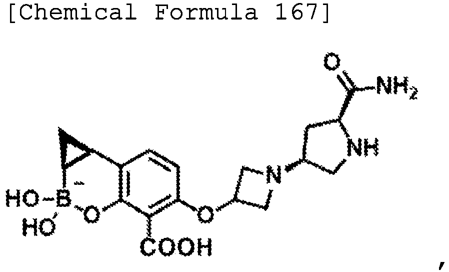
(1aS,7bR)-5-({1-[(3R,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 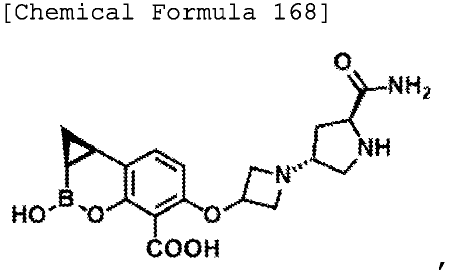
(2R,4S)-9-{1-[(3R,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 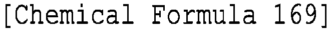 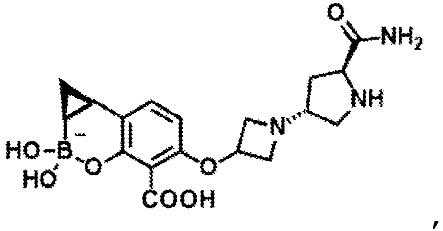
(1aS,7bR)-5-({1-[(3S,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 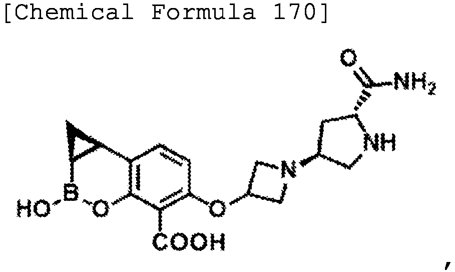
(2R,4S)-9-{1-[(3S,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 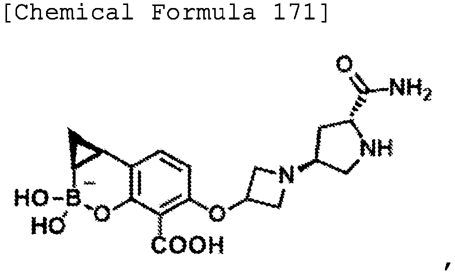
(laS,7bR)-5-({1-[(3R,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 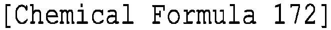 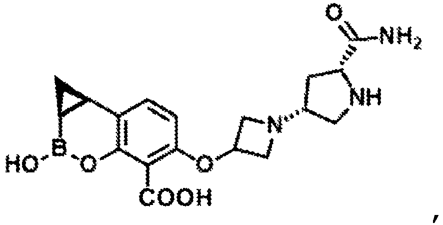
(2R,4S)-9-{1-[(3R,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 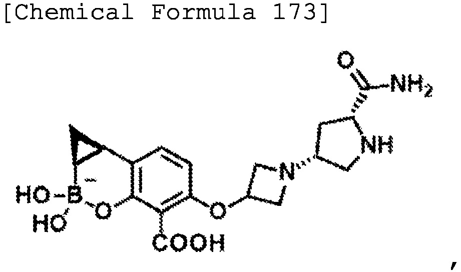
(1aR,7bS)-5-({1-[(3S,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 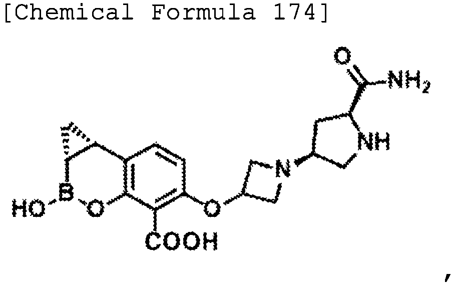
(2S,4R)-9-{1-[(3S,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 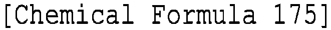 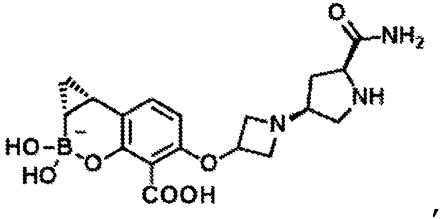
(1aR,7bS)-5-({1-[(3R,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 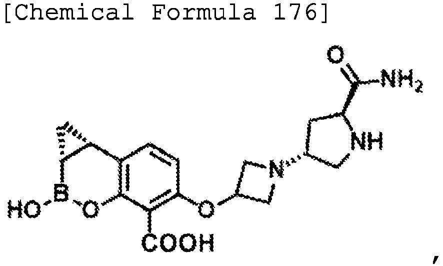
(2S,4R)-9-{1-[(3R,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 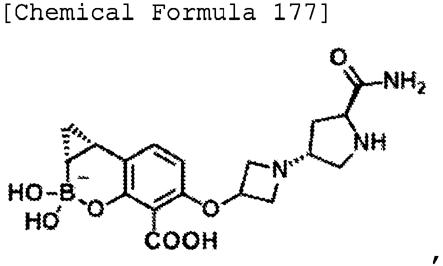
(1aR,7bS)-5-({1-[(3S,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 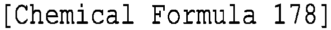 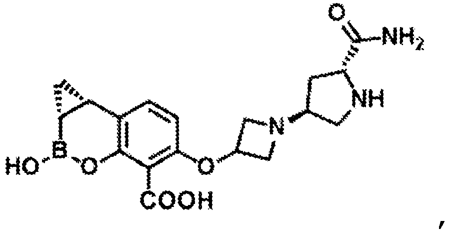
(2S,4R)-9-{1-[(3S,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 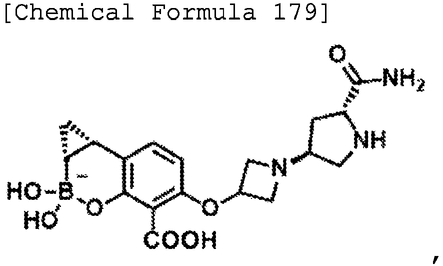
(1aR,7bS)-5-({1-[(3R,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 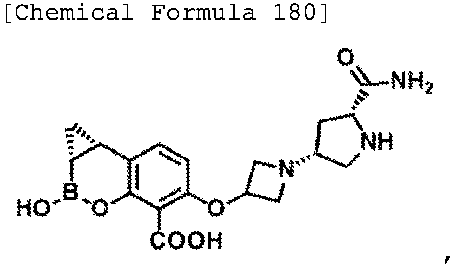
(2S,4R)-9-{1-[(3R,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 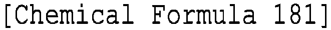 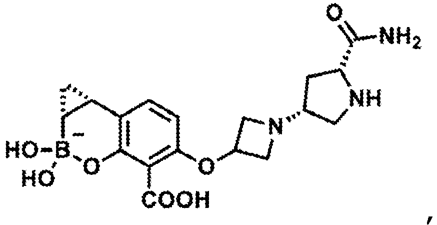
5-{[1-(2-amino-2-oxoethyl)azetidin-3-yl]oxy}-2-hydroxy-1,1a,2,7b-tetrahydrocyclopropa[c][1,2]benzoxaborinine-4-carboxylic acid 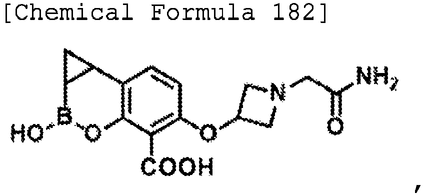
9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylate 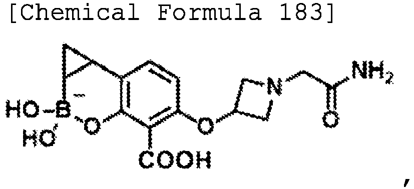
(1aS,7bR)-5-{[1-(2-amino-2-oxoethyl)azetidin-3-yl]oxy}-2-hydroxy-1,1a,2,7b-tetrahydrocyclopropa[c] [1,2]benzoxaborinine-4-carboxylic acid 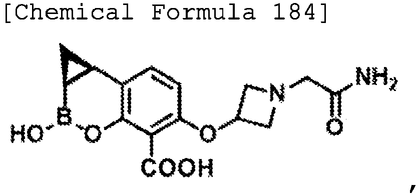
(2R,4S)-9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylate 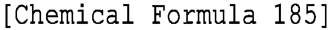 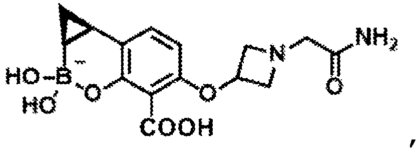
(1aR,7bS)-5-{[1-(2-amino-2-oxoethyl)azetidin-3-yl]oxy}-2-hydroxy-1,1a,2,7b-tetrahydrocyclopropa[c] [1,2]benzoxaborinine-4-carboxylic acid 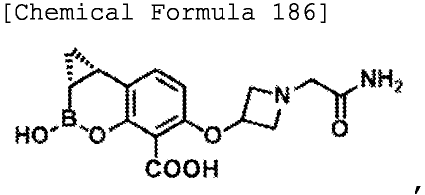
(2S,4R)-9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylate 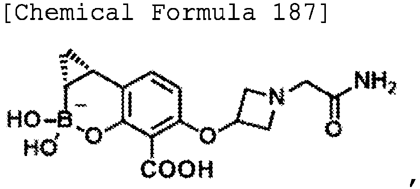
2-hydroxy-5-[(1-{2-[(2-hydroxyethoxy)amino]-2-oxoethyl}azetidin-3-yl)oxy]-1,1a,2,7b-tetrahydrocyclopropa[c] [1,2]benzoxaborinine-4-carboxylic acid 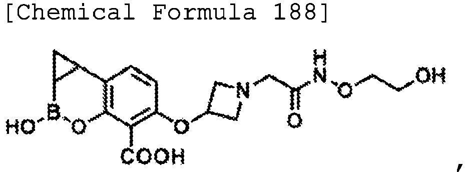
5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid 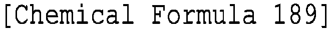 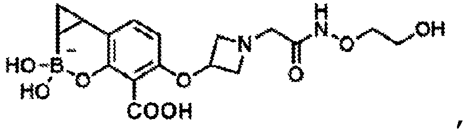
(1aS,7bR)-2-hydroxy-5-[(1-{2-[(2-hydroxyethoxy)amino]-2-oxoethyl}azetidin-3-yl)oxy]-1,1a,2,7b-tetrahydrocyclopropa[c][1,2]benzoxaborinine-4-carboxylic acid 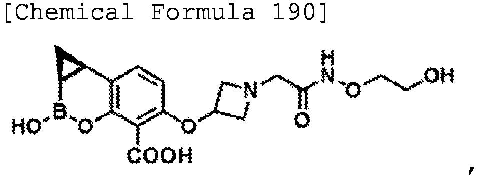
(2R,4S)-5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid 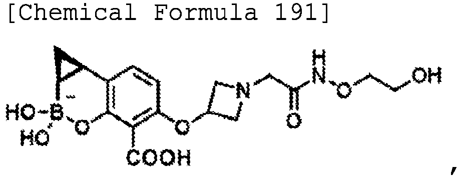
(1aR,7bS)-2-hydroxy-5-[(1-{2-[(2-hydroxyethoxy)amino]-2-oxoethyl}azetidin-3-yl)oxy]-1,1a,2,7b-tetrahydrocyclopropa[c][1,2]benzoxaborinine-4-carboxylic acid 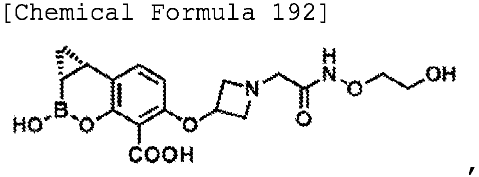
(2S,4R)-5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid 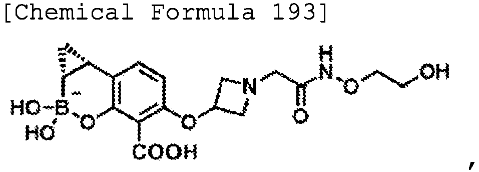
5-({1-[2-amino-3-hydroxy-2-(hydroxymethyl)propyl]azetidin-3-yl}oxy)-2-hydroxy-1,1a,2,7b-tetrahydrocyclopropa[c][1,2]benzoxaborinine-4-carboxylic acid 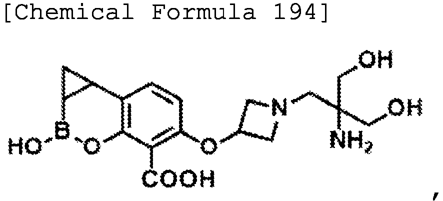
9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylate 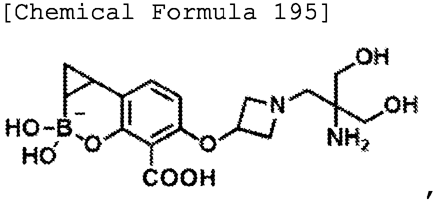
(1aS,7bR)-5-({1-[2-amino-3-hydroxy-2-(hydroxymethyl)propyl]azetidin-3-yl}oxy)-2-hydroxy-1,1a,2,7b-tetrahydrocyclopropa[c][1,2]benzoxaborinine-4-carboxylic acid 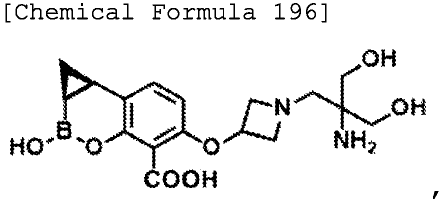
(2R,4S)-9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylate 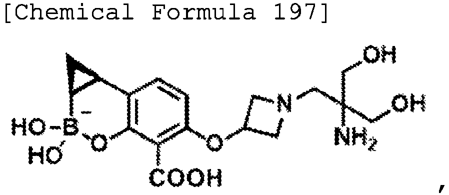
(1aR,7bS)-5-({1-[2-amino-3-hydroxy-2-(hydroxymethyl)propyl]azetidin-3-yl}oxy)-2-hydroxy-1,1a,2,7b-tetrahydrocyclopropa[c][1,2]benzoxaborinine-4-carboxylic acid 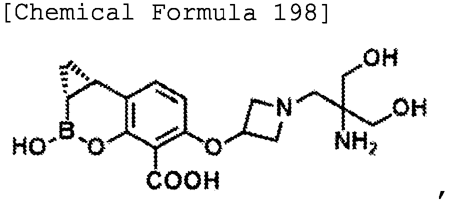
(2S,4R)-9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylate 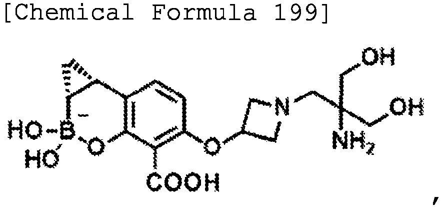
5-({1-[(1-aminocyclopropyl)methyl]azetidin-3-yl}oxy)-2-hydroxy-1,1a,2,7b-tetrahydrocyclopropa[c] [1,2]benzoxaborinine-4-carboxylic acid 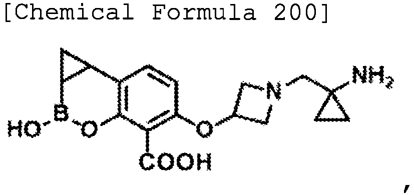
9-{1-[(1-aminocyclopropyl)methyl]azetidin-3-yl}oxy-5,5-dihydroxy-6-oxa-5-boranuidatricyclo[5.4.0.0^{2,4}]undeca-1(7),8,10-triene-8-carboxylate 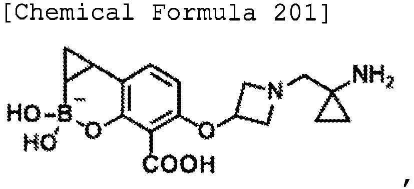
(1aS,7bR)-5-({1-[(1-aminocyclopropyl)methyl]azetidin-3-yl}oxy)-2-hydroxy-1,1a,2,7b-tetrahydrocyclopropa[c] [1,2]benzoxaborinine-4-carboxylic acid 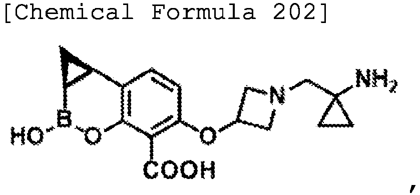
(2R,4S)-9-{1-[(1-aminocyclopropyl)methyl]azetidin-3-yl}oxy-5,5-dihydroxy-6-oxa-5-boranuidatricyclo[5.4.0.0^{2,4}]undeca-1(7),8,10-triene-8-carboxylate 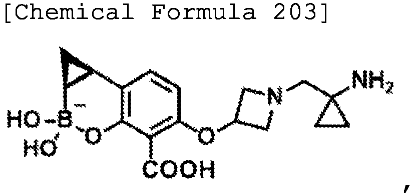
(1aR,7bS)-5-({1-[(1-aminocyclopropyl)methyl]azetidin-3-yl}oxy)-2-hydroxy-1,1a,2,7b-tetrahydrocyclopropa[c] [1,2]benzoxaborinine-4-carboxylic acid 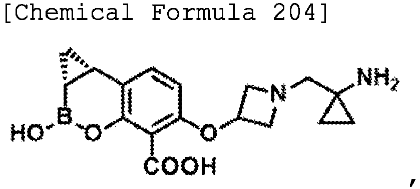
(2S,4R)-9-{1-[(1-aminocyclopropyl)methyl]azetidin-3-yl}oxy-5,5-dihydroxy-6-oxa-5-boranuidatricyclo[5.4.0.0^{2,4}]undeca-1(7),8,10-triene-8-carboxylate 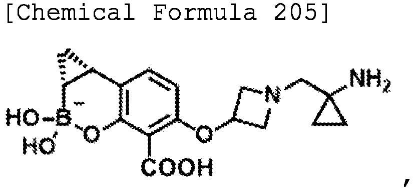
2-hydroxy-5-({1-[(1H-1,2,4-triazol-3-yl)methyl]azetidin-3-yl}oxy)-1,1a,2,7b-tetrahydrocyclopropa[c] [1,2]benzoxaborinine-4-carboxylic acid 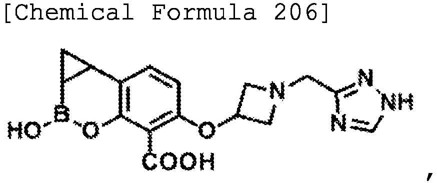
5,5-dihydroxy-9-{1-[(1H-1,2,4-triazol-3-yl)methyl]azetidin-3-yl}oxy-5-boranuidatricyclo[5.4.0.0^{2,4}]undeca-1(11),7,9-triene-8-carboxylic acid 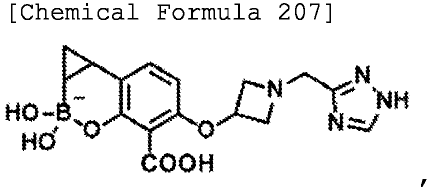
(1aS,7bR)-2-hydroxy-5-({1-[(1H-1,2,4-triazol-3-yl)methyl]azetidin-3-yl}oxy)-1,1a,2,7b-tetrahydrocyclopropa[c] [1,2]benzoxaborinine-4-carboxylic acid 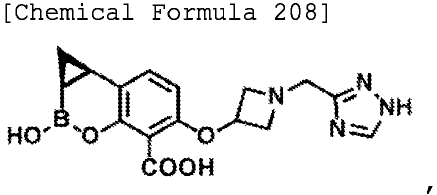
(2R,4S)-5,5-dihydroxy-9-{1-[(1H-1,2,4-triazol-3-yl)methyl]azetidin-3-yl}oxy-5-boranuidatricyclo[5.4.0.0^{2,4}]undeca-1(11),7,9-triene-8-carboxylic acid 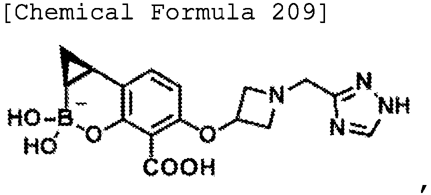
(1aR,7bS)-2-hydroxy-5-({1-[(1H-1,2,4-triazol-3-yl)methyl]azetidin-3-yl}oxy)-1,1a,2,7b-tetrahydrocyclopropa[c] [1,2]benzoxaborinine-4-carboxylic acid 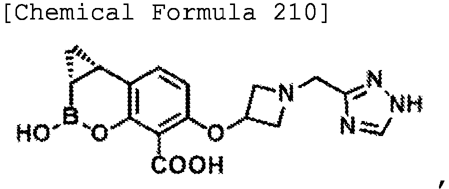
(2S,4R)-5,5-dihydroxy-9-{1-[(1H-1,2,4-triazol-3-yl)methyl]azetidin-3-yl}oxy-5-boranuidatricyclo[5.4.0.0^{2,4}]undeca-1(11),7,9-triene-8-carboxylic acid 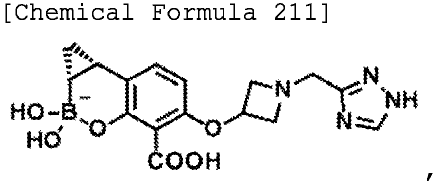
5-{[1-({4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 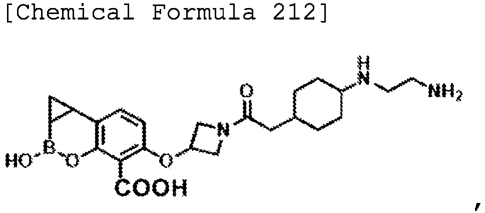
9-[1-({4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 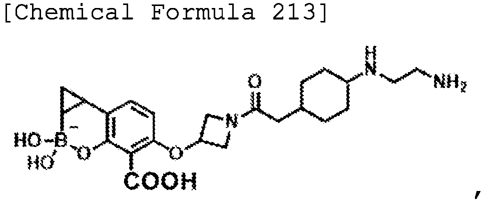
(1aS,7bR)-5-{[1-({(1r,4r)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 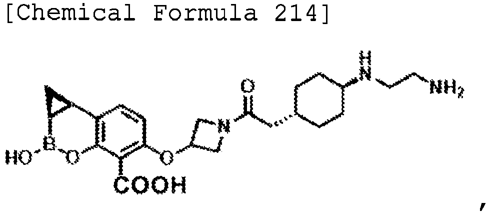
(2R,4S)-9-[1-({(1r,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 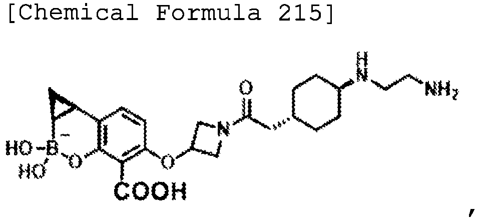
(1aS,7bR)-5-{[1-({(1s,4r)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 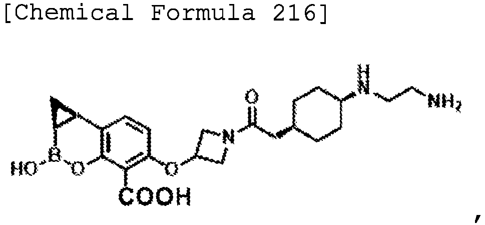
(2R,4S)-9-[1-({(1s,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 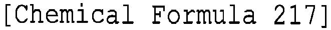 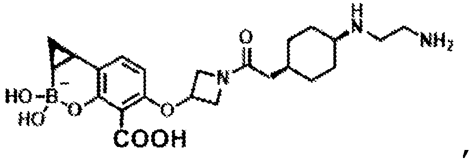
(1aS,7bR)-5-{[1-({(1r,4s)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 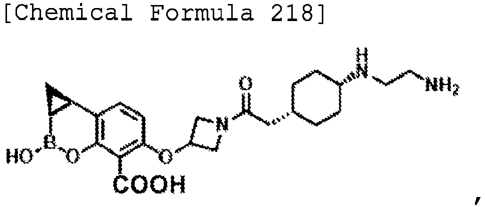
(2R,4S)-9-[1-({(1r,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 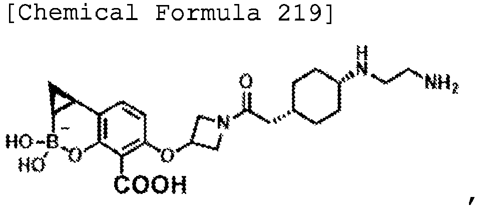
(1aS,7bR)-5-{[1-({(1s,4s)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 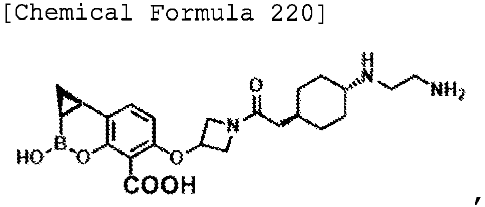
(2R,4S)-9-[1-({(1s,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 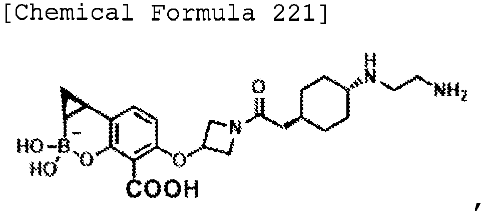
(1aR,7bS)-5-{[1-({(1r,4r)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 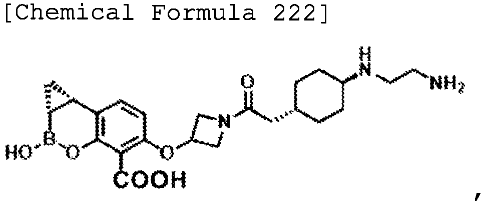
(2S,4R)-9-[1-({(1r,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 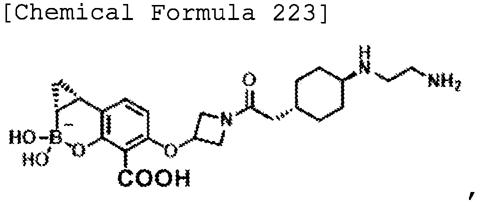
(1aR,7bS)-5-{[1-({(1s,4r)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 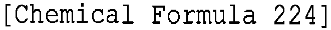 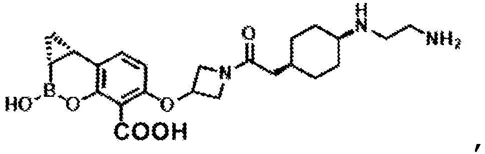
(2S,4R)-9-[1-({(1s,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 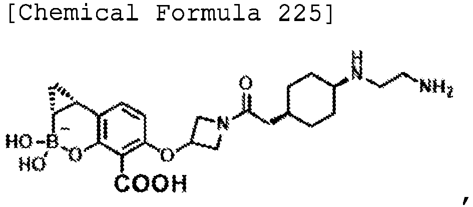
(1aR,7bS)-5-{[1-({(1r,4s)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 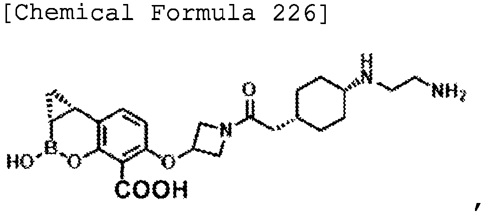
(2S,4R)-9-[1-({(1r,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 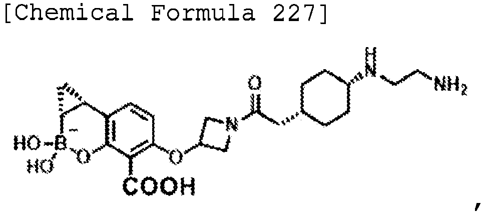
(1aR,7bS)-5-{[1-({(1s,4s)-4-[(2-aminoethyl)amino]cyclohexyl}acetyl)azetidin-3-yl]oxy}-2-hydroxy-1,1a,2,7b-tetrahydrocyclopropa[c] [1,2]benzoxaborinine-4-carboxylic acid 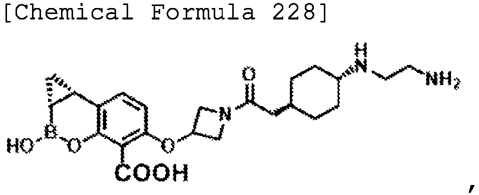
(2S,4R)-9-[1-({(1s,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 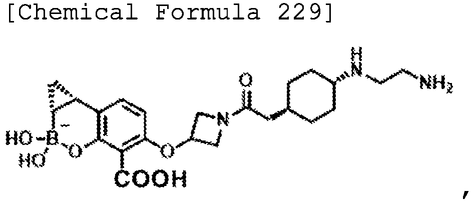
5-[(1-{2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 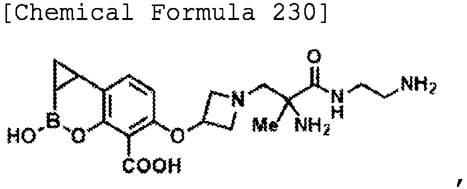
9-(1-{2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 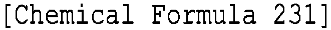 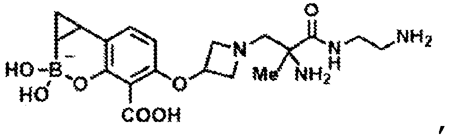
(1aS,7bR)-5-[(1-{ (2R)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 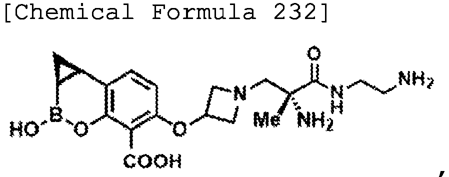
(2R,4S)-9-(1-{ (2R)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 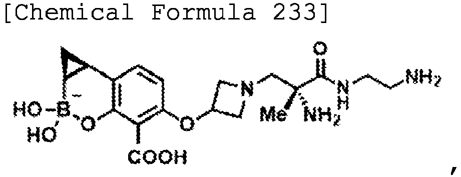
(1aS,7bR)-5-[(1-{ (2S)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 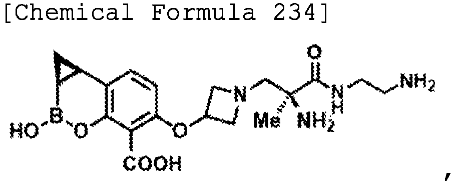
(2R,4S)-9-(1-{ (2S)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 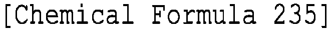 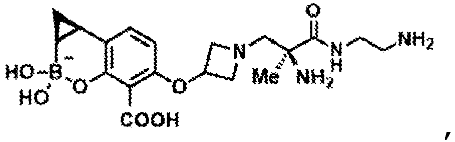
(1aR,7bS)-5-[(1-{ (2R)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 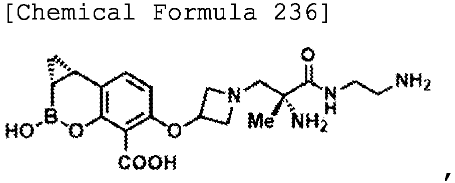
(2S,4R)-9-(1-1(2R)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 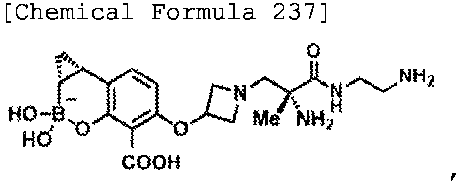
(1aR,7bS)-5-[(1-{ (2S)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 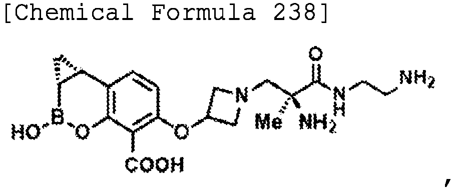
(2S,4R)-9-(1-{ (2S)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 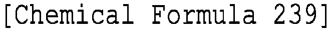 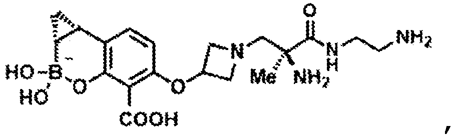
5-[(1-histidylazetidin-3-yl)oxy]-2-hydroxy-1,1a,2,7b-tetrahydrocyclopropa[c][1,2]benzoxaborinine-4-carboxylic acid 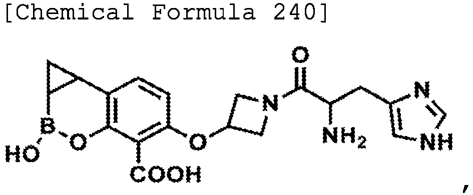
9-(1-histidylazetidin-3-yl)oxy-5,5-dihydroxy-6-oxa-5-boranuidatricyclo[5.4.0.0^{2,4}]undeca-1(7),8,10-triene-8-carboxylic acid 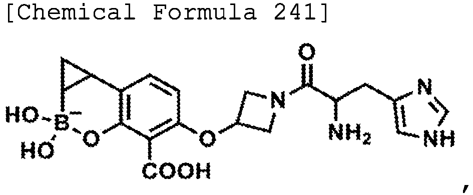
(1aS,7bR)-5-[(1-D-histidylazetidin-3-yl)oxy]-2-hydroxy-1,1a,2,7b-tetrahydrocyclopropa[c][1,2]benzoxaborinine-4-carboxylic acid 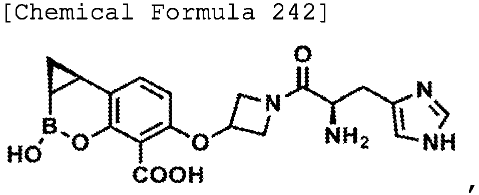
(2R,4S)-9-(1-D-histidylazetidin-3-yl)oxy-5,5-dihydroxy-6-oxa-5-boranuidatricyclo[5.4.0.0^{2,4}]undeca-1(7),8,10-triene-8-carboxylic acid 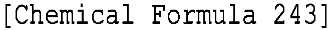 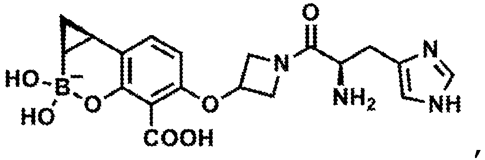
(1aS,7bR)-5-[(1-L-histidylazetidin-3-yl)oxy]-2-hydroxy-1,1a,2,7b-tetrahydrocyclopropa[c][1,2]benzoxaborinine-4-carboxylic acid 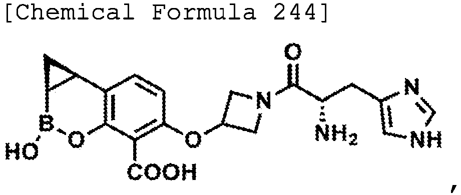
(2R,4S)-9-(1-L-histidylazetidin-3-yl)oxy-5,5-dihydroxy-6-oxa-5-boranuidatricyclo[5.4.0.0^{2,4}]undeca-1(7),8,10-triene-8-carboxylic acid 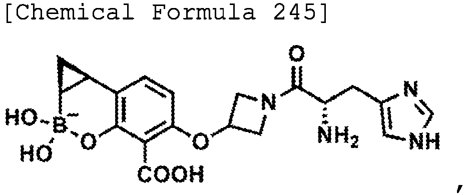
(1aR,7bS)-5-[(1-D-histidylazetidin-3-yl)oxy]-2-hydroxy-1,1a,2,7b-tetrahydrocyclopropa[c][1,2]benzoxaborinine-4-carboxylic acid 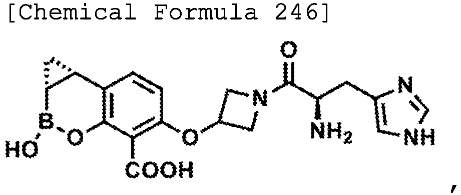
(2S,4R)-9-(1-D-histidylazetidin-3-yl)oxy-5,5-dihydroxy-6-oxa-5-boranuidatricyclo[5.4.0.0^{2,4}]undeca-1(7),8,10-triene-8-carboxylic acid 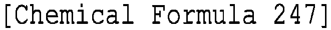 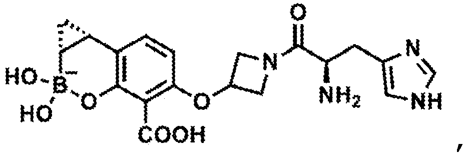
(1aR,7bS)-5-[(1-L-histidylazetidin-3-yl)oxy]-2-hydroxy-1,1a,2,7b-tetrahydrocyclopropa[c][1,2]benzoxaborinine-4-carboxylic acid 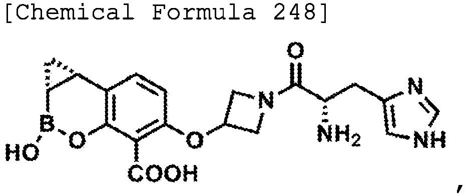
(2S,4R)-9-(1-L-histidylazetidin-3-yl)oxy-5,5-dihydroxy-6-oxa-5-boranuidatricyclo[5.4.0.0^{2,4}]undeca-1(7),8,10-triene-8-carboxylic acid 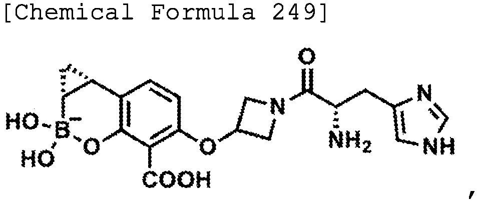
5-{[1-(2-aminoethyl)azetidin-3-yl]oxy}-2-hydroxy-1,1a,2,7b-tetrahydrocyclopropa[c][1,2]benzoxaborinine-4-carboxylic acid 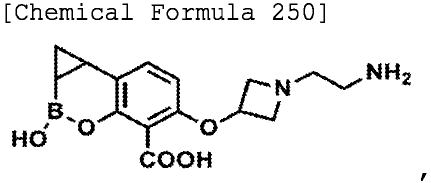
9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid 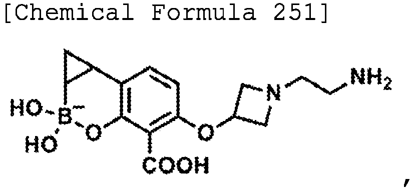
(1aS,7bR)-5-{[1-(2-aminoethyl)azetidin-3-yl]oxy}-2-hydroxy-1,1a,2,7b-tetrahydrocyclopropa[c] [1,2]benzoxaborinine-4-carboxylic acid 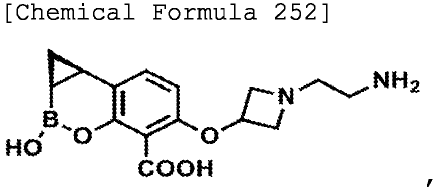
(2R,4S)-9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid 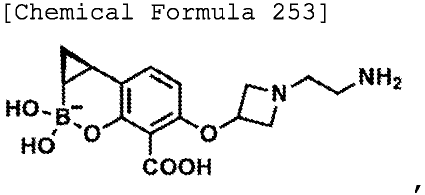
(1aR,7bS)-5-{[1-(2-aminoethyl)azetidin-3-yl]oxy}-2-hydroxy-1,1a,2,7b-tetrahydrocyclopropa[c] [1,2]benzoxaborinine-4-carboxylic acid 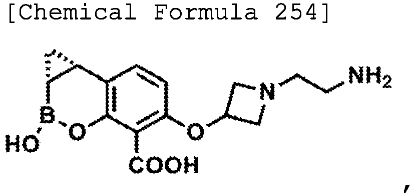
(2S,4R)-9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid 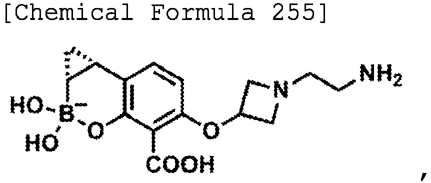
5-[(1-{[5-(aminomethyl)morpholin-2-yl]acetyl}azetidin-3-yl)oxy]-2-hydroxy-1,1a,2,7b-tetrahydrocyclopropa[c] [1,2]benzoxaborinine-4-carboxylic acid 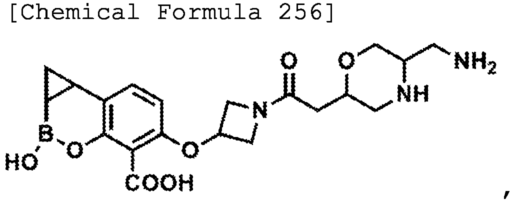
9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid 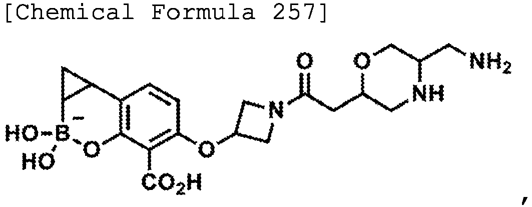
(1aS,7bR)-5-[(1-{ [5-(aminomethyl)morpholin-2-yl]acetyl}azetidin-3-yl)oxy]-2-hydroxy-1,1a,2,7b-tetrahydrocyclopropa[c][1,2]benzoxaborinine-4-carboxylic acid 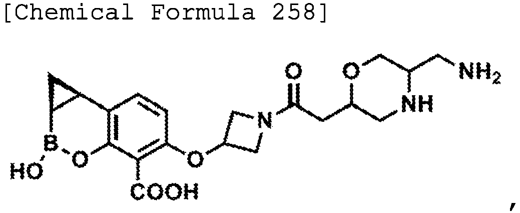
(2R,4S)-9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid 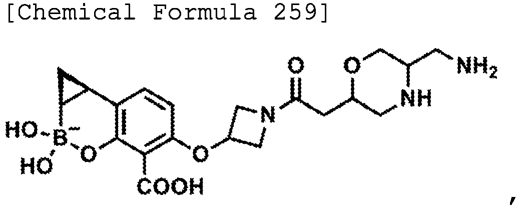
(1aR,7bS)-5-[(1-{ [5-(aminomethyl)morpholin-2-yl]acetyl}azetidin-3-yl)oxy]-2-hydroxy-1,1a,2,7b-tetrahydrocyclopropa[c][1,2]benzoxaborinine-4-carboxylic acid 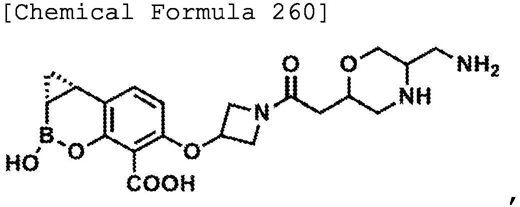
(2S,4R)-9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid 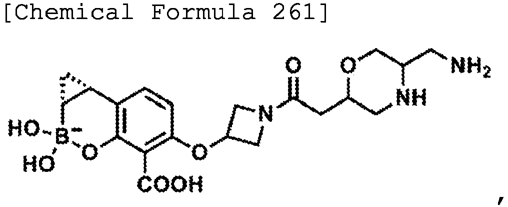
5-({1-[2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c][1,2]benzoxaborinine-4-carboxylic acid 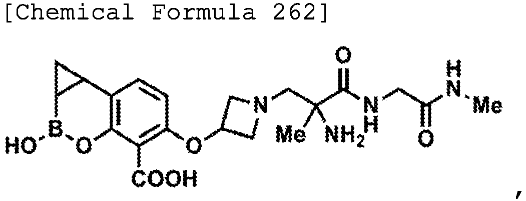
9-({1-[2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 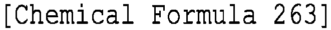 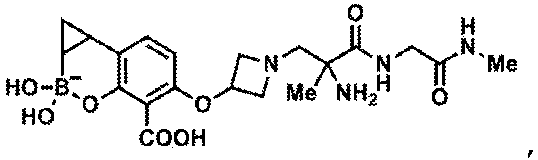
(1aS,7bR)-5-({1-[(2R)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c] [1,2]benzoxaborinine-4-carboxylic acid 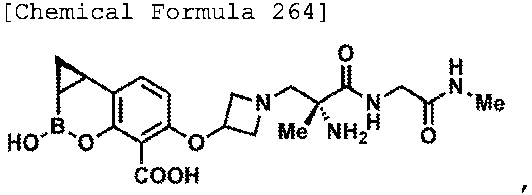
(2R,4S)-9-({1-[(2R)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 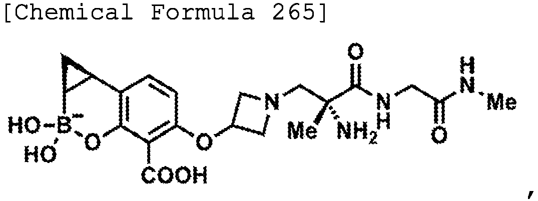
(1aS,7bR)-5-({1-[(2S)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c] [1,2]benzoxaborinine-4-carboxylic acid 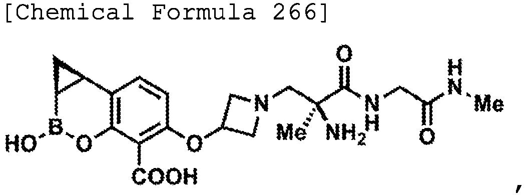
(2R,4S)-9-({l-[(2S)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 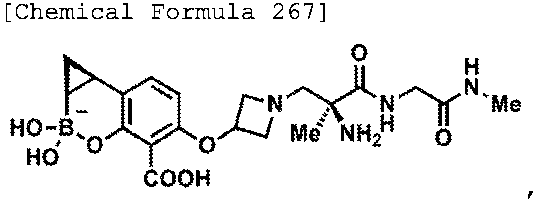
(1aR,7bS)-5-({1-[(2R)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c] [1,2]benzoxaborinine-4-carboxylic acid 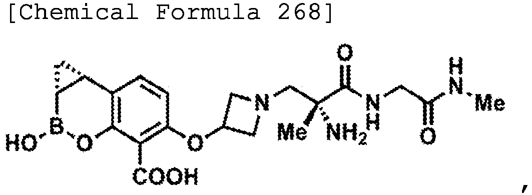
(2S,4R)-9-({1-[(2R)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 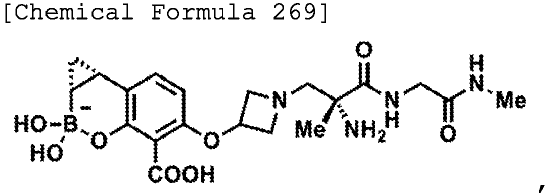
(1aR,7bS)-5-({1-[(2S)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c] [1,2]benzoxaborinine-4-carboxylic acid 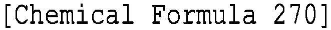 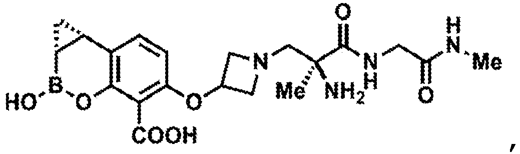
(2S,4R)-9-({1-[(2S)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 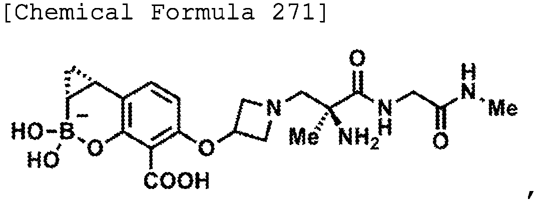
5-({1-[2-amino-3-{[2-(dimethylamino)-2-oxoethyl]amino}-2-methyl-3-oxopropyl]azetidin-3-yl}oxy)-2-hydroxy-1,1a,2,7b-tetrahydrocyclopropa[c][1,2]benzoxaborinine-4-carboxylic acid 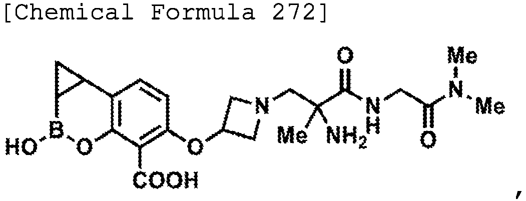
9-({1-[2-amino-3-{[2-(dimethylamino)-2-oxoethyl]amino}-2-methyl-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 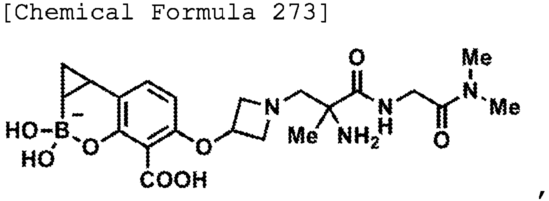
(1aS,7bR)-5-({1-[(2R)-2-amino-3-{[2-(dimethylamino)-2-oxoethyl]amino}-2-methy1-3-oxopropyl]azetidin-3-yl}oxy)-2-hydroxy-1,1a,2,7b-tetrahydrocyclopropa[c] [1,2]benzoxaborinine-4-carboxylic acid 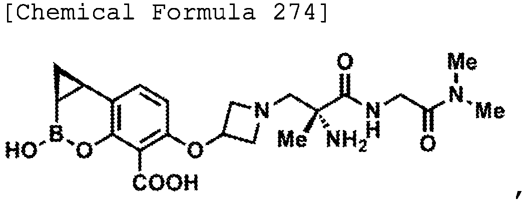
(2R,4S)-9-({1-[(2R)-2-amino-3-{[2-(dimethylamino)-2-oxoethyl]amino}-2-methyl-3-oxopropyl] azetidin-3-yl}oxy )-5,5-dihydroxy-6-oxa-5-boranuidatricyclo[5.4.0.0^{2,4}]undeca-1(7),8,10-triene-8-carboxylic acid 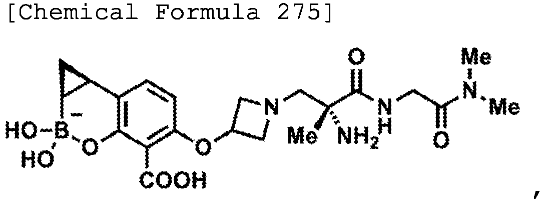
(1aS,7bR)-5-({1-[(2S)-2-amino-3-{[2-(dimethylamino)-2-oxoethyl]amino}-2-methy1-3-oxopropyl]azetidin-3-yl}oxy)-2-hydroxy-1,1a,2,7b-tetrahydrocyclopropa[c] [1,2]benzoxaborinine-4-carboxylic acid 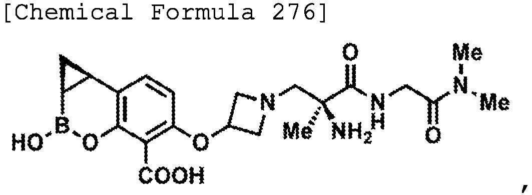
(2R,4S)-9-({1-[(2S)-2-amino-3-{[2-(dimethylamino)-2-oxoethyl]amino}-2-methyl-3-oxopropyl] azetidin-3-yl}oxy )-5,5-dihydroxy-6-oxa-5-boranuidatricyclo[5.4.0.0^{2,4}]undeca-1(7),8,10-triene-8-carboxylic acid 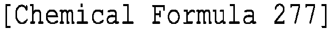 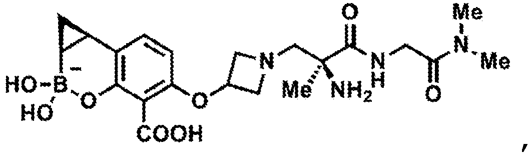
(1aR,7bS)-5-({1-[(2R)-2-amino-3-{[2-(dimethylamino)-2-oxoethyl]amino}-2-methy1-3-oxopropyl]azetidin-3-yl}oxy)-2-hydroxy-1,1a,2,7b-tetrahydrocyclopropa[c] [1,2]benzoxaborinine-4-carboxylic acid 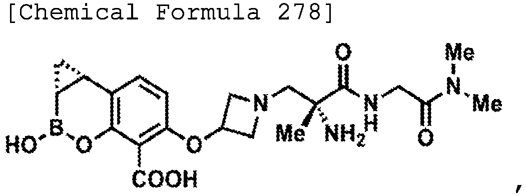
(2S,4R)-9-({1-[(2R)-2-amino-3-{[2-(dimethylamino)-2-oxoethyl]amino}-2-methyl-3-oxopropyl] azetidin-3-yl}oxy )-5,5-dihydroxy-6-oxa-5-boranuidatricyclo[5.4.0.0^{2,4}]undeca-1(7),8,10-triene-8-carboxylic acid 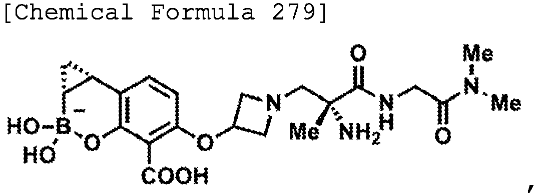
(1aR,7bS)-5-({1-[(2S)-2-amino-3-{[2-(dimethylamino)-2-oxoethyl]amino}-2-methy1-3-oxopropyl]azetidin-3-yl}oxy)-2-hydroxy-1,1a,2,7b-tetrahydrocyclopropa[c] [1,2]benzoxaborinine-4-carboxylic acid 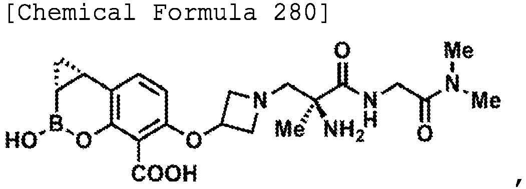
(2S,4R)-9-({1-[(2S)-2-amino-3-{[2-(dimethylamino)-2-oxoethyl]amino}-2-methy1-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 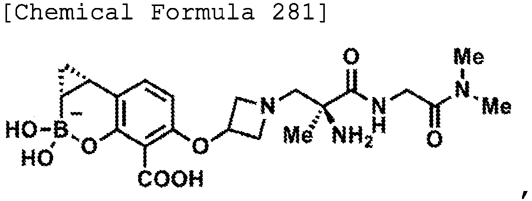
2-hydroxy-5-[(1-{[4-methylmorpholin-2-yl]acetyl}azetidin-3-yl)oxy]-1,1a,2,7b-tetrahydrocyclopropa[c] [1,2]benzoxaborinine-4-carboxylic acid 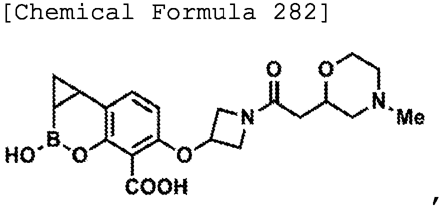
5,5-dihydroxy-9-[(1-{[(4-methylmorpholin-2-yl]acetyl}azetidin-3-yl)oxy]-5-boranuidatricyclo[5.4.0.0^{2,4}]undeca-1(11),7,9-triene-8-carboxylic acid 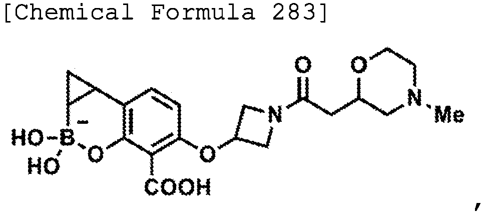
(1aS,7bR)-2-hydroxy-5-[(1-{ [(2R)-4-methylmorpholin-2-yl]acetyl}azetidin-3-yl)oxy]-1,1a,2,7b-tetrahydrocyclopropa[c][1,2]benzoxaborinine-4-carboxylic acid 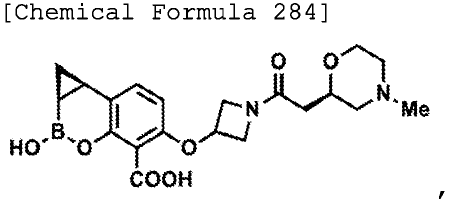
(2R,4S)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid 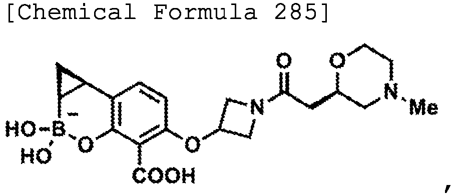
(1aS,7bR)-2-hydroxy-5-[(1-{ [(2S)-4-methylmorpholin-2-yl]acetyl}azetidin-3-yl)oxy]-1,1a,2,7b-tetrahydrocyclopropa[c][1,2]benzoxaborinine-4-carboxylic acid 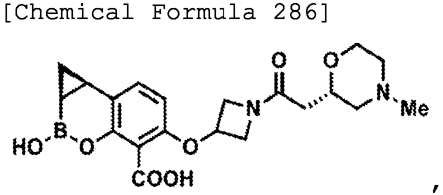
(2R,4S)-5,5-dihydroxy-9-[(1-{[(2S)-4-methylmorpholin-2-yl]acetyl}azetidin-3-yl)oxy]-5-boranuidatricyclo[5.4.0.0^{2,4}]undeca-1(11),7,9-triene-8-carboxylic acid 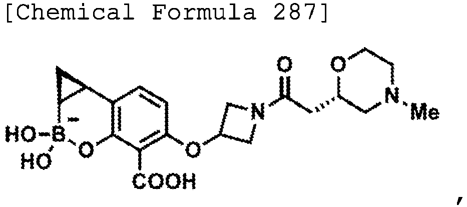
(1aR,7bS)-2-hydroxy-5-[(1-{ [(2R)-4-methylmorpholin-2-yl]acetyl}azetidin-3-yl)oxy]-1,1a,2,7b-tetrahydrocyclopropa[c][1,2]benzoxaborinine-4-carboxylic acid 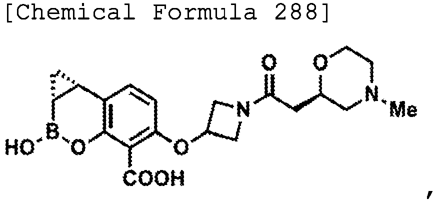
(2S,4R)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid 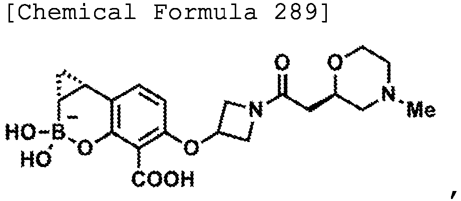
(1aR,7bS)-2-hydroxy-5-[(1-{ [(2S)-4-methylmorpholin-2-yl]acetyl}azetidin-3-yl)oxy]-1,1a,2,7b-tetrahydrocyclopropa[c][1,2]benzoxaborinine-4-carboxylic acid 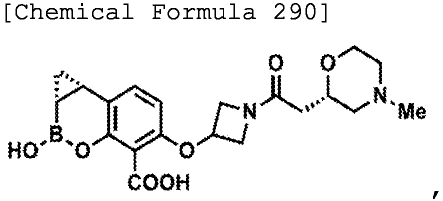
(2S,4R)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid 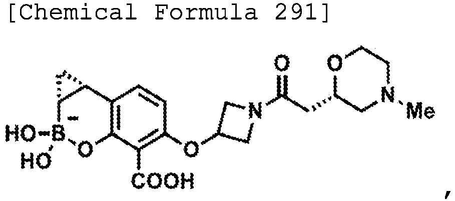
5-[(1-{[4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 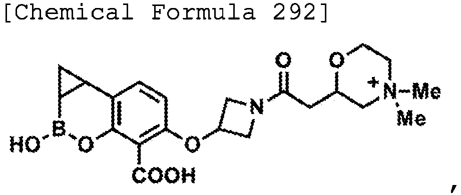
9-[(1-{[4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 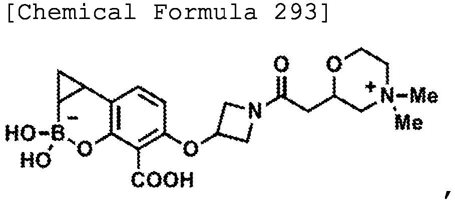
(1aS,7bR)-5-[(1-{[(2R)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 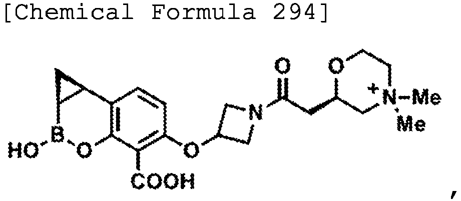
(2R,4S)-9-[(1-{[(2R)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 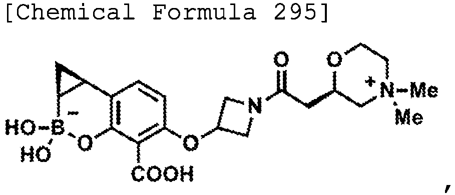
(1aS,7bR)-5-[(1-{[(2S)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 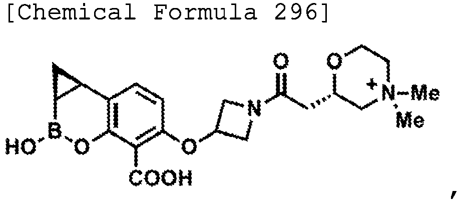
(2R,4S)-9-[(1-{[(2S)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 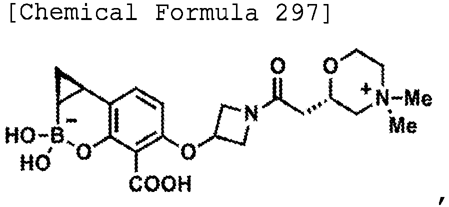
(1aR,7bS)-5-[(1-{[(2R)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 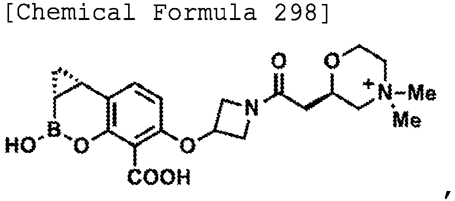
(2S,4R)-9-[(1-{[(2R)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 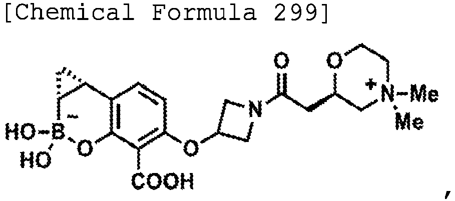
(1aR,7bS)-5-[(1-{[(2S)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 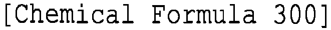 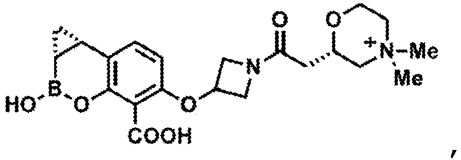
(2S,4R)-9-[(1-{[(2S)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 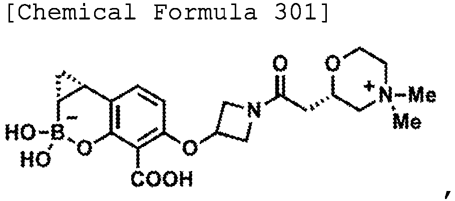
(1aR,7bS)-2-hydroxy-5-({1-[(3-hydroxy-2-methyl-4-oxopyridin-1(4H)-yl)acetyl]azetidin-3-yl}oxy)-1,1a,2,7b-tetrahydrocyclopropa[c][1,2]benzoxaborinine-4-carboxylic acid 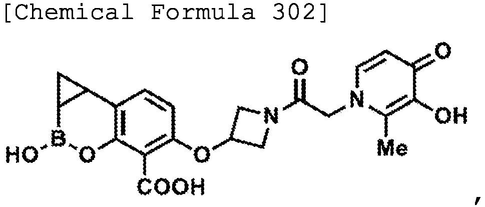
5,5-dihydroxy-9-({1-[(3-hydroxy-2-methyl-4-oxopyridin-1(4H)-yl)acetyl]azetidin-3-yl}oxy)-5-boranuidatricyclo[5.4.0.02,4]undeca-1(11),7,9-triene-8-carboxylic acid 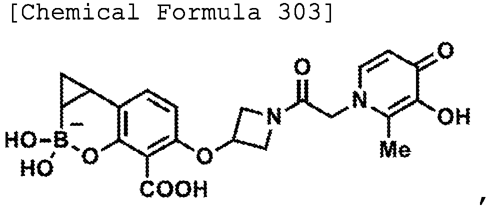
(1aR,7bS)-2-hydroxy-5-({1-[(3-hydroxy-2-methyl-4-oxopyridin-1(4H)-yl)acetyl]azetidin-3-yl}oxy)-1,1a,2,7b-tetrahydrocyclopropa[c][1,2]benzoxaborinine-4-carboxylic acid 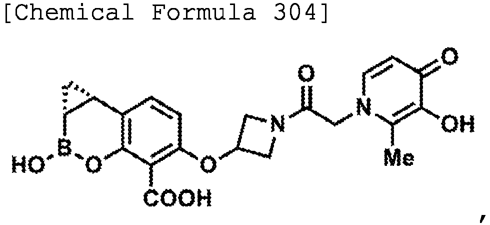
(2S,4R)-5,5-dihydroxy-9-({1-[(3-hydroxy-2-methyl-4-oxopyridin-1(4H)-yl)acetyl]azetidin-3-yl}oxy)-5-boranuidatricyclo[5.4.0.02,4]undeca-1(11),7,9-triene-8-carboxylic acid 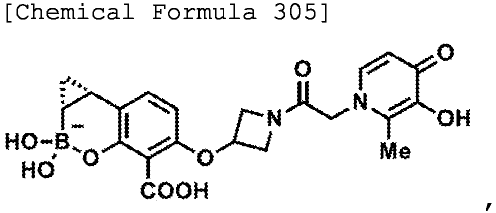
(1aS,7bR)-2-hydroxy-5-({1-[(3-hydroxy-2-methyl-4-oxopyridin-1(4H)-yl)acetyl]azetidin-3-yl}oxy)-1,1a,2,7b-tetrahydrocyclopropa[c][1,2]benzoxaborinine-4-carboxylic acid 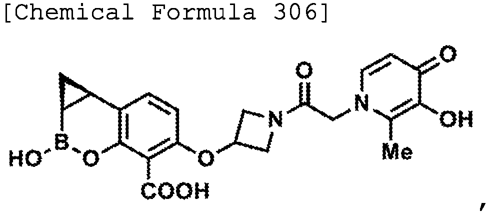 and
(2R,4S)-5,5-dihydroxy-9-({1-[(3-hydroxy-2-methyl-4-oxopyridin-1(4H)-yl)acetyl]azetidin-3-yl}oxy)-5-boranuidatricyclo[5.4.0.02,4]undeca-1(11),7,9-triene-8-carboxylic acid

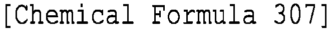

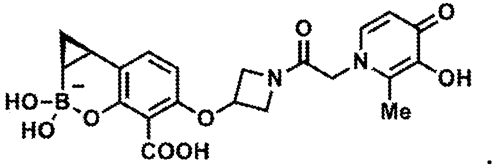

### [Item 103]

A compound represented by formula (11): 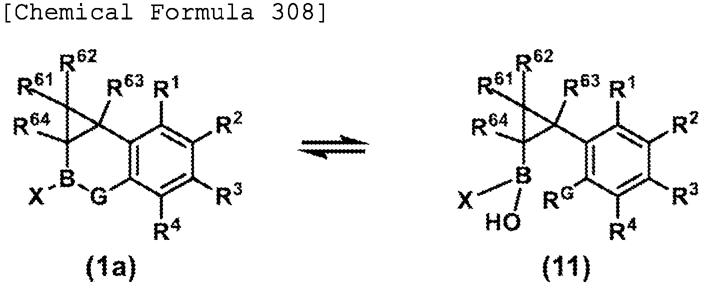 or a pharmaceutically acceptable salt thereof, wherein R^{G} is a hydroxyl group, a thiol group, or -NHR^{a1}, R^{a1}, X, R¹, R², R³, R⁴, R⁶¹, R⁶², R⁶³, and R⁶⁴ are defined the same as the definition according to any of items 1 to 16, and formula (1a) is defined the same as item 1 or 2.

### [Item 104]

The compound or the pharmaceutically acceptable salt thereof according to item 103, wherein the compound of formula (11) is represented by formula (12): 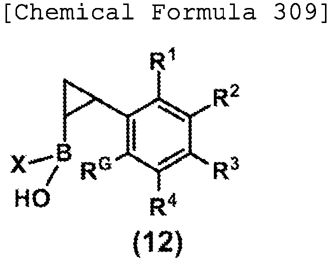 wherein X, R¹, R², R³, and R⁴ are defined the same as the definition according to item 17 or 18.

### [Item 105]

The compound or the pharmaceutically acceptable salt thereof according to item 103 or 104, wherein the compound of formula (11) or formula (12) is represented by formula (13): 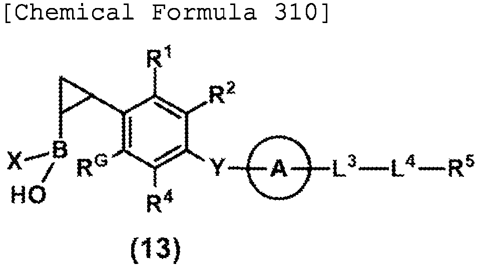 wherein X, Y, ring A, L³, L⁴, R¹, R², R⁴, and R⁵ are defined the same as the definition according to any of items 19 to 23 and 29 to 32.

### [Item 106]

The compound or the pharmaceutically acceptable salt thereof according to item 105, wherein X and R^{G} are hydroxyl groups, R⁴ is a carboxyl group, and ring A is an optionally substituted 4- to 6-membered nitrogen-containing non-aryl heterocycle.

### [Item 107]

The compound or the pharmaceutically acceptable salt thereof according to any one of items 103 to 106, wherein the compound of formula (11), formula (12), or formula (13) is represented by formula (14): 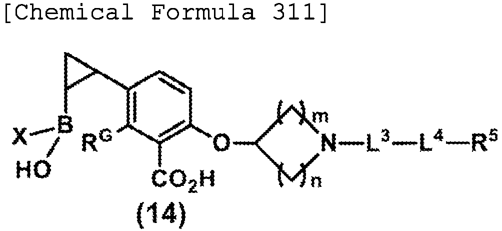 wherein X, L³, L⁴, m, n, and R⁵ are defined the same as the definition according to any one of items 33 to 64.

### [Item 108]

The compound or the pharmaceutically acceptable salt thereof according to item 107, wherein the compound of formula (14) is one of the enantiomers represented by formula (15): 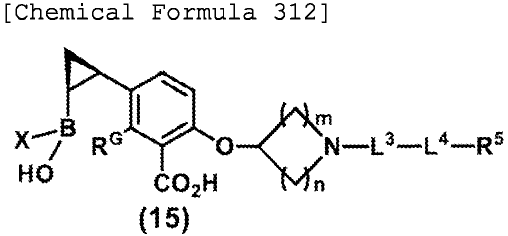 and
formula (16): 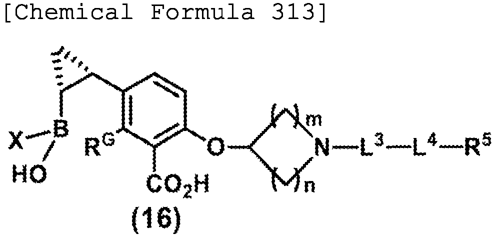 wherein X, L³, L⁴, m, n, and R⁵ are defined the same as the definition according to any one of items 33 to 64.

### [Item 109]

The compound or the pharmaceutically acceptable salt thereof according to any one of items 103 to 108, wherein R^{G} is a hydroxyl group or a thiol group.

### [Item 110]

The compound or the pharmaceutically acceptable salt thereof according to any one of items 103 to 109, wherein R^{G} is a hydroxyl group.

### [Item 111]

The compound or the pharmaceutically acceptable salt thereof according to any one of items 103 to 110, wherein X is a hydroxyl group or a C₁₋₆ alkoxy group.

### [Item 112]

The compound or the pharmaceutically acceptable salt thereof according to any one of items 103 to 111, wherein X is a hydroxyl group.

### [Item 113]

The compound or the pharmaceutically acceptable salt thereof according to any one of items 107 to 112, wherein m is 1 or 2, n is 1 or 2, and m + n is 2 or 3.

### [Item 114]

The compound or the pharmaceutically acceptable salt thereof according to any one of items 107 to 113, wherein m is 1, and n is 1.

### [Item 115]

The compound or the pharmaceutically acceptable salt thereof according to any one of items 103 to 114, wherein L³ is defined the same as the definition according to item 25 or 26.

### [Item 116]

The compound or the pharmaceutically acceptable salt thereof according to any one of items 103 to 115, wherein L⁴ and R⁵ are defined the same as the definition according to any one of items 27 and 66 to 100.

### [Item 117]

The compound or the pharmaceutically acceptable salt thereof according to item 104, selected from the group consisting of the compounds represented by the following names or structural formulas:
6-({1-[amino(1H-imidazol-4-yl)acetyl]azetidin-3-yl}oxy)-3-(2-boronocyclopropyl)-2-hydroxybenzoic acid 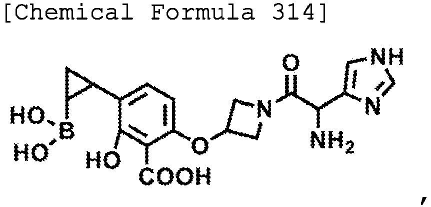
6-({1-[(2R)-2-amino-2-(1H-imidazol-4-yl)acetyl]azetidin-3-yl}oxy)-3-[(1R,2S)-2-boronocyclopropyl]-2-hydroxybenzoic acid 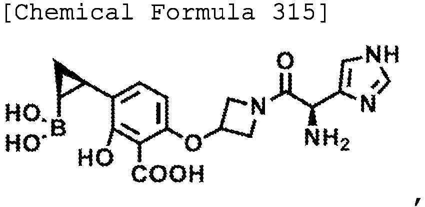
6-({1-[(2S)-2-amino-2-(1H-imidazol-4-yl)acetyl]azetidin-3-yl}oxy)-3-[(1R,2S)-2-boronocyclopropyl]-2-hydroxybenzoic acid 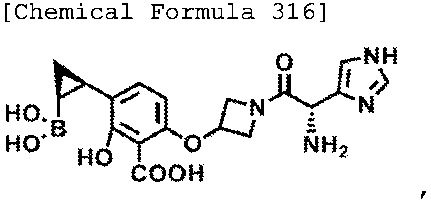
6-({1-[(2R)-2-amino-2-(1H-imidazol-4-yl)acetyl]azetidin-3-yl}oxy)-3-[(1S,2R)-2-boronocyclopropyl]-2-hydroxybenzoic acid 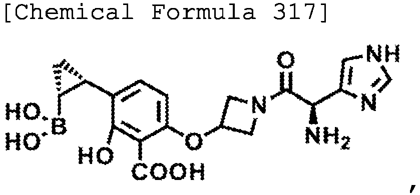
6-({1-[(2S)-2-amino-2-(1H-imidazol-4-yl)acetyl]azetidin-3-yl}oxy)-3-[(1S,2R)-2-boronocyclopropyl]-2-hydroxybenzoic acid 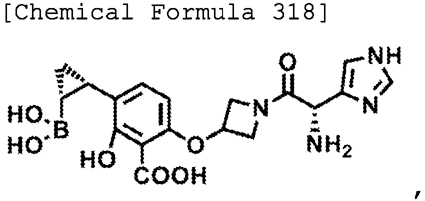
6-({1-(2-amino-2-carboxypropyl)azetidin-3-yl}oxy)-3-(2-boronocyclopropyl)-2-hydroxybenzoic acid 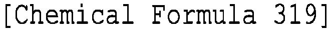 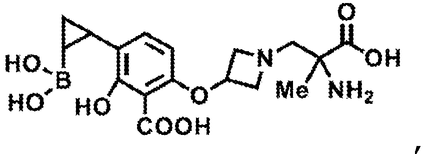
6-({1-[(2R)-2-amino-2-carboxypropyl]azetidin-3-yl}oxy)-3-[(1R,2S)-2-boronocyclopropyl]-2-hydroxybenzoic acid 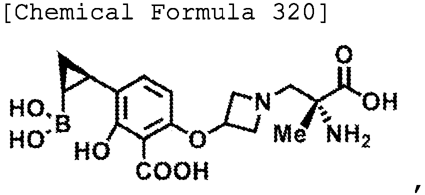
6-({1-[(2R)-2-amino-2-carboxypropyl]azetidin-3-yl}oxy)-3-[(1S,2R)-2-boronocyclopropyl]-2-hydroxybenzoic acid 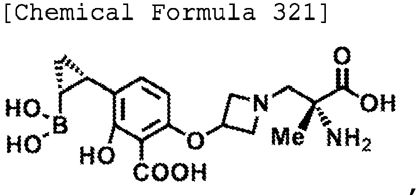
6-({1-[2-amino-2-(1H-imidazol-4-yl)propanoyl]azetidin-3-yl}oxy)-3-(2-boronocyclopropyl)-2-hydroxybenzoic acid 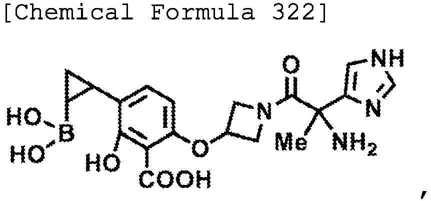
6-({1-[(2R)-2-amino-2-(1H-imidazol-4-yl)propanoyl]azetidin-3-yl}oxy)-3-[(1R,2S)-2-boronocyclopropyl]-2-hydroxybenzoic acid 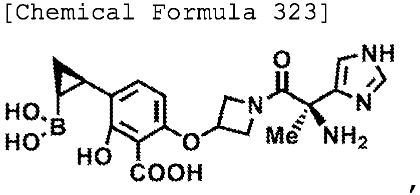
6-({1-[(2S)-2-amino-2-(1H-imidazol-4-yl)propanoyl]azetidin-3-yl}oxy)-3-[(1R,2S)-2-boronocyclopropyl]-2-hydroxybenzoic acid 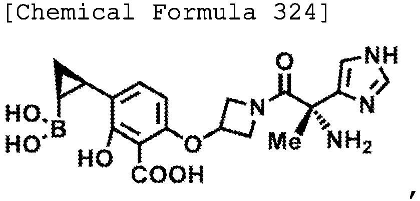
6-({1-[(2R)-2-amino-2-(1H-imidazol-4-yl)propanoyl]azetidin-3-yl}oxy)-3-[(1S,2R)-2-boronocyclopropyl]-2-hydroxybenzoic acid 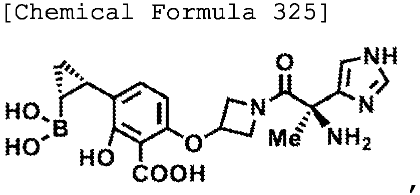 and
6-({1-[(2S)-2-amino-2-(1H-imidazol-4-yl)propanoyl]azetidin-3-yl}oxy)-3-[(1S,2R)-2-boronocyclopropyl]-2-hydroxybenzoic acid

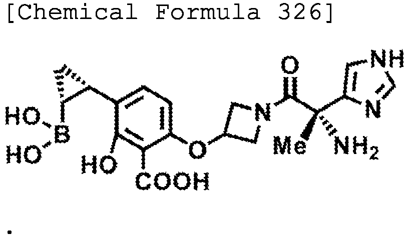

### [Item 118]

The compound or the pharmaceutically acceptable salt thereof according to item 104, selected from the group consisting of the compounds represented by the following names or structural formulas:
2-boronocyclopropyl]-2-hydroxy-6-({1-[(1H-imidazol-4-yl)acetyl]azetidin-3-yl}oxy)benzoic acid 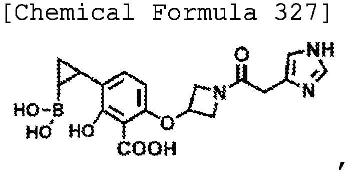
3-[(1R,2S)-2-boronocyclopropyl]-2-hydroxy-6-({1-[(1H-imidazol-4-yl)acetyl]azetidin-3-yl}oxy)benzoic acid 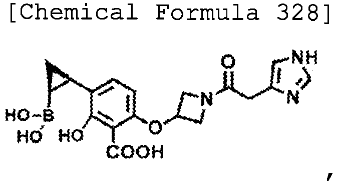
3-[(1S,2R)-2-boronocyclopropyl]-2-hydroxy-6-({1-[(1H-imidazol-4-yl)acetyl]azetidin-3-yl}oxy)benzoic acid 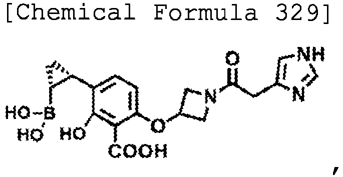
3-[2-boronocyclopropyl]-2-hydroxy-6-[(1-serylazetidin-3-yl)oxy]benzoic acid 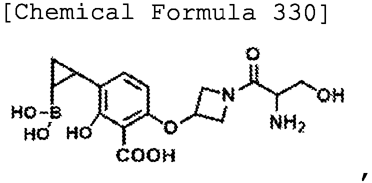
3-[(1R,2S)-2-boronocyclopropyl]-2-hydroxy-6-[(1-D-serylazetidin-3-yl)oxy]benzoic acid 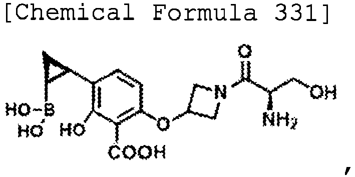
3-[(1R,2S)-2-boronocyclopropyl]-2-hydroxy-6-[(1-L-serylazetidin-3-yl)oxy]benzoic acid 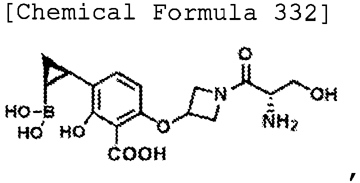
3-[(1S,2R)-2-boronocyclopropyl]-2-hydroxy-6-[(1-D-serylazetidin-3-yl)oxy]benzoic acid 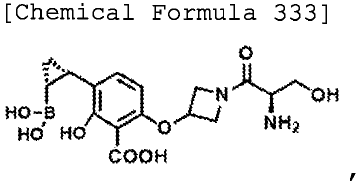
3-[(1S,2R)-2-boronocyclopropyl]-2-hydroxy-6-[(1-L-serylazetidin-3-yl)oxy]benzoic acid 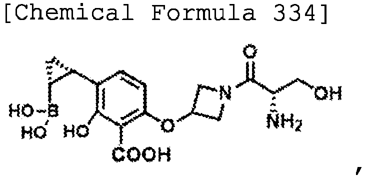
3-[2-boronocyclopropyl]-2-hydroxy-6-({1-[4-hydroxy-L-prolyl]azetidin-3-yl}oxy)benzoic acid 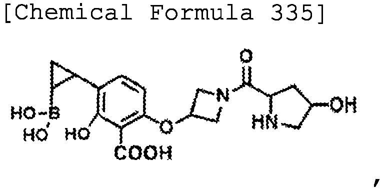
3-[(1R,2S)-2-boronocyclopropyl]-2-hydroxy-6-({1-[(4R)-4-hydroxy-L-prolyl]azetidin-3-yl}oxy)benzoic acid 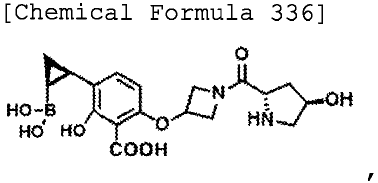
3-[(1R,2S)-2-boronocyclopropyl]-2-hydroxy-6-({1-[(4S)-4-hydroxy-L-prolyl]azetidin-3-yl}oxy)benzoic acid 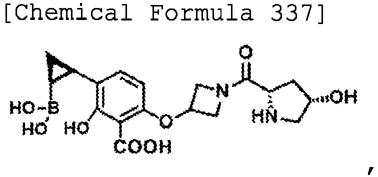
3-[(1R,2S)-2-boronocyclopropyl]-2-hydroxy-6-({1-[(4R)-4-hydroxy-D-prolyl]azetidin-3-yl}oxy)benzoic acid 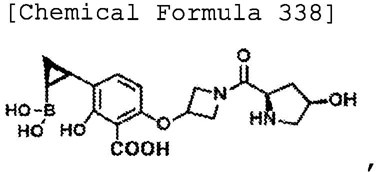
3-[(1R,2S)-2-boronocyclopropyl]-2-hydroxy-6-({1-[(4S)-4-hydroxy-D-prolyl]azetidin-3-yl}oxy)benzoic acid 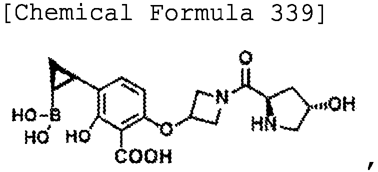
3-[(1S,2R)-2-boronocyclopropyl]-2-hydroxy-6-({1-[(4R)-4-hydroxy-L-prolyl]azetidin-3-yl}oxy)benzoic acid 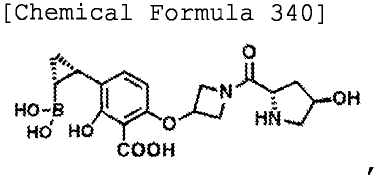
3-[(1S,2R)-2-boronocyclopropyl]-2-hydroxy-6-({1-[(4S)-4-hydroxy-L-prolyl]azetidin-3-yl}oxy)benzoic acid 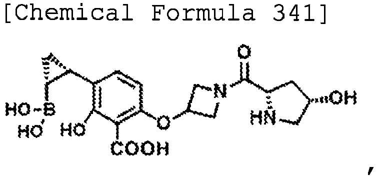
3-[(1S,2R)-2-boronocyclopropyl]-2-hydroxy-6-({1-[(4R)-4-hydroxy-D-prolyl]azetidin-3-yl}oxy)benzoic acid 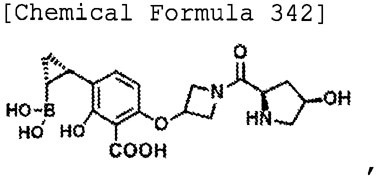
3-[(1S,2R)-2-boronocyclopropyl]-2-hydroxy-6-({1-[(4S)-4-hydroxy-D-prolyl]azetidin-3-yl}oxy)benzoic acid 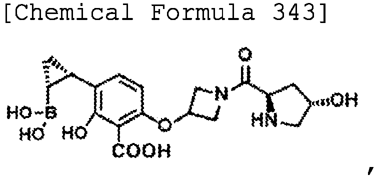
3-[2-boronocyclopropyl]-2-hydroxy-6-{[1-(2-methyl-seryl)azetidin-3-yl]oxy}benzoic acid 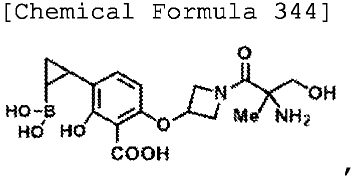
3-[(1R,2S)-2-boronocyclopropyl]-2-hydroxy-6-{[1-(2-methyl-D-seryl)azetidin-3-yl]oxy}benzoic acid 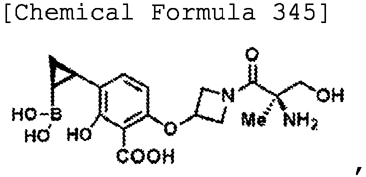
3-[(1R,2S)-2-boronocyclopropyl]-2-hydroxy-6-{[1-(2-methyl-L-seryl)azetidin-3-yl]oxy}benzoic acid 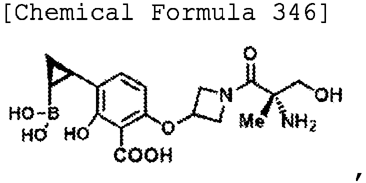
3-[(1S,2R)-2-boronocyclopropyl]-2-hydroxy-6-{[1-(2-methyl-D-seryl)azetidin-3-yl]oxy}benzoic acid 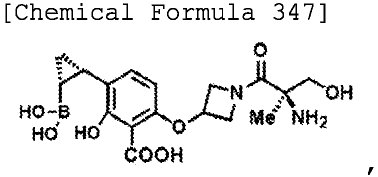
3-[(1S,2R)-2-boronocyclopropyl]-2-hydroxy-6-{[1-(2-methyl-L-seryl)azetidin-3-yl]oxy}benzoic acid 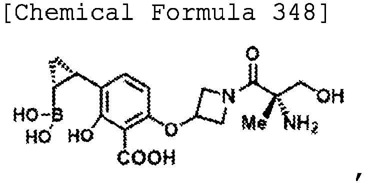
3-[(2-boronocyclopropyl]-2-hydroxy-6-[(1-{ [morpholin-2-yl]acetyl}azetidin-3-yl)oxy]benzoic acid 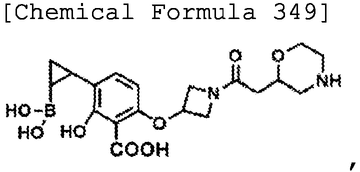
3-[(1R,2S)-2-boronocyclopropyl]-2-hydroxy-6-[(1-{ [(2R)-morpholin-2-yl]acetyl}azetidin-3-yl)oxy]benzoic acid 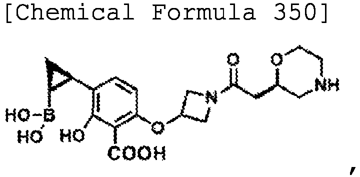
3-[(1R,2S)-2-boronocyclopropyl]-2-hydroxy-6-[(1-{ [(2S)-morpholin-2-yl]acetyl}azetidin-3-yl)oxy]benzoic acid 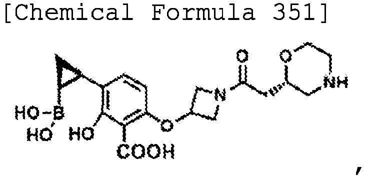
3-[(1S,2R)-2-boronocyclopropyl]-2-hydroxy-6-[(1-{ [(2R)-morpholin-2-yl]acetyl}azetidin-3-yl)oxy]benzoic acid 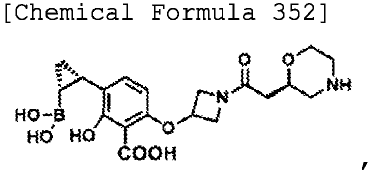
3-[(1S,2R)-2-boronocyclopropyl]-2-hydroxy-6-[(1-{ [(2S)-morpholin-2-yl]acetyl}azetidin-3-yl)oxy]benzoic acid 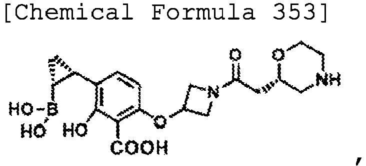
6-({1-[(2S)-2-amino-2-carboxypropyl]azetidin-3-yl}oxy)-3-[(1R,2S)-2-boronocyclopropyl]-2-hydroxybenzoic acid 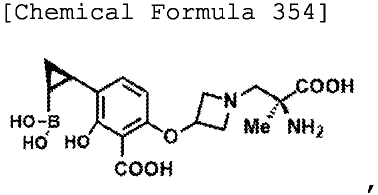
6-({1-[(2S)-2-amino-2-carboxypropyl]azetidin-3-yl}oxy)-3-[(1S,2R)-2-boronocyclopropyl]-2-hydroxybenzoic acid 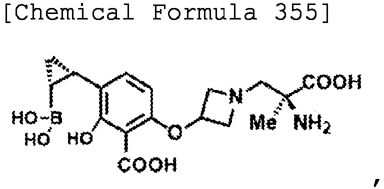
6-[(1-{2-amino-3-[(2-hydroxyethyl)amino]-2-methyl-3-oxopropyl}azetidin-3-yl)oxy]-3-[2-boronocyclopropyl]-2-hydroxybenzoic acid 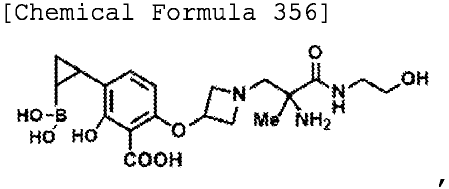
6-[(1-{ (2R)-2-amino-3-[(2-hydroxyethyl)amino]-2-methyl-3-oxopropyl}azetidin-3-yl)oxy]-3-[(1R,2S)-2-boronocyclopropyl]-2-hydroxybenzoic acid 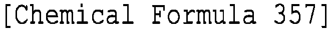 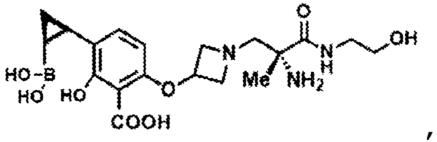
6-[(1-{ (2S)-2-amino-3-[(2-hydroxyethyl)amino]-2-methyl-3-oxopropyl}azetidin-3-yl)oxy]-3-[(1R,2S)-2-boronocyclopropyl]-2-hydroxybenzoic acid 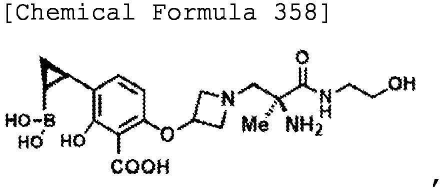
6-[(1-{ (2R)-2-amino-3-[(2-hydroxyethyl)amino]-2-methyl-3-oxopropyl}azetidin-3-yl)oxy]-3-[(1S,2R)-2-boronocyclopropyl]-2-hydroxybenzoic acid 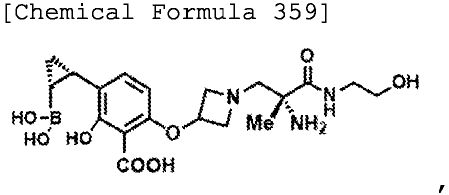
6-[(1-{ (2S)-2-amino-3-[(2-hydroxyethyl)amino]-2-methyl-3-oxopropyl}azetidin-3-yl)oxy]-3-[(1S,2R)-2-boronocyclopropyl]-2-hydroxybenzoic acid 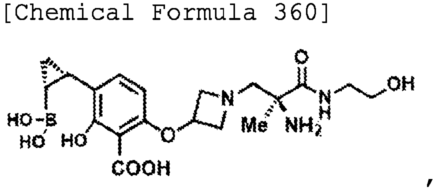
6-[(1-{2-amino-3-[(2-amino-2-oxoethyl)amino]-2-methyl-3-oxopropyl}azetidin-3-yl)oxy]-3-[2-boronocyclopropyl]-2-hydroxybenzoic acid 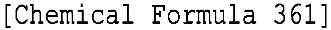 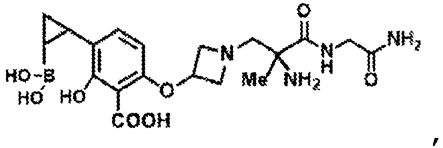
6-[(1-{ (2R)-2-amino-3-[(2-amino-2-oxoethyl)amino]-2-methyl-3-oxopropyl}azetidin-3-yl)oxy]-3-[(1R,2S)-2-boronocyclopropyl]-2-hydroxybenzoic acid 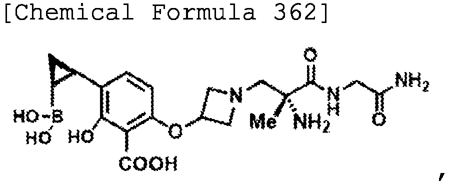
6-[(1-{ (2S)-2-amino-3-[(2-amino-2-oxoethyl)amino]-2-methyl-3-oxopropyl}azetidin-3-yl)oxy]-3-[(1R,2S)-2-boronocyclopropyl]-2-hydroxybenzoic acid 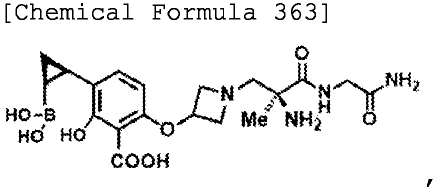
6-[(1-{ (2R)-2-amino-3-[(2-amino-2-oxoethyl)amino]-2-methyl-3-oxopropyl}azetidin-3-yl)oxy]-3-[(1S,2R)-2-boronocyclopropyl]-2-hydroxybenzoic acid 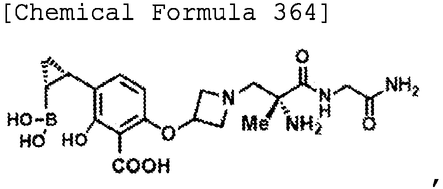
6-[(1-{ (2S)-2-amino-3-[(2-amino-2-oxoethyl)amino]-2-methyl-3-oxopropyl}azetidin-3-yl)oxy]-3-[(1S,2R)-2-boronocyclopropyl]-2-hydroxybenzoic acid 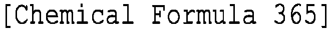 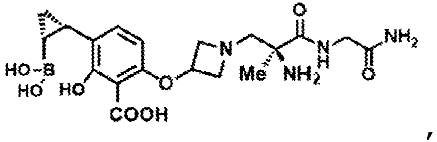
3-[2-boronocyclopropyl]-6-({1-[(5-carbamoylpyrrolidin-3-yl]azetidin-3-yl}oxy)-2-hydroxybenzoic acid 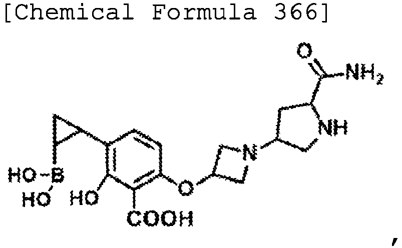
3-[(1R,2S)-2-boronocyclopropyl]-6-({1-[(3S,5S)-5-carbamoylpyrrolidin-3-yl]azetidin-3-yl}oxy)-2-hydroxybenzoic acid 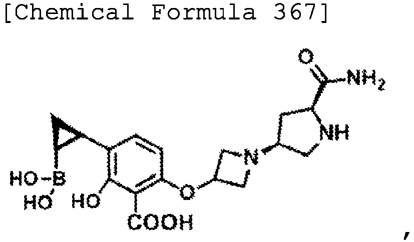
3-[(1R,2S)-2-boronocyclopropyl]-6-({1-[(3R,5S)-5-carbamoylpyrrolidin-3-yl]azetidin-3-yl}oxy)-2-hydroxybenzoic acid 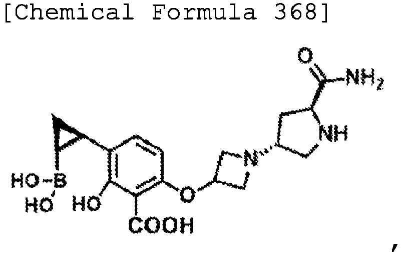
3-[(1R,2S)-2-boronocyclopropyl]-6-({1-[(3S,5R)-5-carbamoylpyrrolidin-3-yl]azetidin-3-yl}oxy)-2-hydroxybenzoic acid 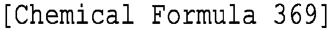 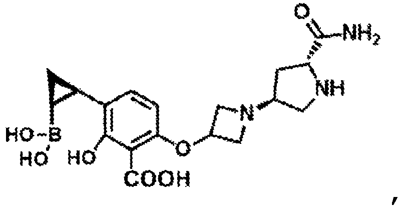
3-[(1R,2S)-2-boronocyclopropyl]-6-({1-[(3R,5R)-5-carbamoylpyrrolidin-3-yl]azetidin-3-yl}oxy)-2-hydroxybenzoic acid 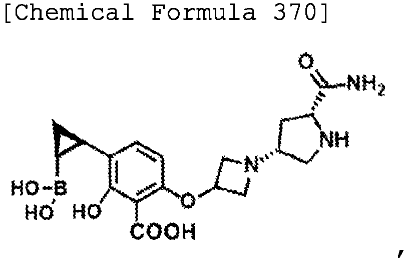
3-[(1S,2R)-2-boronocyclopropyl]-6-({1-[(3S,5S)-5-carbamoylpyrrolidin-3-yl]azetidin-3-yl}oxy)-2-hydroxybenzoic acid 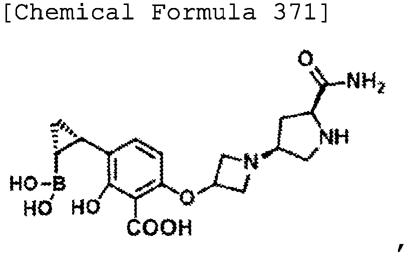
3-[(1S,2R)-2-boronocyclopropyl]-6-({1-[(3R,5S)-5-carbamoylpyrrolidin-3-yl]azetidin-3-yl}oxy)-2-hydroxybenzoic acid 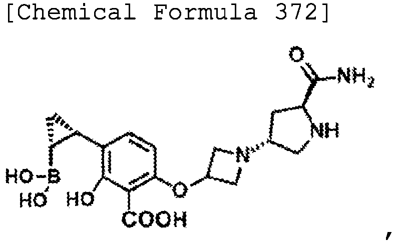
3-[(1S,2R)-2-boronocyclopropyl]-6-({1-[(3S,5R)-5-carbamoylpyrrolidin-3-yl]azetidin-3-yl}oxy)-2-hydroxybenzoic acid 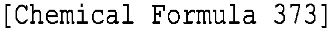 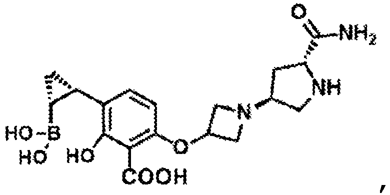
3-[(1S,2R)-2-boronocyclopropyl]-6-({1-[(3R,5R)-5-carbamoylpyrrolidin-3-yl]azetidin-3-yl}oxy)-2-hydroxybenzoic acid 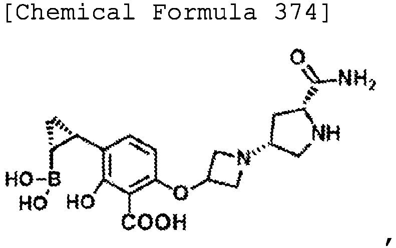
6-{[1-(2-amino-2-oxoethyl)azetidin-3-yl]oxy}-3-[2-boronocyclopropyl]-2-hydroxybenzoic acid 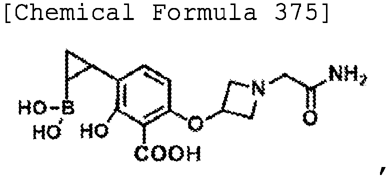
6-{[1-(2-amino-2-oxoethyl)azetidin-3-yl]oxy}-3-[(1R,2S)-2-boronocyclopropyl]-2-hydroxybenzoic acid 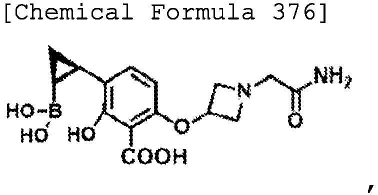
6-{[1-(2-amino-2-oxoethyl)azetidin-3-yl]oxy}-3-[(1S,2R)-2-boronocyclopropyl]-2-hydroxybenzoic acid 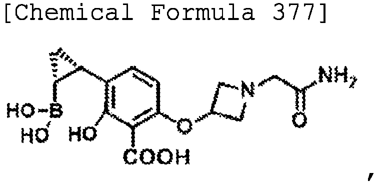
3-[(2-boronocyclopropyl]-2-hydroxy-6-[(1-{2-[(2-hydroxyethoxy)amino]-2-oxoethyl}azetidin-3-yl)oxy]benzoic acid 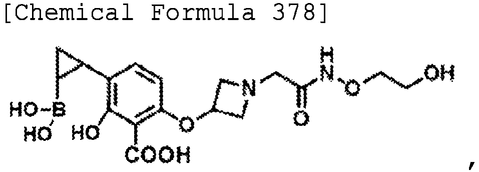
3-[(1R,2S)-2-boronocyclopropyl]-2-hydroxy-6-[(1-{2-[(2-hydroxyethoxy)amino]-2-oxoethyl}azetidin-3-yl)oxy]benzoic acid 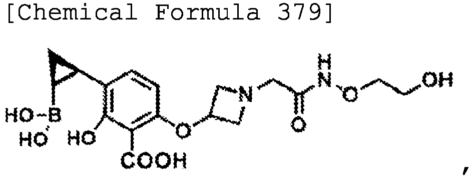
3-[(1S,2R)-2-boronocyclopropyl]-2-hydroxy-6-[(1-{2-[(2-hydroxyethoxy)amino]-2-oxoethyl}azetidin-3-yl)oxy]benzoic acid 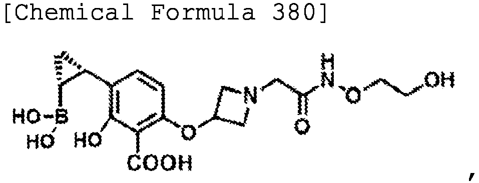
6-({1-[2-amino-3-hydroxy-2-(hydroxymethyl)propyl]azetidin-3-yl}oxy)-3-[2-boronocyclopropyl]-2-hydroxybenzoic acid 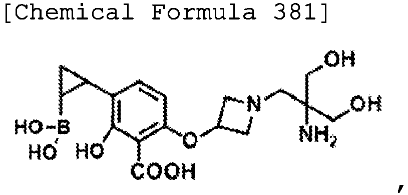
6-({1-[2-amino-3-hydroxy-2-(hydroxymethyl)propyl]azetidin-3-yl}oxy)-3-[(1R,2S)-2-boronocyclopropyl]-2-hydroxybenzoic acid 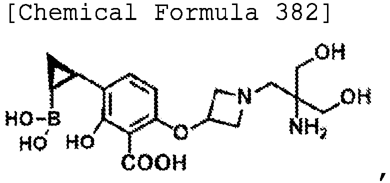
6-({1-[2-amino-3-hydroxy-2-(hydroxymethyl)propyl]azetidin-3-yl}oxy)-3-[(1S,2R)-2-boronocyclopropyl]-2-hydroxybenzoic acid 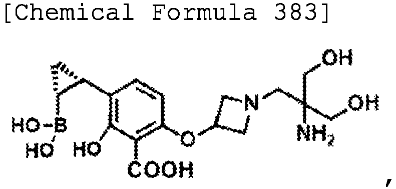
6-({1-[(1-aminocyclopropyl)methyl]azetidin-3-yl}oxy)-3-[2-boronocyclopropyl]-2-hydroxybenzoic acid 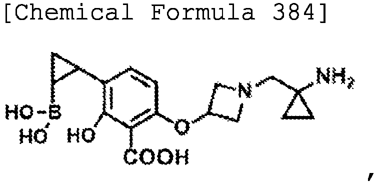
6-({1-[(1-aminocyclopropyl)methyl]azetidin-3-yl}oxy)-3-[(1R,2S)-2-boronocyclopropyl]-2-hydroxybenzoic acid 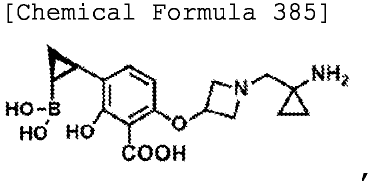
6-({1-[(1-aminocyclopropyl)methyl]azetidin-3-yl}oxy)-3-[(1S,2R)-2-boronocyclopropyl]-2-hydroxybenzoic acid 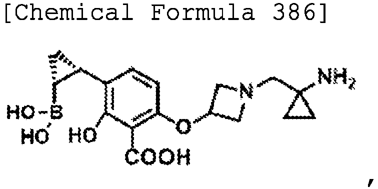
3-[2-boronocyclopropyl]-2-hydroxy-6-({1-[(1H-1,2,4-triazol-3-yl)methyl]azetidin-3-yl}oxy)benzoic acid 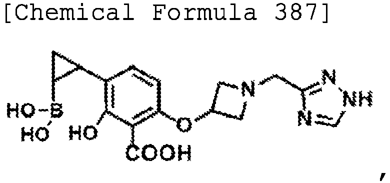
3-[(1R,2S)-2-boronocyclopropyl]-2-hydroxy-6-({1-[(1H-1,2,4-triazol-3-yl)methyl]azetidin-3-yl}oxy)benzoic acid 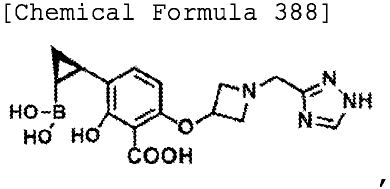
3-[(1S,2R)-2-boronocyclopropyl]-2-hydroxy-6-({1-[(1H-1,2,4-triazol-3-yl)methyl]azetidin-3-yl}oxy)benzoic acid 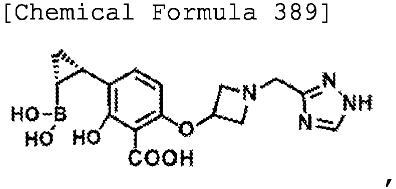
6-{[1-({4-[(2-aminoethyl)amino]cyclohexyl}acetyl)azetidin-3-yl]oxy}-3-[2-boronocyclopropyl]-2-hydroxybenzoic acid 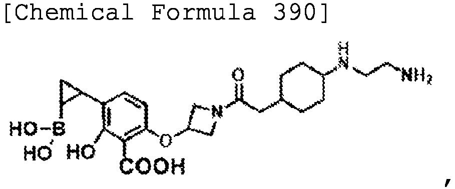
6-{[1-({(1r,4r)-4-[(2-aminoethyl)amino]cyclohexyl}acetyl)azetidin-3-yl]oxy}-3-[(1R,2S)-2-boronocyclopropyl]-2-hydroxybenzoic acid 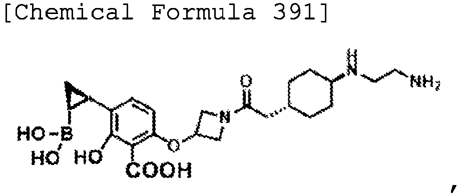
6-{[1-({(1s,4r)-4-[(2-aminoethyl)amino]cyclohexyl}acetyl)azetidin-3-yl]oxy}-3-[(1R,2S)-2-boronocyclopropyl]-2-hydroxybenzoic acid 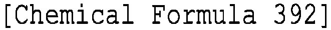 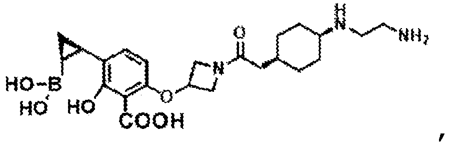
6-{[1-({(1r,4s)-4-[(2-aminoethyl)amino]cyclohexyl}acetyl)azetidin-3-yl]oxy}-3-[(1R,2S)-2-boronocyclopropyl]-2-hydroxybenzoic acid 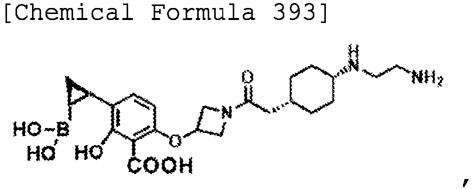
6-{[1-({(1s,4s)-4-[(2-aminoethyl)amino]cyclohexyl}acetyl)azetidin-3-yl]oxy}-3-[(1R,2S)-2-boronocyclopropyl]-2-hydroxybenzoic acid 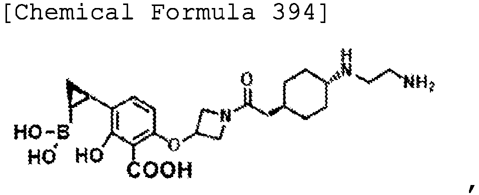
6-{[1-({(1r,4r)-4-[(2-aminoethyl)amino]cyclohexyl}acetyl)azetidin-3-yl]oxy}-3-[(1S,2R)-2-boronocyclopropyl]-2-hydroxybenzoic acid 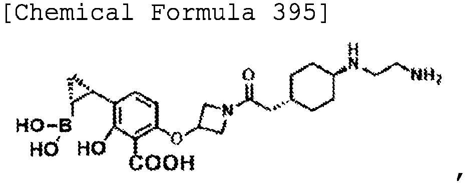
6-{[1-({(1s,4r)-4-[(2-aminoethyl)amino]cyclohexyl}acetyl)azetidin-3-yl]oxy}-3-[(1S,2R)-2-boronocyclopropyl]-2-hydroxybenzoic acid 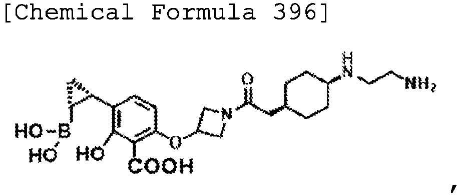
6-{[1-({(1r,4s)-4-[(2-aminoethyl)amino]cyclohexyl}acetyl)azetidin-3-yl]oxy}-3-[(1S,2R)-2-boronocyclopropyl]-2-hydroxybenzoic acid 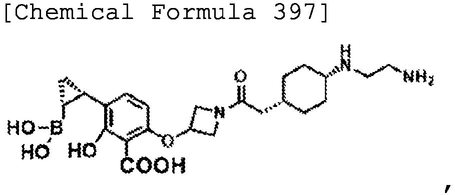
6-{[1-({(1s,4s)-4-[(2-aminoethyl)amino]cyclohexyl}acetyl)azetidin-3-yl]oxy}-3-[(1S,2R)-2-boronocyclopropyl]-2-hydroxybenzoic acid 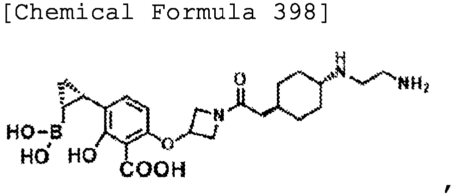
6-[(1-{2-amino-3-[(2-aminoethyl)amino]-2-methyl-3-oxopropyl}azetidin-3-yl)oxy]-3-[2-boronocyclopropyl]-2-hydroxybenzoic acid 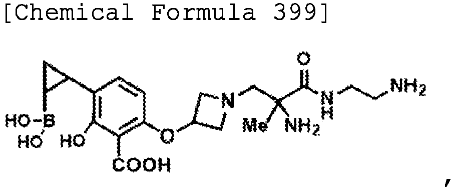
6-[(1-{(2R)-2-amino-3-[(2-aminoethyl)amino]-2-methyl-3-oxopropyl}azetidin-3-yl)oxy]-3-[(1R,2S)-2-boronocyclopropyl]-2-hydroxybenzoic acid 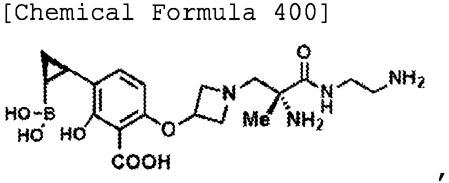
6-[(1-{(2S)-2-amino-3-[(2-aminoethyl)amino]-2-methyl-3-oxopropyl}azetidin-3-yl)oxy]-3-[(1R,2S)-2-boronocyclopropyl]-2-hydroxybenzoic acid 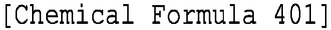 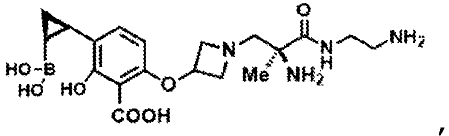
6-[(1-{(2R)-2-amino-3-[(2-aminoethyl)amino]-2-methyl-3-oxopropyl}azetidin-3-yl)oxy]-3-[(1S,2R)-2-boronocyclopropyl]-2-hydroxybenzoic acid 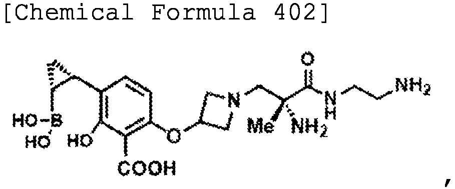
6-[(1-{(2S)-2-amino-3-[(2-aminoethyl)amino]-2-methyl-3-oxopropyl}azetidin-3-yl)oxy]-3-[(1S,2R)-2-boronocyclopropyl]-2-hydroxybenzoic acid 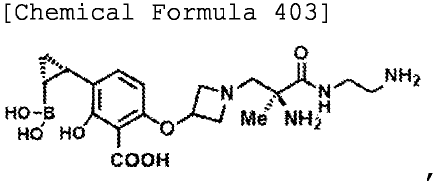
3-(2-boronocyclopropyl)-6-[(1-histidylazetidin-3-yl)oxy]-2-hydroxybenzoic acid 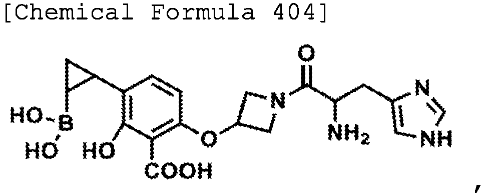
3-[(1R,2S)-2-boronocyclopropyl]-6-[(1-D-histidylazetidin-3-yl)oxy]-2-hydroxybenzoic acid 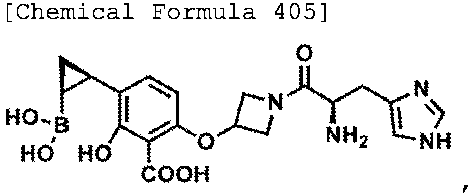
3-[(1R,2S)-2-boronocyclopropyl]-6-[(1-L-histidylazetidin-3-yl)oxy]-2-hydroxybenzoic acid 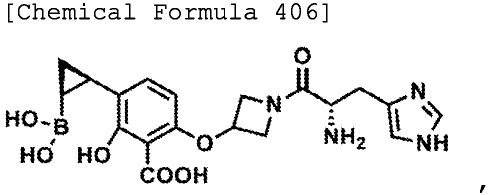
3-[(1S,2R)-2-boronocyclopropyl]-6-[(1-D-histidylazetidin-3-yl)oxy]-2-hydroxybenzoic acid 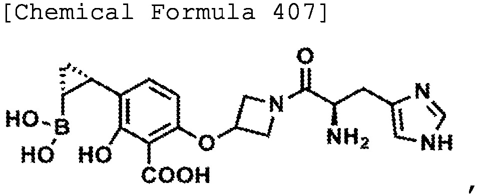
3-[(1S,2R)-2-boronocyclopropyl]-6-[(1-L-histidylazetidin-3-yl)oxy]-2-hydroxybenzoic acid 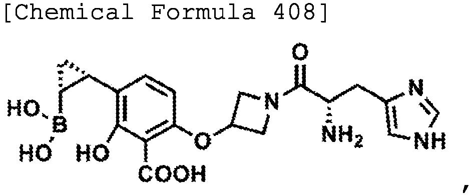
6-{[1-(2-aminoethyl)azetidin-3-yl]oxy}-3-(2-boronocyclopropyl)-2-hydroxybenzoic acid 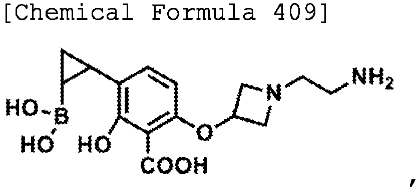
6-{[1-(2-aminoethyl)azetidin-3-yl]oxy}-3-[(1R,2S)-2-boronocyclopropyl]-2-hydroxybenzoic acid 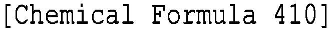 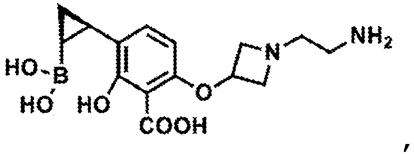
6-{[1-(2-aminoethyl)azetidin-3-yl]oxy}-3-[(1S,2R)-2-boronocyclopropyl]-2-hydroxybenzoic acid 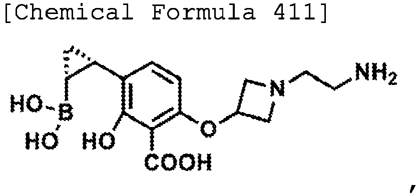
6-[(1-{[5-(aminomethyl)morpholin-2-yl]acetyl}azetidin-3-yl)oxy]-3-(2-boronocyclopropyl)-2-hydroxybenzoic acid 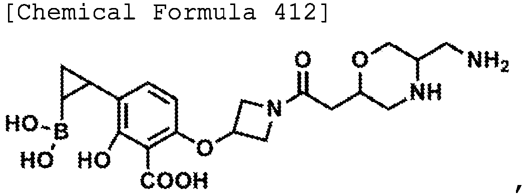
6-[(1-{[5-(aminomethyl)morpholin-2-yl]acetyl}azetidin-3-yl)oxy]-3-[(1R,2S)-2-boronocyclopropyl]-2-hydroxybenzoic acid 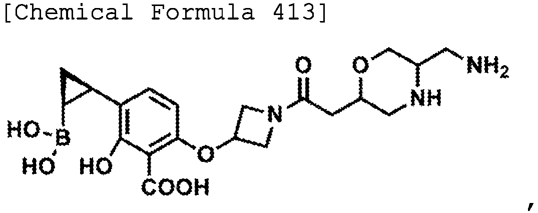
6-[(1-{[5-(aminomethyl)morpholin-2-yl]acetyl}azetidin-3-yl)oxy]-3-[(1S,2R)-2-boronocyclopropyl]-2-hydroxybenzoic acid 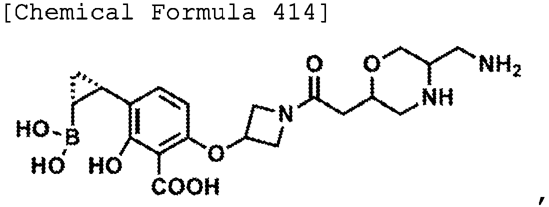
6-({1-[2-amino-2-methyl-3-{[2-(methylamino)-2-oxoethyl]amino}-3-oxopropyl]azetidin-3-yl}oxy)-3-[2-boronocyclopropyl]-2-hydroxybenzoic acid 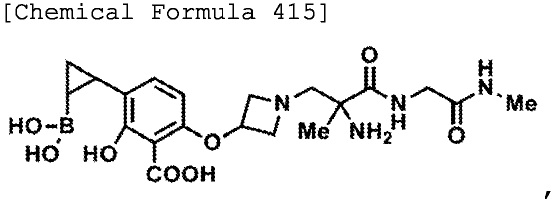
6-({1-[(2R)-2-amino-2-methyl-3-{[2-(methylamino)-2-oxoethyl]amino}-3-oxopropyl]azetidin-3-yl}oxy)-3-[(1R,2S)-2-boronocyclopropyl]-2-hydroxybenzoic acid 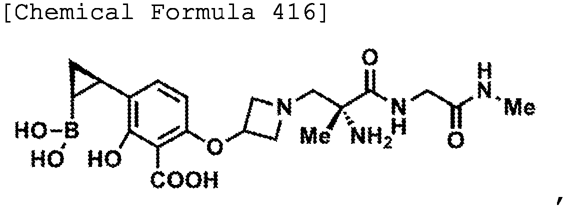
6-({1-[(2S)-2-amino-2-methyl-3-{[2-(methylamino)-2-oxoethyl]amino}-3-oxopropyl]azetidin-3-yl}oxy)-3-[(1R,2S)-2-boronocyclopropyl]-2-hydroxybenzoic acid 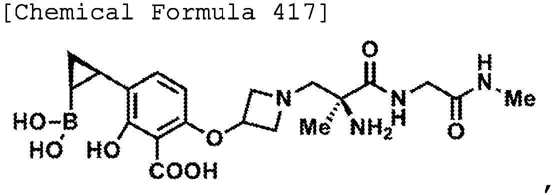
6-({1-[(2R)-2-amino-2-methyl-3-{[2-(methylamino)-2-oxoethyl]amino}-3-oxopropyl]azetidin-3-yl}oxy)-3-[(1S,2R)-2-boronocyclopropyl]-2-hydroxybenzoic acid 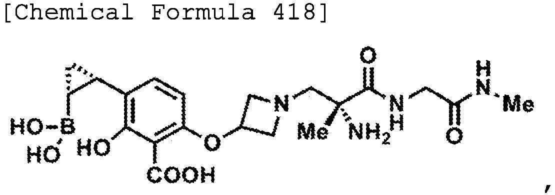
6-({1-[(2S)-2-amino-2-methyl-3-{[2-(methylamino)-2-oxoethyl]amino}-3-oxopropyl]azetidin-3-yl}oxy)-3-[(1S,2R)-2-boronocyclopropyl]-2-hydroxybenzoic acid 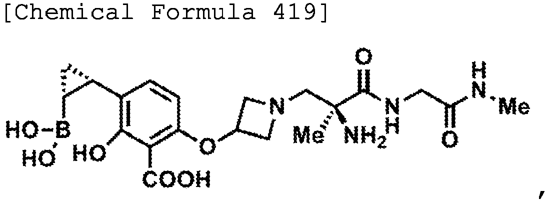
6-({1-[2-amino-3-{[2-(dimethylamino)-2-oxoethyl]amino}-2-methyl-3-oxopropyl]azetidin-3-yl}oxy)-3-[2-boronocyclopropyl]-2-hydroxybenzoic acid 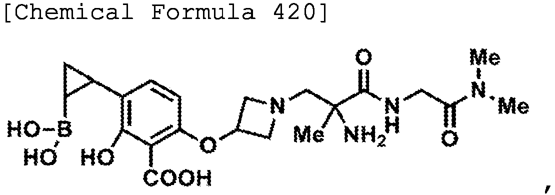
6-({1-[(2R)-2-amino-3-{[2-(dimethylamino)-2-oxoethyl]amino}-2-methyl-3-oxopropyl]azetidin-3-yl}oxy)-3-[(1R,2S)-2-boronocyclopropyl]-2-hydroxybenzoic acid 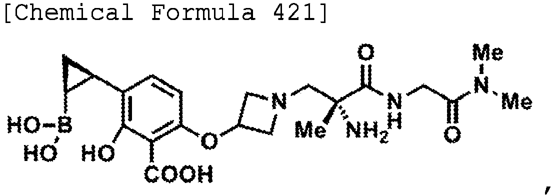
6-({1-[(2S)-2-amino-3-{[2-(dimethylamino)-2-oxoethyl]amino}-2-methyl-3-oxopropyl]azetidin-3-yl}oxy)-3-[(1R,2S)-2-boronocyclopropyl]-2-hydroxybenzoic acid 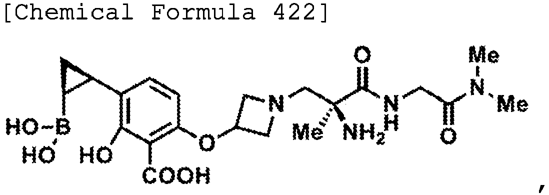
6-({1-[(2R)-2-amino-3-{[2-(dimethylamino)-2-oxoethyl]amino}-2-methyl-3-oxopropyl]azetidin-3-yl}oxy)-3-[(1S,2R)-2-boronocyclopropyl]-2-hydroxybenzoic acid 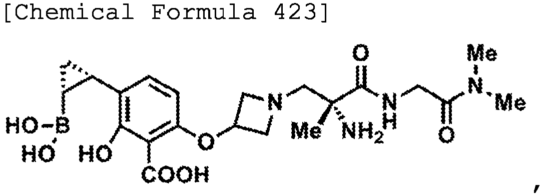
6-({1-[(2S)-2-amino-3-{[2-(dimethylamino)-2-oxoethyl]amino}-2-methyl-3-oxopropyl]azetidin-3-yl}oxy)-3-[(1S,2R)-2-boronocyclopropyl]-2-hydroxybenzoic acid 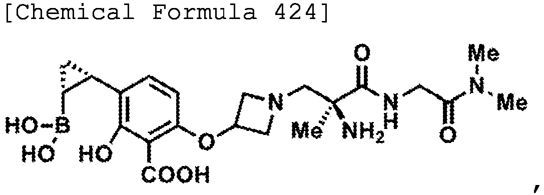
3-[2-boronocyclopropyl]-2-hydroxy-6-[(1-{[4-methylmorpholin-2-yl]acetyl}azetidin-3-yl)oxy]benzoic acid 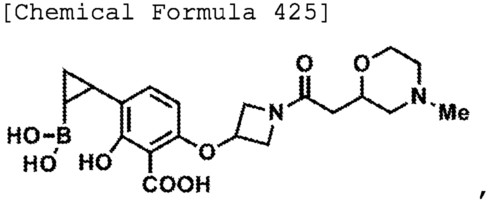
3-[(1R,2S)-2-boronocyclopropyl]-2-hydroxy-6-[(1-{[(2R)-4-methylmorpholin-2-yl]acetyl}azetidin-3-yl)oxy]benzoic acid 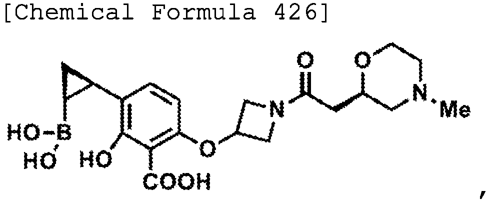
3-[(1R,2S)-2-boronocyclopropyl]-2-hydroxy-6-[(1-{[(2S)-4-methylmorpholin-2-yl]acetyl}azetidin-3-yl)oxy]benzoic acid 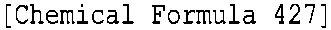 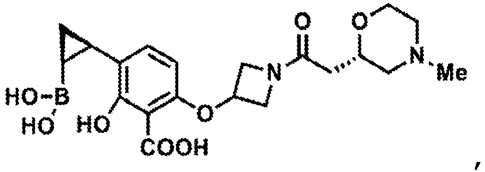
3-[(1S,2R)-2-boronocyclopropyl]-2-hydroxy-6-[(1-{[(2R)-4-methylmorpholin-2-yl]acetyl}azetidin-3-yl)oxy]benzoic acid 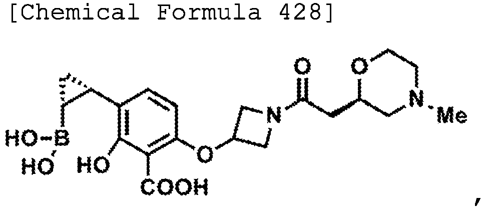
3-[(1S,2R)-2-boronocyclopropyl]-2-hydroxy-6-[(1-{[(2S)-4-methylmorpholin-2-yl]acetyl}azetidin-3-yl)oxy]benzoic acid 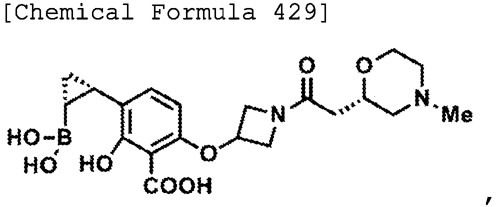
3-[2-boronocyclopropyl]-6-[(1-{[4,4-dimethylmorpholin-4-ium-2-yl]acetyl}azetidin-3-yl)oxy]-2-hydroxybenzoate 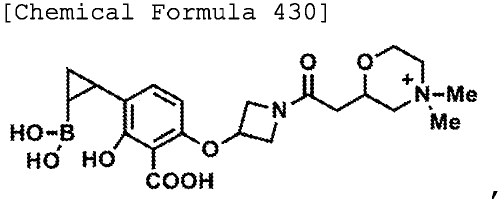
3-[(1R,2S)-2-boronocyclopropyl]-6-[(1-{[(2R)-4,4-dimethylmorpholin-4-ium-2-yl]acetyl}azetidin-3-yl)oxy]-2-hydroxybenzoate 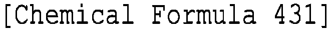 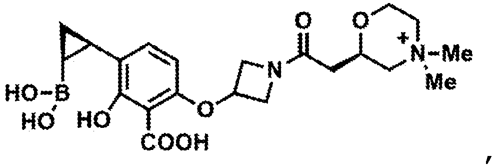
3-[(1R,2S)-2-boronocyclopropyl]-6-[(1-{[(2S)-4,4-dimethylmorpholin-4-ium-2-yl]acetyl}azetidin-3-yl)oxy]-2-hydroxybenzoate 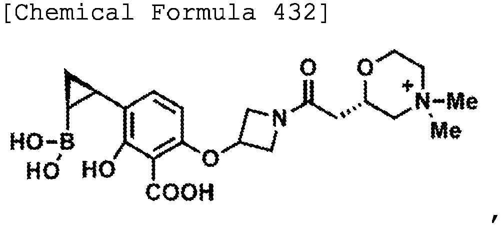
3-[(1S,2R)-2-boronocyclopropyl]-6-[(1-{[(2R)-4,4-dimethylmorpholin-4-ium-2-yl]acetyl}azetidin-3-yl)oxy]-2-hydroxybenzoate 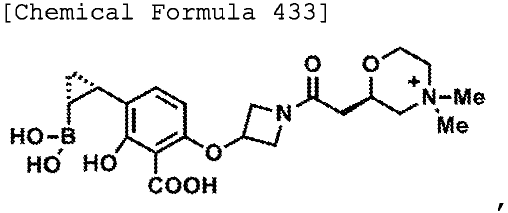
3-[(1S,2R)-2-boronocyclopropyl]-6-[(1-{[(2S)-4,4-dimethylmorpholin-4-ium-2-yl]acetyl}azetidin-3-yl)oxy]-2-hydroxybenzoate 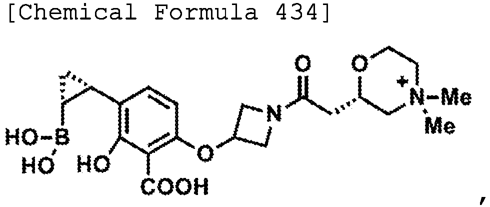
3-(2-boronocyclopropyl)-2-hydroxy-6-({1-[(3-hydroxy-2-methyl-4-oxopyridin-1(4H)-yl)acetyl]azetidin-3-yl}oxy)benzoic acid 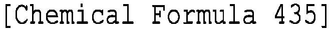 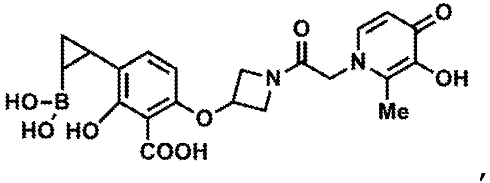
3-[(1S,2R)-2-boronocyclopropyl]-2-hydroxy-6-({1-[(3-hydroxy-2-methyl-4-oxopyridin-1(4H)-yl)acetyl]azetidin-3-yl}oxy)benzoic acid 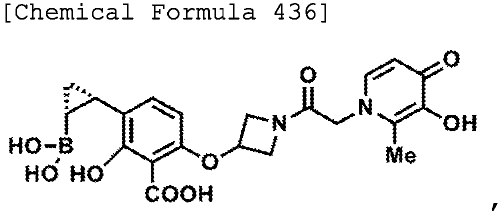 and
3-[(1R,2S)-2-boronocyclopropyl]-2-hydroxy-6-({1-[(3-hydroxy-2-methyl-4-oxopyridin-1(4H)-yl)acetyl]azetidin-3-yl}oxy)benzoic acid

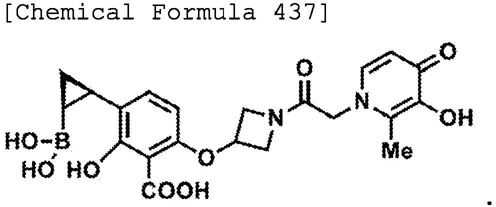

### [Item 119]

A medicament comprising the compound or the pharmaceutically acceptable salt thereof according to any one of items 1 to item 118.

### [Item 120]

The medicament according to item 119, which is a therapeutic drug or a prophylactic drug for a bacterial infection.

### [Item 121]

An agent for inhibiting β-lactamase, comprising the compound or the pharmaceutically acceptable salt thereof according to any one of items 1 to 118 as an active ingredient.

### [Item 122]

A pharmaceutical composition comprising the compound or the pharmaceutically acceptable salt thereof according to any one of items 1 to 118 and a pharmaceutically acceptable carrier.

### [Item 123]

The pharmaceutical composition according to item 122, further comprising an additional agent.

### [Item 124]

The pharmaceutical composition according to item 123, wherein the additional agent is selected from the group consisting of an antibacterial agent, an antifungal agent, an antiviral agent, an anti-inflammatory agent, and an anti-allergic agent.

### [Item 125]

The pharmaceutical composition according to item 123 or 124, wherein the additional agent is a β-lactam agent.

### [Item 126]

The pharmaceutical composition according to item 124 or 125, wherein a β-lactam agent, which is the additional agent, is selected from the group consisting of amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442.

### [Item 127]

The pharmaceutical composition according to item 125 or 126, wherein the β-lactam agent is selected from ceftazidime, biapenem, doripenem, ertapenem, imipenem, meropenem, or panipenem.

### [Item 128]

The pharmaceutical composition according to item 125 or 126, wherein the β-lactam agent is selected from aztreonam, tigemonam, BAL30072, SYN2416, or carumonam.

### [Item 129]

The pharmaceutical composition according to item 122, characterized in that an additional agent is concomitantly administered.

### [Item 130]

The pharmaceutical composition according to item 129, wherein the additional agent is selected from an antibacterial agent, an antifungal agent, an antiviral agent, an anti-inflammatory agent, or an anti-allergic agent.

### [Item 131]

The pharmaceutical composition according to item 129 or 130, wherein the additional agent is a β-lactam agent.

### [Item 132]

The pharmaceutical composition according to item 130 or 131, wherein a β-lactam agent, which is the additional agent, is selected from the group consisting of amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442.

### [Item 133]

The pharmaceutical composition according to item 131 or 132, wherein the β-lactam agent is selected from the group consisting of ceftazidime, biapenem, doripenem, ertapenem, imipenem, meropenem, and panipenem.

### [Item 134]

The pharmaceutical composition according to item 131 or 132, wherein the β-lactam agent is selected from the group consisting of aztreonam, tigemonam, BAL30072, SYN2416, and carumonam.

### [Item 135]

The compound or the pharmaceutically acceptable salt thereof according to any one of items 1 to 118 for use in treating a bacterial infection.

### [Item 136]

The compound or the pharmaceutically acceptable salt thereof according to item 135, wherein the bacterial infection is a bacterial infection in which a bacteria that can have a β-lactamase is involved.

### [Item 137]

The compound or the pharmaceutically acceptable salt thereof according to item 135 or 136, wherein the bacterial infection is sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

### [Item 138]

A medicament comprised of a combination of the compound or the pharmaceutically acceptable salt thereof according to any one of items 1 to 118 and at least one agent selected from the group consisting of therapeutic agents for sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, and an odontogenic infection.

### [Item 139]

A pharmaceutical composition comprising a β-lactam agent, wherein the pharmaceutical composition is administered with the compound or the pharmaceutically acceptable salt thereof according to any one of items 1 to 118.

### [Item 140]

A method for treating a bacterial infection, characterized in that a therapeutically effective amount of the compound or the pharmaceutically acceptable salt thereof according to any one of items 1 to 118 is administered to a patient in need thereof.

### [Item 141]

The method according to item 140, wherein the bacterial infection is a bacterial infection in which a bacteria that can have a β-lactamase is involved.

### [Item 142]

The method according to item 140 or 141, wherein the bacterial infection is sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

### [Item 143]

The method according to any one of items 140 to 142, characterized in that an additional agent is concomitantly administered.

Specifically, the present invention is also the following.

### [Item B1]

A compound represented by formula (1a) or (1b): 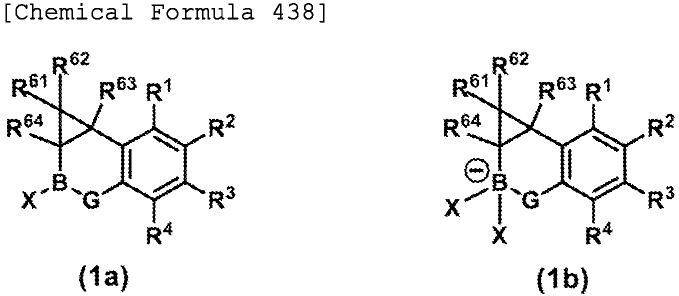 or a pharmaceutically acceptable salt thereof, wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 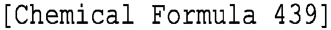 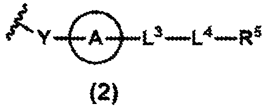 wherein
Y is an oxygen atom, a sulfur atom, or -NR^{j}-, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, -S(=O)-, or -S(=O)₂-,
L⁴ is a single bond, an optionally substituted hydrocarbylene group, or -C(=N-OR^{h1})-,
R^{h1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
R⁵ is a hydrogen atom, a hydroxyl group, an optionally substituted hydrocarbyl group, or an optionally substituted heterohydrocarbyl group,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
   1) -C (=O) R⁸,
   2) -SO₂-L⁶-R⁸,
      (wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene group),
   3) -NR^{a4}R^{b3},
   4) -B (OR^{m1})₂,
   5) -PO(OR^{m1})(OR^{m2}),
   6) optionally substituted heteroaryl,
   7) an optionally substituted non-aryl heterocycle, or
   8) a bioisostere of one of 1) to 7),
      (wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

### [Item B2]

The compound or the pharmaceutically acceptable salt thereof according to item B1, represented by formula (1a) or (1b): 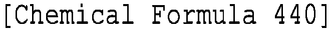 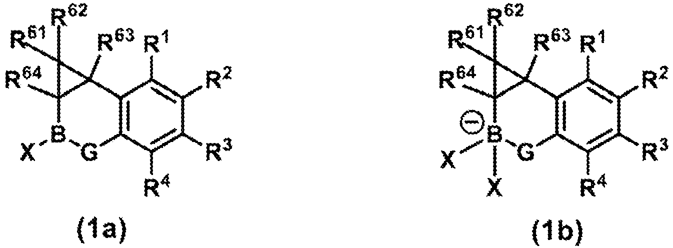 wherein
G is an oxygen atom, a sulfur atom, or -NR^{a1}-,
X is a hydroxyl group, an optionally substituted C₁₋₆ alkoxy group, or -NR^{a2}R^{b1},
R^{a1}, R^{a2}, and R^{b1} are the same or different, each independently
   1) a hydrogen atom,
   2) a C₁₋₆ alkyl group,
   3) a C₃₋₁₀ alicyclic group,
   4) C₆₋₁₀ aryl
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group, or
   15) -OR^{c1},
      (wherein each substituent from 2) to 14) is optionally substituted),
wherein R^{a2} and R^{b1} together may form an optionally substituted 4- to 10-membered nitrogen-containing non-aryl heterocycle,
R^{c1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
L¹ is a single bond, an oxygen atom, a sulfur atom, -SO-, - SO₂-, -NR^{d1}-, -NR^{d1}C(=O) -, or -NR^{d1}SO₂-,
L² is a single bond or an optionally substituted C₁₋₆ alkylene group,
Z is
   1) a hydrogen atom,
   2) a hydroxyl group,
   3) a cyano group,
   4) a carboxyl group,
   5) a C₃₋₁₀ alicyclic group,
   6) C₆₋₁₀ aryl,
   7) 5- or 6-membered heteroaryl,
   8) a 4- to 10-membered non-aryl heterocycle,
   9) a C₁₋₆ alkoxy group,
   10) a C₃₋₁₀ alicyclic oxy group,
   11) a C₆₋₁₀ aryloxy group,
   12) a 5- or 6-membered heteroaryloxy group,
   13) a 4- to 10-membered non-aryl heterocyclyl oxy group,
   14) a C₁₋₆ alkylthio group,
   15) a C₃₋₁₀ alicyclic thio group,
   16) a C₆₋₁₀ arylthio group,
   17) a 5- or 6-membered heteroarylthio group,
   18) a 4- to 10-membered non-aryl heterocyclyl thio group,
      (wherein each substituent from 5) to 18) is optionally substituted),
   19) -SO₂-NR^{e1}R^{f1},
   20) -NR^{e1}-C(=O) OR^{f1},
   21) -NR^{g1}-C(=O) NR^{e1}R^{f1},
   22) -NR^{e1}-C(=S)R^{f1},
   23) -NR^{e1}-C(=S)OR^{f1},
   24) -NR^{g1}-C(=S)NR^{e1}R^{f1},
   25) -NR^{g1}-CR^{e1}(=NR^{f1}),
   26) -NR^{g1}-CR^{e1}(=N-OR^{f1}),
   27) -NR^{h1}-C(=NR^{g1})NR^{e1}R^{f1},
   28) -NR^{h1}-C(=N-OR^{g1})NR^{e1}R^{f1},
   29) -NRⁱ¹-C(=NR^{h1})NR^{g1}-NR^{e1}R^{f1},
   30) -NRⁱ¹-C(=N-OR^{h1})NR^{g1}-NR^{e1}R^{f1},
   31) -NR^{e1}-SO₂-R^{f1},
   32) -NR^{g1}-SO₂-NR^{e1}R^{f1},
   33) -C(=O)OR^{e1},
   34) -C(=S)OR^{e1},
   35) -C(=S)NR^{e1}R^{f1},
   36) -C(=S)NR^{e1}OR^{f1},
   37) -C (=S)NR^{g1}-NR^{e1}R^{f1},
   38) -C(=NR^{e1})R^{f1},
   39) -C(=N-OR^{e1})R^{f1},
   40) -C(=NR^{h1})NR^{g1}-NR^{e1}R^{f1},
   41) -C(=N-OR^{h1})NR^{g1}-NR^{e1}R^{f1},
   42) -NR^{e1}R^{f1},
   43) -NR^{g1}-NR^{e1}R^{f1},
   44) -NR^{e1}OR^{f1},
   45) -NR^{e1}-C(=O) R^{f1},
   46) -C(=O) NR^{e1}R^{f1},
   47) -C(=O)NR^{e1}OR^{f1},
   48) -C(=O)NR^{g1}-NR^{e1}R^{f1},
   49) -C(=O) R^{e1},
   50) -C(=NR^{g1})NR^{e1}R^{f1}, or
   51) -C(=N-OR^{h1})NR^{e1}R^{f1},
one of R¹, R², and R³ is a group represented by formula (2) : 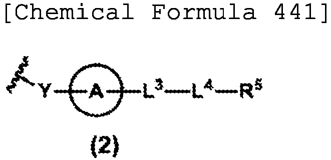 wherein
Y is an oxygen atom, a sulfur atom, or -NR^{j}-,
ring A is an optionally substituted 4- to 20-membered non-aryl heterocycle,
L³ is
   1) an oxygen atom,
   2) a sulfur atom,
   3) -NR^{d2}-,
   4) -NR^{d2}C(=O) -,
   5) -NR^{d2}SO₂-,
   6) a C₁₋₆ alkylene group,
   7) a C₃₋₁₀ cycloalkylene group,
   8) a 4- to 10-membered non-aryl heterocyclylene group, (wherein each substituent from 6) to 8) is optionally substituted),
   9) -C(=O)-,
   10) -S(=O)-, or
   11) -S(=O)₂-,
L⁴ is
   1) a single bond,
   2) a C₁₋₆ alkylene group,
   3) a C₃₋₁₀ cycloalkylene group,
   4) a C₆₋₁₀ arylene group,
   5) a 5- or 6-membered heteroarylene group,
   6) a 4- to 10-membered non-aryl heterocyclylene group, (wherein each substituent from 2) to 6) is optionally substituted), or
   7) -C(=N-OR^{h1})-,
R⁵ is
   1) a hydrogen atom,
   2) a C₁₋₆ alkyl group,
   3) a C₃₋₁₀ alicyclic group,
   4) a 4- to 10-membered non-aryl heterocycle,
   5) C₆₋₁₀ aryl,
   6) 5- to 10-membered heteroaryl,
   7) a C₁₋₆ alkylthio group,
      (wherein each substituent from 2) to 7) is optionally substituted, and if two substituents further substituted with the substituent of 1), 2), or 3) are each substituted on adjacent atoms within a ring, the two substituents together may further form a condensed ring structure),
   8) -NR^{e1}OH,
   9) a carboxyl group (-C(=O)OH),
   10) a carboxylic acid isostere (wherein the carboxylic acid isostere comprises an ester group-C(=O)OR^{20a}),
   11) a sulfo group (sulfonic acid group),
   12) -SO₂R^{e1},
   13) -SO₂-NR^{e1}R^{f1},
   14) -S(=O) (=NR^{f1})R^{e1},
   15) -NR^{e1}-C(=O)R^{f1},
   16) -NR^{e1}-C(=O)OR^{f1},
   17) -NR^{g1}-C(=O) NR^{e1}R^{f1},
   18) -NR^{e1}-SO₂-R^{f1},
   19) -NR^{g1}-SO₂-NR^{e1}R^{f1},
   20) -N=S(=O)R^{e1}R^{f1},
   21) -C(=O)NR⁵⁰R⁵¹, or
   22) -NR^{e1}R^{f1} (wherein if R⁵ is the substituent of 22), -L³-L⁴-R⁵ is not - (CH₂)₁₋₄NR^{e1}R^{f1} (wherein R^{e1} and R^{f1} are a hydrogen atom, optionally substituted C₁₋₄ alkyl, an optionally substituted C₃₋₇ alicyclic group, optionally substituted 4- to 10-membered non-aryl heterocyclic group, optionally substituted C₆₋₁₀ aryl, or optionally substituted 5- to 10-membered heteroaryl)),
R20a is
   1) a C₁₋₆ alkyl group,
   2) a C₃₋₁₀ alicyclic group,
   3) C₆₋₁₀ aryl,
   4) 5- or 6-membered heteroaryl, or
   5) a 4- to 10-membered non-aryl heterocycle
      (wherein each substituent from 1) to 5) is optionally substituted),
R⁵⁰ represents
   1) a hydrogen atom,
   2) a C₁₋₆ alkyl group,
   3) a hydroxyl group,
   4) a C₁₋₆ alkoxy group,
   5) a C₃₋₆ cycloalkoxy group,
   6) a C₃₋₆ alicyclic group,
   7) a 4- to 6-membered non-aryl heterocycle,
   8) C₆₋₁₀ aryl,
   9) 5- to 10-membered heteroaryl
   10) a 4- to 6-membered non-aryl heterocyclyl oxy,
   11) C₆₋₁₀ aryloxy,
   12) 5- to 10-membered heteroaryloxy,
   13) a C₁₋₆ alkylsulfonyl group,
   14) a C₃₋₆ cycloalkoxysulfonyl group, or
   15) a 4- to 6-membered non-aryl heterocyclyl sulfonyl group
      (wherein each substituent of 2) and 4) to 15) is optionally substituted),
R⁵¹ represents
   1) a hydrogen atom,
   2) a C₁₋₆ alkyl group,
   3) a C₃₋₆ alicyclic group,
   4) a 4- to 6-membered non-aryl heterocycle,
   5) C₆₋₁₀ aryl, or
   6) 5- to 10-membered heteroaryl,
      (wherein each substituent from 2) to 6) is optionally substituted), or
R⁵⁰ and R⁵¹ together may form an optionally substituted 4- to 7-membered nitrogen-containing non-aryl heterocycle,
the remaining two (without the structure of formula (2) among R¹, R², and R³) are the same or different, each independently a hydrogen atom, a halogen atom, an optionally substituted C₁₋₆ alkyl group, an optionally substituted C₁₋₆ alkoxy group, an optionally substituted C₁₋₆ alkylthio group, optionally substituted 5- or 6-membered heteroaryl, or -NR^{a3}R^{b2},
R^{d1}, R^{d2}, R^{e1}, R^{f1}, R^{g1}, R^{h1}, Rⁱ¹, and R^{j} are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
a combination of R^{e1} and R^{f1}, when attached to the same nitrogen atom, together may form an optionally substituted 4- to 10-membered nitrogen-containing non-aryl heterocycle,
R⁴ is
   1) -C (=O) R⁸,
   2) -SO₂-L⁶-R⁸,
      (wherein R⁸ in 1) and 2) is -NR^{a5}R^{b4}, -NR^{a5}-L⁷-B (OR^{m1})₂, -OR^{m1}, or an optionally substituted C₁₋₆ alkyl group, and L⁶ is a single bond or -NR^{a6}-),
   3) -NR^{a4}R^{b3},
   4) -B(OR^{m1})₂,
   5) -PO(OR^{m1})(OR^{m2}),
   6) optionally substituted 5-membered heteroaryl,
   7) an optionally substituted 5-membered non-aryl heterocycle, or
   8) a bioisostere of one of 1) to 7),
      (wherein the formulas of 2), 4), 5), and 6) include a carboxylic acid isostere, and 8) may include them in duplicates),
R^{a3}, R^{a4}, R^{a5}, R^{a6}, R^{b2}, R^{b3}, and R^{b4} are the same or different, each independently having the same definition as R^{a1}, R^{a2}, and R^{b1}, wherein a combination of R^{a3} and R^{b2}, R^{a4} and R^{b3}, or R^{a5} and R^{b4}, when attached to the same nitrogen atom, together may form an optionally substituted 4- to 10-membered nitrogen-containing non-aryl heterocycle,
R^{m1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
wherein if R^{m1} is attached to a boron atom via an oxygen atom, two R^{m1}, as C₂₋₄ alkylene, together with the boron atom and two oxygen atoms, may form a 5- to 7-membered non-aryl heterocycle (wherein an alkylene moiety is optionally substituted in the non-aryl heterocycle),
R^{m2} is a hydrogen atom, an optionally substituted C₁₋₆ alkyl group, or an optionally substituted C₃₋₁₀ alicyclic group,
L⁷ is an optionally substituted C₁₋₃ alkylene group,
R⁶¹, R⁶², and R⁶³ are each independently a hydrogen atom, halogen, or an optionally substituted C₁₋₆ alkyl group, and
R⁶⁴ is a hydrogen atom, halogen, an optionally substituted C₁₋₆ alkyl group, or -L¹-L²-Z.

### [Item B3]

The compound or the pharmaceutically acceptable salt thereof according to item B1 or B2, wherein
L³ is
   1) a C₁₋₆ alkylene group,
   2) a C₃₋₁₀ cycloalkylene group, or
   3) a 4- to 10-membered non-aryl heterocyclylene group,
      (wherein each substituent from 1) to 3) is optionally substituted), and
L⁴ is a single bond or an optionally substituted C₁₋₅ alkylene group.

### [Item B4]

The compound or the pharmaceutically acceptable salt thereof according to any one of items B1 to B3, wherein
L³ is an optionally substituted C₁₋₄ alkylene group, and
L⁴ is a single bond or an optionally substituted C₁₋₃ alkylene group.

### [Item B5]

The compound or the pharmaceutically acceptable salt thereof according to any one of items B1 to B4, wherein
L³ is -(CR³⁰R³¹)ₙ₁-,
R³⁰ and R³¹, each independently, or if there are multiple instances of each of them, all of them independently represent
   1) a hydrogen atom,
   2) -NR^{a7}R^{a8},
   3) a C₁₋₄ alkyl group,
   4) C₆₋₁₀ aryl,
   5) 5- to 10-membered heteroaryl,
   6) a C₃₋₆ alicyclic group,
   7) a 4- to 10-membered non-aryl heterocycle,
      (wherein each substituent from 3) to 7) is optionally substituted), or
   8) -OR^{c2}, or
R³⁰ and R³¹, together with the carbon atom to which they are attached, form a C₃₋₆ alicyclic group or a 4- to 6-membered non-aryl heterocycle,
R^{a7} and R^{a8} are the same or different, each independently
   1) a hydrogen atom,
   2) a C₁₋₆ alkyl group,
   3) a C₃₋₁₀ alicyclic group,
   4) C₆₋₁₀ aryl
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group, or
   15) -OR^{c3},
      (wherein each substituent from 2) to 14) is optionally substituted),
wherein R^{a7} and R^{a8} together may form an optionally substituted 4- to 10-membered nitrogen-containing non-aryl heterocycle,
R^{c2} and R^{c3} are each independently,
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
n1 is an integer 1, 2, 3, or 4,
L⁴ is -(CR⁴⁰R⁴¹)ₙ₂-,
R⁴⁰ and R⁴¹, each independently, or if there are multiple instances of each of them, all of them independently represent
   1) a hydrogen atom,
   2) -NR^{a9}R^{a10},
   3) a C₁₋₄ alkyl group,
   4) C₆₋₁₀ aryl,
   5) 5- to 10-membered heteroaryl,
   6) a C₃₋₆ alicyclic group,
   7) a 4- to 10-membered non-aryl heterocycle,
      (wherein each substituent from 3) to 7) is optionally substituted), or
   8) -OR^{c4}, or
R⁴⁰ and R⁴¹, together with the carbon atom to which they are attached, form a C₃₋₆ alicyclic group or a 4- to 6-membered non-aryl heterocycle,
R^{a9} and R^{a10} are the same or different, each independently
   1) a hydrogen atom,
   2) a C₁₋₆ alkyl group,
   3) a C₃₋₁₀ alicyclic group,
   4) C₆₋₁₀ aryl
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group,
      (wherein each substituent from 2) to 14) is optionally substituted), or
   15) -OR^{c5},
wherein R^{a9} and R^{a10} together may form an optionally substituted 4- to 10-membered nitrogen-containing non-aryl heterocycle,
R^{c4} and R^{c5} are each independently defined the same as R^{c2} and R^{c3}, and
n2 is an integer 0 (i.e., when L⁴ is a single bond), 1, 2, or 3.

### [Item B6]

The compound or the pharmaceutically acceptable salt thereof according to any one of items B1 to B5, wherein -L³-L⁴- is an optionally substituted C₁₋₂ alkylene group.

### [Item B7]

The compound or the pharmaceutically acceptable salt thereof according to any one of items B1 to B6, wherein -L³-L⁴- is a C₁₋₂ alkylene group optionally substituted with a C₁₋₃ alkyl group, an amino group, or a hydroxymethyl group, or a plurality of the same or different groups thereamong (wherein two C₁₋₃ alkyl groups, when attached to the same carbon atom, together with the carbon atom to which they are attached, may form a C₃₋₆ alicyclic group).

### [Item B8]

The compound or the pharmaceutically acceptable salt thereof according to any one of items B1 to B7, wherein
R⁵ is
   1) a C₃₋₁₀ alicyclic group,
   2) C₆₋₁₀ aryl,
   3) 5- to 10-membered heteroaryl,
   4) a C₁₋₆ alkylthio group,
      (wherein each substituent from 1) to 4) is optionally substituted, and if two substituents further substituted with the substituent of 2) or 3) are each substituted on adjacent atoms within a ring, the two substituents together may further form a condensed ring structure),
   5) -NR^{e1}OH,
   6) -C(=O)NR⁵⁰R⁵¹,
   7) -SO₂-NR^{e1}R^{f1},
   8) -NR^{e1}-SO₂-R^{f1},
   9) -C(=O) OR²⁰, or
   10) -NR^{e1}R^{f1} (wherein if R⁵ is the substituent of 10), L³ and/or L⁴ is a C₁₋₆ alkylene group that is necessarily substituted with one or more groups other than a hydrogen atom (wherein L³ or L⁴, together with said substituent, may form a C₃₋₁₀ alicyclic group or a 4- to 10-membered non-aryl heterocycle)), and
R²⁰ is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted).

### [Item B9]

The compound or the pharmaceutically acceptable salt thereof according to any one of items B1 to B8, wherein
R⁵ is
1) C₆₋₁₀ aryl,
2) 5- to 10-membered heteroaryl,
3) -C(=O)NR⁵⁰R⁵¹,
4) -C(=O) OR²⁰, or
5) -NR^{e1}R^{f1} (wherein if R⁵ is the substituent of 5), L³ and/or L⁴ is a C₁₋₆ alkylene group that is necessarily substituted with one or more groups other than a hydrogen atom, and together with said substituent forms at least one C₃₋₁₀ alicyclic group or 4- to 10-membered non-aryl heterocycle),
   (wherein each substituent from 1) to 2) is optionally substituted, and any two groups thereof, when each is attached to adjacent atoms within a ring, together may further form a condensed ring structure).

### [Item B10]

The compound or the pharmaceutically acceptable salt thereof according to any one of items B1 to B9, wherein Z-L²-L¹ is an optionally substituted C₁₋₆ alkylthio group.

### [Item B11]

The compound or the pharmaceutically acceptable salt thereof according to any one of items B1 to B10, wherein R⁶¹, R⁶², R⁶³, and R⁶⁴ are each independently a hydrogen atom, a fluorine atom, or a C₁₋₃ alkyl group optionally substituted with a fluorine atom.

### [Item B12]

The compound or the pharmaceutically acceptable salt thereof according to any one of items B1 to B11, wherein R⁶¹, R⁶², R⁶³, and R⁶⁴ are each independently a hydrogen atom or a fluorine atom.

### [Item B13]

The compound or the pharmaceutically acceptable salt thereof according to any one of items B1 to B12, wherein G is an oxygen atom.

### [Item B14]

The compound or the pharmaceutically acceptable salt thereof according to any one of items B1 to B13, wherein X is a hydroxyl group or an optionally substituted C₁₋₆ alkoxy group.

### [Item B15]

The compound or the pharmaceutically acceptable salt thereof according to any one of items B1 to B14, wherein X is a hydroxyl group.

### [Item B16]

The compound or the pharmaceutically acceptable salt thereof according to any one of items B1 to B15, wherein the compounds of formulas (1a) and (1b) are represented by formulas (3a) and (3b), respectively: 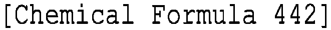 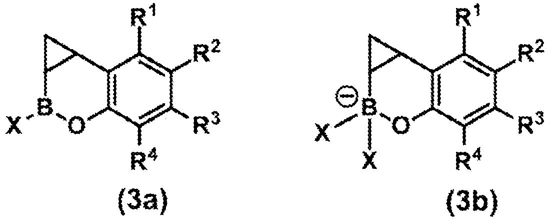 wherein X, R¹, R², and R³ are defined the same as any one of items B1 to B15, and
R⁴ is selected from the group consisting of
1) -C(=O)OR^{m1} (wherein R^{m1} is a hydrogen atom, a C₁₋₆ alkyl group, a C₃₋₁₀ alicyclic group, C₆₋₁₀ aryl, 5- or 6-membered heteroaryl, or a 4- to 10-membered non-aryl heterocycle, wherein the C₁₋₆ alkyl group, the C₃₋₁₀ alicyclic group, the C₆₋₁₀ aryl, the 5- or 6-membered heteroaryl, and the 4- to 10-membered non-aryl heterocycle are each optionally substituted), and
2) a bioisostere of 1).

### [Item B17]

The compound or the pharmaceutically acceptable salt thereof according to any one of items B1 to B16, wherein R⁴ is 1) -C(=O)OH (i.e., a carboxyl group), or 2) a carboxylic acid isostere.

### [Item B18]

The compound or the pharmaceutically acceptable salt thereof according to any one of items B1 to B17, wherein the compounds of formulas (1a) and (1b) or the compounds of formulas (3a) and (3b) are represented by formulas (4a) and (4b), respectively: 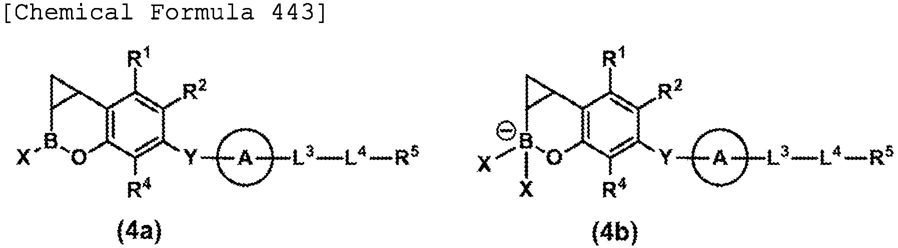 wherein X, R⁴, Y, ring A, L³, L⁴, and R⁵ are defined the same as any one of items B1 to B17, and
R¹ and R² are the same or different, each independently a hydrogen atom, a halogen atom, a C₁₋₆ alkyl group, a C₁₋₆ alkoxy group, or a C₁₋₆ alkylthio group (wherein the C₁₋₆ alkyl group, the C₁₋₆ alkoxy group, and the C₁₋₆ alkylthio group are optionally substituted).

### [Item B19]

The compound or the pharmaceutically acceptable salt thereof according to any one of items B1 to B18, wherein ring A is an optionally substituted 4- to 10-membered non-aryl heterocycle.

### [Item B20]

The compound or the pharmaceutically acceptable salt thereof according to any one of items B1 to B19, wherein ring A is an optionally substituted 4- to 7-membered non-aryl heterocycle.

### [Item B21]

The compound or the pharmaceutically acceptable salt thereof according to any one of items B1 to B20, wherein Y is an oxygen atom or a sulfur atom.

### [Item B22]

The compound or the pharmaceutically acceptable salt thereof according to any one of items B1 to B21, wherein Y is an oxygen atom.

### [Item B23]

The compound or the pharmaceutically acceptable salt thereof according to any one of items B1 to B22, wherein the compounds of formulas (1a) and (1b) or the compounds of formulas (3a) and (3b) or the compounds of formulas (4a) and (4b) are represented by formulas (5a) and (5b), respectively: 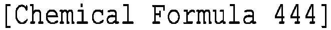 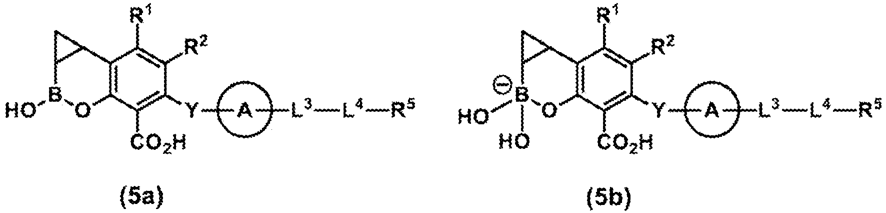 wherein R¹, R², Y, L³, L⁴, and R⁵ are defined the same as any one of items B1 to B22, and ring A is an optionally substituted 4- to 6-membered nitrogen-containing non-aryl heterocycle.

### [Item B24]

The compound or the pharmaceutically acceptable salt thereof according to any one of items B1 to B2 and 10 to 23, wherein L³ is -C(=O)- or -S(=O)₂-.

### [Item B25]

The compound or the pharmaceutically acceptable salt thereof according to any one of items B1 to B2 and B10 to B24, wherein L³ is -C(=O)-.

### [Item B26]

The compound or the pharmaceutically acceptable salt thereof according to any one of items B1 to B2 and B10 to B25, wherein L⁴ is a single bond, -C(=N-OR^{h1})-, or an optionally substituted C₁₋₆ alkylene group, wherein R^{h1} is an optionally substituted C₁₋₆ alkyl group.

### [Item B27]

The compound or the pharmaceutically acceptable salt thereof according to any one of items B1 to B26, wherein R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
   (wherein each substituent from 3) to 5) is optionally substituted).

### [Item B28]

The compound or the pharmaceutically acceptable salt thereof according to any one of items B1 to B27, wherein R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
   (wherein each substituent from 3) to 5) is optionally substituted with a halogen atom).

### [Item B29]

The compound or the pharmaceutically acceptable salt thereof according to any one of items B1 to B28, wherein R¹ and R² are the same or different, each independently selected from the group consisting of
1) a hydrogen atom,
2) a halogen atom, and
3) a C₁₋₆ alkyl group optionally substituted with a halogen atom.

### [Item B30]

The compound or the pharmaceutically acceptable salt thereof according to any one of items B1 to B29, wherein R¹ and R² are the same or different, each independently selected from the group consisting of
1) a hydrogen atom,
2) a fluorine atom, and
3) a C₁₋₃ alkyl group optionally substituted with a fluorine atom.

### [Item B31]

The compound or the pharmaceutically acceptable salt thereof according to any one of items B1 to B30, wherein R¹ and R² are both hydrogen atoms.

### [Item B32]

The compound or the pharmaceutically acceptable salt thereof according to any one of items B1 to B31, wherein the compounds of formulas (1a) and (1b) or the compounds of formulas (3a) and (3b) or the compounds of formulas (4a) and (4b) or the compounds of formulas (5a) and (5b) are represented by formulas (6a) and (6b), respectively: 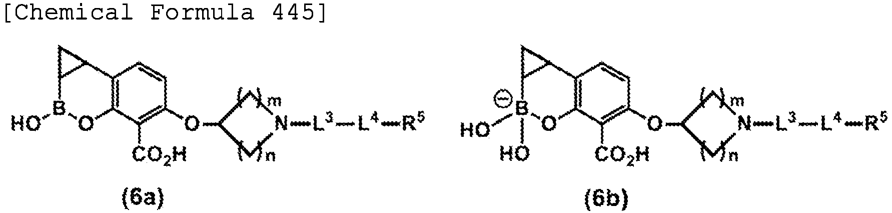 wherein L³, L⁴, and R⁵ are defined the same as any one of items B1 to B31,
m is an integer 1, 2, or 3,
n is an integer 1, 2, or 3, and
m + n is 2, 3, or 4.

### [Item B33]

The compound or the pharmaceutically acceptable salt thereof according to item B32, wherein the compounds of formulas (6a) and (6b) are enantiomers represented by formulas (7a) and (7b): 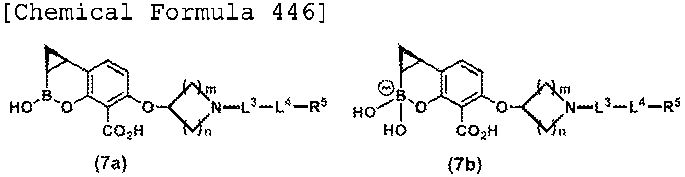 or formulas (8a) and (8b): 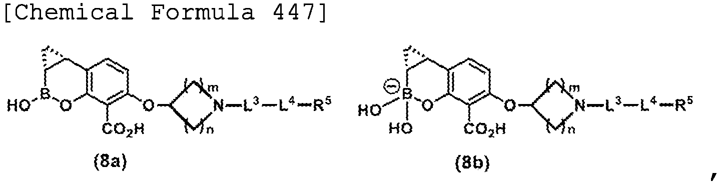 respectively, wherein L³, L⁴, and R⁵ are defined the same as any one of items B1 to B32, and
m and n are defined the same as item B32.

### [Item B34]

The compound or the pharmaceutically acceptable salt thereof according to any one of items B1 to B33, wherein m is 1 or 2, n is 1 or 2, and m + n is 2 or 3.

### [Item B35]

The compound or the pharmaceutically acceptable salt thereof according to any one of items B1 to B34, wherein m is 1, and n is 1.

### [Item B36]

The compound or the pharmaceutically acceptable salt thereof according to any one of items B1 to B5, B8 to B23, and B27 to B35, wherein
L³ is -(CR³⁰R³¹)ₙ₁-,
R³⁰ and R³¹ each independently, or if there are multiple instances of each of them, all of them independently represent
   1) a hydrogen atom,
   2) an optionally substituted C₁₋₄ alkyl group, or
   3) optionally substituted C₆₋₁₀ aryl,
n1 is 1, 2, or 3,
L⁴ is -(CR⁴⁰R⁴¹)ₙ₂-,
R⁴⁰ and R⁴¹ each independently, or if there are multiple instances of each of them, all of them independently represent
   1) a hydrogen atom,
   2) -NR^{a9}R^{a10},
   3) an optionally substituted C₁₋₄ alkyl group, or
   4) -OR^{c4} or,
R⁴⁰ and R⁴¹, together with the carbon atom to which they are attached, form a C₃₋₆ alicyclic group or a 4- to 6-membered non-aryl heterocycle,
R^{a9}, R^{a10}, and R^{c4} are the same or different, each independently
   1) a hydrogen atom,
   2) an optionally substituted C₁₋₆ alkyl group, or
   3) an optionally substituted C₃₋₁₀ alicyclic group,
wherein R^{a9} and R^{a10} together may form an optionally substituted 4- to 10-membered nitrogen-containing non-aryl heterocycle, and
n2 is 0, 1, or 2.

### [Item B37]

The compound or the pharmaceutically acceptable salt thereof according to item B36, wherein n1 is 1 or 2, and n2 is 0 or 1.

### [Item B38]

The compound or the pharmaceutically acceptable salt thereof according to any one of items B1 to B5, B8 to B23, and B27 to B37, wherein
L³ is -CR³⁰R³¹-,
R³⁰ and R³¹ each independently represent
   1) a hydrogen atom,
   2) a C₁₋₄ alkyl group (wherein the group is optionally substituted with halogen, -NR^{a11}R^{a12}, or -OR^{c6}), or
   3) C₆ aryl (wherein the group is optionally substituted with halogen, -NR^{a13}R^{a14}, -OR^{c7}, or a C₁₋₃ alkyl group (wherein the group is optionally substituted with halogen, -NR^{a15}R^{a16}, or -OR^{c8})),
L⁴ is -(CR⁴⁰R⁴¹)ₙ₂-,
R⁴⁰ and R⁴¹ each independently represent
   1) a hydrogen atom,
   2) -NR^{a9}R^{a10},
   3) a C₁₋₄ alkyl group (wherein the group is optionally substituted with halogen, -NR^{a17}R^{a18}, or -OR^{c9}), or
   4) -OR^{c4}, or
R⁴⁰ and R⁴¹, together with the carbon atom to which they are attached, form a C₃₋₆ alicyclic group,
R^{a9}, R^{a10}, R^{a11}, R^{a12}, R^{a13}, R^{a14}, R^{a15}, R^{a16}, R^{a17}, R^{a18}, R^{c4}, R^{c6}, R^{c7}, R^{c8}, and R^{c9} are the same or different, each independently representing
   1) a hydrogen atom, or
   2) a C₁₋₄ alkyl group (wherein the group is optionally substituted with halogen, -NR^{a19}R^{a20}, or -OR^{c10}),
R^{a19}, R^{a20}, and R^{c10} are the same or different, each independently representing
   1) a hydrogen atom, or
   2) a C₁₋₄ alkyl group (wherein the group is optionally substituted with halogen),
wherein each combination of R^{a9} and R^{a10}, R^{a11} and R^{a12}, R^{a13} and R^{a14}, R^{a15} and R^{a16}, R^{a17} and R^{a18}, or R^{a19} and R^{a20} together may form an optionally substituted 4- to 7-membered nitrogen-containing non-aryl heterocycle, and
n2 is 0 or 1.

### [Item B39]

The compound or the pharmaceutically acceptable salt thereof according to any one of items B1 to B5, B8 to B23, and B27 to B38, wherein
L³ is -CH₂-, -CH(CH₂NH₂)-, or -CH(CH₂OH)-, and
L⁴ is a single bond, -CH₂-, -CH(NH₂)-, -CMe(NH₂)-, -CEt(NH₂)-, -C(iso-Pr) (NH₂)-, -CH(CH₂NH₂)-, -CH(OH)-, -CH(CH₂OH)-, or

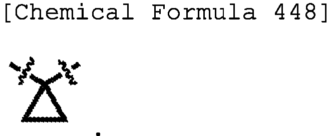

### [Item B40]

The compound or the pharmaceutically acceptable salt thereof according to any one of items B1 to B23 and B27 to B39, wherein R⁵ is
1) C₆₋₁₀ aryl, or
2) 5- to 10-membered heteroaryl,
   (wherein each substituent from 1) to 2) is optionally substituted, and if two substituents further substituted with the substituent of 1) or 2) are each substituted on adjacent atoms within a ring, the two substituents together may further form a condensed ring structure).

### [Item B41]

The compound or the pharmaceutically acceptable salt thereof according to any one of items B1 to B23 and B27 to B40, wherein
R⁵ is C₆ aryl (i.e., phenyl) or 5-membered, 6-membered, 9-membered, or 10-membered heteroaryl,
each group of R⁵ is optionally substituted with each of R^{6a} or R^{6b} at all chemically substitutable positions on a carbon atom or a nitrogen atom within a ring thereof,
wherein R^{6a}, which are substituents on the carbon atom, if there are multiple instances on the same ring, are all independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e2}R^{f2},
   13) -NR^{g2}-CR^{e2}(=NR^{f2}),
   14) -NR^{g2}-CR^{e2}(=N-OR^{f2}),
   15) -NR^{h2}-C(=NR^{g2})NR^{e2}R^{f2},
   16) -NR^{h2}-C(=N-OR^{g2})NR^{e2}R^{f2},
   17) -NRⁱ²-C(=NR^{h2})NR^{g2}-NR^{e2}R^{f2},
   18) -NRⁱ²-C(=N-OR^{h2})NR^{g2}-NR^{e2}R^{f2},
   19) -C (=NR^{e2})R^{f2},
   20) -C(=N-OR^{e2})R^{f2},
   21) -C (=NR^{h2})-NR^{e2}R^{f2},
   22) -C (=NR^{h2})NR^{g2}-NR^{e2}R^{f2},
   23) -C(=N-OR^{h2})NR^{g2}-NR^{e2}R^{f2},
   24) -NR^{e2}R^{f2},
   25) -NR^{g2}-NR^{e2}R^{f2},
   26) -NR^{e2}OR^{f2},
   27) -NR^{e2}-C(=O) R^{f2},
   28) -C (=O) NR^{e2}R^{f2},
   29) -C(=O)NR^{e2}OR^{f2},
   30) -C(=O) NR^{g2}-NR^{e2}R^{f2},
   31) -C(=O) R^{e2},
   32) -C (=O) OR^{e2}, and
   33) -C (=N-OR^{h2})NR^{e2}R^{f2},
wherein R^{6b}, which are substituents on the nitrogen atom, if there are multiple instances on the same ring, are all independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C(=NR^{e2})R^{f2},
   6) -C(=N-OR^{e2})R^{f2},
   7) -SO₂-NR^{e2}R^{f2},
   8) -C(=NR^{h2})-NR^{e2}R^{f2},
   9) -C(=NR^{h2})NR^{g2}-NR^{e2}R^{f2},
   10) -C(=N-OR^{h2})NR^{g2}NR^{e2}R^{f2},
   11) -C (=O)NR^{e2}R^{f2},
   12) -C(=O)NR^{e2}OR^{f2},
   13) -C(=O) NR^{g2}-NR^{e2}R^{f2},
   14) -C(=O)R^{e2}, and
   15) -C(=N-OR^{h2})NR^{e2}R^{f2}, or
      if a combination of two R^{6a} or R^{6a} and R^{6b} are each substituted on adjacent atoms within a ring, the two substituents together may form an optionally substituted 5- to 6-membered heteroaryl ring or an optionally substituted 5- to 7-membered non-aryl heterocycle, which further fuses to an attachment moiety between the adjacent atoms within the ring,
R^{e2}, R^{f2}, R^{g2}, R^{h2}, and Rⁱ² are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted), and
a combination of R^{e2} and R^{f2}, when attached to the same nitrogen atom, together may form an optionally substituted 4- to 10-membered nitrogen-containing non-aryl heterocycle.

### [Item B42]

The compound or the pharmaceutically acceptable salt thereof according to any one of items B1 to B23 and B27 to B41, wherein
R⁵ is C₆ aryl or 5-membered, 6-membered, 9-membered, or 10-membered heteroaryl selected from the group consisting of 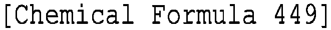 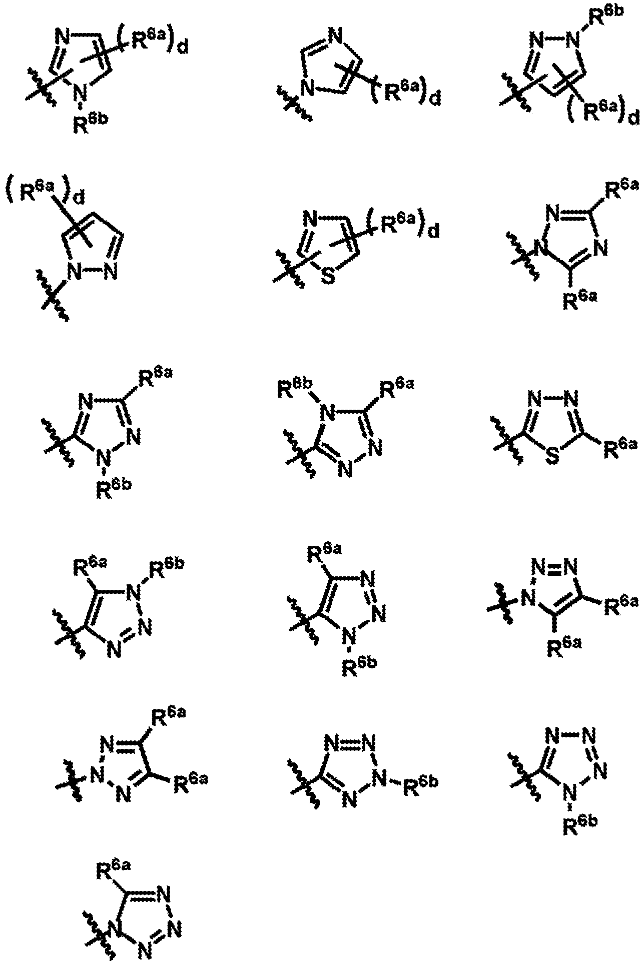 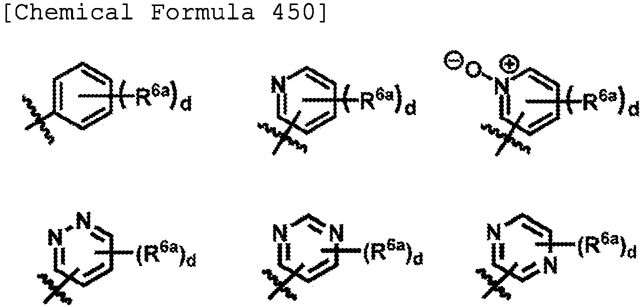 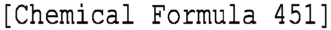 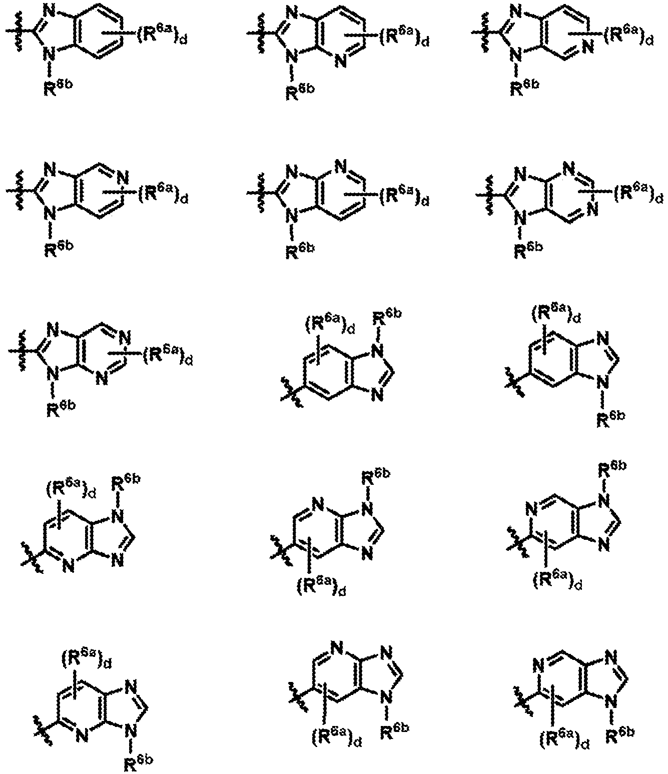 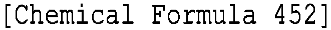 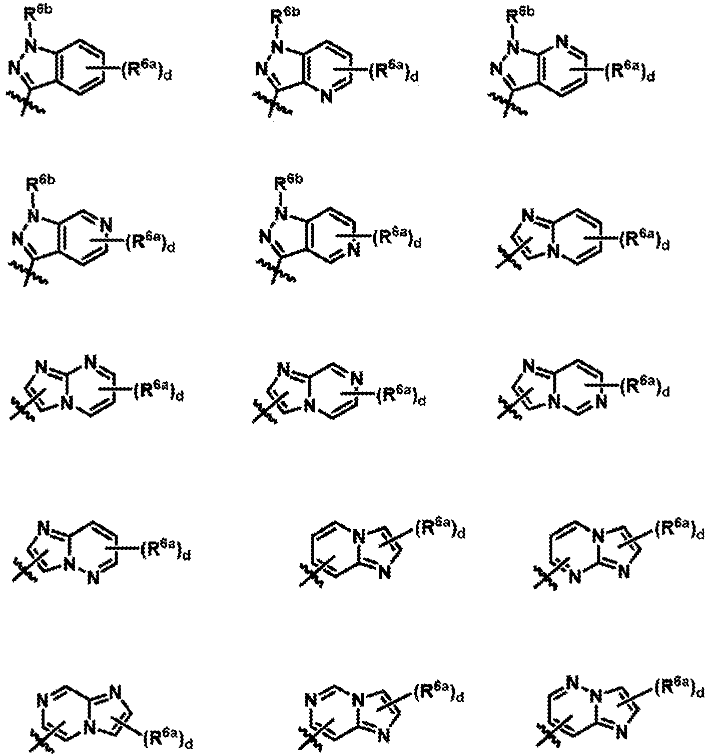 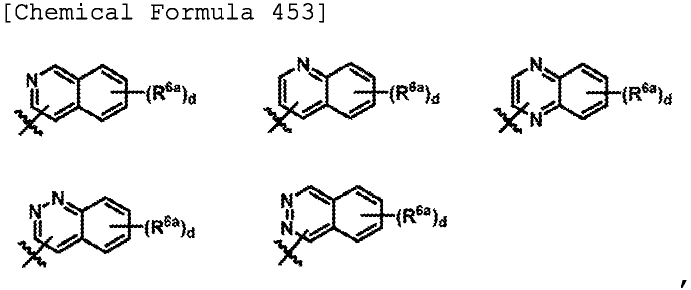
d is the number of chemically substitutable positions on a ring of each group by R^{6a}, and
each R^{6a} and each R^{6b} are defined the same as item B41.

### [Item B43]

The compound or the pharmaceutically acceptable salt thereof according to item B41 or B42, wherein
R^{6a}, if there are multiple instances on the same ring, are all independently selected from the group consisting of
   1) a hydrogen atom,
   2) a hydroxyl group,
   3) halogen,
   4) a C₁₋₄ alkyl group
      (wherein the alkyl group is optionally substituted with NR^{e2}R^{f2}, - C(=O)OR^{f2}, -C (=O) NR^{e2}R^{f2}, -C (=O) NR^{e2} (OR^{f2}), or a hydroxyl group),
   5) a C₁₋₄ alkoxy group,
   6) -NR^{e2}R^{f2},
   7) -C (=O) NR^{e2}R^{f2}, and
   8) -C (=O) OR^{e2}, and
each R^{6b}, if there are multiple instances on the same ring, are all independently selected from the group consisting of
   1) a hydrogen atom,
   2) a hydroxyl group, and
   3) a C₁₋₄ alkyl group
      (wherein the alkyl group is optionally substituted with NR^{e2}R^{f2}, - C(=O)OR^{f2}, -C (=O) NR^{e2}R^{f2}, -C (=O) NR^{e2} (OR^{f2}), or a hydroxyl group).

### [Item B44]

The compound or the pharmaceutically acceptable salt thereof according to any one of items B1 to B23 and B27 to B43, wherein R^{e2} and R^{f2} are the same or different, each independently a hydrogen atom, an optionally substituted C₁₋₆ alkyl group, or an optionally substituted C₃₋₁₀ alicyclic group.

### [Item B45]

The compound or the pharmaceutically acceptable salt thereof according to any one of items B1 to B23 and B27 to B44, wherein R^{e2} and R^{f2} are the same or different, each independently a hydrogen atom or an optionally substituted C₁₋₆ alkyl group.

### [Item B46]

The compound or the pharmaceutically acceptable salt thereof according to any one of items B1 to B23 and B27 to B45, wherein R^{e2} and R^{f2} are hydrogen atoms.

### [Item B47]

The compound or the pharmaceutically acceptable salt thereof according to any one of items B41 to B46, wherein R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

### [Item B48]

The compound or the pharmaceutically acceptable salt thereof according to any one of items B1 to B23 and B27 to B39, wherein R⁵ is C (=O)NR⁵⁰R⁵¹.

### [Item B49]

The compound or the pharmaceutically acceptable salt thereof according to any one of items B1 to B23, B27 to B39, and B48, wherein
R⁵⁰ represents
   1) a hydrogen atom,
   2) an optionally substituted C₁₋₄ alkyl group,
   3) a hydroxyl group,
   4) an optionally substituted C₁₋₄ alkoxy group, or
   5) an optionally substituted C₁₋₆ alkylsulfonyl group,
R⁵¹ represents
   1) a hydrogen atom, or
   2) an optionally substituted C₁₋₄ alkyl group, or
R⁵⁰ and R⁵¹ together may form a 4- to 6-membered nitrogen-containing non-aryl heterocycle.

### [Item B50]

The compound or the pharmaceutically acceptable salt thereof according to any one of items B1 to B23, B27 to B39, and B48 to B49, wherein
R⁵⁰ represents
   1) a hydrogen atom,
   2) a C₁₋₄ alkyl group (wherein the group is optionally substituted with halogen, a C₃₋₆ alicyclic group, -NR^{a21}R^{a22}, or -OR^{c11}),
   3) a hydroxyl group,
   4) a C₁₋₄ alkoxy group (wherein the group is optionally substituted with halogen, a C₃₋₆ alicyclic group, -NR^{a23}R^{a24}, or -OR^{c12}), or
   5) a C₁₋₄ alkylsulfonyl group (wherein the group is optionally substituted with halogen, a C₃₋₆ alicyclic group, -NR^{a21}R^{a22}, or - OR^{c11}),
R^{a21}, R^{a22}, R^{a23}, R^{a24}, R^{c11}, and R^{c12} are the same or different, each independently representing
   1) a hydrogen atom, or
   2) a C₁₋₄ alkyl group (wherein the group is optionally substituted with halogen),
wherein each combination of R^{a21} and R^{a22} or R^{a23} and R^{a24} together may form an optionally substituted 4- to 7-membered nitrogen-containing non-aryl heterocycle, and
R⁵¹ is a hydrogen atom or C₁₋₄ alkyl (wherein the group is optionally substituted with halogen).

### [Item B51]

The compound or the pharmaceutically acceptable salt thereof according to any one of items B1 to B23, B27 to B39, and B48 to B50, wherein
R⁵⁰ represents
   1) a hydrogen atom,
   2) a C₁₋₄ alkyl group (wherein the group is optionally substituted with a cyclopropyl group, -NH₂, -NHMe, or a -hydroxyl group),
   3) a hydroxyl group,
   4) a C₁₋₄ alkoxy group (wherein the group is optionally substituted with a cyclopropyl group, -NH₂, -NHMe, or a -hydroxyl group), or
   5) a C₁₋₄ alkylsulfonyl group, and
R⁵¹ is a hydrogen atom.

### [Item B52]

The compound or the pharmaceutically acceptable salt thereof according to any one of items B1 to B23 and B27 to B39, wherein R⁵ is -C(=O)OR²⁰.

### [Item B53]

The compound or the pharmaceutically acceptable salt thereof according to item B52, wherein R²⁰ is
1) a hydrogen atom, or
2) an optionally substituted C₁₋₄ alkyl group.

### [Item B54]

The compound or the pharmaceutically acceptable salt thereof according to item B53, wherein
L³ is -CH₂-, and
L⁴ is -CH(NH₂)- or -CMe(NH₂)-.

### [Item B55]

The compound or the pharmaceutically acceptable salt thereof according to item B54, wherein L⁴ is 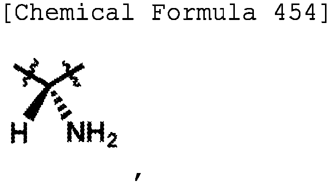 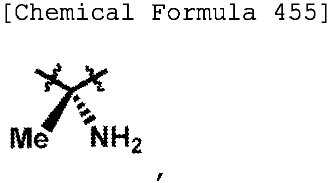 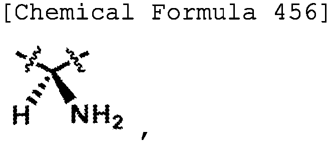 or

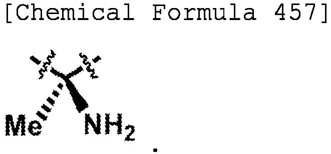

### [Item B56]

The compound or the pharmaceutically acceptable salt thereof according to item B54 or B55, wherein L⁴ is 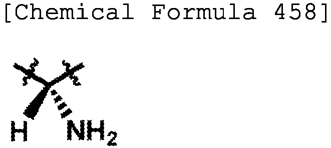 or

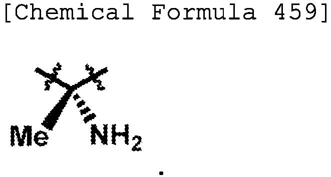

### [Item B57]

The compound or the pharmaceutically acceptable salt thereof according to any one of items B52 to B56, wherein R²⁰ is a hydrogen atom.

### [Item B58]

The compound or the pharmaceutically acceptable salt thereof according to any one of items B1 to B23 and B27 to B39, wherein
R⁵ is -NR^{e1}R^{f1},
L³ is -CH₂-,
L⁴ is -CR⁴⁰R⁴¹-, and
R⁴⁰ and R⁴¹, together with the carbon atom to which they are attached, form a C₃₋₆ alicyclic group.

### [Item B59]

The compound or the pharmaceutically acceptable salt thereof according to item B58, wherein
R^{e1} and R^{f1} are the same or different, each independently
   1) a hydrogen atom, or
   2) an optionally substituted C₁₋₃ alkyl group, and
L⁴ is

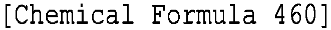

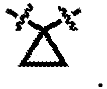

### [Item B60]

The compound or the pharmaceutically acceptable salt thereof according to any one of items B1 to B2 and B10 to B35, wherein L⁴ is a single bond or a C₁₋₆ alkylene group optionally substituted with -NR²¹R²² or =NOR²³, wherein R²¹, R²², and R²³ are each independently a hydrogen atom, an optionally substituted C₁₋₆ alkyl group, or an optionally substituted 4- to 10-membered non-aryl heterocyclyl carbonyl group.

### [Item B61]

The compound or the pharmaceutically acceptable salt thereof according to any one of items B1 to B2, B10 to B35, and B60, wherein L⁴ is a single bond, -CH₂-, -CH(NH₂)-, -CMe(NH₂)-, - CH(NHMe)-, -CD(NH₂)- (wherein D represents a heavy hydrogen atom), -CH₂CH₂-, or -CH(NH₂)-CH₂-, wherein if an amino group is present in L⁴, carbon that attaches to the amino group attaches to L³.

### [Item B62]

The compound or the pharmaceutically acceptable salt thereof according to item B61, wherein
L³ is -C(=O)-, and
L⁴ is -CH(NH₂)- or -CMe (NH₂)-.

### [Item B63]

The compound or the pharmaceutically acceptable salt thereof according to item B62, wherein L⁴ is an isomeric structure of one of 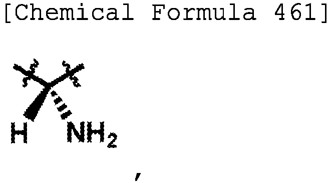 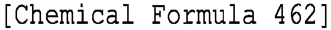 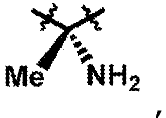 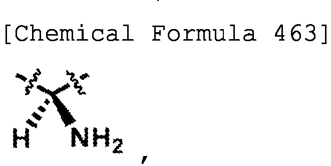 and

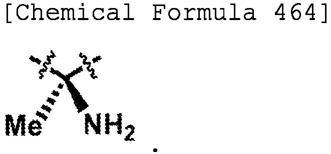

### [Item B64]

The compound or the pharmaceutically acceptable salt thereof according to item B62 or B63, wherein L⁴ is 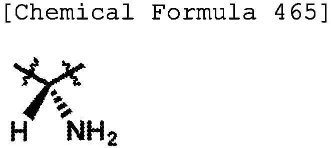 or

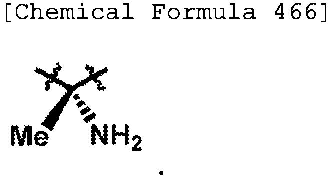

### [Item B65]

The compound or the pharmaceutically acceptable salt thereof according to any one of items B1 to B2, B10 to B35, and B60 to B64, wherein R⁵ is a hydrogen atom, an optionally substituted C₁₋₆ alkyl group, an optionally substituted 4- to 10-membered non-aryl heterocycle, optionally substituted C₆₋₁₀ aryl, optionally substituted 5- or 6-membered heteroaryl, an optionally substituted C₁₋₆ alkylthio group, or -NR^{e1}OH, wherein R^{e1} is a hydrogen atom or an optionally substituted C₁₋₆ alkyl group.

### [Item B66]

The compound or the pharmaceutically acceptable salt thereof according to any one of items B1 to B2, B10 to B35, and B60 to B65, wherein R⁵ is optionally substituted 5- or 6-membered heteroaryl or optionally substituted C₆₋₁₀ aryl.

### [Item B67]

The compound or the pharmaceutically acceptable salt thereof according to any one of items B1 to B2, B10 to B35, and B60 to B66, wherein R⁵ is optionally substituted 5- or 6-membered heteroaryl.

### [Item B68]

The compound or the pharmaceutically acceptable salt thereof according to any one of items B1 to B2, B10 to B35, and B60 to B67, wherein R⁵ is an optionally substituted 4- to 10-membered non-aryl heterocycle.

### [Item B69]

The compound or the pharmaceutically acceptable salt thereof according to any one of items B1 to B2, B10 to B35, and B60 to B65, wherein L⁴ is a single bond, and R⁵ is -NR^{e1}OH, wherein R^{e1} is a hydrogen atom or an optionally substituted C₁₋₆ alkyl group.

### [Item B70]

The compound or the pharmaceutically acceptable salt thereof according to any one of items B1 to B2 and B10 to B35, wherein
L⁴ is
   1) -(CH₂)ₚ-CR¹⁰(NHR¹¹)-,
   2) -(CH₂)_{q}-CR¹²R¹³, or
   3) -(CH₂)ₚ-CR¹⁰(NHR¹¹)-(CH₂)_{q}-CR¹²R¹³- (wherein p and q are independently 0 or 1),
R¹⁰ is
   1) a hydrogen atom,
   2) a carboxyl group, or
   3) -C(=O)NR^{10a}R^{10b},
R¹¹ is
   1) a hydrogen atom,
   2) -C(=O)R^{11a}, or
   3) an optionally substituted 5- or 6-membered non-aryl heterocyclyl carbonyl group,
      wherein if R¹⁰ is -C(=O)NR^{10a}R^{10b}, R^{10b} and R¹¹ together may form - CH₂CH₂-,
R¹² is
   1) a hydrogen atom, or
   2) an optionally substituted C₁₋₄ alkyl group,
R¹³ is
   1) a hydrogen atom,
   2) a hydroxyl group,
   3) an optionally substituted C₁₋₄ alkyl group,
   4) a sulfanyl group,
   5) a carboxyl group,
   6) an optionally substituted C₁₋₄ alkylthio group,
   7) -NR^{13a}R^{13b},
   8) -NR^{13a}-C(=O)R^{13b},
   9) an optionally substituted 5- or 6-membered non-aryl heterocyclyl carbonylamino group,
   10) -NR^{13a}-C(=O)NR^{13b}R^{13c},
   11) -C(=O)NR^{13a}R^{13b},
   12) -C(=O)NR^{13a}OR^{13b},
   13) -S(=O)₂R^{13a},
   14) -S(=O)₂-NR^{13a}R^{13b},
   15) -C(=O)NR^{13a}-S(=O)₂-R^{13b} or
   16) -C(=O)NR^{13a}-S(=O)₂-NR^{13b}R^{13c}, and
R^{10a}, R^{10b}, R^{11a}, R^{13a}, R^{13b}, and R^{13c} are each independently a hydrogen atom or an optionally substituted C₁₋₄ alkyl group.

### [Item B71]

The compound or the pharmaceutically acceptable salt thereof according to item B70, wherein R⁵ is a hydrogen atom or an optionally substituted C₁₋₄ alkyl group.

### [Item B72]

The compound or the pharmaceutically acceptable salt thereof according to any one of items B1 to B2, B10 to B35, and B60 to B67, wherein
R⁵ is selected from the group consisting of 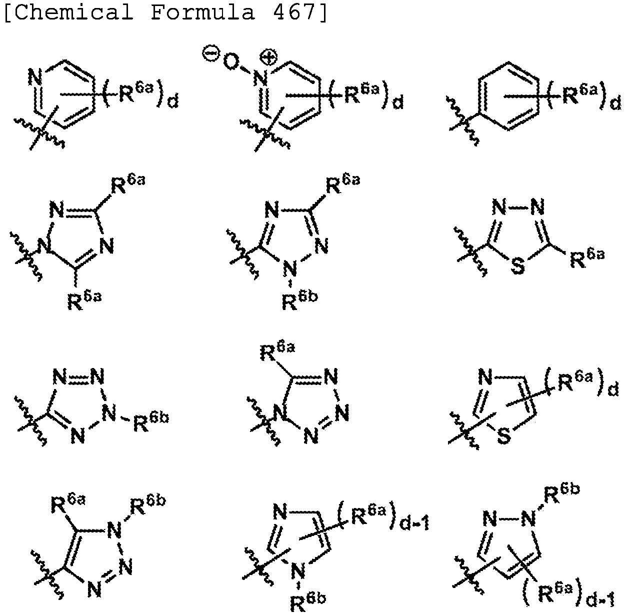 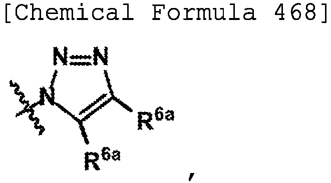
subscript d is the number of substitutable positions on a ring of R⁵,
each R^{6a} is independently selected from the group consisting of
   1) a hydrogen atom,
   2) a hydroxyl group,
   3) a cyano group,
   4) a nitro group,
   5) halogen,
   6) a C₁₋₄ alkyl group,
   7) a C₃₋₁₀ alicyclic group,
   8) a C₁₋₄ alkoxy group,
   9) a C₃₋₁₀ alicyclic oxy group,
   10) a C₆₋₁₀ aryloxy group,
   11) a 5- or 6-membered heteroaryloxy group,
   12) a 4- to 10-membered non-aryl heterocyclyl oxy group,
      (wherein each substituent from 6) to 12) is optionally substituted),
   13) -SO₂-NR^{e2}R^{f2},
   14) -NR⁹²-CR^{e2}(=NR^{f2}),
   15) -NR^{g2}-CR^{e2}(=N-OR^{f2}),
   16) -NR^{h2}-C(=NR^{g2})NR^{e2}R^{f2},
   17) -NR^{h2}-C(=N-OR^{g2})NR^{e2}R^{f2},
   18) -NRⁱ²-C(=NR^{h2})NR^{g2}-NR^{e2}R^{f2},
   19) -NRⁱ²-C(=N-OR^{h2})NR^{g2}-NR^{e2}R^{f2},
   20) -C(=NR^{e2})R^{f2},
   21) -C(=N-OR^{e2})R^{f2},
   22) -C(=NR^{h2})-NR^{e2}R^{f2},
   23) -C(=NR^{h2})NR^{g2}-NR^{e2}R^{f2},
   24) -C(=N-OR^{h2})NR^{g2}-NR^{e2}R^{f2},
   25) -NR^{e2}R^{f2},
   26) -NR^{g2}-NR^{e2}R^{f2},
   27) -NR^{e2}OR^{f2},
   28) -NR^{e2}-C(=O)R^{f2},
   29) -C(=O)NR^{e2}R^{f2},
   30) -C(=O)NR^{e2}OR^{f2},
   31) -C(=O)NR^{g2}-NR^{e2}R^{f2},
   32) -C(=O)R^{e2},
   33) -C(=O)OR^{e2}, and
   34) -C(=N-OR^{h2})NR^{e2}R^{f2}, and
each R^{6b} is independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C(=NR^{e2})R^{f2},
   6) -C(=N-OR^{e2})R^{f2},
   7) -SO₂-NR^{e2}R^{f2},
   8) -C(=NR^{h2})-NR^{e2}R^{f2},
   9) -C (=NR^{h2})NR^{g2}-NR^{e2}R^{f2},
   10) -C(=N-OR^{h2})NR⁹²-NR^{e2}R^{f2},
   11) -C(=O)NR^{e2}R^{f2},
   12) -C(=O)NR^{e2}OR^{f2},
   13) -C(=O)NR^{g2}-NR^{e2}R^{f2},
   14) -C(=O)R^{e2}, and
   15) -C(=N-OR^{h2})NR^{e2}R^{f2}.

### [Item B73]

The compound or the pharmaceutically acceptable salt thereof according to any one of items B1 to B2, B10 to B35, B60 to B67, and B72, wherein
R⁵ is 5- or 6-membered aryl or heteroaryl selected from the group consisting of 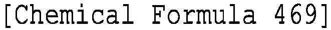 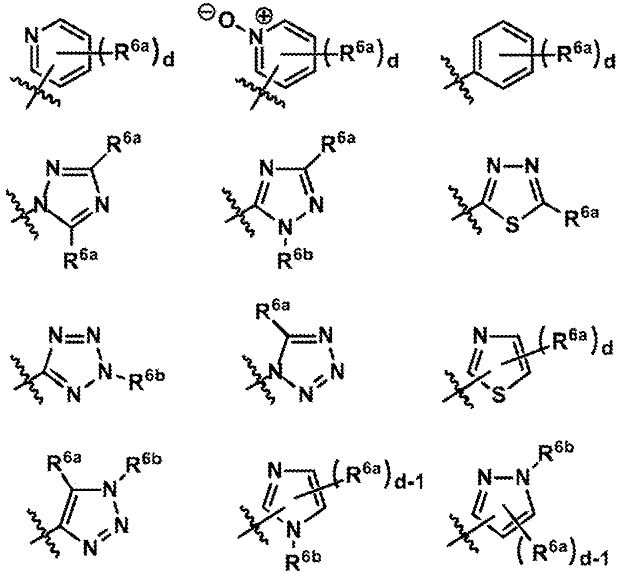 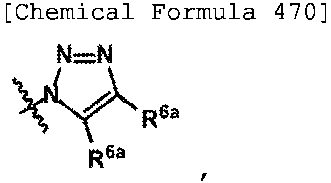
subscript d is the number of substitutable positions on a ring of R⁵,
each R^{6a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e2}R^{f2}, a 5- or 6-membered non-aryl heterocycle, -C(=O)OR^{f2}, or a hydroxyl group),
   5) a C₁₋₄ alkoxy group,
   6) -NR^{e2}R^{f2}, and
   7) -C(=O)OR^{e2}, and
each R^{6b} is independently selected from the group consisting of
   1) a hydrogen atom,
   2) a hydroxyl group, and
   3) a C₁₋₄ alkyl group
      (wherein the alkyl group is optionally substituted with NR^{e2}R^{f2}, - C(=O)NR^{e2}R^{f2}, -C(=O)OR^{f2}, or a hydroxyl group).

### [Item B74]

The compound or the pharmaceutically acceptable salt thereof according to any one of items B1 to B2, B10 to B35, B60 to B67, and B72 to B73, wherein
R⁵ is

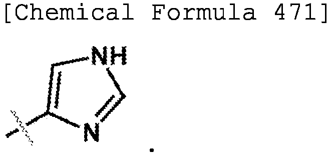

### [Item B75]

The compound or the pharmaceutically acceptable salt thereof according to any one of items B1 to B2, B10 to B35, B60 to B67, and B72 to B73, wherein R^{e2} and R^{f2} are the same or different, each independently a hydrogen atom, an optionally substituted C₁₋₆ alkyl group, or an optionally substituted C₃₋₁₀ alicyclic group.

### [Item B76]

The compound or the pharmaceutically acceptable salt thereof according to any one of items B1 to B2, B10 to B35, B60 to B67, B72 to B73, and B75, wherein R^{e2} and R^{f2} are the same or different, each independently a hydrogen atom or an optionally substituted C₁₋₆ alkyl group.

### [Item B77]

The compound or the pharmaceutically acceptable salt thereof according to any one of items B1 to B2, B10 to B35, B60 to B67, B72 to B73, and B75 to B76, wherein R^{e2} and R^{f2} are hydrogen atoms.

### [Item B78]

The compound or the pharmaceutically acceptable salt thereof according to any one of items B72 to 73 and B75 to B76, wherein R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

### [Item B79]

The compound or the pharmaceutically acceptable salt thereof according to any one of items B1 to B2, B10 to B35, B60 to B65, and B68, wherein
R⁵ is a 4- to 6-membered non-aryl heterocycle selected from the group consisting of 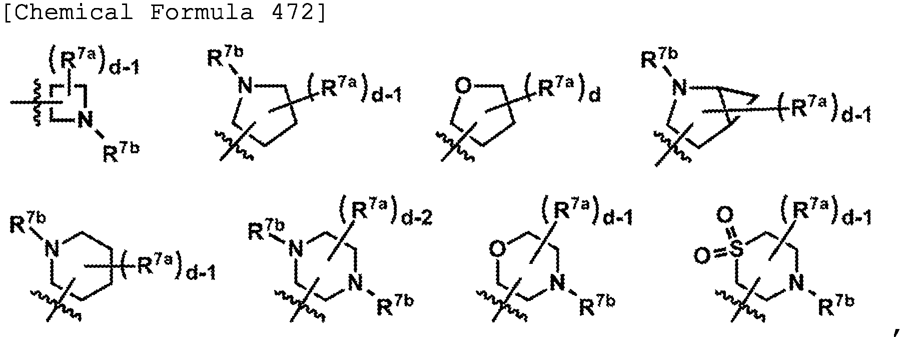
subscript d is the number of substitutable positions on a ring of R⁵,
each R^{7a} is independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e3}R^{f3},
   13) -NR^{g2}-CR^{e3}(=NR^{f3}),
   14) -NR^{g2}-CR^{e3}(=N-OR^{f3}),
   15) -NR^{h2}-C(=NR^{g2})NR^{e3}R^{f3},
   16) -NR^{h2}-C(=N-OR^{g2})NR^{e3}R^{f3},
   17) -NRⁱ²-C(=NR^{h2})NR^{g2}-NR^{e3}R^{f3},
   18) -NRⁱ²-C(=N-OR^{h2})NR^{g2}-NR^{e3}R^{f3},
   19) -C(=NR^{e3})R^{f3},
   20) -C(=N-OR^{e3})R^{f3},
   21) -C(=NR^{h2})-NR^{e3}R^{f3},
   22) -C(=NR^{h2})NR^{g2}-NR^{e3}R^{f3},
   23) -C(=N-OR^{h2})NR^{g2}-NR^{e3}R^{f3},
   24) -NR^{e3}R^{f3},
   25) -NR^{g2}-NR^{e3}R^{f3},
   26) -NR^{e3}OR^{f3},
   27) -NR^{e3}-C(=O)R^{f3},
   28) -C(=O)NR^{e3}R^{f3},
   29) -C(=O)NR^{e3}OR^{f3},
   30) -C(=O)NR^{g2}-NR^{e3}R^{f3},
   31) -C(=O)R^{e3},
   32) -C(=O)OR^{e3}, and
   33) -C(=N-OR^{h2})NR^{e3}R^{f3},
each R^{7b} is independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C(=NR^{e3})R^{f3},
   6) -C(=N-OR^{e3})R^{f3},
   7) -SO₂-NR^{e3}R^{f3},
   8) -C(=NR^{h2})-NR^{e3}R^{f3},
   9) -C(=NR^{h2})NR^{g2}-NR^{e3}R^{f3},
   10) -C(=N-OR^{h2})NR^{g2}-NR^{e3}R^{f3},
   11) -C(=O)NR^{e3}R^{f3},
   12) -C(=O)NR^{e3}OR^{f3},
   13) -C(=O)NR^{g2}-NR^{e3}R^{f3},
   14) -C(=O)R^{e3}, and
   15) -C(=N-OR^{h2})NR^{e3}R^{f3}, and
R^{e3} and R^{f3} are defined the same as R^{e2} and R^{f2} according to item B2.

### [Item B80]

The compound or the pharmaceutically acceptable salt thereof according to any one of items B1 to B2, B10 to B35, B60 to B65, B68, and B79, wherein
R⁵ is a 4- to 6-membered non-aryl heterocycle selected from the group consisting of 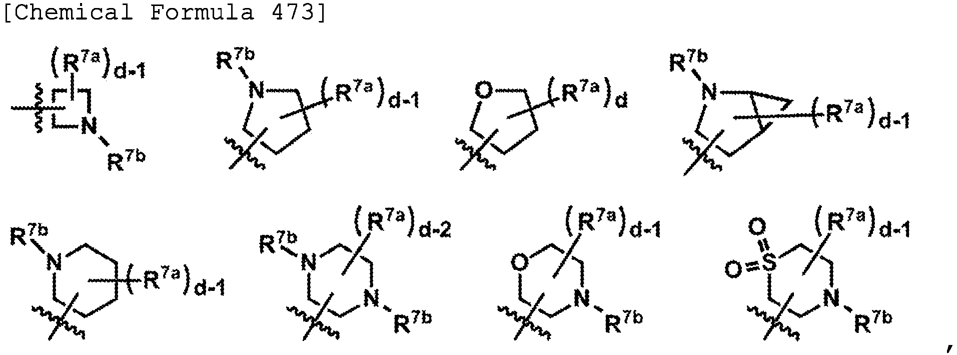
subscript d is the number of substitutable positions on a ring of R⁵,
each R^{7a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e3}R^{f3}, a 5- or 6-membered non-aryl heterocycle, -C(=O)OR^{f3}, or a hydroxyl group),
   5) a C₁₋₄ alkoxy group,
   6) -NR^{e3}R^{f3},
   7) -C(=O)OR^{e3},
   8) C₆₋₁₀ aryl, and
   9) -C(=O)NR^{e3}R^{f3},
each R^{7b} is independently selected from the group consisting of
   1) a hydrogen atom,
   2) a hydroxyl group, and
   3) a C₁₋₄ alkyl group
      (wherein the alkyl group is optionally substituted with NR^{e3}R^{f3}, - C(=O)OR^{f3}, or a hydroxyl group), and
R^{e3} and R^{f3} are defined the same as R^{e2} and R^{f2} according to any of items B75 to B77.

### [Item B81]

The compound or the pharmaceutically acceptable salt thereof according to any one of items B1 to B2, B10 to B35, and B70 to B71, wherein
L⁴ is -CH(NH₂)-CHR¹³-, wherein carbon that attaches to the NH₂ attaches to L³,
R⁵ is a hydrogen atom, and
R¹³ is
   1) -NH-C(=O)CH₃,
   2) -NH-C(=O)NH₂,
   3) -NH-C(=O)CH(NH₂)-CH₂C(=O)NH₂,
   4) -NH-C(=O)CH₂-NH₂,
   5) -NH-C(=O)CH(NH₂)-CH₂OH, or
   6) a pyrrolidin-2-ylcarbonylamino group.

### [Item B82]

The compound or the pharmaceutically acceptable salt thereof according to any one of items B1 to B2, B10 to B35, and B70 to B71, wherein
L⁴ is -CH (NH₂)-CR¹²R¹³, wherein carbon that attaches to the NH₂ attaches to L³,
R⁵ is a hydrogen atom or methyl,
R¹² is a hydrogen atom or methyl, and
R¹³ is a benzylthio group or a sulfanyl group.

### [Item B83]

The compound or the pharmaceutically acceptable salt thereof according to any one of items B1 to B2, B10 to B35, and B70 to B71, wherein
L⁴ is -CH(NH₂)-(CH₂)_{q}-CHR¹³-, wherein q is 0 or 1, and carbon that attaches to the NH₂ attaches to L³,
R⁵ is a hydrogen atom, and
R¹³ is
   1) a carboxyl group,
   2) -C(=O)NH₂,
   3) -C(=O)NH(CH₃),
   4) -C(=O)N(CH₃)₂,
   5) -C(=O)NH-(CH₂)₂-OH,
   6) -C(=O)NH-(CH₂)₂-NH₂,
   7) -C(=O)NH-S(=O)₂-CH₃,
   8) -C(=O)NHOH,
   9) -S(=O)₂-NH₂,
   10) -S(=O)₂-CH₃, or
   11) a hydroxyl group.

### [Item B84]

The compound or the pharmaceutically acceptable salt thereof according to any one of items B1 to B2, B10 to B35, and B70 to B71, wherein
L⁴ is -CH(NHR¹¹)-CH₂-, wherein carbon that attaches to the NHR¹¹ attaches to L³,
R⁵ is hydrogen, and
R¹¹ is
   1) -C(=O)CH(NH₂)-CH₂C(=O)NH₂,
   2) -C(=O)CH₂-NH₂,
   3) -C(=O)CH(CH₃)-NH₂,
   4) -C(=O)CH(NH₂)-CH₂OH, or
   5) pyrrolidin-2-ylcarbonyl.

### [Item B85]

The compound or the pharmaceutically acceptable salt thereof according to any one of items B1 to B2, B10 to B35, and B70 to B71, wherein
L⁴ is -CH (NHR¹¹) -CH (COOH) -, wherein carbon that attaches to the NHR¹¹ attaches to L³,
R⁵ is hydrogen, and
R¹¹ is
   1) -C(=O)CH(NH₂)-CH₂C(=O)NH₂,
   2) -C(=O)CH₂-NH₂,
   3) -C(=O)CH(CH₃)-NH₂,
   4) -C(=O)CH(NH₂)-CH₂OH, or
   5) pyrrolidin-2-ylcarbonyl.

### [Item B86]

The compound or the pharmaceutically acceptable salt thereof according to any one of items B1 to B2, B10 to B35, and B70 to B71, wherein
L⁴ is -CHR¹³- or -CH₂-CHR¹³-,
R⁵ is hydrogen, and
R¹³ is -C(=O)NH₂ or -C(=O)NHOH.

### [Item B87]

The compound or the pharmaceutically acceptable salt thereof according to any one of items B1 to B2, B10 to B35, and B70 to B71, wherein
L⁴ is -CH₂-CR¹⁰(NH₂)-, wherein the CH₂ group attaches to L³,
R⁵ is hydrogen, and
R¹⁰ is a carboxy group or -C(=O)NH₂.

### [Item B88]

The compound or the pharmaceutically acceptable salt thereof according to any one of items B1 to B2, B10 to B35, and B70 to B71, wherein
L⁴ is -(CH₂)ₚ-CR¹⁰(NHR¹¹)-(CH₂)_{q}-CHR¹³- or -CHR¹³-(CH₂)_{q}-CR¹⁰(NHR¹¹)-(CH₂)ₚ-, wherein q is 0 or 1,
R⁵ is hydrogen,
   (1) if L⁴ is -CHR¹³-(CH₂)_{q}-CR¹⁰(NHR¹¹)-(CH₂)ₚ-,
      carbon of the -CHR¹³- group attaches to L³,
      p is 0,
      R¹⁰ is a hydrogen atom, a carboxyl group, or -C(=O)NHR^{10b},
      R¹¹ is a hydrogen atom,
      R^{10b} is a hydrogen atom,
         wherein if R¹⁰ is -C(=O)NHR^{10b}, R^{10b} and R¹¹ together may form - CH₂CH₂-, and
      R¹³ is a hydrogen atom, and
   (2) if L⁴ is -(CH₂)ₚ-CR¹⁰(NHR¹¹)-(CH₂)_{q}-CHR¹³-, carbon of the -(CH₂)ₚ- group attaches to L³,
      p is 1,
      R¹⁰ and R¹¹ are both hydrogen atoms,
      R¹³ is a carboxyl group or -C(=O)NR^{13a}R^{13b}, and
      R^{13a} and R^{13b} are each independently a hydrogen atom or an optionally substituted C₁₋₄ alkyl group.

### [Item B89]

The compound or the pharmaceutically acceptable salt thereof according to any one of items B1 to B2, B10 to B35, and B70 to B71, wherein
L⁴ is -CR¹² (NH₂)-,
R¹² is a hydrogen atom or a methyl group, and
R⁵ is a C₁₋₄ alkyl group optionally substituted with a hydroxyl group.

### [Item B90]

The compound or the pharmaceutically acceptable salt thereof according to item B1, selected from the group consisting of the compounds represented by the following names or structural formulas:
5-({1-[amino(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 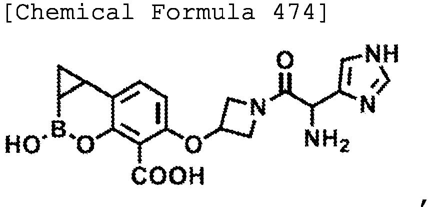
9-[1-[2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 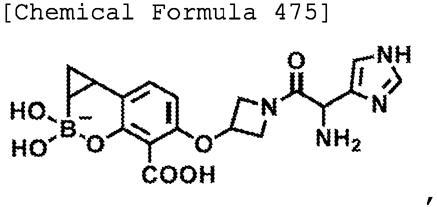
(1aS,7bR)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 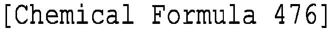 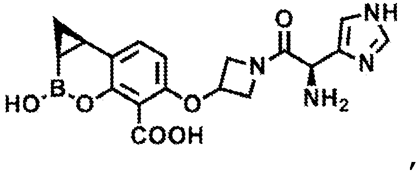
(2R,4S)-9-[1-[(2R)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 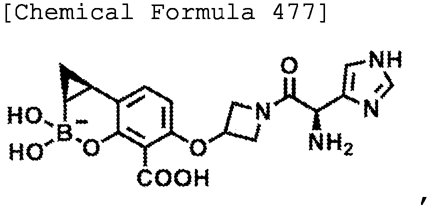
(1aS,7bR)-5-({1-[(2S)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 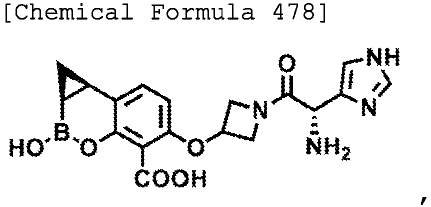
(2R,4S)-9-[1-[(2S)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 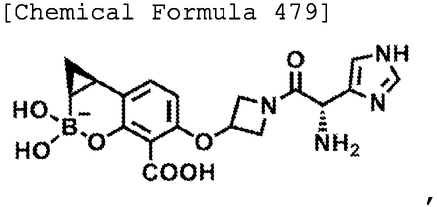
(1aR,7bS)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 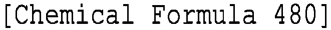 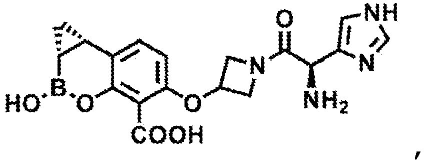
(2S,4R)-9-[1-[(2R)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 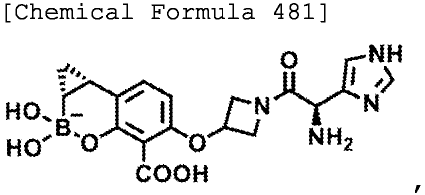
(1aR,7bS)-5-({1-[(2S)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 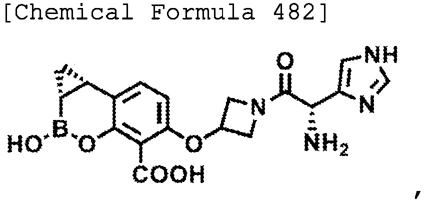
(2S,4R)-9-[1-[(2S)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 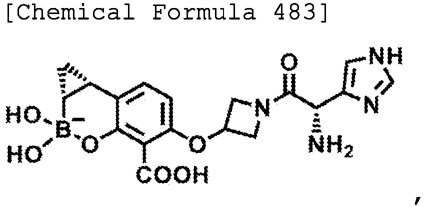
5-({1-(2-amino-2-carboxypropyl)azetidin-3-yl}oxy)-2-hydroxy-1,1a,2,7b-tetrahydrocyclopropa[c][1,2]benzoxaborinine-4-carboxylic acid 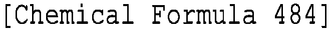 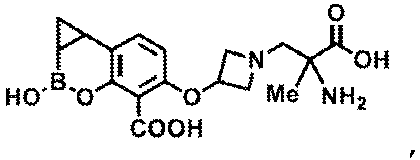
9-[1-(2-amino-2-carboxylatopropyl)azetidin-3-yl]oxy-5,5-dihydroxy-6-oxa-5-boranuidatricyclo[5.4.0.0^{2,4}]undeca-1(7),8,10-triene-8-carboxylate 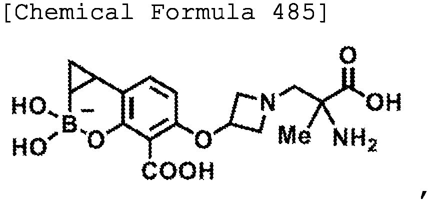
(1aS,7bR)-5-({1-[(2R)-2-amino-2-carboxypropyl]azetidin-3-yl}oxy)-2-hydroxy-1,1a,2,7b-tetrahydrocyclopropa[c][1,2]benzoxaborinine-4-carboxylic acid 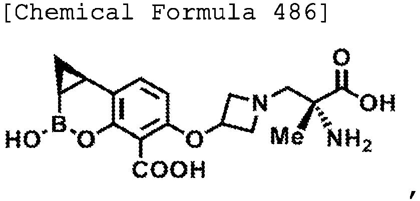
(2R,4S)-9-[1-[(2R)-2-amino-2-carboxylatopropyl]azetidin-3-yl]oxy-5,5-dihydroxy-6-oxa-5-boranuidatricyclo[5.4.0.0^{2,4}]undeca-1(7),8,10-triene-8-carboxylate 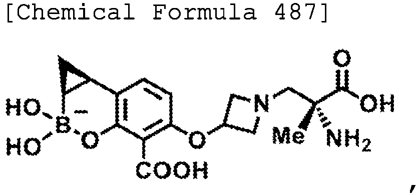
(1aR,7bS)-5-({1-[(2R)-2-amino-2-carboxypropyl]azetidin-3-yl}oxy)-2-hydroxy-1,1a,2,7b-tetrahydrocyclopropa[c][1,2]benzoxaborinine-4-carboxylic acid 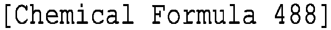 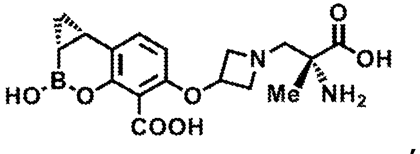
(2S,4R)-9-[1-[(2R)-2-amino-2-carboxylatopropyl]azetidin-3-yl]oxy-5,5-dihydroxy-6-oxa-5-boranuidatricyclo[5.4.0.0^{2,4}]undeca-1(7),8,10-triene-8-carboxylate 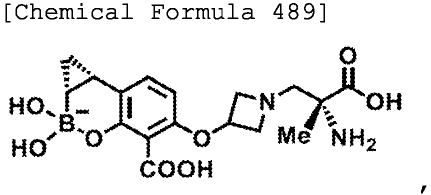
5-({1-[2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 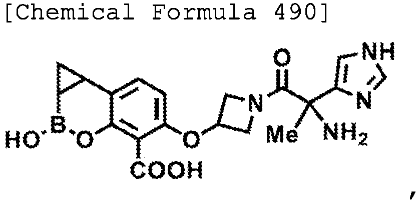
9-[1-[2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 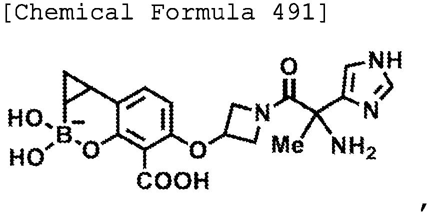
(1aS,7bR)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 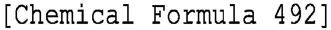 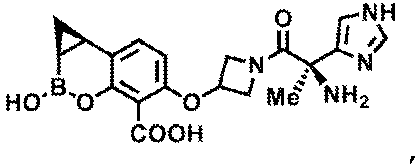
(2R,4S)-9-[1-[(2R)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 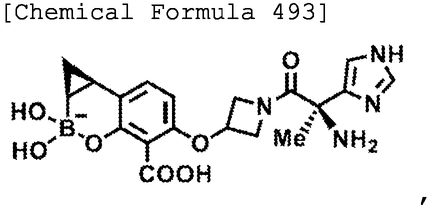
(1aS,7bR)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 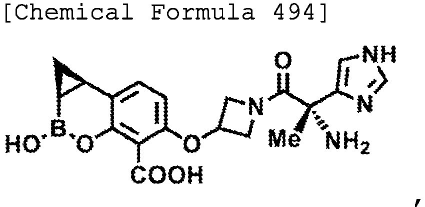
(2R,4S)-9-[1-[(2S)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 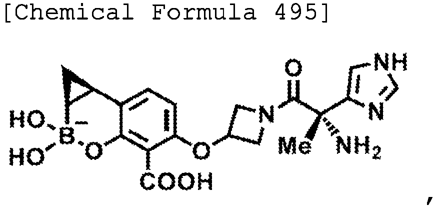
(1aR,7bS)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 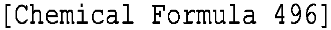 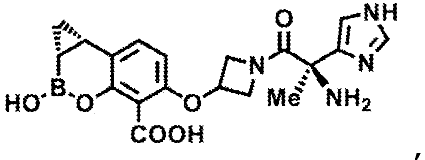
(2S,4R)-9-[1-[(2R)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 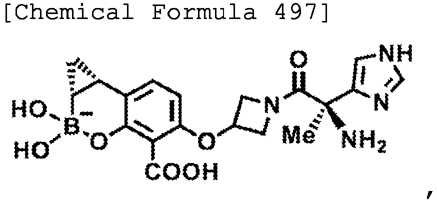
(1aR,7bS)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 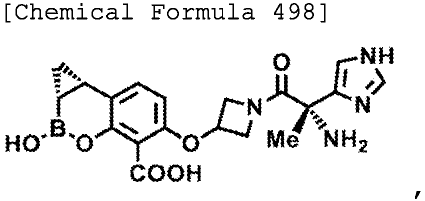 and
(2S,4R)-9-[1-[(2S)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid

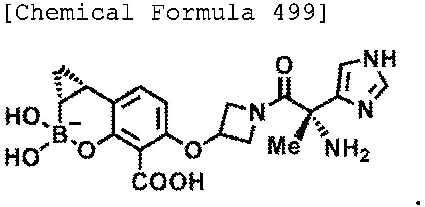

### [Item B91]

A compound represented by formula (11): 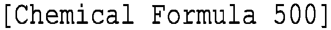 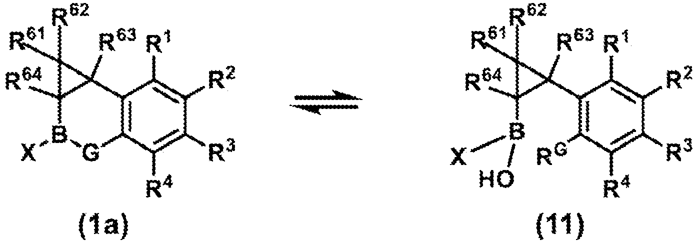 or a pharmaceutically acceptable salt thereof,
wherein R^{G} is a hydroxyl group, a thiol group, or -NHR^{a1}, R^{a1}, X, R¹, R², R³, R⁴, R⁶¹, R⁶², R⁶³, and R⁶⁴ are defined the same as the definition according to any one of items B1 to B15, and formula (1a) is defined the same as item B1.

### [Item B92]

The compound or the pharmaceutically acceptable salt thereof according to item B91, wherein the compound of formula (11) is represented by formula (12): 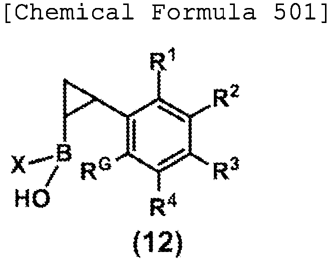 wherein X, R¹, R², R³, and R⁴ are defined the same as the definition according to item B16 or B17.

### [Item B93]

The compound or the pharmaceutically acceptable salt thereof according to item B91 or B92, wherein the compound of formula (11) or formula (12) is represented by formula (13): 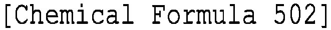 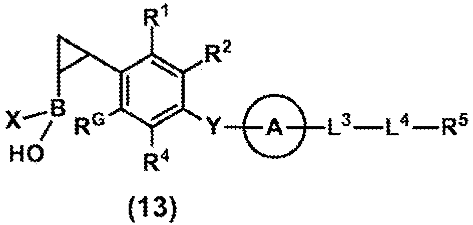 wherein X, Y, ring A, L³, L⁴, R¹, R², R⁴, and R⁵ are defined the same as the definition according to any one of items B18 to B22 and B28 to B31.

### [Item B94]

The compound or the pharmaceutically acceptable salt thereof according to item B93, wherein X and R^{G} are hydroxyl groups, R⁴ is a carboxyl group, and ring A is an optionally substituted 4- to 6-membered nitrogen-containing non-aryl heterocycle.

### [Item B95]

The compound or the pharmaceutically acceptable salt thereof according to any one of items B91 to B94, wherein the compound of formula (11), formula (12), or formula (13) is represented by formula (14): 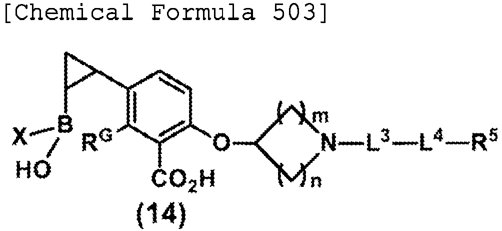 wherein X, L³, L⁴, m, n, and R⁵ are defined the same as the definition according to any one of items B32 to B59.

### [Item B96]

The compound or the pharmaceutically acceptable salt thereof according to item B95, wherein the compound of formula (14) is one of the enantiomers represented by formula (15): 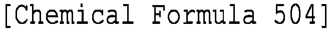 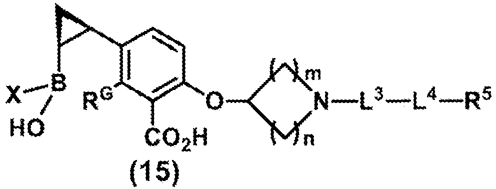 and formula (16): 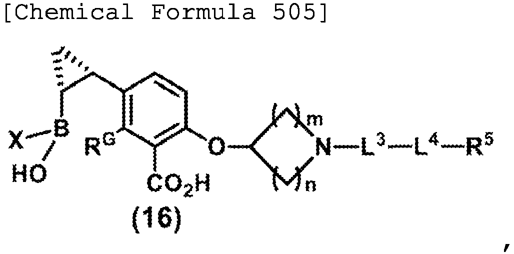 wherein X, L³, L⁴, m, n, and R⁵ are defined the same as the definition according to any one of items B32 to B59.

### [Item B97]

The compound or the pharmaceutically acceptable salt thereof according to any one of items B91 to B96, wherein R^{G} is a hydroxyl group or a thiol group.

### [Item B98]

The compound or the pharmaceutically acceptable salt thereof according to any one of items B91 to B97, wherein R^{G} is a hydroxyl group.

### [Item B99]

The compound or the pharmaceutically acceptable salt thereof according to any one of items B91 to B98, wherein X is a hydroxyl group or a C₁₋₆ alkoxy group.

### [Item B100]

The compound or the pharmaceutically acceptable salt thereof according to any one of items B91 to B99, wherein X is a hydroxyl group.

### [Item B101]

The compound or the pharmaceutically acceptable salt thereof according to any one of items B95 to B100, wherein m is 1 or 2, n is 1 or 2, and m + n is 2 or 3.

### [Item B102]

The compound or the pharmaceutically acceptable salt thereof according to any one of items B95 to B101, wherein m is 1, and n is 1.

### [Item B103]

The compound or the pharmaceutically acceptable salt thereof according to any one of items B91 to B102, wherein L³ is defined the same as the definition according to item B24 or B25.

### [Item B104]

The compound or the pharmaceutically acceptable salt thereof according to any one of items B91 to B103, wherein L⁴ and R⁵ are defined the same as the definition according to any one of items B26 and B60 to B89.

### [Item B105]

The compound or the pharmaceutically acceptable salt thereof according to item B92, selected from the group consisting of the compounds represented by the following names or structural formulas:
6-({1-[amino(1H-imidazol-4-yl)acetyl]azetidin-3-yl}oxy)-3-(2-boronocyclopropyl)-2-hydroxybenzoic acid 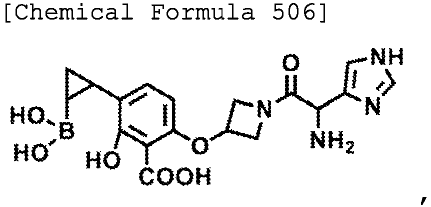
6-({1-[(2R)-2-amino-2-(1H-imidazol-4-yl)acetyl]azetidin-3-yl}oxy)-3-[(1R,2S)-2-boronocyclopropyl]-2-hydroxybenzoic acid 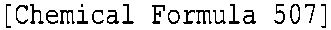 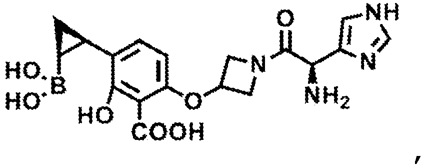
6-({1-[(2S)-2-amino-2-(1H-imidazol-4-yl)acetyl]azetidin-3-yl}oxy)-3-[(1R,2S)-2-boronocyclopropyl]-2-hydroxybenzoic acid 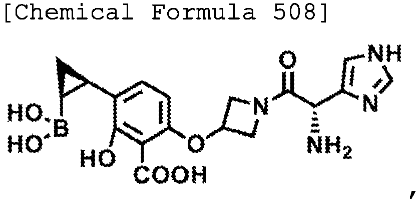
6-({1-[(2R)-2-amino-2-(1H-imidazol-4-yl)acetyl]azetidin-3-yl}oxy)-3-[(1S,2R)-2-boronocyclopropyl]-2-hydroxybenzoic acid 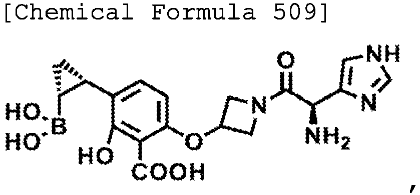
6-({1-[(2S)-2-amino-2-(1H-imidazol-4-yl)acetyl]azetidin-3-yl}oxy)-3-[(1S,2R)-2-boronocyclopropyl]-2-hydroxybenzoic acid 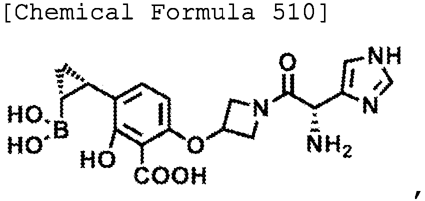
6-({1-(2-amino-2-carboxypropyl)azetidin-3-yl}oxy)-3-(2-boronocyclopropyl)-2-hydroxybenzoic acid 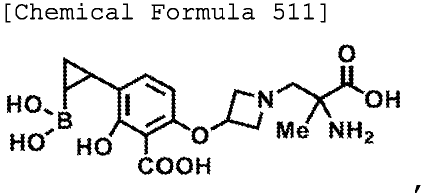
6-({1-[(2R)-2-amino-2-carboxypropyl]azetidin-3-yl}oxy)-3-[(1R,2S)-2-boronocyclopropyl]-2-hydroxybenzoic acid 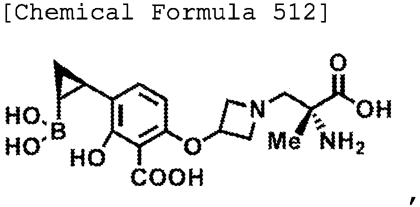
6-({1-[(2R)-2-amino-2-carboxypropyl]azetidin-3-yl}oxy)-3-[(1S,2R)-2-boronocyclopropyl]-2-hydroxybenzoic acid 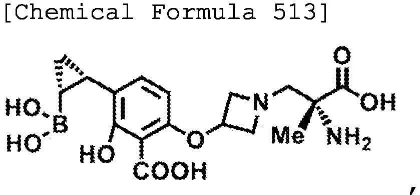
6-({1-[2-amino-2-(1H-imidazol-4-yl)propanoyl]azetidin-3-yl}oxy)-3-(2-boronocyclopropyl)-2-hydroxybenzoic acid 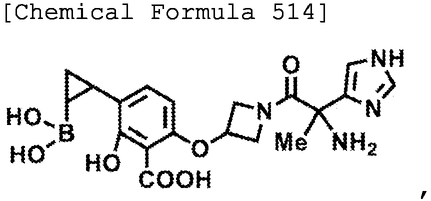
6-({1-[(2R)-2-amino-2-(1H-imidazol-4-yl)propanoyl]azetidin-3-yl}oxy)-3-[(1R,2S)-2-boronocyclopropyl]-2-hydroxybenzoic acid 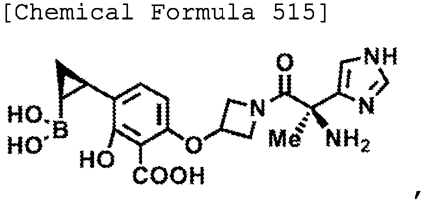
6-({1-[(2S)-2-amino-2-(1H-imidazol-4-yl)propanoyl]azetidin-3-yl}oxy)-3-[(1R,2S)-2-boronocyclopropyl]-2-hydroxybenzoic acid 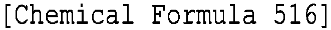 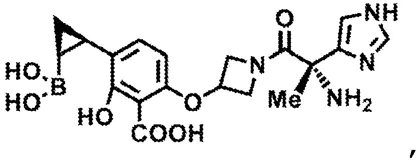
6-({1-[(2R)-2-amino-2-(1H-imidazol-4-yl)propanoyl]azetidin-3-yl}oxy)-3-[(1S,2R)-2-boronocyclopropyl]-2-hydroxybenzoic acid 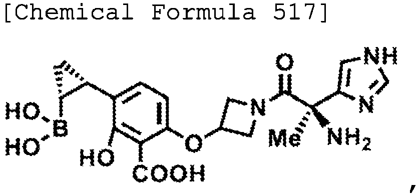 and
6-({1-[(2S)-2-amino-2-(1H-imidazol-4-yl)propanoyl]azetidin-3-yl}oxy)-3-[(1S,2R)-2-boronocyclopropyl]-2-hydroxybenzoic acid

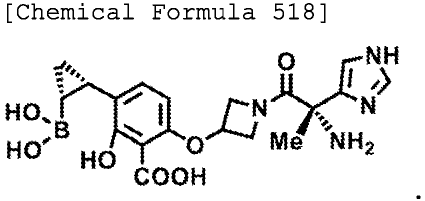

### [Item B106]

A medicament comprising the compound or the pharmaceutically acceptable salt thereof according to any one of items B1 to B105.

### [Item B107]

The medicament according to item B106, which is a therapeutic drug or a prophylactic drug for a bacterial infection.

### [Item B108]

An agent for inhibiting β-lactamase, comprising the compound or the pharmaceutically acceptable salt thereof according to any one of items B1 to B105 as an active ingredient.

### [Item B109]

A pharmaceutical composition comprising the compound or the pharmaceutically acceptable salt thereof according to any one of items B1 to B105 and a pharmaceutically acceptable carrier.

### [Item B110]

The pharmaceutical composition according to item B109, further comprising an additional agent.

### [Item B111]

The pharmaceutical composition according to item B110, wherein the additional agent is selected from the group consisting of an antibacterial agent, an antifungal agent, an antiviral agent, an anti-inflammatory agent, and an anti-allergic agent.

### [Item B112]

The pharmaceutical composition according to item B110 or B111, wherein the additional agent is a β-lactam agent.

### [Item B113]

The pharmaceutical composition according to item B111 or B112, wherein a β-lactam agent, which is the additional agent, is selected from the group consisting of amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442.

### [Item B114]

The pharmaceutical composition according to item B112 or B113, wherein the β-lactam agent is selected from ceftazidime, biapenem, doripenem, ertapenem, imipenem, meropenem, or panipenem.

### [Item B115]

The pharmaceutical composition according to item B112 or B113, wherein the β-lactam agent is selected from aztreonam, tigemonam, BAL30072, SYN2416, or carumonam.

### [Item B116]

The pharmaceutical composition according to item B109, characterized in that an additional agent is concomitantly administered.

### [Item B117]

The pharmaceutical composition according to item B116, wherein the additional agent is selected from an antibacterial agent, an antifungal agent, an antiviral agent, an anti-inflammatory agent, or an anti-allergic agent.

### [Item B118]

The pharmaceutical composition according to item B116 or B117, wherein the additional agent is a β-lactam agent.

### [Item B119]

The pharmaceutical composition according to item B117 or B118, wherein a β-lactam agent, which is the additional agent, is selected from the group consisting of amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442.

### [Item B120]

The pharmaceutical composition according to item B118 or B119, wherein the β-lactam agent is selected from the group consisting of ceftazidime, biapenem, doripenem, ertapenem, imipenem, meropenem, and panipenem.

### [Item B121]

The pharmaceutical composition according to item B118 or B119, wherein the β-lactam agent is selected from the group consisting of aztreonam, tigemonam, BAL30072, SYN2416, and carumonam.

### [Item B122]

The compound or the pharmaceutically acceptable salt thereof according to any one of items B1 to B105 for use in treating a bacterial infection.

### [Item B123]

The compound or the pharmaceutically acceptable salt thereof according to item B122, wherein the bacterial infection is a bacterial infection in which a bacteria that can have a β-lactamase is involved.

### [Item B124]

The compound or the pharmaceutically acceptable salt thereof according to item B122 or B123, wherein the bacterial infection is sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, eye infection, or an odontogenic infection.

### [Item B125]

A medicament comprised of a combination of the compound or the pharmaceutically acceptable salt thereof according to any one of items B1 to B105 and at least one agent selected from the group consisting of therapeutic agents for sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, eye infection, and an odontogenic infection.

### [Item B126]

A pharmaceutical composition comprising a β-lactam agent, wherein the pharmaceutical composition is administered with the compound or the pharmaceutically acceptable salt thereof according to any one of items B1 to B105.

### [Item B127]

A method for treating a bacterial infection, characterized in that a therapeutically effective amount of the compound or the pharmaceutically acceptable salt thereof according to any one of items B1 to B105 is administered to a patient in need thereof.

### [Item B128]

The method according to item B127, wherein the bacterial infection is a bacterial infection in which a bacteria that can have a β-lactamase is involved.

### [Item B129]

The method according to item B127 or B128, wherein the bacterial infection is sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

### [Item B130]

The method of any one of items B127 to B129, characterized in that an additional agent is concomitantly administered.

The present invention is intended so that one or more of the features described above can be provided not only as the explicitly disclosed combinations, but also as other combinations thereof. Additional embodiments and advantages of the invention are recognized by those skilled in the art by reading and understanding the following detailed description as needed.

### [Advantageous Effects of Invention]

The compound of the invention has excellent inhibitory action against serine-p-lactamase with a serine residue at the center of enzymatic activity. A better embodiment of the compound of the invention is expected to have a broad β-lactamase inhibitory action or metallo-β-lactamase inhibitory action with zinc (Zn²⁺) at the center of enzymatic activity against multiple types of β-lactamases. Therefore, the compound of the invention is useful alone or in concomitant use with a β-lactam agent as a therapeutic agent and/or prophylactic agent for a bacterial infection in which a bacteria that can have a β-lactamase is involved, i.e., sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

### [Description of Embodiments]

The present invention is described hereinafter in more detail.

Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

The terms and the general technologies that are used herein are first described.

Unless specifically noted otherwise, the term "group" refers to a monovalent group. Examples of groups that are not a monovalent group include alkylene groups (divalent) and the like. The term "group" may also be omitted in the following descriptions of substituents or the like.

Unless specifically limited, the number of substituents when defined as "optionally substituted" or "substituted" is not particularly limited herein, as long as a substitution is possible. The number of substituents is one or multiple substituents. Moreover, unless indicated otherwise, the description for each substituent is also applicable when the substituent is a part of or a substituent of another group.

A substituent in "optionally substituted" is not particularly limited, but is preferably selected from substituent group α that consists of the following. The substituent is optionally substituted with 1 to 5 of the same or different substituents. While not particularly limited by the type of substituent, if an atom to which the substituent attaches is an oxygen atom, a nitrogen atom, or a sulfur atom, the substituent is limited to the following substituents that attaches to a carbon atom.
Substituent group α includes
   1) a halogen atom
   2) a hydroxyl group
   3) a carboxyl group
   4) a cyano group
   5) a C₁₋₆ alkyl group
   6) a C₂₋₆ alkenyl group
   7) a C₂₋₆ alkynyl group
   8) a C₁₋₆ alkoxy group
   9) a C₁₋₆ alkylthio group
   10) a C₁₋₆ alkylcarbonyl group
   11) a C₁₋₆ alkylsulfonyl group
      (wherein each substituent from 5) to 11) is optionally substituted with 1 to 5 of the same or different substituents selected from substituent group β)
   12) a C₃₋₁₀ alicyclic group
   13) a C₃₋₁₀ alicyclic oxy group
   14) a C₆₋₁₀ aryloxy group
   15) a 5- or 6-membered heteroaryloxy group
   16) a 4- to 10-membered non-aryl heterocyclyl oxy group
   17) a C₃₋₁₀ alicyclic thio group
   18) a C₆₋₁₀ arylthio group
   19) a 5- or 6-membered heteroarylthio group
   20) a 4- to 10-membered non-aryl heterocyclyl thio group
   21) C₆₋₁₀ aryl
   22) 5- or 6-membered heteroaryl
   23) a 4- to 10-membered non-aryl heterocycle
   24) a C₃₋₁₀ alicyclic carbonyl group
   25) a C₆₋₁₀ arylcarbonyl group
   26) a 5- or 6-membered heteroarylcarbonyl group
   27) a 4- to 10-membered non-aryl heterocyclyl carbonyl group
   28) a C₃₋₁₀ alicyclic sulfonyl group
   29) a C₆₋₁₀ arylsulfonyl group
   30) a 5- or 6-membered heteroarylsulfonyl group
   31) a 4- to 10-membered non-aryl heterocyclyl sulfonyl group (wherein each substituent from 12) to 31) is optionally substituted with 1 to 5 of substituent group β or 1) a C₁₋₆ alkyl group)
   32) -NR^{10a}R^{11a}
   33) -SO₂-NR^{10b}R^{11b}
   34) -NR^{10c}-C(=O)R^{11c}
   35) -NR^{10d}-C(=O)OR¹¹d
   36) -NR^{12a}-C(=O)NR^{10e}R^{11e}
   37) -NR^{10f}-C(=S)R^{11f}
   38) -NR^{10g}-C(=S)OR^{11g},
   39) -NR^{12b}-C(=S)NR^{10h}R^{11h}
   40) -NR¹⁰ⁱ-SO₂-R¹¹ⁱ
   41) -NR^{12c}-SO₂-NR^{10j}R^{11j}
   42) -C(=O)OR^{10k}
   43) -C(=O)NR^{10l}R^{11k}
   44) -C(=O)NR^{10m}OR^{11l}
   45) -C(=O)NR^{12d}-NR¹⁰ⁿR^{11m}
   46) -C(=S)OR^{10o}
   47) -C(=S)NR^{10p}R¹¹ⁿ
   48) -C(=S)NR^{10q}OR^{11o}
   49) -C(=S)NR^{12e}-NR^{10r}R^{11p}
   50) -C(=NR^{13a})R^{10s}
   51) -C(=NR^{13b})CHO
   52) -C(=NR^{13c})NR^{10t}R^{11q}
   53) -C(=NR^{13d})NR^{12f}-NR^{10u}R^{11r}
   54) -NR^{17c}-C(=NR^{13k})R^{17d}
   55) -NR^{12g}-C(=NR13e)-NR^{10v}R^{11s}
   56) -NR¹⁴-C(=NR^{13f})-NR^{12h}-NR^{10w}R^{11t}
   57) -OC(=O)R^{10x}
   58) -OC(=O)OR¹⁰Y
   59) -OC(=O)NR^{10z1}R^{11u}
   60) -NR¹²ⁱ-NR10^{z2}R^{11v}
   61) -NR^{10z3}OR^{11w}
   62) -C(=N-OR^{13a})R^{10s}
   63) -C(=N-OR^{13b})CHO
   64) -C(=N-OR^{13c})NR^{10t}R^{11q} and
   65) -C(=N-OR^{13d})NR^{12f}-NR^{10u}R^{11r},
substituent group β is a group consisting of
   1) a halogen atom,
   2) a hydroxyl group,
   3) a carboxyl group,
   4) a cyano group,
   5) a C₃₋₁₀ alicyclic group,
   6) a C₁₋₆ alkoxy group,
   7) a C₃₋₁₀ alicyclic oxy group,
   8) a C₁₋₆ alkylthio group,
   9) a 5- or 6-membered heteroarylthio group,
   10) C₆₋₁₀ aryl,
   11) 5- or 6-membered heteroaryl,
   12) a 4- to 10-membered non-aryl heterocycle,
   13) a C₁₋₆ alkylcarbonyl group,
   14) a C₃₋₁₀ alicyclic carbonyl group,
   15) a C₆₋₁₀ arylcarbonyl group,
   16) a 5- or 6-membered heteroarylcarbonyl group,
   17) a 4- to 10-membered non-aryl heterocyclyl carbonyl group,
   18) -NR^{15a}R^{16a},
   19) -SO₂-NR^{15b}R^{16b},
   20) -NR^{15c}-C(=O)R^{16c},
   21) -NR^{17a}-C(=O)NR^{15d}R^{16d},
   22) -C(=O)NR15eR16e,
   23) -C(=NR^{13g})R^{15f},
   24) -C(=NR^{13h})NR^{15g}R^{16f},
   25) -NR^{16g}-C(=NR¹³ⁱ)R^{15h},
   26) -NR^{17b}-C(=NR^{13j})-NR¹⁵ⁱR^{16h},
   27) -C(=N-OR^{13g})R^{15f}, and
   28) -C(=N-OR^{13h})NR^{15g}R^{16f},
      (wherein each substituent from 5) to 17) in substituent group β is optionally substituted with 1 to 5 substituents selected from the group consisting of a halogen atom, a hydroxyl group, a cyano group, a carboxyl group, and -NR^{18a}R^{18b}),
R^{13a}, R^{13b}, R^{13c}, R^{13d}, R^{13e}, R^{13f}, R^{13g}, R^{13h}, R¹³ⁱ, R^{13j}, and R^{13k} are the same or different, each independently a hydrogen atom, a hydroxyl group, a C₁₋₆ alkyl group, or a C₁₋₆ alkoxy group,
R^{10a}, R^{10b}, R^{10c}, R^{10d}, R^{10e}, R^{10f}, R^{10g}, R^{10h}, R¹⁰ⁱ, R^{10j}, R^{10k}, R^{10l}, R^{10m}, R¹⁰ⁿ, R^{10o}, R^{10p}, R^{10q}, R^{10r}, R^{10s}, R^{10t}, R^{10u}, R^{10v}, R^{10w}, R^{10x}, R^{10y}, R^{10z1}, R^{10z2}, R^{10z3}, R^{11a}, R^{11b}, R^{11c}, R^{11d}, R^{11e}, R^{11f}, R^{11g}, R^{11h}, R¹¹ⁱ, R^{11j}, R^{11k}, R¹¹¹, R^{11m}, R¹¹ⁿ, R^{11o}, R^{11p}, R^{11q}, R^{11r}, ^{11s}, R^{11t}, R^{11u}, R^{11v}, R^{11w}, R^{12a}, R^{12b}, R^{12c}, R^{12d}, R^{12e}, R^{12f}, R^{12g}, R^{12h}, R¹²ⁱ, R¹⁴, R^{15a}, R^{15b}, R^{15c}, R^{15d}, R^{15e}, R^{15f}, R^{15g}, R^{15h}, R¹⁵ⁱ, R^{16a}, R^{16b}, R^{16c}, R^{16d}, R^{16e}, R^{16f}, R^{16g}, R^{16h}, R^{17a}, R^{17b}, R^{17c}, and R^{17d} are the same or different, each independently a hydrogen atom or a C₁₋₆ alkyl group (wherein the group is optionally substituted with 1 to 3 of the same or different substituents selected from a hydroxyl group, a cyano group, a C₁₋₆ alkoxy group, and -NR^{18a}R^{18b}), and
R^{18a} and R^{18b} are the same or different, each independently a hydrogen atom or a C₁₋₆ alkyl group.

Preferred examples of substituents in "optionally substituted" include the following substituents.

Preferred substituent group α includes
1) a halogen atom
2) a hydroxyl group
3) a carboxyl group
4) a cyano group
5) a C₁₋₆ alkyl group
6) a C₁₋₆ alkoxy group
7) a C₁₋₆ alkylthio group
8) a C₁₋₆ alkylcarbonyl group
   (wherein each substituent from 5) to 8) is optionally substituted with 1 to 5 of the same or different substituents selected from substituent group β)
9) a C₃₋₁₀ alicyclic group
10) a C₃₋₁₀ alicyclic oxy group
11) a C₆₋₁₀ aryloxy group
12) a 5- or 6-membered heteroaryloxy group
13) a 4- to 10-membered non-aryl heterocyclyl oxy group
14) a C₃₋₁₀ alicyclic thio group
15) a C₆₋₁₀ arylthio group
16) a 5- or 6-membered heteroarylthio group
17) a 4- to 10-membered non-aryl heterocyclyl thio group
18) C₆₋₁₀ aryl
19) 5- or 6-membered heteroaryl
20) a 4- to 10-membered non-aryl heterocycle
21) a C₃₋₁₀ alicyclic carbonyl group
22) a C₆₋₁₀ arylcarbonyl group
23) a 5- or 6-membered heteroarylcarbonyl group
24) a 4- to 10-membered non-aryl heterocyclyl carbonyl group (wherein each substituent from 9) to 24) is optionally substituted with 1 to 5 of substituent group β or 1) a C₁₋₆ alkyl group)
25) -NR^{10a}R^{11a}
26) -SO₂NR^{10b}R^{11b}
27) -NR^{10c}-C(=O)R^{11c}
28) -NR^{12a}-C(=O)NR^{10d}R^{11d}
29) -NR^{10e}-SO₂R^{11e}
30) -NR^{12b}-SO₂-NR^{10f}R^{11f}
31) -C(=O)NR^{10g}R^{11g}
32) -C(=NR^{13a})R^{10h}
33) -C(=NR13b)NR¹⁰ⁱR^{11h}
34) -NR^{11f}-C(=NR^{13c})R^{10g}
35) -NR^{12c}-C(=NR^{13d})-NR^{10j}R¹¹ⁱ
36) -C(=N-OR^{13a})R^{10h} and
37) -C(=N-OR^{13b})NR¹⁰ⁱR^{11h},

substituent group β is preferably a group consisting of
   1) a halogen atom
   2) a hydroxyl group
   3) a cyano group
   4) a C₃₋₁₀ alicyclic group
   5) a C₁₋₆ alkoxy group
   6) a C₁₋₆ alkylthio group
   7) a 5- or 6-membered heteroarylthio group
   8) 5- or 6-membered heteroaryl
   9) a 4- to 10-membered non-aryl heterocycle
   10) a C₁₋₆ alkylcarbonyl group
   11) a C₃₋₁₀ alicyclic carbonyl group
   12) a C₆₋₁₀ arylcarbonyl group
   13) a 5- or 6-membered heteroarylcarbonyl group
   14) a 4- to 10-membered non-aryl heterocyclyl carbonyl group
   15) -NR^{15a}R^{16a}
   16) -NR^{15b}-C(=O)R^{16b}
   17) -NR^{17a}-C(=O)NR^{15c}R^{16c}
   18) -C(=O)NR^{15d}R^{16d}
   19) -C(=NR^{13e})R^{15e}
   20) -C(=NR^{13f})NR^{15f}R^{16e}
   21) -NR^{16f}-C(=NR^{13g})R^{15g}
   22) -NR^{17b}-C(=NR^{13h})-NR^{15h}R^{16g}
   23) -C (=N-OR^{13e})R^{15e} and
   24) -C(=N-OR^{13f})NR^{15f}R^{16e}
      (wherein each substituent from 4) to 14) in substituent group β is optionally substituted with 1 to 5 substituents selected from the group consisting of a halogen atom, a hydroxyl group, a cyano group, a carboxyl group, and -NR^{18a}R^{18b}),
R^{13a}, R^{13b}, R^{13c}, R^{13d}, R^{13e}, R^{13f}, R^{13g}, and R^{13h} are the same or different, each independently a hydrogen atom, a hydroxyl group, a C₁₋₆ alkyl group, or a C₁₋₆ alkoxy group,
R^{10a}, R^{10b}, R^{10c}, R^{10d}, R^{10e}, R^{10f}, R^{10g}, R^{10h}, R¹⁰ⁱ, R^{10j}, R^{11a}, R^{11b}, R^{11c}, R^{11d}, R^{11e}, R^{11f}, R^{11g}, R^{11h}, R¹¹ⁱ, R^{12a}, R^{12b}, R^{12c}, R^{15a}, R^{15b}, ^{R15c}, R^{15d}, R^{15e}, R^{15f}, R^{15g}, R^{15h}, R^{16a}, R^{16b}, R^{16c}, R^{16d}, R^{16e}, R^{16f}, R^{16g}, R^{17a}, and R^{17b} are the same or different, each independently a hydrogen atom or a C₁₋₆ alkyl group (wherein the group is optionally substituted with 1 to 3 of the same or different substituents selected from a hydroxyl group, a cyano group, a C₁₋₆ alkoxy group, and -NR^{18a}R^{18b}), and
R^{18a} and R^{18b} are the same or different, each independently a hydrogen atom or a C₁₋₆ alkyl group.

More preferred examples of substituents in "optionally substituted" include the following substituents.

More preferred substituent group α includes
1) a halogen atom
2) a hydroxyl group
3) a cyano group
4) a C₁₋₆ alkyl group
5) a C₁₋₆ alkoxy group
6) a C₁₋₆ alkylthio group
7) a C₁₋₆ alkylcarbonyl group
   (wherein each substituent from 4) to 7) is optionally substituted with 1 to 5 of the same or different substituents selected from substituent group β)
8) a 5- or 6-membered heteroaryloxy group
9) a 4- to 10-membered non-aryl heterocyclyl oxy group
10) a 5- or 6-membered heteroarylthio group
11) a 4- to 10-membered non-aryl heterocyclyl thio group
12) C₆₋₁₀ aryl
13) 5- or 6-membered heteroaryl
14) a 4- to 10-membered non-aryl heterocycle
   (wherein each substituent from 4) to 14) is optionally substituted with 1 to 5 of substituent group β or 1) a C₁₋₆ alkyl group) 15) -NR^{10a}R^{11a}
16) -NR^{11b}-C(=O)R^{10b}
17) -NR^{12a}-C(=O)NR^{10c}R^{11c}
18) -C (=O)NR^{10d}R^{11d}
19) -C (=NR^{13a})R^{10e}
20) -C (=NR^{13b})NR^{10f}R^{11e}
21) -NR^{11f}-C(=NR^{13c})R^{10g}
22) -NR^{12b}-C(=NR^{13d})-NR^{10h}R^{11g}
23) -C (=N-OR^{13a})R^{10e} and
24) -C(=N-OR^{13b})NR^{10f}R^{11e},

substituent group β is more preferably
   1) a halogen atom,
   2) a hydroxyl group,
   3) a cyano group,
   4) -NR^{15a}R^{16a},
   5) -NR^{15b}-C(=O)R^{16b},
   6) -NR^{17a}-C(=O)NR^{15c}R^{16c},
   7) -C(=O)NR^{15d}R^{16d},
   8) -C(=NR^{13e})R^{15e},
   9) -C (=NR^{13f})NR^{15f}R^{16e},
   10) -NR^{16f}-C(=NR^{13g})R^{15g},
   11) -NR^{17b}-C(=NR^{13h})-NR^{15h}R^{16g}
   12) -C (=N-OR^{13e})R^{15e}, and
   13) -C(=N-OR^{13f})NR^{15f}R^{16e},
R^{13a}, R^{13b}, R^{13c}, R^{13d}, R^{13e}, R^{13f}, R^{13g}, and R^{13h} are the same or different, each independently a hydrogen atom, a hydroxyl group, a C₁₋₆ alkyl group, or a C₁₋₆ alkoxy group,
R^{10a}, R^{10b}, R^{10c}, R^{10d}, R^{10e}, R^{10f}, R^{10g}, R^{10h}, R^{11a}, R^{11b}, R^{11c}, R^{11d}, R^{11e}, R^{11f}, R^{11g}, R^{12a}, R^{12b}, R^{15a}, R^{15b}, R^{15c}, R^{15d}, R^{15e}, R^{15f}, R^{15g}, R^{15h}, R^{16a}, R^{16b}, R^{16c}, R^{16d}, R^{16e}, R^{16f}, R^{16g}, R^{17a}, and R^{17b} are the same or different, each independently a hydrogen atom or a C₁₋₆ alkyl group (wherein the group is optionally substituted with 1 to 3 of the same or different substituents selected from a hydroxyl group, a cyano group, a C₁₋₆ alkoxy group, and -NR^{18a}R^{18b}), and
R^{18a} and R^{18b} are the same or different, each independently a hydrogen atom or a C₁₋₆ alkyl group.

"C₁₋₆" means that the number of carbon atoms is 1 to 6. The same applies to other numbers. For example, "C₁₋₄" means that the number of carbon atoms is 1 to 4.

A "heteroatom" refers to an oxygen atom, a nitrogen atom, a sulfur atom, or the like.

A "halogen atom" refers to a fluorine atom, chlorine atom, bromine atom, or iodine atom, preferably a fluorine atom or chlorine atom, and still more preferably a fluorine atom. A "halogen atom" is also referred to as "halogen".

"C₁₋₆ alkyl group" refers to a linear or branched saturated hydrocarbon group with 1 to 6 carbon atoms. "C₁₋₆ alkyl group" is preferably a "C₁₋₄ alkyl group", more preferably a "C₁₋₃ alkyl group", and still more preferably a "C₁₋₂ alkyl group". Specific examples of "C₁₋₆ alkyl group" include, but are not limited to, methyl, ethyl, propyl, butyl, isopropyl, isobutyl, tert-butyl, sec-butyl, isopentyl, neopentyl, tert-pentyl, 1,2-dimethylpropyl, and the like.

"C₂₋₆ alkenyl group" refers to a linear or branched unsaturated hydrocarbon group with 2 to 6 carbon atoms, comprising one or two or more carbon-carbon double bonds. "C₂₋₆ alkenyl group" is preferably a "C₂₋₄ alkenyl group". Specific examples of "C₂₋₆ alkenyl group" include, but are not limited to, a vinyl group, 1-propylenyl group, 2-propylenyl group, 1-butenyl group, 2-butenyl group, 3-butenyl group, 2-methyl-1-propylenyl group, 2-methyl-2-propylenyl group, and the like.

"C₂₋₆ alkynyl group" refers to a linear or branched unsaturated aliphatic hydrocarbon group comprising one or two or more triple bonds. "C₂₋₆ alkynyl group" is preferably a "C₂₋₄ alkynyl group". Specific examples thereof include, but are not limited to, an ethynyl group, 1-propynyl group, 2-propynyl group, 1-butynyl group, 1-methyl-2-propynyl group, 3-butynyl group, 1-pentynyl group, 1-hexynyl group, and the like.

"C₃₋₂₀ alicyclic group" refers to a monocyclic or bicyclic non-aromatic hydrocarbon ring with 3 to 20 carbon atoms, including those with a partially unsaturated bond, those with a partially crosslinked structure, those that have a partially spiro form, and those having one or two or more carbonyl structures. "Alicyclic group" encompasses cycloalkyl groups, cycloalkenyl groups, and cycloalkynyl groups. "C₃₋₂₀ alicyclic group" is preferably a "C₃₋₁₀ alicyclic group", and more preferably a "C₃₋₆ alicyclic group". Specific examples of "C₃₋₂₀ alicyclic group" include, but are not limited to, a cyclopropyl group, cyclobutyl group, cyclopentyl group, cyclohexyl group, cycloheptyl group, cyclooctyl group, cyclohexadinyl group, cycloheptadinyl group, cyclooctadinyl group, adamantyl, norbornyl, and the like.

Specific examples of "C₃₋₂₀ alicyclic group" with a partially crosslinked structure include, but are not limited to, those with a structure shown below and the like.

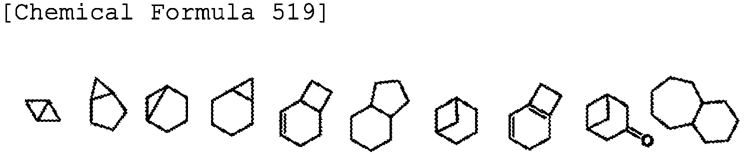

"C₃₋₂₀ alicyclic group" also encompasses compounds fused to an aromatic ring. Specific examples thereof include the groups represented by the following and the like.

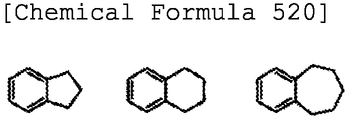

"C₃₋₁₀ alicyclic group" and "C₃₋₆ alicyclic group" refer to the "C₃₋₂₀ alicyclic group" described above wherein the "C₃₋₁₀ alicyclic group" and "C₃₋₆ alicyclic group" are a monovalent group, respectively.

"C₆₋₁₀ aryl" refers to a monocyclic or bicyclic aromatic hydrocarbon ring with 6 to 10 carbon atoms. Specific examples thereof include, as C₆ aryl, a phenyl group, and, as C₁₀ aryl, 1-naphthyl group, 2-naphthyl group, and the like. Preferred C₆₋₁₀ aryl includes C₆ aryl and C₁₀ aryl.

"Heteroaryl" refers to an aromatic heterocycle comprising one or more of the same or different heteroatoms selected from the group consisting of an oxygen atom, a nitrogen atom, and a sulfur atom. Hereroaryl is preferably the "5- or 6-membered heteroaryl", "5- to 10-membered heteroaryl", or "9- or 10-membered heteroaryl" described below.

"5- or 6-membered heteroaryl" refers to a monocyclic aromatic heterocycle consisting of 5 to 6 atoms, comprising 1 to 4 of the same or different heteroatoms selected from the group consisting of an oxygen atom, a nitrogen atom, and a sulfur atom. "5- or 6-membered heteroaryl" is also referred to as a "5- to 6-membered heteroaryl ring".

"5- to 10-membered heteroaryl" refers to a monocyclic or bicyclic aromatic heterocycle consisting of 5 to 10 atoms, comprising 1 to 4 of the same or different heteroatoms selected from the group consisting of an oxygen atom, a nitrogen atom, and a sulfur atom.

"9- or 10-membered heteroaryl" refers to a bicyclic aromatic heterocycle consisting of 9 to 10 atoms, comprising 1 to 4 of the same or different heteroatoms selected from the group consisting of an oxygen atom, a nitrogen atom, and a sulfur atom.

"5- or 6-membered nitrogen-containing heteroaryl" refers to a monocyclic aromatic heterocycle consisting of 5 to 6 atoms, comprising 0 to 3 of the same or different heteroatoms selected from the group consisting of an oxygen atom, a nitrogen atom, and a sulfur atom, in addition to 1 nitrogen atom. "5- or 6-membered nitrogen-containing heteroaryl" is also referred to as a "5- to 6-membered nitrogen-containing heteroaryl ring".

Specific examples of "6-membered heteroaryl" include, but are not limited to, pyridine, pyridazine, pyrimidine, pyrazine, and the like.

Specific examples of "5-membered heteroaryl" include, but are not limited to, thiophene, pyrrole, thiazole, isothiazole, pyrazole, imidazole, furan, oxazole, isoxazole, oxadiazole, thiadiazole, triazole, tetrazole, and the like. 5-membered heteroaryl is preferably triazole or imidazole, and more preferably imidazole.

Specific examples of "5- or 6-membered heteroaryl" include the specific examples for "5-membered heteroaryl" and "6-membered heteroaryl" described above.

Specific examples of "9-membered heteroaryl" include, but are not limited to, indole, isoindole, indazole, benzimidazole, imidazopyridine, benzothiazole, azaindole, purine, and the like.

Specific examples of "10-membered heteroaryl" include, but are not limited to, quinoline, isoquinoline, quinazoline, phthalazine, and the like.

Specific examples of "9- or 10-membered heteroaryl" include the specific examples for the "9-membered heteroaryl" and "10-membered heteroaryl" described above.

"Optionally substituted heteroaryl" can be substituted with a substituent in "optionally substituted" described above. For example, pyridine, when substituted with a hydroxyl group at position 2, has the following tautomers 2-hydroxypyridine and 2-pyridone.

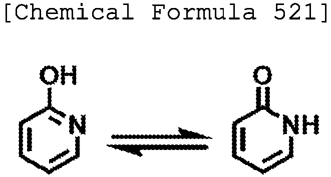

Thus, 2-pyridone is also encompassed by "optionally substituted heteroaryl" herein. Heteroaryl may form a condensed ring with an alicyclic group, aryl or a non-aryl heterocycle.

"Non-aryl heterocycle" refers to a monocyclic or bicyclic nonaromatic heterocycle comprising one or two or more of the same or different heteroatoms selected from the group consisting of an oxygen atom, a nitrogen atom, and a sulfur atom, including those with a partially unsaturated bond, those with a partially crosslinked structure, and those that have a partially spiro form. A non-aryl heterocycle may form a condensed ring with aryl or heteroaryl and is preferably the following "4- to 20-membered non-aryl heterocycle".

"4- to 20-membered non-aryl heterocycle" refers to a monocyclic or bicyclic non-aromatic heterocycle comprised of 4 to 20 atoms, comprising one or two or more of the same or different heteroatoms selected from the group consisting of an oxygen atom, a nitrogen atom, and a sulfur atom, including those with a partially unsaturated bond, those with a partially crosslinked structure, and those that have a partially spiro form. A non-aryl heterocycle may form a condensed ring with aryl or heteroaryl. When fused to, for example, C₆₋₁₀ aryl or 5- or 6-membered heteroaryl, such a heterocycle is still encompassed by a non-aryl heterocycle. Such a heterocycle may comprise one or two or more carbonyl, thiocarbonyl, sulfinyl, or sulfonyl to make up the non-aryl heterocycle. For example, lactam, thiolactam, lactone, thiolactone, cyclic imide, cyclic carbamate, cyclic thiocarbamate, and other cyclic groups are also encompassed by said non-aryl heterocycle. In this regard, oxygen atoms of carbonyl, sulfinyl, and sulfonyl and sulfur atoms of thiocarbonyl are not included in the number of 4 to 20 members (size of ring) or the number of heteroatoms constituting the ring. Specific examples of "4- to 20-membered non-aryl heterocycle" include, but are not limited to, azetidine, pyrrolidine, piperidine, piperazine, morpholine, homopiperidine, oxetane, tetrahydrofuran, tetrahydropyran, and the like, those with a structure shown below, and the like.

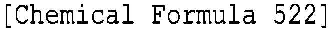

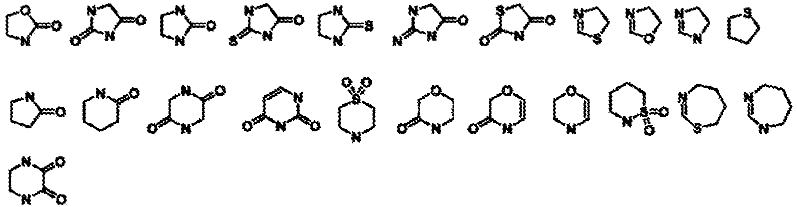

Specific examples of "4- to 20-membered non-aryl heterocycle" with partial crosslinking or spiro structure include, but are not limited to, those with a structure shown below and the like.

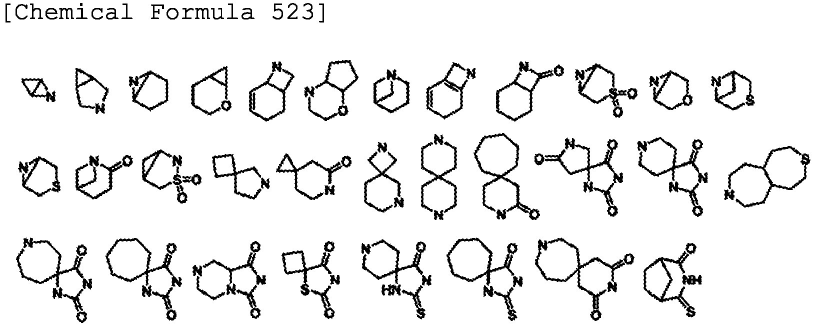

"Nitrogen-containing non-aryl heterocycle" refers to a monocyclic or bicyclic nonaromatic heterocycle comprising 0 or 1 or more of the same or different heteroatoms selected from the group consisting of an oxygen atom, a nitrogen atom, and a sulfur atom, in addition to one nitrogen atom, including those with a partially unsaturated bond, those with a partially crosslinked structure, and those that have a partially spiro form and is preferably the following "4- to 20-membered nitrogen-containing non-aryl heterocycle".

"4- to 20-membered nitrogen-containing non-aryl heterocycle" refers to a monocyclic or bicyclic non-aromatic heterocycle comprised of 4 to 20 atoms, comprising 0 or 1 of the same or different heteroatoms selected from the group consisting of an oxygen atom, a nitrogen atom, and a sulfur atom, in addition to 1 nitrogen atom, including those with a partially unsaturated bond, those with a partially crosslinked structure, and those that have a partially spiro form.

"4- to 10-membered non-aryl heterocycle" refers to the "4- to 20-membered non-aryl heterocycle" described above wherein "4- to 10-membered non-aryl heterocycle" is a substituent that is a monovalent group.

"4- to 10-membered nitrogen-containing non-aryl heterocycle", "4- to 7-membered nitrogen-containing non-aryl heterocycle", "5-to 7-membered nitrogen-containing non-aryl heterocycle", and "4-to 6-membered nitrogen-containing non-aryl heterocycle" refer to the "4- to 20-membered nitrogen-containing non-aryl heterocycle" described above wherein the "4- to 10-membered nitrogen-containing non-aryl heterocycle", "4- to 7-membered nitrogen-containing non-aryl heterocycle", "5- to 7-membered nitrogen-containing non-aryl heterocycle", and " 4- to 6-membered nitrogen-containing non-aryl heterocycle" are substituents that are a monovalent group, respectively.

"5- to 7-membered non-aryl heterocycle" refers to the "4- to 20-membered non-aryl heterocycle" described above wherein "5- to 7-membered non-aryl heterocycle" is a substituent that is a monovalent group.

"4- to 7-membered non-aryl heterocycle" and "4- to 6-membered non-aryl heterocycle" refer to the "4- to 20-membered non-aryl heterocycle" described above wherein "4- to 7-membered non-aryl heterocycle" and "4- to 6-membered non-aryl heterocycle" are substituents that are a monovalent group, respectively.

Specific examples of "4-membered non-aryl heterocycle" include, but are not limited to, azetidine, oxetane, thietane, and the like.

Specific examples of "4-membered non-aryl heterocycle" with a partially unsaturated bond include, but are not limited to, those with a structure shown below and the like.

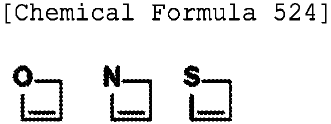

Specific examples of "5-membered non-aryl heterocycle" include, but are not limited to, pyrrolidine, pyrrolidone, oxazolidinone, tetrahydrofuran, tetrahydrothiophene, and the like.

Specific examples of "5-membered non-aryl heterocycle" with a partially unsaturated bond include, but are not limited to, those with a structure shown below and the like.

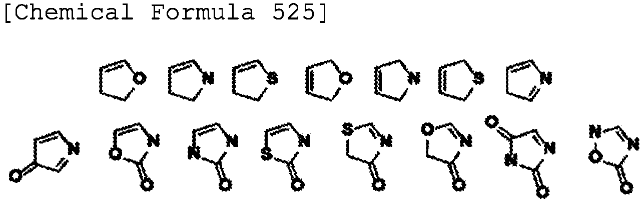

Specific examples of "5-membered non-aryl heterocycle" with a partially crosslinked structure include, but are not limited to, those with a structure shown below and the like.

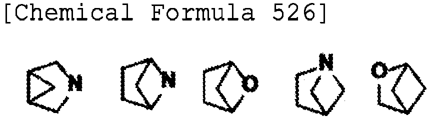

Specific examples of "5-membered non-aryl heterocycle" comprising carbonyl, thiocarbonyl, or the like include, but are not limited to, those with a structure shown below and the like.

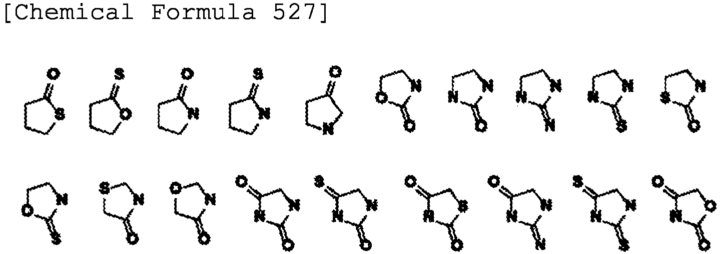

Specific examples of "6-membered non-aryl heterocycle" include, but are not limited to, piperidine, piperazine, morpholine, tetrahydropyran, tetrahydrothiopyran, and the like.

Specific examples of "6-membered non-aryl heterocycle" with a partially unsaturated bond include, but are not limited to, those with a structure shown below and the like.

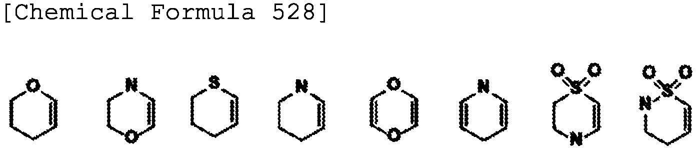

Specific examples of "6-membered non-aryl heterocycle" with a partially crosslinked structure include, but are not limited to, those with a structure shown below and the like.

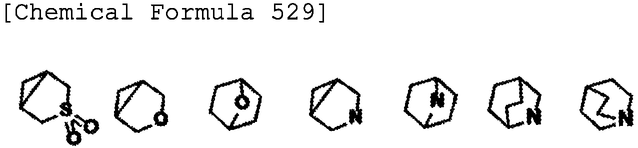

"C₁₋₆ alkoxy group" refers to a "C₁₋₆ alkyloxy group", and the C₁₋₆ alkyl moiety is defined the same as the C₁₋₆ alkyl group described above. "C₁₋₆ alkoxy group" is preferably a "C₁₋₄ alkoxy group", more preferably a "C₁₋₃ alkoxy group", and still more preferably a "C₁₋₂ alkoxy group". Specific examples of "C₁₋₆ alkoxy group" include, but are not limited to, a methoxy group, ethoxy group, propoxy group, butoxy group, isopropoxy group, isobutoxy group, tert-butoxy group, sec-butoxy group, isopentyloxy group, neopentyloxy group, tert-pentyloxy group, 1,2-dimethylpropoxy group, and the like.

"C₃₋₁₀ alicyclic oxy group" refers to a (C₃₋₁₀ alicyclic group) - O- group, and the C₃₋₁₀ alicyclic moiety is defined the same as a C₃₋₁₀ alicyclic group. "C₃₋₆ alicyclic oxy group" refers to a (C₃₋₆ alicyclic group)-O- group, and the C₃₋₆ alicyclic moiety is defined the same as a C₃₋₆ alicyclic group. "C₃₋₆ alicyclic oxy group" is preferably a "C₃₋₅ alicyclic oxy group". Specific examples of "C₃₋₆ alicyclic oxy group" include, but are not limited to, a cyclopropyloxy group, cyclobutyloxy group, cyclopentyloxy group, cyclohexyloxy group, and the like.

The C₆₋₁₀ aryl moiety of a "C₆₋₁₀ aryloxy group" is defined the same as the C₆₋₁₀ aryl described above. "C₆₋₁₀ aryloxy group" is preferably a "C₆ or C₁₀ aryloxy group". Specific examples of "C₆₋₁₀ aryloxy group" include, but are not limited to, a phenoxy group, 1-naphthyloxy group, 2-naphthyloxy group, and the like.

The 5- or 6-membered heteroaryl moiety of "5- or 6-membered heteroaryloxy group" is defined the same as the "5-membered heteroaryl" or "6-membered heteroaryl" described above. Specific examples of "5- or 6-membered heteroaryloxy group" include, but are not limited to, a pyrazoyloxy group, triazoyloxy group, thiazoyloxy group, thiadiazoyloxy group, pyridyloxy group, pyridazoyloxy group, and the like.

The 4- to 10-membered non-aryl heterocycle moiety of "4- to 10-membered non-aryl heterocyclyl oxy group" is defined the same as the "4- to 10-membered non-aryl heterocycle" described above. "4- to 10-membered non-aryl heterocyclyl oxy group" is preferably a "4- to 6-membered non-aryl heterocyclyl oxy group". Specific examples of "4- to 10-membered non-aryl heterocyclyl oxy group" include, but are not limited to, a tetrahydrofuranyloxy group, tetrahydropyranyloxy group, azetidinyloxy group, pyrrolidinyloxy group, piperidinyloxy group, and the like.

The C₁₋₆ alkyl moiety of "C₁₋₆ alkylthio group" is defined the same as the C₁₋₆ alkyl described above. "C₁₋₆ alkylthio group" is preferably a "C₁₋₄ alkylthio group", and more preferably a "C₁₋₃ alkylthio group". Specific examples of "C₁₋₆ alkylthio group" include, but are not limited to, a methylthio group, ethylthio group, propylthio group, butylthio group, isopropylthio group, isobutylthio group, tert-butylthio group, sec-butylthio group, isopentylthio group, neopentylthio group, tert-pentylthio group, 1,2-dimethylpropylthio group, and the like.

"C₃₋₁₀ alicyclic thio group" refers to a (C₃₋₁₀ alicyclic group)-S- group, and the C₃₋₁₀ alicyclic moiety is defined the same as the C₃₋₁₀ alicyclic group described above. "C₃₋₁₀ alicyclic thio group" is preferably a "C₃₋₆ alicyclic thio group". Specific examples of "C₃₋₆ alicyclic thio group" include, but are not limited to, a cyclopropylthio group, cyclobutylthio group, cyclopentylthio group, cyclohexylthio group, and the like.

The C₆₋₁₀ aryl moiety of "C₆₋₁₀ arylthio group" is defined the same as the C₆₋₁₀ aryl described above. "C₆₋₁₀ arylthio group" is preferably a "C₆ or C₁₀ arylthio group". Specific examples of "C₆₋₁₀ arylhthio group" include, but are not limited to, a phenylthio group, 1-naphthylthio group, 2-naphthylthio group, and the like.

The 5- or 6-membered heteroaryl moiety of "5- or 6-membered heteroarylthio group" is defined the same as the "5-membered heteroaryl" or "6-membered heteroaryl" described above. Specific examples of "5- or 6-membered heteroarylthio group" include, but are not limited to, a pyrazoylthio group, triazoylthio group, thiazoylthio group, thiadiazoylthio group, pyridylthio group, pyridazoylthio group, and the like.

The 4- to 10-membered non-aryl heterocycle moiety of "4- to 10-membered non-aryl heterocyclyl thio group" is defined the same as the "4- to 10-membered non-aryl heterocycle" described above. "4- to 10-membered non-aryl heterocyclyl thio group" is preferably a "4- to 6-membered non-aryl heterocyclyl thio group". Specific examples of "4- to 10-membered non-aryl heterocyclyl thio group" include, but are not limited to, a tetrahydropyranylthio group, piperidinylthio group, and the like.

"C₁₋₆ alkylcarbonyl group" refers to a carbonyl group substituted with the "C₁₋₆ alkyl group" described above. "C₁₋₆ alkylcarbonyl group" is preferably a "C₁₋₄ alkylcarbonyl group". Specific examples of "C₁₋₆ alkylcarbonyl group" include, but are not limited to, an acetyl group, propionyl group, butyryl group, and the like.

"C₃₋₁₀ alicyclic carbonyl group" refers to a carbonyl group substituted with the "C₃₋₁₀ alicyclic group" described above. "C₃₋₁₀ alicyclic carbonyl group" is preferably a "C₃₋₆ alicyclic carbonyl group". Specific examples of "C₃₋₁₀ alicyclic carbonyl group" include, but are not limited to, a cyclopropylcarbonyl group, cyclopentylcarbonyl group, and the like.

"C₆₋₁₀ arylcarbonyl group" refers to a carbonyl group substituted with the "C₆₋₁₀ aryl" described above. "C₆₋₁₀ arylcarbonyl group" is preferably a "C₆ or C₁₀ arylcarbonyl group". Specific examples of "C₆₋₁₀ arylcarbonyl group" include, but are not limited to, a benzoyl group, 1-naphthylcarbonyl group, 2-naphthylcarbonyl group, and the like.

"5- or 6-membered heteroarylcarbonyl group" refers to a carbonyl group substituted with the "5- or 6-membered heteroaryl" described above. Specific examples of "5- or 6-membered heteroarylcarbonyl group" include, but are not limited to, a pyrazoylcarbonyl group, triazoylcarbonyl group, thiazoylcarbonyl group, thiadiazoylcarbonyl group, pyridylcarbonyl group, pyridazoylcarbonyl group, and the like.

"4- to 10-membered non-aryl heterocyclyl carbonyl group" refers to a carbonyl group substituted with the "4- to 10-membered non-aryl heterocycle" described above. "4- to 10-membered non-aryl heterocyclyl carbonyl group" is preferably a "4- to 6-membered non-aryl heterocyclyl carbonyl group". Specific examples of "4- to 10-membered non-aryl heterocyclyl carbonyl group" include, but are not limited to, an azetidinylcarbonyl group, pyrrolidinylcarbonyl group, piperidinylcarbonyl group, morpholinylcarbonyl group, and the like.

"C₁₋₆ alkylsulfonyl group" refers to a sulfonyl group substituted with the "C₁₋₆ alkyl group" described above. "C₁₋₆ alkylsulfonyl group" is preferably a "C₁₋₄ alkylsulfonyl group". Specific examples of "C₁₋₆ alkylsulfonyl group" include, but are not limited to, a methylsulfonyl group, propylsulfonyl group, butylsulfonyl group, and the like.

"C₃₋₁₀ alicyclic sulfonyl group" refers to a sulfonyl group substituted with the "C₃₋₁₀ alicyclic group" described above. "C₃₋₁₀ alicyclic sulfonyl group" is preferably a "C₃₋₆ alicyclic sulfonyl group". Specific examples of "C₃₋₁₀ alicyclic sulfonyl group" include, but are not limited to, a cyclopropylsulfonyl group, cyclobutylsulfonyl group, cyclopentylsulfonyl group, cyclohexylsulfonyl group, and the like.

"C₆₋₁₀ arylsulfonyl group" refers to a sulfonyl group substituted with the "C₆₋₁₀ aryl" described above. "C₆₋₁₀ arylsulfonyl group" is preferably a "C₆ or C₁₀ arylsulfonyl group". Specific examples of "C₆₋₁₀ arylsulfonyl group" include, but are not limited to, a phenylsulfonyl group, 1-naphthylsulfonyl group, 2-naphthylsulfonyl group, and the like.

"5- or 6-membered heteroarylsulfonyl group" refers to a sulfonyl group substituted with the "5- or 6-membered heteroaryl" described above. Specific examples of "5- or 6-membered heteroarylsulfonyl group" include a pyrazoylsulfonyl group, triazoylsulfonyl group, thiazoylsulfonyl group, thiadiazoylsulfonyl group, pyridylsulfonyl group, pyridazoylsulfonyl group, and the like.

"C₁₋₆ alkylene group" refers to a substituent that is a divalent group due to removing two hydrogen atoms from saturated hydrocarbon with 1 to 6 carbon atoms. "C₁₋₃ alkylene group" and "C₂₋₄ alkylene group" refer to substituents that are divalent groups due to removing two hydrogen atoms from saturated hydrocarbon with 1 to 3 carbon atoms and 2 to 4 carbon atoms, respectively.

"C₃₋₁₀ cycloalkylene group" refers to a substituent that is a divalent group due to removing two hydrogen atoms from saturated cyclic hydrocarbon with 3 to 10 carbon atoms. "C₃₋₆ cycloalkylene group" and "C₄₋₆ cycloalkylene group" refer to substituents that are divalent groups due to removing two hydrogen atoms from saturated cyclic hydrocarbon with 3 to 6 carbon atoms and 4 to 6 carbon atoms, respectively.

"C₆₋₁₀ arylene group" refers to a substituent that is a divalent group due to removing two hydrogen atoms from aromatic hydrocarbon with 6 to 10 carbon atoms. "C₆ arylene group" refers to a substituent that is a divalent group due to removing two hydrogen atoms from aromatic hydrocarbon with 6 carbon atoms.

"5- or 6-membered heteroarylene group" refers to a substituent that is a divalent group due to removing two hydrogen atoms from a 5- or 6-membered heteroaryl ring. "5-membered heteroarylene group" and "6-membered heteroarylene group" refer to substituents that are divalent groups due to removing two hydrogen atoms from 5-membered and 6-membered heteroaryl rings, respectively.

"4- to 10-membered non-aryl heterocyclylene group" refers to a substituent that is a divalent group due to removing two hydrogen atoms from a 4- to 10-membered non-aryl heterocycle. "4-to 5-membered non-aryl heterocyclylene group" and "4- to 6-membered non-aryl heterocyclylene group" refer to substituents that are divalent groups due to removing two hydrogen atoms from 4- to 5-membered and 4- to 6-membered non-aryl heterocycles, respectively.

"Hydrocarbyl group", as used herein, refers to a monovalent group produced by removing a hydrogen atom from hydrocarbon consisting of only a carbon atom and hydrogen atoms. Hydrocarbyl can be aliphatic (linear or branched), alicyclic group, or aromatic (aryl). Examples thereof include a methyl group, an ethyl group, an n-propyl group, an isopropyl group, a cyclopentyl group, a cyclohexyl group, a phenyl group, and the like. "Optionally substituted hydrocarbyl group" refers to a hydrocarbyl group that is substituted with a substituent described above. "Optionally substituted hydrocarbyl group" is optionally substituted with an oxo group (=O group), a thioxo group (=S group), or an imino group (=NR). Examples thereof include a formyl group, an acety group, and the like.

"Hydrocarbylene group", as used herein, refers to a divalent group produced by removing two hydrogen atoms from hydrocarbon consisting of only a carbon atom and hydrogen atoms. Hydrocarbylene can be aliphatic (linear or branched), alicyclic group, or aromatic (aryl). Examples thereof include a methylene group, an ethylene group, an n-propylene group, an isopropylene group, a cyclopentandiyl group, a cyclohexandiyl group, a phenylene group, and the like. "Optionally substituted hydrocarbylene group" refers to a hydrocarbylene group that is substituted with a substituent described above. "Optionally substituted hydrocarbylene group" is optionally substituted with an oxo group (=O group), a thioxo group (=S group), or an imino group (=NR).

"Heterohydrocarbyl group", as used herein, refers to a group wherein at least one carbon atom (may be all carbon atoms) of a hydrocarbyl group is substituted with a heteroatom. Examples of heteroatom include O, N, S, and P. Examples of heterohydrocarbyl group include heteroaryl, non-aryl heterocycle, alkoxy group, alkylthio group, carboxy group, carboxylic acid isostere (wherein the carboxylic acid isostere comprises an ester group-C (=O)OR^{20a}), -NR^{e1}-C (=O)R^{f1}, -NR^{e1}-C (=O)OR^{f1}, -NR^{g1}-C (=O)NR^{e1}R^{f1}-C(=O)NR⁵⁰R⁵¹-C(=O)NR⁵⁰R⁵¹ (wherein R^{20a}, R^{e1}, R^{f1}, R^{g1}, R⁵⁰, and R⁵¹ are defined the same as item 1 or 2 herein), and the like. Examples thereof include a methoxy group, an ethoxy group, a cyano group, an imidazole group, a pyridinyl group, a pyrazole group, a triazole group, a pyrazinyl group, a pyridazinyl group, a pyrimidinyl group, and the like. "Optionally substituted heterohydrocarbyl group" refers to a heterohydrocarbyl group that is substituted with a substituent described above. "Optionally substituted heterohydrocarbyl group" is optionally substituted with an oxo group (=O group), a thioxo group (=S group), or an imino group (=NR). Examples thereof include a carboxyl group, a methoxycarbonyl group, an aminocarbonyl group, a hydroxyaminocarbonyl group, and the like.

"Heterohydrocarbylene group", as used herein, refers to a group wherein at least one carbon atom (may be all carbon atoms) of a hydrocarbylene group is substituted with a heteroatom. Examples of heteroatom include O, N, S, and P. Examples of heterohydrocarbylene group include heteroarylene, a non-aryl heterocyclyl diyl group, an alkyleneoxy group, an alkylenethio group, and the like. Examples thereof include a methyleneoxy group, an ethyleneoxy group, an imino group (-NR- group), an imidazole diyl group, a pyridine diyl group, a pyrazole diyl group, a triazole diyl group, a pyradine diyl group, a pyridazine diyl group, a pyrimidine diyl group, and the like. "Optionally substituted heterohydrocarbylene group" refers to a heterohydrocarbylene group substituted with a substitutent described above. "Optionally substituted heterohydrocarbylene group" is optionally substituted with an oxo group (=O group), a thioxo group (=S group), or an imino group (=NR). Examples thereof include a carbonyloxy group, a methyleneoxycarbonyl group, an aminocarbonyl group, a hydroxyaminocarbonyl group, an iminocarbonyl group (-NRC(=O)- group), an iminosulfonyl group (-NRSO₂- group), optionally substituted -NH-, optionally substituted -NH-SO₂-, and the like.

A bond intersecting a wavy line in the description of a specific structure of R⁵ indicates a bond with L⁴. A bond intersecting a bond between ring atoms means that there are variables (e.g., R^{6a}, R^{7a}, and the like) at each of the substitutable positions on a monocycle or fused polycycle including the ring atoms. For example, for a monocyclic 5-membered ring (heteroaryl), 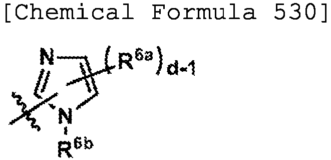 (wherein d is 3) is one of 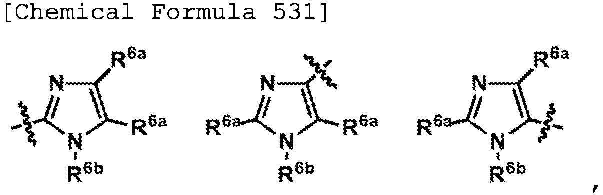 and L⁴ attaches to a ring carbon atom of the 5-membered ring. For example, for a monocyclic 6-membered ring (heteroaryl), 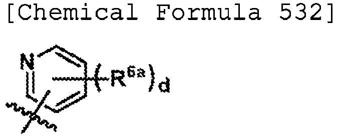 (wherein d is 4) is one of 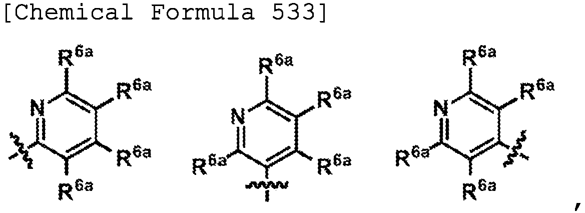 and L⁴ attaches to a ring carbon atom of the 6-membered ring.

Alternatively, for example, for a monocyclic 5-membered ring (non-aryl heterocycle), 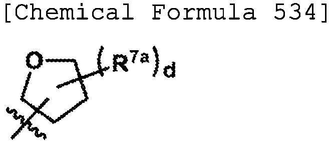 (wherein d is 7) is one of 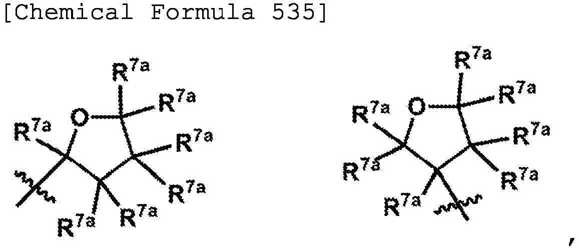 and L⁴ attaches to a ring carbon atom of the 5-membered ring. For example, for a monocyclic 6-membered ring (non-aryl heterocycle), 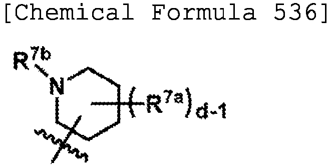 (wherein d is 10) is one of 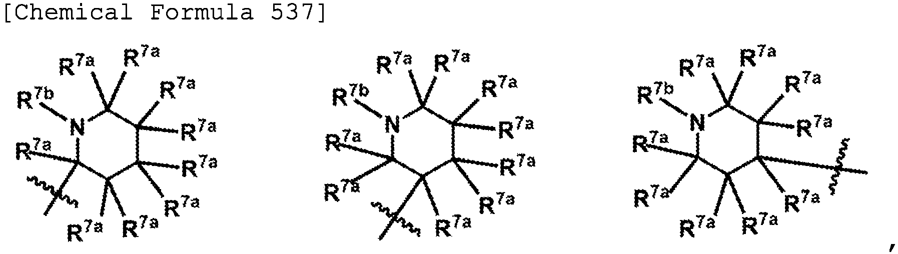 and L⁴ attaches to a ring carbon atom of the 6-membered ring.

Subscript d is the number of substitutable positions on a ring of R⁵, but is a number of substitutable positions excluding the attachment position to L⁴.

"Bioisostere" refers to another partial structure (functional group) serving the same biological role as a group (e.g., carboxyl group) in a drug molecule (prodrug structures are also encompassed as a concept of a bioisostere in the present invention). "Carboxylic acid isostere" refers to a bioisostere of carboxylic acid. Examples of the carboxylic acid isostere include, but are not limited to, an ester group-C (=O)OR^{20a}, -SO₃H, -SO₂NHR^{19a}, - B(OR^{m1})₂, -PO(OR^{m1})(OR^{m2}), -CONHR^{19a}, -CONHSO₂R^{19a}, -CONR^{19a}CN, - CONHNHSO₂R^{19a}, and substituents represented by the formulas (8A), (8B), (8C), (8D), (8E), (8F), (8G), (8H), (8I), (8J), (8K), (8L), (8M), (8N), (8O), (8P), (8Q), (8R), (8S), (8T), (8U), (8V), and (8W) described below (each of the substituents is further optionally substituted with 1 to 3 of the same or different R^{19b} at a chemical substitutable position), 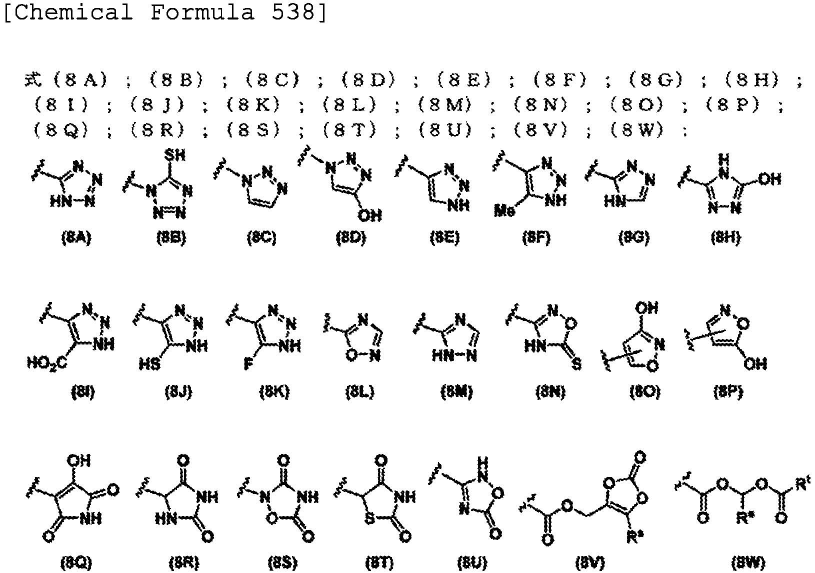 wherein [in (8V) and (8W),
R^{s} is a hydrogen atom, a C₁₋₆ alkyl group, or a C₃₋₁₀ alicyclic group (wherein the C₁₋₆ alkyl group or C₃₋₁₀ alicyclic group is optionally substituted with 1 to 5 halogen atoms),
R^{t} is a hydrogen atom, a C₁₋₆ alkyl group, a C₁₋₆ alkoxy group, (wherein the C₁₋₆ alkyl group or C₁₋₆ alkoxy group is optionally substituted with 1 to 5 halogen atoms), a C₃₋₁₀ alicyclic group, a C₃₋₁₀ alicyclic oxy group, a phenyl group, a phenoxy group, a pyridyl group, or a pyridyloxy group (wherein the C₃₋₁₀ alicyclic group, C₃₋₁₀ alicyclic oxy group, phenyl group, phenoxy group, pyridyl group, or pyridyloxy group is optionally substituted with 1 to 5 substituents selected from the group consisting of a halogen atom, a C₁₋₆ alkyl group, and a C₁₋₆ alkoxy group)],
R^{19a} and R^{19b} are the same or different, each independently representing a hydrogen atom, a hydroxyl group, a C₁₋₆ alkyl group, C₆₋₁₀ aryl, 5- or 6-membered heteroaryl, or a 4- to 10-membered non-aryl heterocycle,
R^{20a} is
   1) a C₁₋₆ alkyl group,
   2) a C₃₋₁₀ alicyclic group,
   3) C₆₋₁₀ aryl,
   4) 5- or 6-membered heteroaryl, or
   5) a 4- to 10-membered non-aryl heterocycle,
      (wherein each substituent from 1) to 5) is optionally substituted),
R^{m1} represents
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
wherein if R^{m1} is attached to a boron atom via an oxygen atom, two R^{m1}, as C₂₋₄ alkylene, together with the boron atom and two oxygen atoms, may form a 5- to 7-membered non-aryl heterocycle (wherein an alkylene moiety is optionally substituted in the non-aryl heterocycle), and
R^{m2} represents a hydrogen atom, an optionally substituted C₁₋₆ alkyl group, or an optionally substituted C₃₋₁₀ alicyclic group, wherein, preferably,
R^{s} is a hydrogen atom or a C₁₋₆ alkyl group, and
R^{t} is a hydrogen atom, a C₁₋₆ alkyl group, a C₁₋₆ alkoxy group, a C₃₋₁₀ alicyclic group, or a C₃₋₁₀ alicyclic oxy group, or alternatively preferably,
R^{19a} and R^{19b} are the same or different, each independently a hydrogen atom, a hydroxyl group, or a C₁₋₆ alkyl group, or also preferably
R^{m1} and R^{m2} are the same or different, each independently a hydrogen atom, a C₁₋₆ alkyl group, or a C₃₋₁₀ alicyclic group.

An example of an embodiment of the compounds of the invention includes a compound represented by formula (1a) or (1b): 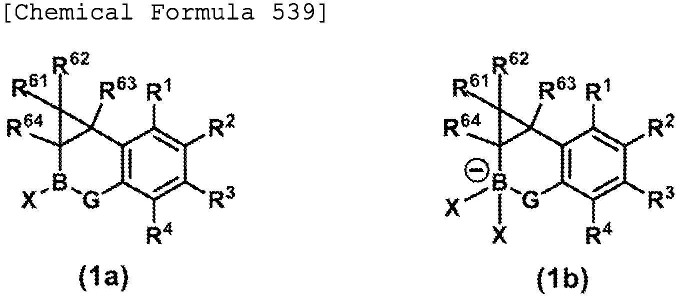 or a pharmaceutically acceptable salt thereof,
wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 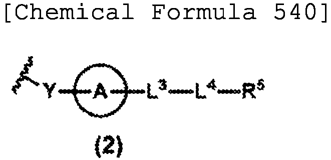 wherein
Y is an oxygen atom, a sulfur atom, or -NR^{j-}, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, optionally substituted -NH-, optionally substituted -NH-SO₂-, -S(=O)-, or -S(=O)₂-,
L⁴ is a single bond, an optionally substituted hydrocarbylene group, an optionally substituted heterohydrocarbylene group, or - C(=N-OR^{h1})-,
R^{h1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
R⁵ is a hydrogen atom, a hydroxyl group, an optionally substituted hydrocarbyl group, an optionally substituted heterohydrocarbyl group, optionally substituted -NHOH, a sulfo group (sulfonic acid group), optionally substituted -SO₂H, optionally substituted -SO₂-NH₂, optionally substituted - S(=O)(=NH)H, optionally substituted -NH-SO₂-H, optionally substituted -NH-SO₂-NH₂, optionally substituted -N=S(=O)H₂, or optionally substituted -NH₂,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
   1) -C(=O)R⁸,
   2) -SO₂-L⁶-R⁸,
      (wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene group),
   3) -NR^{a4}R^{b3},
   4) -B(OR^{m1})₂,
   5) -PO(OR^{m1})(OR^{m2}),
   6) optionally substituted heteroaryl,
   7) an optionally substituted non-aryl heterocycle, or
   8) a bioisostere of one of 1) to 7),
      (wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

Another example of an embodiment of the compounds of the invention includes a compound represented by (1a) or (1b): 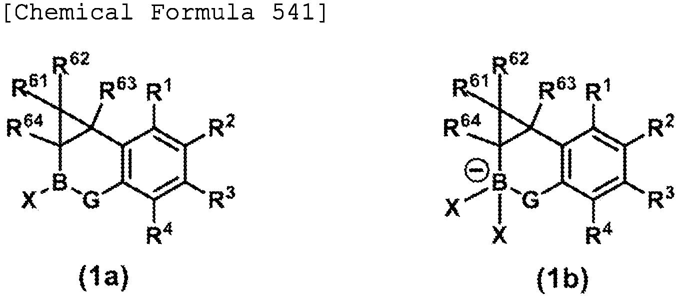 or a pharmaceutically acceptable salt thereof,
wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 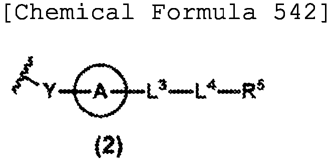 wherein
Y is an oxygen atom, a sulfur atom, or -NR^{j}-, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, -S(=O)- or -S(=O)₂-,
L⁴ is a single bond, an optionally substituted hydrocarbylene group, or -C(=N-OR^{h1})-,
R^{h1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
R⁵ is a hydrogen atom, a hydroxyl group, an optionally substituted hydrocarbyl group, or an optionally substituted heterohydrocarbyl group,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
   1) -C(=O)R⁸,
   2) -SO₂-L⁶-R⁸,
      (wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene group),
   3) -NR^{a4}R^{b3},
   4) -B(OR^{m1})₂,
   5) -PO(OR^{m1})(OR^{m2}),
   6) optionally substituted heteroaryl,
   7) an optionally substituted non-aryl heterocycle, or
   8) a bioisostere of one of 1) to 7),
      (wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

In some embodiments, in formula (1a) or (1b),
G is an oxygen atom, a sulfur atom, or -NR^{a1}-,
X is a hydroxyl group, an optionally substituted C₁₋₆ alkoxy group, or -NR^{a2}R^{b1},
R^{a1}, R^{a2}, and R^{b1} are the same or different, each independently
   1) a hydrogen atom,
   2) a C₁₋₆ alkyl group,
   3) a C₃₋₁₀ alicyclic group,
   4) C₆₋₁₀ aryl
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group, or
   15) -OR^{c1},
      (wherein each substituent from 2) to 14) is optionally substituted),
wherein R^{a2} and R^{b1}, together may form an optionally substituted 4- to 10-membered nitrogen-containing non-aryl heterocycle,
R^{c1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
L¹ is a single bond, an oxygen atom, a sulfur atom, -SO-, - SO₂-, -NR^{d1}-, -NR^{d1}C(=O)-, or -NR^{d1}SO₂-,
L² is a single bond or an optionally substituted C₁₋₆ alkylene group,
Z is
   1) a hydrogen atom,
   2) a hydroxyl group,
   3) a cyano group,
   4) a carboxyl group,
   5) a C₃₋₁₀ alicyclic group,
   6) C₆₋₁₀ aryl,
   7) 5- or 6-membered heteroaryl,
   8) a 4- to 10-membered non-aryl heterocycle,
   9) a C₁₋₆ alkoxy group,
   10) a C₃₋₁₀ alicyclic oxy group,
   11) a C₆₋₁₀ aryloxy group,
   12) a 5- or 6-membered heteroaryloxy group,
   13) a 4- to 10-membered non-aryl heterocyclyl oxy group,
   14) a C₁₋₆ alkylthio group,
   15) a C₃₋₁₀ alicyclic thio group,
   16) a C₆₋₁₀ arylthio group,
   17) a 5- or 6-membered heteroarylthio group,
   18) a 4- to 10-membered non-aryl heterocyclyl thio group,
      (wherein each substituent from 5) to 18) is optionally substituted),
   19) -SO₂-NR^{e1}R^{f1},
   20) -NR^{e1}-C(=O)OR^{f1},
   21) -NR^{g1}-C(=O)NR^{e1}R^{f1},
   22) -NR^{e1}-C(=S)R^{f1},
   23) -NR^{e1}-C(=S)OR^{f1},
   24) -NR^{g1}-C(=S)NR^{e1}R^{f1},
   25) -NR^{g1}-CR^{e1}(=NR^{f1}),
   26) -NR^{g1}-CR^{e1}(=N-OR^{f1}),
   27) -NR^{h1}-C(=NR^{g1})NR^{e1}R^{f1},
   28) -NR^{h1}-C(=N-OR^{g1})NR^{e1}R^{f1},
   29) -NRⁱ¹-C(=NR^{h1})NR^{g1}-NR^{e1}R^{f1},
   30) -NRⁱ¹-C(=N-OR^{h1})NR^{g1}-NR^{e1}R^{f1},
   31) -NR^{e1}-SO₂-R^{f1},
   32) -NR^{g1}-SO₂-NR^{e1}R^{f1},
   33) -C(=O)OR^{e1},
   34) -C(=S)OR^{e1},
   35) -C(=S)NR^{e1}R^{f1},
   36) -C(=S)NR^{e1}OR^{f1},
   37) -C(=S)NR^{g1}-NR^{e1}R^{f1},
   38) -C(=NR^{e1})R^{f1},
   39) -C(=N-OR^{e1})R^{f1},
   40) -C(=NR^{h1})NR^{g1}-NR^{e1}R^{f1},
   41) -C(=N-OR^{h1})NR^{g1}-NR^{e1}R^{f1},
   42) -NR^{e1}R^{f1},
   43) -NR^{g1}-NR^{e1}R^{f1},
   44) -NR^{e1}OR^{f1},
   45) -NR^{e1}-C(=O)R^{f1},
   46) -C(=O)NR^{e1}R^{f1},
   47) -C(=O)NR^{e1}OR^{f1},
   48) -C(=O)NR^{g1}-NR^{e1}R^{f1},
   49) -C(=O)R^{e1},
   50) -C(=NR^{g1})NR^{e1}R^{f1}, or
   51) -C(=N-OR^{h1})NR^{e1}R^{f1},
one of R¹, R², and R³ is a group represented by formula (2) : 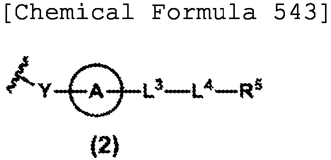 wherein
Y is an oxygen atom, a sulfur atom, or -NR^{j-}, ring A is an optionally substituted 4- to 20-membered non-aryl heterocycle,
L³ is
   1) an oxygen atom,
   2) a sulfur atom,
   3) -NR^{d2}-,
   4) - NR^{d2}C(=O)-,
   5) -NR^{d2}SO₂-,
   6) a C₁₋₆ alkylene group,
   7) a C₃₋₁₀ cycloalkylene group,
   8) a 4- to 10-membered non-aryl heterocyclylene group, (wherein each substituent from 6) to 8) is optionally substituted),
   9) -C(=O)-,
   10) -S(=O)-, or
   11) -S(=O)₂-,
L⁴ is
   1) a single bond,
   2) a C₁₋₆ alkylene group,
   3) a C₃₋₁₀ cycloalkylene group,
   4) a C₆₋₁₀ arylene group,
   5) a 5- or 6-membered heteroarylene group,
   6) a 4- to 10-membered non-aryl heterocyclylene group, (wherein each substituent from 2) to 6) is optionally substituted), or
   7) -C(=N-OR^{h1})-,
R⁵ is
   1) a hydrogen atom,
   2) a C₁₋₆ alkyl group,
   3) a C₃₋₁₀ alicyclic group,
   4) a 4- to 10-membered non-aryl heterocycle,
   5) C₆₋₁₀ aryl,
   6) 5- to 10-membered heteroaryl,
   7) a C₁₋₆ alkylthio group,
      (wherein each substituent from 2) to 7) is optionally substituted, and if two substituents further substituted with the substituent of 1), 2), or 3) are each substituted on adjacent atoms within a ring, the two substituents together may further form a condensed ring structure),
   8) -NR^{e1}OH,
   9) a carboxyl group (-C(=O)OH)
   10) a carboxylic acid isostere (wherein the carboxylic acid isostere comprises an ester group-C(=O)OR^{20a}),
   11) a sulfo group (sulfonic acid group),
   12) -SO₂R^{e1},
   13) -SO₂-NR^{e1}R^{f1},
   14) -S(=O)(=NR^{f1})R^{e1},
   15) -NR^{e1}-C(=O)R^{f1},
   16) -NR^{e1}-C(=O)OR^{f1},
   17) -NR^{g1}-C(=O)NR^{e1}R^{f1},
   18) -NR^{e1}-SO₂-R^{f1},
   19) -NR^{g1}-SO₂-NR^{e1}R^{f1},
   20) -N=S(=O)R^{e1}R^{f1},
   21) -C(=O)NR⁵⁰R⁵¹, or
   22) -NR^{e1}R^{f1} (wherein if R⁵ is the substituent of 22), -L³-L⁴-R⁵ is not -(CH₂)₁₋₄NR^{e1}R^{f1} (wherein R^{e1} and R^{f1} are a hydrogen atom, optionally substituted C₁₋₄ alkyl, an optionally substituted C₃₋₇ alicyclic group, an optionally substituted 4- to 10-membered non-aryl heterocyclic group, optionally substituted C₆₋₁₀ aryl, or optionally substituted 5- to 10-membered heteroaryl)),
R20a is
   1) a C₁₋₆ alkyl group,
   2) a C₃₋₁₀ alicyclic group,
   3) C₆₋₁₀ aryl,
   4) 5- or 6-membered heteroaryl, or
   5) a 4- to 10-membered non-aryl heterocycle,
      (wherein each substituent from 1) to 5) is optionally substituted),
R⁵⁰ represents
   1) a hydrogen atom,
   2) a C₁₋₆ alkyl group,
   3) a hydroxyl group,
   4) a C₁₋₆ alkoxy group,
   5) a C₃₋₆ cycloalkoxy group,
   6) a C₃₋₆ alicyclic group,
   7) a 4- to 6-membered non-aryl heterocycle,
   8) C₆₋₁₀ aryl,
   9) 5- to 10-membered heteroaryl,
   10) a 4- to 6-membered non-aryl heterocyclyl oxy,
   11) C₆₋₁₀ aryloxy,
   12) 5- to 10-membered heteroaryloxy,
   13) a C₁₋₆ alkylsulfonyl group,
   14) a C₃₋₆ cycloalkoxysulfonyl group, or
   15) a 4- to 6-membered non-aryl heterocyclyl sulfonyl group, (wherein each substituent of 2) and 4) to 15) is optionally substituted),
R⁵¹ represents
   1) a hydrogen atom,
   2) a C₁₋₆ alkyl group,
   3) a C₃₋₆ alicyclic group,
   4) a 4- to 6-membered non-aryl heterocycle,
   5) C₆₋₁₀ aryl, or
   6) 5- to 10-membered heteroaryl,
      (wherein each substituent from 2) to 6) is optionally substituted), or
R⁵⁰ and R⁵¹ together may form an optionally substituted 4- to 7-membered nitrogen-containing non-aryl heterocycle,
the remaining two (without the structure of formula (2) among R¹, R², and R³) are the same or different, each independently a hydrogen atom, a halogen atom, an optionally substituted C₁₋₆ alkyl group, an optionally substituted C₁₋₆ alkoxy group, an optionally substituted C₁₋₆ alkylthio group, optionally substituted 5- or 6-membered heteroaryl, or -NR^{a3}R^{b2},
R^{d1}, R^{d2}, R^{e1}, R^{f1}, R^{g1}, R^{h1}, Rⁱ¹, and R^{j} are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
a combination of R^{e1} and R^{f1}, when attached to the same nitrogen atom, together may form an optionally substituted 4- to 10-membered nitrogen-containing non-aryl heterocycle,
R⁴ is
   1) -C(=O)R⁸,
   2) -SO₂-L⁶-R⁸,
      (wherein R⁸ in 1) and 2) is -NR^{a5}R^{b4}, -NR^{a5}-L⁷-B(OR^{m1})₂, -OR^{m1}, or an optionally substituted C₁₋₆ alkyl group, and L⁶ is a single bond or -NR^{a6}-),
   3) -NR^{a4}R^{b3},
   4) -B(OR^{m1})₂,
   5) -PO(OR^{m1})(OR^{m2}),
   6) optionally substituted 5-membered heteroaryl,
   7) an optionally substituted 5-membered non-aryl heterocycle, or
   8) a bioisostere of one of 1) to 7),
      (wherein the formulas of 2), 4), 5), and 6) include a carboxylic acid isostere, and 8) may include them in duplicates),
R^{a3}, R^{a4}, R^{a5}, R^{a6}, R^{b2}, R^{b3}, and R^{b4} are the same or different, each independently having the same definition as R^{a1}, R^{a2}, and R^{b1}, wherein a combination of R^{a3} and R^{b2}, R^{a4} and R^{b3}, or R^{a5} and R^{b4}, when attached to the same nitrogen atom, together may form an optionally substituted 4- to 10-membered nitrogen-containing non-aryl heterocycle,
R^{m1} is
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
wherein if R^{m1} is attached to a boron atom via an oxygen atom, two R^{m1}, as C₂₋₄ alkylene, together with the boron atom and two oxygen atoms, may form a 5- to 7-membered non-aryl heterocycle (wherein an alkylene moiety is optionally substituted in the non-aryl heterocycle),
R^{m2} is a hydrogen atom, an optionally substituted C₁₋₆ alkyl group, or an optionally substituted C₃₋₁₀ alicyclic group,
L⁷ is an optionally substituted C₁₋₃ alkylene group,
R⁶¹, R⁶², and R⁶³ are each independently a hydrogen atom, halogen or an optionally substituted C₁₋₆ alkyl group, and
R⁶⁴ is a hydrogen atom, halogen, an optionally substituted C₁₋₆ alkyl group, or -L¹-L²-Z.

In some embodiments, L³ is
1) a C₁₋₆ alkylene group,
2) a C₃₋₁₀ cycloalkylene group, or
3) a 4- to 10-membered non-aryl heterocyclylene group,
   (wherein each substituent from 1) to 3) is optionally substituted), and
L⁴ is a single bond or an optionally substituted C₁₋₅ alkylene group.

In some embodiments, L³ is an optionally substituted C₁₋₄ alkylene group, and
L⁴ is a single bond or an optionally substituted C₁₋₃ alkylene group.

In some embodiments, L³ is -(CR³⁰R³¹)ₙ₁-,
R³⁰ and R³¹ each independently, or if there are multiple instances of each of them, all of them independently represent
   1) a hydrogen atom,
   2) -NR^{a7}R^{a8},
   3) a C₁₋₄ alkyl group,
   4) C₆₋₁₀ aryl,
   5) 5- to 10-membered heteroaryl,
   6) a C₃₋₆ alicyclic group,
   7) a 4- to 10-membered non-aryl heterocycle,
      (wherein each substituent from 3) to 7) is optionally substituted), or
   8) -OR^{c2}, or
R³⁰ and R³¹, together with the carbon atom to which they are attached, form a C₃₋₆ alicyclic group or a 4- to 6-membered non-aryl heterocycle,
R^{a7} and R^{a8} are the same or different, each independently
   1) a hydrogen atom,
   2) a C₁₋₆ alkyl group,
   3) a C₃₋₁₀ alicyclic group,
   4) C₆₋₁₀ aryl
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group, or
   15) -OR^{c3},
      (wherein each substituent from 2) to 14) is optionally substituted),
wherein R^{a7} and R^{a8} together may form an optionally substituted 4- to 10-membered nitrogen-containing non-aryl heterocycle,
R^{c2} and R^{c3} are each independently,
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted),
n1 is an integer 1, 2, 3, or 4,
L⁴ is -(CR⁴⁰R⁴¹)ₙ₂-,
R⁴⁰ and R⁴¹ each independently, or if there are multiple instances of each of them, all of them independently represent
   1) a hydrogen atom,
   2) -NR^{a9}R^{a10},
   3) a C₁₋₄ alkyl group,
   4) C₆₋₁₀ aryl,
   5) 5- to 10-membered heteroaryl,
   6) a C₃₋₆ alicyclic group,
   7) a 4- to 10-membered non-aryl heterocycle,
      (wherein each substituent from 3) to 7) is optionally substituted), or
   8) -OR^{c4}, or
R⁴⁰ and R⁴¹, together with the carbon atom to which they are attached, form a C₃₋₆ alicyclic group or a 4- to 6-membered non-aryl heterocycle,
R^{a9} and R^{a10} are the same or different, each independently
   1) a hydrogen atom,
   2) a C₁₋₆ alkyl group,
   3) a C₃₋₁₀ alicyclic group,
   4) C₆₋₁₀ aryl
   5) 5- or 6-membered heteroaryl,
   6) a 4- to 10-membered non-aryl heterocycle,
   7) a C₁₋₆ alkylcarbonyl group,
   8) a C₃₋₁₀ alicyclic carbonyl group,
   9) a C₆₋₁₀ arylcarbonyl group,
   10) a 5- or 6-membered heteroarylcarbonyl group,
   11) a C₁₋₆ alkylsulfonyl group,
   12) a C₃₋₁₀ alicyclic sulfonyl group,
   13) a C₆₋₁₀ arylsulfonyl group,
   14) a 5- or 6-membered heteroarylsulfonyl group,
      (wherein each substituent from 2) to 14) is optionally substituted), or
   15) -OR^{c5},
wherein R^{a9} and R^{a10} together may form an optionally substituted 4- to 10-membered nitrogen-containing non-aryl heterocycle,
R^{c4} and R^{c5} are each independently defined the same as R^{c2} and R^{c3}, and
n2 is an integer 0 (i.e., when L⁴ is a single bond), 1, 2, or 3.

In some embodiments, -L³-L⁴- is an optionally substituted C₁₋₂ alkylene group.

In some embodiments, -L³-L⁴- is a C₁₋₂ alkylene group optionally substituted with a C₁₋₃ alkyl group, an amino group, or a hydroxymethyl group, or a plurality of the same or different groups thereamong (wherein two C₁₋₃ alkyl groups, when attached to the same carbon atom, together with the carbon atom to which they are attached, may form a C₃₋₆ alicyclic group).

R⁵ is
1) a C₃₋₁₀ alicyclic group,
2) C₆₋₁₀ aryl,
3) 5- to 10-membered heteroaryl,
4) a C₁₋₆ alkylthio group,
   (wherein each substituent from 1) to 4) is optionally substituted, and if two substituents further substituted with the substituent of 2) or 3) are each substituted on adjacent atoms within a ring, the two substituents together may further form a condensed ring structure),
5) -NR^{e1}OH,
6) -C(=O)NR⁵⁰R⁵¹,
7) -SO₂-NR^{e1}R^{f1},
8) -NR^{e1}-SO₂-R^{f1},
9) -C(=O)OR²⁰, or
10) -NR^{e1}R^{f1} (wherein if R⁵ is the substituent of 10), L³ and/or L⁴ is a C₁₋₆ alkylene group that is necessarily substituted with one or more groups other than a hydrogen atom (wherein L³ or L⁴, together with said substituent, may form a C₃₋₁₀ alicyclic group or a 4- to 10-membered non-aryl heterocycle)), and
R²⁰ is
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
   (wherein each substituent from 2) to 6) is optionally substituted).

In some embodiments, R⁵ is
1) C₆₋₁₀ aryl,
2) 5- to 10-membered heteroaryl,
3) -C(=O)NR⁵⁰R⁵¹,
4) -C(=O)OR²⁰, or
5) -NR^{e1}R^{f1} (wherein if R⁵ is the substituent of 5), L³ and/or L⁴ is a C₁₋₆ alkylene group that is necessarily substituted with one or more groups other than a hydrogen atom, and together with said substituent forms at least one C₃₋₁₀ alicyclic group or 4- to 10-membered non-aryl heterocycle)
   (wherein each substituent from 1) to 2) is optionally substituted, and any two groups thereof, when each is attached to adjacent atoms within a ring, together may further form a condensed ring structure).

In some embodiments, Z-L²-L¹ is an optionally substituted C₁₋₆ alkylthio group.

In some embodiments, R⁶¹, R⁶², R⁶³, and R⁶⁴ are each independently a hydrogen atom, a fluorine atom, or a C₁₋₃ alkyl group optionally substituted with a fluorine atom.

In some embodiments, R⁶¹, R⁶², R⁶³, and R⁶⁴ are each independently a hydrogen atom or a fluorine atom.

In some embodiments, G is an oxygen atom.

In some embodiments, X is a hydroxyl group or an optionally substituted C₁₋₆ alkoxy group.

In some embodiments, X is a hydroxyl group.

In some embodiments, R⁴ is
1) -C(=O)OH (i.e., a carboxyl group), or
2) a carboxylic acid isostere.

In some embodiments, ring A is an optionally substituted 4-to 20-membered non-aryl heterocycle. In some embodiments, ring A is an optionally substituted 4- to 10-membered non-aryl heterocycle. In some embodiments, ring A is an optionally substituted 4- to 7-membered non-aryl heterocycle. In some embodiments, ring A is an optionally substituted 4- to 7-membered nitrogen-containing non-aryl heterocycle. In a certain embodiment, ring A is 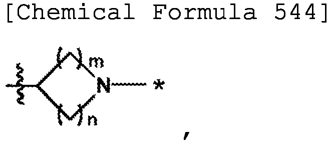 wherein m is 1, 2, or 3, n is 1, 2, or 3, m + n is 2, 3, 4, or 5, a bond that is orthogonal to a wavy line indicates a bond with Y, and a bond with * indicates a bond with L³. In some embodiments, m + n is 2, 3, or 4.

In some embodiments, ring A is an optionally substituted 4-to 6-membered non-aryl heterocycle. In some embodiments, ring A is an optionally substituted 4- to 6-membered nitrogen-containing non-aryl heterocycle.

In some embodiments, ring A is 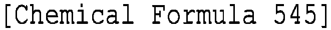 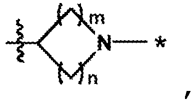 wherein m + n is 2 or 3. In a preferred embodiment, m + n is 2. In a more preferred embodiment, m = 1 and n = 1.

In some embodiments, m is 1 or 2, n is 1 or 2, and m + n is 2 or 3. In some embodiments, m is 1, and n is 1.

In some embodiments, ring A is an optionally substituted azetidine ring. In a certain embodiment in such embodiments, ring A is 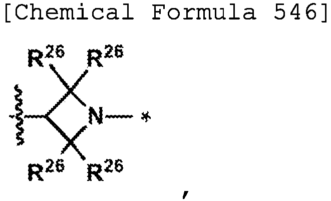 wherein R²⁶ represents a substituent on an azetidine ring and is defined the same as R^{6a}, a bond that is orthogonal to a wavy line indicates a bond with Y, and a bond with * indicates a bond with L³. In a preferred embodiment, R²⁶ are the same or different, each independently selected from the group consisting of
1) a hydrogen atom,
2) a halogen atom,
3) a C₁₋₆ alkyl group, and
4) a C₁₋₆ alkoxy group,
   (wherein each of substituents 3) and 4) is optionally substituted with a halogen atom) (wherein a halogen atom in 2), 3), and 4) is more preferably a fluorine atom), and
in a more preferred embodiment, are selected from the group consisting of
1) a hydrogen atom,
2) a halogen atom, and
3) a C₁₋₆ alkyl group optionally substituted with a halogen atom, (wherein a halogen atom in 2) and 3) is more preferably a fluorine atom), and
   most preferably are hydrogen atoms.

In some embodiments, Y is an oxygen atom or a sulfur atom. In some embodiments, Y is an oxygen atom.

In some embodiments, L³ is -C(=O)- or -S(=O)₂-. In some embodiments, L³ is -C(=O)-.

In some embodiments, L⁴ is a single bond, -C(=N-OR^{h1})-, or an optionally substituted C₁₋₆ alkylene group, wherein R^{h1} is an optionally substituted C₁₋₆ alkyl group.

In some embodiments, R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
   (wherein each substituent from 3) to 5) is optionally substituted).

In some embodiments, R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
   (wherein each substituent from 3) to 5) is optionally substituted with a halogen atom).

In some embodiments, R¹ and R² are the same or different, each independently selected from the group consisting of
1) a hydrogen atom,
2) a halogen atom,
3) a C₁₋₆ alkyl group optionally substituted with a halogen atom.

In some embodiments, R¹ and R² are the same or different, each independently selected from the group consisting of
1) a hydrogen atom,
2) a fluorine atom,
3) a C₁₋₃ alkyl group optionally substituted with a fluorine atom.

In some embodiments, R¹ and R² are both hydrogen atoms.

In some embodiments, L³ is -(CR³⁰R³¹)ₙ₁- or 4- to 10-membered non-aryl heterocyclylene,
R³⁰ and R³¹ each independently, or if there are multiple instances of each of them, all of them independently represent
   1) a hydrogen atom,
   2) an optionally substituted C₁₋₄ alkyl group, or
   3) optionally substituted C₆₋₁₀ aryl,
n1 is 1, 2, or 3,
L⁴ is a single bond or - (CR⁴⁰R⁴¹)ₙ₂-,
R⁴⁰ and R⁴¹ each independently, or if there are multiple instances of each of them, all of them independently represent
   1) a hydrogen atom,
   2) -NR^{a9}R^{a10},
   3) an optionally substituted C₁₋₄ alkyl group, or
   4) -OR^{c4}, or
R⁴⁰ and R⁴¹, together with the carbon atom to which they are attached, form a C₃₋₆ alicyclic group or a 4- to 6-membered non-aryl heterocycle,
R^{a9}, R^{a10}, and R^{c4} are the same or different, each independently
   1) a hydrogen atom,
   2) an optionally substituted C₁₋₆ alkyl group, or
   3) an optionally substituted C₃₋₁₀ alicyclic group,
wherein R^{a9} and R^{a10} together may form an optionally substituted 4- to 10-membered nitrogen-containing non-aryl heterocycle, and
n2 is 0, 1, or 2.

In some embodiments, L³ is -(CR³⁰R³¹)ₙ₁-,
R³⁰ and R³¹ each independently, or if there are multiple instances of each of them, all of them independently represent
   1) a hydrogen atom,
   2) an optionally substituted C₁₋₄ alkyl group, or
   3) optionally substituted C₆₋₁₀ aryl,
n1 is 1, 2, or 3,
L⁴ is -(CR⁴⁰R⁴¹)ₙ₂-,
R⁴⁰ and R⁴¹ each independently, or if there are multiple instances of each of them, all of them independently represent
   1) a hydrogen atom,
   2) -NR^{a9}R^{a10},
   3) an optionally substituted C₁₋₄ alkyl group, or
   4) -OR^{c4}, or
R⁴⁰ and R⁴¹, together with the carbon atom to which they are attached, form a C₃₋₆ alicyclic group or a 4- to 6-membered non-aryl heterocycle,
R^{a9}, R^{a10}, and R^{c4} are the same or different, each independently
   1) a hydrogen atom,
   2) an optionally substituted C₁₋₆ alkyl group, or
   3) an optionally substituted C₃₋₁₀ alicyclic group,
wherein R^{a9} and R^{a10} together may form an optionally substituted 4- to 10-membered nitrogen-containing non-aryl heterocycle, and
n2 is 0, 1, or 2.

In some embodiments, n1 is 1 or 2, and n2 is 0 or 1.

In some embodiments, L³ is -CR³⁰R³¹- or 4- to 10-membered non-aryl heterocyclylene,
R³⁰ and R³¹ each independently represent,
   1) a hydrogen atom,
   2) a C₁₋₄ alkyl group (wherein the group is optionally substituted with halogen, -NR^{a11}R^{a12}, or -OR^{c6}), or
   3) C₆ aryl (wherein the group is optionally substituted with halogen, -NR^{a13}R^{a14}, -OR^{c7}, or a C₁₋₃ alkyl group (wherein the group is optionally substituted with halogen, -NR^{a15}R^{a16}, or -OR^{c8})),
L⁴ is a single bond or - (CR⁴⁰R⁴¹)ₙ₂-,
R⁴⁰ and R⁴¹ each independently represent,
   1) a hydrogen atom,
   2) -NR^{a9}R^{a10},
   3) a C₁₋₄ alkyl group (wherein the group is optionally substituted with halogen, -NR^{a17}R^{a18}, or -OR^{c9}), or
   4) -OR^{c4}, or
R⁴⁰ and R⁴¹, together with the carbon atom to which they are attached, form a C₃₋₆ alicyclic group,
R^{a9}, R^{a10}, R^{a11}, R^{a12}, R^{a13}, R^{a14}, R^{a15}, R^{a16}, R^{a17}, R^{a18}, R^{c4}, R^{c6}, R^{c7}, R^{c8}, and R^{c9} are the same or different, each independently representing
   1) a hydrogen atom, or
   2) a C₁₋₄ alkyl group (wherein the group is optionally substituted with halogen, -NR^{a19}R^{a20}, or -OR^{c10}),
R^{a19}, R^{a20}, and R^{c10} are the same or different, each independently representing
   1) a hydrogen atom, or
   2) a C₁₋₄ alkyl group (wherein the group is optionally substituted with halogen),
wherein each combination of R^{a9} and R^{a10}, R^{a11} and R^{a12}, R^{a13} and R^{a14}, R^{a15} and R^{a16}, R^{a17} and R^{a18}, or R^{a19} and R^{a20} together may form an optionally substituted 4- to 7-membered nitrogen-containing non-aryl heterocycle, and
n2 is 0 or 1.

In some embodiments, L³ is -CR³⁰R³¹-,
R³⁰ and R³¹ each independently represent,
   1) a hydrogen atom,
   2) a C₁₋₄ alkyl group (wherein the group is optionally substituted with halogen, -NR^{a11}R^{a12}, or -OR^{c6}), or
   3) C₆ aryl (wherein the group is optionally substituted with halogen, -NR^{a13}R^{a14}, -OR^{c7}, or a C₁₋₃ alkyl group (wherein the group is optionally substituted with halogen, -NR^{a15}R^{a16}, or -OR^{c8})),
L⁴ is -(CR⁴⁰R⁴¹)ₙ₂-,
R⁴⁰ and R⁴¹ each independently represent,
   1) a hydrogen atom,
   2) -NR^{a9}R^{a10},
   3) a C₁₋₄ alkyl group (wherein the group is optionally substituted with halogen, -NR^{a17}R^{a18}, or -OR^{c9}), or
   4) -OR^{c4}, or
R⁴⁰ and R⁴¹, together with the carbon atom to which they are attached, form a C₃₋₆ alicyclic group,
R^{a9}, R^{a10}, R^{a11}, R^{a12}, R^{a13}, R^{a14}, R^{a15}, R^{a16}, R^{a17}, R^{a18}, R^{c4}, R^{c6}, R^{c7}, R^{c8}, and R^{c9} are the same or different, each independently representing
   1) a hydrogen atom or
   2) a C₁₋₄ alkyl group (wherein the group is optionally substituted with halogen, -NR^{a19}R^{a20}, or -OR^{c10}),
R^{a19}, R^{a20}, and R^{c10} are the same or different, each independently representing
   1) a hydrogen atom, or
   2) a C₁₋₄ alkyl group (wherein the group is optionally substituted with halogen),
wherein each combination of R^{a9} and R^{a10}, R^{a11} and R^{a12}, R^{a13} and R^{a14}, R^{a15} and R^{a16}, R^{a17} and R^{a18}, or R^{a19} and R^{a20} together may form an optionally substituted 4- to 7-membered nitrogen-containing non-aryl heterocycle, and
n2 is 0 or 1.

In some embodiments,
L³ is -CH₂-, -CH(CH₂NH₂)-, or -CH(CH₂OH)-, and
L⁴ and a single bond, -CH₂-, -CH(NH₂)-, -CMe(NH₂)-, -CEt(NH₂)-, -C(iso-Pr)(NH₂)-, -CH (CH₂NH₂)-, -CH(OH)-, -CH(CH₂OH)-, -C(CH₂OH)₂-, or

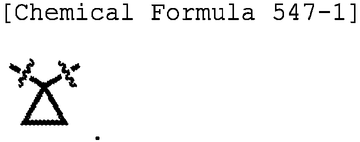

In some embodiments,
L³ is -CH₂-, -CH (CH₂NH₂)-, or -CH(CH₂OH)-, and
L⁴ is a single bond, -CH₂-, -CH(NH₂)-, -CMe(NH₂)-, -CEt(NH₂)-, -C(iso-Pr)(NH₂)-, -CH (CH₂NH₂)-, -CH(OH)-, -CH(CH₂OH)-, or

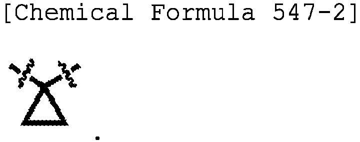

In some embodiments, L³ is 4- to 10-membered non-aryl heterocyclylene.

In some embodiments, L³ is 5-membered non-aryl heterocyclylene.

In some embodiments, L³ is 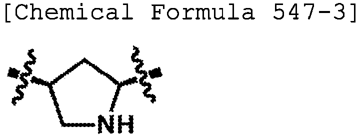 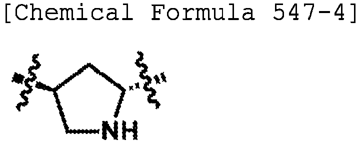 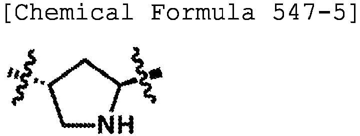 or

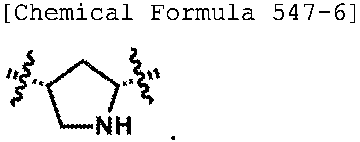

In some embodiments, L⁴ is a single bond.

In some embodiments, R⁵ is
1) C₆₋₁₀ aryl, or
2) 5- to 10-membered heteroaryl,
   (wherein each substituent from 1) to 2) is optionally substituted, and if two substituents further substituted with the substituent of 1) or 2) are each substituted on adjacent atoms within a ring, the two substituents together may further form a condensed ring structure).

In some embodiments, R⁵ is C₆ aryl (i.e., phenyl) or 5-membered, 6-membered, 9-membered, or 10-membered heteroaryl,
each group of R⁵ is optionally substituted with each of R^{6a} or R^{6b} at all chemically substitutable positions on a carbon atom or a nitrogen atom within a ring thereof,
wherein R^{6a}, which are substituents on the carbon atom, if there are multiple instances on the same ring, are all independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e2}R^{f2},
   13) -NR^{g2}-CR^{e2}(=NR^{f2}),
   14) -NR^{g2}-CR^{e2}(=N-OR^{f2}),
   15) -NR^{h2}-C(=NR^{g2})NR^{e2}R^{f2},
   16) -NR^{h2}-C(=N-OR^{g2})NR^{e2}R^{f2},
   17) -NRⁱ²-C(=NR^{h2})NR^{g2}-NR^{e2}R^{f2},
   18) -NRⁱ²-C(=N-OR^{h2})NR^{g2}-NR^{e2}R^{f2},
   19) -C(=NR^{e2})R^{f2},
   20) -C(=N-OR^{e2})R^{f2},
   21) -C(=NR^{h2})-NR^{e2}R^{f2},
   22) -C(=NR^{h2})NR^{g2}-NR^{e2}R^{f2},
   23) -C(=N-OR^{h2})NR^{g2}-NR^{e2}R^{f2},
   24) -NR^{e2}R^{f2},
   25) -NR^{g2}-NR^{e2}R^{f2},
   26) -NR^{e2}OR^{f2},
   27) -NR^{e2}-C(=O)R^{f2},
   28) -C(=O)NR^{e2}R^{f2},
   29) -C(=O)NR^{e2}OR^{f2},
   30) -C(=O)NR^{g2}-NR^{e2}R^{f2},
   31) -C (=O)R^{e2},
   32) -C (=O)OR^{e2}, and
   33) -C(=N-OR^{h2})NR^{e2}R^{f2},
wherein R^{6b}, which are substituents on the nitrogen atom, if there are multiple instances on the same ring, are all independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C (=NR^{e2})R^{f2},
   6) -C (=N-OR^{e2})R^{f2},
   7) -SO₂-NR^{e2}R^{f2},
   8) -C (=NR^{h2})-NR^{e2}R^{f2},
   9) -C (=NR^{h2})NR^{g2}-NR^{e2}R^{f2}
   10) -C (=N-OR^{h2})NR^{g2}-NR^{e2}R^{f2},
   11) -C (=O)NR^{e2}R^{f2},
   12) -C(=O)NR^{e2}OR^{f2},
   13) -C(=O)NR^{g2}-NR^{e2}R^{f2},
   14) -C(=O)R^{e2}, and
   15) -C (=N-OR^{h2})NR^{e2}R^{f2}, or
if a combination of two R^{6a} or R^{6a} and R^{6b} are each substituted on adjacent atoms within a ring, the two substituents together may form an optionally substituted 5- to 6-membered heteroaryl ring or an optionally substituted 5- to 7-membered non-aryl heterocycle, which further fuses to an attachment moiety between the adjacent atoms within the ring,
R^{e2}, R^{f2}, R^{g2}, R^{h2}, and Rⁱ² are the same or different, each independently
   1) a hydrogen atom,
   2) a C₁₋₆ alkyl group,
   3) a C₃₋₁₀ alicyclic group,
   4) C₆₋₁₀ aryl,
   5) 5- or 6-membered heteroaryl, or
   6) a 4- to 10-membered non-aryl heterocycle,
      (wherein each substituent from 2) to 6) is optionally substituted), and
a combination of R^{e2} and R^{f2}, when attached to the same nitrogen atom, together may form an optionally substituted 4- to 10-membered nitrogen-containing non-aryl heterocycle.

R⁵ is C₆ aryl or 5-membered, 6-membered, 9-membered, or 10-membered heteroaryl selected from the group consisting of 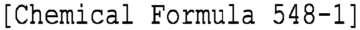 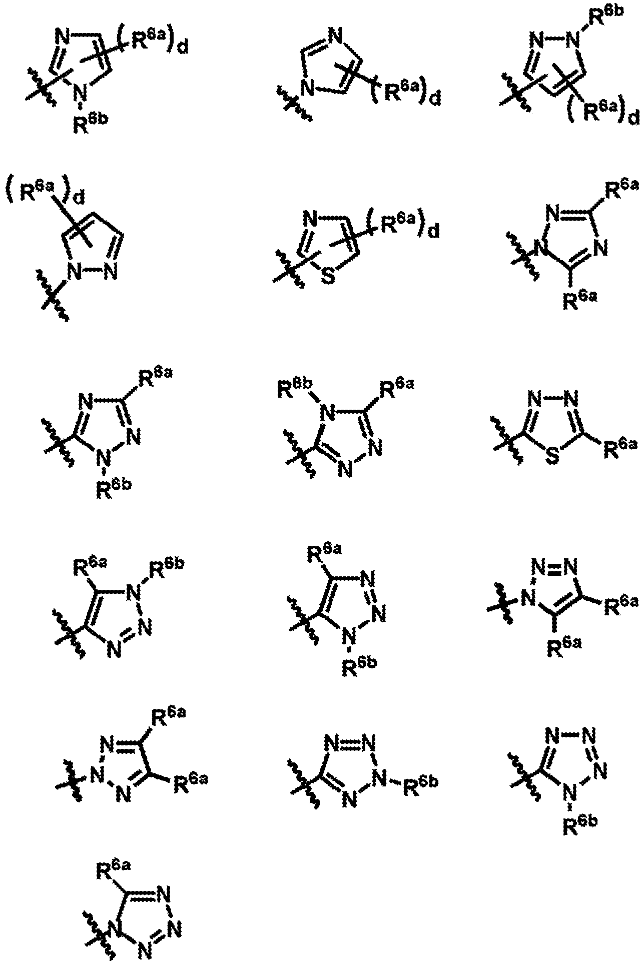 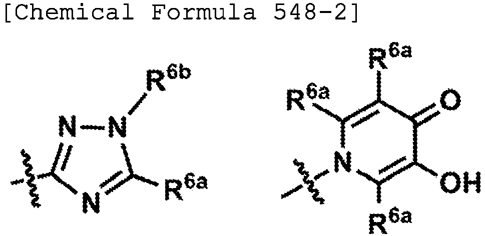 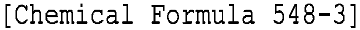 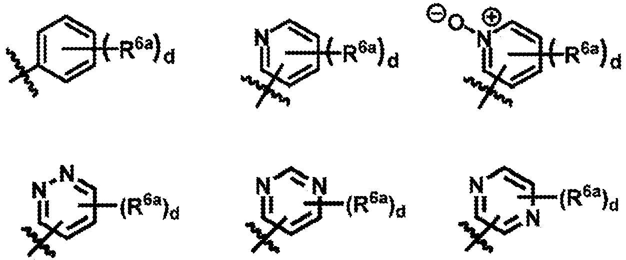 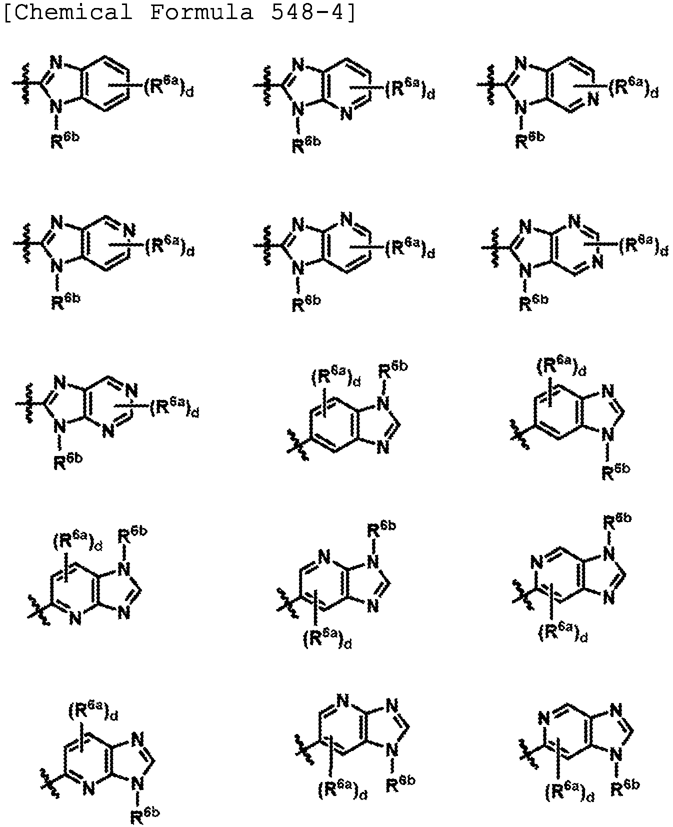 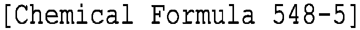 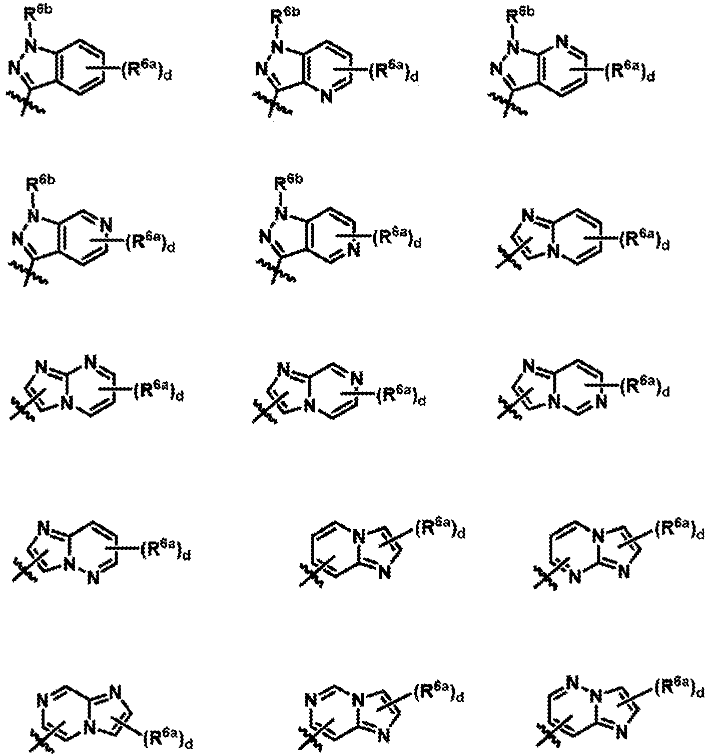 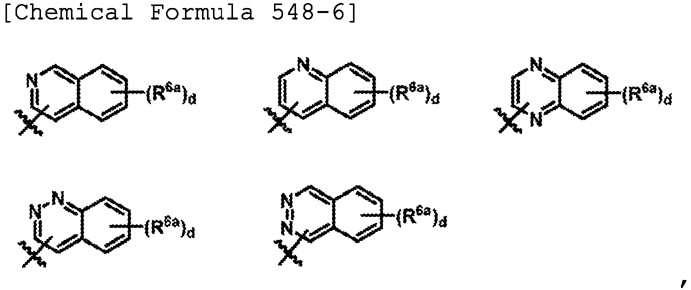 d is the number of chemically substitutable positions on a ring of each group by R^{6a}, and
each R^{6a} and each R^{6b} are defined the same as the present specification (e.g., item 46).

In some embodiments, R⁵ is C₆ aryl or 5-membered, 6-membered, 9-membered, or 10-membered heteroaryl selected from the group consisting of 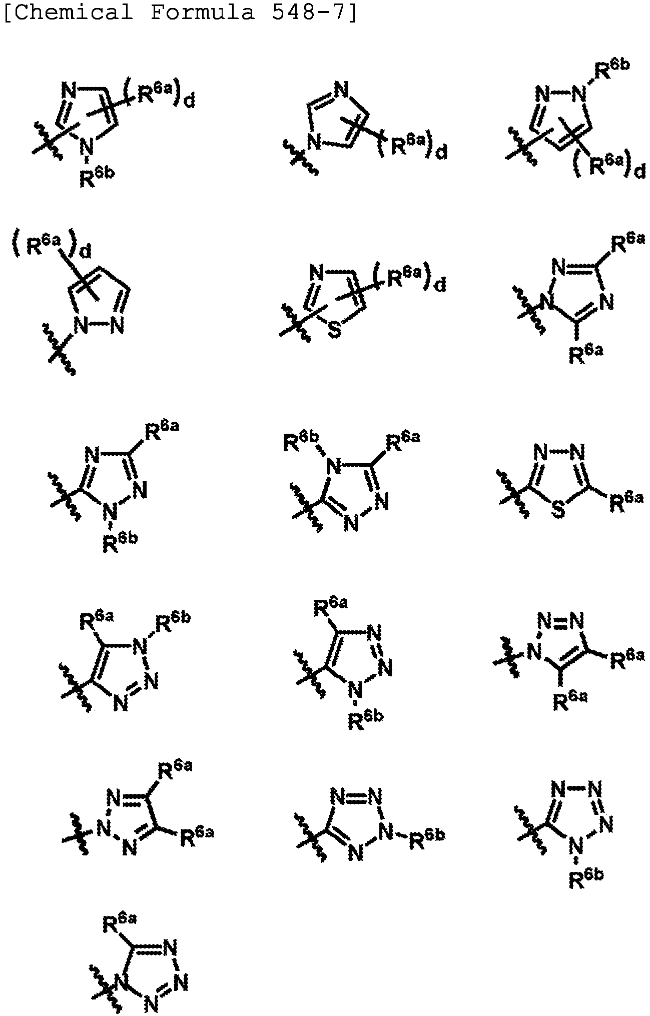 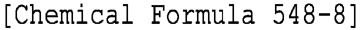 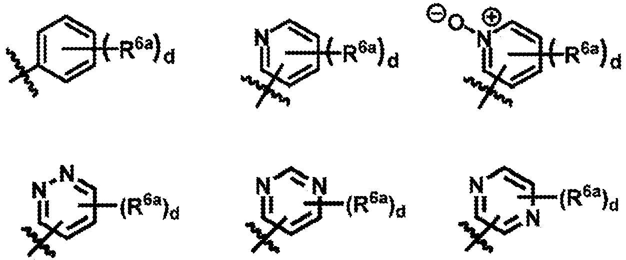 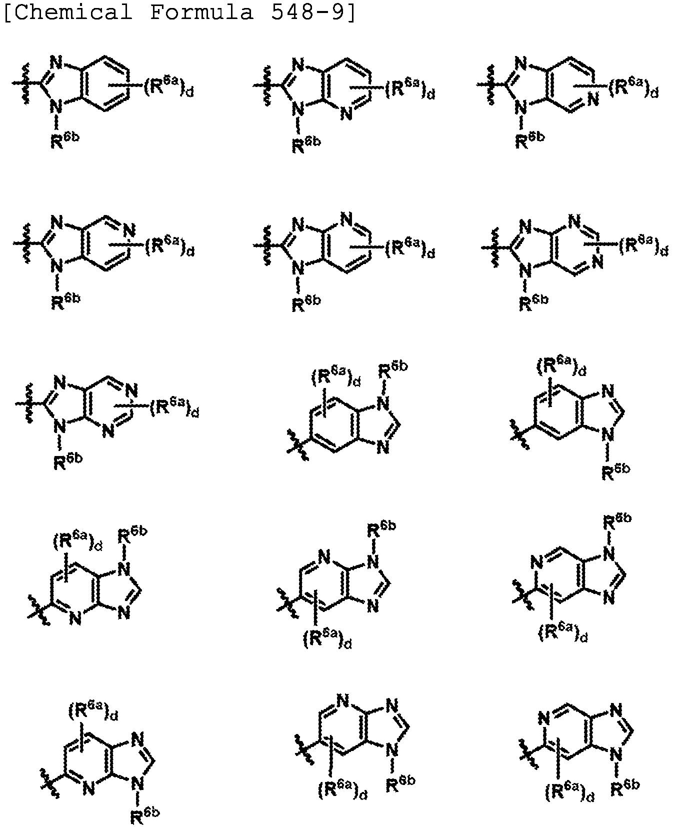 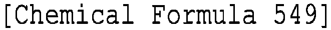 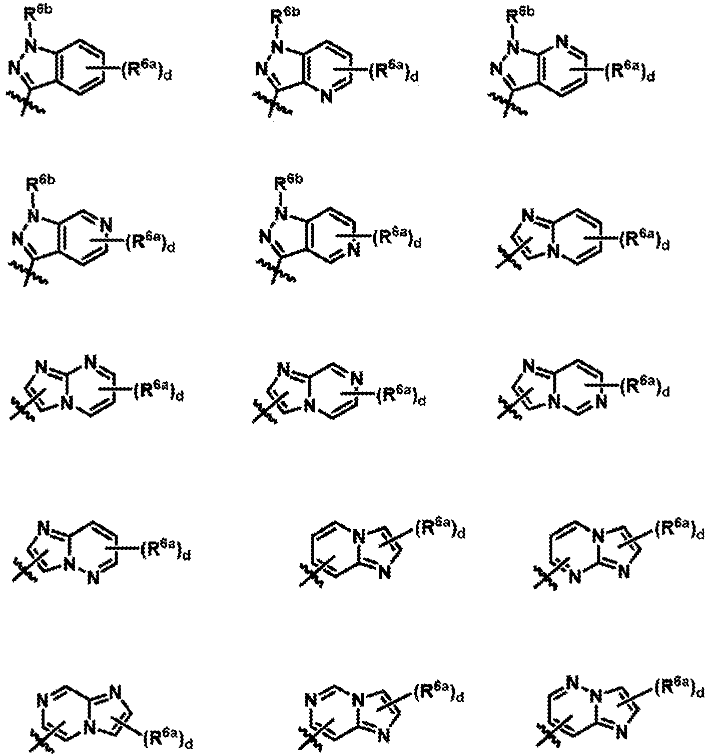 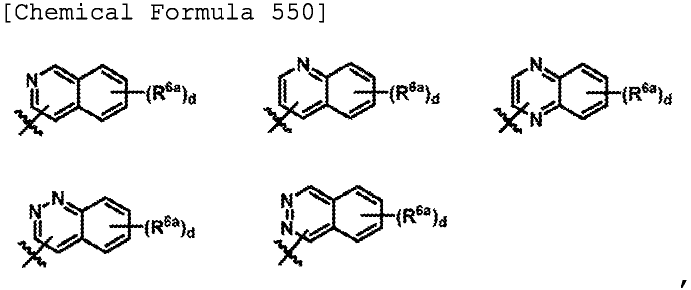 d is the number of chemically substitutable positions on a ring of each group by R^{6a}, and
each R^{6a} and each R^{6b} are defined the same as the definition herein (e.g., item 41).

In some embodiments, R^{6a}, if there are multiple instances on the same ring, are all independently selected from the group consisting of
1) a hydrogen atom,
2) a hydroxyl group,
3) halogen,
4) a C₁₋₄ alkyl group
   (wherein the alkyl group is optionally substituted with NR^{e2}R^{f2}, - C(=O)OR^{f2}, -C (=O)NR^{e2}R^{f2}, -C (=O)NR^{e2}(OR^{f2}), or a hydroxyl group),
5) a C₁₋₄ alkoxy group,
6) -NR^{e2}R^{f2},
7) -C (=O)NR^{e2}R^{f2}, and
8) -C (=O)OR^{e2}, and
each R^{6b}, if there are multiple instances on the same ring, are all independently selected from the group consisting of 1) a hydrogen atom, 2) a hydroxyl group, and 3) a C₁₋₄ alkyl group (wherein the alkyl group is optionally substituted with NR^{e2}R^{f2}, - C (=O)OR^{f2}, -C (=O)NR^{e2}R^{f2}, -C (=O)NR^{e2}(OR^{f2}), or a hydroxyl group).

In some embodiments, R^{e2} and R^{f2} are the same or different, each independently a hydrogen atom, an optionally substituted C₁₋₆ alkyl group, or an optionally substituted C₃₋₁₀ alicyclic group. In some embodiments, R^{e2} and R^{f2} are the same or different, each independently a hydrogen atom or an optionally substituted C₁₋₆ alkyl group. In some embodiments, R^{e2} and R^{f2} are hydrogen atoms.

In some embodiments, R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

In some embodiments, R⁵ is C (=O)NR⁵⁰R⁵¹.

In some embodiments, R⁵⁰ represents
1) a hydrogen atom,
2) an optionally substituted C₁₋₄ alkyl group,
3) a hydroxyl group,
4) an optionally substituted C₁₋₄ alkoxy group, or
5) an optionally substituted C₁₋₆ alkylsulfonyl group,
R⁵¹ represents 1) a hydrogen atom or 2) an optionally substituted C₁₋₄ alkyl group, or
R⁵⁰ and R⁵¹ together may form a 4- to 6-membered nitrogen-containing non-aryl heterocycle.

In some embodiments, R⁵⁰ represents
1) a hydrogen atom,
2) a C₁₋₄ alkyl group (wherein the group is optionally substituted with halogen, a C₃₋₆ alicyclic group, -NR^{a21}R^{a22}, or -OR^{c11}),
3) a hydroxyl group,
4) a C₁₋₄ alkoxy group (wherein the group is optionally substituted with halogen, a C₃₋₆ alicyclic group, -NR^{a23}R^{a24}, or -OR^{c12}), or
5) a C₁₋₄ alkylsulfonyl group (wherein the group is optionally substituted with halogen, a C₃₋₆ alicyclic group, -NR^{a21}R^{a22}, or -OR^{c11}),
R^{a21}, R^{a22}, R^{a23}, R^{a24}, R^{c11}, and R^{c12} are the same or different, each independently representing 1) a hydrogen atom or 2) a C₁₋₄ alkyl group (wherein the group is optionally substituted with halogen),
wherein each combination of R^{a21} and R^{a22} or R^{a23} and R^{a24} together may form an optionally substituted 4- to 7-membered nitrogen-containing non-aryl heterocycle, and
R⁵¹ is a hydrogen atom or C₁₋₄ alkyl (wherein the group is optionally substituted with halogen).

In some embodiments, R⁵⁰ represents
1) a hydrogen atom,
2) a C₁₋₄ alkyl group (wherein the group is optionally substituted with a cyclopropyl group, -NH₂, -NHMe, -C(=O)NH₂, -C(=O)NHMe, - C(=O)NMe₂, or a -hydroxyl group),
3) a hydroxyl group,
4) a C₁₋₄ alkoxy group (wherein the group is optionally substituted with a cyclopropyl group, -NH₂, -NHMe, or a -hydroxyl group), or
5) a C₁₋₄ alkylsulfonyl group, and,
R⁵¹ is a hydrogen atom.

In some embodiments, R⁵⁰ represents
1) a hydrogen atom,
2) a C₁₋₄ alkyl group (wherein the group is optionally substituted with a cyclopropyl group, -NH₂, -NHMe, or a -hydroxyl group),
3) a hydroxyl group,
4) a C₁₋₄ alkoxy group (wherein the group is optionally substituted with a cyclopropyl group, -NH₂, -NHMe, or a -hydroxyl group), or
5) a C₁₋₄ alkylsulfonyl group, and
R⁵¹ is a hydrogen atom.

In some embodiments, R⁵ is -C(=O)OR²⁰.

In some embodiments, R²⁰ is 1) a hydrogen atom or 2) an optionally substituted C₁₋₄ alkyl group.

In some embodiments, L³ is -CH₂-, and L⁴ is -CH (NH₂)- or - CMe (NH₂)- .

In some embodiments, L³ is -CH₂-, and
L⁴ is a single bond, -CH(NH₂)-, or -CMe(NH₂)-.

In some embodiments, L³ is -CH₂-, and L⁴ is 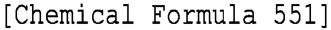 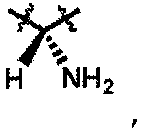 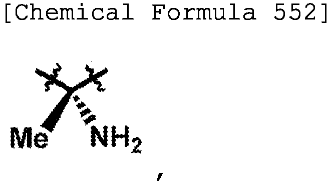 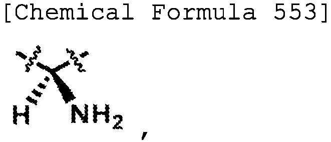 or

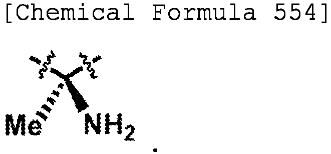

In some embodiments, L³ is -CH₂-, and L⁴ is 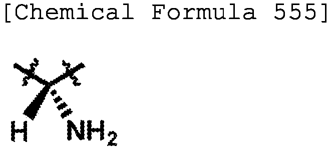 or

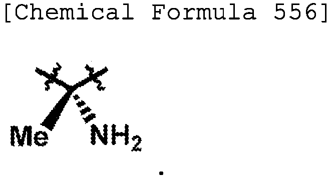

In some embodiments, R²⁰ is a hydrogen atom. In such a case, R⁵ is, specifically, -C(=O)OH (i.e., a carboxyl group).

In some embodiments, R⁵ is -NR^{e1}R^{f1},
L³ is -CH₂-,
L⁴ is -CR⁴⁰R⁴¹-, and
R⁴⁰ and R⁴¹ are each independently a C₁₋₄ alkyl group substituted with a hydroxyl group, or together with the carbon atom to which they are attached, form a C₃₋₆ alicyclic group.

In some embodiments, R⁵ is -NR^{e1}R^{f1}, L³ is -CH₂-, L⁴ is -CR⁴⁰R⁴¹-, and R⁴⁰ and R⁴¹, together with the carbon atom to which they are attached, form a C₃₋₆ alicyclic group.

In some embodiments, R^{e1} and R^{f1} are the same or different, each independently
1) a hydrogen atom, or
2) an optionally substituted C₁₋₃ alkyl group, and
L⁴ is

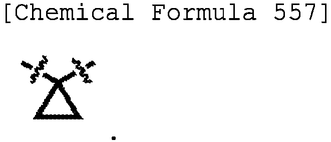

In some embodiments, R^{e1} and R^{f1} are the same or different, each independently
1) a hydrogen atom, or
2) an optionally substituted C₁₋₃ alkyl group, and
L⁴ is a C₁₋₄ alkylene group substituted with a hydroxyl group.

In some embodiments, L⁴ is a single bond or a C₁₋₆ alkylene group optionally substituted with -NR²¹R²² or =NOR²³, wherein R²¹, R²², and R²³ are each independently a hydrogen atom, an optionally substituted C₁₋₆ alkyl group, or an optionally substituted 4- to 10-membered non-aryl heterocyclyl carbonyl group.

In some embodiments, L⁴ is a single bond, -CH₂-, -CH(NH₂)-, - CMe(NH₂)-, -CH(NHMe)-, -CD(NH₂)- (wherein D represents a heavy hydrogen atom), -CH₂CH₂-, or -CH(NH₂)-CH₂-, wherein if an amino group is present in L⁴, carbon that attaches to the amino group attaches to L³.

In some embodiments, L³ is -C(=O)-, and
L⁴ is a single bond, -CH₂-, -CH(NH₂)-, -CMe(NH₂)-, or - CH(NH₂)-CH₂-.

In some embodiments, L³ is -C(=O)-, and L⁴ is -CH(NH₂)- or - CMe(NH₂)-.

In some embodiments, L⁴ is an isomeric structure of one of 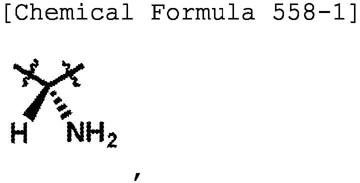 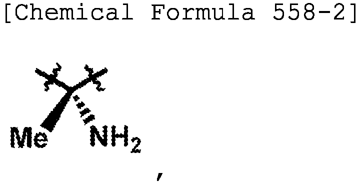 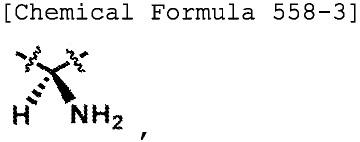 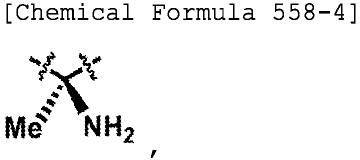 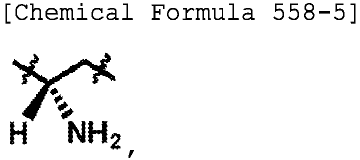 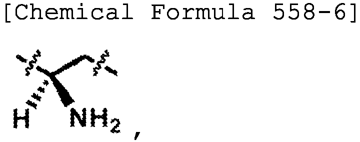 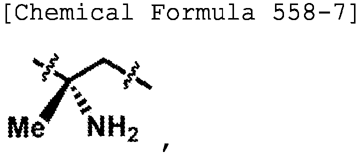 and

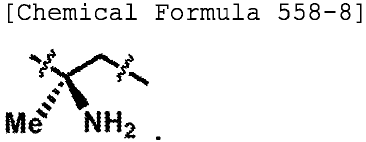

In some embodiments, L³ is -C(=O)-, and L⁴ is an isomeric structure of one of 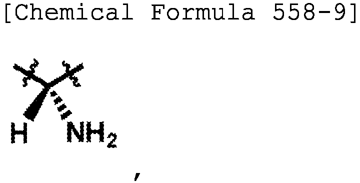 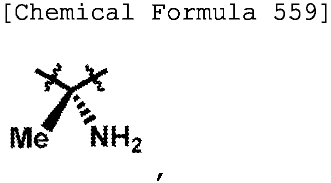 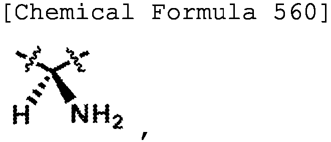 and

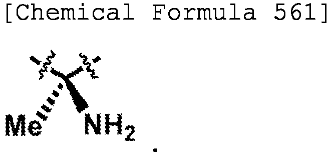

In some embodiments, L³ is -C(=O)-, and L⁴ is 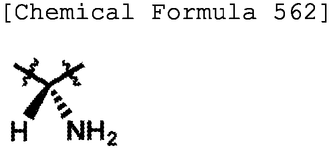 or

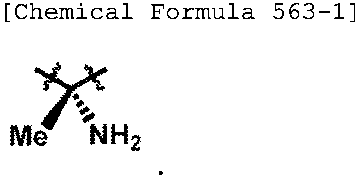

In some embodiments, L⁴ is 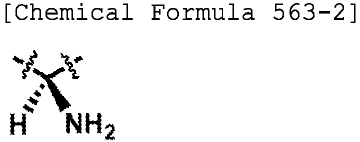 or

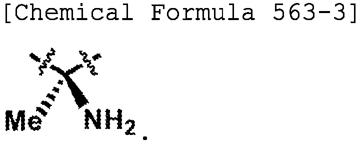

In some embodiments, L⁴ is 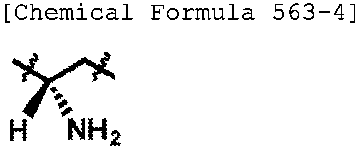 or

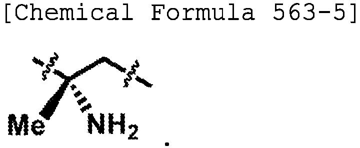

In some embodiments, L⁴ is 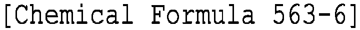 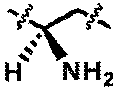 or

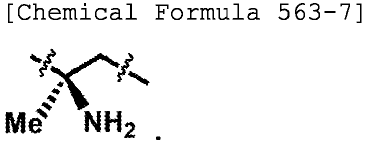

In some embodiments, R⁵ is a hydrogen atom, an optionally substituted C₁₋₆ alkyl group, an optionally substituted C₃₋₁₀ alicyclic group, an optionally substituted 4- to 10-membered non-aryl heterocycle, optionally substituted C₆₋₁₀ aryl, optionally substituted 5- or 6-membered heteroaryl, an optionally substituted C₁₋₆ alkylthio group, or -NR^{e1}OH, wherein R^{e1} is a hydrogen atom or an optionally substituted C₁₋₆ alkyl group.

In some embodiments, R⁵ is a hydrogen atom, an optionally substituted C₁₋₆ alkyl group, an optionally substituted 4- to 10-membered non-aryl heterocycle, optionally substituted C₆₋₁₀ aryl, optionally substituted 5- or 6-membered heteroaryl, an optionally substituted C₁₋₆ alkylthio group, or -NR^{e1}OH, wherein R^{e1} is a hydrogen atom or an optionally substituted C₁₋₆ alkyl group.

In some embodiments, R⁵ is optionally substituted 5- or 6-membered heteroaryl or optionally substituted C₆₋₁₀ aryl.

In some embodiments, R⁵ is optionally substituted 5- or 6-membered heteroaryl.

In some embodiments, R⁵ is an optionally substituted C₄₋₁₀ alicyclic group or an optionally substituted 4- to 10-membered non-aryl heterocycle.

In some embodiments, R⁵ is an optionally substituted 4- to 10-membered non-aryl heterocycle.

In some embodiments, L⁴ is a single bond, and R⁵ is -NR^{e1}OH, wherein R^{e1} is a hydrogen atom or an optionally substituted C₁₋₆ alkyl group.

In some embodiments, L⁴ is
1) - (CH₂)ₚ-CR¹⁰(NHR¹¹)-,
2) - (CH₂)_{q}-CR¹²R¹³-, or
3) - (CH₂)ₚ-CR¹⁰(NHR¹¹)- (CH₂)_{q}-CR¹²R¹³- (wherein p and q are independently 0 or 1),

R¹⁰ is
   1) a hydrogen atom,
   2) a carboxyl group, or
   3) -C(=O)NR^{10a}R^{10b},
R¹¹ is
   1) a hydrogen atom,
   2) -C (=O)R^{11a}, or
   3) an optionally substituted 5- or 6-membered non-aryl heterocyclyl carbonyl group,
      wherein if R¹⁰ is -C (=O)NR^{10a}R^{10b}, R^{10b} and R¹¹ together may form - CH₂CH₂-,
R¹² is
   1) a hydrogen atom, or
   2) an optionally substituted C₁₋₄ alkyl group,
R¹³ is
   1) a hydrogen atom,
   2) a hydroxyl group,
   3) an optionally substituted C₁₋₄ alkyl group,
   4) a sulfanyl group,
   5) a carboxyl group,
   6) an optionally substituted C₁₋₄ alkylthio group,
   7) -NR^{13a}R^{13b},
   8) -NR^{13a}-C(=O)R^{13b},
   9) an optionally substituted 5- or 6-membered non-aryl heterocyclyl carbonylamino group,
   10) -NR^{13a}-C(=O)NR^{13b}R^{13c},
   11) -C(=O)NR^{13a}R^{13b},
   12) -C (=O)NR^{13a}OR^{13b},
   13) -S(=O)₂-R^{13a},
   14) -S(=O)₂-NR^{13a}R^{13b},
   15) -C (=O)NR^{13a}-S(=O)₂-R^{13b}, or
   16) -C (=O)NR^{13a}-S(=O)₂-NR^{13b}R^{13c}, and
R^{10a}, R^{10b}, R^{11a}, R^{13a}, R^{13b}, and R^{13c} are each independently a hydrogen atom or an optionally substituted C₁₋₄ alkyl group. In such a case, in some embodiments, R⁵ is a hydrogen atom or an optionally substituted C₁₋₄ alkyl group.

In some embodiments, R⁵ is selected from the group consisting of 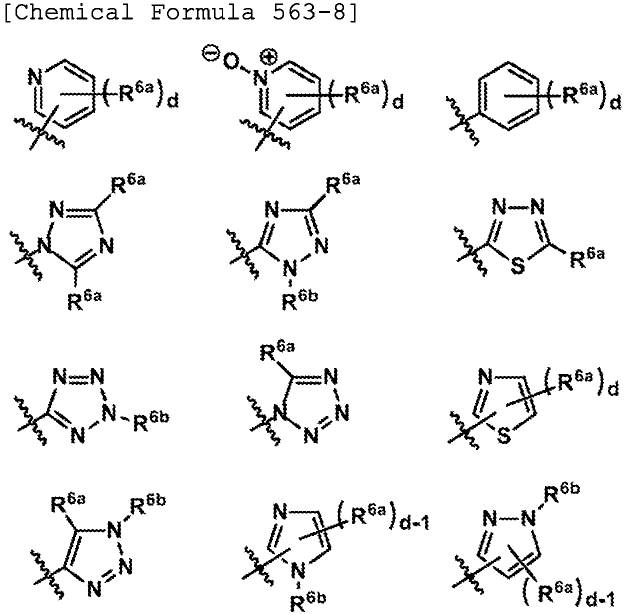 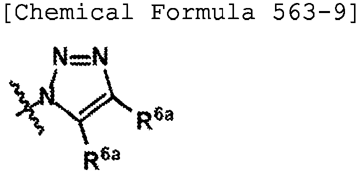 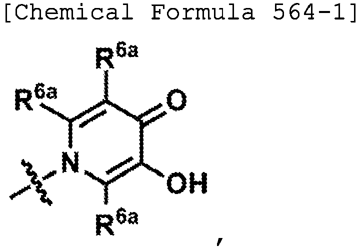 subscript d is the number of substitutable positions on a ring of R⁵,
each R^{6a} is independently selected from the group consisting of
   1) a hydrogen atom,
   2) a hydroxyl group,
   3) a cyano group,
   4) a nitro group,
   5) halogen,
   6) a C₁₋₄ alkyl group,
   7) a C₃₋₁₀ alicyclic group,
   8) a C₁₋₄ alkoxy group,
   9) a C₃₋₁₀ alicyclic oxy group,
   10) a C₆₋₁₀ aryloxy group,
   11) a 5- or 6-membered heteroaryloxy group,
   12) a 4- to 10-membered non-aryl heterocyclyl oxy group,
      (wherein each substituent from 6) to 12) is optionally substituted),
   13) -SO₂-NR^{e2}R^{f2},
   14) -NR^{g2}-CR^{e2}(=NR^{f2}),
   15) -NR^{g2}-CR^{e2}(=N-OR^{f2}),
   16) -NR^{h2}-C(=NR^{g2})NR^{e2}R^{f2},
   17) -NR^{h2}-C(=N-OR^{g2})NR^{e2}R^{f2},
   18) -NRⁱ²-C(=NR^{h2})NR^{g2}-NR^{e2}R^{f2},
   19) -NRⁱ²-C(=N-OR^{h2})NR^{g2}-NR^{e2}R^{f2},
   20) -C (=NR^{e2})R^{f2},
   21) -C(=N-OR^{e2})R^{f2},
   22) -C (=NR^{h2})-NR^{e2}R^{f2},
   23) -C (=NR^{h2})NR^{g2}-NR^{e2}R^{f2},
   24) -C(=N-OR^{h2})NR^{g2}-NR^{e2}R^{f2},
   25) -NR^{e2}R^{f2},
   26) -NR^{g2}-NR^{e2}R^{f2},
   27) -NR^{e2}OR^{f2},
   28) -NR^{e2}-C(=O)R^{f2},
   29) -C (=O)NR^{e2}R^{f2},
   30) -C(=O)NR^{e2}OR^{f2},
   31) -C(=O)NR^{g2}-NR^{e2}R^{f2},
   32) -C(=O)R^{e2},
   33) -C (=O)OR^{e2}, and
   34) -C (=N-OR^{h2})NR^{e2}R^{f2}, and
each R^{6b} is selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C(=NR^{e2})R^{f2},
   6) -C(=N-OR^{e2})R^{f2},
   7) -SO₂-NR^{e2}R^{f2},
   8) -C(=NR^{h2})-NR^{e2}R^{f2},
   9) -C(=NR^{h2})NR⁹²-NR^{e2}R^{f2},
   10) -C(=N-OR^{h2})NR^{g2}-NR^{e2}R^{f2},
   11) -C(=O)NR^{e2}R^{f2},
   12) -C(=O)NR^{e2}OR^{f2},
   13) -C(=O)NR^{g2}-NR^{e2}R^{f2},
   14) -C(=O)R^{e2}, and
   15) -C(=N-OR^{h2})NR^{e2}R^{f2}.

In some embodiments, R⁵ is 5- or 6-membered aryl or heteroaryl selected from the group consisting of 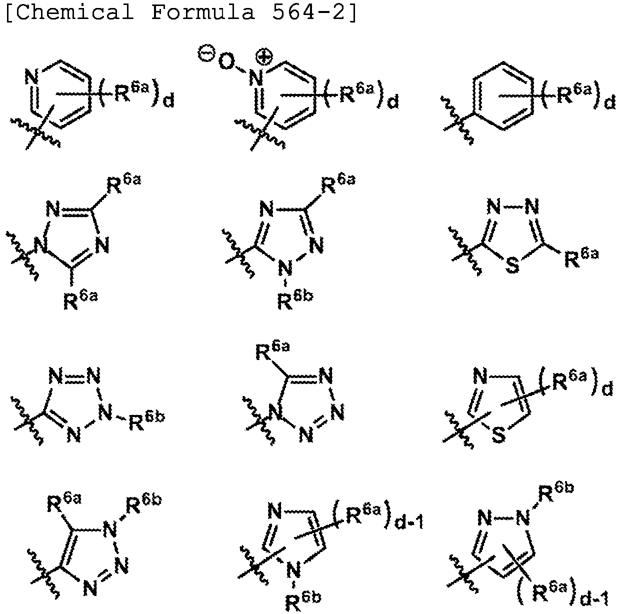 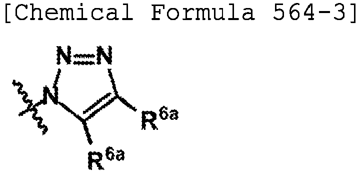 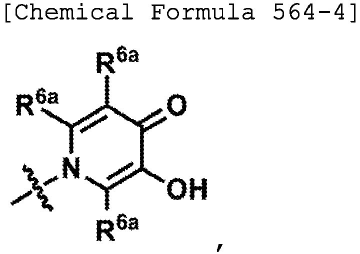 subscript d is the number of substitutable positions on a ring of R⁵,
each R^{6a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e2}R^{f2}, a 5- or 6-membered non-aryl heterocycle, -C(=O)OR^{f2}, or a hydroxyl group),
   5) a C₁₋₄ alkoxy group,
   6) -NR^{e2}R^{f2}, and
   7) -C (=O)OR^{e2}, and
each R^{6b} is independently selected from the group consisting of
   1) a hydrogen atom,
   2) a hydroxyl group, and
   3) a C₁₋₄ alkyl group
      (wherein the alkyl group is optionally substituted with NR^{e2}R^{f2}, - C (=O)NR^{e2}R^{f2}, -C(=O)OR^{f2}, or a hydroxyl group).

In some embodiments, R⁵ is selected from the group consisting of 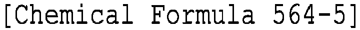 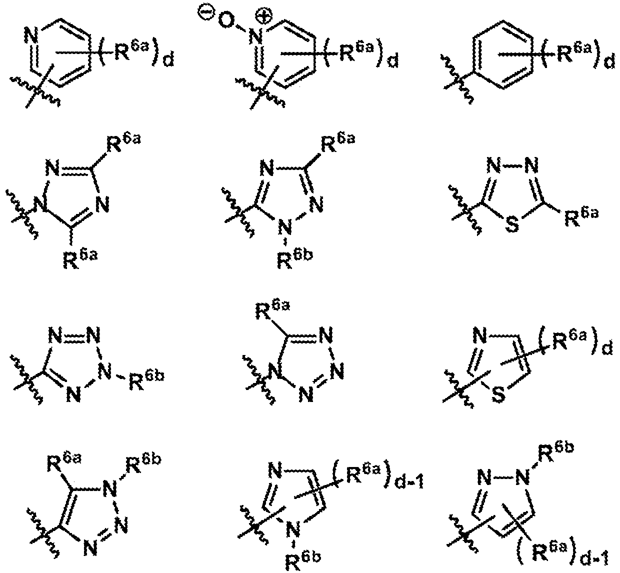 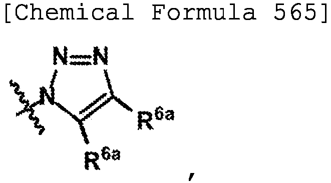 subscript d is the number of substitutable positions on a ring of R⁵,
each R^{6a} is independently selected from the group consisting of
   1) a hydrogen atom,
   2) a hydroxyl group,
   3) a cyano group,
   4) a nitro group,
   5) halogen,
   6) a C₁₋₄ alkyl group,
   7) a C₃₋₁₀ alicyclic group,
   8) a C₁₋₄ alkoxy group,
   9) a C₃₋₁₀ alicyclic oxy group,
   10) a C₆₋₁₀ aryloxy group,
   11) a 5- or 6-membered heteroaryloxy group,
   12) a 4- to 10-membered non-aryl heterocyclyl oxy group,
      (wherein each substituent from 6) to 12) is optionally substituted),
   13) -SO₂-NR^{e2}R^{f2},
   14) -NR^{g2}-CR^{e2}(=NR^{f2}),
   15) -NR^{g2}-CR^{e2}(=N-OR^{f2}),
   16) -NR^{h2}-C(=NR^{g2})NR^{e2}R^{f2},
   17) -NR^{h2}-C(=N-OR^{g2})NR^{e2}R^{f2},
   18) -NRⁱ²-C(=NR^{h2})NR^{g2}-NR^{e2}R^{f2},
   19) -NRⁱ²-C(=N-OR^{h2})NR^{g2}-NR^{e2}R^{f2},
   20) -C (=NR^{e2})R^{f2},
   21) -C (=N-OR^{e2})R^{f2},
   22) -C(=NR^{h2})-NR^{e2}R^{f2},
   23) -C(=NR^{h2})NR^{g2}-NR^{e2}R^{f2},
   24) -C(=N-OR^{h2})NR^{g2}-NR^{e2}R^{f2},
   25) -NR^{e2}R^{f2},
   26) -NR^{g2}-NR^{e2}R^{f2},
   27) -NR^{e2}OR^{f2},
   28) -NR^{e2}-C(=O)R^{f2},
   29) -C(=O)NR^{e2}R^{f2},
   30) -C(=O)NR^{e2}OR^{f2},
   31) -C(=O)NR^{g2}-NR^{e2}R^{f2},
   32) -C(=O)R^{e2},
   33) -C (=O)OR^{e2}, and
   34) -C(=N-OR^{h2})NR^{e2}R^{f2}, and
each R^{6b} is independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C (=NR^{e2})R^{f2},
   6) -C(=N-OR^{e2})R^{f2},
   7) -SO₂-NR^{e2}R^{f2},
   8) -C(=NR^{h2})-NR^{e2}R^{f2},
   9) -C (=NR^{h2})NR^{g2}-NR^{e2}R^{f2},
   10) -C(=N-OR^{h2})NR^{g2}-NR^{e2}R^{f2},
   11) -C(=O)NR^{e2}R^{f2},
   12) -C(=O)NR^{e2}OR^{f2},
   13) -C(=O)NR^{g2}-NR^{e2}R^{f2},
   14) -C(=O)R^{e2}, and
   15) -C (=N-OR^{h2})NR^{e2}R^{f2}.

In some embodiments, R⁵ is 5- or 6-membered aryl or heteroaryl selected from the group consisting of 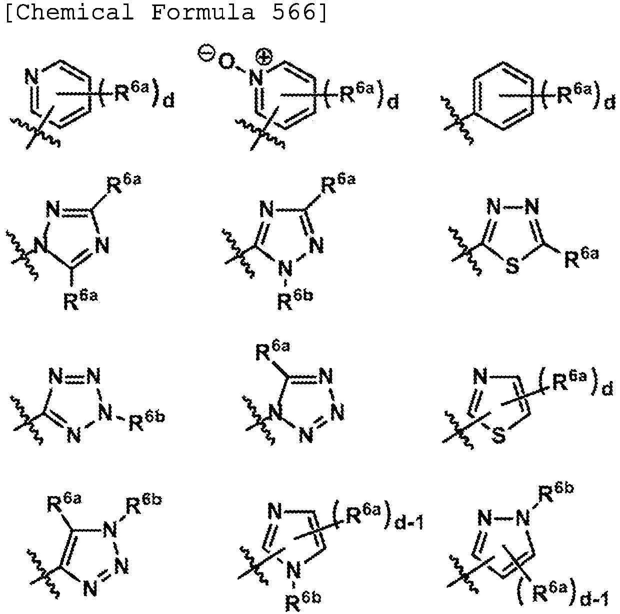 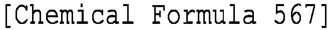 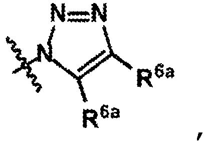 subscript d is the number of substitutable positions on a ring of R⁵,
each R^{6a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e2}R^{f2}, a 5- or 6-membered non-aryl heterocycle, -C(=O)OR^{f2}, or a hydroxyl group),
   5) a C₁₋₄ alkoxy group,
   6) -NR^{e2}R^{f2}, and
   7) -C (=O)OR^{e2}, and
each R^{6b} is independently selected from the group consisting of
   1) a hydrogen atom,
   2) a hydroxyl group, and
   3) a C₁₋₄ alkyl group
      (wherein the alkyl group is optionally substituted with NR^{e2}R^{f2}, - C (=O)NR^{e2}R^{f2}, -C(=O)OR^{f2}, or a hydroxyl group).

In some embodiments, R⁵ is

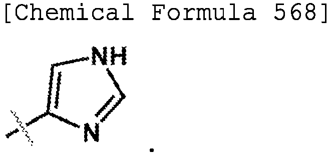

In some embodiments, R^{e2} and R^{f2} are the same or different, each independently a hydrogen atom, an optionally substituted C₁₋₆ alkyl group, or an optionally substituted C₃₋₁₀ alicyclic group. In some embodiments, R^{e2} and R^{f2} are the same or different, each independently a hydrogen atom or an optionally substituted C₁₋₆ alkyl group. In some embodiments, R^{e2} and R^{f2} are hydrogen atoms.

In some embodiments, R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

In some embodiments, R⁵ is a 4- to 6-membered non-aryl heterocycle selected from the group consisting of 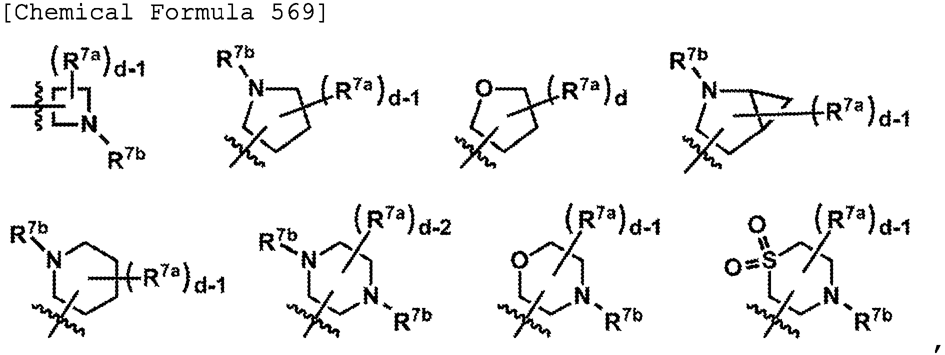 subscript d is the number of substitutable positions on a ring of R⁵,
each R^{7a} is independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group,
      (wherein each substituent from 5) and 11) is optionally substituted),
   12) -SO₂-NR^{e3}R^{f3},
   13) -NR^{g2}-CR^{e3}(=NR^{f3}),
   14) -NR^{g2}-CR^{e3}(=N-OR^{f3}),
   15) -NR^{h2}-C(=NR^{g2})NR^{e3}R^{f3},
   16) -NR^{h2}-C(=N-OR^{g2})NR^{e3}R^{f3},
   17) -NRⁱ²-C(=NR^{h2})NR^{g2}-NR^{e3}R^{f3},
   18) -NRⁱ²-C(=N-OR^{h2})NR^{g2}-NR^{e3}R^{f3},
   19) -C(=NR^{e3})R^{f3},
   20) -C(=N-OR^{e3})R^{f3},
   21) -C(=NR^{h2})-NR^{e3}R^{f3},
   22) -C (=NR^{h2})NR^{g2}-NR^{e3}R^{f3},
   23) -C(=N-OR^{h2})NR^{g2}-NR^{e3}R^{f3},
   24) -NR^{e3}R^{f3},
   25) -NR^{g2}-NR^{e3}R^{f3},
   26) -NR^{e3}OR^{f3},
   27) -NR^{e3}-C(=O)R^{f3},
   28) -C(=O)NR^{e3}R^{f3},
   29) -C(=O)NR^{e3}OR^{f3},
   30) -C(=O)NR^{g2}-NR^{e3}R^{f3},
   31) -C(=O)R^{e3},
   32) -C (=O)OR^{e3}, and
   33) -C(=N-OR^{h2})NR^{e3}R^{f3},
each R^{7b} is independently selected from the group consisting of
   1) a hydrogen atom,
   2) a hydroxyl group,
   3) a C₁₋₄ alkyl group
      (wherein the alkyl group is optionally substituted),
   4) a C₃₋₁₀ alicyclic group
      (wherein the alicyclic group is optionally substituted),
   5) -C(=NR^{e3})R^{f3},
   6) -C (=N-OR^{e3})R^{f3},
   7) -SO₂-NR^{e3}R^{f3},
   8) -C(=NR^{h2})-NR^{e3}R^{f3},
   9) -C (=NR^{h2})NR^{g2}-NR^{e3}R^{f3},
   10) -C(=N-OR^{h2})NR^{g2}-NR^{e3}R^{f3},
   11) -C (=O)NR^{e3}R^{f3},
   12) -C(=O)NR^{e3}OR^{f3},
   13) -C(=O)NR^{g2}-NR^{e3}R^{f3},
   14) -C (=O)R^{e3}, and
   15) -C(=N-OR^{h2})NR^{e3}R^{f3}, and
R^{e3}, R^{f3}, R^{g2}, R^{h2}, and Rⁱ² are defined the same as R^{e2}, R^{f2}, R^{g2}, R^{h2}, and Rⁱ² defined herein.

In some embodiments, R⁵ is a 4- to 6-membered non-aryl heterocycle selected from the group consisting of 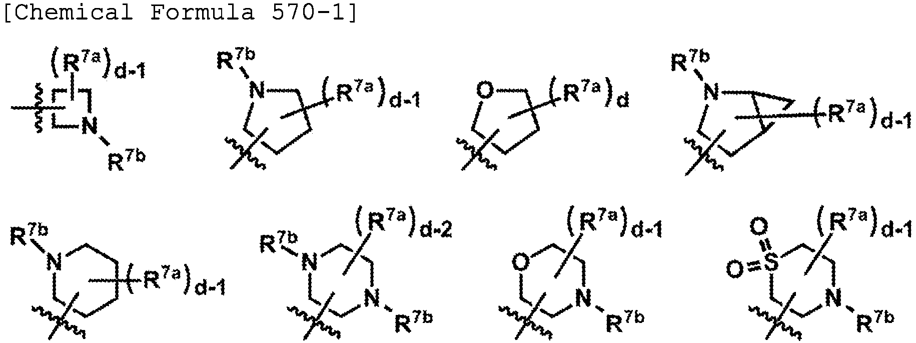 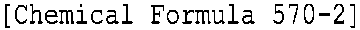 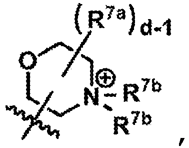 subscript d is the number of substitutable positions on a ring of R⁵,
each R^{7a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e3}R^{f3}, a 5- or 6-membered non-aryl heterocycle, -C(=O)OR^{f3}, or a hydroxyl group),
   5) a C₁₋₄ alkoxy group,
   6) -NR^{e3}R^{f3},
   7) -C (=O)OR^{e3},
   8) C₆₋₁₀ aryl, and
   9) -C(=O)NR^{e3}R^{f3},
each R^{7b} is independently selected from the group consisting of
   1) a hydrogen atom,
   2) a hydroxyl group, and
   3) a C₁₋₄ alkyl group
      (wherein the alkyl group is optionally substituted with NR^{e3}R^{f3}, - C(=O)OR^{f3}, or a hydroxyl group), and
R^{e3} and R^{f3} are defined the same as R^{e2} and R^{f2} described herein (e.g., any one of items 84 to 86).

In some embodiments, R⁵ is a 4- to 6-membered non-aryl heterocycle selected from the group consisting of 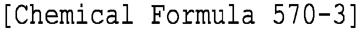 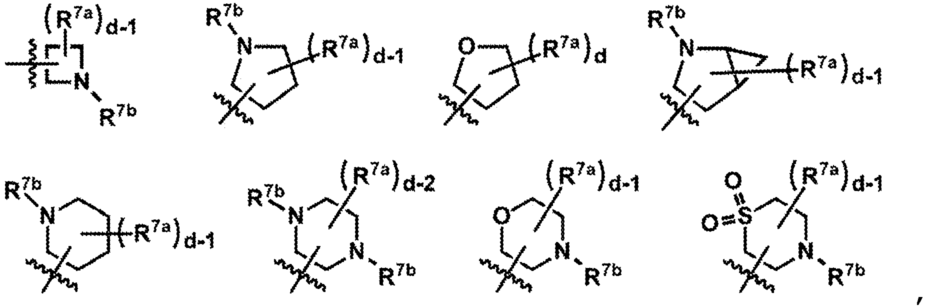 subscript d is the number of substitutable positions on a ring of R⁵,
each R^{7a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e3}R^{f3}, a 5- or 6-membered non-aryl heterocycle, -C(=O)OR^{f3}, or a hydroxyl group),
   5) a C₁₋₄ alkoxy group,
   6) -NR^{e3}R^{f3},
   7) -C(=O)OR^{e3},
   8) C₆₋₁₀ aryl, and
   9) -C(=O)NR^{e3}R^{f3},
each R^{7b} is independently selected from the group consisting of
   1) a hydrogen atom,
   2) a hydroxyl group, and
   3) a C₁₋₄ alkyl group
      (wherein the alkyl group is optionally substituted with NR^{e3}R^{f3}, - C(=O)OR^{f3}, or a hydroxyl group), and
R^{e3} and R^{f3} are defined the same as R^{e2} and R^{f2} defined herein.

In some embodiments, R⁵ is C₄₋₆ cycloalkyl selected from the group consisting of 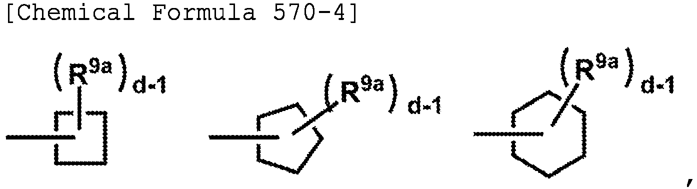 subscript d is the number of substitutable positions on a ring of R⁵,
each R^{9a} is independently selected from the group consisting of
   1) a hydrogen atom,
   2) a hydroxyl group,
   3) a cyano group,
   4) halogen,
   5) a C₁₋₄ alkyl group,
   6) a C₃₋₁₀ alicyclic group,
   7) a C₁₋₄ alkoxy group,
   8) a C₃₋₁₀ alicyclic oxy group,
   9) a C₆₋₁₀ aryloxy group,
   10) a 5- or 6-membered heteroaryloxy group,
   11) a 4- to 10-membered non-aryl heterocyclyl oxy group, (wherein each substituent from 5) and 11) is optionally substituted),
   12) -SO₂-NR^{e3}R^{f3},
   13) -NR^{g2}-CR^{e3}(=NR^{f3}),
   14) -NR^{g2}-CR^{e3}(=N-OR^{f3}),
   15) -NR^{h2}-C(=NR^{g2})NR^{e3}R^{f3},
   16) -NR^{h2}-C(=N-OR^{g2})NR^{e3}R^{f3},
   17) -NR¹²-C(=NR^{h2})NR^{g2}-NR^{e3}R^{f3},
   18) -NR¹²-C(=N-OR^{h2})NR^{g2}-NR^{e3}R^{f3},
   19) -C(=NR^{e3})R^{f3},
   20) -C(=N-OR^{e3})R^{f3},
   21) -C (=NR^{h2})-NR^{e3}R^{f3},
   22) -C (=NR^{h2})NR^{g2}-NR^{e3}R^{f3},
   23) -C(=N-OR^{h2})NR^{g2}-NR^{e3}R^{f3},
   24) -NR^{e3}R^{f3},
   25) -NR^{g2}-NR^{e3}R^{f3},
   26) -NR^{e3}OR^{f3},
   27) -NR^{e3}-C(=O)R^{f3},
   28) -C(=O)NR^{e3}R^{f3},
   29) -C(=O)NR^{e3}OR^{f3},
   30) -C(=O)NR^{g2}-NR^{e3}R^{f3},
   31) -C(=O)R^{e3},
   32) -C(=O)OR^{e3}, and
   33) -C (=N-OR^{h2})NR^{e3}R^{f3}, and
R^{e3}, R^{f3}, R^{g2}, R^{h2}, and R¹² are defined the same as R^{e2} and R^{f2} described herein (e.g., item 3).

In some embodiments, R⁵ is C₄₋₆ cycloalkyl selected from the group consisting of 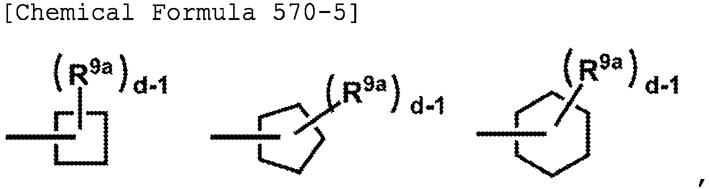 subscript d is the number of substitutable positions on a ring of R⁵,
each R^{9a} is independently selected from the group consisting of
   1) a hydrogen atom,
   2) a hydroxyl group,
   3) halogen,
   4) a C₁₋₄ alkyl group
      (wherein the alkyl group is optionally substituted with NR^{e3}R^{f3}, a 5- or 6-membered non-aryl heterocycle, -C(=O)OR^{f3}, or a hydroxyl group),
   5) a C₁₋₄ alkoxy group,
   6) -NR^{e3}R^{f3},
   7) -C (=O)OR^{e3},
   8) C₆₋₁₀ aryl, and
   9) -C (=O)NR^{e3}R^{f3}, and
R^{e3} and R^{f3} are defined the same as R^{e2} and R^{f2} described herein (e.g., any of items 75 to 77).

In some embodiments, L⁴ is -CH (NH₂)-CHR¹³-, wherein carbon that attaches to the NH₂ attaches to L³,
R⁵ is a hydrogen atom, and
R¹³ is
   1) -NH-C (=O)CH₃,
   2) -NH-C (=O)NH₂,
   3) -NH-C(=O)CH(NH₂)-CH₂C(=O)NH₂,
   4) -NH-C (=O)CH₂-NH₂,
   5) -NH-C (=O)CH (NH₂)-CH₂OH, or
   6) a pyrrolidin-2-ylcarbonylamino group.

In some embodiments, L⁴ is -CH (NH₂)-CR¹²R¹³-, wherein carbon that attaches to the NH₂ attaches to L³,
R⁵ is a hydrogen atom or methyl,
R¹² is a hydrogen atom or methyl, and
R¹³ is a benzylthio group or a sulfanyl group.

In some embodiments, L⁴ is -CH (NH₂)- (CH₂)_{q}-CHR¹³-, wherein q is 0 or 1, and carbon that attaches to the NH₂ attaches to L³,
R⁵ is a hydrogen atom, and
R¹³ is
   1) a carboxyl group,
   2) -C(=O)NH₂,
   3) -C(=O)NH(CH₃),
   4) -C(=O)N(CH₃)₂,
   5) -C(=O)NH-(CH₂)₂-OH,
   6) -C(=O)NH- (CH₂)₂-NH₂,
   7) -C(=O)NH-S (=O)₂-CH₃,
   8) -C(=O)NHOH,
   9) -S (=O)₂-NH₂,
   10) -S(=O)₂-CH₃, or
   11) a hydroxyl group.

In some embodiments, L⁴ is -CH (NHR¹¹) -CH₂-, wherein carbon that attaches to the NHR¹¹ attaches to L³,
R⁵ is hydrogen, and
R¹¹ is
   1) -C(=O)CH(NH₂)-CH₂C(=O)NH₂,
   2) -C(=O)CH₂-NH₂,
   3) -C(=O)CH(CH₃)-NH₂,
   4) -C(=O)CH(NH₂)-CH₂OH, or
   5) pyrrolidin-2-ylcarbonyl.

In some embodiments, L⁴ is -CH (NHR¹¹) -CH (COOH) -, wherein carbon that attaches to the NHR¹¹ attaches to L³,
R⁵ is hydrogen, and
R¹¹ is
   1) -C(=O)CH(NH₂)-CH₂C(=O)NH₂,
   2) -C(=O)CH₂-NH₂,
   3) -C(=O)CH(CH₃)-NH₂,
   4) -C(=O)CH(NH₂)-CH₂OH, or
   5) pyrrolidin-2-ylcarbonyl.

In some embodiments, L⁴ is -CHR¹³- or -CH₂-CHR¹³-,
R⁵ is hydrogen, and
R¹³ is -C(=O)NH₂ or -C(=O)NHOH.

In some embodiments, L⁴ is -CH₂CR¹⁰ (NH₂)-, wherein the CH₂ group attaches to L³,
R⁵ is hydrogen, and
R¹⁰ is a carboxy group or -C(=O)NH₂-

In some embodiments, L⁴ is - (CH₂)ₚ-CR¹⁰(NHR¹¹) - (CH₂)_{q}-CHR¹³- or -CHR¹³-(CH₂)_{q}-CR¹⁰(NHR¹¹)-(CH₂)ₚ-, wherein q is 0 or 1,
R⁵ is hydrogen,
   (1) if L⁴ is -CHR¹³-(CH₂)_{q}-CR¹⁰(NHR¹¹)-(CH₂)ₚ-, carbon of the -CHR¹³- group attaches to L³,
      p is 0,
      R¹⁰ is a hydrogen atom, a carboxyl group, or -C(=O)NHR^{10b},
      R¹¹ is a hydrogen atom,
      R^{10b} is a hydrogen atom,
         wherein if R¹⁰ is -C (=O)NHR^{10b}, R^{10b} and R¹¹ together may form - CH₂CH₂-, and
      R¹³ is a hydrogen atom, and
   (2) if L⁴ is -(CH₂)ₚ-CR¹⁰(NHR¹¹)-(CH₂)_{q}-CHR¹³-, carbon of the -(CH₂)ₚ- group attaches to L³,
      p is 1,
      R¹⁰ and R¹¹ are both hydrogen atoms,
      R¹³ is a carboxyl group or -C (=O)NR^{13a}R^{13b}, and
      R^{13a} and R^{13b} are each independently a hydrogen atom or an optionally substituted C₁₋₄ alkyl group.

In some embodiments, L⁴ is -CR¹²(NH₂)-,
R¹² is a hydrogen atom or a methyl group, and
R⁵ is an optionally substituted C₁₋₄ alkyl group with a hydroxyl group.

A specific example of a specific embodiment of the compound of the invention includes compounds represented by formulas (3a) and (3b): 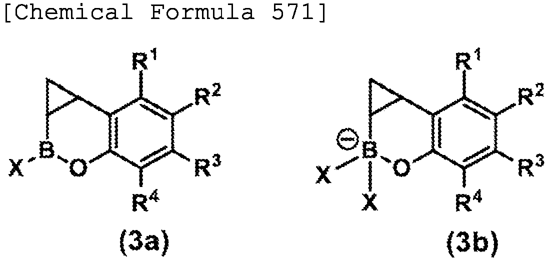 or a pharmaceutically acceptable salt thereof, wherein X, R¹, R², and R³ are as defined herein, and R⁴ is selected from the group consisting of
1) -C(=O)OR^{m1} (wherein R^{m1} is a hydrogen atom, a C₁₋₆ alkyl group, a C₃₋₁₀ alicyclic group, C₆₋₁₀ aryl, 5- or 6-membered heteroaryl, or a 4- to 10-membered non-aryl heterocycle, wherein the C₁₋₆ alkyl group, the C₃₋₁₀ alicyclic group, the C₆₋₁₀ aryl, the 5- or 6-membered heteroaryl, and the 4- to 10-membered non-aryl heterocycle are each optionally substituted), and
2) a bioisostere of 1).

A specific example of a preferred embodiment of the compound of the invention includes compounds represented by formulas (4a) and (4b): 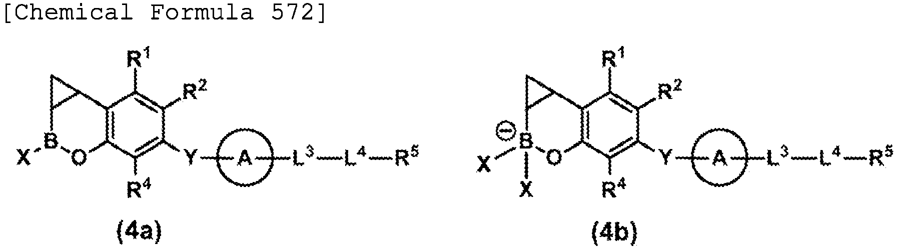 or a pharmaceutically acceptable salt thereof. In formulas (4a) and (4b), X, R⁴, Y, ring A, L³, L⁴, and R⁵ are defined the same as the definitions herein, and R¹ and R² are the same or different, each independently a hydrogen atom, a halogen atom, a C₁₋₆ alkyl group, a C₁₋₆ alkoxy group, or a C₁₋₆ alkylthio group (wherein the C₁₋₆ alkyl group, C₁₋₆ alkoxy group, and C₁₋₆ alkylthio group are optionally substituted).

A specific example of a more preferred embodiment of the compound of the invention includes compounds represented by formulas (5a) and (5b): 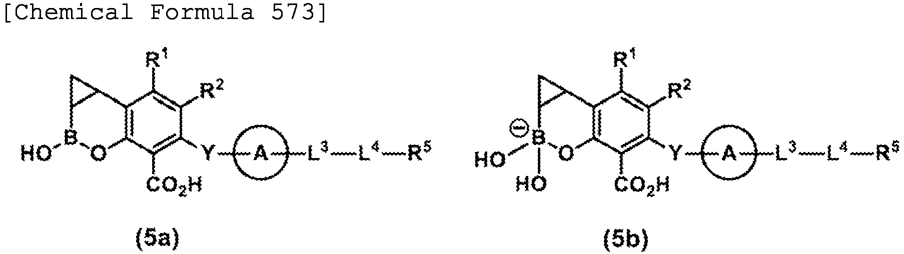 or a pharmaceutically acceptable salt thereof. In formulas (5a) and (5b), R¹, R², Y, L³, L⁴, and R⁵ are defined the same as the definitions herein, and ring A is an optionally substituted 4- to 6-membered nitrogen-containing non-aryl heterocycle.

A specific example of a still more preferred embodiment of the compound of the invention includes compounds represented by formulas (6a) and (6b): 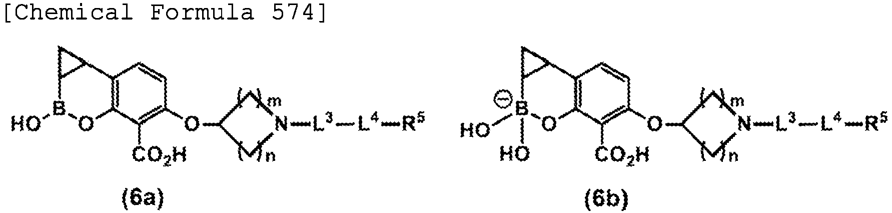 or a pharmaceutically acceptable salt thereof. In formulas (6a) and (6b), L³, L⁴, and R⁵ are defined the same as the definitions herein,
m is an integer 1, 2, or 3,
n is an integer 1, 2, or 3, and
m + n is 2, 3, or 4.

A specific example of a yet still more preferred embodiment of the compound of the invention includes enantiomers represented by formulas (7a) and (7b): 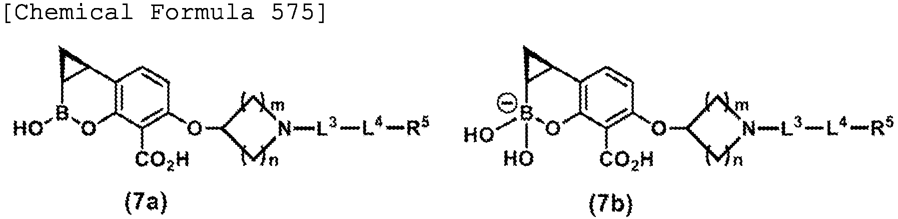 or formulas (8a) and (8b): 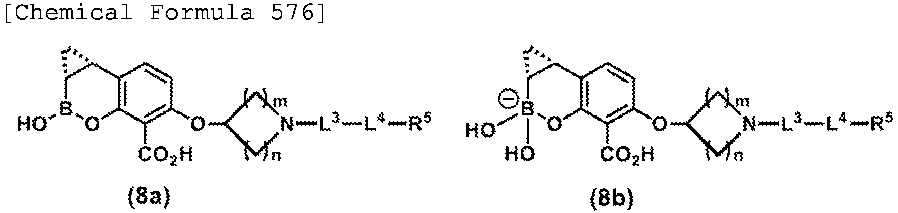 or a pharmaceutically acceptable salt thereof. In formulas (7a), (7b), (8a), and (8b), L³, L⁴, R⁵, m, and n are defined the same as the definitions herein.

A specific example of a preferred embodiment of the compound of the invention includes the following compounds:
compounds represented by 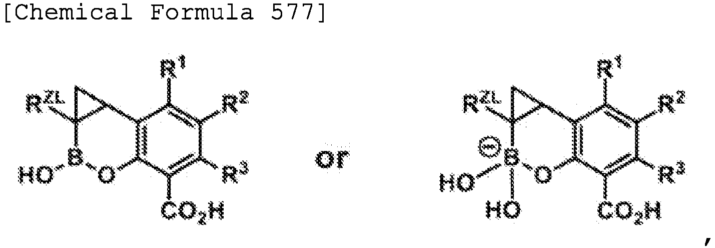 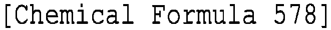 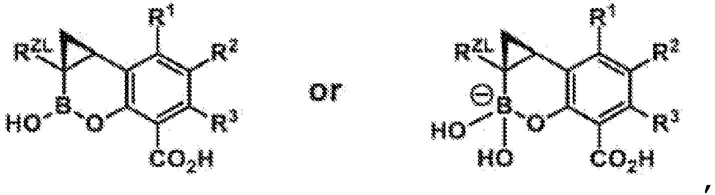 or 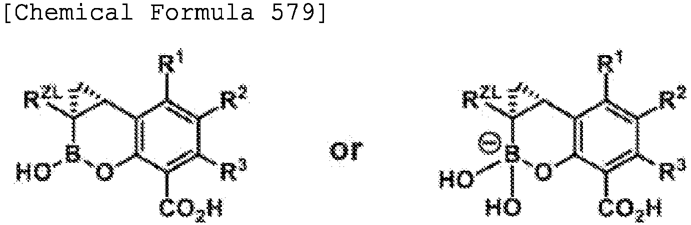 or a pharmaceutically acceptable salt thereof, wherein
R^{ZL} is a substituent selected from the group consisting of the Z1 to Z4 described below,
one of R¹, R², and R³ is 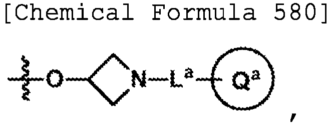 and
the remaining two are hydrogen atoms, linking group L^{a} is a substituent selected from the group consisting of L1 to L69 described below, and substituent Q^{a} is a substituent selected from the group consisting of Q1 to Q161 described below;

### R^{ZL}:

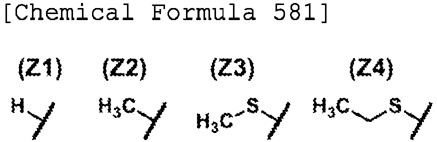

### linking group L^{a}:

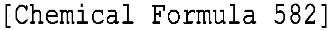

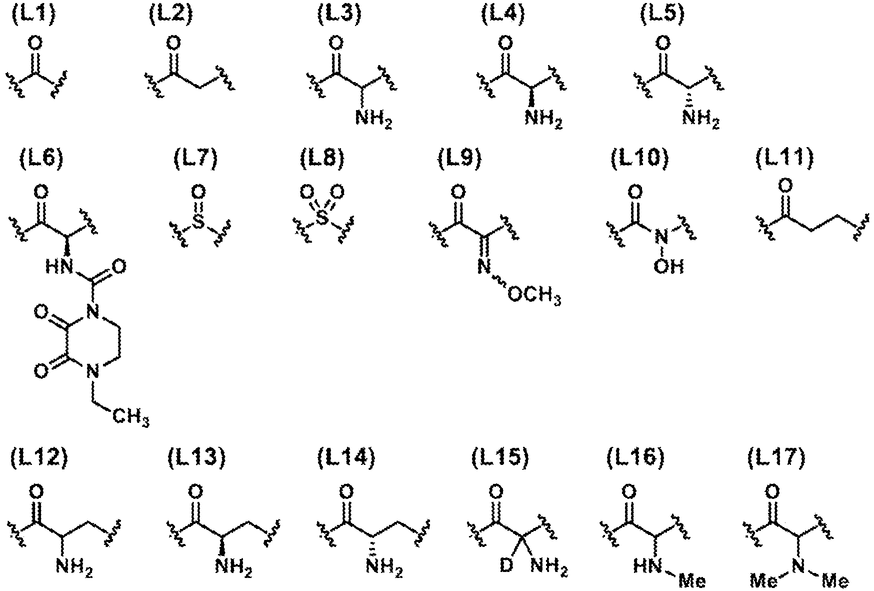

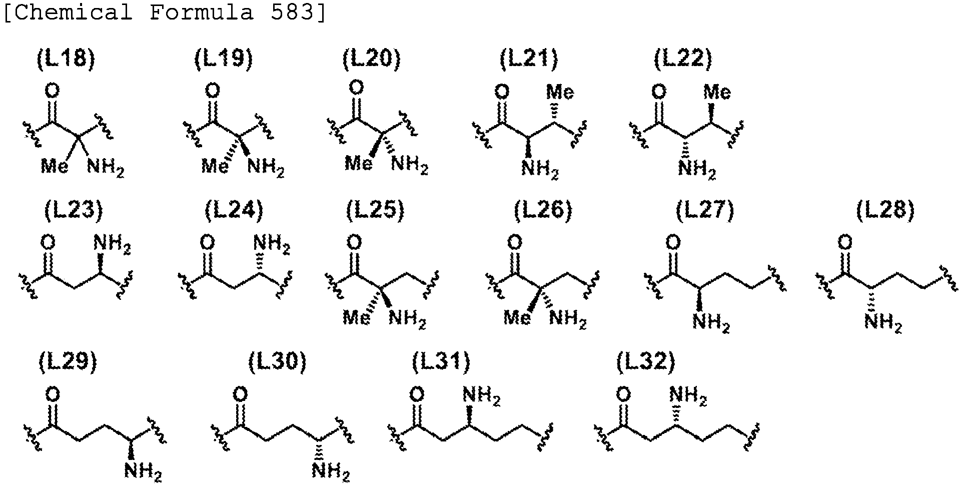

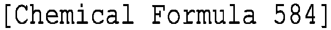

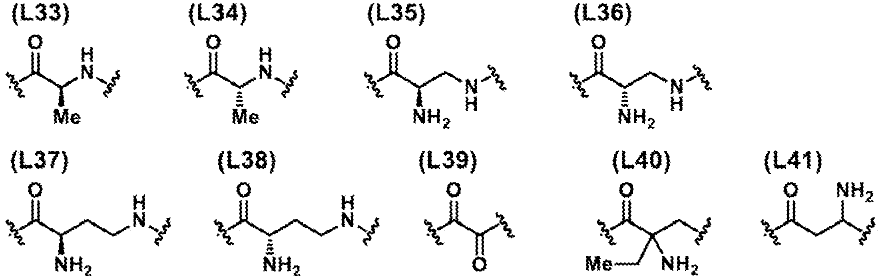

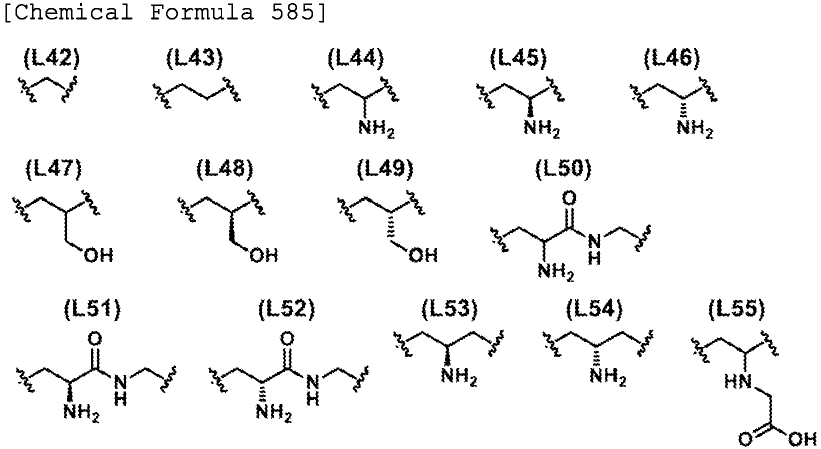

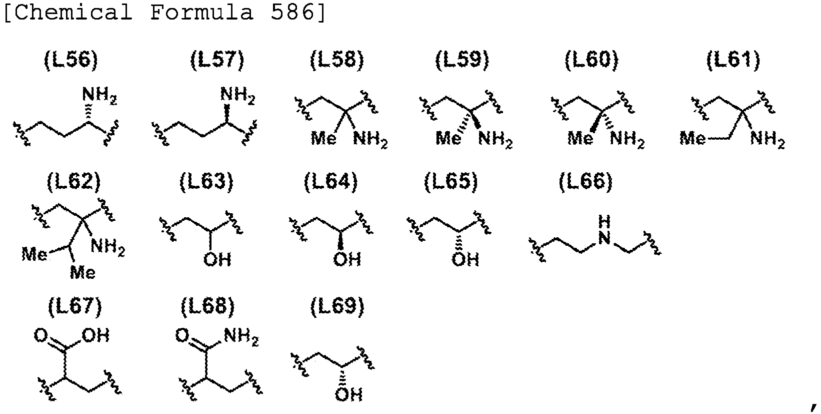

and

### substituent Q^{a}:

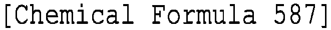

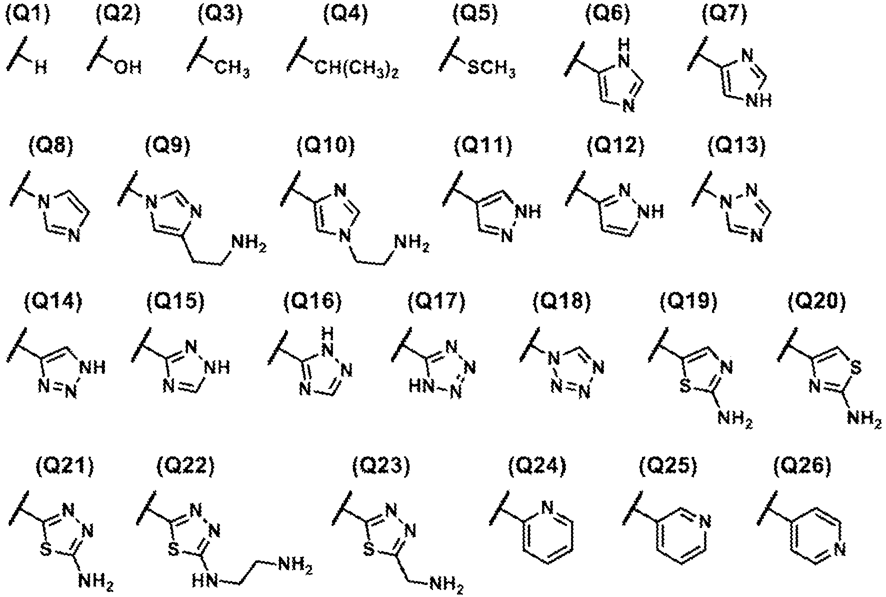

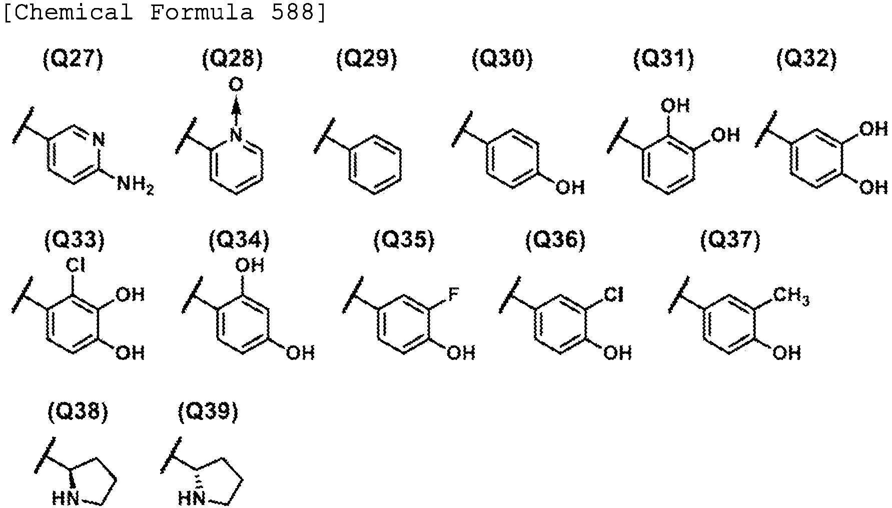

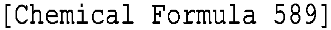

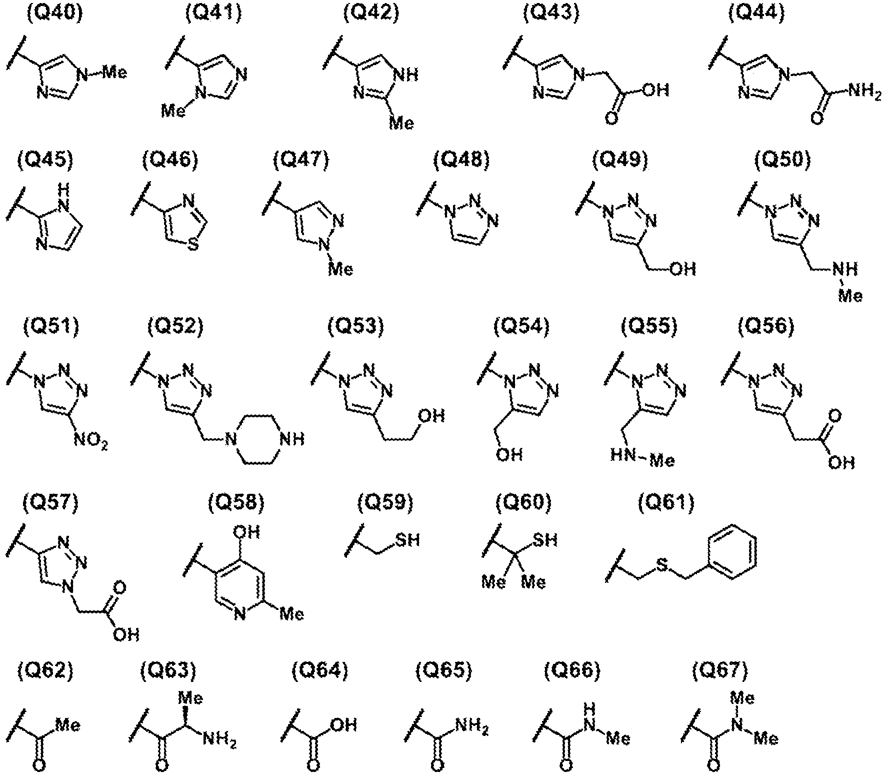

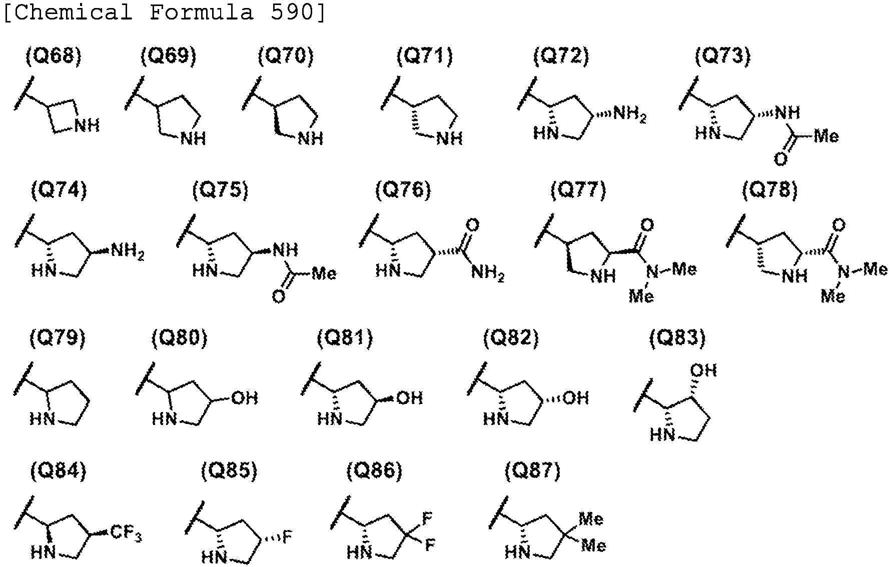

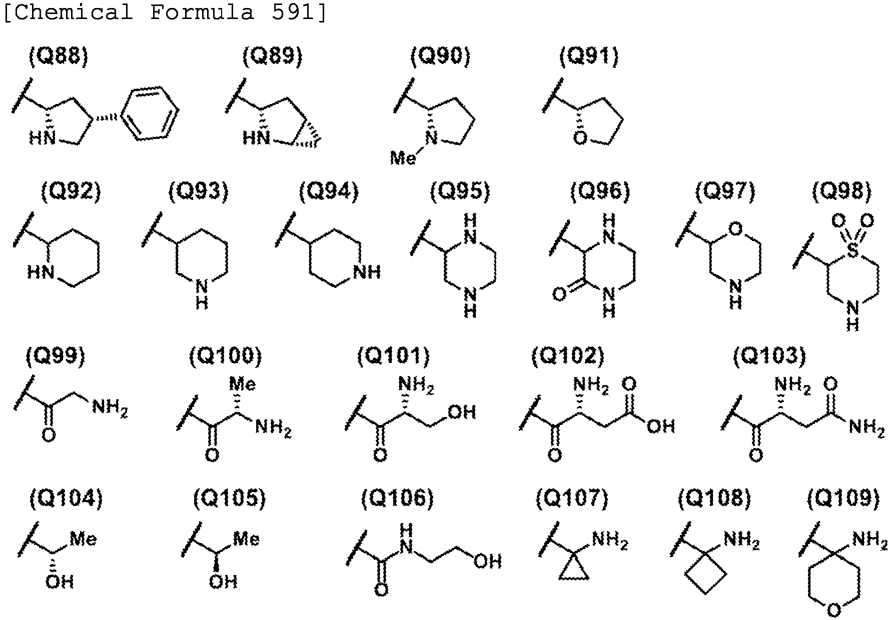

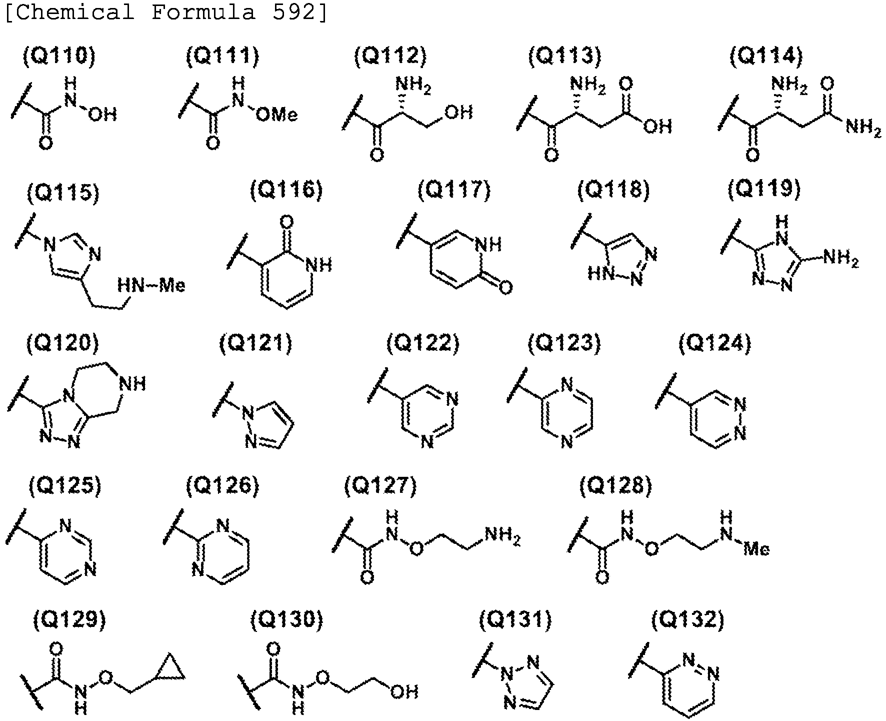

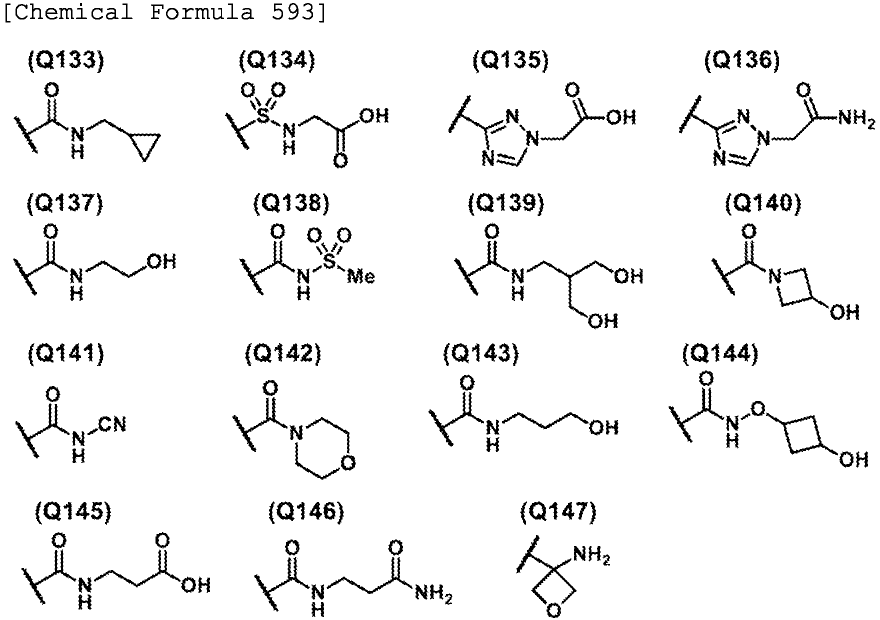

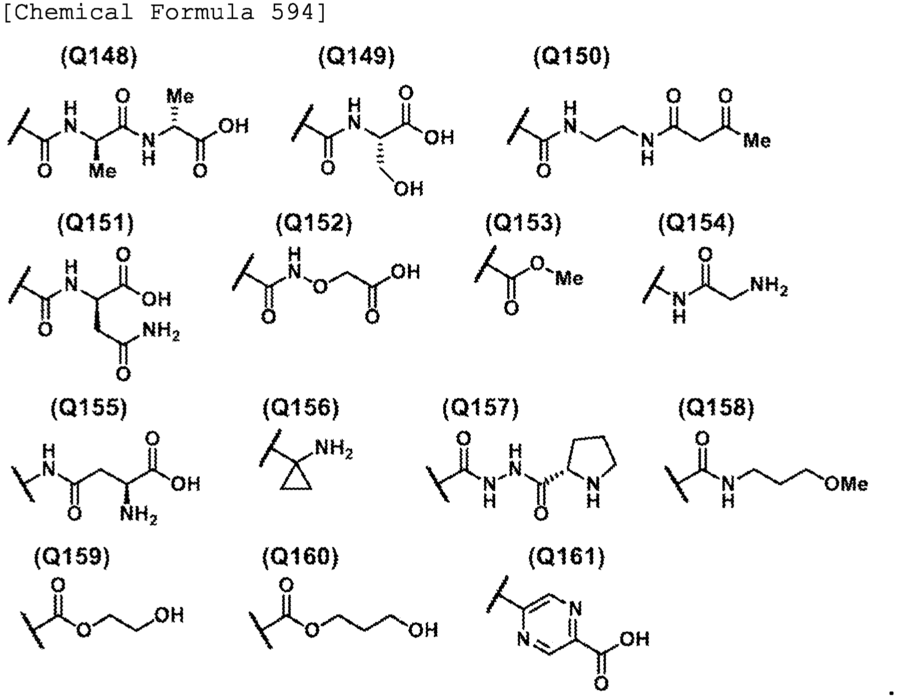

A specific example of a more preferred embodiment of the compound of the invention includes a compound of the following formula: 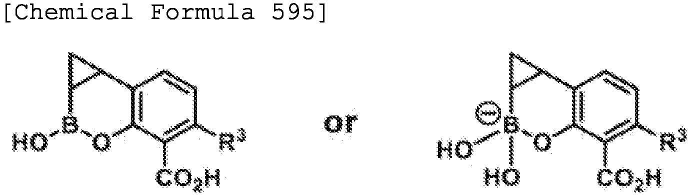 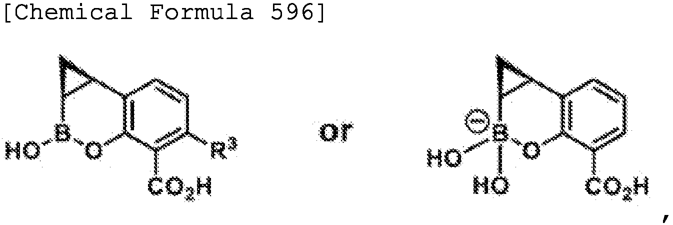 or 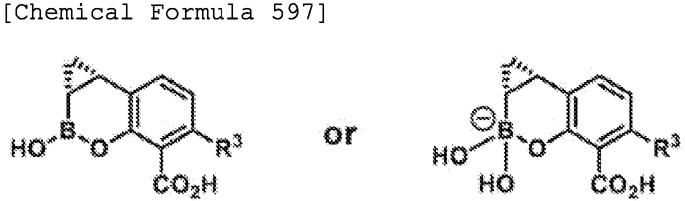 or a pharmaceutically acceptable salt thereof, wherein R³ is 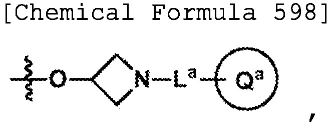 wherein linking group L^{a} is a substituent selected from the group consisting of L1 to L69 described above, and substituent Q^{a} is a substituent selected from the group consisting of Q1 to Q161 described above.

Examples of a more preferred embodiment of the compound of the invention include the compounds of the following Table (1) or a pharmaceutically acceptable salt thereof. 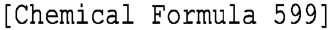 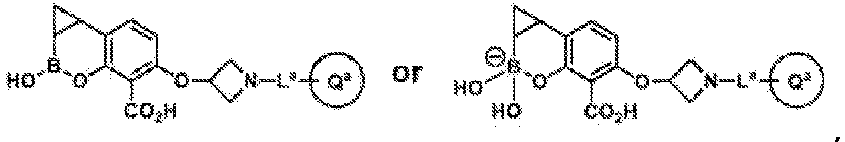 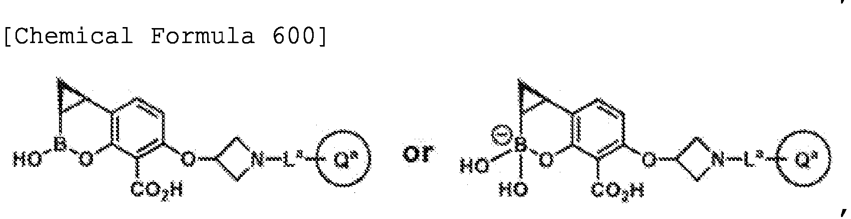 or

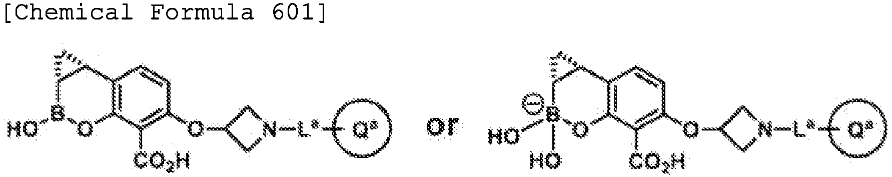

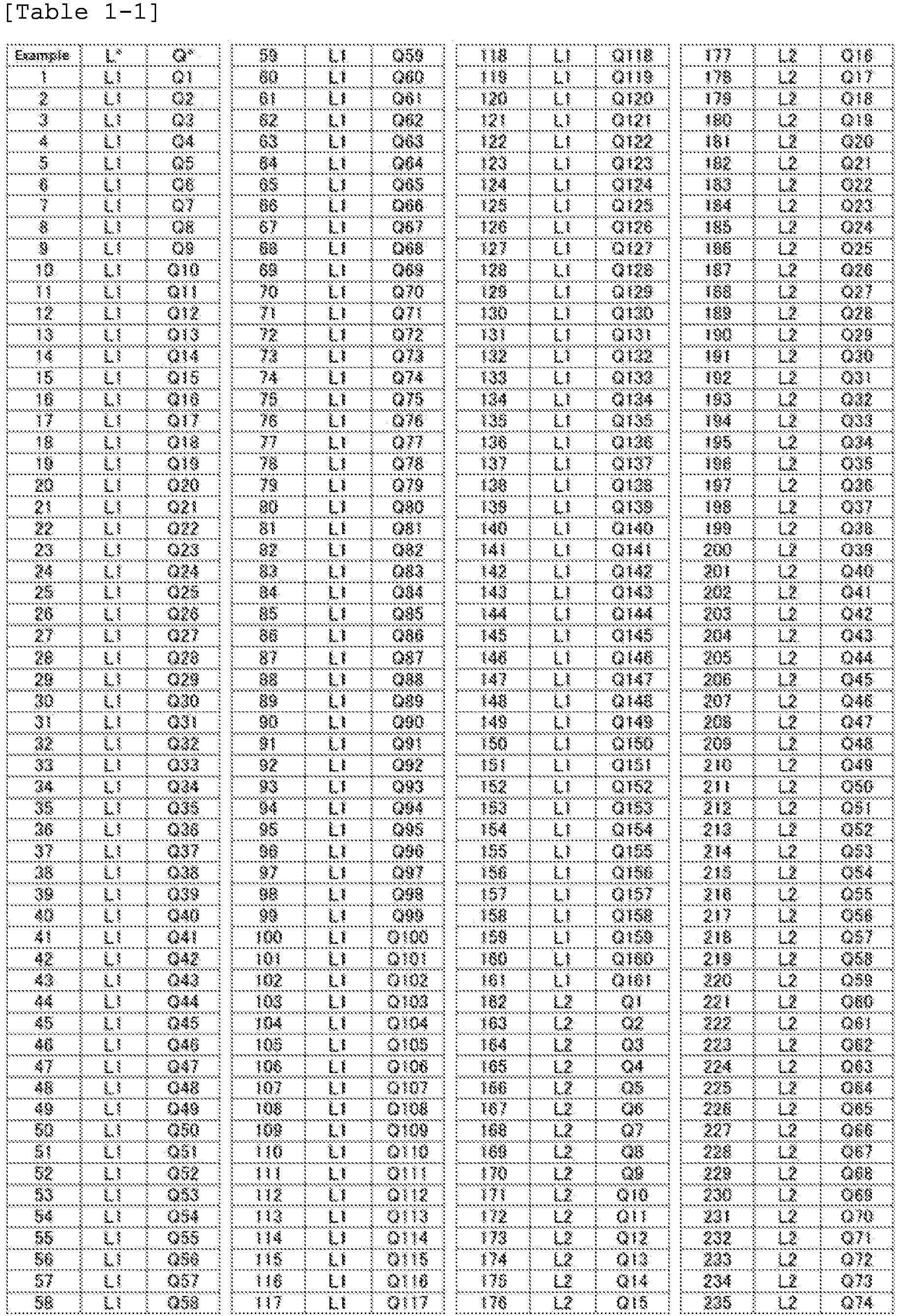

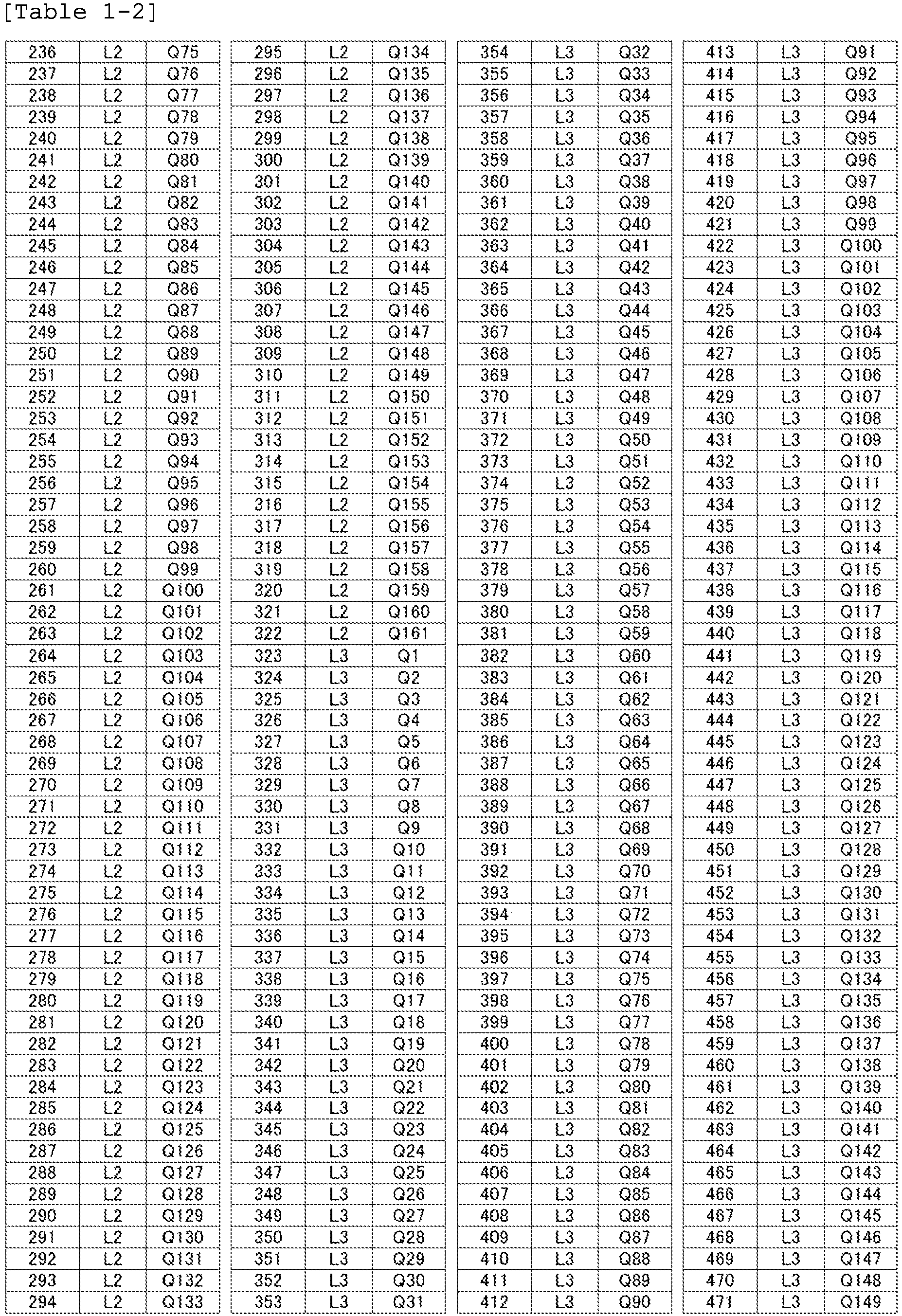

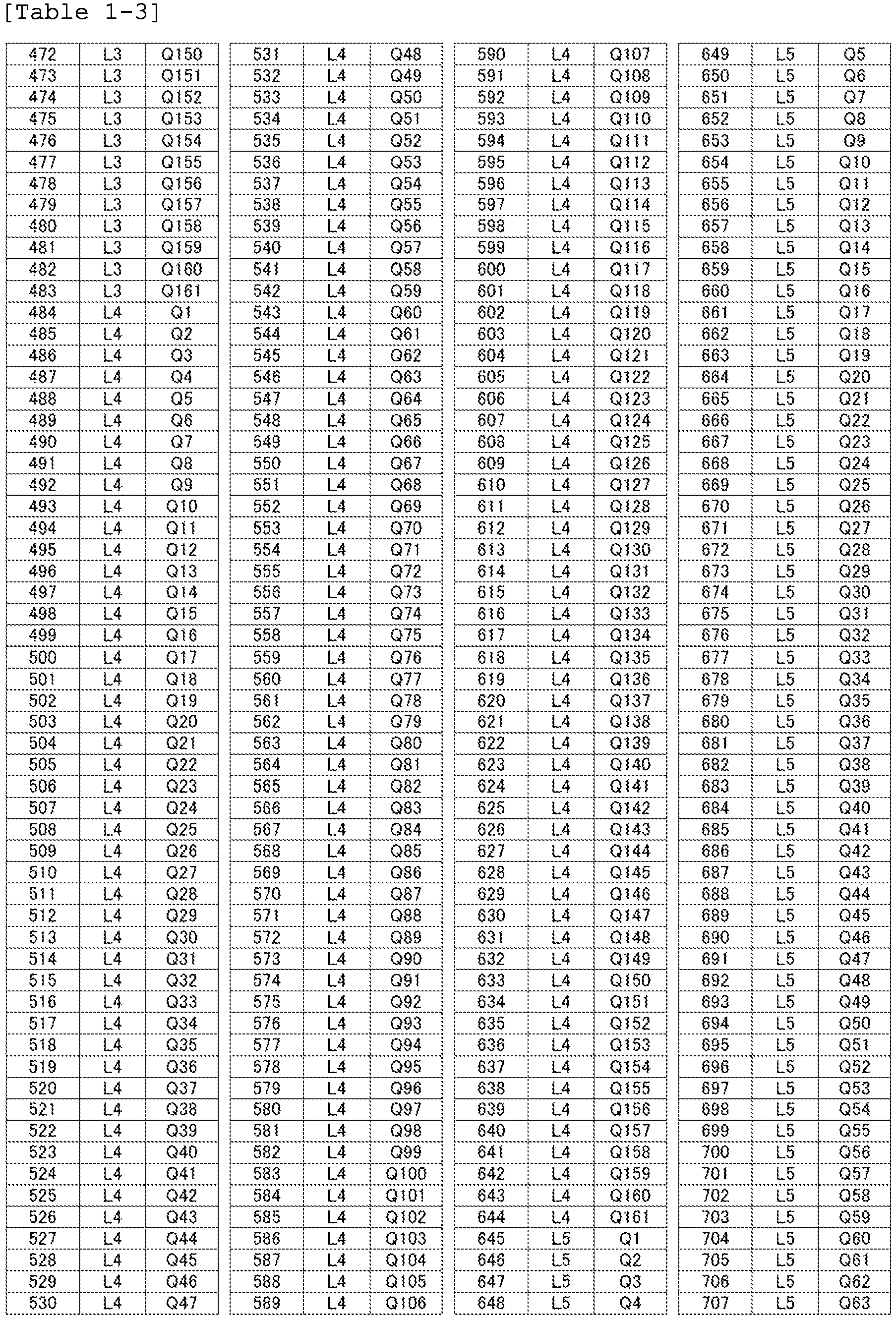

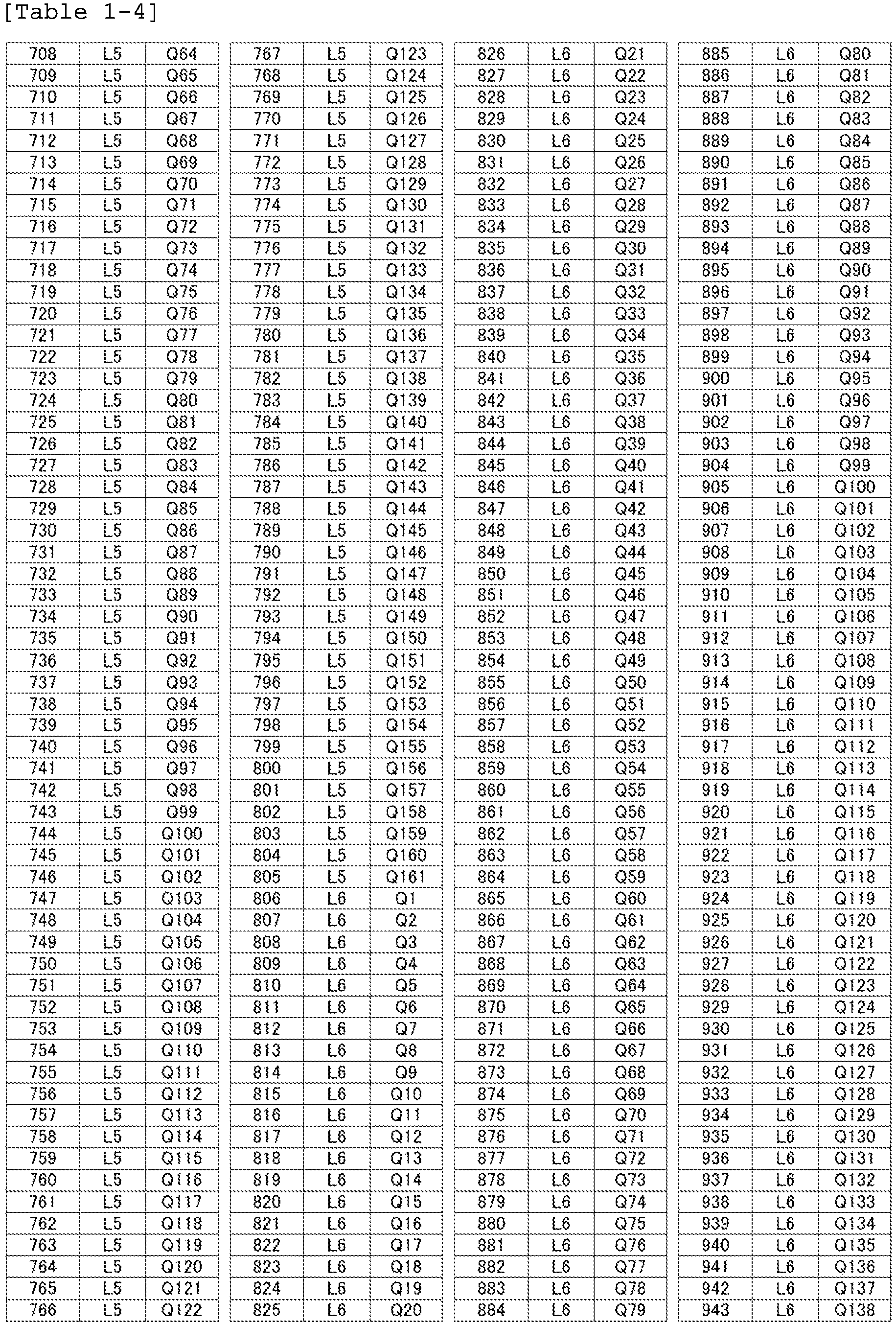

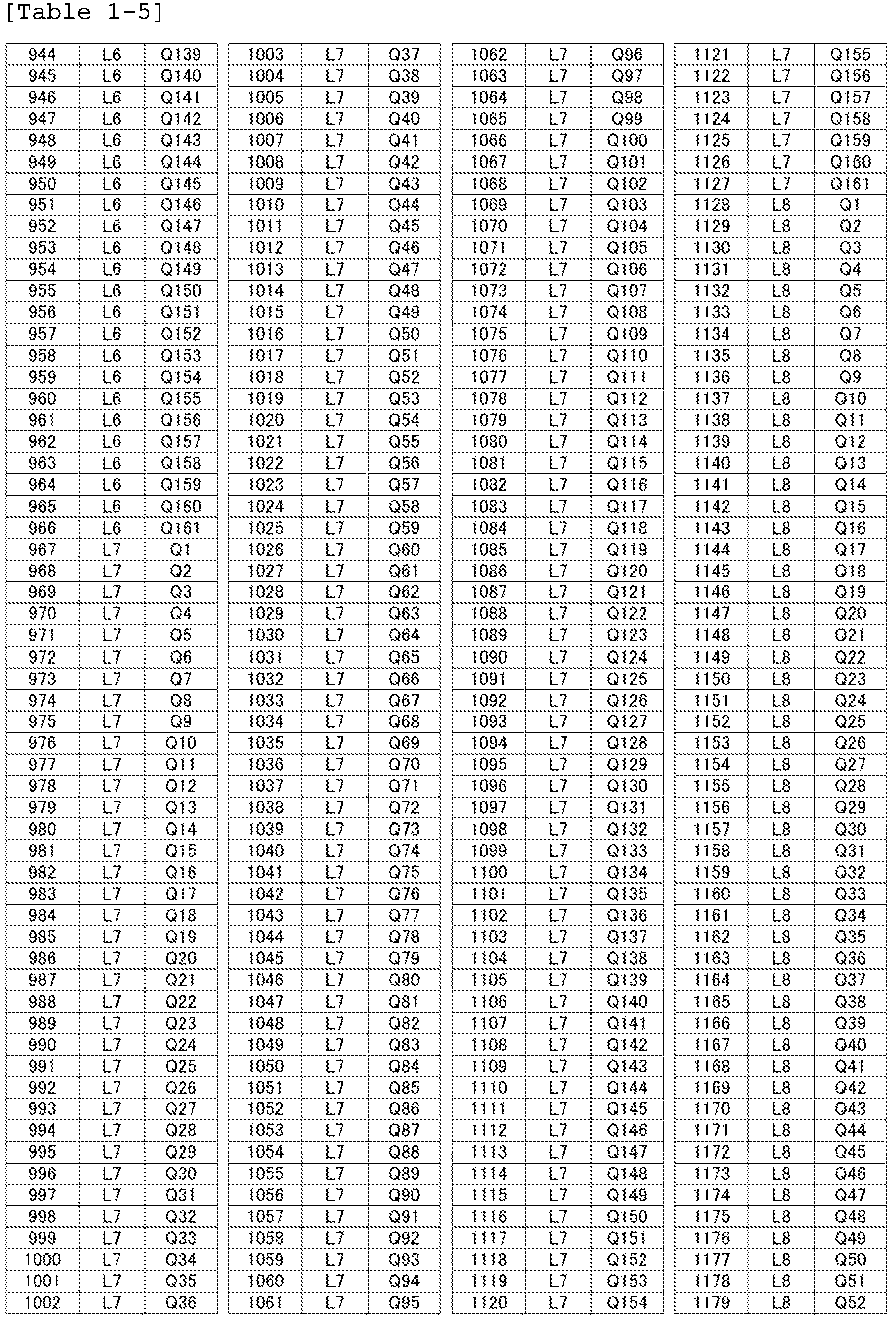

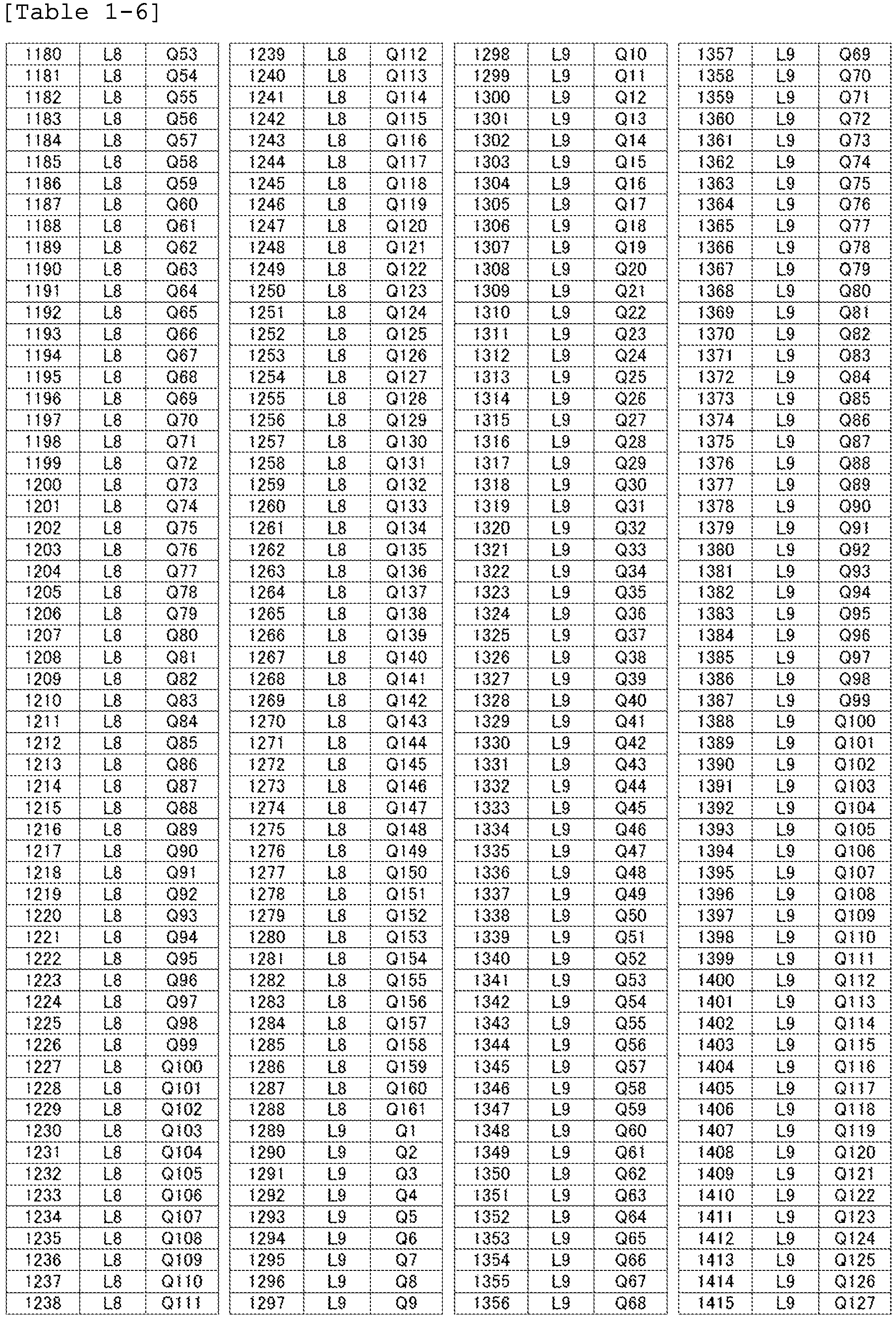

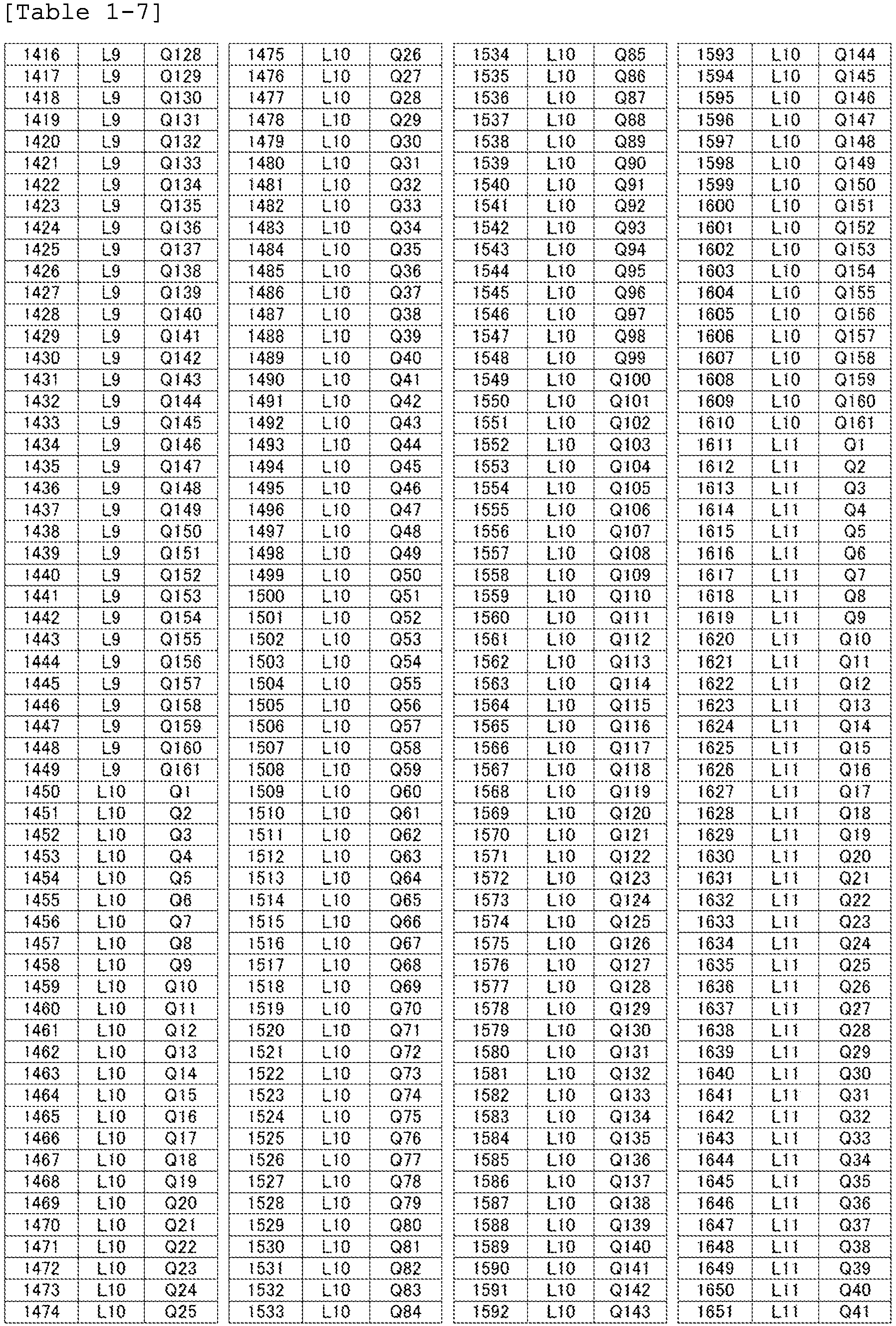

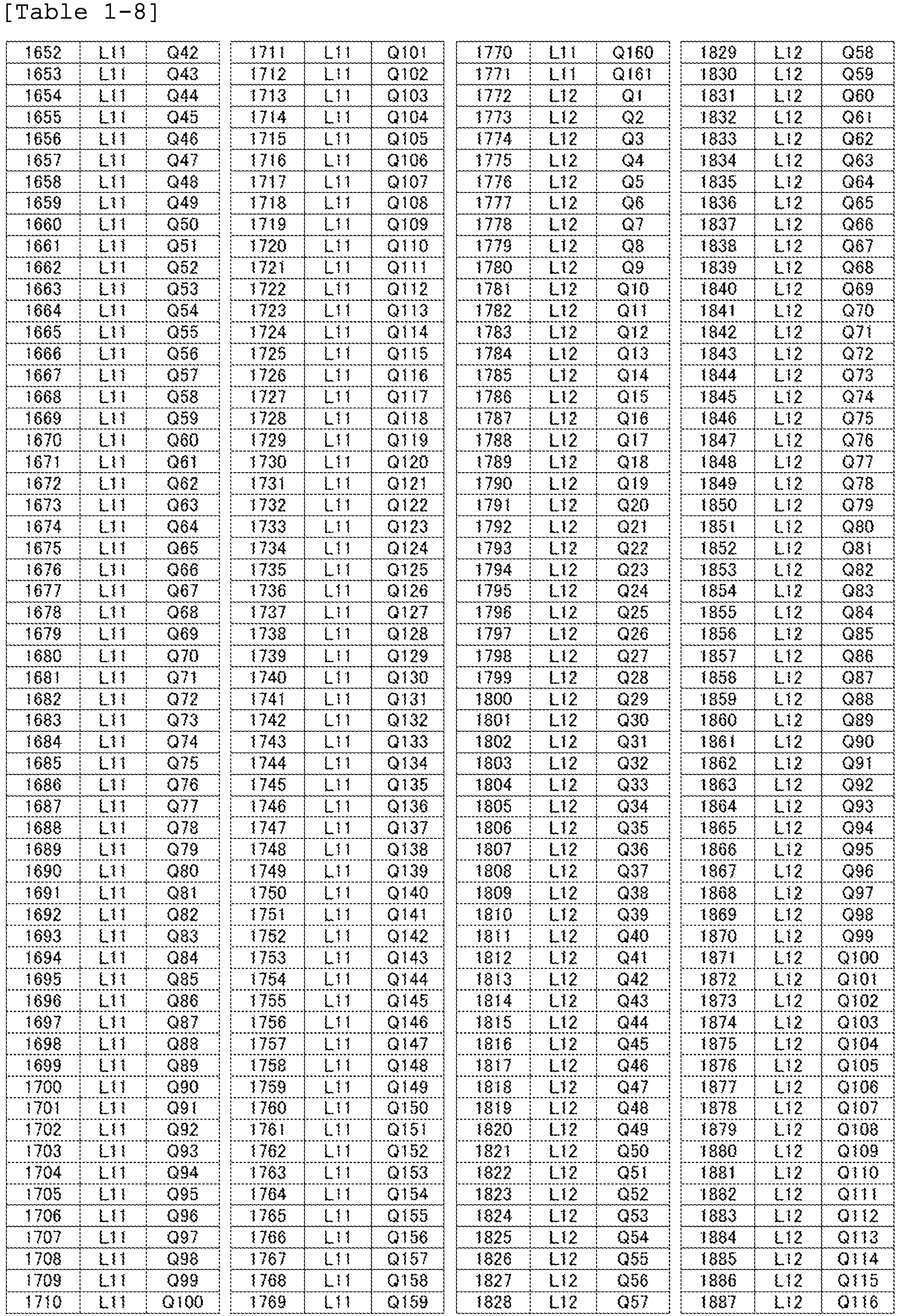

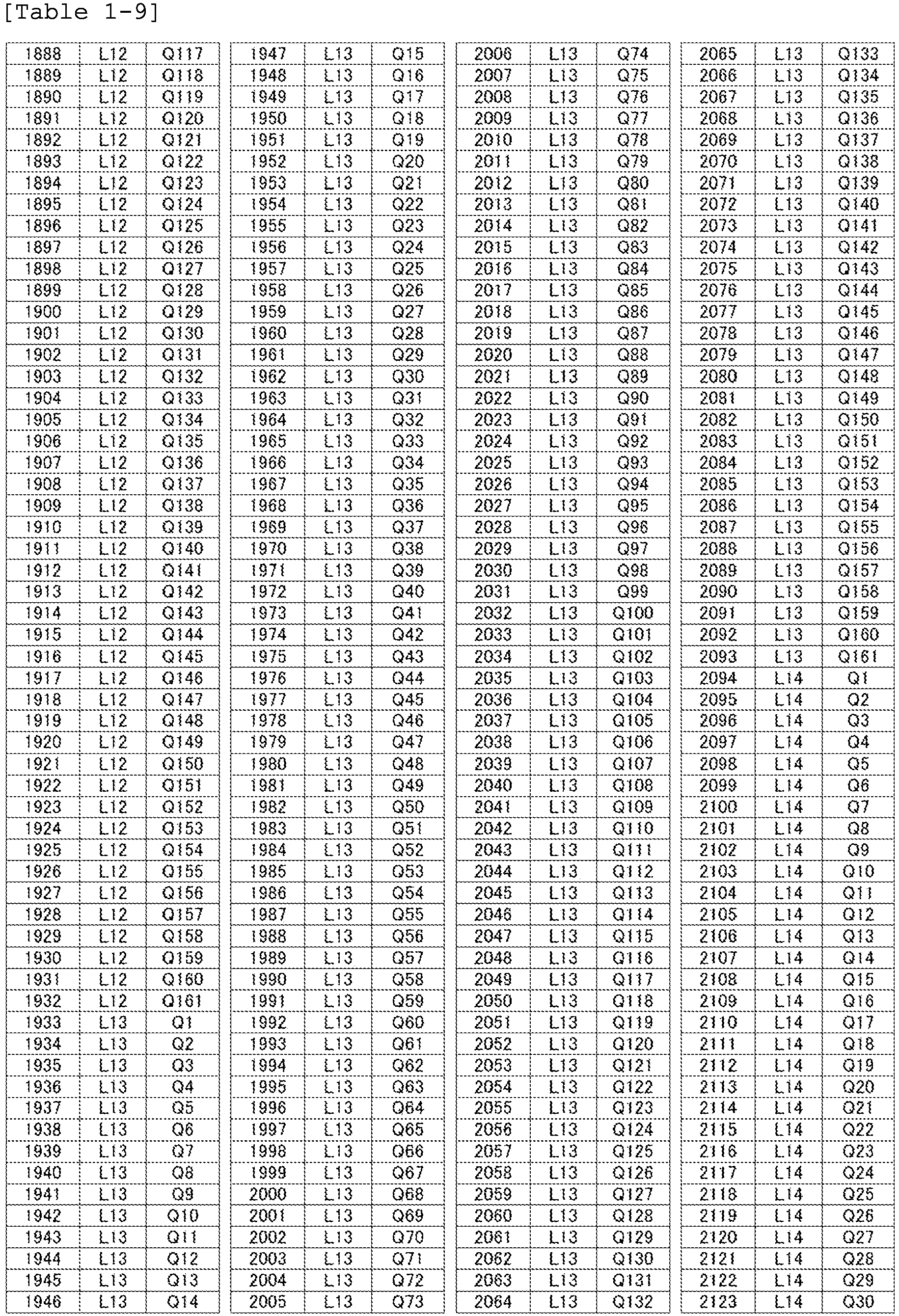

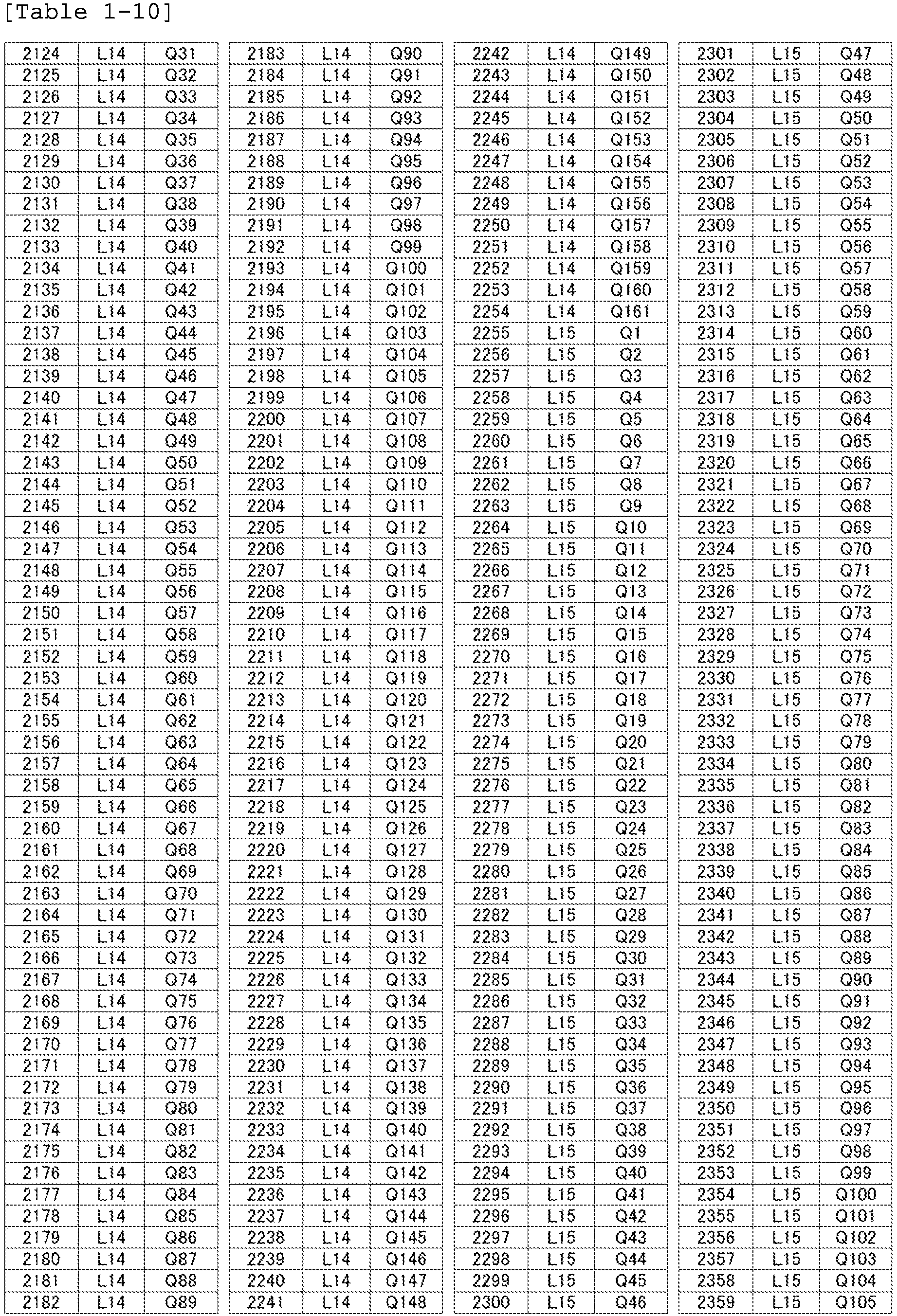

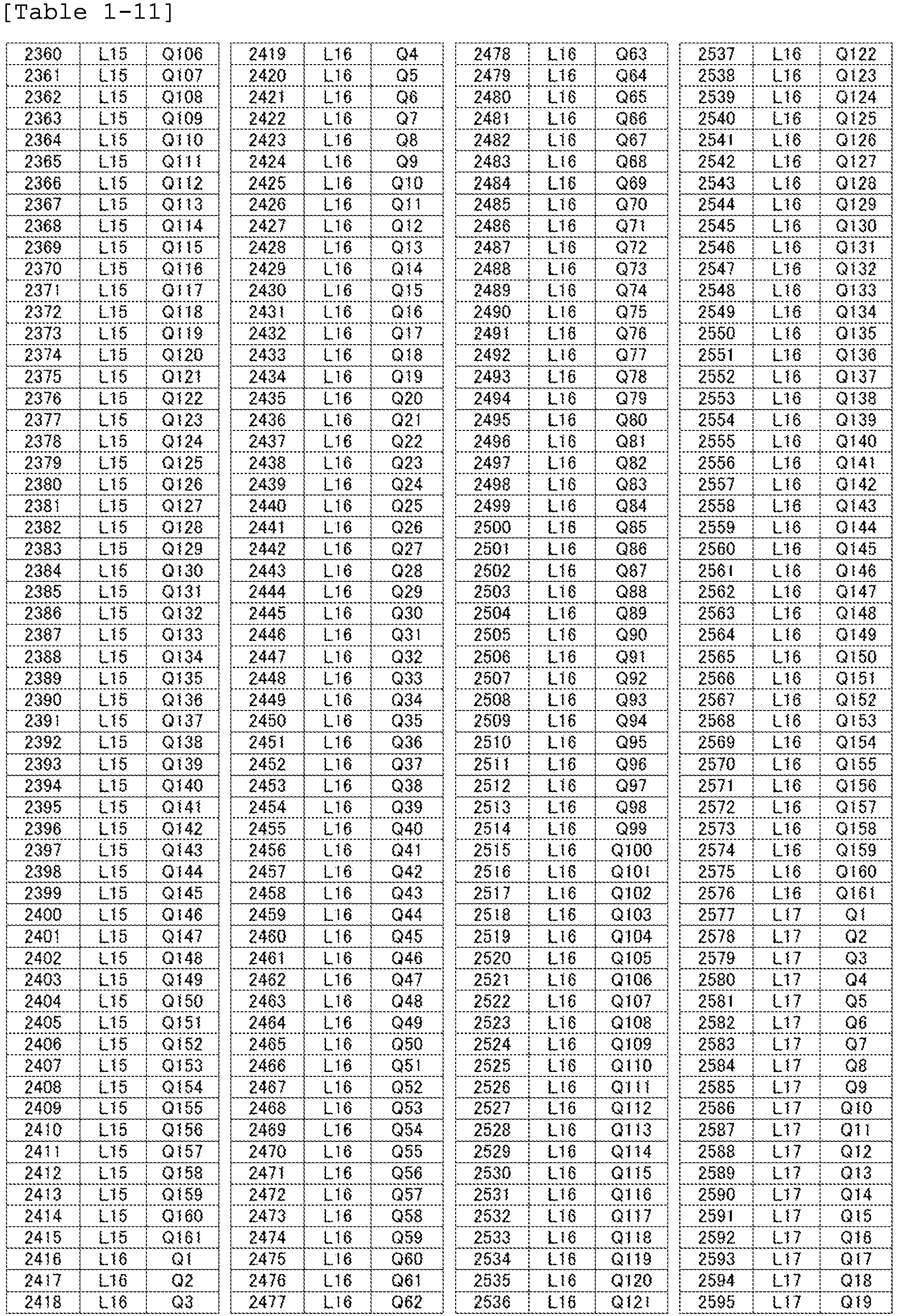

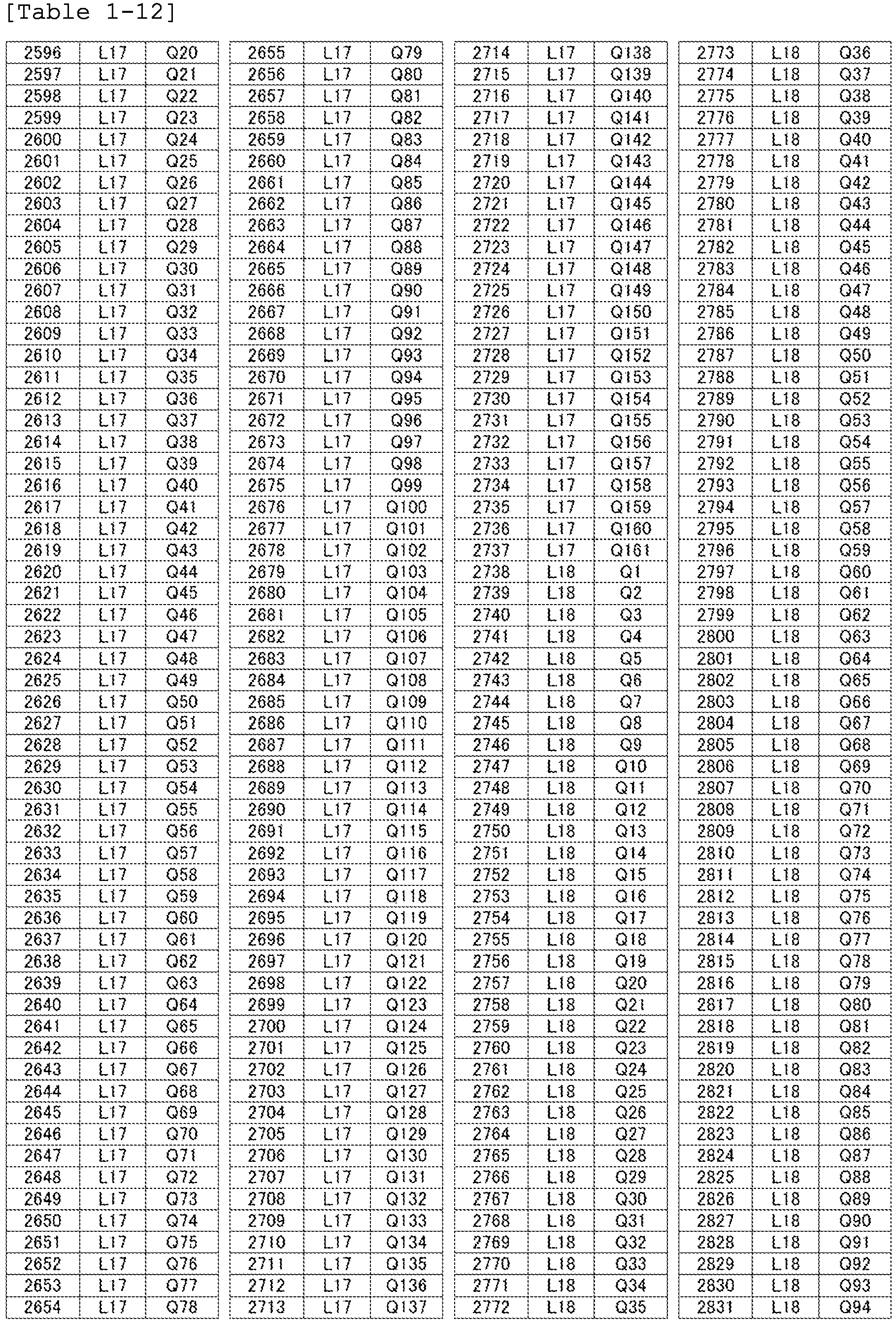

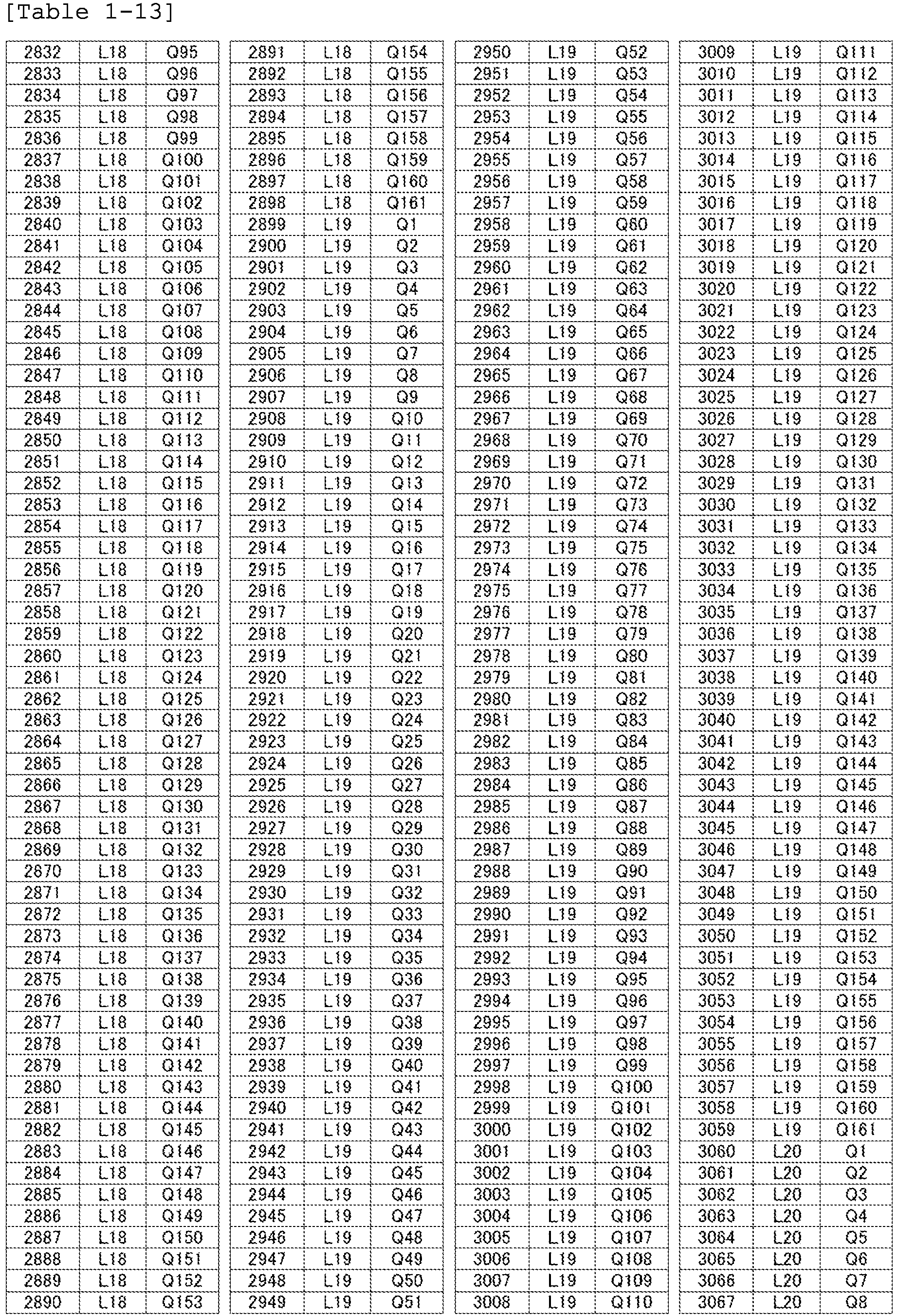

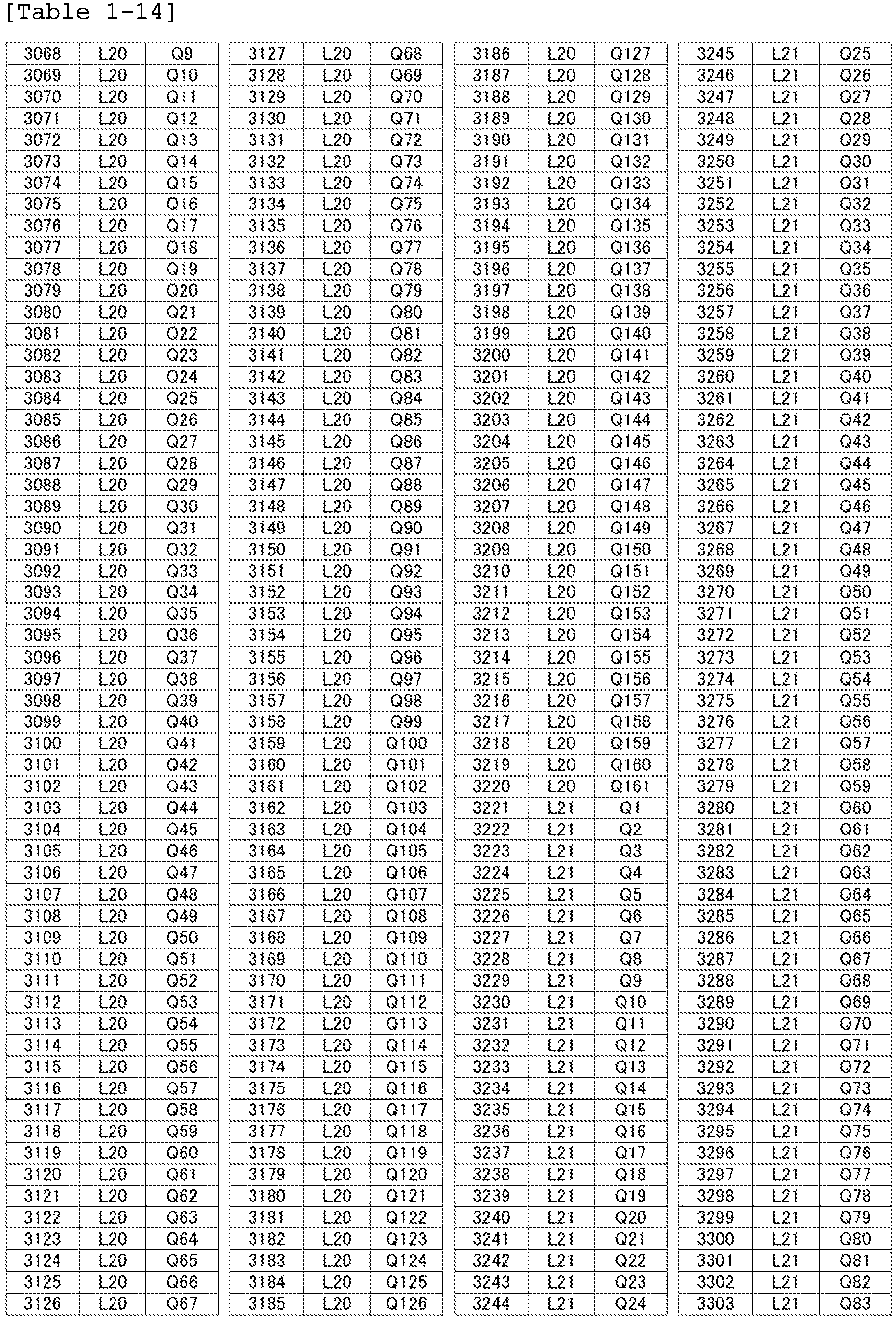

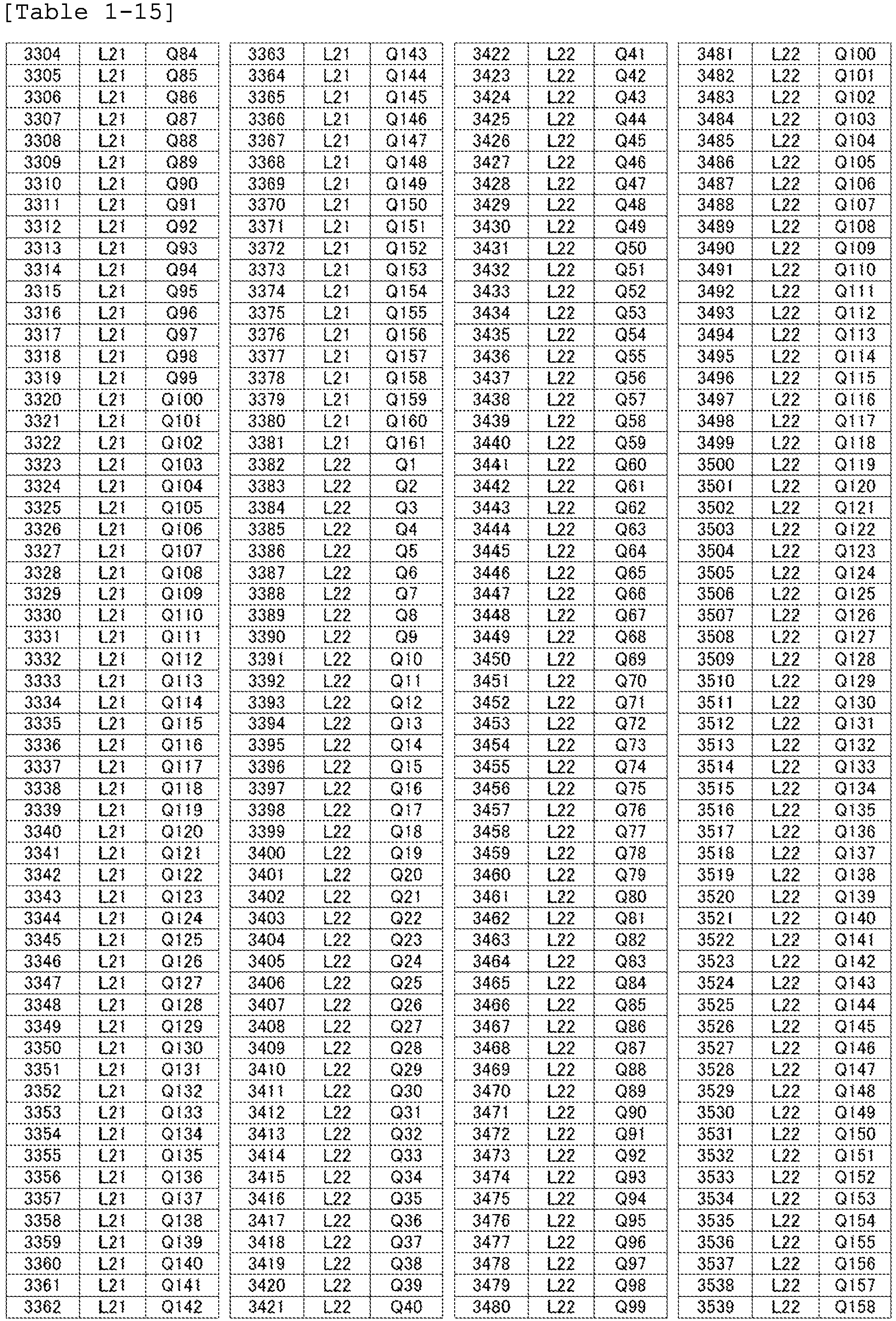

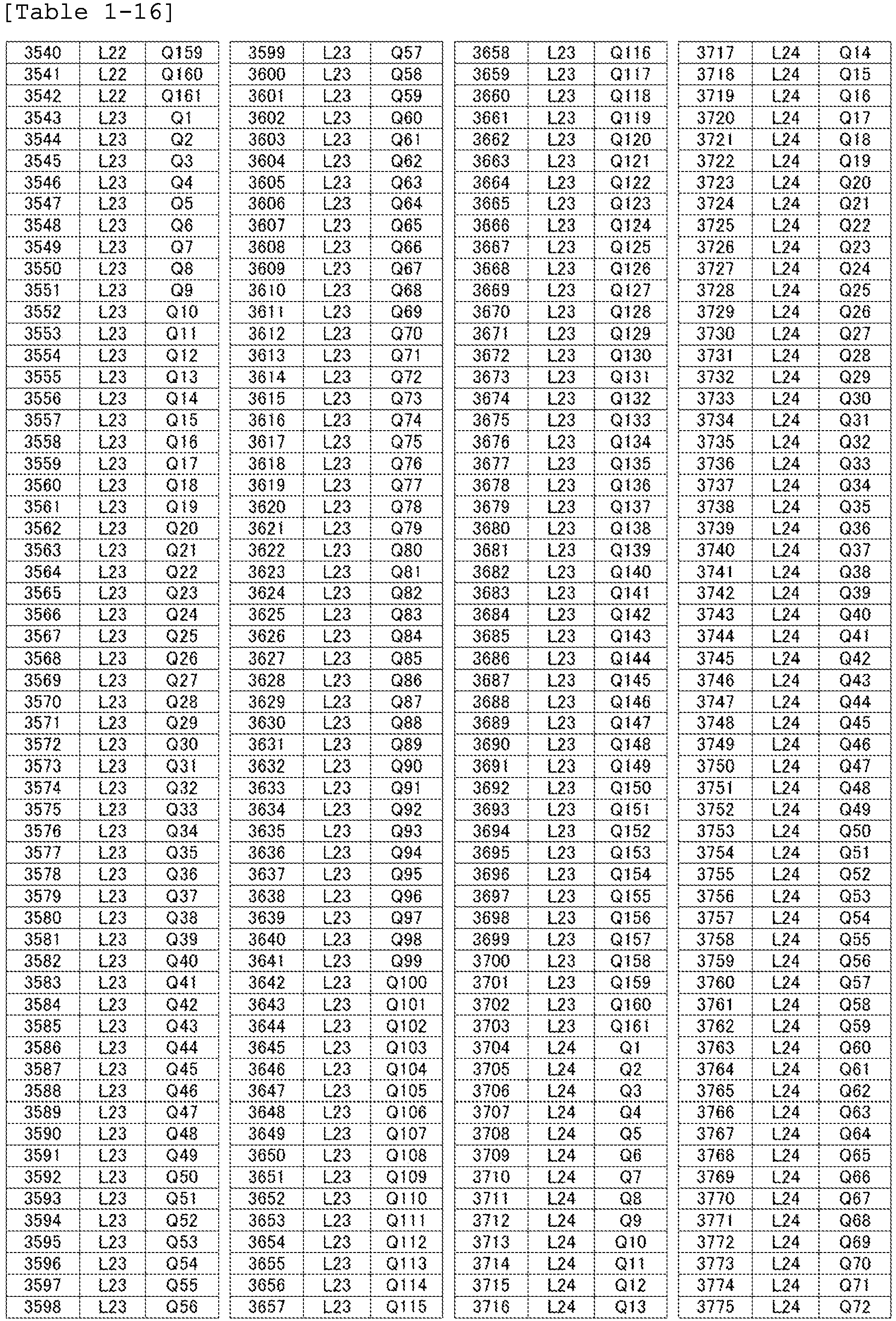

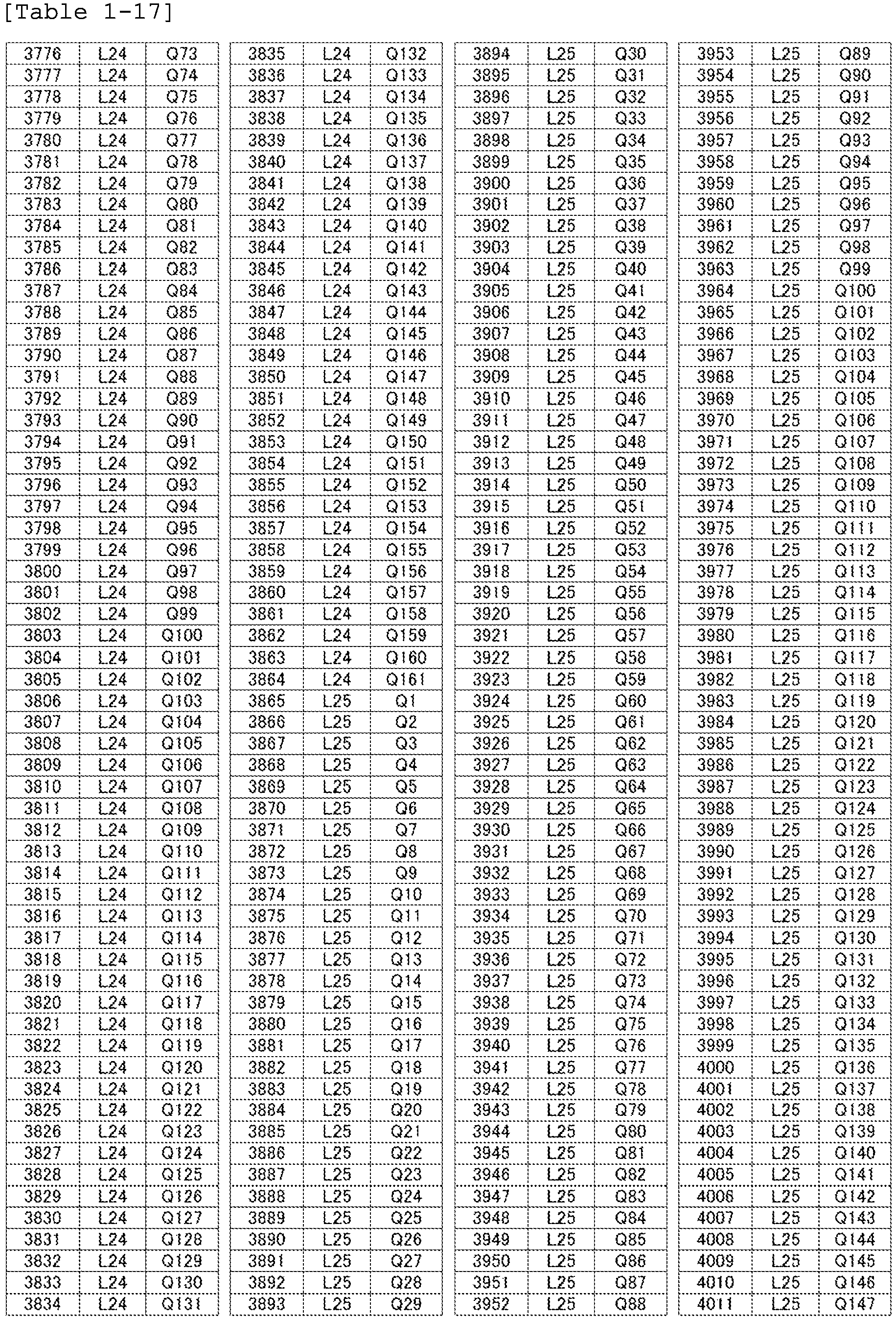

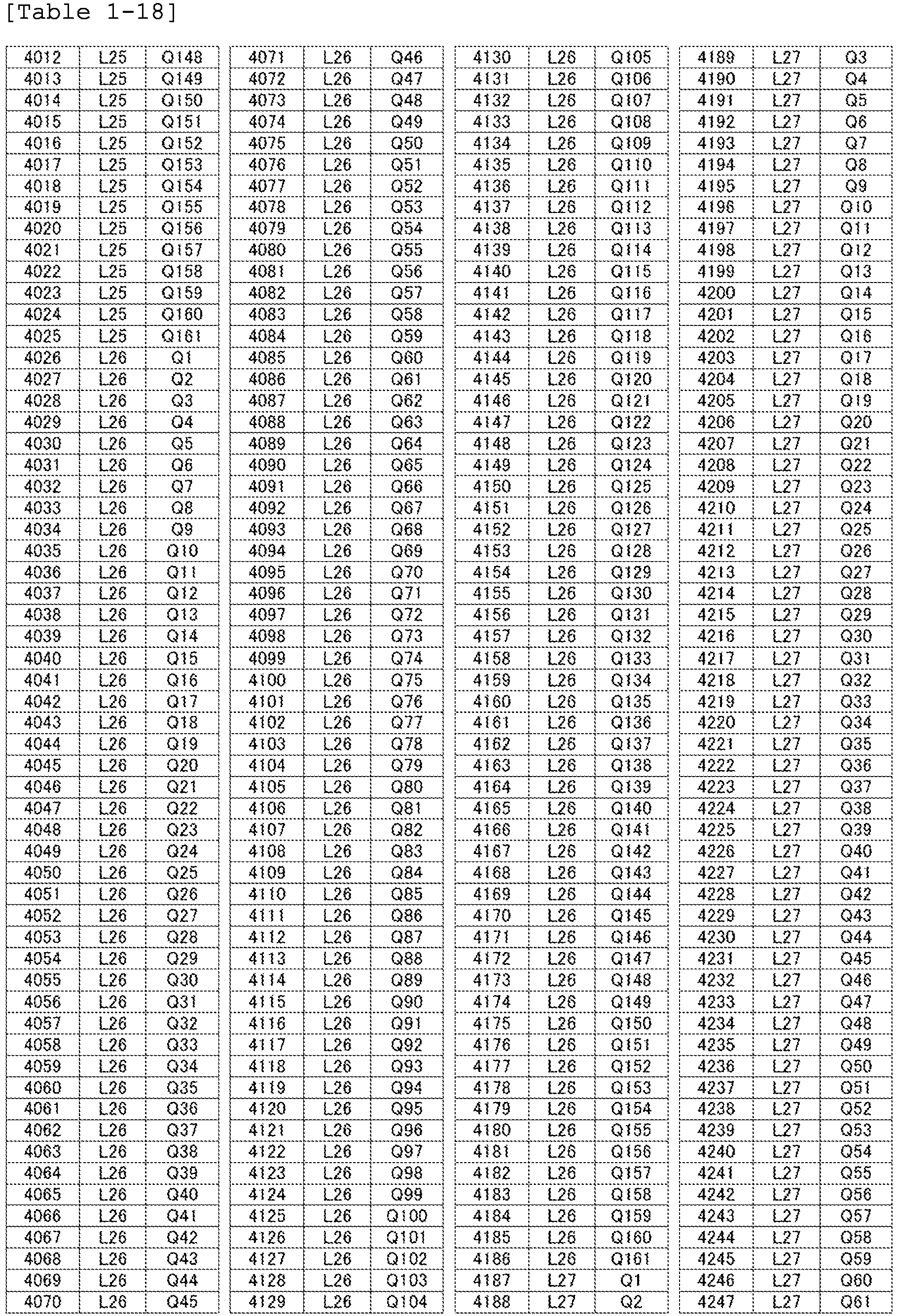

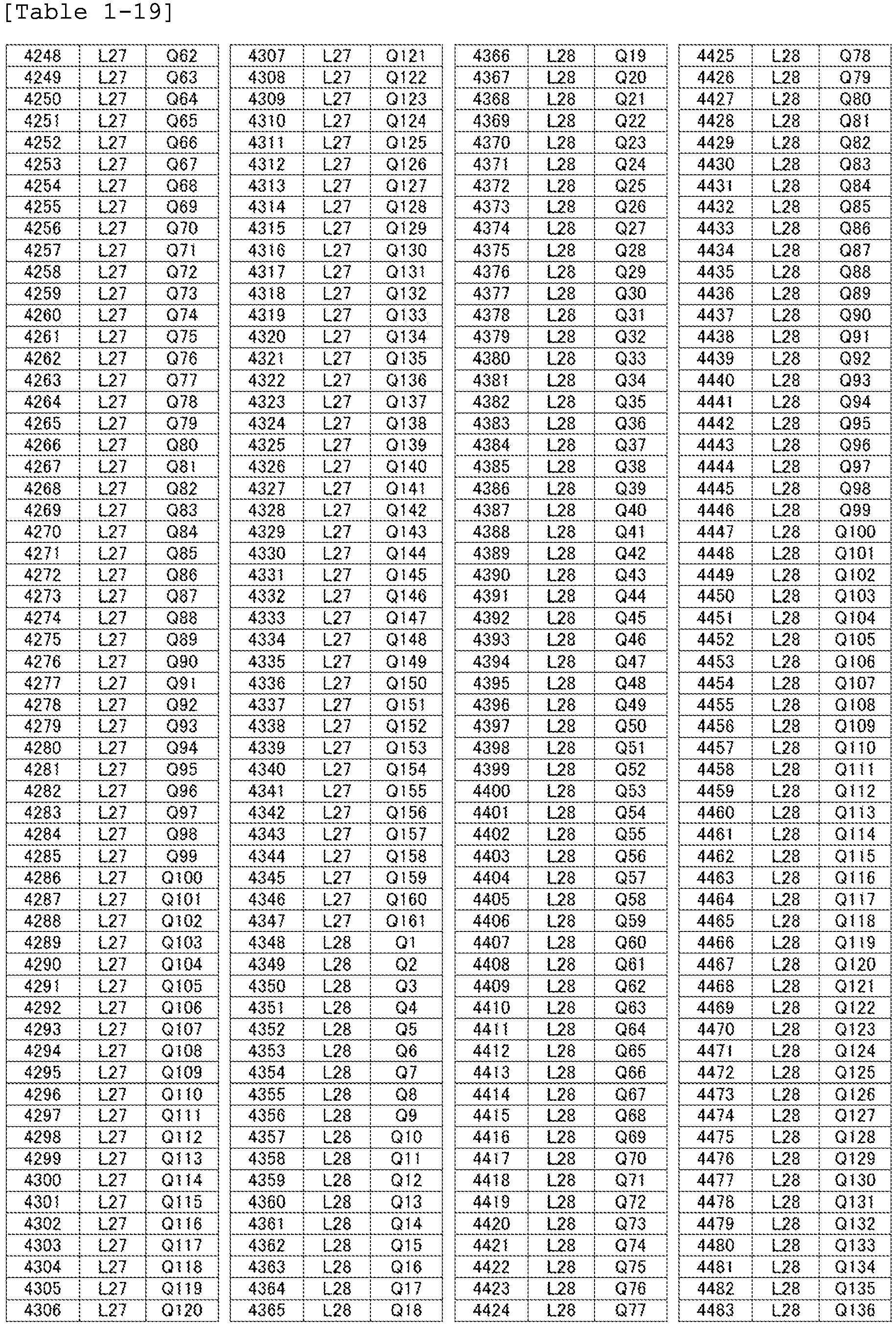

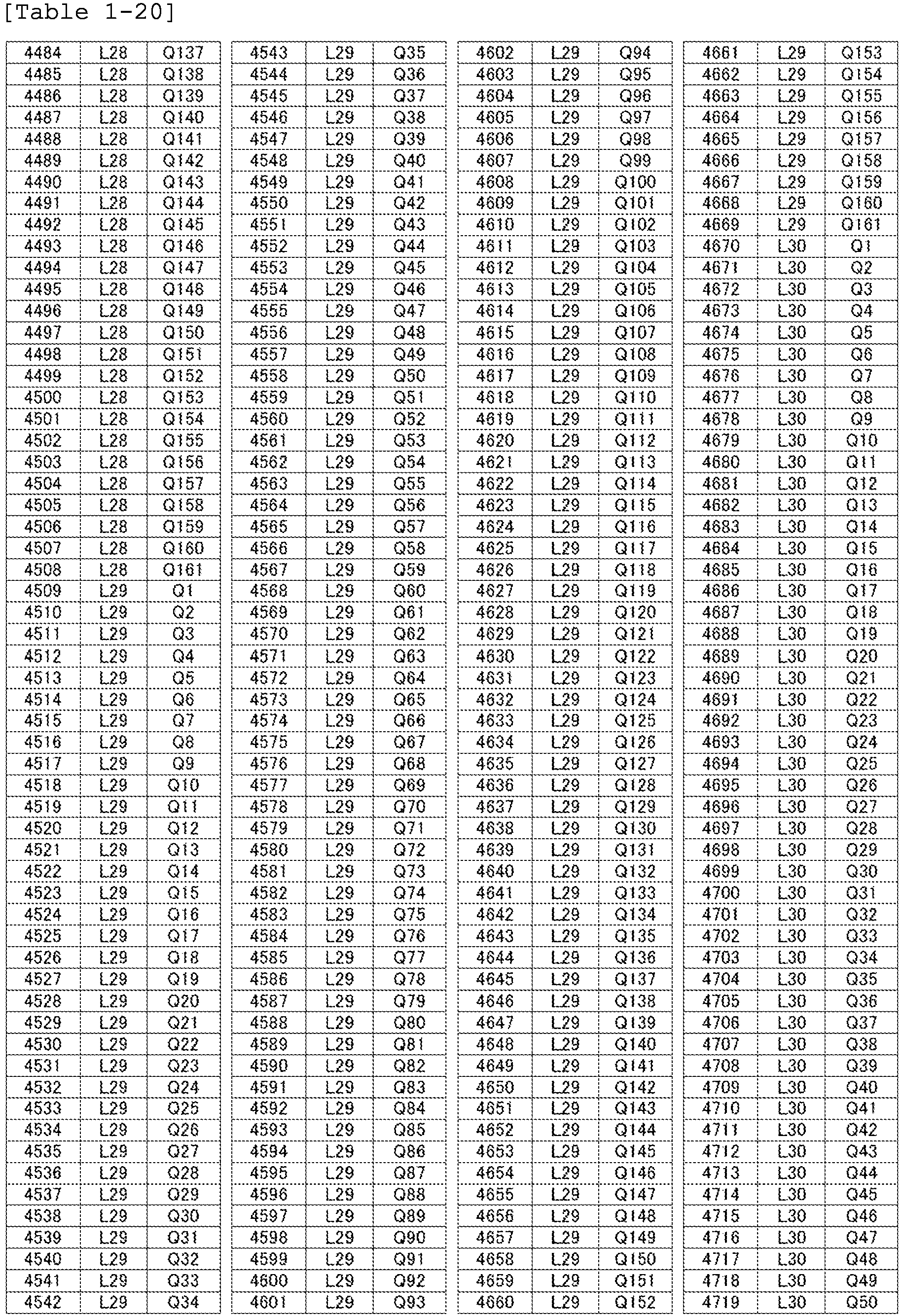

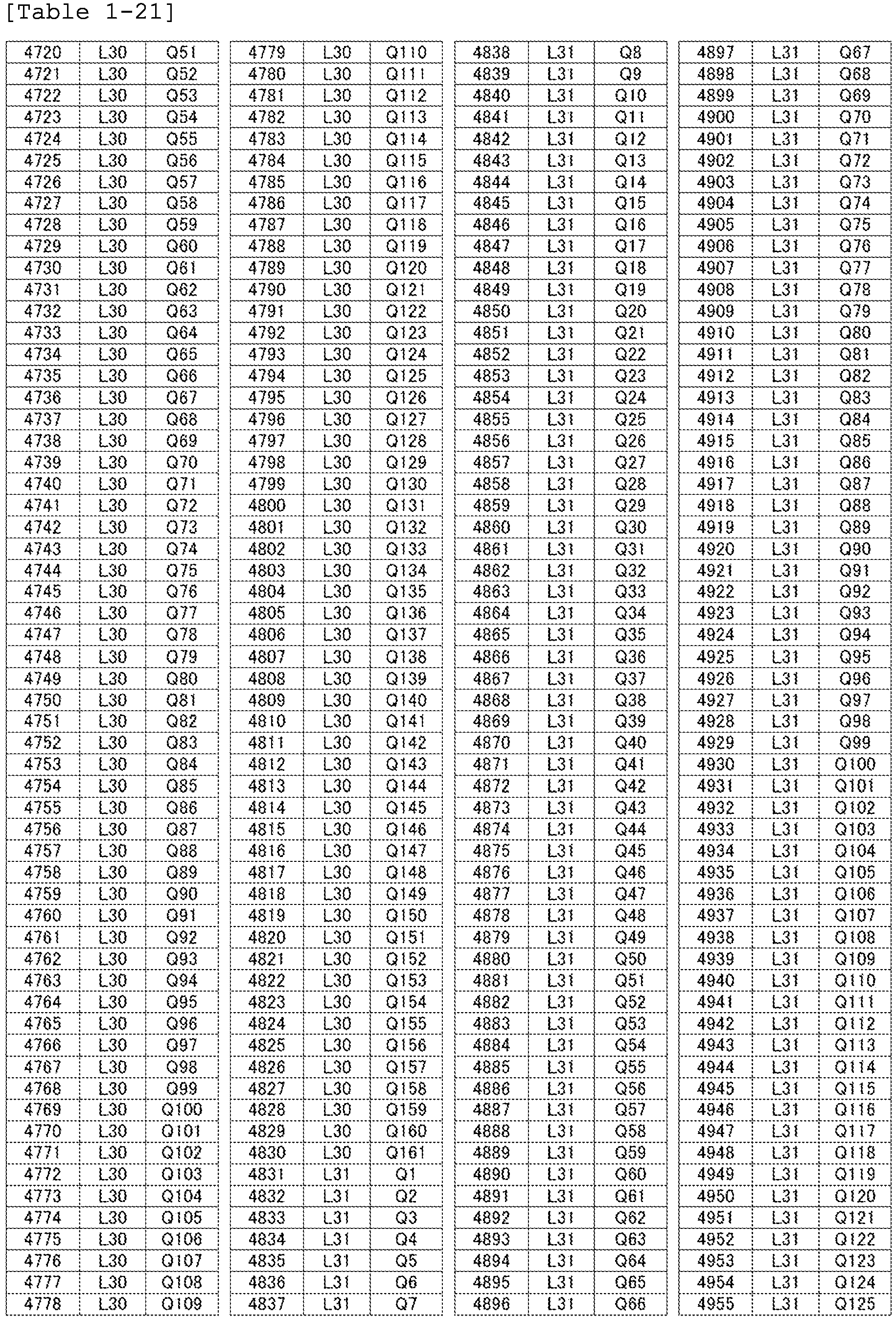

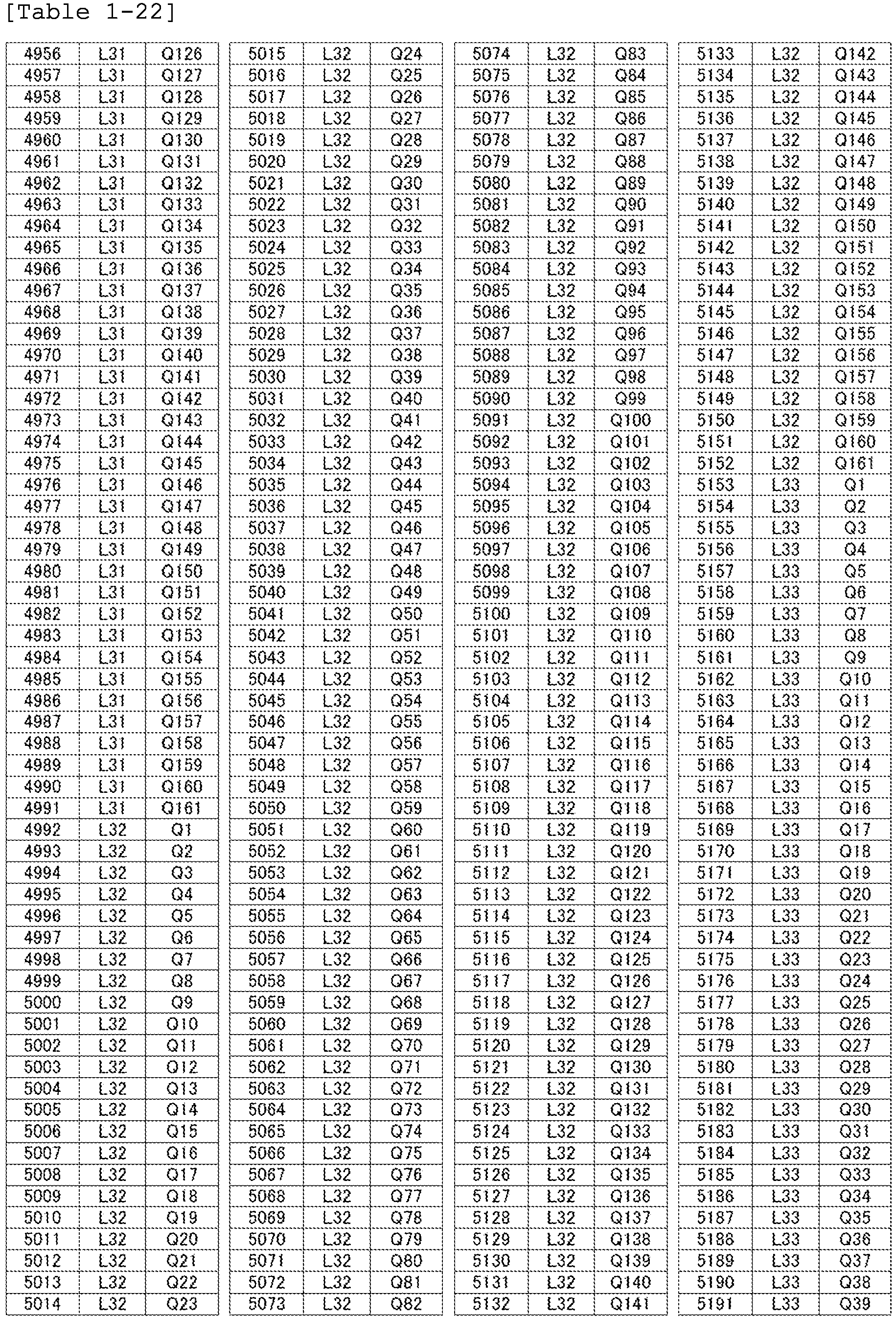

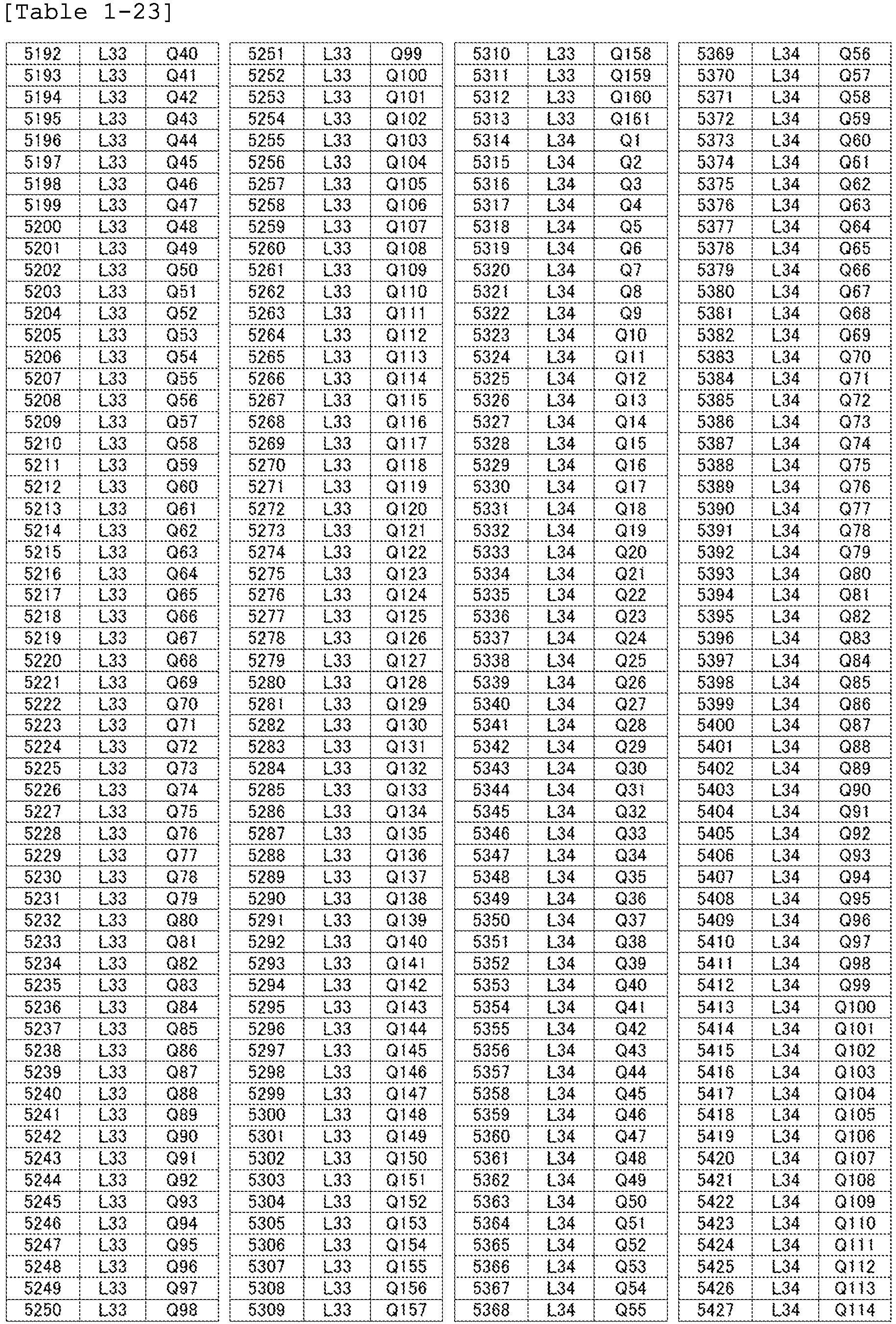

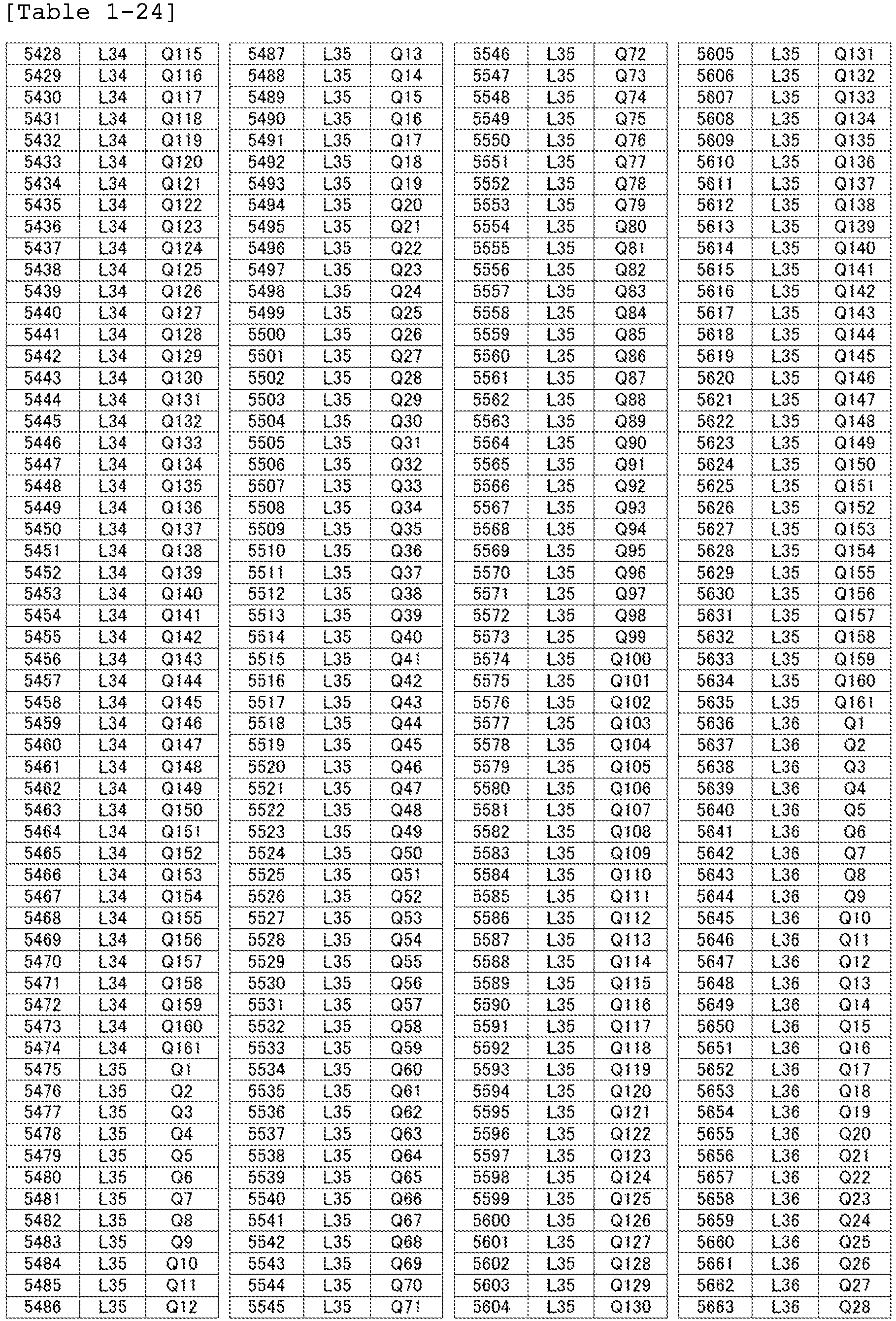

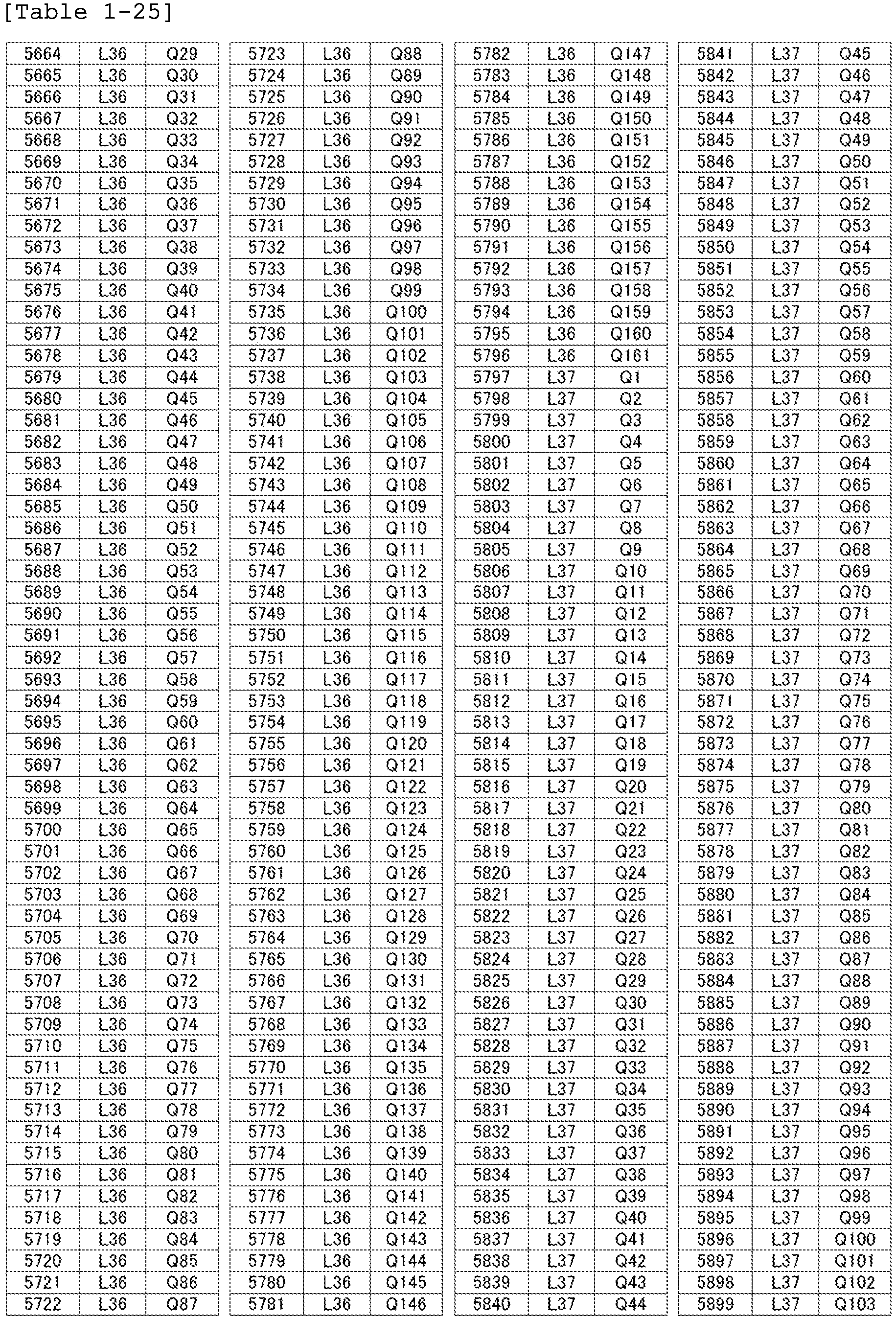

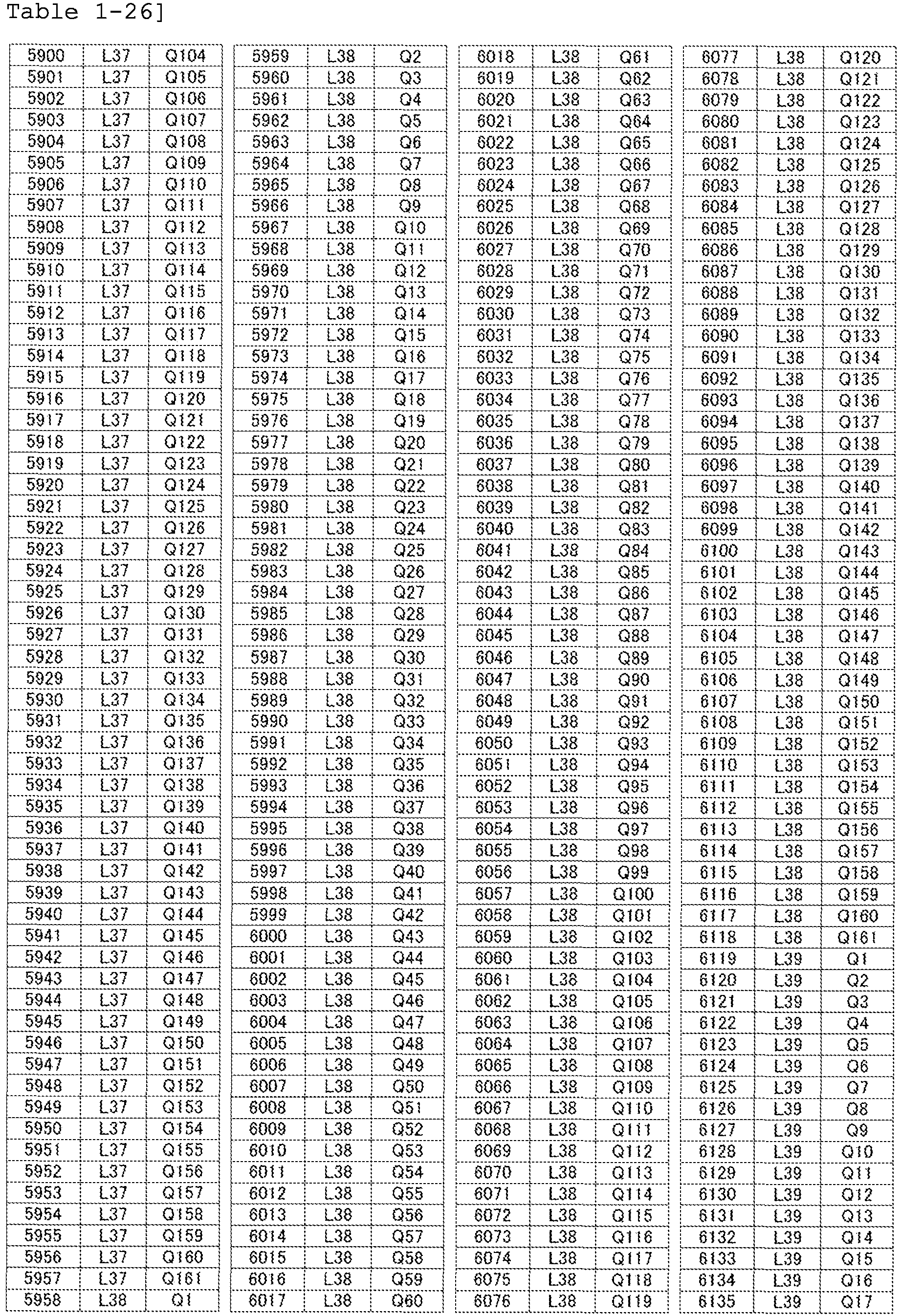

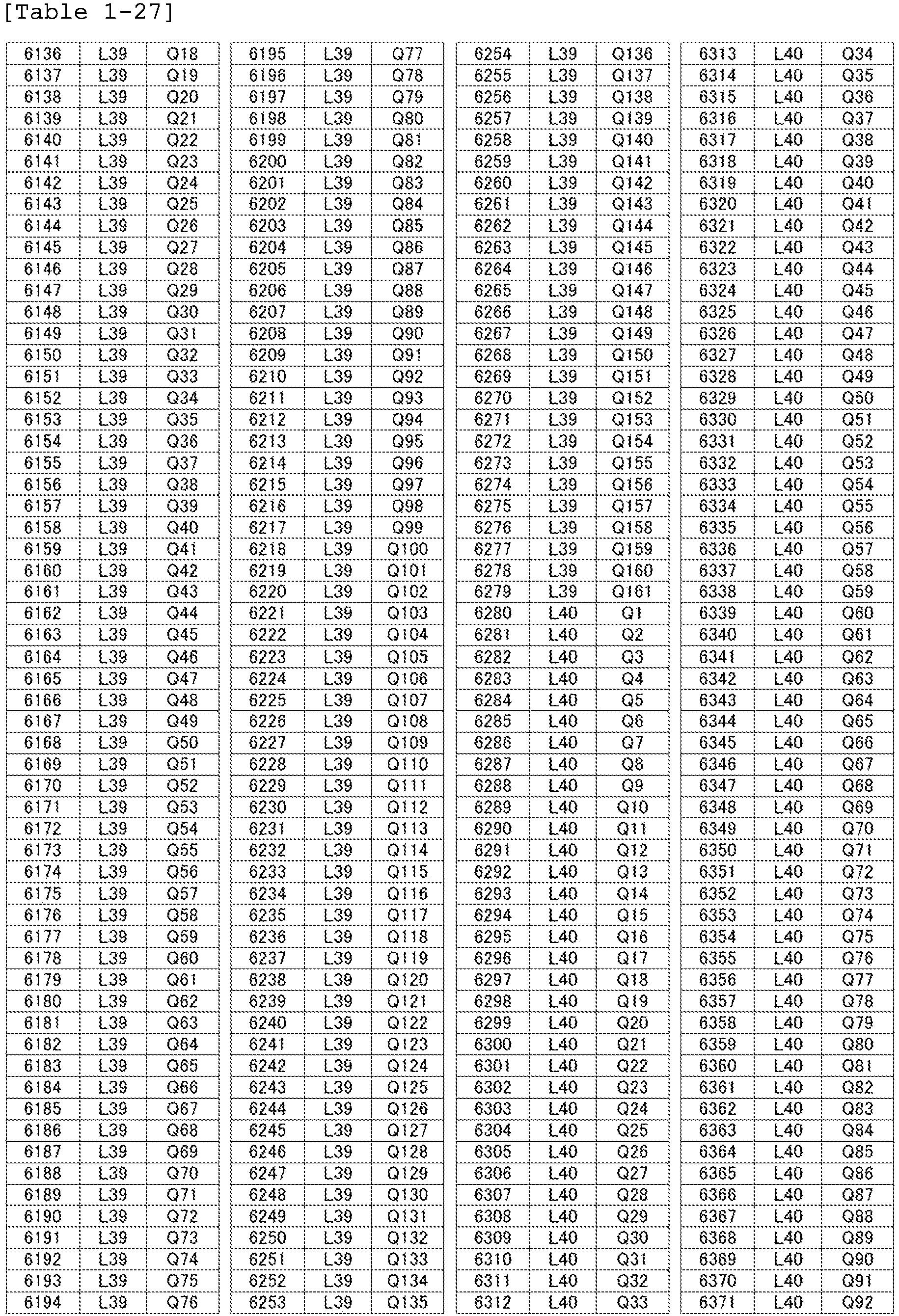

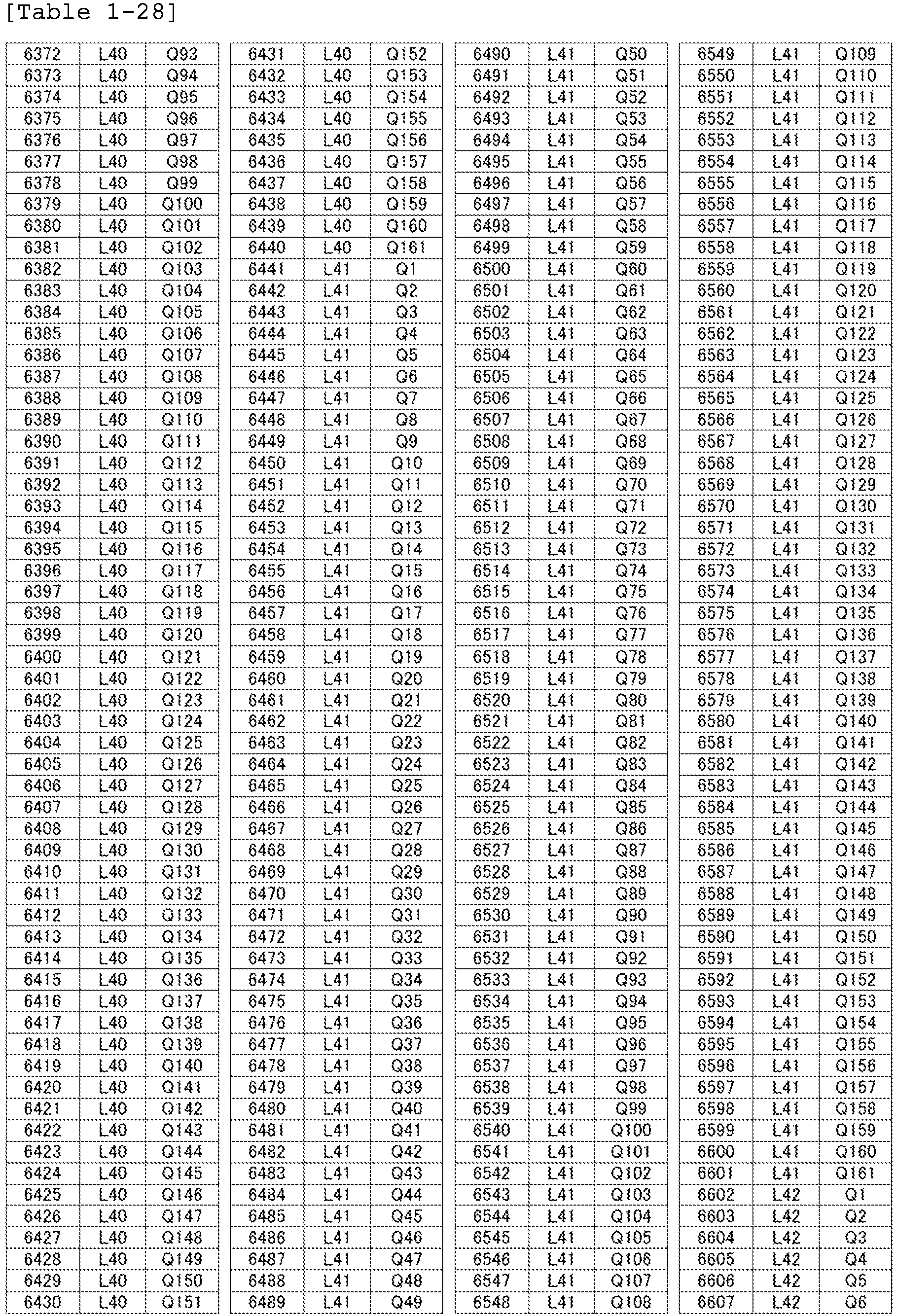

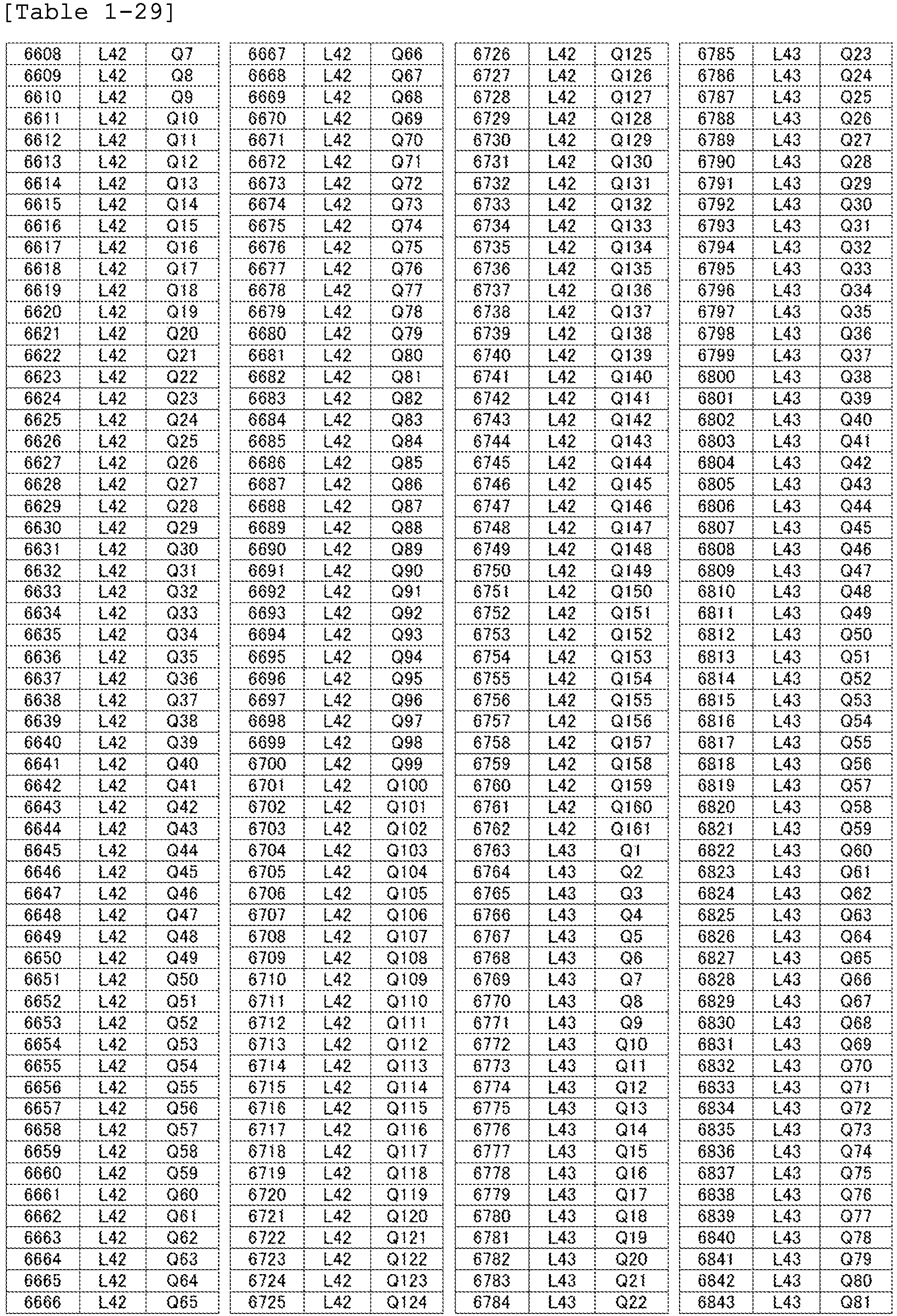

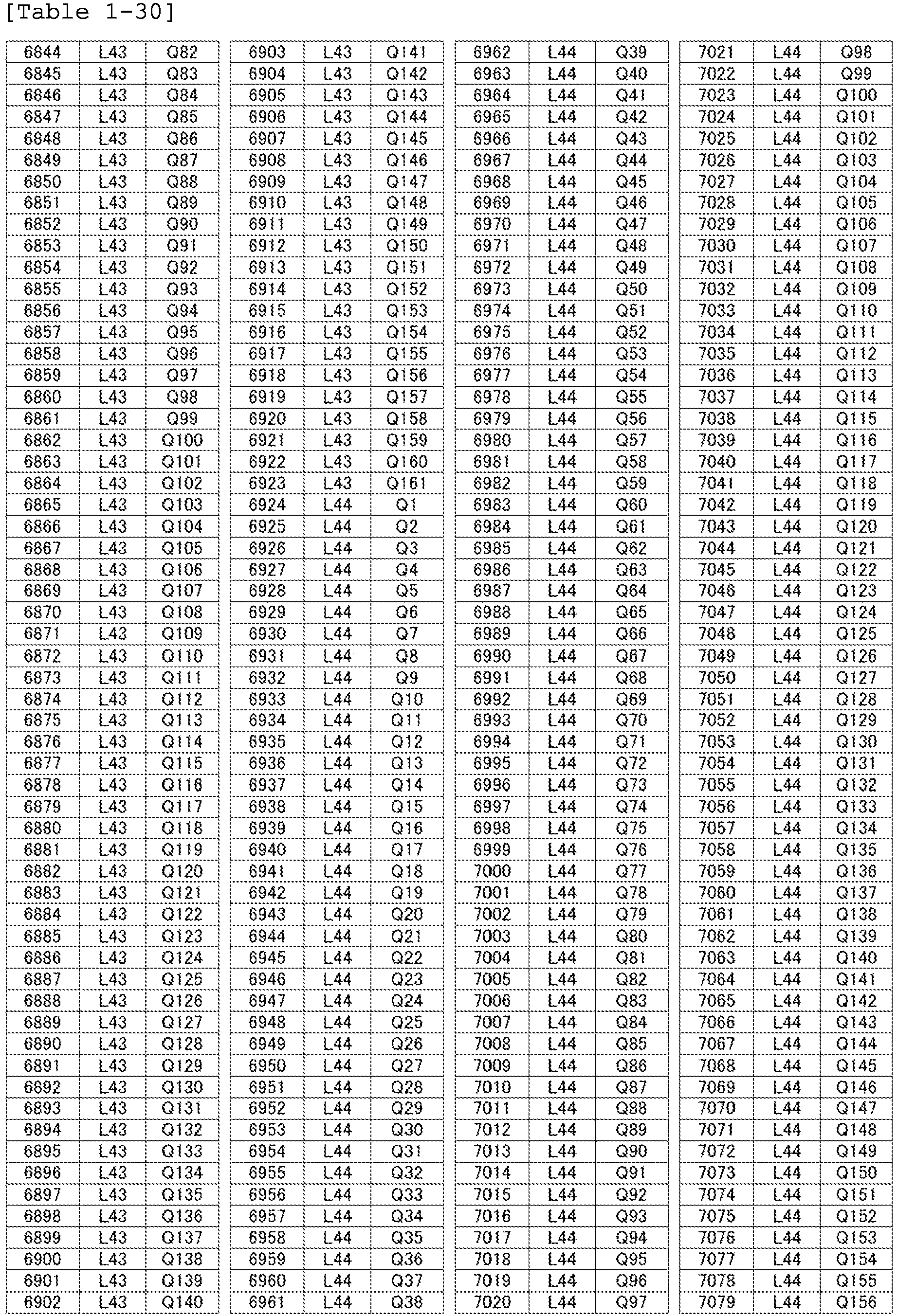

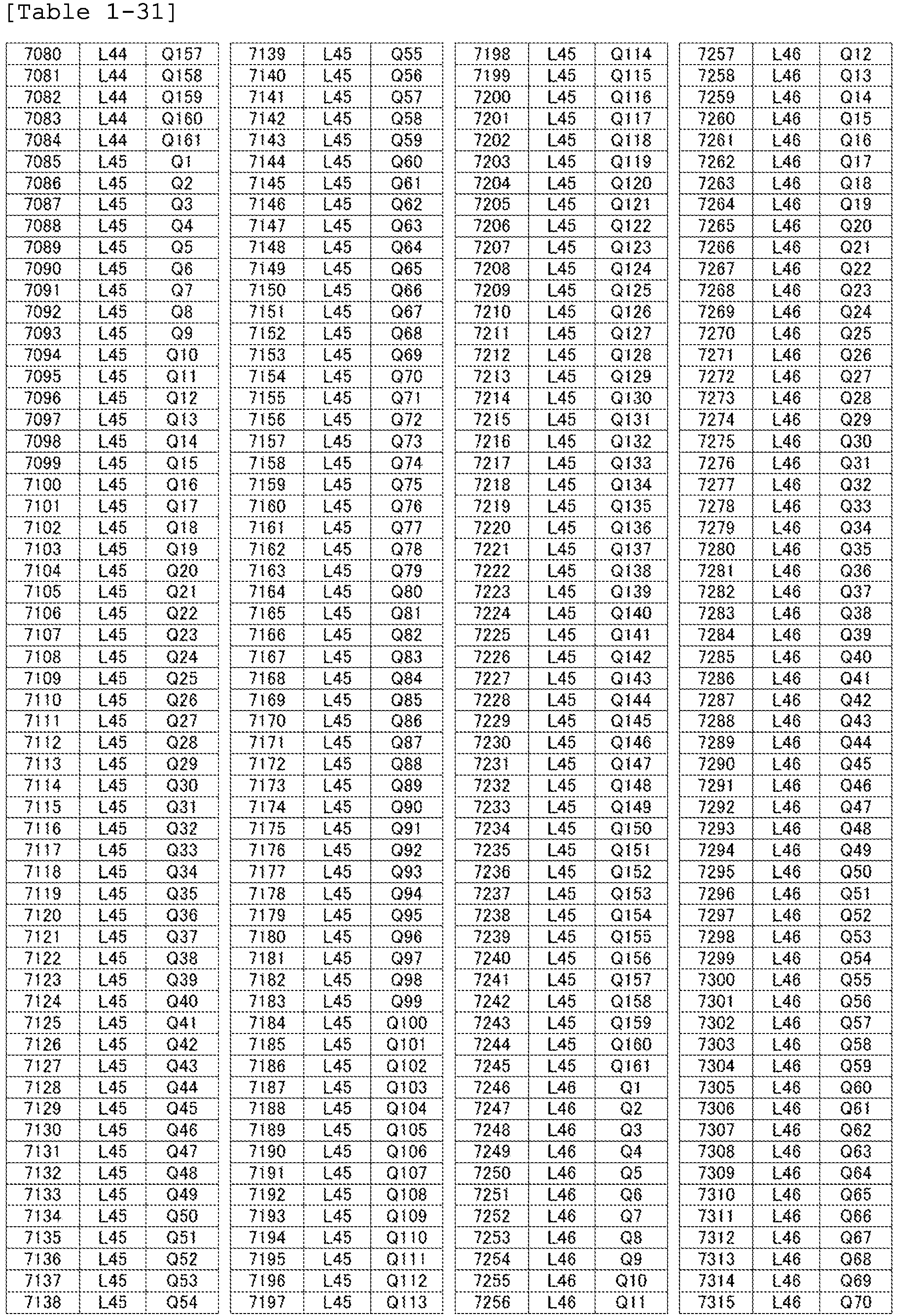

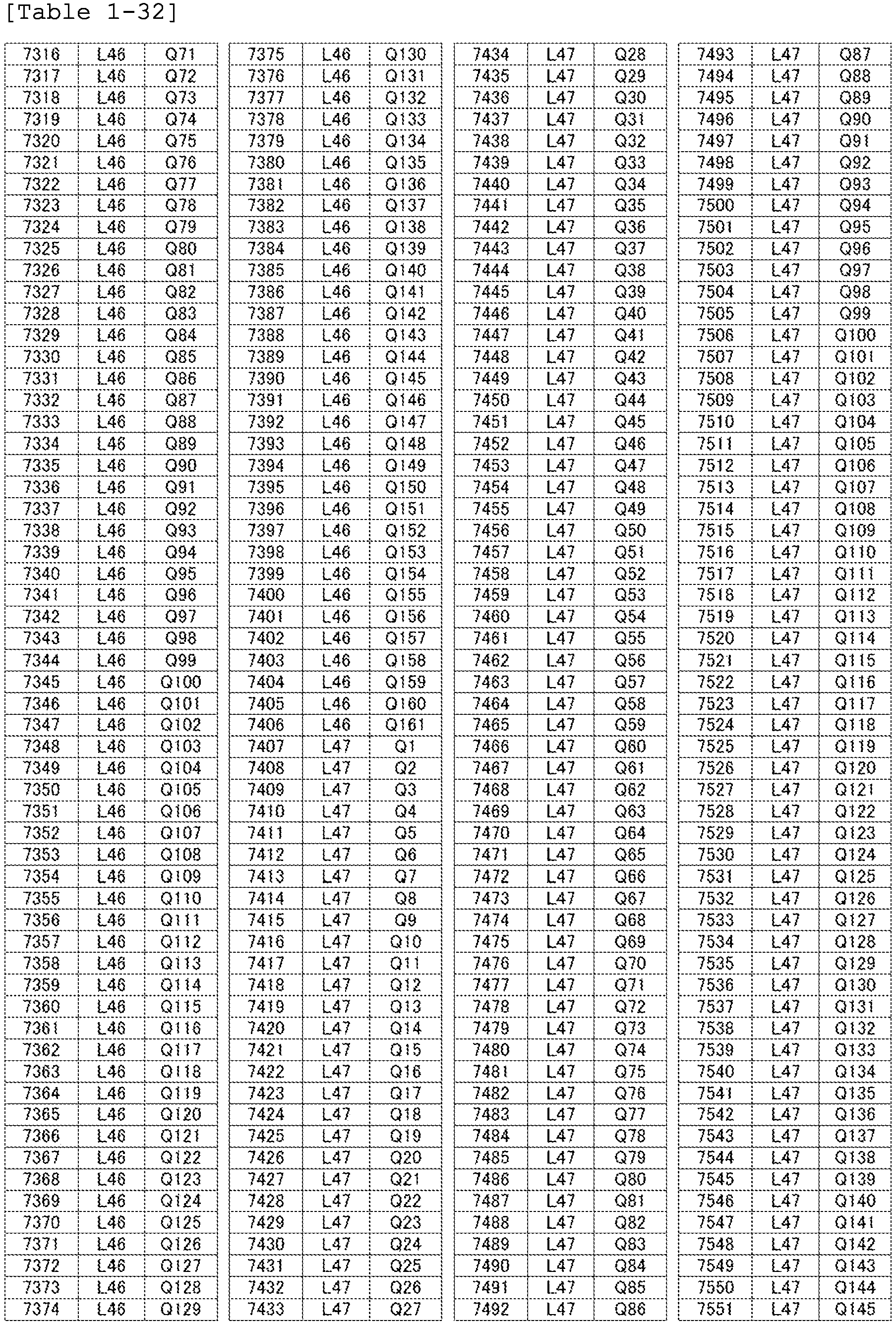

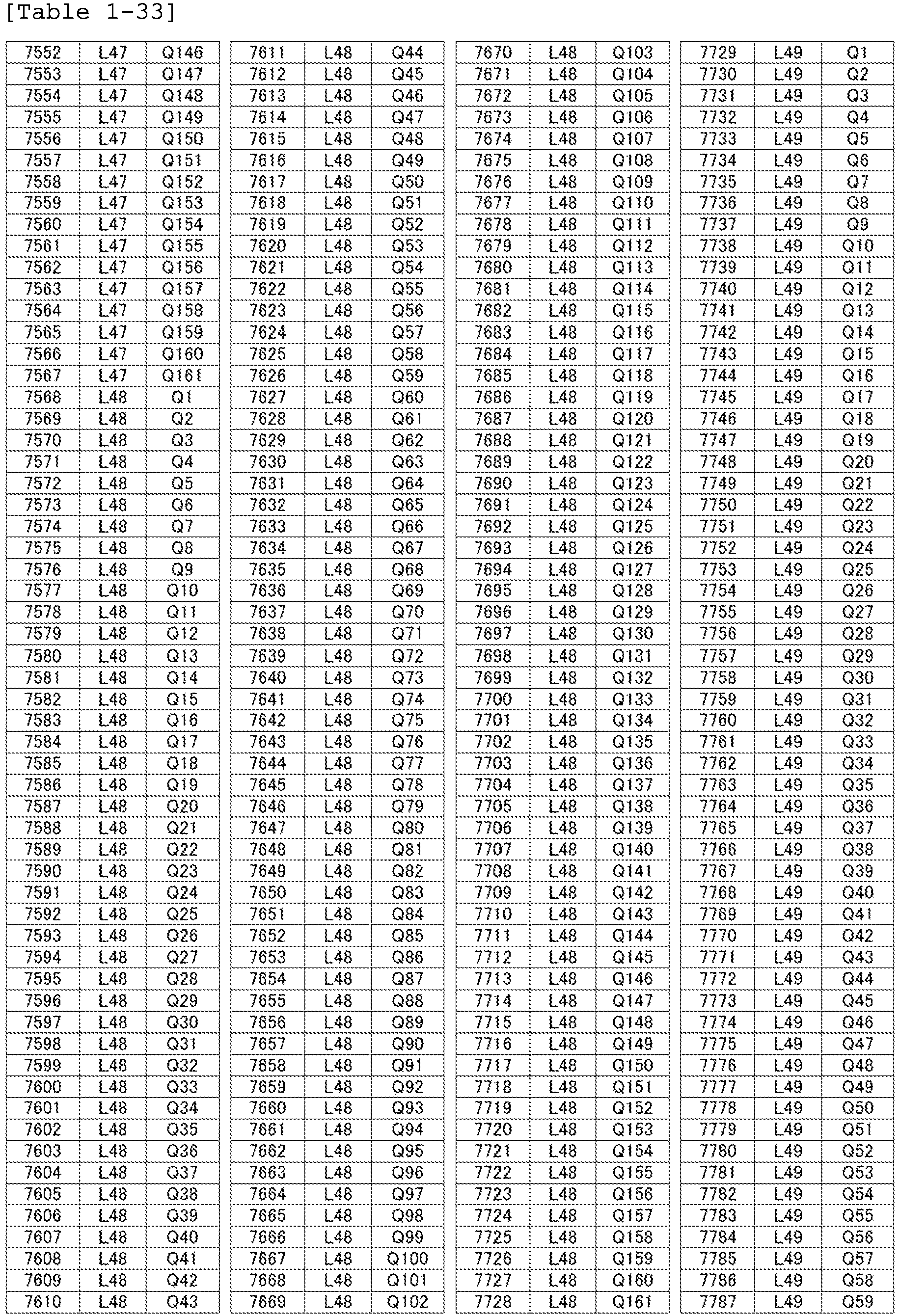

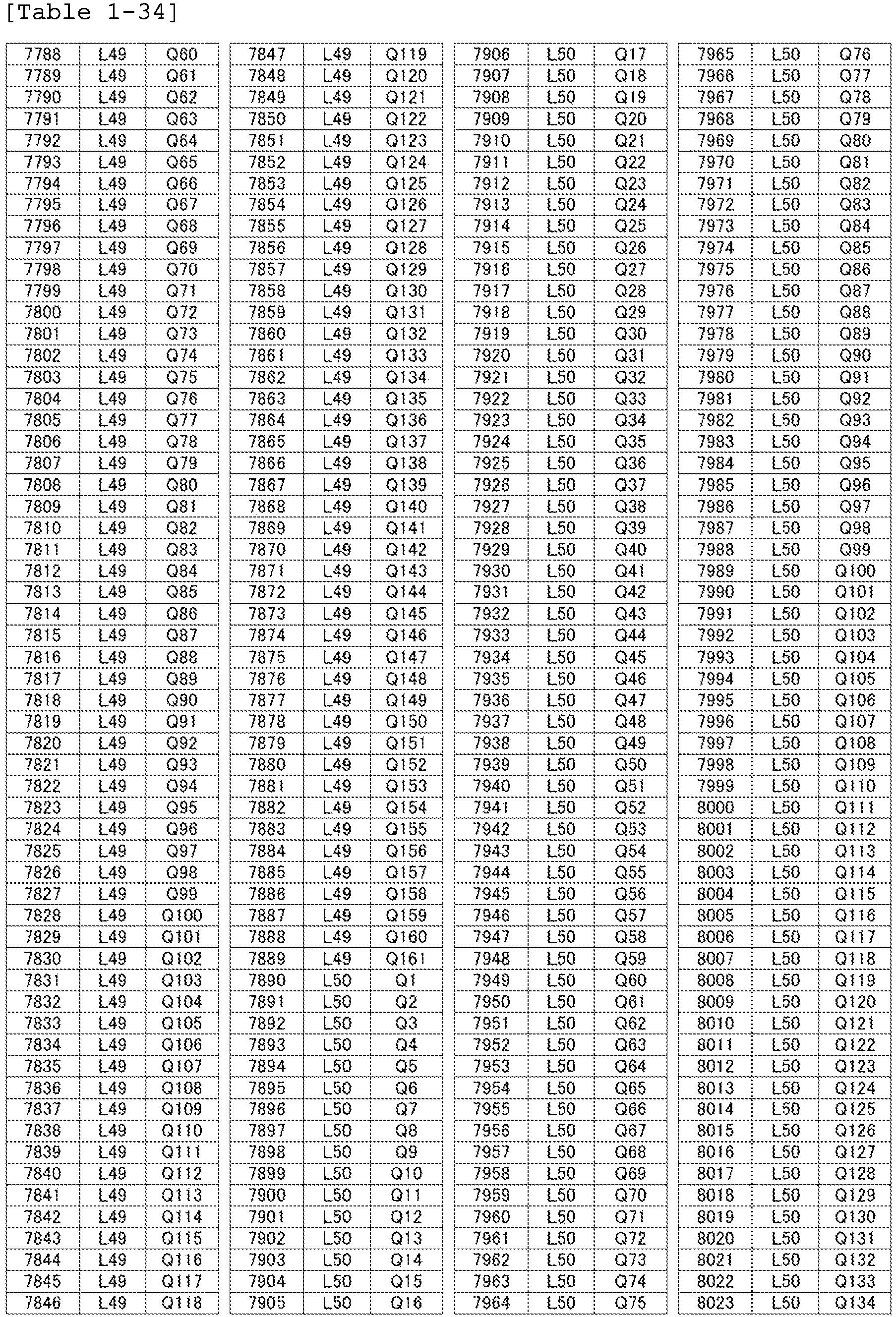

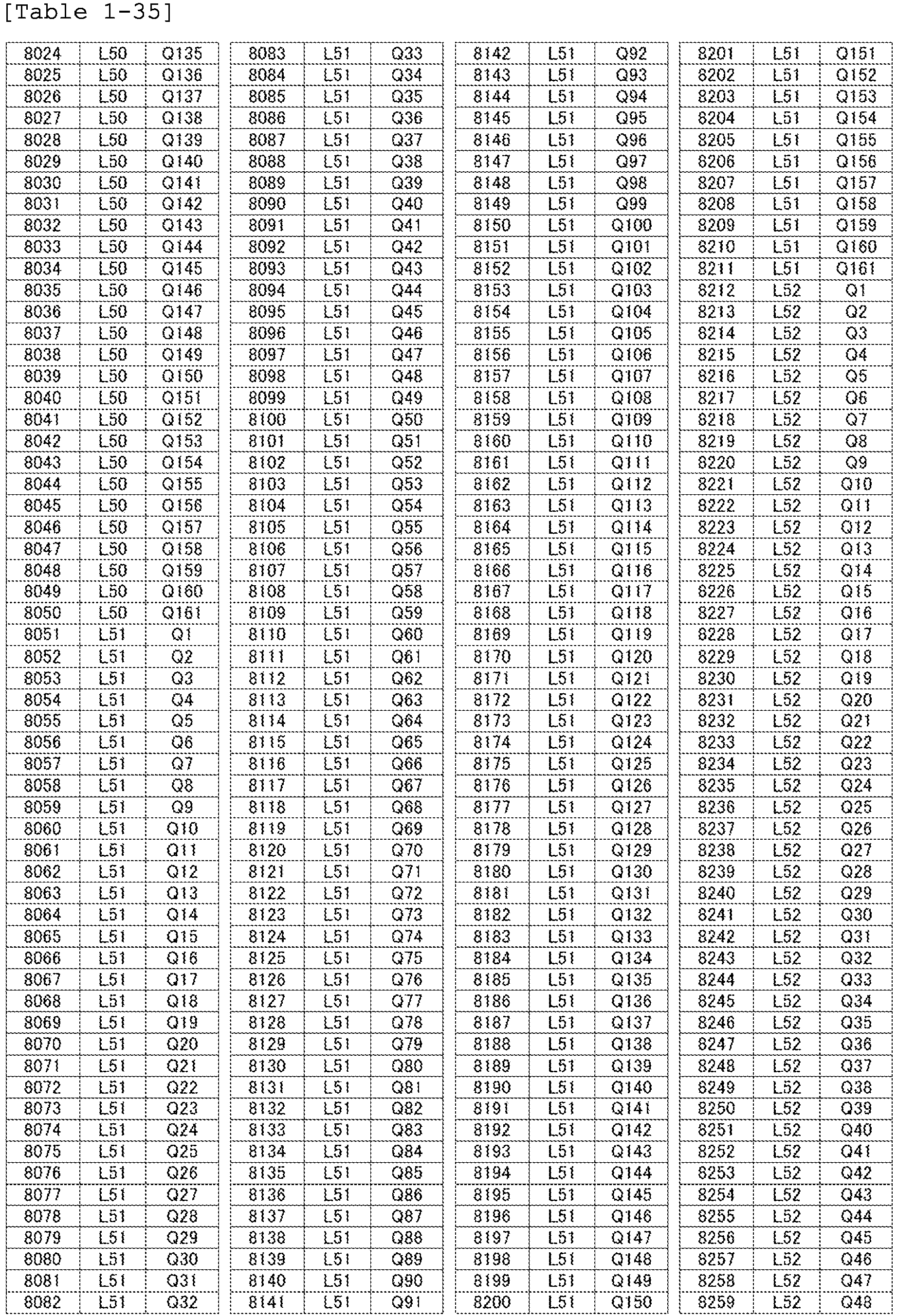

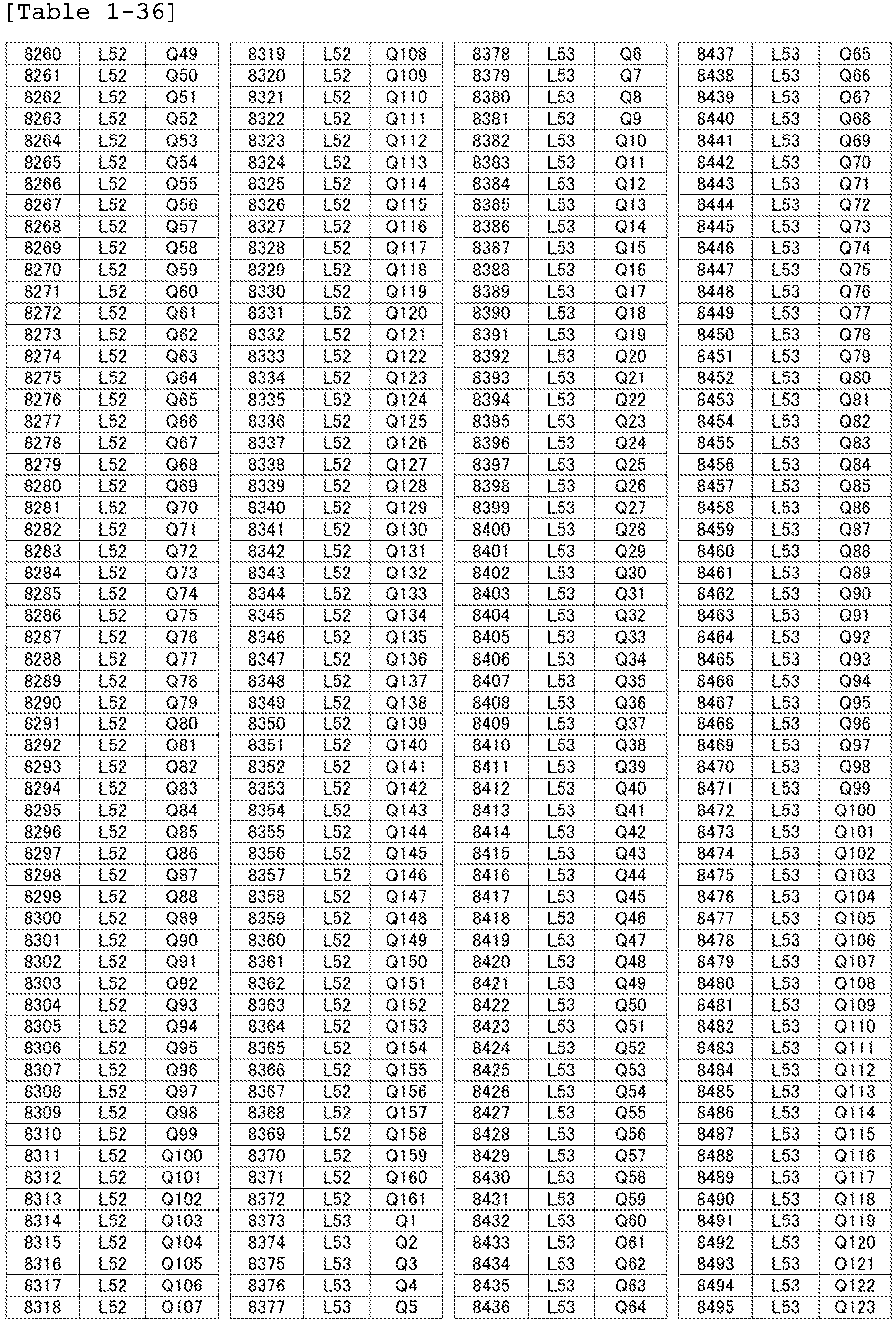

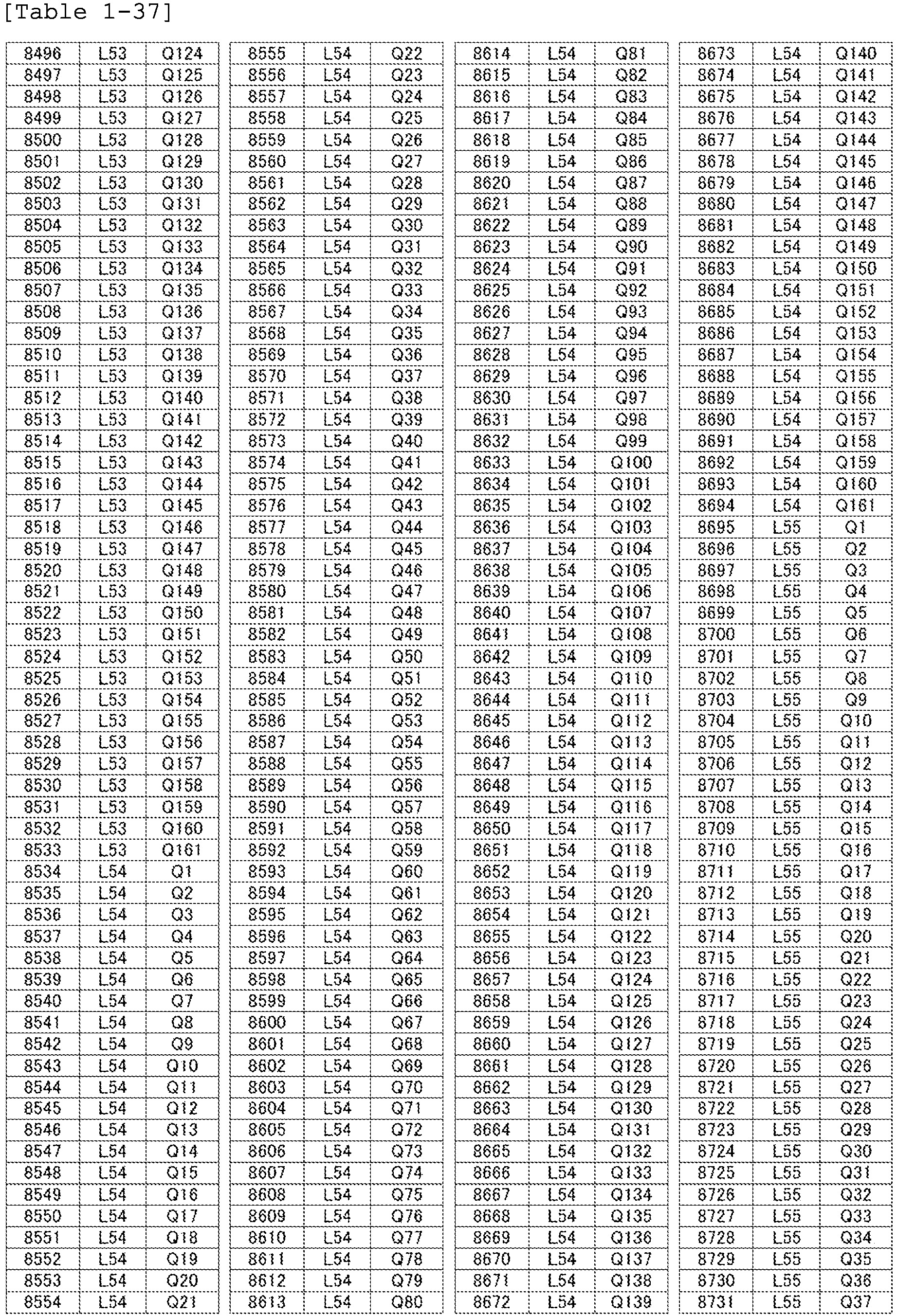

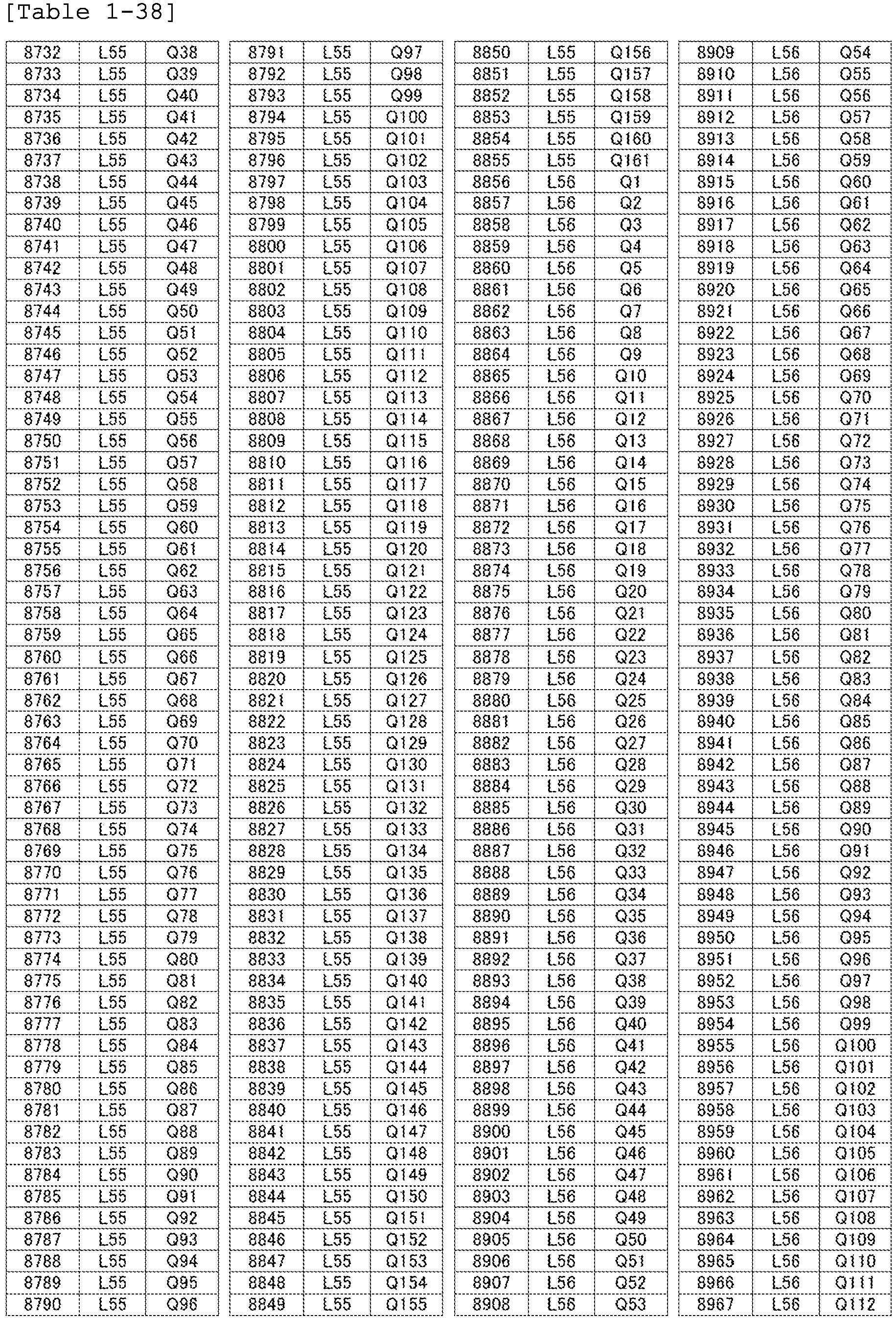

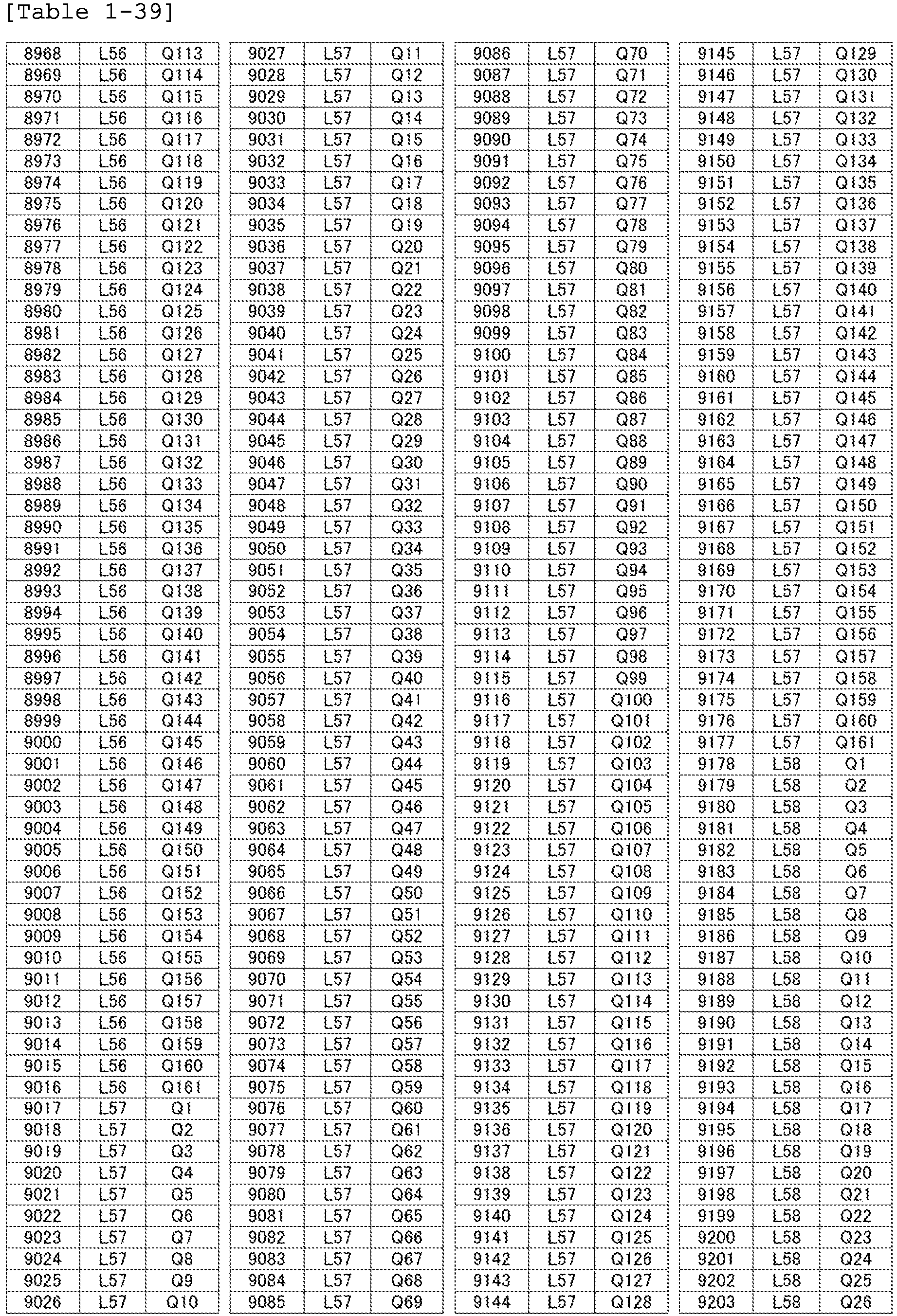

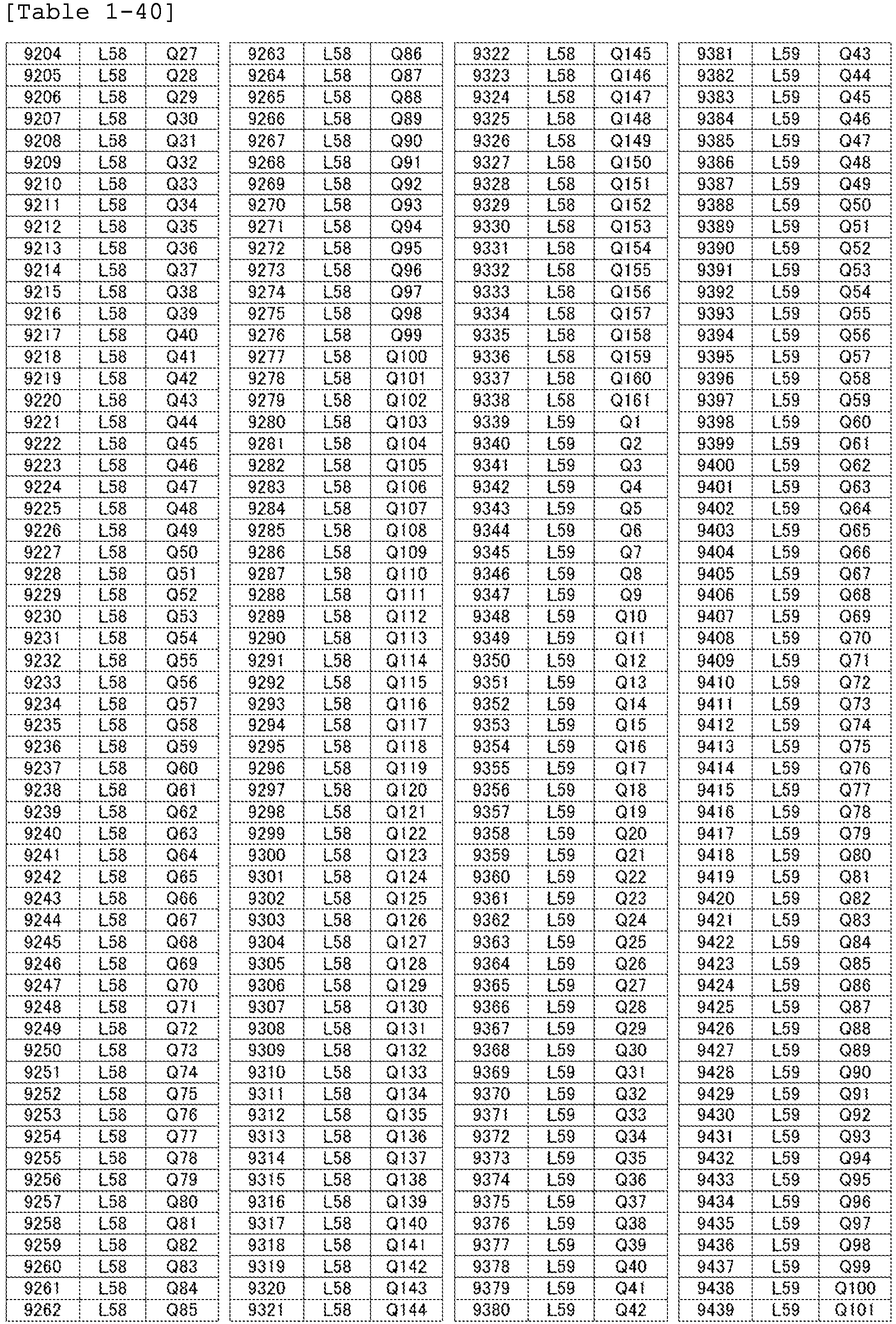

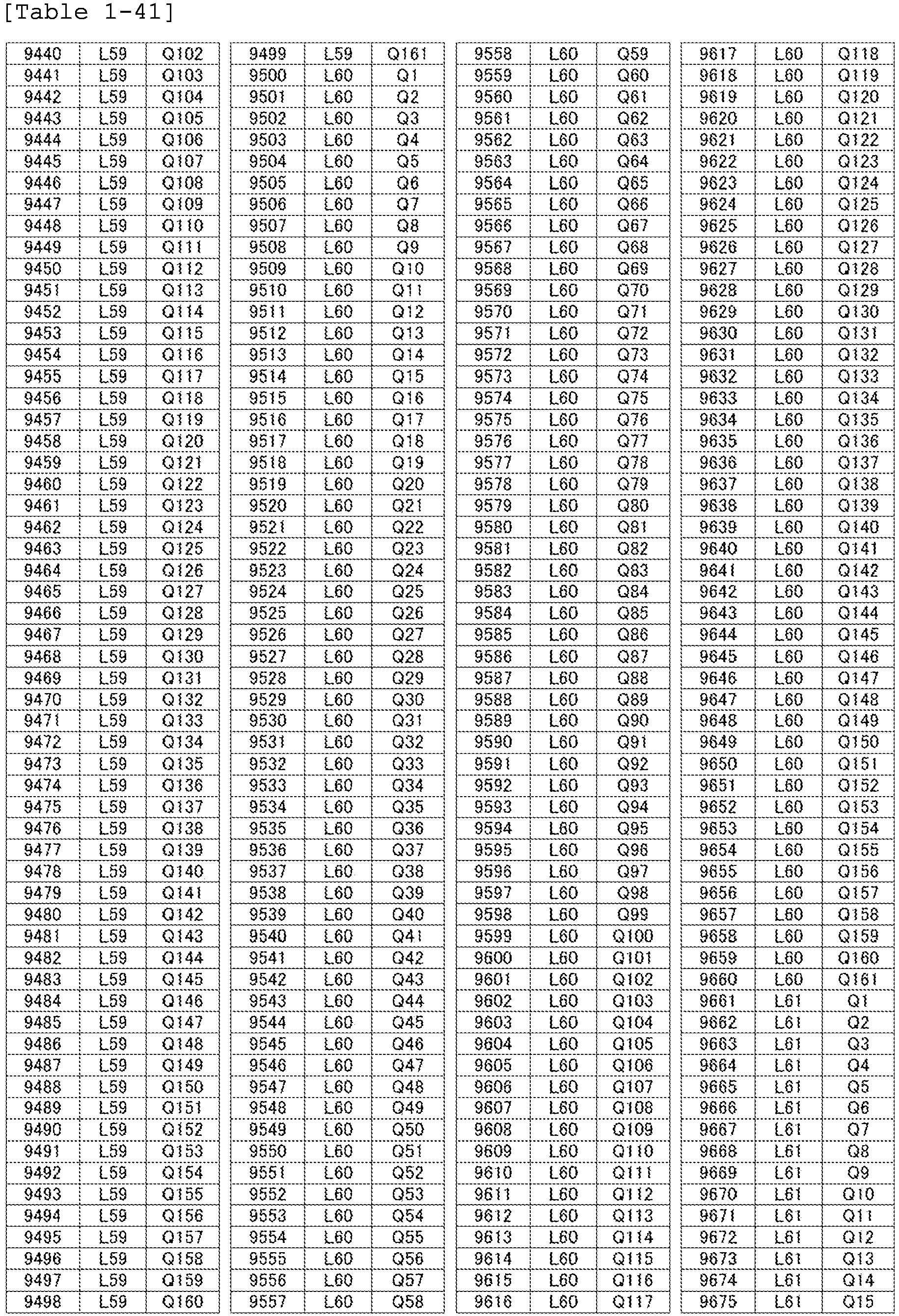

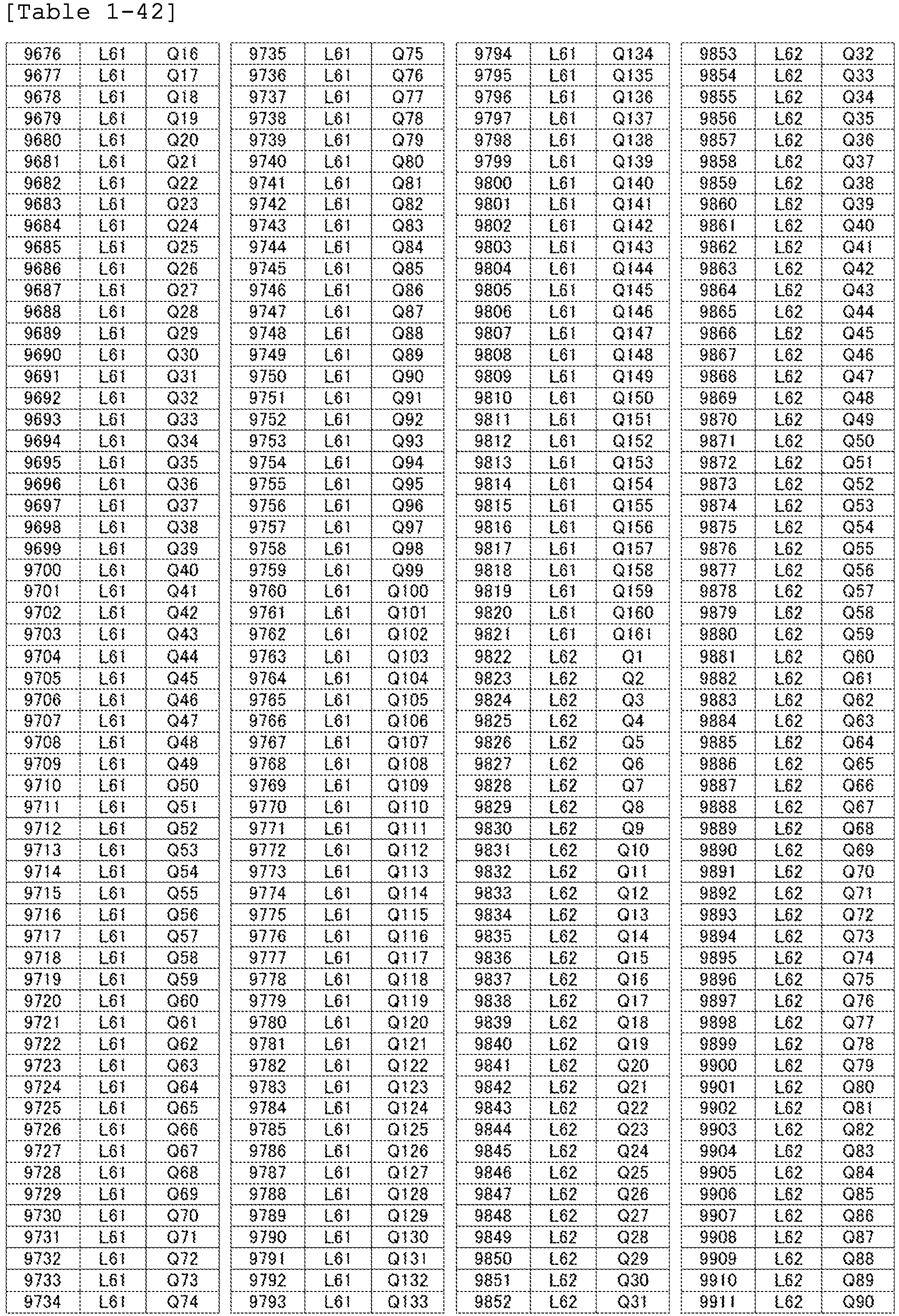

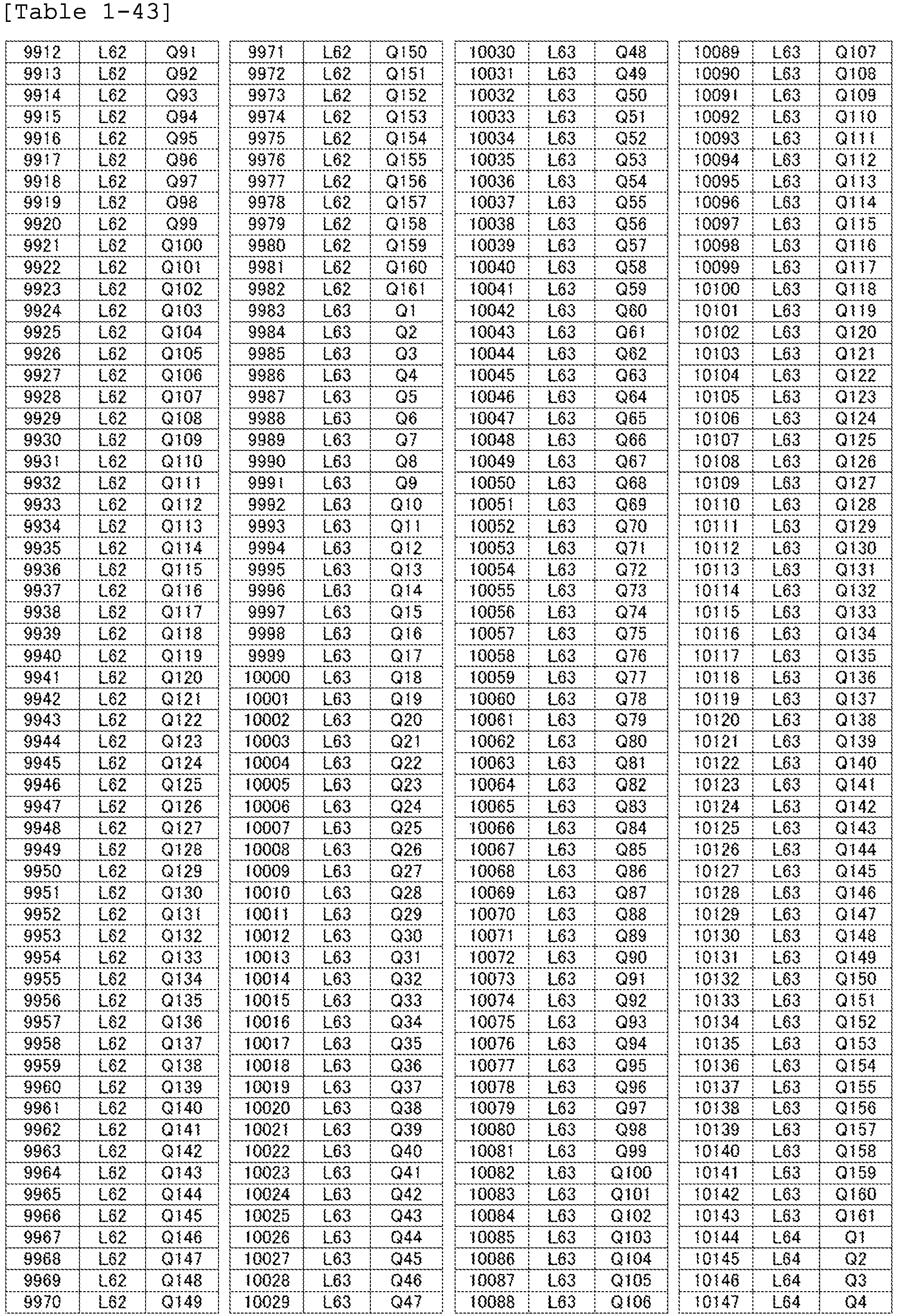

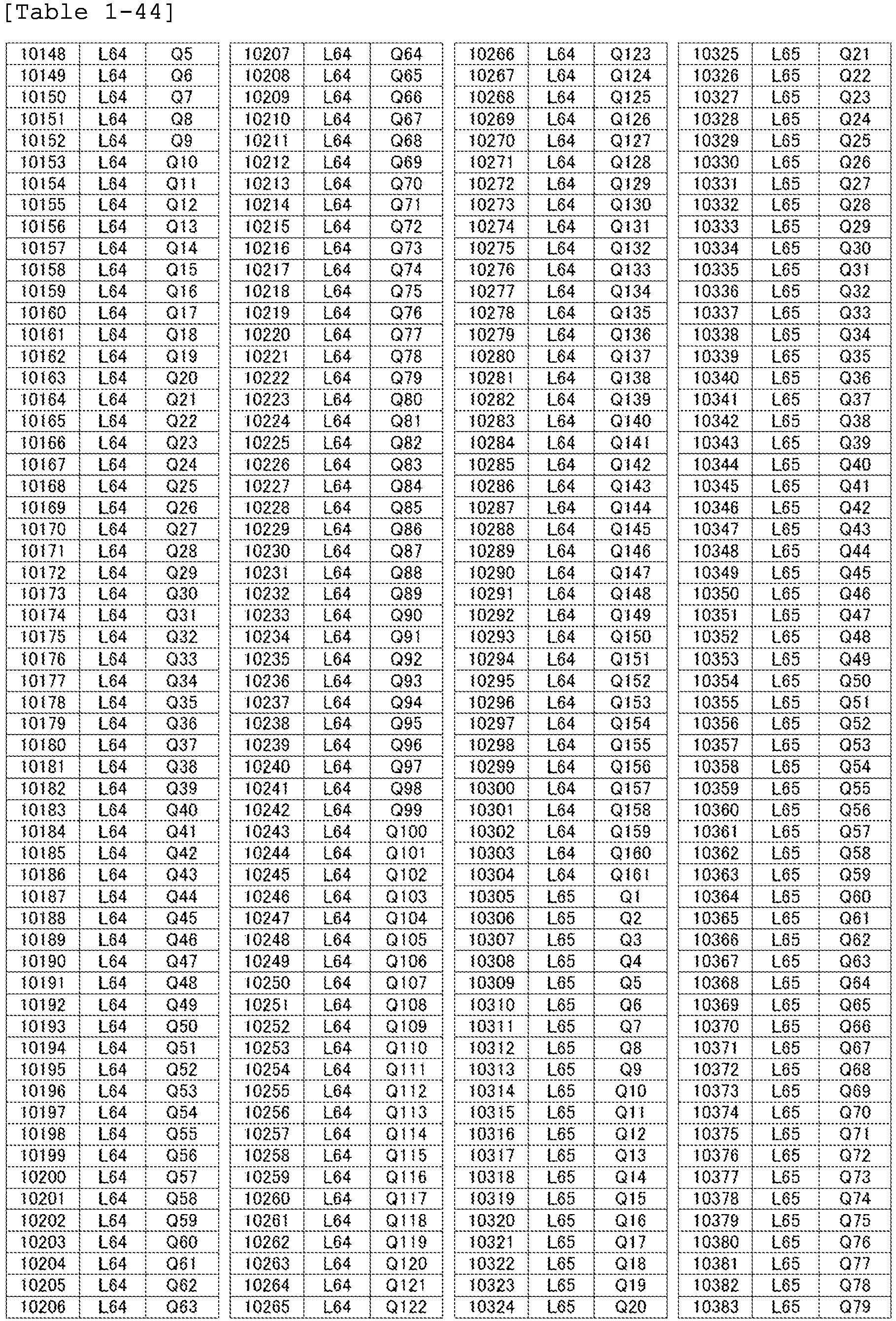

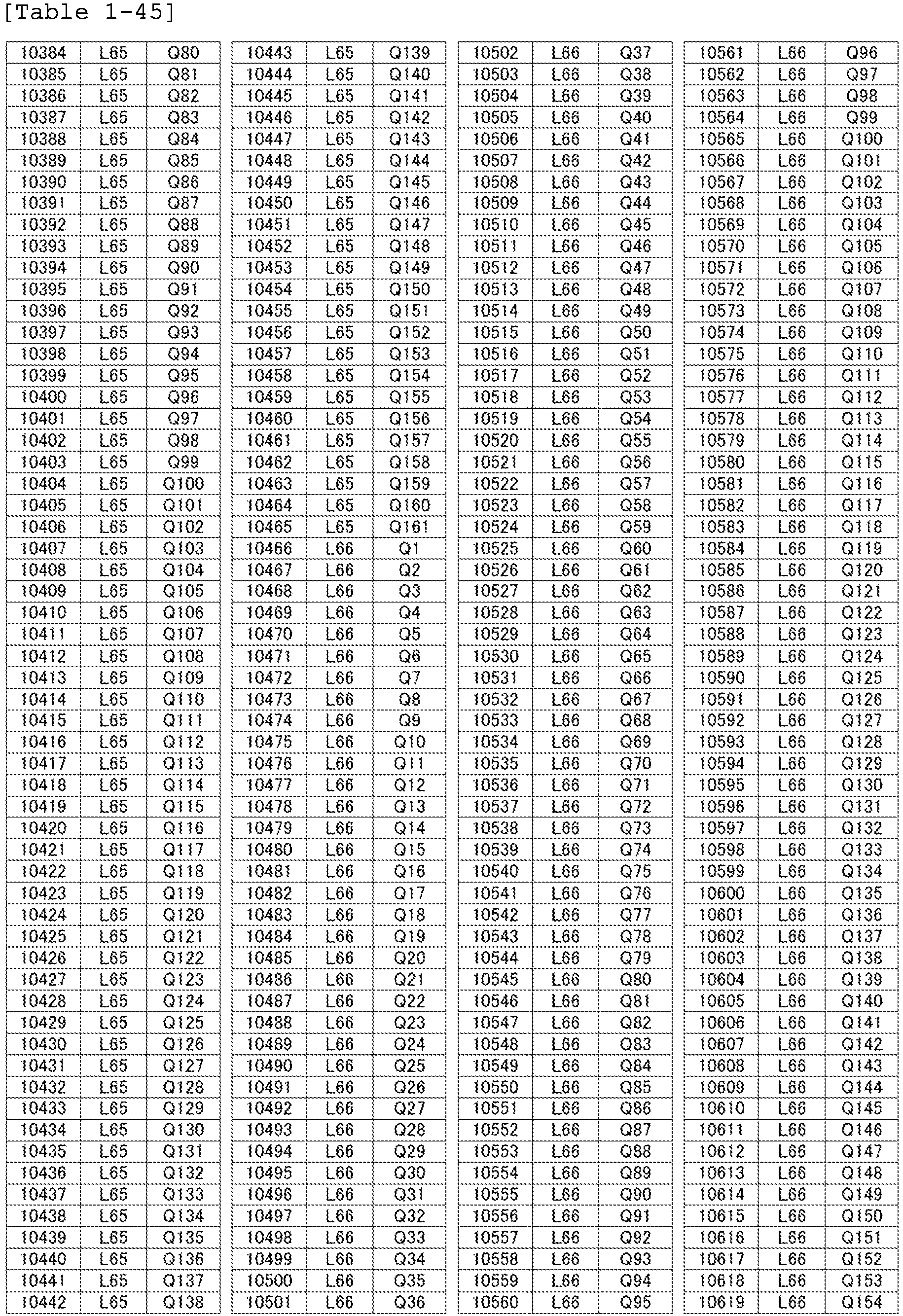

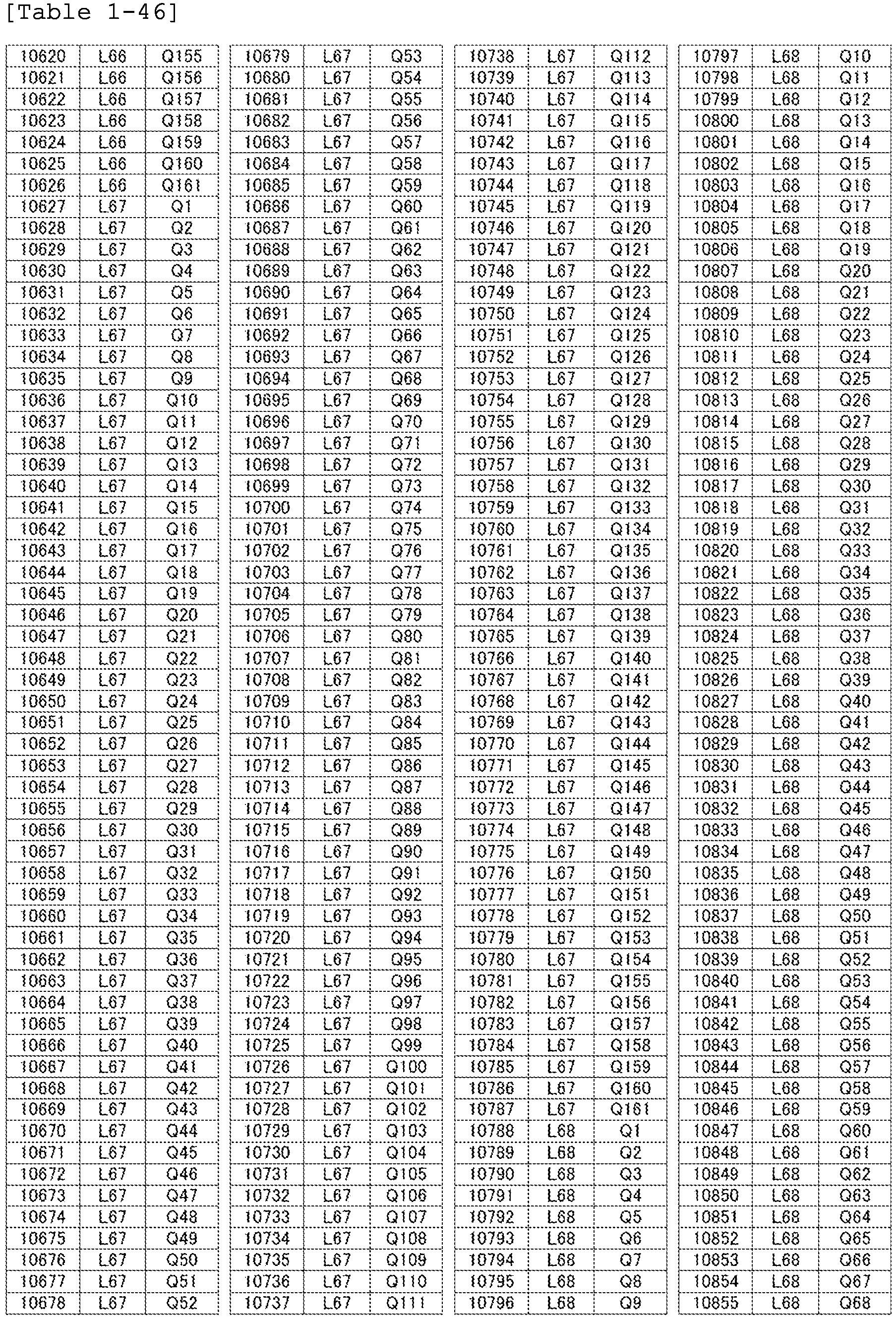

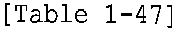

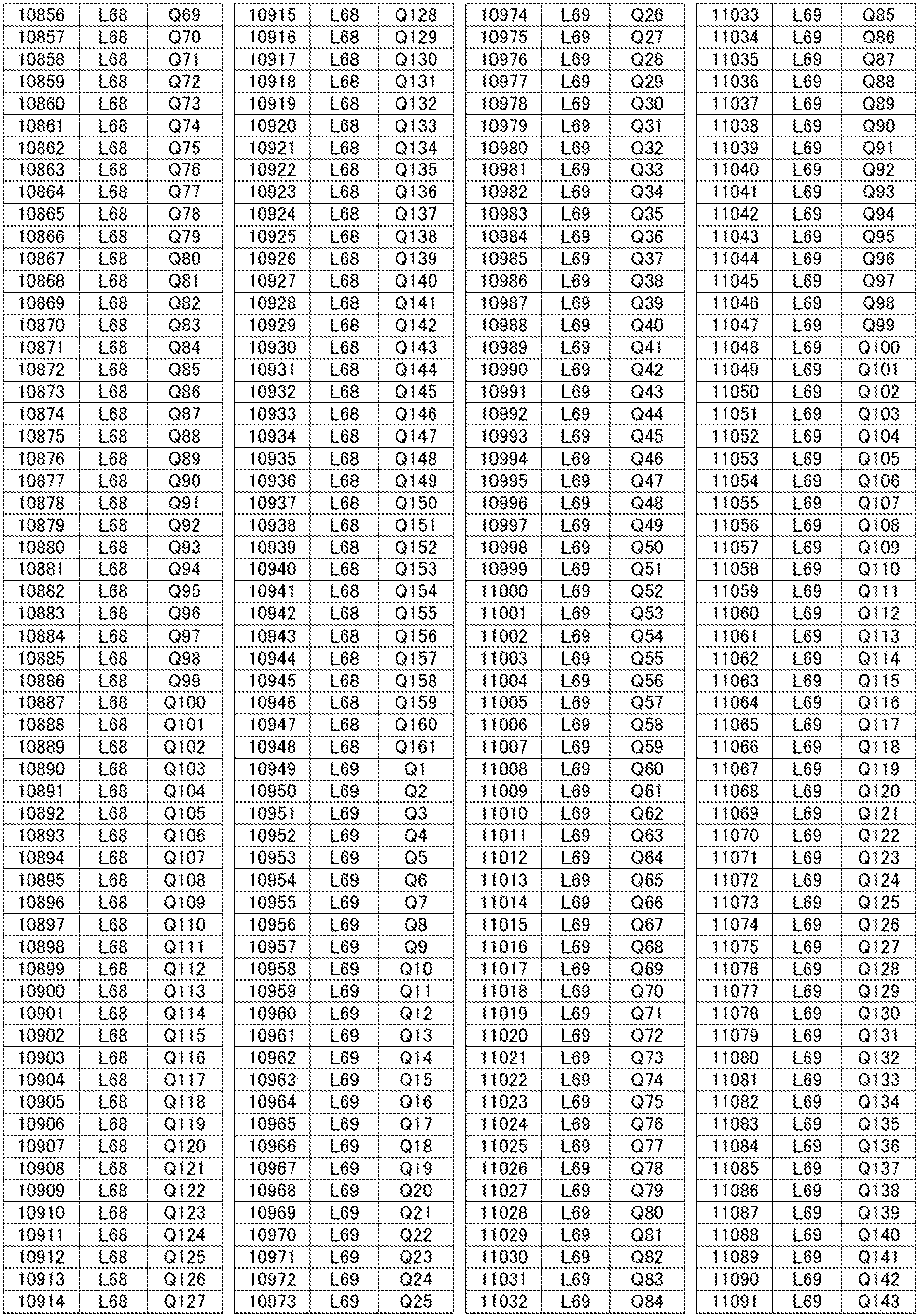

**[Table 1-48]**

| | | | | | | | | | | |
|---|---|---|---|---|---|---|---|---|---|---|
| 11092 | L69 | Q144 | | 11098 | L69 | Q150 | | 11104 | L69 | Q156 |
| 11093 | L69 | Q145 | | 11099 | L69 | Q151 | | 11105 | L69 | Q157 |
| 11094 | L69 | Q146 | | 11100 | L69 | Q152 | | 11106 | L69 | Q158 |
| 11095 | L69 | Q147 | | 11101 | L69 | Q153 | | 11107 | L69 | Q159 |
| 11096 | L69 | Q148 | | 11102 | L69 | Q154 | | 11108 | L69 | Q160 |
| 11097 | L69 | Q149 | | 11103 | L69 | Q155 | | 11109 | L69 | Q161 |

A specific example of another embodiment of the compound of the invention includes a compound represented by formula (11): 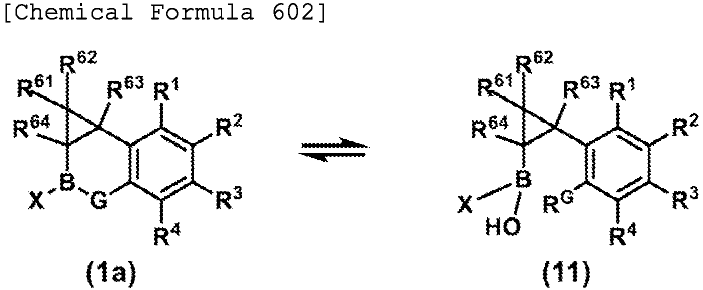 or a pharmaceutically acceptable salt thereof. In formula (11), R^{G} is a hydroxyl group, a thiol group, or -NHR^{a1}, R^{a1}, X, R¹, R², R³, R⁴, R⁶¹, R⁶², R⁶³, and R⁶⁴ are defined the same as the definition herein (e.g., any one of items 1 to 16 and items B1 to B15), and formula (1a) is defined the same as the definition herein.

A specific example of another embodiment of the compound of the invention includes a compound represented by formula (12): 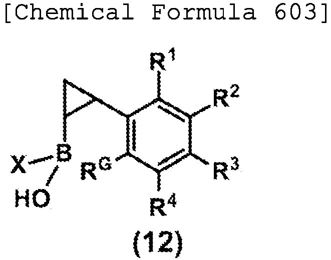 or a pharmaceutically acceptable salt thereof. In formula (12), R^{G} is a hydroxyl group, a thiol group, or -NHR^{a1}, R^{a1}, X, R¹, R², R³, and R⁴ are defined the same as the definition herein (e.g., item 16 or 17 or item B16 or B17). A compound of formula (12) is in an interchangeable relationship with, and thus can be biologically equivalent with, a compound of formula (1a) or (3a) due to an equilibrium reaction in an aqueous solution or in the body.

In some embodiments, X and R^{G} are hydroxyl groups, R⁴ is a carboxyl group, and ring A is an optionally substituted 4- to 6-membered nitrogen-containing non-aryl heterocycle.

In some embodiments, R^{G} is a hydroxyl group or a thiol group. In some embodiments, R^{G} is a hydroxyl group.

In some embodiments, X is a hydroxyl group or a C₁₋₆ alkoxy group. In some embodiments, X is a hydroxyl group.

In some embodiments, m is 1 or 2, n is 1 or 2, and m + n is 2 or 3. In some embodiments, m is 1, and n is 1.

In some embodiments, L³ is defined the same as the definition herein (e.g., item 25 or 26 or B24 or B25).

In some embodiments, L⁴ and R⁵ are defined the same as the definition herein (e.g., any one of items 27 and 66 to 100 and B26 and B60 to B89).

A specific example of another embodiment of the compound of the invention includes a compound represented by formula (13): 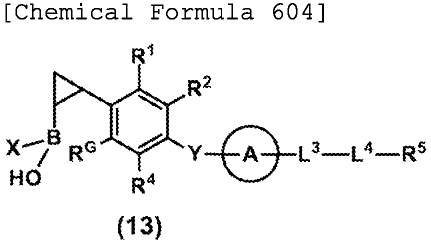 or a pharmaceutically acceptable salt thereof. In formula (13), X, Y, ring A, L³, L⁴, R¹, R², R⁴, R⁵, and R^{G} are defined the same as the definition herein (e.g., any one of items 19 to 23 and 29 to 32, 103, 104, B18 to B22, B28 to B31, B91, B92, etc.).

A specific example of another embodiment of the compound of the invention includes a compound represented by formula (14): 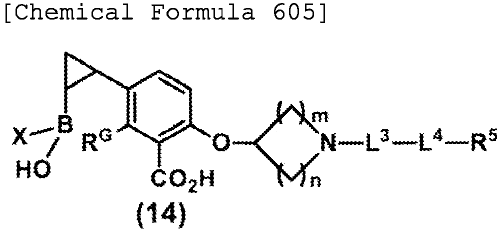 or a pharmaceutically acceptable salt thereof. In formula (14), X, L³, L⁴, m, n, R⁵, and R^{G} are defined the same as the definition herein (e.g., any one of items 33 to 65, 103, 104, B32 to B59, B91, B92, etc.).

A specific example of another embodiment of the compound of the invention includes a compound represented by formula (15): 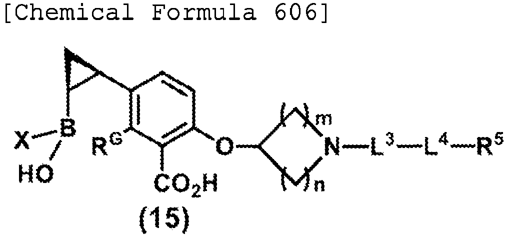 or formula (16): 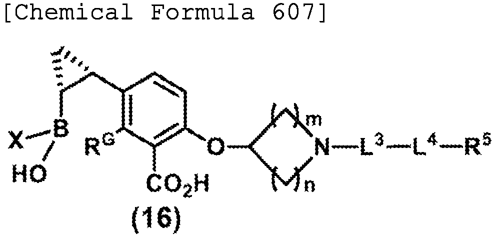 or a pharmaceutically acceptable salt thereof. In formula (15) or formula (16), X, L³, L⁴, m, n, R⁵, and R^{G} are defined the same as the definition herein (e.g., any one of items 33 to 65, 103, 104, B32 to B59, B91, item B92, etc.).

The compound of the invention is described further hereinafter.

The compound of the invention can have, depending on the type of substituent, a tautomer, stereoisomers such as geometric isomer, and enantiomer, which are encompassed by the present invention. Specifically, if the compound of the invention has one or more asymmetric carbon atoms, there is a diastereomer or an enantiomer, where a mixture of such a diastereomer or enantiomer or isolated diastereomer or enantiomer are also encompassed by the compound of the invention. Accordingly, a description of a structure of the compound of the invention herein is interpreted as encompassing all possible isomers, unless specifically noted otherwise. For example with regard to tautomers, a description of one of two descriptions of the following imidazole groups, when either one is described, is interpreted as encompassing both tautomers and mixtures of any ratio of the tautomers in the compound of the invention. 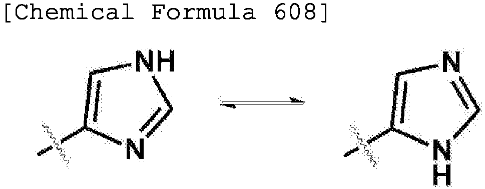 For example with regard to asymmetric carbon atoms, if the structure of a bond with a substituent is described with a solid line (see, for example, the following description examples) or this is described only by a formula without an explicit description of isomers (e.g., -CH(NH₂)-, -CMe(NH₂)-, or the like), possible diastereomers, enantiomers, and other isomers are interpreted so that even if an individual isomer is explicitly described separately herein, regardless of the description, a mixture of such isomers of any ratio and each of any isolated possible isomer are encompassed by the compound of the invention by the description of the structure with a solid line or a description with only a formula.

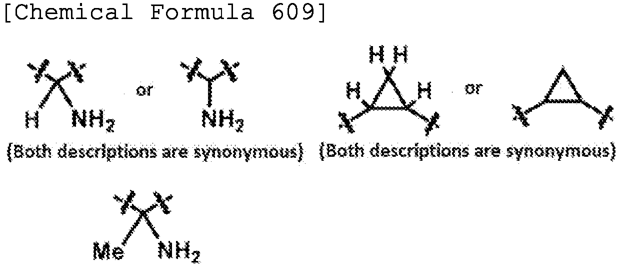

The compound of the invention can also have a structure represented by the following formula (11) due to an equilibrium state or the like, depending on the environment conditions such as temperature or humidity, or a physical factor in a solid, liquid, solution, or the like. The compound of the invention also encompasses compounds with such a structure. 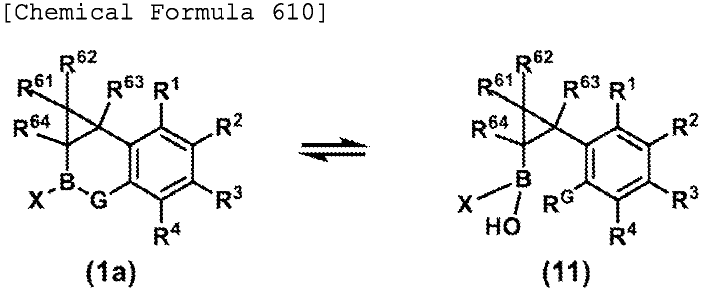 In formula (11), X represents a hydroxyl group, a thiol group, or -NHR^{a1}, R^{G}, R¹, R², R³, R⁴, R^{a1}, R⁶¹, R⁶², R⁶³, and R⁶⁴ are defined the same as the definitions herein, and formula (1a) is defined the same as the definition herein.

For example, the structures of the compounds in the Examples herein are based on estimation considered the most appropriate by those skilled in the art using proton nuclear magnetic resonance spectrum (¹H-NMR), liquid chromatography mass spectrometry (LCMS), or the like, but the structures are just estimates under each specific measurement environment. In particular, the structure of formula (1a), the structure of formula (1b), and the structure of formula (11) are possibly converted to each other or partially converted to one of the structures and mixed due to a property unique to each compound, various environmental conditions such as temperature or humidity, or physical factor in a solid, liquid, solution or the like.

The compound of the invention also includes various hydrates, solvates, and crystalline polymorphisms.

Furthermore, the compound of the invention may be substituted with an isotope (e.g., ²H (or D), ³H (or T), ¹¹C, ¹³C, ¹⁴C, ¹³N, ¹⁵N, ¹⁵O, ³⁵S, ¹⁸F, ¹²⁵I, or the like). Such compounds are also encompassed by the compound of the invention.

Prodrugs of the compound of the invention are also within the scope of the invention. As used herein, a prodrug refers to a derivative that results in the compound of formula (1a), (1b), or (11) by acid hydrolysis or enzymatic degradation in the body. If, for example, the compound of formula (1a), (1b), or (11) has a hydroxyl group, amino group, or carboxyl group, these groups can be modified in accordance with a conventional method to manufacture a prodrug.

Examples for a compound with a carboxy group include compounds whose carboxyl group has been converted to an alkoxycarbonyl group, alkylthiocarbonyl group, or alkylaminocarbonyl group.

Examples for a compound with an amino group include compounds whose amino group has been substituted with an alkanoyl group to be converted to an alkanoylamino group, substituted with an alkoxycarbonyl group to be converted to an alkoxycarbonylamino group, modified to an alkanoyloxymethylamino group, or converted to a hydroxylamine.

Examples for a compound with a hydroxyl group include compounds whose hydroxyl group has been substituted with the alkanoyl group described above to be converted to an alkanoyloxy group, converted to a phosphate ester, or converted to an alkanoyloxymethyloxy group.

Examples of the alkyl moiety of a group used in producing prodrugs include the alkyl group described above. The alkyl group is optionally substituted with, for example, an alkoxy group or the like. Preferred examples thereof include the following.

Examples of compounds whose carboxyl group has been converted to an alkoxycarbonyl group include alkoxycarbonyl such as methoxycarbonyl and ethoxycarbonyl, and alkoxycarbonyl substituted with an alkoxy group such as methoxymethoxycarbonyl, ethoxymethoxycarbonyl, 2-methoxyethoxycarbonyl, 2-methoxyethoxymethoxycarbonyl, and pivaloyloxymethoxycarbonyl.

As used herein, "pharmaceutically acceptable salt" refers to an acid addition salt or base addition salt which is pharmaceutically acceptable for use. Examples of "pharmaceutically acceptable salts" include, but are not limited to, acid addition salts such as acetate, propionate, butyrate, formate, trifluoroacetate, maleate, fumarate, tartrate, citrate, stearate, succinate, ethylsuccinate, malonate, lactobionate, gluconate, glucoheptonate, benzoate, methanesulfonate, benzenesulfonate, para-toluenesulfonate (tosylate), laurylsulfate, malate, ascorbate, mandelate, saccharinate, xinafoate, pamoate, cinnamate, adipate, cysteine salt, N-acetyl cysteine salt, hydrochloride, hydrobromide, phosphate, sulfate, hydroiodide, nicotinate, oxalate, picrate, thiocyanate, undecanoate, acrylic acid polymer salt, and carboxyvinyl polymer; inorganic base addition salts such as lithium salt, sodium salt, potassium salt, and calcium salt; organic base addition salts such as morpholine and piperidine; amino acid addition salts wherein the amino acid is aspartic acid or glutamic acid; and the like.

The compounds of the invention can be administered directly, or as a formulation, medicament, or a pharmaceutical composition using a suitable dosage form, by oral or parenteral administration. Specific examples of such dosage forms include, but are not limited to, tablets, capsules, powder, granules, liquid agents, suspension, injections, patches, poultice, and the like. These formulations can be manufactured by a known method using an additive that is commonly used as a pharmaceutical additive.

As these additives, an excipient, disintegrant, binding agent, fluidizer, lubricant, coating agent, solubilizing agent, solubilization promotor, thickener, dispersant, stabilizer, sweetener, flavoring agent, or the like can be used depending on the objective. Specific examples of these additives include, but are not limited to, lactose, mannitol, crystalline cellulose, lowsubstituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, and the like.

The dosage of the compound of the invention is appropriately selected depending on the animal targeted for administration, route of administration, disease, patient's age, body weight, and symptom. For example, the dosage is 0.01 mg as the lower limit (preferably 100 mg) and 10000 mg as the upper limit (preferably 6000 mg) per day for adults for oral administration. This amount can be administered once daily, or divided into several doses.

The compound of the invention is a compound with inhibitory activity against β-lactamase. Thus, the compound can be a prophylactic or therapeutic agent that is useful for a bacterial infection by combined use with an antimicrobial agent. Specific examples of such bacterial infections include sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, an odontogenic infection, and the like.

The compound of the invention can be used in combination with at least one agent selected from an antimicrobial agent, an antifungal agent, an antiviral agent, an anti-inflammatory agent, or an anti-allergic agent for treating one or more bacterial infections described herein. The agent is preferably an antimicrobial agent, and more preferably a β-lactam agent. Specific examples thereof include amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442. The timing of dosing of the compound of the invention and therapeutic agents thereof is not limited. The compound and therapeutic agent can be administered concurrently or sequentially to a subject being administered therewith. The compound of the invention and the therapeutic agents can be formulated as a combined agent. The dosage of the therapeutic agent can be appropriately selected based on the clinically used dose. The ratio of the compound of the invention and the therapeutic agents can be appropriately selected depending on the subject of administration, route of administration, target disease, symptom, combination, or the like.

In another embodiment of the invention, the compound of the invention can be combined and administered concomitantly or administered at different times upon use of a pharmaceutical composition comprising an antimicrobial agent such as a β-lactam agent. Such a pharmaceutical composition comprising a β-lactam agent is also within the scope of the invention, and can be used for treating or preventing a bacterial infection such as sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

Such a medicament, formulation, or pharmaceutical composition can be manufactured by mixing the compound of the invention and/or an additional agent (e.g., antimicrobial agent such as a β-lactam agent) with any suitable component, together or separately, as a combined agent or as separate agents using any technology that is known in the art. An appropriate formulation such as a tablet, capsule, powder, granule, liquid agent, suspension, injection, patch, or poultice can be formulated by using any technology that is known in the art. If the compound of the invention and/or an additional agent (e.g., antimicrobial agent such as a β-lactam agent) are prepared as separate agents, they can be provided as a kit of two agents. The kit can provide one of the components as a single agent, with instructions (package insert or the like) instructing to combine and administer the other component (for the compound of the invention, the additional agent (e.g., antimicrobial agent such as a β-lactam agent); for the addition agent (e.g., antimicrobial agent such as a β-lactam agent), the compound of the invention) concurrently or at different times.

If the compound of the invention is used as an active ingredient of a medicament, the compound can be intended for use in not just humans, but also animals other than humans (cat, dog, cow, chicken, fish, and the like).

Hereinafter, the method of manufacturing the compound of the invention is described with examples, but the present invention is not limited thereto.

The compound of the invention can be manufactured by, for example, the manufacturing methods described below, but the methods are not limited to such methods. These manufacturing methods can be appropriately improved upon based on the expertise of those skilled in the art of organic synthetic chemistry. Salts of the compounds used as a starting material can be used in the manufacturing method described below, as long as the reaction is not affected.

In the manufacturing methods described below, even if use of a protecting group is not specifically described, a functional group other than those at the reaction point can be protected as needed and deprotected after the completion of a reaction or after a series of reactions to obtain a compound of interest if one of the functional groups other than those at the reaction point is altered under the reaction condition or if it is unsuitable for post-reaction processing. Common protecting groups described in the document (T. W. Greene and P. G. M. Wuts, "Protective Group in Organic Synthesis", 3rd Ed., John Wiley and Sons, Inc., New York (1999)) or the like can be used as the protecting groups used in these processes. A protecting group can be introduced or removed by a method that is commonly used in organic synthetic chemistry (e.g., method described in the aforementioned document or the like) or a method in accordance therewith.

The starting material and intermediate in the manufacturing methods described below can be purchased as a commercially available product or are available by synthesis in accordance with a method described in a known document or a known method from a known compound. Salts of the starting material and intermediate can also be used, as long as the reaction is not affected.

The intermediate and compound of interest in the manufacturing methods described below can also be converted into another compound encompassed by the present invention by appropriately converting their functional groups. A functional group can be converted, in doing so, by a method that is commonly used in organic synthetic chemistry (e.g., the method described in R. C. Larock, "Comprehensive Organic Transformations", 2nd Ed., John Wiley and Sons, Inc., New York (1999) or the like) or a method in accordance therewith.

An inert solvent in the manufacturing methods described below refers to a solvent that does not react with starting materials, reagents, bases, acids, catalysts, ligands, or the like used in a reaction (hereinafter, also referred to as "starting materials or the like used in a reaction"). A solvent used in each step can be used as an inert solvent even if the solvent reacts with the starting materials or the like used in the reaction, as long as the reaction of interest proceeds to result in a compound of interest.

### Manufacturing Method 1

Compound (1a-1), which is a compound represented by formula (1a) (hereinafter, also referred to as compound (1a); the same applies to other compounds) wherein G = O, can be manufactured, for example, by the following manufacturing method. The compound (1a) defined in item 1 can also be manufactured in the same manner. 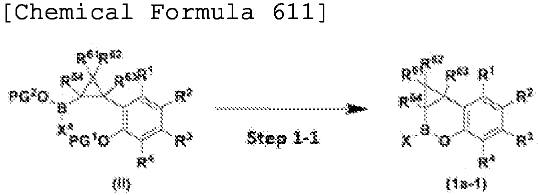 wherein X, R¹, R², R³, R⁴, R⁶¹, R⁶², R⁶³, and R⁶⁴ are defined the same as item 1, PG¹ represents a protecting group of a hydroxyl group (e.g., a tert-butoxycarbonyl group, acetyl group, methoxymethyl group, p-methoxybenzyl group, tert-butyldimethylsilyl group, trimethylsilyl group, or the like), X^{a} is OPG³ or defined the same as X defined in item 1, and PG² and PG³ represent protecting groups of boronic acid (e.g., an optionally substituted C₁₋₆ alkyl group, a structure represented by the following formula formed together by PG² and PG³ as -B(OPG²) (OPG³), or the like) .

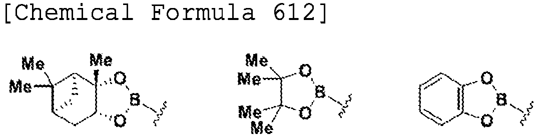

Step 1-1: In this reaction step, compound (1a-1) can be manufactured in accordance with a known method (e.g., WO 2014/151958, WO 2015/191907, WO 2016/003929, WO 2018/005662, etc.) by using the compound represented by formula (II) (hereinafter, also referred to as compound (II)) as a raw material. More specifically, the compound can be manufactured, for example, by using step 1-1-1 or step 1-1-2 described below. Compound (II) can be manufactured, for example, in the same manner as Manufacturing Methods 4, 8, 15-19, or the like described below or a known method from a corresponding raw material that can be purchased or prepared.

Step 1-1-1: Compound (1a-1) can be manufactured by reacting boronic acid with compound (II) in an inert solvent under an acidic condition. Examples of boronic acid include phenylboronic acid and 2-methylpropyl boronic acid. The boronic acid can be used in the range of 0.001 to 100 equivalents with respect to compound (II), which is preferably 1 to 3 equivalents. Examples of acids include hydrochloric acid, trifluoroacetic acid, and the like. An acid can be used in the range of 0.001 to 100 equivalents with respect to compound (II), which is preferably 1 to 20 equivalents. Specific examples of inert solvents include halogenated hydrocarbon solvents such as dichloromethane and dichloroethane, hydrocarbon solvents such as hexane and heptane, ether solvents such as tetrahydrofuran (THF) and cyclopentyl methyl ether (CPME), nitrile solvents such as acetonitrile and propionitrile, acetic acid, and water, which can be used alone or as a mixture solvent. The acids described above can also be directly used as a solvent. A mixture solvent of hexane/acetonitrile, acetic acid, or CPME is preferably used as a solvent. The reaction temperature is selected from the range of about -10°C to about 100°C, but is more preferably in the range of about 0°C to room temperature.

Step 1-1-2: compound (1a-1) can be manufactured by reacting triethylsilane with compound (II) in a trifluoroacetic acid solvent. The triethylsilane can be used in the range of 0.001 to 100 equivalents with respect to compound (II), which is preferably 1 to 50 equivalents. The reaction temperature is selected from the range of about -10°C to about 70°C.

### Manufacturing Method 2

Compound (1a-2), which is compound (1a-1) wherein R⁶¹, R⁶², R⁶³, and R⁶⁴ are hydrogen atoms, can be manufactured, for example, by the manufacturing method described below. 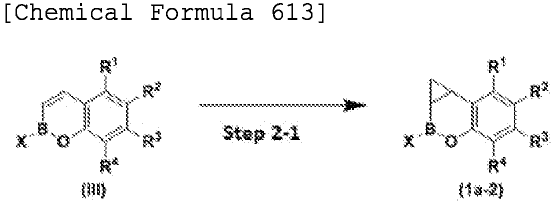 wherein X, R¹, R², R³, and R⁴ are defined the same as item 1.

Step 2-1: In this reaction step, compound (1a-2) can be manufactured in accordance with a known method (e.g., WO 2019/075084, etc.) by using a corresponding compound (III) as a raw material. Compound (III) can be manufactured, for example, in the same manner as Manufacturing Methods 12, 13, 23, or 24 described below or a known method from a corresponding raw material that can be purchased or prepared.

More specifically, compound (1a-2) can be manufactured, for example, by reacting metal carbenoid or halogenated organic zinc with compound (III) in an inert solvent. Examples of metal carbenoid include diazomethane/palladium (II) acetate (wherein (II) indicates that palladium is divalent). The diazomethane can be used in the range of 1 to 100 equivalents with respect to compound (III), which is preferably 1 to 3 equivalents. The palladium (II) acetate can be used in the range of 0.001 to 10 equivalents with respect to compound (III), which is preferably 0.01 to 1 equivalent. Examples of halogenated organic zinc include diiodomethane/diethylzinc. The diiodomethane can be used in the range of 1 to 100 equivalents with respect to compound (III), which is preferably 1 to 20 equivalents. The diethylzinc can be used in the range of 1 to 100 equivalents with respect to compound (III), which is preferably 1 to 10 equivalents. Specific examples of inert solvents include halogenated hydrocarbon solvents such as dichloromethane and dichloroethane, ether solvents such as THF and 1,2-dimethoxyethane (DME), which can be used alone or as a mixture solvent. The reaction temperature is selected from the range of about -100°C to the boiling point of the solvent used, but is more preferably in the range of about -78°C to room temperature.

### Manufacturing Method 3

Compound (1a-1) can be obtained as compound (1b-1), which is a compound represented by formula (1b) wherein G = O, by, for example, reacting with a reagent that generates nucleophilic X⁻ (X anion) (e.g., alkali metal salt generating a hydroxide anion HO⁻, alkali metal salt of C₁₋₆ alkoxide generating a C₁₋₆ alkoxide anion, the alkali metal salt of amide generating amide anion R^{a2}R^{b1}N⁻, or the like), depending on the property of compound. Compound (1b) defined in item 1 can be manufactured in the same manner. 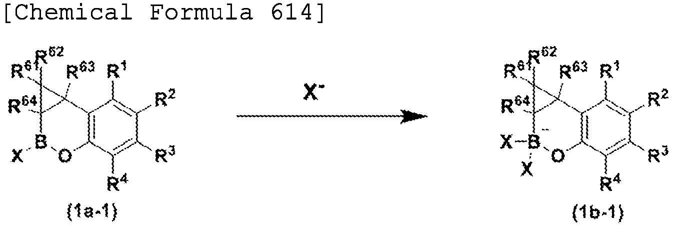 wherein X, R¹, R², R³, R⁴, R⁶¹, R⁶², R⁶³, and R⁶⁴ are defined the same as item 1.

For example, a compound of formula (1a-3), which is a compound represented by formula (1a-1) wherein X is a hydroxyl group, can be treated with an alkali aqueous solution to obtain a compound of formula (1b-3) as an alkali metal salt. As an alkali aqueous solution, an aqueous solution of the alkali metal salt generating a hydroxide anion HO⁻ described above (e.g., potassium carbonate, sodium carbonate, potassium hydrogen carbonate, sodium hydrogen carbonate, potassium dihydrogen phosphate, dipotassium hydrogen phosphate, potassium phosphate, sodium dihydrogen phosphate, disodium hydrogen phosphate, sodium phosphate, potassium hydroxide, sodium hydroxide, or the like) can be used. More specifically, for exmaple, compound (1a-3) can be treated with an aqueous sodium hydroxide solution to obtain a compound of formula (1b-3) as a sodium salt depending on the property of compound. Compound (1a-3) can be treated with an alkali aqueous solution upon purification or post-processing after a manufacturing reaction to be isolated as compound (1b-3), or converted into compound (1b-3) by applying said treatment on the isolated compound (1a-3). 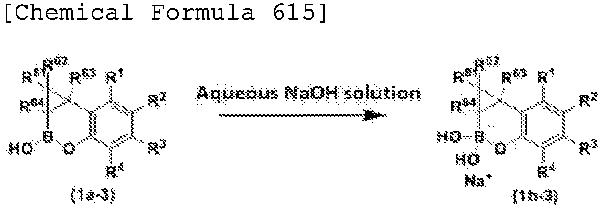 wherein R¹, R², R³, R⁴, R⁶¹, R⁶², R⁶³, and R⁶⁴ are defined the same as item 1.

For example, a compound of formula (1a-4), which is a compound represented by formula (1a-1) wherein X is a hydroxyl group and R⁴ is a carboxyl group, can be obtained as a disodium salt compound of formula (1b-4) by treatment with an aqueous sodium hydroxide solution depending on the property of the compound. 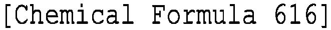 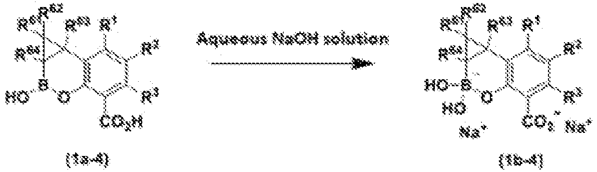 wherein R¹, R², R³, R⁶¹, R⁶², R⁶³, and R⁶⁴ are defined the same as item 1. In formula (1b-4), two sodium ions Na⁺ are described separately in the vicinity of two types of negatively charged groups, but this is synonymous to describing collectively as 2Na⁺ and represents disodium salt.

For example, a compound of formula (5a-1), which is a compound represented by formula (5a) defined in item 24 wherein R⁵ is a carboxyl group, is obtained in some cases as a trisodium salt compound of formula (5b-1), depending on the property of the compound, by treatment with an aqueous sodium hydroxide solution. 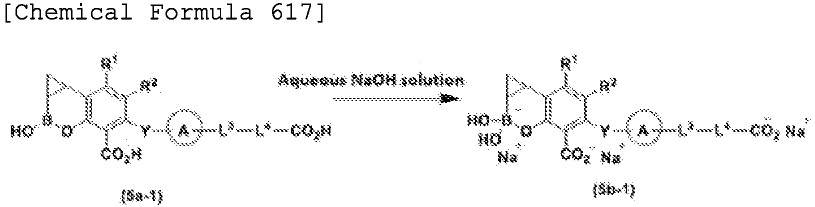 wherein A, L³, L⁴, Y, R¹, and R² are defined the same as any one of items 1 to 24. In formula (5b-1), three sodium ions Na⁺ are described separately in the vicinity of three types of negatively charged groups, but this is synonymous to describing collectively as 3Na⁺ and represents trisodium salt.

### Manufacturing Method 4

Compound (II), which is represented by formula (II-1) described below can be manufactured, for example, by the manufacturing method described below. 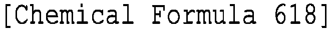 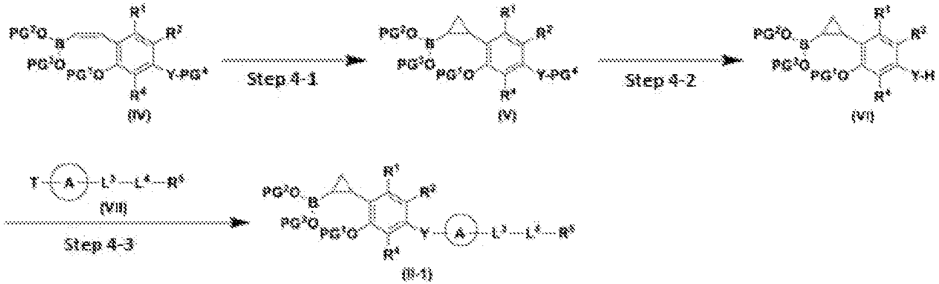 wherein Y, A, L³, L⁴, R¹, R², R⁴, and R⁵ are defined the same as item 1. PG⁴ represents a protecting group of a hydroxyl group (e.g., tert-butoxycarbonyl group, acetyl group, methoxymethyl group, p-methoxybenzyl group, tert-butyldimethylsilyl group, trimethylsilyl group, methyl group, or the like). In formula (VI), H in group -Y-H is a hydrogen atom, T represents a hydroxyl group or a leaving group (e.g., a halogen atom such as chlorine, bromine, or iodine, a lower alkylsulfonyloxy group such as methanesulfonyloxy, a trihalogenomethanesulfonyloxy group such as trifluoromethanesulfonyloxy, an arylsulfonyloxy group such as benzenesulfonyloxy or p-toluenesulfonyloxy, or the like), and PG¹, PG², and PG³ are each defined the same as the definition described in Manufacturing Method 1.

Step 4-1: Compound (V) can be manufactured by a method according to step 2-1 of Manufacturing Method 2 by using compound (IV) as a raw material. Compound (IV) can be manufactured, for example, in the same manner as Manufacturing Methods 5 to 7 described below or a known method from a corresponding raw material that can be purchased or prepared.

Step 4-2: Compound (VI) can be manufactured by deprotecting protecting group PG⁴ of compound (V). This step can be performed, for example, in accordance with the method described in a reference (T. W. Greene and P. G. M. Wuts, "Protective Group in Organic Synthesis", 3rd Ed., John Wiley and Sons, Inc., New York (1999) or the like).

Step 4-3: If Y is an oxygen atom, and T is a hydroxyl group, compound (II) can be manufactured by reacting compound (VII) with compound (VI) under the so-called Mitsunobu reaction in an inert solvent in the presence of an azo compound analog and an organic phosphorous compound or phosphorane compound under normal pressure or under pressure. Specific examples of inert solvents include ether solvents such as THF and DME, hydrocarbon solvents such as toluene and benzene, and the like. Examples of azo compound analogs include diethyl azodicarboxylate, diisopropyl azodicarboxylate, and the like. An azo compound analog can be used at 0.001 to 100 equivalents with respect to compound (VI), which is preferably 1 to 10 equivalents. Examples of organic phosphorous compounds include triphenylphosphine, tributylphosphine, and the like. An organic phosphorous compound can be used at 0.001 to 100 equivalents with respect to compound (VI), which is preferably 1 to 10 equivalents. Examples of phosphorane compounds include (cyanomethylene)tributylphosphorane, (cyanomethylene)trimethylphosphorane, and the like. A phosphorane compound can be used at 0.001 to 100 equivalents with respect to compound (VI), which is preferably 1 to 10 equivalents. The reaction temperature is selected from the range of about -10°C to about 100°C.

Alternatively, if Y is an oxygen atom, a sulfur atom, or - NR^{j}-, and T is a leaving group (e.g., a halogen atom such as chlorine, bromine, or iodine, a lower alkylsulfonyloxy group such as a methanesulfonyloxy group, a trihalogenomethanesulfonyloxy group such as a trifluoromethanesulfonyloxy group, an arylsulfonyloxy group such as a benzenesulfonyloxy group or p-toluenesulfonyloxy group, or the like), compound (II) can be manufactured by reacting compound (VII) with compound (VI) in an inert solvent in the presence of a base under normal pressure or under pressure. Specific examples of inert solvents include ether solvents such as THF and DME, halogenated hydrocarbon solvents such as dichloromethane and dichloroethane, aprotic solvents such as N,N-dimethylformamide (DMF), N-methylpyrrolidone (NMP), and dimethyl sulfoxide (DMSO), and the like. Examples of bases include potassium tert-butoxy, sodium hydride, triethylamine, diisopropylethylamine, potassium carbonate, sodium carbonate, cesium carbonate, and the like. A base can be used at 0.001 to 100 equivalents with respect to compound (VI), which is preferably 0.5 to 10 equivalents. Compound (VII) can be used at 0.001 to 100 equivalents with respect to compound (VI), which is preferably 1 to 10 equivalents. The reaction temperature is selected from the range of about -10°C to about 100°C.

### Manufacturing Method 5

Compound (IV) can be manufactured, for example, by the manufacturing method described below by using compound (VIII) as a raw material. 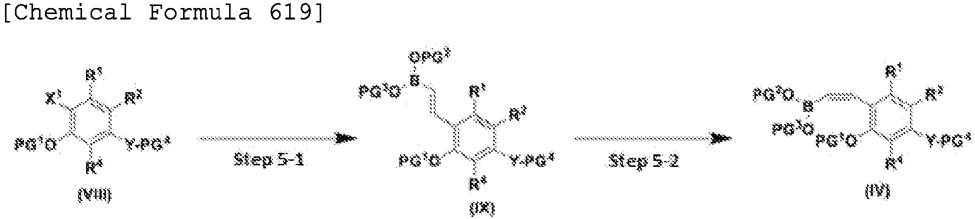 wherein Y, R¹, R², and R⁴ are defined the same as item 1. X¹ represents an iodine atom, a bromine atom, a chlorine atom, or a substituted sulfonyloxy group (e.g., methanesulfonyloxy group, trifluoromethanesulfonyloxy group, p-toluenesulfonyloxy group, or the like), PG¹, PG², and PG³ are each defined the same as the definition described in Manufacturing Method 1, and PG⁴ is defined the same as the definition described in Manufacturing Method 4.

A commercially available product that is purchased or a compound synthesized in accordance with a known method (e.g., the method described in R. C. Larock, "Comprehensive Organic Transformations", 2nd Ed., John Wiley and Sons, Inc., New York (1999), or the method described in WO 2016/003929, WO 2016/149393, or the like) from a known compound as the starting material compound (VIII).

Step 5-1: Compound (IX) can be manufactured by a coupling reaction of vinyl boronate ester with compound (VIII) in an inert solvent in the presence of a transition metal catalyst and a base under a so-called Heck reaction condition. The reaction may be performed in the presence of a ligand as needed. Specific examples of vinyl boronate ester include 4,4,5,5-tetramethyl-2-vinyl-1,3,2-dioxaborolane. The vinyl boronate ester can be used in the range of 1 to 100 equivalents with respect to compound (VIII), which is preferably 1 to 3 equivalents. Specific examples of transition metal catalysts include palladium (II) acetate, tris(dibenzylideneacetone)dipalladium(0), and bis(tri-tert-butylphosphine)palladium(0) (wherein (II) and (0) indicate that palladium is divalent and zerovalent, respectively). The transition metal catalyst can be used in the range of 0.001 to 10 equivalents with respect to compound (VIII), which is preferably 0.01 to 1 equivalent. Specific examples of bases include organic bases such as triethylamine and inorganic bases such as potassium carbonate, silver carbonate, and potassium acetate. The base can be used in the range of 1 to 100 equivalents with respect to compound (VIII), which is preferably 1 to 5 equivalents. Specific examples of ligands include BINAP and tri(o-tolyl)phosphine. The ligand can be used in the range of 0.001 to 10 equivalents with respect to compound (VIII), which is preferably 0.01 to 2 equivalents. Specific examples of inert solvents include halogenated hydrocarbon solvents such as dichloromethane and dichloroethane, ether solvents such as THF and CPME, aromatic hydrocarbon solvents such as toluene and benzene, and aprotic polar solvents such as DMF and N,N-dimethylacetamide (DMA), which can be used alone or as a mixture solvent. Toluene is preferably used as a solvent. The reaction temperature is selected from the range of room temperature to the boiling point of the solvent used, but is more preferably in the range of about 50°C to about 130°C.

Step 5-2: Compound (IV) can be manufactured from corresponding compound (IX) in accordance with a known method (e.g., Angewandte Chemie International Edition (2018), 57(XII), 3168-3172).

More specifically, compound (IV) is a compound in which a geometric isomerism of a double bond is a (Z) form (or cis form). This compound can be manufactured through an EZ isomerization reaction by light irradiation on compound (IX) obtained primarily as an (E) form (or trans form) in the previous step in the presence of a photoreaction catalyst (photosensitizer) in an inert solvent. Specific examples of photoreaction catalysts include tris(2-phenylpyridinato)iridium(III). The photoreaction catalyst can be used in the range of 0.001 to 10 equivalents with respect to compound (IX), which is preferably 0.01 to 2 equivalents. Preferred examples of wavelengths of irradiated light include 450 nm. Specific examples of inert solvents include acetonitrile. The reaction temperature is selected from the range of about -10°C to the boiling point of the solvent used, but is more preferably in the range of about 0°C to about 50°C.

### Manufacturing Method 6

Compound (IV) can be manufactured, for example, by the manufacturing method described below by using compound (VIII) as a raw material. Compound (IV) can also be manufactured from compound (VIII) in accordance with a known method (e.g., WO 2018/005662, etc.) . 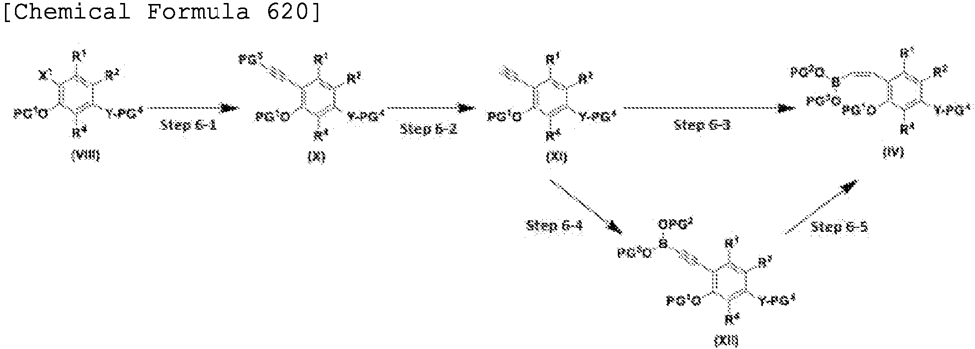 wherein Y, R¹, R², and R⁴ are defined the same as item 1. PG⁵ is a protecting group of terminal alkyne (trimethylsilyl group, tert-butyldimethylsilyl group, or the like), X¹ is defined the same as the definition described in Manufacturing Method 5, PG¹, PG², and PG³ are each defined the same as the definition described in Manufacturing Method 1, and PG⁴ is defined the same as the definition described in Manufacturing Method 4.

Step 6-1: Compound (X) can be manufactured through a coupling reaction of a terminal alkyne compound with only one end of acetylene protected by PG⁵ with compound (VIII) in an inert solvent in the presence of a transition metal catalyst, copper catalyst, and base under a so-called Sonogashira reaction condition. The reaction can be performed in the presence of a ligand as needed. Specific examples of terminal alkyne compounds include trimethylsilylacetylene and (tert-butyldimethylsilyl)acetylene. The terminal alkyne compound can be used in the range of 1 to 100 equivalents with respect to compound (VIII), which is preferably 1 to 3 equivalents. Specific examples of transition metal catalysts include tetrakis(triphenylphosphine)palladium(0), dichlorobis(triphenylphosphine)palladium(II), dichlorobis(acetonitrile)palladium(II), and palladium(II) chloride (wherein (0) and (II) indicate that palladium is zerovalent and divalent, respectively). The transition metal catalyst can be used in the range of 0.001 to 10 equivalents with respect to compound (VIII), which is preferably 0.01 to 1 equivalent. Specific examples of copper catalysts include copper powder, halogenated copper (I), copper (I) acetate, and the like, and preferred examples include copper (I) iodide (wherein (I) indicates that copper is monovalent). The copper catalyst can be used in the range of 0.001 to 10 equivalents with respect to compound (VIII), which is preferably 0.01 to 1 equivalent. Specific examples of bases include diethylamine, triethylamine, and the like. The base can be used in the range of 1 to 100 equivalents with respect to compound (VIII), which is preferably 1 to 5 equivalents. Specific examples of ligands include triphenylphosphine, tri-tert-butylphosphine, 2,2-bis(diphenylphosphino)-1,1-binaphthyl, 2-(ditert-butylphosphino)biphenyl, 1,1'-bis(diphenylphosphino)ferrocene, and the like. The ligand can be used in the range of 0.001 to 10 equivalents with respect to compound (VIII), which is preferably 0.01 to 2 equivalents. Specific examples of inert solvents include ether solvents such as tetrahydrofuran (THF) and diethyl ether, and aprotic polar solvents such as N,N-dimethylformamide (DMF), which can be used alone or as a mixture solvent. Toluene is preferably used as a solvent. The reaction temperature is selected from the range of about -78°C to the boiling point of the solvent used.

Step 6-2: Compound (XI) can be manufactured by removing protecting group PG⁵ of compound (X) from using a known deprotecting reaction (see, for example, T. W. Greene and P. G. M. Wuts, "Protective Group in Organic Synthesis", 3rd Ed., John Wiley and Sons, Inc., New York (1999)).

Step 6-3: Compound (IV) can be manufactured from a corresponding compound (XI) in accordance with a known method (e.g., Journal of the American Chemical Society (2000), 122(XX), 4990-4991).

Step 6-4: Compound (XII) can be manufactured from a corresponding compound (XI) in accordance with a known method (e.g., Chemical Science (2017), 8(1), 165-168).

Step 6-5: Compound (IV) can be manufactured by catalytic hydrogenation on compound (XII) in an inert solvent in the presence of a catalyst and under a hydrogen atmosphere. Specific examples of catalysts include a Lindlar's catalyst. The catalyst can be used in the range of 0.005 to 50 equivalents with respect to compound (XII), which is preferably 0.01 to 10 equivalents. Specific examples of inert solvents include ester solvents such as ethyl acetate and alcohol solvents such as methanol and ethanol, which can be used alone or as a mixture solvent. The reaction temperature is selected from the range of about -20°C to the boiling point of the solvent used, but is more preferably in the range of about 0°C to about 50°C. Hydrogen pressure is generally normal pressure, but can be adjusted to medium or high pressure in accordance with the reactivity of a compound.

### Manufacturing Method 7

Compound (IV) can be manufactured, for example, by the manufacturing method described below by using compound (VIII) as a raw material. 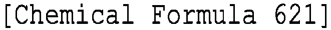 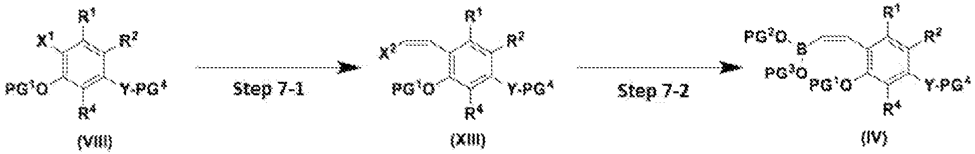 wherein Y, R¹, R², and R⁴ are defined the same as item 1. X² is an iodine atom, a bromine atom, a chlorine atom, or a substituted sulfonyloxy group (e.g., a methanesulfonyloxy group or a trifluoromethanesulfonyloxy group), X¹ is defined the same as the definition described in Manufacturing Method 5, PG¹, PG², and PG³ are each defined the same as the definition described in Manufacturing Method 1, and PG⁴ is defined the same as the definition described in Manufacturing Method 4.

Step 7-1: Compound (XIII) can be manufactured from a corresponding compound (VIII) in accordance with a known method (e.g., WO 2018/005662, WO 2019/075084, etc.).

Step 7-2: Compound (IV) can be manufactured by a coupling reaction of compound (XIII) with a corresponding borane compound in an inert solvent in the presence of a transition metal catalyst and a base under a so-called Miyaura-Ishiyama borylation reaction condition. The reaction can be performed in the presence of a ligand as needed. Specific examples of borane compounds include pinacolborane, pinacoldiborane, and diboronic acid. A borane compound can be used in the range of 1 to 100 equivalents with respect to compound (XIII), which is preferably 1 to 3 equivalents. Specific examples of transition metal catalysts include [1,1'bis(diphenylphosphino)ferrocene]palladium(II) dichloride, tris(dibenzylideneacetone)palladium(0), and the like (wherein (II) and (0) indicate that palladium is divalent and zerovalent, respectively). An transition metal catalyst can be used in the range of 0.001 to 10 equivalents with respect to compound (XIII), which is preferably 0.01 to 1 equivalents. Specific examples of ligands include triphenylphosphine, XPhos, 2-(di-tert-butylphosphino)biphenyl, 1,1'-bis(diphenylphosphino)ferrocene, and the like. A ligand can be used in the range of 0.001 to 10 equivalents with respect to compound (XIII), which is preferably 0.01 to 2 equivalents. Specific examples of bases include potassium acetate, potassium phenoxide, and the like. A base can be used in the range of 1 to 100 equivalents with respect to compound (XIII), which is preferably 1 to 5 equivalents. Specific examples of inert solvents include halogenated hydrocarbon solvents such as dichloromethane and dichloroethane, ether solvents such as THF and 1,4-dioxane, and aprotic polar solvents such as DMF and DMSO, which can be used alone or as a mixture solvent. The reaction temperature is selected from the range of about -20°C to about 180°C, but is more preferably in the range of about 50°C to about 130°C.

### Manufacturing Method 8

Compound (II-1) can be manufactured, for example, by the manufacturing method described below by using compound (XIV) as a raw material. 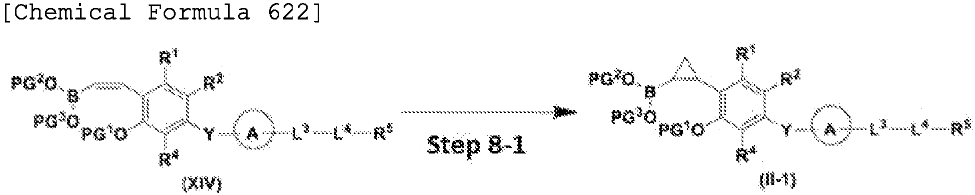 wherein Y, A, L³, L⁴, R¹, R², R⁴, and R⁵ are defined the same as item 1. PG¹, PG², and PG³ are each defined the same as the definition described in Manufacturing Method 1.

Step 8-1: Compound (II) can be manufactured by the method in accordance with step 2-1 in Manufacturing Method 2 by using compound (XIV) as a raw material. Compound (XIV) can be manufactured, for example, in the same manner as Manufacturing Methods 9 to 11 described below or a known method from a corresponding raw material that can be purchased or prepared.

### Manufacturing Method 9

Compound (XIV) can be manufactured, for example, by the manufacturing method described below by using compound (IV) as a raw material. 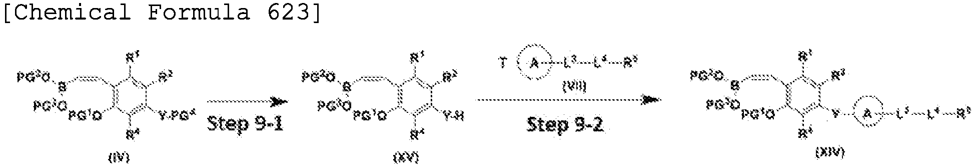 wherein Y, A, L³, L⁴, R¹, R², R⁴, and R⁵ are defined the same as item 1. PG¹, PG², and PG³ are each defined the same as the definition described in Manufacturing Method 1, and T and PG⁴ are defined the same as the definition described in Manufacturing Method 4.

Step 9-1: compound (XV) can be manufactured by a method in accordance with step 4-2 in Manufacturing Method 4 by using compound (IV) as a raw material.

Step 9-2: Compound (XIV) can be manufactured by a method in accordance with step 4-3 in Manufacturing Method 4 by using compound (XV) as a raw material.

### Manufacturing Method 10

Compound (XIV) can also be manufactured, for example, by the manufacturing method described below by using compound (IX) as a raw material. 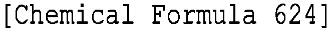 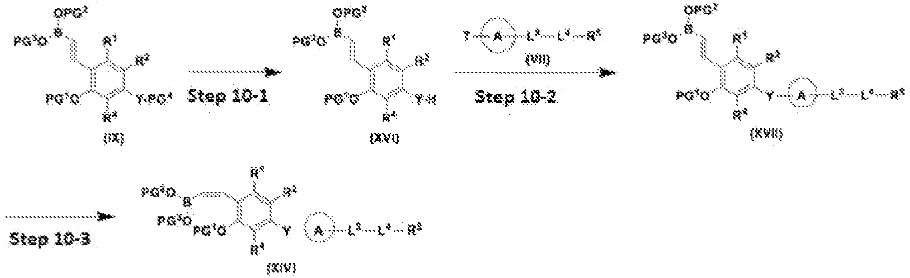 wherein Y, A, L³, L⁴, R¹, R², R⁴, and R⁵ are defined the same as item 1. PG¹, PG², and PG³ are each defined the same as the definition described in Manufacturing Method 1, H of group -Y-H in formula (XVI) is a hydrogen atom, and T and PG⁴ are defined the same as the definition described in Manufacturing Method 4.

Step 10-1: Compound (XVI) can be manufactured by a method in accordance with step 4-2 in Manufacturing Method 4 by using compound (IX) as a raw material.

Step 10-2: Compound (XVII) can be manufactured by a method in accordance with step 4-3 in Manufacturing Method 4 by using compound (XVI) as a raw material.

Step 10-3: Compound (XIV) can be manufactured by a method in accordance with step 5-2 in Manufacturing Method 5 by using compound (XVII) as a raw material.

### Manufacturing Method 11

Compound (XIV) can also be manufactured, for example, by the manufacturing method described below by using compound (XII) as a raw material. 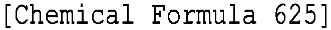 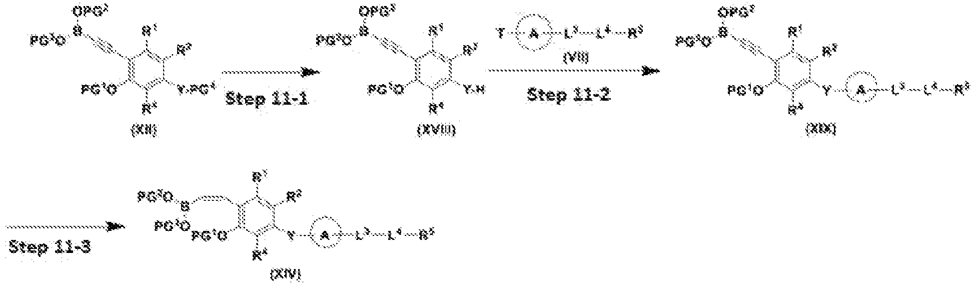 wherein Y, A, L³, L⁴, R¹, R², R⁴, and R⁵ are defined the same as item 1. PG¹, PG², and PG³ are each defined the same as the definition described in Manufacturing Method 1, H of group -Y-H in formula (XVIII) is a hydrogen atom, and T and PG⁴ are defined the same as the definition described in Manufacturing Method 4.

Step 11-1: Compound (XVIII) can be manufactured by a method in accordance with step 4-2 in Manufacturing Method 4 by using compound (XII) as a raw material.

Step 11-2: Compound (XIX) can be manufactured by a method in accordance with step 4-3 in Manufacturing Method 4 by using compound (XVIII) as a raw material.

Step 11-3: Compound (XIV) can be manufactured by a method in accordance with step 6-5 in Manufacturing Method 6 by using compound (XIX) as a raw material.

### Manufacturing Method 12

Compound (III) represented by formula (III-1) can be manufactured, for example, by the manufacturing method described below by using compound (XIV) as a raw material. 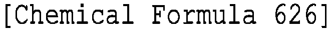 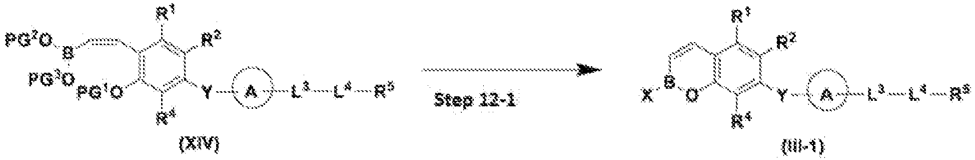 wherein X, Y, A, L³, L⁴, R¹, R², R⁴, and R⁵ are defined the same as item 1. PG¹, PG², and PG³ are each defined the same as the definition described in Manufacturing Method 1.

Step 12-1: Compound (III-1) can be manufactured by a method in accordance with step 1-1, step 1-1-1, or step 1-1-2 in Manufacturing Method 1 by using compound (XIV) as a raw material.

### Manufacturing Method 13

Compound (III-1) can also be manufactured, for example, by the manufacturing method described below by using compound (X) as a raw material. 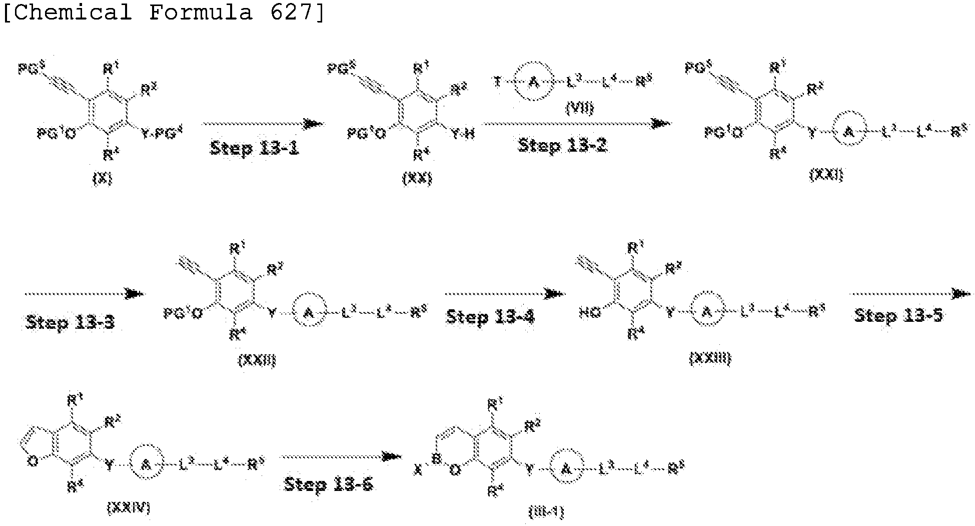 wherein Y, A, L³, L⁴, R¹, R², R⁴, and R⁵ are defined the same as item 1. PG¹, PG², and PG³ are each defined the same as the definition described in Manufacturing Method 1, H of group -Y-H in formula (XX) is a hydrogen atom, T and PG⁴ are defined the same as the definition described in Manufacturing Method 4, and PG⁵ is defined the same as the definition described in Manufacturing Method 6.

Step 13-1: Compound (XX) can be manufactured by a method in accordance with step 4-2 in Manufacturing Method 4 by using compound (X) as a raw material.

Step 13-2: Compound (XXI) can be manufactured by a method in accordance with step 4-3 in Manufacturing Method 4 by using compound (XX) as a raw material.

Step 13-3: Compound (XXII) can be manufactured by a method in accordance with step 6-2 in Manufacturing Method 6 by using compound (XXI) as a raw material.

Step 13-4: Compound (XXIII) can be manufactured by a method in accordance with step 4-2 in Manufacturing Method 4 by using compound (XXII) as a raw material.

Step 13-5: Compound (XXIV) can be manufactured by an intramolecular cyclization reaction on compound (XXIII) in an inert solvent in the presence of a catalyst and a base. Specific examples of catalysts include copper iodide and the like. A catalyst can be used in the range of 0.001 to 10 equivalents with respect to compound (XXIII), which is preferably 0.01 to 1 equivalent. Specific examples of bases include triethylamine, sodium carbonate, and the like. A base can be used in the range of 1 to 200 equivalents with respect to compound (XXIII), which is preferably 1 to 50 equivalents. Specific examples of inert solvents include alcohol solvents such as ethanol and aprotic polar solvents such as DMF and DMSO, which can be used alone or as a mixture solvent. The reaction temperature is selected from the range of about 0°C to about 180°C, but is more preferably in the range of about 50°C to about 130°C.

Step 13-6: Compound (III-1) can be manufactured from a corresponding compound (XXIV) in accordance with a known method (e.g., Journal of the American Chemical Society (2016), 138 (47), 15315-15318, WO 2019/075084, etc.). More specifically, compound (III-1) can be manufactured by reacting diboron with compound (XXIV) in an inert solvent in the presence of a transition metal catalyst and a base. Examples of diboron include bis(pinacolato)diboron and bis(pinanediolato)diboron. Diboron can be used in the range of 1 to 100 equivalents with respect to compound (XXIV), which is preferably 1 to 3 equivalents. Examples of transition metal catalysts include [1,3-bis(2,6-diisopropylphenyl)imidazol-2-ylidene]triphenylphosphine nickel (II) dichloride and bis(1,5-cyclooctadiene) nickel(0)/1,3-dimethylimidazol-2-ylidene (wherein (II) and (0) indicate that nickel is divalent and zerovalent, respectively). A transition metal catalyst can be used in the range of 0.001 to 10 equivalents with respect to compound (XXIV), which is preferably 0.01 to 1 equivalent. Specific examples of bases include potassium carbonate and cesium carbonate, and cesium carbonate is preferably used. A base can be used in the range of 1 to 100 equivalents with respect to compound (XXIV), which is preferably 1 to 5 equivalents. Specific examples of inert solvents include halogenated hydrocarbon solvents such as dichloromethane and dichloroethane, ether solvents such as THF and CPME, and aromatic hydrocarbon such as toluene and benzene, which can be used alone or as a mixture solvent. Toluene is preferably used as an inert solvent. The reaction temperature is selected from the range of room temperature to the boiling point of the solvent used, but is more preferably in the range of about 80°C to about 110°C.

### Manufacturing Method 14

Compound (XXIV) can also be manufactured, for example, by the manufacturing method described below by using compound (X) as a raw material. 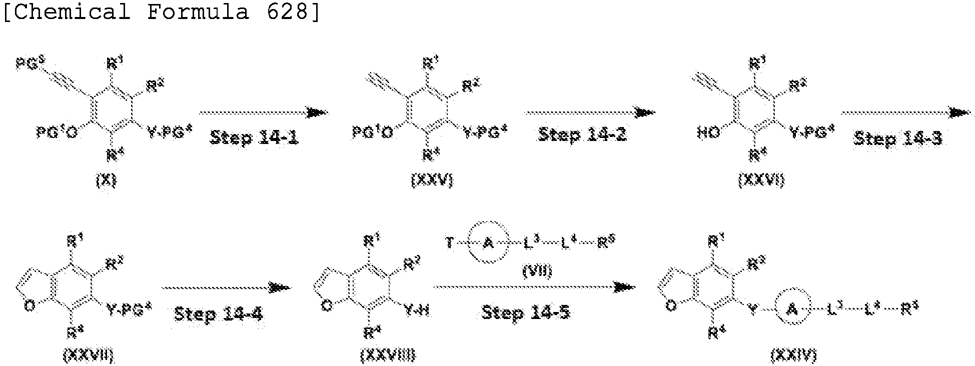 wherein Y, A, L³, L⁴, R¹, R², R⁴, and R⁵ are defined the same as item 1. PG¹, PG², and PG³ are each defined the same as the definition described in Manufacturing Method 1, H of group -Y-H in formula (XXVIII) is a hydrogen atom, T and PG⁴ are defined the same as the definition described in Manufacturing Method 4, and PG⁵ is defined the same as the definition described in Manufacturing Method 6.

Step 14-1: Compound (XXV) can be manufactured by a method in accordance with step 6-2 in Manufacturing Method 6 by using compound (X) as a raw material.

Step 14-2: Compound (XXVI) can be manufactured by a method in accordance with step 4-2 in Manufacturing Method 4 by using compound (XXV) as a raw material.

Step 14-3: Compound (XXVII) can be manufactured by a method in accordance with step 13-5 in Manufacturing Method 13 by using compound (XXVI) as a raw material.

Step 14-4: Compound (XXVIII) can be manufactured by a method in accordance with step 4-2 in Manufacturing Method 4 by using compound (XXVII) as a raw material.

Step 14-5: Compound (XXIV) can be manufactured by a method in accordance with step 4-3 in Manufacturing Method 4 by using compound (XXVIII) as a raw material.

### Manufacturing Method 15

A compound represented by formula (II), which is represented by formula (II-2), can be manufactured, for example, by the manufacturing method described below by using compound (XXIX) as a raw material. 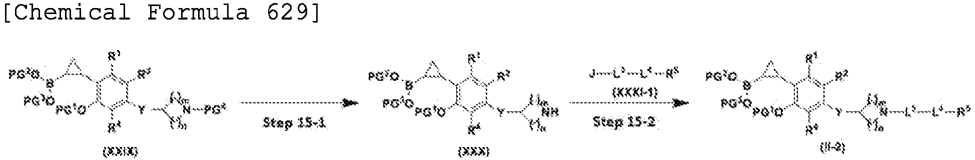 wherein Y, L³, L⁴, R¹, R², R⁴, and R⁵ are defined the same as item 1, and m and n are defined the same as item 33. PG¹, PG², and PG³ are each defined the same as the definition described in Manufacturing Method 1, PG⁶ is a protecting group of an amino group (e.g., an ethoxycarbonyl group, tert-butoxycarbonyl group, acetyl group, benzoyl group, trifluoroacetyl group, benzyloxycarbonyl group, 3or 4-chlorobenzyloxycarbonyl group, triphenylmethyl group, methanesulfonyl group, p-toluenesulfonyl group, trimethylsilyl group, benzyloxycarbonyl group, 3- or 4-chlorobenzyloxycarbonyl group, benzylsulfonyl group, benzyl group, 4-nitrobenzyl group, 4-methoxybenzyl group, methyl group, ethyl group, or the like), and J is a leaving group (e.g., a halogen atom such as chlorine, bromine, or iodine, or a substituted sulfonyloxy group (e.g., a methanesulfonyloxy group, a trifluoromethanesulfonyloxy group, a p-toluenesulfonyloxy group, or the like)).

Step 15-1: Compound (XXX) can be manufactured by deprotecting protecting group PG⁶ of compound (XXIX). This step can be performed, for example, in accordance with a method described in a reference (T. W. Greene and P. G. M. Wuts, "Protective Group in Organic Synthesis", 3rd Ed., John Wiley and Sons, Inc., New York (1999)) or the like. Compound (XXIX) can be manufactured, for example, in the same manner as Manufacturing Method 18 or 19 described below or a known method from a corresponding raw material that can be purchased or prepared.

Step 15-2: Compound (II-2) can be manufactured by reacting compound (XXXI-1) in an inert solvent in the presence of a base by using compound (XXX) as a raw material. The reaction can be performed in the presence of a suitable phase transfer catalyst as needed. Compound (XXXI-1) can be used at 0.001 to 100 equivalents with respect to compound (XXX), which is preferably 1 to 10 equivalents.

Specific examples of bases include organic bases such as triethylamine, N,N-diisopropylethylamine, and pyridine, inorganic bases such as potassium carbonate, sodium carbonate, cesium carbonate, potassium hydrogen carbonate, sodium hydrogen carbonate, potassium dihydrogen phosphate, dipotassium hydrogen phosphate, potassium phosphate, sodium dihydrogen phosphate, disodium hydrogen phosphate, sodium phosphate, potassium hydroxide, sodium hydroxide, and sodium hydride, and metal alkoxide such as sodium methoxide and potassium tert-butoxide. A base can be used at 0.001 to 100 equivalents with respect to compound (XXX), which is preferably 0.5 to 10 equivalents. Specific examples of phase transfer catalysts include tetrabutylammonium hydrogensulfate and the like. A phase transfer catalyst can be used at 0.001 to 100 equivalents with respect to compound (XXX), which is preferably 0.1 to 10 equivalents. Specific examples of inert solvents include halogenated hydrocarbons such as chloroform and dichloromethane, aromatic hydrocarbons such as benzene and toluene, ether solvents such as tetrahydrofuran (THF), diethyl ether, 1,4-dioxane, and 1,2-dimethoxyethane (DME), lower alcohol such as methanol, ethanol, and 2-propanol, aprotic polar solvents such as acetonitrile, N,N-dimethylformamide (DMF), and N-methyl-2-pyrrolidone (NMP), water, and mixture solvents thereof. The reaction temperature is selected from the range of about -20°C to the boiling point of the solvent used.

### Manufacturing Method 16

A compound of formula (II) represented by formula (II-3) can be manufactured, for example, by the manufacturing method described below by using compound (XXX) as a raw material. 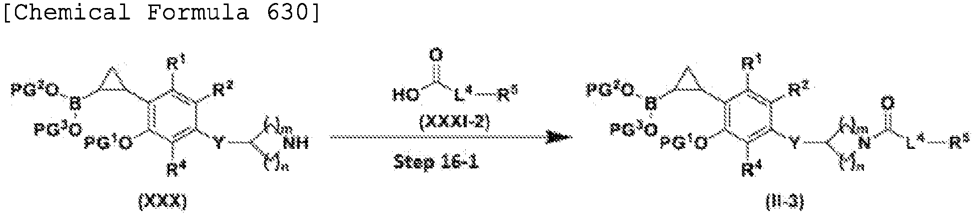 wherein Y, L⁴, R¹, R², R⁴, and R⁵ are defined the same as item 1, and m and n are defined the same as item 33. PG¹, PG², and PG³ are each defined the same as the definition described in Manufacturing Method 1, and PG⁶ is defined the same as the definition described in Manufacturing Method 15.

Step 16-1: Compound (II-3) can be manufactured by a condensation reaction of compound (XXX) with compound (XXXI-2) in an inert solvent in the presence of a condensing agent. Compound (XXXI-2) can be used at 0.001 to 100 equivalents with respect to compound (XXX), which is preferably 1 to 10 equivalents. Various condensing agents that are used in a conventional method can be used as the condensing agent. Examples thereof include 1-ethyl-3-(3-dimethylaminopropyl)carbodiimide (EDC, EDCI) (and hydrochlorides thereof), O-(7-azabenzotriazol-1-yl)-N,N,N',N',-tetramethyluronium hexafluorophosphate (HATU), and the like. A condensing agent can be used at 0.001 to 100 equivalents with respect to compound (XXX), which is preferably 1 to 10 equivalents. Examples of bases include diisopropylethylamine, triethylamine, and the like. A base can be used at 0.001 to 100 equivalents with respect to compound (XXX), which is preferably 1 to 10 equivalents. Specific examples of inert solvents include ether solvents such as THF and DME, halogenated hydrocarbon solvents such as dichloromethane and chloroform, aprotic solvents such as DMF, NMP, and DMSO, and the like. The reaction temperature is selected from the range of about -78°C to about 100°C.

### Manufacturing Method 17

A compound of formula (II) represented by formula (II-4) can be manufactured, for example, by the manufacturing method described below by using compound (XXX) as a raw material,. 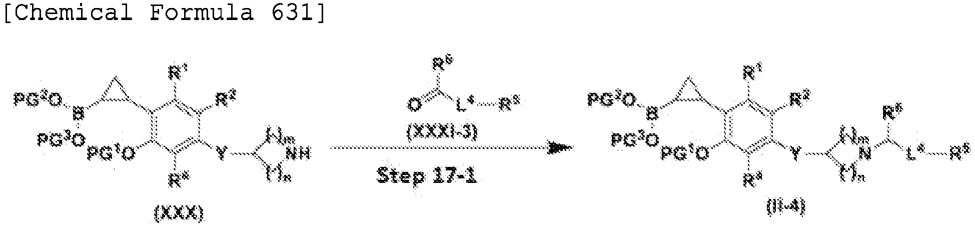 wherein Y, L⁴, R¹, R², R⁴, and R⁵ are defined the same as item 1, and m and n are defined the same as item 33. PG¹, PG², and PG³ are each defined the same as the definition described in Manufacturing Method 1, PG⁶ is defined the same as the definition described in Manufacturing Method 15, and R⁶ represents a hydrogen atom or an optionally substituted C₁₋₆ alkyl group.

Step 17-1: Compound (II-4) can be manufactured by a so-called reductive amination reaction of compound (XXXI-3) with compound (XXX) in an inert solvent in the presence of a reducing agent. The reaction can be performed in the presence of a base, an acid, or other additives as needed. Compound (XXXI-3) can be used at 0.001 to 100 equivalents with respect to compound (XXX), which is preferably 1 to 10 equivalents. Specific exmaples of reducing agents include lithium aluminum hydride, sodium borohydride, sodium triacetoxyborohydride, sodium cyanoborohydride, diisobutylaluminium hydride, lithium tri(sec-butyl)borohydride, sodium tri(sec-butyl)borohydride, potassium tri(secbutyl)borohydride, borane-dimethylsulfide complex, boranetetrahydrofuran complex, lithium triethyl borohydride, ammonium formate, and the like. Alternatively, a hydrogenation reaction can also be performed using a metal catalyst such as palladium on carbon or palladium oxide instead of a reducing agent. A reducing agent can be used at 0.001 to 100 equivalents with respect to compound (XXX), which is preferably 0.5 to 10 equivalents. Specific examples of bases include organic bases such as triethylamine, diisopropylethylamine, and pyridine; inorganic bases such as potassium carbonate, sodium carbonate, cesium carbonate, potassium hydrogen carbonate, sodium hydrogen carbonate, potassium dihydrogen phosphate, dipotassium hydrogen phosphate, potassium phosphate, sodium dihydrogen phosphate, disodium hydrogen phosphate, sodium phosphate, potassium hydroxide, sodium hydroxide, and sodium hydride; metal alkoxides such as sodium methoxide and potassium tert-butoxide; and the like. A base can be used at 0.001 to 100 equivalents with respect to compound (XXX), which is preferably 0.5 to 10 equivalents. Specific examples of acids include organic acids such as acetic acid, trifluoroacetic acid, and methanesulfonic acid; inorganic acids such as hydrochloric acid and sulfuric acid, and the like. An acid can be used at 0.001 to 100 equivalents with respect to compound (XXX), which is preferably 0.5 to 10 equivalents. Examples of other additives include dehydrating agents such as tetramethyl orthosilicate and methyl orthoformate; zinc chloride, titanium tetrachloride, lanthanum sulfate, magnesium sulfate-p-pyridinium p-toluenesulfonate, magnesium bromide, indium chloride, zirconium chloride, magnesium triflate, ytterbium(III)triflate (wherein (III) indicates that ytterbium is trivalent), scandium triflate, alumina, copper sulfate, titanium isopropoxide, titanium tetraethoxide, and other Lewis acids. An additive can be used at 0.001 to 100 equivalents with respect to compound (XXX), which is preferably 0.5 to 10 equivalents. Specific examples of inert solvents include water, acetonitrile; halogenated hydrocarbons such as chloroform and dichloromethane; aromatic hydrocarbons such as benzene and toluene; ether solvents such as DME, THF, and 1,4-dioxane; alcohol solvents such as methanol, ethanol, and 2-propanol; aprotic polar solvents such as DMF and NMP; mixture solvents thereof; and the like. The reaction temperature is selected from the range of about -20°C to the boiling point of the solvent used.

### Manufacturing Method 18

Compound (XXIX) can be manufactured, for example, by the manufacturing method described below by using compound (VI) as a raw material,. 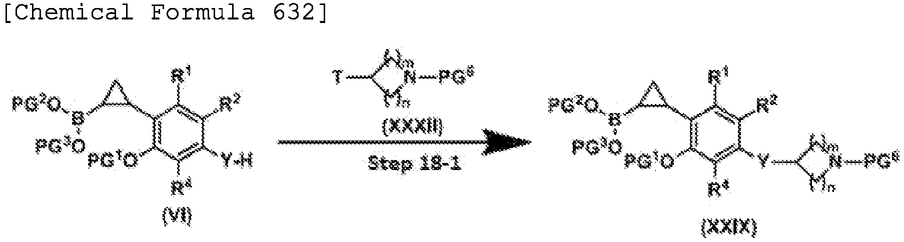 wherein Y, R¹, R², and R⁴ are defined the same as item 1, and m and n are defined the same as item 33. H of group -Y-H in formula (VI) is a hydrogen atom, PG¹, PG², and PG³ are each defined the same as the definition described in Manufacturing Method 1, T is defined the same as the definition described in Manufacturing Method 4, and PG⁶ is defined the same as the definition described in Manufacturing Method 15.

Step 18-1: Compound (XXIX) can be manufactured by a method in accordance with step 4-3 in Manufacturing Method 4 by using compound (VI) as a raw material.

### Manufacturing Method 19

Compound (XXIX) can also be manufactured, for example, by the manufacturing method described below by using compound (XXXIII) as a raw material. 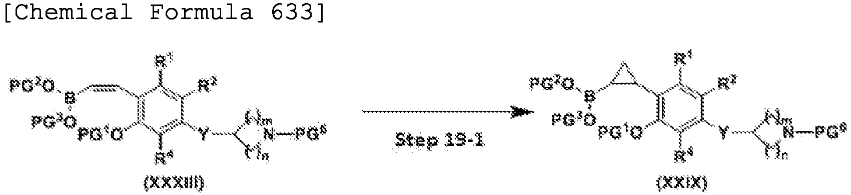 wherein Y, R¹, R², and R⁴ are defined the same as item 1, and m and n are defined the same as item 33. PG¹, PG², and PG³ are each defined the same as the definition described in Manufacturing Method 1, and PG⁶ is defined the same as the definition described in Manufacturing Method 15.

Step 19-1: Compound (XXIX) can be manufactured by a method in accordance with step 2-1 in Manufacturing Method 2 by using compound (XXXIII) as a raw material. Compound (XXXIII) can be manufactured, for example, in the same manner as Manufacturing Methods 20 to 22 described below or a known method from a corresponding raw material that can be purchased or prepared.

### Manufacturing Method 20

Compound (XXXIII) can be manufactured, for example, by the manufacturing method described below by using compound (XV) as a raw material. 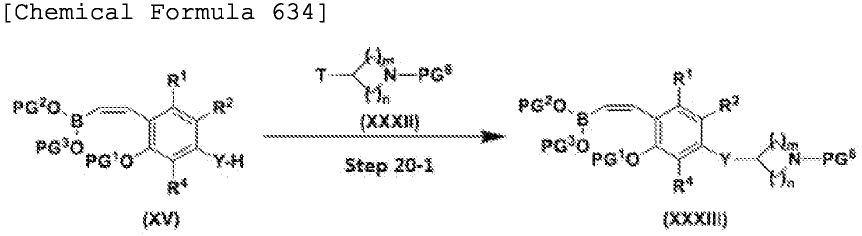 wherein Y, R¹, R², and R⁴ are defined the same as item 1, and m and n are defined the same as item 33. H of group -Y-H in formula (XV) is a hydrogen atom, PG¹, PG², and PG³ are each defined the same as the definition described in Manufacturing Method 1, T is defined the same as the definition described in Manufacturing Method 4, and PG⁶ is defined the same as the definition in Manufacturing Method 15.

Step 20-1: Compound (XXXIII) can be manufactured by a method in accordance with step 4-3 in Manufacturing Method 4 by using compound (XV) as a raw material.

### Manufacturing Method 21

Compound (XXXIII) can also be manufactured, for example, by the manufacturing method described below by using compound (XVI) as a raw material. 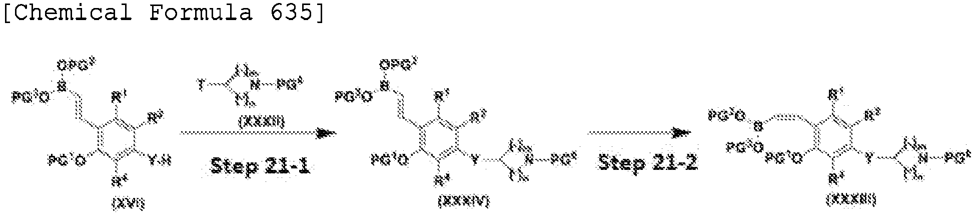 wherein Y, R¹, R², and R⁴ are defined the same as item 1, and m and n are defined the same as item 33. H of group -Y-H in formula (XVI) is a hydrogen atom, PG¹, PG², and PG³ are each defined the same as the definition described in Manufacturing Method 1, T is defined the same as the definition described in Manufacturing Method 4, and PG⁶ is defined the same as the definition described in Manufacturing Method 15.

Step 21-1: Compound (XXXIV) can be manufactured by a method in accordance with step 4-3 in Manufacturing Method 4 by using compound (XVI) as a raw material.

Step 21-2: Compound (XXXIII) can be manufactured by a method in accordance with step 5-2 in Manufacturing Method 5 by using compound (XXXIV) as a raw material.

### Manufacturing Method 22

Compound (XXXIII) can also be manufactured, for example, by the manufacturing method described below by using compound (XVIII) as a raw material. 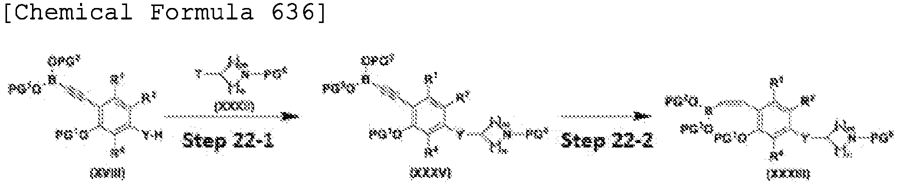 wherein Y, R¹, R², and R⁴ are defined the same as item 1, and m and n are defined the same as item 33. H of group -Y-H in formula (XVIII) is a hydrogen atom, PG¹, PG², and PG³ are each defined the same as the definition described in Manufacturing Method 1, T is defined the same as the definition described in Manufacturing Method 4, and PG⁶ is defined the same as the definition described in Manufacturing Method 15.

Step 22-1: Compound (XXXV) can be manufactured by a method in accordance with step 4-3 in Manufacturing Method 4 by using compound (XVIII) as a raw material.

Step 22-2: Compound (XXXIII) can be manufactured by a method in accordance with step 6-5 in Manufacturing Method 6 by using compound (XXXV) as a raw material.

### Manufacturing Method 23

A compound of formula (III) represented by formula (III-2) can be manufactured, for example, by the manufacturing method described below by using compound (XXXIII) as a raw material. 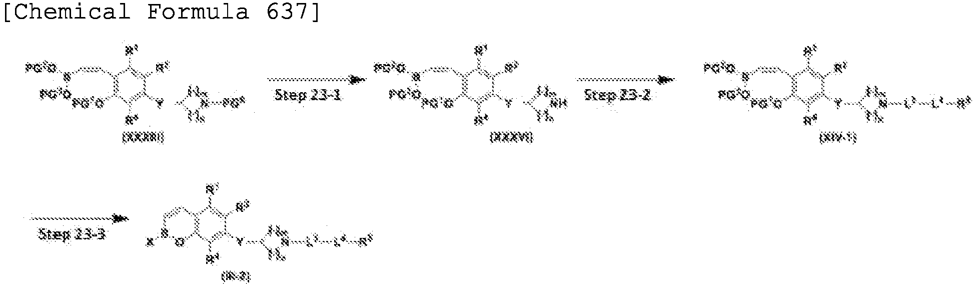 wherein Y, L³, L⁴, R¹, R², R⁴, and R⁵ are defined the same as item 1, and m and n are defined the same as item 33. PG¹, PG², and PG³ are each defined the same as the definition described in Manufacturing Method 1, T is defined the same as the definition described in Manufacturing Method 4, and PG⁶ is defined the same as the definition described in Manufacturing Method 15.

Step 23-1: Compound (XXXVI) can be manufactured by a method in accordance with step 15-1 in Manufacturing Method 15 by using compound (XXXIII) as a raw material.

Step 23-2: Compound (XIV-1) can be manufactured by a method in accordance with step 15-2 in Manufacturing Method 15, step 16-1 in Manufacturing Method 16, or step 17-1 in Manufacturing Method 17 by using compound (XXXVI) as a raw material.

Step 23-3: Compound (III-2) can be manufactured by a method in accordance with step 1-1, step 1-1-1, or step 1-1-2 in Manufacturing Method 1 (same as step 12-1 in Manufacturing Method 12) by using compound (XIV-1) as a raw material.

### Manufacturing Method 24

A compound represented by formula (III-2) can be manufactured, for example, by the manufacturing method described below by using compound (XXVIII) as a raw material. 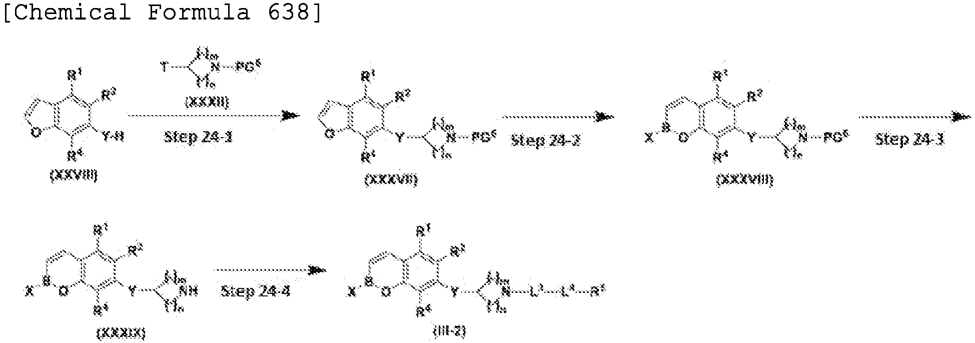 wherein Y, L³, L⁴, R¹, R², R⁴, and R⁵ are defined the same as item 1, and m and n are defined the same as item 33. H of group -Y-H in formula (XXVIII) is a hydrogen atom, T is defined the same as the definition described in Manufacturing Method 4, and PG⁶ is defined the same as the definition described in Manufacturing Method 15.

Step 24-1: Compound (XXXVII) can be manufactured by a method in accordance with step 4-3 in Manufacturing Method 4 by using compound (XXVIII) as a raw material.

Step 24-2: Compound (XXXVIII) can be manufactured by a method in accordance with step 13-6 in Manufacturing Method 13 by using compound (XXXVII) as a raw material.

Step 24-3: Compound (XXXIX) can be manufactured by a method in accordance with step 15-1 in Manufacturing Method 15 by using compound (XXXVIII) as a raw material.

Step 24-4: Compound (III-2) can be manufactured by a method in accordance with step 15-2 in Manufacturing Method 15, step 16-1 in Manufacturing Method 16, or step 17-1 in Manufacturing Method 17 by using compound (XXXIX) as a raw material.

### Manufacturing Method 25

Compound (XXXVII) can also be manufactured, for example, by the manufacturing method described below by using compound (XX) as a raw material. 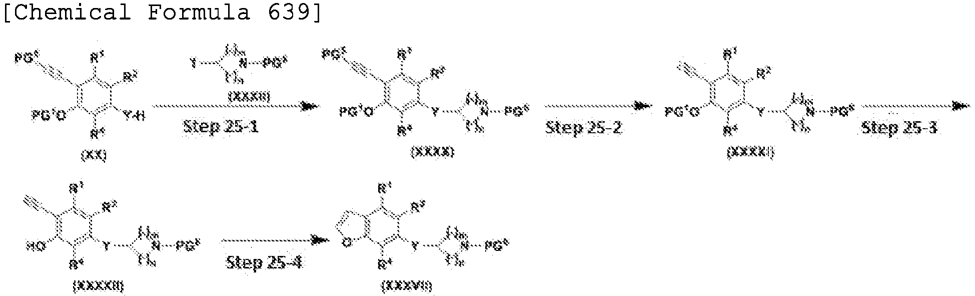 wherein Y, R¹, R², and R⁴ are defined the same as item 1, and m and n are defined the same as item 33. H of group -Y-H in formula (XX) is a hydrogen atom, PG¹ is defined the same as the definition described in Manufacturing Method 1, T is defined the same as the definition described in Manufacturing Method 4, PG⁵ is defined the same as the definition described in Manufacturing Method 6, and PG⁶ is defined the same as the definition described in Manufacturing Method 15.

Step 25-1: Compound (XXXX) can be manufactured by a method in accordance with step 4-3 in Manufacturing Method 4 by using compound (XX) as a raw material.

Step 25-2: Compound (XXXXI) can be manufactured by a method in accordance with step 6-2 in Manufacturing Method 6 by using compound (XXXX) as a raw material.

Step 25-3: Compound (XXXXII) can be manufactured by a method in accordance with step 4-2 in Manufacturing Method 4 by using compound (XXXXI) as a raw material.

Step 25-4: Compound (XXXVII) can be manufactured by a method in accordance with step 13-5 in Manufacturing Method 13 by using compound (XXXXII) as a raw material.

### Manufacturing Method 26

A compound represented by formula (4a) defined in item 19 (hereinafter, also referred to as compound (4a)) and a compound represented by formula (4b) (hereinafter, also referred to as compound (4b)) can be manufactured, for example, by the manufacturing method described below by using compound (II-1) or compound (III-1) as a raw material,. 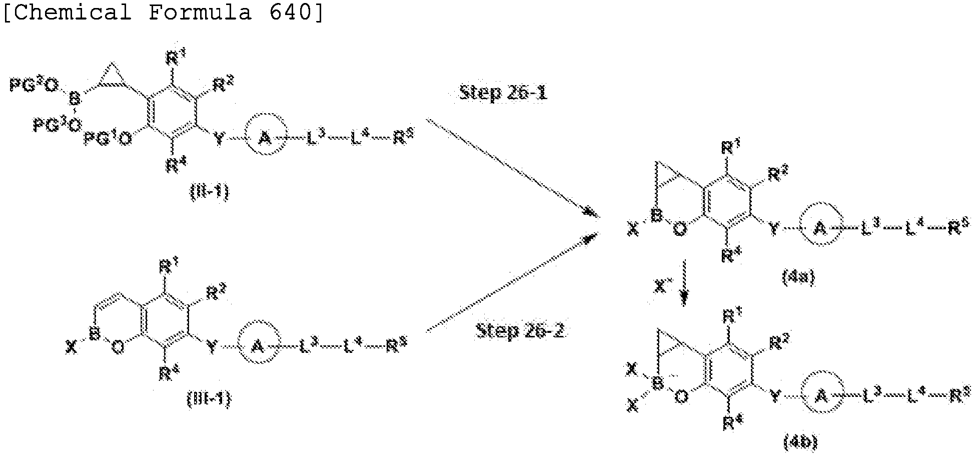 wherein A, X, Y, L³, L⁴, R¹, R², R⁴, and R⁵ are defined the same as any of items 1 to 19. PG¹, PG², and PG³ in formula (II-1) are each defined the same as the definition described in Manufacturing Method 1.

Step 26-1: Compound (4a) can be manufactured by a method in accordance with step 1-1, step 1-1-1, or step 1-1-2 in Manufacturing Method 1 by using compound (II-1) as a raw material. Compound (4b) can also be obtained by a method in accordance with Manufacturing Method 3, by using compound (4a) as a raw material or in a step of manufacturing compound (4a).

Step 26-2: Compound (4a) can be manufactured by a method in accordance with step 2-1 in Manufacturing Method 2 by using compound (III-1) as a raw material. In the same manner, compound (4b) can be obtained in accordance with Manufacturing Method 3, by using compound (4a) as a raw material or in a step of manufacturing compound (4a).

### Manufacturing Method 27

Compound (4a) and compound (4b) defined in item 19 represented by formula (4a-2) and formula (4b-2) described below can be manufactured, for example, by the manufacturing method described below by using compound (II-2) or compound (III-2) as a raw material. 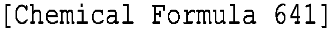 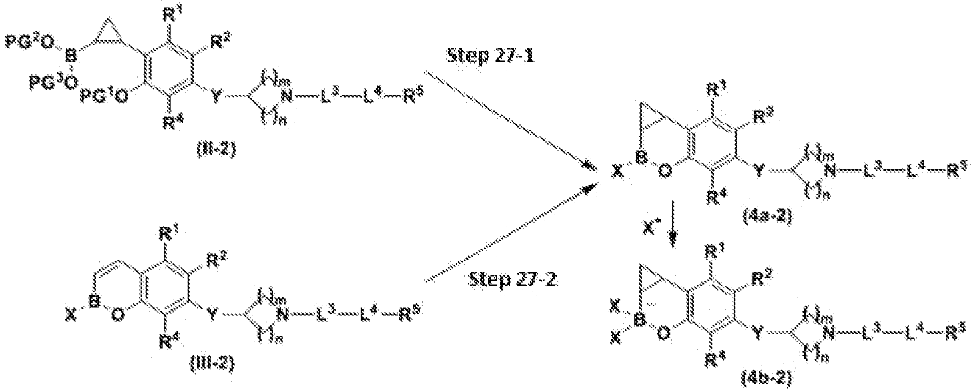 wherein X, Y, L³, L⁴, R¹, R², R⁴, and R⁵ are defined the same as any of items 1 to 19, and m and n are defined the same as item 33. PG¹, PG², and PG³ in formula (II-2) are each defined the same as the definition described in Manufacturing Method 1.

Step 27-1: Compound (4a-2) can be manufactured by a method in accordance with step 1-1, step 1-1-1, or step 1-1-2 in Manufacturing Method 1 by using compound (II-2) as a raw material. Compound (4b-2) can also be obtained by a method in accordance with Manufacturing Method 3, by using compound (4a-2) as a raw material or in a step of manufacturing compound (4a-2).

Step 27-2: Compound (4a-2) can be manufactured by a method in accordance with step 2-1 in Manufacturing Method 2 by using compound (III-2) as a raw material. In the same manner, compound (4b-2) can be obtained by a method in accordance with Manufacturing Method 3, by using compound (4a-2) as a raw material or in a step of manufacturing compound (4a-2).

### Manufacturing Method 28

Compound (4a) and compound (4b) defined in item 19 represented by formula (4a-3) and formula (4b-3) described below can be manufactured, for example, by the manufacturing method described below by using compound (II-3) as a raw material. 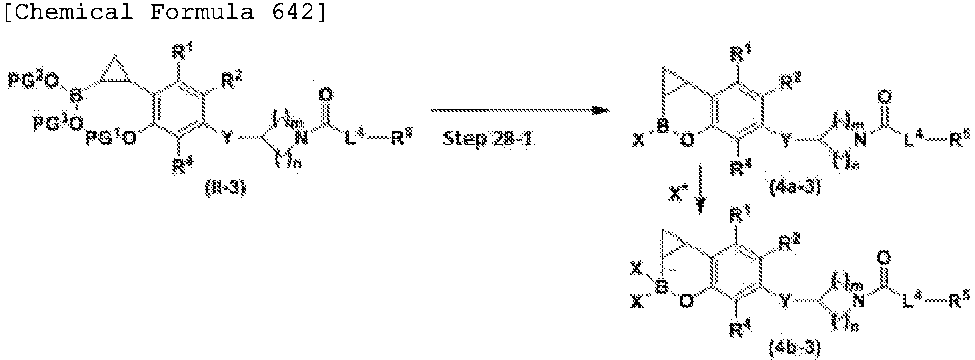 wherein X, Y, L⁴, R¹, R², R⁴, and R⁵ are defined the same as any of items 1 to 19, and m and n are defined the same as item 33. PG¹, PG², and PG³ in formula (II-3) are each defined the same as the definition described in Manufacturing Method 1.

Step 28-1: Compound (4a-3) can be manufactured by a method in accordance with step 1-1, step 1-1-1, or step 1-1-2 in Manufacturing Method 1 by using compound (II-3) as a raw material. Compound (4b-3) can be obtained by a method in accordance with Manufacturing Method 3, by using compound (4a-3) as a raw material or in a step of manufacturing compound (4a-3).

### Manufacturing Method 29

Compound (4a) and compound (4b) defined in item 19 represented by formula (4a-4) and formula (4b-4) described below can be manufactured, for example, by the manufacturing method described below by using compound (II-4) as a raw material. 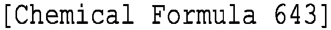 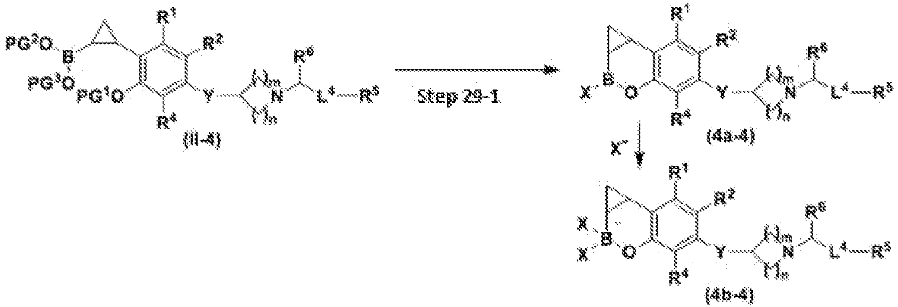 wherein X, Y, L⁴, R¹, R², R⁴, and R⁵ are defined the same as any of items 1 to 19, and m and n are defined the same as item 33. R⁶ is defined the same as the definition described in Manufacturing Method 17, and PG¹, PG², and PG³ in formula (II-4) are each defined the same as the definition described in Manufacturing Method 1.

Step 29-1: Compound (4a-4) can be manufactured by a method in accordance with step 1-1, step 1-1-1, or step 1-1-2 in Manufacturing Method 1 by using compound (II-4) as a raw material. Compound (4b-4) can be obtained by a method in accordance with Manufacturing Method 3, by using compound (4a-4) as a raw material or in a step of manufacturing compound (4a-4).

The intermediate and compound of interest in the manufacturing methods described above can be isolated and purified by subjecting them to a purification method that is commonly used in organic synthesis chemistry, e.g., neutralization, filtration, extraction, washing, drying, concentration, recrystallization, various chromatography, or the like. Each intermediate can also be subjected to the subsequent reaction without any particular purification.

Optically active forms of the compound of the invention can be manufactured by using an optically active starting material or intermediate, or by optically resolving a racemate of the final product or intermediate. Examples of optical resolution methods include physical separation methods using an optically active column and chemical separation methods such as fractional crystallization method. A diastereomer of the compound of the invention can be manufactured by, for example, a separation method such as column chromatography or fractional crystallization.

A pharmaceutically acceptable salt of a compound represented by formula (1a) or (1b) can be manufactured by, for example, mixing a compound represented by formula (1a) or formula (1b) with a pharmaceutically acceptable acid or base in a solvent such as water, methanol, ethanol, 2-propanol, ethyl acetate, or acetone, but the manufacturing method is not limited thereto.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described. The US patent publications corresponding to the aforementioned prior art documents, i.e., Patent Literatures 1 to 10 (Patent Literature 1: US 2014/0194382A1; US 2014/0194385A1; Patent Literature 2: US 2014/0194381A1; US 2014/0194384A1; Patent Literature 3: US 2017/0088561A1; Patent Literature 4: US 2017/0136047A1; Patent Literature 5: US 2018/051041A1; Patent Literature 6: US 2014/0171390A1; Patent Literature 7: US 2014/0194386A1; Patent Literature 8: US 2016/0024121A1; Patent Literature 9: US 2015/0361107A1; US 2015/0361108A1; Patent Literature 10: US 2018/0002351A1) are also incorporated herein by reference to the same extent that the entirety of each document is specifically described.

As described above, the present invention has been described while showing preferred embodiments to facilitate understanding. The present invention is described hereinafter based on Examples. The above descriptions and the following Examples are not provided to limit the present invention, but for the sole purpose of exemplification. Thus, the scope of the present invention is not limited to the embodiments and Examples specifically described herein and is limited only by the scope of claims.

### [Examples]

While the present invention is described more specifically with Reference Examples, Examples, and Test Examples hereinafter, the preset invention is not limited thereto.

Compounds were identified using proton nuclear magnetic resonance spectrum (¹H-NMR), liquid chromatography-mass spectrometry (LCMS), or the like. Tetramethylsilane was used as an internal standard for nuclear magnetic resonance spectrum.

For column chromatography in the Reference Examples and Examples, Yamazen Corporation's silica gel column, YMC's ODS-A column, YMC's YMC-Actus Triart C18, or YMC's YMC-Actus pro C18 were used. For TLC (silica gel plate) in purification using a thin layer chromatography (TLC), Silica gel 60F254 (Merck) was used, and for TLC (NH silica gel plate), TLC plate NH (Fuji Silysia) was used.

Various data described in the Reference Examples and Example were obtained with the following equipment.
NMR spectrum: 400 MHz or 270 MHz: JEOL JNM-AL series AL400 or JEOL EX270
LC-MS spectrum: Waters ACQUITY^{™} UltraPerformance LC

The compound names described in the Reference Examples and Examples were named using ACD/Name (ACD/Labs 12.0, Advanced Chemistry Development Inc.), which are not necessarily in accordance with the IUPAC nomenclature.

*1 and *2 appended to asymmetric carbon atoms in the structural formulas in the Reference Examples and the Examples indicate that the configuration of the carbon atom is not a racemate of a mixture of any ratio, but is substantially independent. If, for example, there are descriptions of structures of a compound (isomer 1) having a 3-membered ring (cyclopropane ring) or the three-membered ring further forming a condensed ring structure and having two *1 appended two asymmetric carbons constituting the three-membered ring (corresponding to two bridgehead position carbons in case of a condensed ring structure) and compound (isomer 2) similarly appended with two *2, this indicates that two bonds to three-membered ring methylene carbon from the two symmetric carbons in the same molecular structure have a configuration orientated toward the same direction in either a direction from the page surface to the backside or the front side (i.e., cis configuration), and indicates that isomer 1 and isomer 2 are different isomers. Specifically, this indicates that if one of isomer 1 and isomer 2 has a configuration in which the two bonds to a three-membered ring methylene carbon from two asymmetric carbons is oriented from the page surface to the back side, the other isomer is a configuration in which the bonds are oriented in the direction from the page surface to the front side, or the two isomers are enantiomers of each other with respect to the partial structure of the two asymmetric carbons. For example, the following table shows that the relationship between isomer 1 and isomer 2 is either case 1 or case 2 (the denotation describing a hydrogen atom (left side) and a denotation omitting a hydrogen atom (right side) for a stereochemical structure described in parallel in the following table are synonymous herein).

**[Table 2]**

| | Isomer 1 | isomer 2 |
|---|---|---|
| Examples of description of isomer | 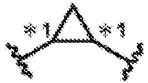 | 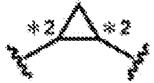 |
| Case 1 | 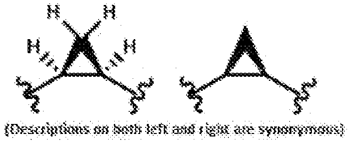 | 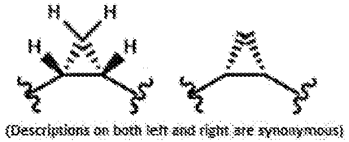 |
| Case 2 | 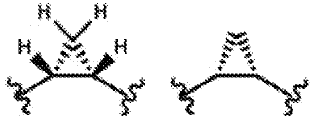 | 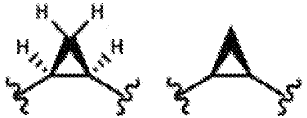 |

The measuring conditions (hereinafter, also referred to as the measurement methods) for a high performance liquid chromatography-mass spectrometry (LCMS) system are described below. The observed mass spectrometry value [MS(m/z)] is indicated by [M+1]⁺ (or denoted as [M+H]⁺), etc., and the time of retention at which the mass spectrometry value was observed is indicated by Rt (min). The measurement conditions A to I used for measurement are denoted in each actual measurement value. For example, "LCMS: [M+H]⁺/Rt = 620/1.32^{A}" expresses that measurement was taken under measurement condition A.

### Measurement Condition A

Measuring equipment: Waters ACQUITY^{™} UltraPerformance LC
Column: ACQUITY UPLC BEH C18 1.7 µm 2.1 × 30 mm column
Solvent: solution A: 0.05% HCOOH/H₂O, solution B: CH₃CN
Gradient condition:
   0.0 to 1.3 minutes; A/B = 99/1 to 5/95 (linear gradient)
   1.3 to 1.5 minutes; A/B = 99/1
Flow rate: 0.80 mL/min
UV: 220 nm, 254 nm
Column temperature: 40°C

### Measurement Condition B

Measuring equipment: Waters ACQUITY^{™} UltraPerformance LC
Column: ACQUITY UPLC BEH C18 1.7 µm 2.1 × 30 mm column
Solvent: solution A: 0.05% HCOOH/H₂O, solution B: CH₃CN
Gradient condition:
   0.0 to 1.3 minutes; A/B = 90/10 to 5/95 (linear gradient)
   1.3 to 1.5 minutes; A/B = 90/10
Flow rate: 0.80 mL/min
UV: 220 nm, 254 nm
Column temperature: 40°C

### Measurement Condition C

Measuring equipment: Waters ACQUITY^{™} UltraPerformance LC
Column: ACQUITY UPLC BEH C18 1.7 µm 2.1 × 30 mm column
Solvent: solution A: 0.05% HCOOH/H₂O, solution B: CH₃CN
Gradient condition:
   0.0 to 1.3 minutes; A/B = 60/40 to 5/95 (linear gradient)
   1.3 to 1.5 minutes; A/B = 60/40
Flow rate: 0.80 mL/min
UV: 220 nm, 254 nm
Column temperature: 40°C

### Measurement Condition D

Measuring equipment: Shimadzu LCMS-2020
Column: Phenomenex Kinetex 1.7 µm C18 (50 mm x 2.10 mm)
Solvent: solution A: 0.05% TFA/H₂O, solution B: CH₃CN
Gradient condition:
   0.0 to 1.9 minutes; A/B = 90/10 to 1/99 (linear gradient)
   1.91 to 3.00 minutes; A/B = 1/99
Flow rate: 0.50 mL/min
UV: 220 nm, 254 nm
Column temperature: 40°C

### Measurement Condition E

Measuring equipment: Shimadzu LCMS-2020
Column: Phenomenex Kinetex 1.7 µm C18 (50 mm x 2.10 mm)
Solvent: solution A: 0.05% TFA/H₂O, solution B: CH₃CN
Gradient condition:
   0.0 to 1.9 minutes; A/B = 40/60 to 1/99 (linear gradient)
   1.91 to 3.00 minutes; A/B = 1/99
Flow rate: 0.50 mL/min
UV: 220 nm, 254 nm
Column temperature: 40°C

### Measurement Condition F

Measuring equipment: Shimadzu LCMS-2020
Column: Phenomenex Kinetex 1.7 µm C18 (50 mm x 2.10 mm)
Solvent: solution A: 0.05% TFA/H₂O, solution B: CH₃CN
Gradient condition:
   0.0 to 1.9 minutes; A/B = 99/1 to 1/99 (linear gradient)
   1.91 to 3.00 minutes; A/B = 1/99
Flow rate: 0.50 mL/min
UV: 220 nm, 254 nm
Column temperature: 40°C

### Measurement Condition G

Measuring equipment: Waters AQUITY^{™} UPLC H-Class System
Column: ACQUITY UPLC HSS T3 1.8 µm 2.1 × 35 mm column
Solvent: solution A: 0.1% HCOOH/H₂O, solution B: 0.1% HCOOH/CH₃CN
Gradient condition:
   0.0 to 2.4 minutes; A/B = 90/10 to 0/100 (linear gradient)
   2.4 to 3.2 minutes; A/B = 0/100
Flow rate: 0.7 mL/min
UV: 190 to 800 nm
Column temperature: 40°C

### Measurement Condition H

Measuring equipment: Waters ACQUITY^{™} UltraPerformance LC
Column: ACQUITY UPLC BEH C18 1.7 µm 2.1 × 30 mm column
Solvent: solution A: 0.06% HCOOH/H₂O, solution B: 0.06% HCOOH/CH₃CN
Gradient condition:
   0.0 to 1.3 minutes; A/B = 98/2 to 4/96 (linear gradient)
   1.3 to 1.5 minutes; A/B = 98/2
Flow rate: 0.80 mL/min
UV: 220 nm, 254 nm
Column temperature: 40°C

### Measurement Condition I

Measuring equipment: Shimadzu LCMS-2020
Column: Phenomenex Kinetex 1.7 µm C18 (50 mm × 2.10 mm)
Solvent: solution A: 0.05% TFA/H₂O, solution B: CH₃CN
Gradient condition:
   0.0 to 1.9 minutes; A/B = 70/30 to 1/99 (linear gradient)
   1.91 to 3.00 minutes; A/B = 1/99
Flow rate: 0.50 mL/min
UV: 220, 254 nm
Column temperature: 40°C

Throughout the specification, the abbreviations described above and the following abbreviations are used, for example, in the Reference Examples, Examples, and Test Examples in some cases to simplify the description.
CDCl₃: deuterated chloroform
CD₃OD: deuterated methanol
s: singlet
d: doublet
t: triplet
q: quadruplet
m: multiplet
br: broad
dd: double doublet
J: coupling constant
Hz: Hertz
δ: chemical shift
min: minute
THF: tetrahydrofuran
DMF: N,N-dimethylformamide
Me: methyl
MeCN: acetonitrile
Boc or BOC: tert-butoxycarbonyl
tBu or ^{t}Bu or t-Bu: tert-butyl
Cbz: benzyloxycarbonyl
HCl: hydrogen chloride; represents hydrochloride especially when denoted in a structural formula of a basic compound such as amine
LED: Light Emitting Diode
MEPM: meropenem
MIC: minimum inhibitory concentration
HPLC: high performance liquid chromatography

Reference Example 1: tert-butyl 6-[(1-{[(tert-butoxycarbonyl)amino](1H-imidazol-4-yl)acetyl}azetidin-3-yl oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (derived from diastereomer 1-5-2; diastereo mixture)

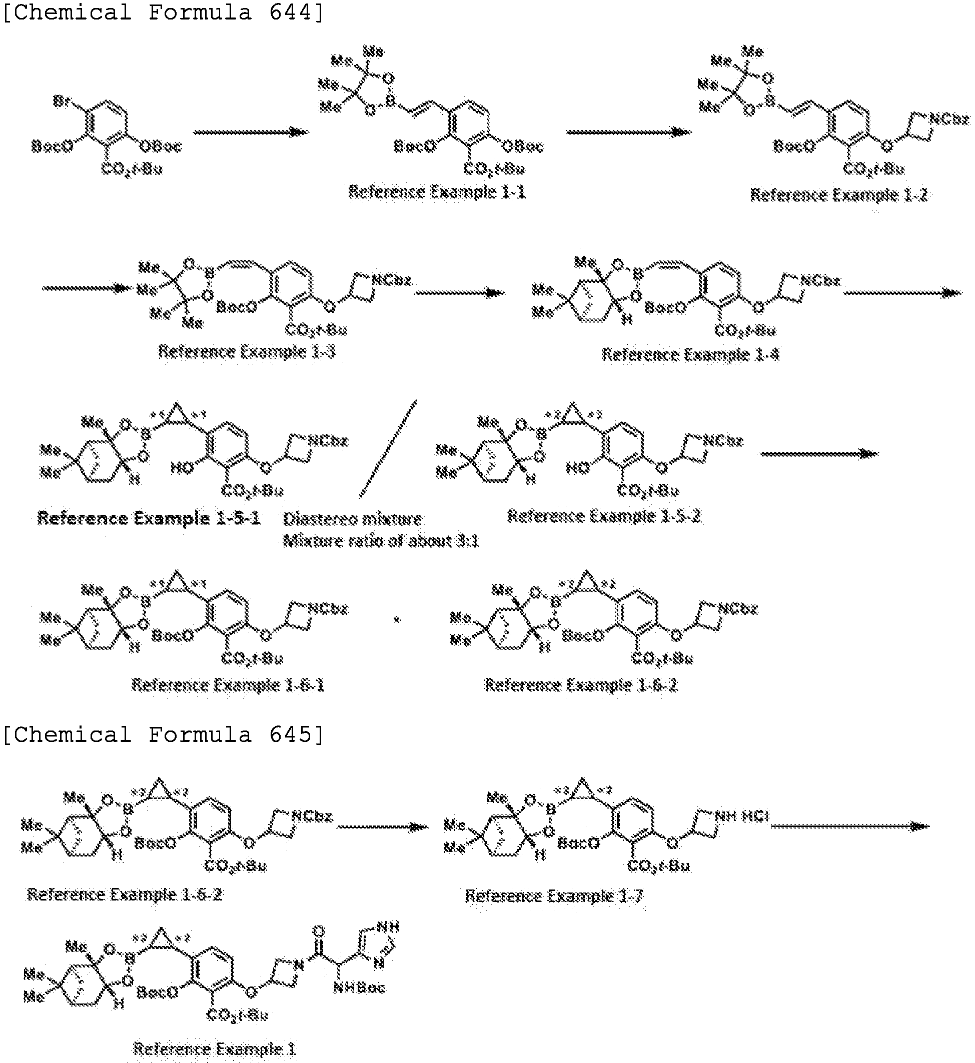

Reference Example 1-1: tert-butyl 2,6-bis[(tert-butoxycarbonyl)oxyl-3-[(E)-2-(4,4,5,5-tetramethyl-1,3,2-dioxaborolan-2-yl)ethenyl]benzoate

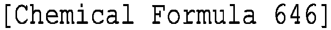

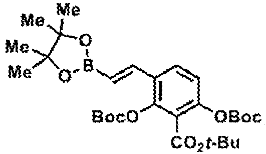

Triethylamine (6.10 mL, 43.7 mmol), 4,4,5,5-tetramethyl-2-vinyl-1,3,2-dioxaborolane (4.45 mL, 26.2 mmol), and bis(tri-tert-butylphosphine)palladium(0) (1.14 g, 2.22 mmol) were added to a toluene (87 mL) solution of tert-butyl 3-bromo-2,6-bis{(tert-butoxycarbonyl)oxy}benzoate (10.7 g, 21.9 mmol). The reaction mixture was stirred for 4 hours at 80°C. The reaction mixture was cooled to room temperature, and then filtered through celite to concentrate the filtrate. The resulting residue was purified by silica gel column chromatography to obtain the title compound (7.37 g).

¹H-NMR (400 MHZ, CDCl₃) δ: 7.61 (1H, d, J = 8.5 Hz), 7.36 (1H, d, J = 18.3 Hz), 7.08 (1H, d, J = 8.5 Hz), 6.11 (1H, d, J = 18.7 Hz), 1.54 (9H, s), 1.51 (9H, s), 1.51 (9H, s), 1.25 (12H, s).

Reference Example 1-2: benzyl 3-{2-(tert-butoxycarbonyl)-3-[(tert-butoxycarbonyl)oxy]-4-[(E)-2-(4,4,5,5-tetramethyl-1,3,2-dioxaborolan-2-yl)ethenyl]phenoxy}azetidine-1-carboxylate

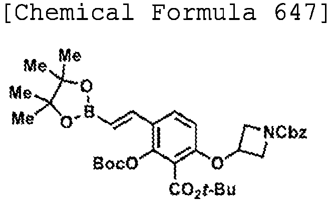

Pyrrolidine (1.03 mL, 12.5 mmol) was added to a THF (33 mL) solution of the compound of Reference Example 1-1 (7.37 g, 13.1 mmol), and the reaction mixture was stirred for 1 hour at room temperature. 1-N-Cbz-3-hydroxyazetidine (3.26 g, 15.7 mmol) was added to the reaction mixture, which is referred to as mixture A hereinafter.

Tri-n-butylphosphine (8.18 mL, 32.8 mmol) was added to a toluene (65.5 mL) solution of N,N,N',N'tetramethylazodicarboxamide (6.71 g, 39.0 mmol) while cooling with ice. The reaction mixture was directly stirred for 10 minutes while cooling with ice. This is referred to as mixture B hereinafter.

Mixture A was added to mixture B while cooling with ice, and the newly produced reaction mixture was stirred for 2.5 hours at 65°C. The reaction mixture was cooled to room temperature, then the precipitate was filtered out, and the filtrate was concentrated. The resulting residue was purified by silica gel column chromatography to obtain the title compound (6.34 g).

¹H-NMR (400 MHZ, CDCl₃) δ: 7.51 (1H, d, J = 8.7 Hz), 7.34-7.26 (7H, m), 6.41 (1H, d, J = 8.7 Hz), 6.02 (1H, d, J = 18.3 Hz), 5.09 (2H, s), 4.93-4.91 (1H, m), 4.35 (2H, dd, J = 10.5, 6.4 Hz), 4.10-4.05 (2H, m), 1.54 (9H, s), 1.51 (9H, s), 1.25 (13H, s).

Reference Example 1-3: benzyl 3-{2-(tert-butoxycarbonyl)-3-[(tert-butoxycarbonyl)oxy]-4-[(Z)-2-(4,4,5,5-tetramethyl-1,3,2-dioxaborolan-2-yl)ethenyl]phenoxy}azetidine-1-carboxylate

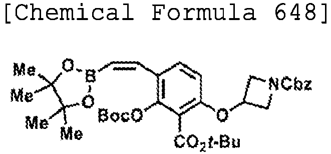

Tris(2-phenylpyridinato)iridium(III) (5.5 mg, 0.0084 mmol) was added to an acetonitrile (0.5 mL) solution of the compound of Reference Example 1-2 (66 mg, 0.101 mmol), and the reaction mixture was stirred for 3.5 hours at room temperature under irradiation of a blue LED light (450 nm) under a nitrogen atmosphere. The reaction mixture was concentrated. The resulting residue was purified by silica gel column chromatography to obtain the title compound (35 mg).

¹H-NMR (400 MHZ, CDCl₃) δ: 7.55 (1H, d, J = 8.5 Hz), 7.34-7.29 (5H, m), 7.05 (1H, d, J = 14.6 Hz), 6.36 (1H, d, J = 8.5 Hz), 5.63 (1H, d, J = 14.6 Hz), 5.09 (2H, s), 4.93 (1H, t, J = 6.4 Hz), 4.35 (2H, dd, J = 10.1, 7.0 Hz), 4.09 (2H, dd, J = 10.4, 4.3 Hz), 1.54 (9H, s), 1.48 (9H, s), 1.22 (12H, s).

Reference Example 1-4: benzyl 3-[2-(tert-butoxycarbonyl)-3-[(tert-butoxycarbonyl)oxy]-4-{(Z)-2-[(3aS,4S,6S,7aR)-3a,5,5-trimethylhexahydro-2H-4,6-methano-1,3,2-benzodioxaborol-2-yl]ethenyl}phenoxy]azetidine-1-carboxylate

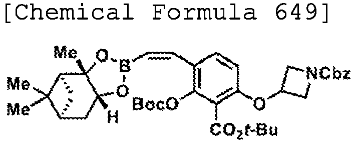

(1S,2S,3R,5R)-(+)-pinanediol (2.63 g, 15.5 mmol) was added to a THF (16.1 mL) solution of the compound of Reference Example 1-3 (2.52 g, 3.87 mmol), and the reaction mixture was stirred for 48 hours at room temperature. The reaction mixture was concentrated. The resulting residue was purified by silica gel column chromatography to obtain the title compound (1.76 g).

¹H-NMR (400 MHZ, CDCl₃) δ: 7.56 (1H, d, J = 8.7 Hz), 7.35-7.30 (5H, m), 7.08 (1H, d, J = 14.6 Hz), 6.36 (1H, d, J = 8.7 Hz), 5.67 (1H, d, J = 14.6 Hz), 5.09 (2H, s), 4.95-4.91 (1H, m), 4.35 (2H, dd, J = 10.1, 6.4 Hz), 4.24 (1H, dd, J = 8.9, 2.1 Hz), 4.10-4.06 (2H, m), 2.29-2.24 (1H, m), 2.19-2.14 (1H, m), 2.00 (1H, t, J = 5.5 Hz), 1.89-1.85 (1H, m), 1.82-1.78 (1H, m), 1.54 (9H, s), 1.48 (9H, s), 1.35 (3H, s), 1.26 (3H, s), 1.19 (1H, d, J = 11.0 Hz), 0.81 (3H, s) .

Reference Example 1-5: benzyl 3-[2-(tert-butoxycarbonyl)-3-hydroxy-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate (diastereo mixture of diastereomer 1-5-1 and diastereomer 1-5-2)

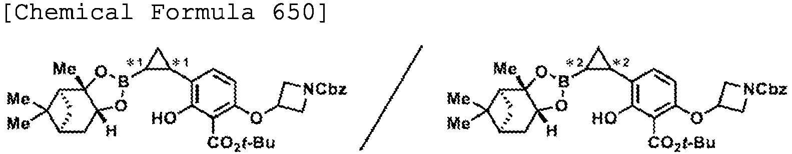

A hexane (20.5 mL) solution of diethylzinc (20.5 mmol) (concentration: 1 mol/L) was dissolved in dichloromethane (14.7 mL), and diiodomethane (2.5 mL, 31.0 mmol) was slowly added at - 78°C. The reaction mixture was stirred for 5 minutes while cooling with ice, then cooled again to -78°C. A dichloromethane (6 mL) solution of the compound of Reference Example 1-4 (1.76 g, 2.49 mmol) was added dropwise. The reaction mixture was stirred for 10 minutes at -78°C, then warmed to room temperature, and stirred for 2.5 hours at room temperature. An aqueous saturated ammonium chloride solution was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography to obtain the title compound (847 mg) as a diastereo mixture of diastereomer 1-5-1 and diastereomer 1-5-2. The ratio of mixture of both isomers (1-5-1:1-5-2) was about 3:1 (according to ¹H-NMR integration ratio).

Reference Example 1-5-1: benzyl 3-[2-(tert-butoxycarbonyl)-3-hydroxy-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate (diastereomer 1-5-1)

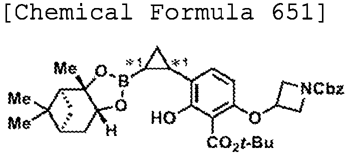

¹H-NMR (400 MHZ, CDCl₃) δ: 11.71 (1H, s), 7.33-7.29 (5H, m), 7.12 (1H, d, J = 8.6 Hz), 5.89 (1H, d, J = 8.6 Hz), 5.10 (2H, s), 4.88-4.85 (1H, m), 4.36-4.30 (2H, m), 4.10-4.07 (2H, m), 4.01-3.97 (2H, m), 2.30-2.23 (1H, m), 2.06-1.98 (1H, m), 1.87 (1H, t, J = 5.8 Hz), 1.75-1.73 (1H, m), 1.66-1.61 (1H, m), 1.46 (9H, s), 1.19 (3H, s), 1.10 (3H, s), 1.09-1.05 (2H, m), 0.79 (1H, d, J = 11.0 Hz), 0.70 (3H, s), 0.52-0.45 (1H, m).

Reference Example 1-5-2: benzyl 3-[2-(tert-butoxycarbonyl)-3-hydroxy-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate(diastereomer 1-5-2)

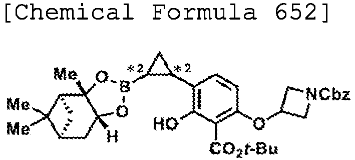

¹H-NMR (400 MHZ, CDCl₃) δ: 11.67 (1H, s), 7.33-7.29 (5H, m), 7.09 (1H, d, J = 8.6 Hz), 5.85 (1H, d, J = 8.6 Hz), 5.11 (2H, s), 4.88-4.85 (1H, m), 4.36-4.30 (2H, m), 4.08-4.04 (2H, m), 4.01-3.97 (1H, m), 3.95-3.90 (1H, m), 2.30-2.23 (1H, m), 2.20-2.13 (1H, m), 1.87 (1H, t, J = 5.8 Hz), 1.75-1.73 (1H, m), 1.66-1.61 (1H, m), 1.46 (9H, s), 1.19 (3H, s), 1.10 (3H, s), 1.09-1.05 (2H, m), 0.79 (1H, d, J = 11.0 Hz), 0.71 (3H, s), 0.51-0.44 (1H, m).

Reference Example 1-6-1: benzyl 3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate (diastereomer 1-6-1), and
Reference Example 1-6-2: benzyl 3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate (diastereomer 1-6-2)

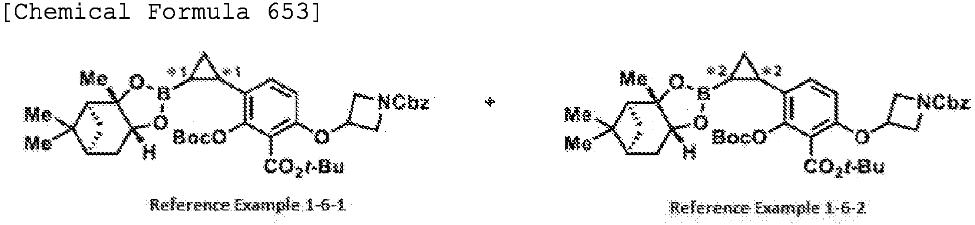

Di-tert-butyl dicarbonate (0.637 mL, 2.74 mmol), triethylamine (0.382 mL, 2.74 mmol), and 4-dimethylaminopyridine (20.2 mg, 0.165 mmol) were added to a dichloromethane (5 mL) solution of the compound of Reference Example 1-5 (diastereo mixture) (847 mg, 1.37 mmol). The reaction mixture was stirred for 48 hours at room temperature. The reaction mixture was concentrated, the residue was dissolved in dichloromethane (5 mL), and di-tert-butyl dicarbonate (2.25 g, 10.3 mmol) and 4-dimethylaminopyridine (0.3 g, 2.46 mmol) were added. The reaction mixture was stirred for 4 hours at room temperature and 3 hours at 40°C. The reaction mixture was concentrated. The resulting residue was purified by silica gel column chromatography to separate and obtain the compound of Reference Example 1-6-1 (identified by TLC (silica gel plate) Rf value: 0.46 (hexane/ethyl acetate = 2/1)) (285 mg) and the compound of Reference Example 1-6-2 (identified by TLC Rf value: 0.52 under the same condition as above) (95.3 mg) as different diastereomer 1-6-1 and diastereomer 1-6-2, respectively.

Reference Example 1-6-1: benzyl 3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate (diastereomer 1-6-1)

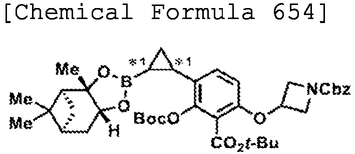

LCMS: [M-Boc]⁺/Rt = 617.6/1.433 min^{B}

¹H-NMR (400 MHZ, CDCl₃) δ: 7.34-7.28 (5H, m), 7.17 (1H, d, J = 8.5 Hz), 6.33 (1H, d, J = 8.5 Hz), 5.08 (2H, s), 4.90-4.85 (1H, m), 4.33-4.28 (2H, m), 4.07-3.99 (3H, m), 2.15-2.04 (3H, m), 1.85 (1H, t, J = 5.5 Hz), 1.76-1.72 (1H, m), 1.52 (9H, s), 1.51 (9H, s), 1.34-1.29 (1H, m), 1.19 (3H, s), 1.13-1.01 (2H, m), 1.06 (3H, s), 0.99 (1H, d, J = 11.0 Hz), 0.70 (3H, s), 0.39 (1H, td, J = 9.6, 6.9 Hz).

Reference Example 1-6-2: benzyl 3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidine-1-carboxylate (diastereomer 1-6-2)

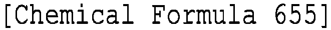

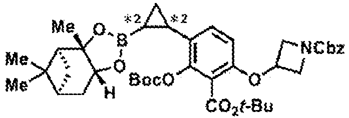

¹H-NMR (400 MHZ, CDCl₃) δ: 7.33-7.28 (5H, m), 7.14 (1H, d, J = 8.5 Hz), 6.31 (1H, d, J = 8.5 Hz), 5.09 (2H, s), 4.91-4.86 (1H, m), 4.35-4.28 (2H, m), 4.13-3.99 (2H, m), 3.93 (1H, dd, J = 8.9, 2.1 Hz), 2.14-2.07 (1H, m), 1.95-1.90 (1H, m), 1.81 (2H, t, J = 5.5 Hz), 1.76-1.73 (1H, m), 1.65 (1H, d, J = 16.5 Hz), 1.53 (9H, s), 1.52 (9H, s), 1.18 (3H, s), 1.12-0.97 (2H, m), 1.07 (3H, s), 0.80 (1H, d, J = 11.0 Hz), 0.71 (3H, s), 0.42-0.33 (1H, m).

Reference Example 1-7: tert-butyl 6-[(azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate hydrochloride (diastereomer derived from diastereomer 1-5-2)

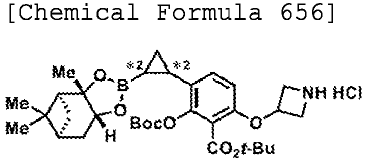

Palladium/carbon (Pd: 10%, wetted with ca. 55% water, 37.8 mg) and an aqueous 1 mol/L hydrochloric acid solution (0.133 mL, 0.133 mmol) were added to a methanol (3 mL) solution of the compound of Reference Example 1-6-2 (95.3 mg, 0.133 mmol), and the reaction mixture was stirred at room temperature for 1.5 hours under a hydrogen atmosphere. The reaction mixture was filtered through celite, and the filtrate was concentrated. The resulting residue was purified by silica gel column chromatography to obtain the title compound (82 mg).

LCMS: [M+H]⁺/Rt = 584.5/1.099 min^{B}

Reference Example 1: tert-butyl 6-[(1-{[(tert-butoxycarbonyl)amino](lH-imidazol-4-yl)acetyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (derived from diastereomer 1-5-2; diastereo mixture)

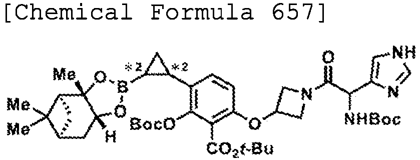

1-hydroxybenzotriazole (44.7 mg, 0.331 mmol), triethylamine (0.0461 mL, 0.331 mmol), and 1-(3-dimethylaminopropyl)-3-ethylcarbodiimide hydrochloride (63.4 mg, 0.331 mmol) were added to a DMF (2 mL) solution of [(tert-butoxycarbonyl)amino]-2-(1H-imidazol-4-yl)acetate (racemate) (50.6 mg, 0.210 mmol) while cooling with ice. The reaction mixture was stirred for 25 minutes while cooling with ice, then the compound of Reference Example 1-7 (82 mg, 0.132 mmol), triethylamine (0.02 mL), and 4-dimethylaminopyridine (4.8 mg, 0.039 mmol) were added. The reaction mixture was stirred for 16 hours at room temperature, then an aqueous saturated sodium hydrogen carbonate solution was added. The reaction mixture was extracted with ethyl acetate. The organic layer was washed with saturated saline, then dried with anhydrous sodium sulfate, and filtered, and the filtrate was concentrated. The resulting residue was purified by silica gel column chromatography to obtain the title compound (73.7 mg) as a diastereo mixture comprising two types of diastereomers due to the reagent used, i.e., [(tert-butoxycarbonyl)amino]-2-(1H-imidazol-4-yl) acetate, being a racemate and having an asymmetric center derived from diastereomer 1-5-2.

LCMS: [M+H]⁺/Rt = 807.6/1.228 min^{A}

Reference Example 2: tert-butyl 6-[(1-{[(tert-butoxycarbonyl)amino](1H-imidazol-4-yl)acetyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (derived from diastereomer 1-5-1; diastereo mixture)

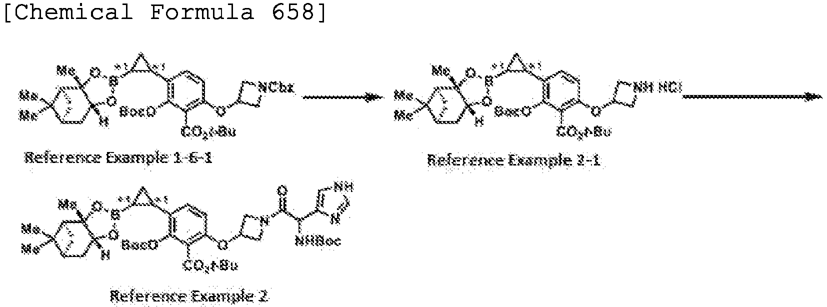

Reference Example 2-1: tert-butyl 6-[(azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate hydrochloride (diastereomer derived from diastereomer 1-5-1)

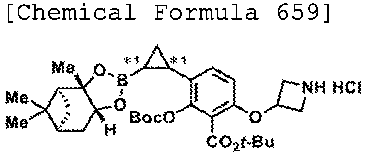

The compound of Reference Example 1-6-1 (101 mg) was used as a raw material, and a reaction and post-processing were performed in accordance with the same method as the method described in Reference Example 1-7 to obtain the title compound (81.8 mg).

LCMS: [M+H]⁺/Rt = 584.6/1.095 min^{B}

Reference Example 2: tert-butyl 6-[(1-{[(tert-butoxycarbonyl)amino](1H-imidazol-4-yl)acetyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (derived from diastereomer 1-5-1; diastereo mixture)

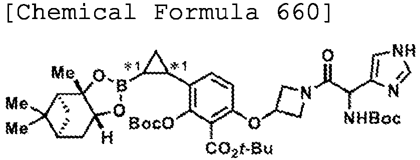

The compound of Reference Example 2-1 (81.8 mg) was used as a raw material, and a reaction and post-processing were performed in accordance with the same method as the method described in Reference Example 1 to obtain the title compound (91.1 mg) as a diastereo mixture comprising two types of diastereomers due to the reagent used, i.e., [(tert-butoxycarbonyl)amino]-2-(1H-imidazol-4-yl) acetate, being a racemate and having an asymmetric center derived from diastereomer 1-5-1.

LCMS: [M+H]⁺/Rt = 807.7/1.261 min^{A}

Reference Example 3: (2R)-2-[(tert-butoxycarbonyl)amino]-3-{3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidin-l-yl}-2-methylpropanoate (diastereomer derived from diastereomer 1-5-1)

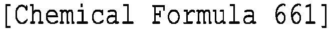

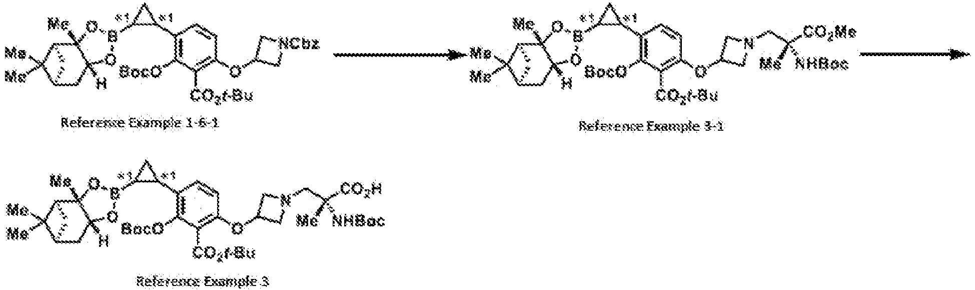

Reference Example 3-1: tert-butyl 6-[(1-{(2R)-2-[(tert-butoxycarbonyl)amino]-3-methoxy-2-methyl-3-oxopropyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (diastereomer derived from diastereomer 1-5-1)

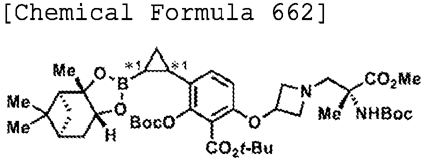

Palladium/carbon (Pd content: 10%, wetted with ca. 55% water, 36.7 mg) was added to a methanol (3 mL) solution of the compound of Reference Example 1-6-1 (108.5 mg, 0.151 mmol), and the reaction mixture was stirred for 1 hour under a hydrogen atmosphere at room temperature. The reaction mixture was filtered through celite, and the filtrate was concentrated. The resulting residue was dissolved in dichloromethane (2.5 mL), and methyl (2R)-2-[(tert-butoxycarbonyl)amino]-2-methyl-3-oxopropanoate (110 mg, 0.476 mmol), copper sulfate (13.1 mg, 0.082 mmol), and acetic acid (0.020 mL, 0.345 mmol) were added. The reaction mixture was stirred for 1 hour at room temperature. The reaction mixture was added with sodium triacetoxyborohydride (112 mg, 0.529 mmol) and stirred for 1 hour at room temperature. An aqueous saturated sodium hydrogen carbonate solution was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography to obtain the title compound (102.7 mg).

LCMS: [M+H]⁺/Rt = 799.45/1.154 min^{B}

Reference Example 3: (2R)-2-[(tert-butoxycarbonyl)amino]-3-{3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidin-1-yl}-2-methylpropanoate (diastereomer derived from diastereomer 1-5-1)

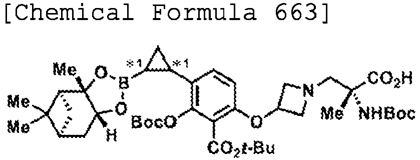

An aqueous 1 mol/L lithium hydroxide solution (0.77 mL, 0.77 mmol) was added to a THF (0.74 mL)-water (0.33 mL) mixture of the compound of Reference Example 3-1 (102.7 mg, 0.129 mmol) while cooling with ice, and the reaction mixture was stirred for 40 hours at room temperature. An aqueous 2 mol/L hydrochloric acid solution was added to the reaction mixture to achieve a pH near 4.0, and the reaction mixture was extracted with ethyl acetate. The organic layer was washed with saturated saline, dried with anhydrous sodium sulfate, and filtered, and the filtrate was concentrated to obtain the title compound (21.1 mg).

¹H-NMR (400 MHZ, CDCl₃) δ: 7.16 (1H, d, J = 8.5 Hz), 6.41 (1H, d, J = 8.5 Hz), 6.34 (1H, br s), 4.96-4.91 (1H, m), 4.86-4.79 (1H, m), 4.72-4.65 (1H, m), 4.02-3.90 (2H, m), 3.82 (1H, d, J = 12.2 Hz), 3.73-3.67 (1H, m), 3.57 (1H, d, J = 12.2 Hz), 2.12-2.03 (4H, m), 1.84 (1H, t, J = 5.5 Hz), 1.75-1.72 (1H, m), 1.53 (9H, s), 1.51 (9H, s), 1.40 (9H, s), 1.33-1.27 (1H, m), 1.20 (3H, d, J = 2.4 Hz), 1.11-1.02 (4H, m), 1.05 (3H, s), 1.00 (1H, d, J = 11.0 Hz), 0.71 (3H, s), 0.37 (1H, td, J = 9.6, 6.9 Hz).

Reference Example 4: tert-butyl 6-[(1-{[(tert-butoxycarbonyl)amino](1H-imidazol-4-yl)propanoyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (derived from diastereomer 1-5-2; diastereo mixture)

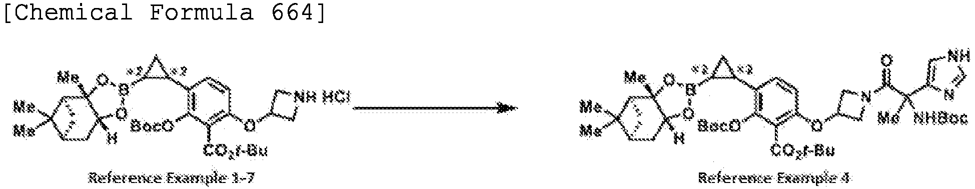

The compound of Reference Example 1-7 (25.7 mg) was used as a raw material, and [(tert-butoxycarbonyl)amino]-2-(1H-imidazol-4-yl)propionate (racemate) was used instead of [(tert-butoxycarbonyl)amino]-2-(1H-imidazol-4-yl)acetate (racemate), and a reaction and post-processing were performed in accordance with the same method as the method described in Reference Example 1 to obtain the title compound (13.8 mg) as a diastereo mixture comprising two types of diastereomers due to the reagent used, i.e., [(tert-butoxycarbonyl)amino]-2-(1H-imidazol-4-yl) propionate, being a racemate and having an asymmetric center derived from diastereomer 1-5-2.

LCMS: [M+H]⁺/Rt = 821.63/1.295 min^{A}

Reference Example 5: tert-butyl 6-[(1-{[(tert-butoxycarbonyl)amino](1H-imidazol-4-yl)propanoyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate (derived from diastereomer 1-5-1; diastereo mixture)

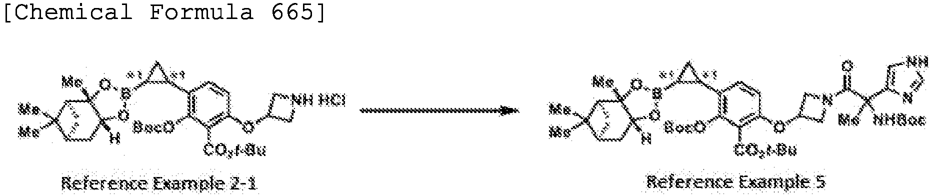

The compound of Reference Example 2-1 (27 mg) was used as a raw material, and [(tert-butoxycarbonyl)amino]-2-(1H-imidazol-4-yl)propionate (racemate) was used instead of [(tert-butoxycarbonyl)amino]-2-(1H-imidazol-4-yl) acetate (racemate), and a reaction and post-processing were performed in accordance with the same method as the method described in Reference Example 1 to obtain the title compound (11.8 mg) as a diastereo mixture comprising two types of diastereomers due to the reagent used, i.e., [(tert-butoxycarbonyl)amino]-2-(1H-imidazol-4-yl) propionate, being a racemate and having an asymmetric center derived from diastereomer 1-5-1.

LCMS: [M+H]⁺/Rt = 821.54/1.244 min^{A}

For example, the compounds of Reference Examples 1, 2, 4, and 5 that are diastereo mixtures can be optically resolved by chiral chromatography under the following condition. Accordingly, intermediates of Reference Examples can be further fractionated and identified to create and identify any of each diastereomer for the diastereo mixture of the compounds of the Examples described below.

Column: CHIRALPAK IG 20 mmϕ x 250 mm (Daicel Corporation)
Mobile phase: diethylamine/ethyl acetate (diethylamine: 0.1%)

Reference Example (R)-6: tert-butyl 6-({1-[(2R)-2-[(tert-butoxycarbonyl)amino]-2-(1H-imidazol-4-yl)acetyl]azetidin-3-yl}oxy)-2-[(tert-butoxycarbonyl)oxyl-3-{(2-[(3aS,4S,6S)-3a,5,5-trimethylhexahydro-2H-4,6-methano-1,3,2-benzodioxaborol-2-yl]cyclopropyl}benzoate
Reference Example (S)-6: tert-butyl 6-({1-[(2S)-2-[(tert-butoxycarbonyl)amino]-2-(1H-imidazol-4-yl)acetyl]azetidin-3-yl}oxy)-2-[(tert-butoxycarbonyl)oxy]-3-{(2-[(3aS,4S,6S)-3a,5,5-trimethylhexahydro-2H-4,6-methano-1,3,2-benzodioxaborol-2-yl]cyclopropyl}benzoate

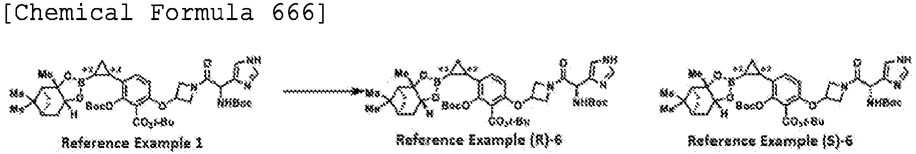

The compound of Reference Example 1 (amount loaded per injection: 6.17 mg) was dissolved in 0.300 mL of ethyl acetate. Isomers were obtained by optical resolution through chiral chromatography under the following conditions.
Column: CHIRALPAK IC 20 mmϕ x 250 mm (Daicel Corporation)
Mobile phase: hexane/ethanol ( 1:1)
Flow rate: 10 mL/min
Temperature: 40°C
Column retention times for both optical isomers were as follows.
Reference Example (R)-6: 9.25 min
Reference Example (S)-6: 13.04 min

Reference Example 7: tert-butyl 2-[(tert-butoxycarbonyl)oxy]-6-({1-[(1H-imidazol-4-yl)acetyl]azetidin-3-yl}oxy)-3-{2-[(3aS,4S,6S)-3a,5,5-trimethylhexahydro-2H-4,6-methano-1,3,2-benzodioxaborol-2-yl]cyclopropyl}benzoate

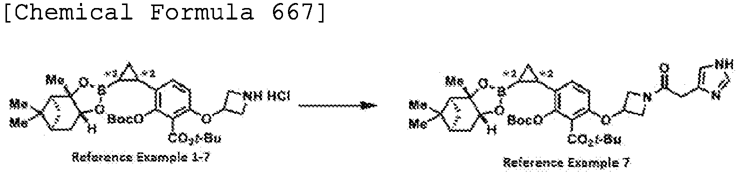

N,N-diisopropylethylamine (0.199 mL, 0.684 mmol) was added to a THF solution (0.9 mL) of the compound of Reference Example 1-7 (106 mg, 0.171 mmol), and 2-(1H-imidazol-4-yl)acetic acid hydrochloride (36 mg, 0.222 mmol) and pyBOP (133 mg, 0.256 mmol) were added in an ice bath. After confirming completion of the reaction by TLC, an aqueous saturated sodium hydrogen carbonate solution was added. The reaction mixture was extracted with ethyl acetate. The organic layer was washed with saturated saline, then dried with anhydrous sodium sulfate, and filtered, and the filtrate was concentrated. The resulting residue was purified by silica gel column chromatography (chloroform/methanol) to obtain the title compound (110 mg).

LCMS: [M+H]⁺/Rt = 692.40/0.686 min^{c}

Reference Example 8: tert-butyl 2-[(tert-butoxycarbonyl)oxy]-6-({1-[N-(tert-butoxycarbonyl)-D-seryl]azetidin-3-yl}oxy)-3-{2-[(3aS,4S,6S)-3a,5,5-trimethylhexahydro-2H-4,6-methano-1,3,2-benzodioxaborol-2-yl]cyclopropyl}benzoate

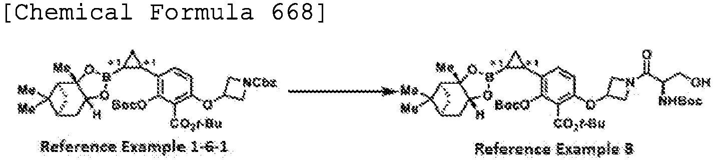

Palladium/carbon (Pd: 10%, wetted with ca. 55% water, 10 mg) was added to a methanol (2 mL) solution of the compound of Reference Example 1-6-1 (100 mg, 0.139 mmol), and the reaction mixture was stirred for 30 minutes under a hydrogen atmosphere at room temperature. The reaction mixture was filtered through celite, and the filtrate was concentrated. The resulting residue was dissolved in DMF (1 mL) as solution A.

DMT-MM (46.3 mg, 0.167 mmol) was added to a methanol solution (1 mL) of N-tert-butoxycarbonyl-D-serine (43 mg, 0.209 mmol), and the reaction mixture was stirred for 15 minutes at room temperature. Solution A was added, and the reaction mixture was stirred for 1 hour at room temperature. Saturated saline was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography (dichloromethane/methanol) to obtain the title compound (112 mg) as a colorless amorphous compound.

¹H-NMR (270 MHz, CDCl₃) δ: 7.24-7.19 (1H, m), 6.39-6.35 (1H, m), 5.56-5.45 (1H, m), 4.97-4.91 (1H, m), 4.75-4.56 (1H, m), 4.44-4.22 (3H, m), 4.09-4.03 (2H, m), 3.88-3.82 (1H, m), 3.74-3.67 (1H, m), 2.19-2.03 (3H, m), 1.91-1.85 (1H, m), 1.80-1.74 (1H, m), 1.63-1.48 (19H, m), 1.45-1.22 (11H, m), 1.17-0.84 (7H, m), 0.73 (3H, s), 0.46-0.36 (1H, m).

Reference Example 9: tert-butyl (2S,4R)-2-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidine-1-carbonyl}-4-hydroxypyrrolidine-1-carboxylic acid

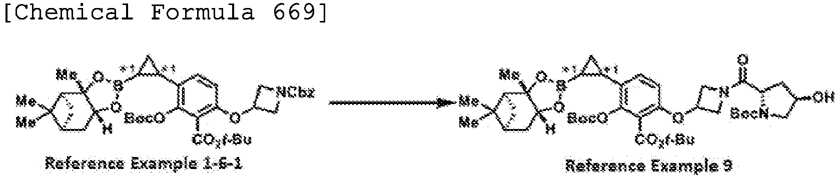

The compound of Reference Example 1-6-1 (100 mg, 0.139 mmol) and trans-N-(tert-butoxycarbonyl)-4-hydroxy-L-proline (48.3 mg, 0.209 mmol) were used as a raw material, and a reaction and post-processing were performed in accordance with the same method as the method described in Reference Example 8 to obtain the title compound (110 mg) as a colorless amorphous compound.

¹H-NMR (270 MHz, CDCl₃) δ: 9.27-8.66 (1H, m), 7.26-7.23 (1H, m), 6.38-6.35 (1H, m), 5.48-5.27 (1H, m), 5.04-4.87 (1H, m), 4.58-4.29 (4H, m), 4.23-3.95 (3H, m), 3.70-3.42 (2H, m), 2.18-2.04 (6H, m), 1.90-1.41 (23H, m), 1.37-1.22 (7H, m), 1.16-1.00 (6H, m), 0.73 (3H, s), 0.46-0.36 (1H, m).

Each of Reference Example compounds 10 to 13 shown in Table 4 was obtained by performing a reaction, post-processing, and purification in accordance with the same method as the method described in Reference Example 1 while using the compound of Reference Example 1-7 and carboxylic acid corresponding to each of the following Reference Examples as a raw material.

**[Table 4-1]**

| Reference Example | Structural formula | LCMS and/or **NMR** |
|---|---|---|
| 10 | 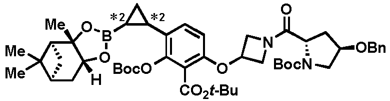 | LCMS:[M+H]⁺/Rt=887.46/1.386min^{c} |
| 11 | 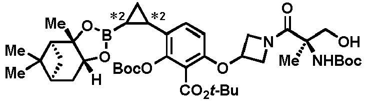 | LCMS:[M+H]⁺/Rt=785.37/1.410min^{A 1}H-NMR (CDCl₃) δ: 7.16 (1H, d, *J* = 8.5 Hz), 6.32 (1H, d, *J* = 8.5 Hz), 5.09-4.96 (1H, m), 4.91-4.86 (1H, m), 4.67-4.60 (1H, m), 4.42-4.28 (2H, m), 4.12-3.97 (2H, m), 3.92 (1H, dd, J = 8.5, 1.8 Hz), 3.53-3.44 (1H, m), 2.16-2.07 (2H, m), 1.99-1.91 (1H, m), 1.85-1.80 (1H, m), 1.78-1.74 (1H, m), 1.66-1.62 (1H, m), 1.53 (9H, s), 1.51 (9H, s), 1.44-1.39 (12H, m), 1.25 (3H, s), 1.19 (3H, s), 1.11-1.06 (1H, m), 1.02-0.97 (1H, m), 0.87-0.80 (1H, m), 0.72 (3H, s), 0.38-0.34 (1H, m). |
| 12 | 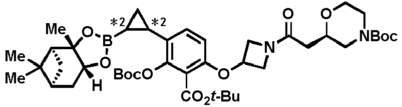 | LCMS:[M+H]⁺/RT=811.39/1.479min^{A 1}H-NMR (CDCl₃) δ: 7.15 (1H, d, *J* = 8.5 Hz), 6.33 (1H, d, *J* = 8.5 Hz), 4.93-4.88 (1H, m), 4.54-4.22 (3H, m), 4.14-4.02 (2H, m), 3.96-3.90 (2H, m), 3.85-3.79 (2H, m), 3.56-3.48 (1H, m), 3.39-3.30 (1H, m), 2.90-2.82 (1H, m), 2.66-2.59 (1H, m), 2.33-2.26 (1H, m), 2.18-2.08 (2H, m), 1.96-1.91 (1H, m), 1.84-1.80 (1H, m), 1.78-1.74 (1H, m), 1.68-1.61 (1H, m), 1.54 (9H, s), 1.52 (9H, s), 1.44 (9H, s), 1.27-1.24 (3H, m), 1.19 (3H, s), 1.11-1.05 (1H, m), 1.03-0.98 (1H, m), 0.85-0.79 (1H, m), 0.72 (3H, s), 0.39-0.34 (1H, m). |

**[Table 4-2]**

| | |
|---|---|
| 13 | 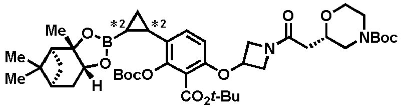 LCMS:[M+H]⁺/RT=811.20/2.60min^{A} |

Reference Example 14: (2R)-2-[(tert-butoxycarbonyl)amino]-3-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-l-yl}-2-methylpropanoate

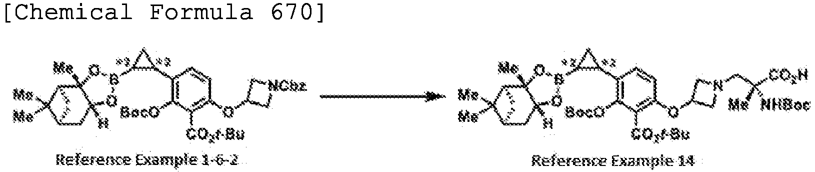

The compound of Reference Example 1-6-2 (0.183 g, 0.255 mmol) was used as a raw material, and a reaction and post-processing were performed in accordance with the same method as the method described in Reference Example 3 to obtain the title compound (109 mg) .

LCMS: [M+H]⁺/RT = 785/2.067 min^{D}

Reference Example 15: tert-butyl 6-[(1-1(2R)-2-[(tert-butoxycarbonyl)amino]-3-[(2-hydroxyethyl)amino]-2-methyl-3-oxopropyl}azetidin-3-yl)oxy]-2-[(tert-butoxycarbonyl)oxy]-3-{2-[(3aS,4S,6S)-3a,5,5-trimethylhexahydro-2H-4,6-methano-1,3,2-benzodioxaborol-2-yl]cyclopropyl}benzoate

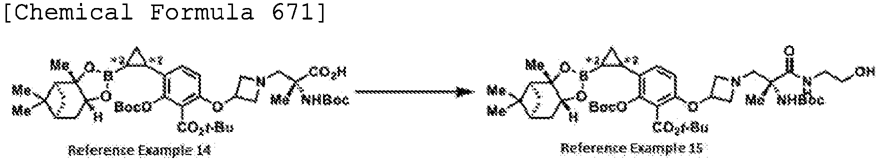

1-hydroxybenzotriazole (50.1 mg, 0.371 mmol), triethylamine (0.0517 mL, 0.371 mmol), and 1-(3-dimethylaminopropyl)-3-ethylcarbodiimide hydrochloride (71.1 mg, 0.371 mmol) were added to a DMF solution (1.2 mL) of the compound of Reference Example 14 (116 mg, 0.148 mmol) in an ice bath. The reaction mixture was stirred for 30 minutes at room temperature, and then 2-aminoethanol (0.020 mL, 0.331) was added in an ice bath. The reaction mixture was stirred for 6 hours at room temperature, and then an aqueous saturated sodium hydrogen carbonate solution was added. The reaction mixture was extracted with ethyl acetate. The organic layer was washed with saturated saline, then dried with anhydrous sodium sulfate, and filtered, and the filtrate was concentrated. The resulting residue was purified by silica gel column chromatography to obtain the title compound as a diastereo mixture (37.6 mg).

LCMS: [M+H]⁺/RT = 828.51/1.195 min^{A}

Reference Example 16: tert-butyl 6-[(1-{(2R)-3-[(2-amino-2-oxoethyl)amino]-2-[(tert-butoxycarbonyl)amino]-2-methyl-3-oxopropyl}azetidin-3-yl)oxy]-2-[(tert-butoxycarbonyl)oxy]-3-{2-[(3aS,4S,6S)-3a,5,5-trimethylhexahydro-2H-4,6-methano-1,3,2-benzodioxaborol-2-yl]cyclopropyl}benzoate

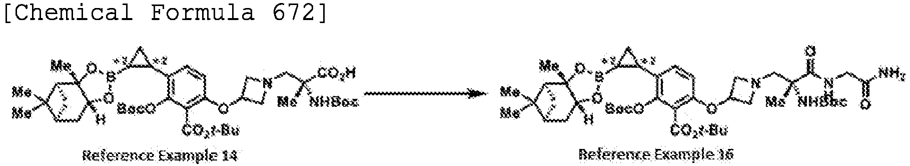

Glycinamide hydrochloride (69 mg, 0.624 mmol), N,N-diisopropylethylamine (0.322 mL, 1.85 mmol), and TBTU (201.7 mg, 0.628 mmol) were added to a dichloromethane solution (8.2 mL) of the compound of Reference Example 14 (322.4 mg, 0.411 mmol) in an ice bath. The reaction mixture was stirred for 30 minutes in an ice bath, and then the reaction mixture was stirred for 4 hours at room temperature. An aqueous saturated sodium hydrogen carbonate solution was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography to obtain the title compound (323.4 mg) as a white solid.

LCMS: [M+H]⁺/RT = 841.67/1.117 min^{A}

¹H-NMR (CD₃OD) δ: 7.20 (1H, d, J = 8.5 Hz), 6.58 (1H, d, J = 8.5 Hz), 4.00 (1H, dd, J = 8.5, 1.8 Hz), 3.89-3.85 (2H, m), 3.82-3.76 (2H, m), 3.26-3.22 (1H, m), 3.20-3.17 (1H, m), 2.95 (2H, dd, J = 18.0, 13.1 Hz), 2.21-2.15 (1H, m), 2.08 (1H, dd, J = 15.6, 7.6 Hz), 1.97-1.91 (1H, m), 1.89 (1H, s), 1.76 (2H, d, J = 6.1 Hz), 1.67 (1H, d, J = 12.8 Hz), 1.56 (9H, s), 1.53 (9H, s), 1.44 (9H, s), 1.40 (3H, s), 1.27 (3H, s), 1.22 (3H, s), 1.12 (1H, td, J = 8.9, 3.9 Hz), 1.06-1.01 (1H, m), 0.77 (3H, s), 0.75 (1H, d, J = 11.0 Hz), 0.35 (1H, td, J = 9.6, 7.1 Hz).

Reference Example 17: tert-butyl (2S,4S)-4-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}-2-carbamoylpyrrolidine-1-carboxylic acid

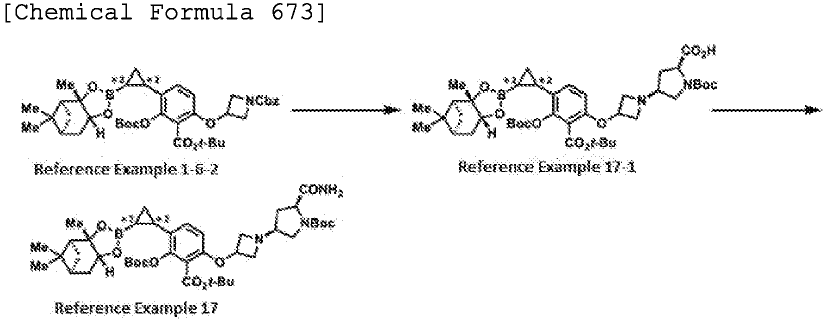

Reference Example 17-1: (48)-1-(tert-butoxycarbonyl)-4-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}-L-proline

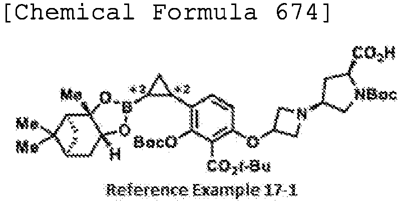

The compound of Reference Example 1-6-2 (0.145 g, 0.248 mmol) and N-tert-butoxycarbonyl-4-oxo-L-proline methyl ester (0.090 g, 0.372 mmol) were used as a raw material, and a reaction and post-processing were performed in accordance with the same method as the method described in Reference Example 3 to obtain the title compound (84.8 mg).

LCMS: [M+H]⁺/RT = 797/2.072 min^{D}

Reference Example 17: tert-butyl (2S,4S)-4-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}-2-carbamoylpyrrolidine-1-carboxylic acid

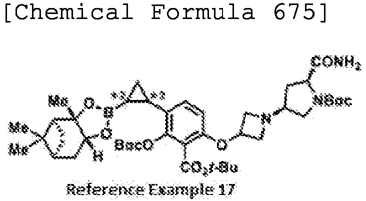

HATU (0.061 g, 0.160 mmol) was added to a dichloromethane solution (1.5 mL) of the compound of Reference Example 17-1 (84.8 mg, 0.106 mmol) and N,N-diisopropylethylamine (0.093 mL, 0.532 mmol). The reaction mixture was stirred for 5 minutes at room temperature, and then ammonium chloride (0.017 g, 0.319 mmol) was added. After stirring for 2.5 hours at room temperature, an aqueous saturated ammonium chloride solution was added, and the reaction mixture was extracted with ethyl acetate. The organic layer was washed with an aqueous saturated sodium hydrogen carbonate solution followed by saturated saline, then dried with anhydrous sodium sulfate, and filtered, and the filtrate was concentrated. The resulting residue was purified by silica gel column chromatography (ethyl acetate/methanol) to obtain the title compound (31.6 mg).

LCMS: [M+H]⁺/RT = 796/2.067 min^{D}

Reference Example 18: tert-butyl (2S,4S)-4-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}-2-carbamoylpyrrolidine-1-carboxylic acid

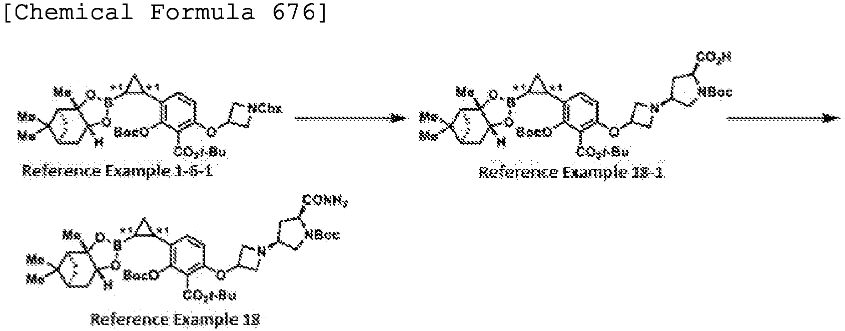

Reference Example 18-1: (48)-1-(tert-butoxycarbonyl)-4-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}-L-proline

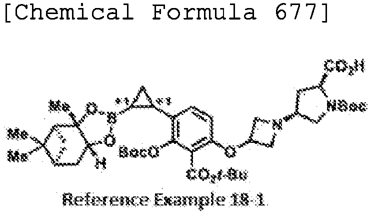

The compound of Reference Example 1-6-1 (100 g, 0.139 mmol) was used as a raw material, and a reaction and post-processing were performed in accordance with the same method as the method described in Reference Example 17-1 to obtain the title compound (49.2 mg) as a colorless solid.

¹H-NMR (270 MHz, CDCl₃) δ: 7.22 (1H, d, J = 8.1 Hz), 6.42 (1H, d, J = 8.1 Hz), 4.82-4.78 (1H, m), 4.26-4.22 (1H, m), 4.07-3.94 (3H, m), 3.65-3.60 (1H, m), 3.35-3.25 (4H, m), 2.25-1.99 (5H, m), 1.91-1.87 (1H, m), 1.80-1.74 (1H, m), 1.61-1.39 (19H, m), 1.33-1.27 (9H, m), 1.17-1.00 (6H, m), 0.94-0.83 (3H, m), 0.74 (3H, s), 0.45-0.36 (1H, m).

Reference Example 18: tert-butyl (2S,4S)-4-{3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}-2-carbamoylpyrrolidine-1-carboxylic acid

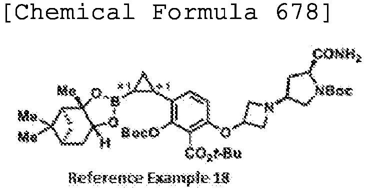

The compound of Reference Example 18-1 (49.2 mg, 0.0618 mmol) was dissolved in dichloromethane (1 mL), and TBTU (21.8 mg, 0.0679 mmol) and N,N-diisopropylethylamine (0.0315 mL, 0.185 mmol) were added. The reaction mixture was stirred for 15 minutes at room temperature, and ammonium (0.5 mol/L dioxane solution, 0.247 mL, 0.124 mmol) was added. The reaction mixture was stirred for 15 minutes at room temperature. A saturated sodium hydrogen carbonate solution and saturated saline were added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography (chloroform/methanol) to obtain the title compound (41.3 mg) as a colorless oily substance.

¹H-NMR (270 MHz, CDCl₃) δ: 7.62 (1H, br), 7.26-7.17 (2H, m), 6.41 (1H, d, J = 8.1 Hz), 5.24 (1H, br), 4.71-4.67 (1H, m), 4.23-4.04 (2H, m), 3.80-3.60 (2H, m), 3.48-3.43 (1H, m), 3.33-3.27 (1H, m), 3.15-3.00 (3H, m), 2.25-1.96 (4H, m), 1.90-1.86 (1H, m), 1.79-1.73 (1H, m), 1.60-1.22 (28H, m), 1.15-0.94 (9H, m), 0.74 (3H, s), 0.44-0.35 (1H, m).

Reference Example 19: tert-butyl 6-{[1-(2-amino-2-oxoethyl)azetidin-3-yl]oxy}-2-[(tert-butoxycarbonyl)oxy]-3-{2-[(3aS,4S,6S)-3a,5,5-trimethylhexahydro-2H-4,6-methano-1,3,2-benzodioxaborol-2-yl]cyclopropyl}benzoate

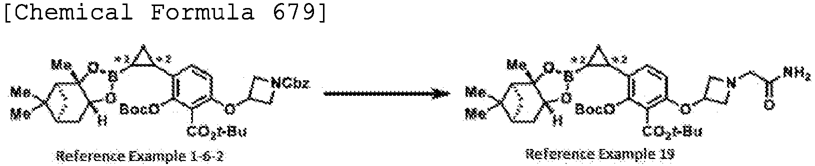

Palladium/carbon (Pd: 10%, wetted with ca. 55% water, 20 mg) was added to a methanol (5 mL) solution of the compound of Reference Example 1-6-2 (200 mg, 0.279 mmol), and the reaction mixture was stirred for 2 hours under a hydrogen atmosphere at room temperature. The reaction mixture was filtered through celite, and the filtrate was concentrated. The resulting residue was dissolved in DMF (5 mL), and 2-iodoacetamide (77 mg, 0.418 mmol) and cesium carbonate (182 mg, 0.557 mmol) were added while cooling with ice. The reaction mixture was stirred for 1 hour at a reaction temperature of 0 to 5°C. Water was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography (chloroform/methanol) to obtain the title compound (70 mg).

LCMS: [M+H]⁺/Rt = 641.65/1.186 min^{H}

Reference Example 20: tert-butyl 2-[(tertbutoxycarbonyl)oxy]-6-{[1-(8,8,9,9-tetramethyl-2-oxo-4,7-dioxa-3-aza-8-siladecan-1-yl)azetidin-3-yl]oxy}-3-{2-[(3aS,4S,6S)-3a,5,5-trimethylhexahydro-2H-4,6-methano-1,3,2-benzodioxaborol-2-yl]cyclopropyl}benzoate

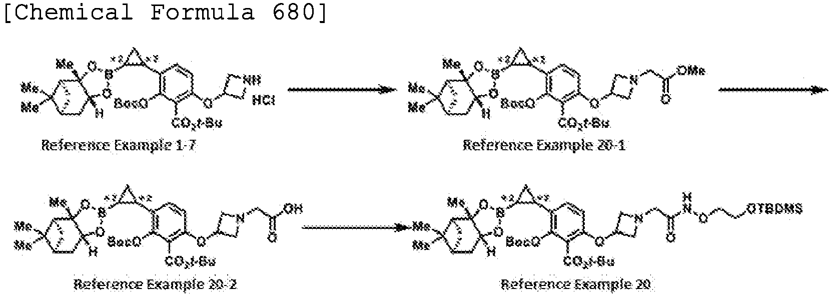

Reference Example 20-1: tert-butyl 2-[(tert-butoxycarbonyl)oxy]-6-{[1-(2-methoxy-2-oxoethyl)azetidin-3-yl]oxy}-3-{(2-[(3aS,4S,6S)-3a,5,5-trimethylhexahydro-2H-4,6-methano-1,3,2-benzodioxaborol-2-yl]cyclopropyl}benzoate

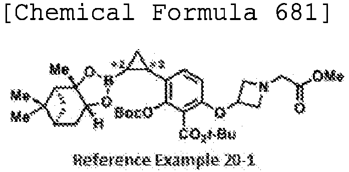

Triethylamine (0.049 mL, 0.352 mmol), methyl bromoacetate (0.020 mL, 0.211 mmol) and cesium carbonate (182 mg,0.557 mmol) were added to a THF (0.7 mL) solution of the compound of Reference Example 1-7 (87.4 mg, 0.141 mmol) while cooling with ice, and the reaction mixture was stirred for 14 hours at room temperature. The reaction mixture was concentrated. The resulting residue was purified by silica gel column chromatography to obtain the title compound (33.6 mg) as a colorless oil substance.

¹H-NMR (CDCl₃) δ: 7.14 (1H, d, J = 8.5 Hz), 6.40 (1H, d, J = 8.5 Hz), 4.90-4.85 (1H, m), 4.17-4.12 (2H, m), 3.95-3.93 (1H, m), 3.72 (3H, s), 3.61 (1H, d, J = 7.9 Hz), 3.47-3.43 (1H, m), 3.33-3.27 (1H, m), 2.15-2.07 (3H, m), 1.95-1.89 (1H, m), 1.81 (1H, t, J = 5.5 Hz), 1.78-1.74 (1H, m), 1.67-1.63 (1H, m), 1.55 (9H, s), 1.52 (9H, s), 1.26 (3H, s), 1.19 (3H, s), 1.10-1.05 (1H, m), 1.03-0.98 (1H, m), 0.80 (1H, d, J = 11.0 Hz), 0.72 (3H, s), 0.36 (1H, td, J = 9.6, 7.1 Hz).

Reference Example 20-2: {3-[2-(tert-butoxycarbonyl)-3-[(tert-butoxycarbonyl)oxy]-4-{2-[(3aS,4S,6S)-3a,5,5-trimethylhexahydro-2H-4,6-methano-1,3,2-benzodioxaborol-2-yl]cyclopropyl}phenoxy]azetidin-1-yl}acetate

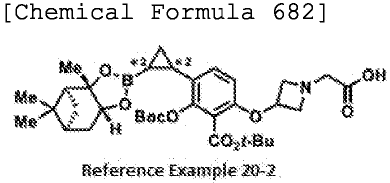

The compound of Reference Example 20-1 (33.6 mg, 0.051 mmol) was used as a raw material, and a reaction and post-processing were performed in accordance with the same method as the method described in Reference Example 3 to obtain the title compound (28 mg) .

LCMS: [M+H]⁺/Rt = 642.4/1.090 min^{B}

Reference Example 20: tert-butyl 2-[(tert-butoxycarbonyl)oxy]-6-{[1-(8,8,9,9-tetramethyl-2-oxo-4,7-dioxa-3-aza-8-siladecan-1-yl)azetidin-3-yl]oxy}-3-{2-[(3aS,4S,6S)-3a,5,5-trimethylhexahydro-2H-4,6-methano-1,3,2-benzodioxaborol-2-yl]cyclopropyl}benzoate

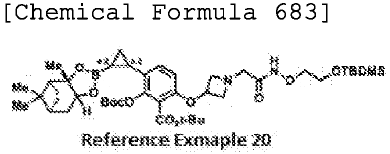

The compound of Reference Example 20-2 (28 mg, 0.044 mmol) and O-[2-{(tert-butyldimethylsilyl)oxy}ethyl]hydroxylamine hydrochloride (22 mg, 0.097 mmol) were used as a raw material, and a reaction and post-processing were performed in accordance with the same method as the method described in Reference Example 17 to obtain the title compound (45.1 mg).

LCMS: [M+H]⁺/Rt = 815.49/1.26 min^{A}

Reference Example 21: tert-butyl 6-{[1-({5-[(tertbutoxycarbonyl)amino]-2,2-dimethyl-1,3-dioxa-5-yl}methyl)azetidin-3-yl]oxy}-2-[(tert-butoxycarbonyl)oxy]-3-{2-[(3aS,4S,6S)-3a,5,5-trimethylhexahydro-2H-4,6-methano-1,3,2-benzodioxaborol-2-yl]cyclopropyl}benzoate

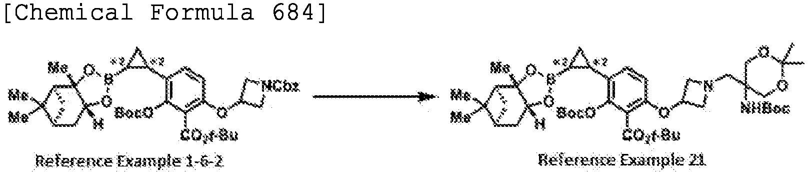

Palladium/carbon (Pd: 10%, wetted with ca. 55% water, 50 mg) was added to a methanol (5 mL) solution of the compound of Reference Example 1-6-2 (0.100 g, 0.139 mmol), and the reaction mixture was stirred for 1 hour under a hydrogen atmosphere at room temperature. The reaction mixture was filtered through celite, and the filtrate was concentrated. The resulting residue was dissolved in dichloromethane (1 mL), and tert-butyl(5-formyl-2,2-dimethyl-1,3-dioxan-5-yl)carbamate (72.1 mg, 0.278 mmol) and acetic acid (0.00836 mL, 0.146 mmol) were added. The reaction mixture was stirred for 1 hour at room temperature. Sodium triacetoxyborohydride (88 mg, 0.417 mmol) was added to the reaction mixture, which was stirred at room temperature. After completion of the reaction, an aqueous saturated sodium hydrogen carbonate solution was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography (hexane/ethyl acetate) to obtain the title compound (81.4 mg).

LCMS: [M+H]⁺/RT = 827/2.283 min^{D}

The compound of Reference Example 1-6-1 and aldehyde corresponding to each of the following Reference Examples were used as the starting materials, and a reaction, post-processing, and purification were performed in accordance with the same method as the method described in Reference Example 21 to obtain each of Reference Compounds 22 and 23 shown in Table 5.

**[Table 5]**

| Reference Example | Structural formula | LCMS and/or NMR |
|---|---|---|
| Reference Example 22 | 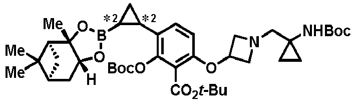 | LCMS: [M+H]⁺/RT=753/2.179 min^{D} |
| Reference Example 23 | 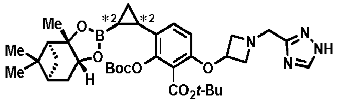 | LCMS: [M+H]⁺/RT=665/1.908 min^{D} |

Reference Example 24: tert-butyl 6-{[1-({(1r,4r)-4-[(tert-butoxycarbonyl){2-[(tert-butoxycarbonyl)amino]ethyl}amino]cyclohexyl}acetyl)azetidin-3-yl]oxy}-2-[(tert-butoxycarbonyl)oxy]-3-{2-[(3aS,4S,6S)-3a,5,5-trimethylhexahydro-2H-4,6-methano-1,3,2-benzodioxaborol-2-yl]cyclopropyl}benzoate

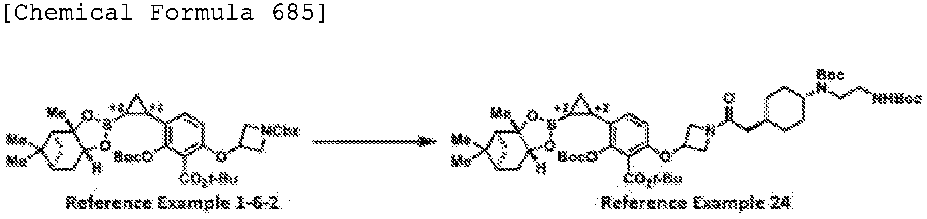

Palladium/carbon (Pd: 10%, wetted with ca. 55% water, 270 mg) was added to a methanol (5 mL) solution of the compound of Reference Example 1-6-2 (0.327 g, 0.456 mmol), and the reaction mixture was stirred for 1 hour under a hydrogen atmosphere at room temperature. The reaction mixture was filtered through celite, and the filtrate was concentrated.

HATU (0.290 g, 0.762 mmol) was added to a DMF (0.8 mL) solution of 2-[(1r,4r)-4-{[(tert-butoxy)carbonyl](2-{[(tert-butoxy)carbonyl]amino}ethyl)amino}cyclohexyl]acetate (0.305 g, 0.762 mmol) and triethylamine (0.222 mL, 1.596 mmol) in a known document (e.g., J. Med. Chem. 2019, 62, 8544., etc.), and the reaction mixture was stirred for 45 minutes at room temperature. The reaction mixture was added with a DMF (1.6 mL) solution of the residue obtained from the catalytic hydrogenation reaction described above and stirred at room temperature. After 1.5 hours, an aqueous saturated ammonium chloride solution was added to the reaction mixture, which was extracted with ethyl acetate. The organic layer was washed with an aqueous saturated sodium hydrogen carbonate solution followed by saturated saline, then dried with anhydrous sodium sulfate, and filtered, and the filtrate was concentrated under reduced pressure. The resulting residue was purified by silica gel column chromatography (hexane/ethyl acetate) to obtain the title compound (182.2 mg).

LCMS: [M+H]⁺/RT = 966/2.052 min^{E}

Reference Example 25: tert-butyl 6-[(1-{(2R)-3-[(2-aminoethyl)amino]-2-[(tert-butoxycarbonyl)amino]-2-methyl-3-oxopropyl}azetidin-3-yl)oxy]-2-[(tert-butoxycarbonyl)oxy]-3-{2-[(3aS,4S,6S)-3a,5,5-trimethylhexahydro-2H-4,6-methano-1,3,2-benzodioxaborol-2-yl]cyclopropyl}benzoate

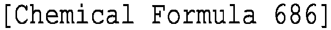

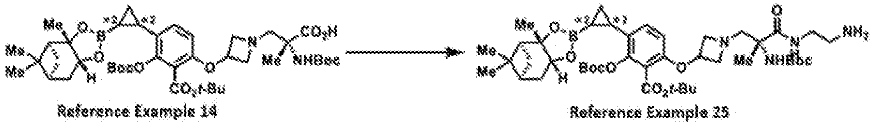

The compound of Reference Example 14 (130 mg, 0.166 mmol) and ethylenediamine (0.017 mL, 0.248 mmol) were used as a raw material, and a reaction and post-processing were performed in accordance with the same method as the method described in Reference Example 16 to obtain the title compound (30 mg).

LCMS: [M+H]⁺/RT = 419/1.167 min^{F}
1H-NMR (400 MHz, D₂O) δ: 7.06 (1H, d, J = 8.5 Hz), 6.12 (1H, d, J = 8.5 Hz), 3.79 (2H, m), 3.65 (1H, m), 3.55 (3H, m), 3.21 (2H, m), 3.04 (1H, d, J = 12.8 Hz), 2.75 (1H, d, J = 12.8 Hz), 1.84 (1H, td, J = 8.0, 4.4 Hz), 1.35 (3H, s), 0.86 (1H, td, J = 8.0, 2.4 Hz). 0.34 (3H, m)

Reference Example 26: tert-butyl 6-({1-[N-(tert-butoxycarbonyl)-L-histidinyl]azetidin-3-yl}oxy)-2-[(tert-butoxycarbonyl)oxy]-3-{(2-[(3aS,4S,6S,7aR)-3a,5,5-trimethylhexahydro-2H-4,6-methano-1,3,2-benzodioxaborol-2-yl]cyclopropyl}benzoate

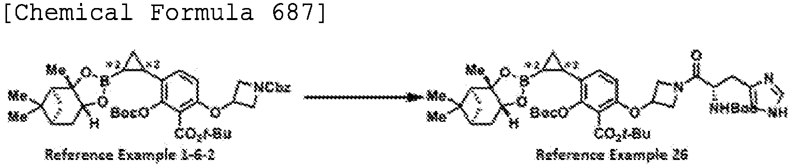

The compound of Reference Example 1-6-2 (100 mg, 0.139 mmol) and N^{α}-(tert-butoxycarbonyl)-L-histidine (53.4 mg, 0.209 mmol) were used as a raw material, and a reaction and post-processing were performed in accordance with the same method as the method described in Reference Example 8 to obtain the title compound (68.3 mg) as a colorless solid.

¹H-NMR (270 MHz, CDCl3) δ: 7.21-6.90 (2H,m), 6.78-6.73 (1H, m), 6.00-5.96 (1H, m), 5.30-5.22 (1H, m), 4.73-4.63 (1H, m), 4.40-3.92 (6H, m), 3.18-2.76 (2H, m), 2.25-2.05 (3H, m), 1.99-1.95 (1H, m), 1.88-0.97 (37H, m), 0.75 (3H, s), 0.48-0.35 (1H, m).

Reference Example 27: tert-butyl 6-[(1-{2-[(tert-butoxycarbonyl)amino]ethyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate

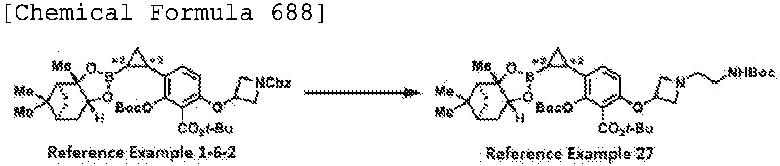

Palladium/carbon (Pd: 10%, wetted with ca. 55% water, 20 mg) and N-(tert-butoxycarbonyl)-2-aminoacetaldehyde (48.8 mg, 0.307 mmol) were added to a methanol (3 mL) solution of the compound of Reference Example 1-6-2 (200 mg, 0.279 mmol), and the reaction mixture was stirred for 4 hours under a hydrogen atmosphere at room temperature. The reaction mixture was filtered through celite, and the filtrate was concentrated. The resulting residue was purified by silica gel column chromatography (dichloromethane/methanol) to obtain the title compound (115 mg) as a colorless oily substance.

¹H-NMR (270 MHz, CDCl3) δ: 7.15 (1H, d, J = 8.1 Hz), 6.41 (1H, d, J = 8.1 Hz), 4.90 (1H, br), 4.82-4.73 (1H, m), 3.98-3.94 (1H, m), 3.84-3.79 (2H, m), 3.13-3.06 (4H, m), 2.60 (2H, t, J = 5.4Hz), 2.19-2.03 (2H, m), 1.99-1.75 (2H, m), 1.70-1.21 (32H, m), 1.13-0.99 (3H, m), 0.92-0.79 (2H, m), 0.74 (3H, s), 0.42-0.33 (1H, m).

Reference Example 28: tert-butyl 5-{[(tert-butoxycarbonyl)amino]methyl}-2-(2-{3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidin-1-yl}-2-oxoethyl)morpholine-4-carboxylic acid

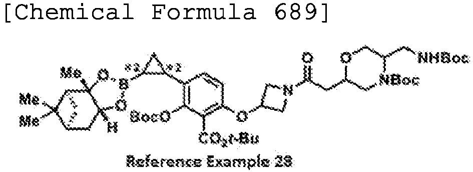

Reference Example 28-1: [4-(tert-butoxycarbonyl)-5-{[(tert-butoxycarbonyl)amino]methyl}morpholin-2-yl]acetic acid

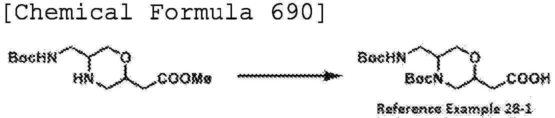

An aqueous 2 mol/L sodium hydroxide solution (3.5 mL) and di-tert-butyl dicarbonate (0.163 mL, 0.707 mmol) were added to a THF solution (3.5 mL) of methyl 2-(5-[{(tert-butoxycarbonyl)amino}methyl]morpholin-2-yl)acetic acid (102 mg, 0.354 mmol) in a known document (e.g., Bioorg. Med. Chem. Lett., 1996, 6, 1529, etc.), and the reaction mixture was stirred for 4 hours at room temperature. Water (50 mL) and 2 mol/L hydrochloric acid (5 mL) were added to the reaction mixture, which was extracted with ethyl acetate. The organic layer was washed with an aqueous saturated sodium hydrogen carbonate solution followed by saturated saline, then dried with anhydrous sodium sulfate, and filtered, and the filtrate was concentrated under reduced pressure. The resulting residue was purified by silica gel column chromatography (dichloromethane/methanol) to obtain the title compound (99.7 mg) as a colorless oily substance.

LCMS: [M+H]⁺/Rt = 375.6/1.75 min, 375.6/1.80 min^{G} (diastereomer mixture)

1H-NMR (270 MHz, CDCl3) δ: 4.86-4.62 (1H, m), 4.32-4.25 (1H, m), 4.14-3.97 (1H, m), 3.92-3.41 (5H, m), 3.28-3.08 (1H, m), 3.00-2.76 (1H, m), 2.66-2.45 (1H, m), 1.47-1.43 (18H, m).

Reference Example 28: tert-butyl 5-{[(tert-butoxycarbonyl)amino]methyl}-2-(2-{3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidin-1-yl}-2-oxoethyl)morpholine-4-carboxylic acid

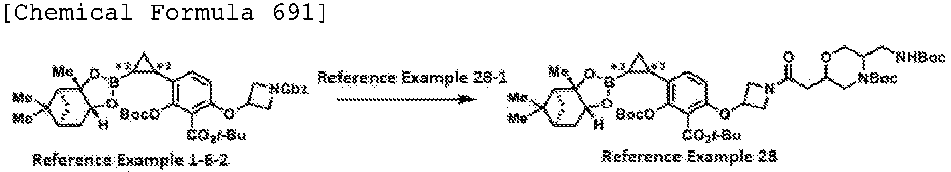

Palladium/carbon (Pd: 10%, wetted with ca. 55% water, 17 mg) was added to a methanol (3 mL) solution of the compound of Reference Example 1-6-2 (170 mg, 0.237 mmol), and the reaction mixture was stirred for 30 minutes under a hydrogen atmosphere at room temperature. The reaction mixture was filtered through celite, and the filtrate was concentrated. The resulting residue was dissolved in DMF (1 mL) as solution A.

HATU (94.6 mg, 0.249 mmol) and triethylamine (0.0719 mL, 0.261 mmol) were added to a DMF solution of the compound of Reference Example 28-1 (97.6 mg, 0.261 mmol). The reaction mixture was stirred for 30 minutes at room temperature. Solution A was added, and the reaction mixture was stirred for 2 hours at room temperature. Saturated saline was added to the reaction mixture, which was extracted with ethyl acetate. The organic layer was washed with saturated saline, then dried with anhydrous sodium sulfate, and filtered, and the filtrate was concentrated. The resulting residue was purified by silica gel column chromatography (hexane/ethyl acetate) to obtain the title compound (150 mg) as a colorless amorphous compound.

1H-NMR (270 MHz, CDCl3) δ: 7.20-7.15 (1H, m), 6.35 (1H, d, J = 8.1 Hz), 4.94-3.21 (15H, m), 2.57-2.34 (2H, m), 2.19-2.08 (2H, m), 2.01-1.92 (1H, m), 1.88-1.76 (2H, m), 1.69-0.98 (39H, m), 0.91-0.81 (6H, m), 0.74 (3H, s), 0.44-0.35 (1H, m).

Reference Example 29: Synthesis of Reference Example 1-6-2 (1g scale)

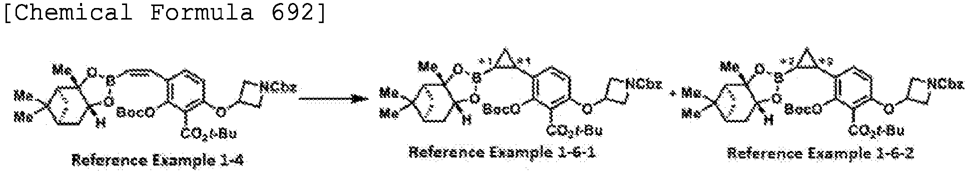

A 1 mol/L hexane (11.4 mL, 11.4 mmol) solution of diethylzinc was dissolved in dichloromethane (8.4 mL) under a nitrogen atmosphere at -78°C, and diiodomethane (1.37 mL, 17.4 mmol) was slowly added. The reaction mixture was stirred for 5 minutes at - 20°C and then cooled again to -78°C, and a dichloromethane (4 mL) solution of the compound of Reference Example 1-4 (999 mg, 1.42 mmol) was added dropwise. The reaction mixture was stirred for 5 minutes at -78°C, then stirred for 30 minutes at -20°C, and then stirred for 22 hours at -5°C. The reaction mixture was added with a dichloromethane (2 mL) solution of di-tert-butyl dicarbonate (2.97 mL, 12.8 mmol), a dichloromethane (2 mL) solution of 4-dimethylaminopyridine (0.173 g, 1.42 mmol), and triethylamine (3.96 mL, 28.4 mmol) and stirred for 15 minutes at -5°C and warmed to room temperature. An aqueous saturated ammonium chloride solution was added to the reaction mixture, which was extracted with chloroform. The organic layer was washed with saturated saline, then dried with anhydrous sodium sulfate, and filtered, and the filtrate was concentrated (crude product comprises a diastereomer mixture of the compound of Reference Example 1-6-1 and the compound of Reference Example 1-6-2, and the mixture ratio (1-6-1:1-6-2) was about 1:3 (according to ¹H-NMR integration ratio)). The resulting residue was purified by silica gel column chromatography to obtain the compound of Reference Example 1-6-2 (identified by TLC (silica gel plate) Rf value: 0.52 (hexane/ethyl acetate = 2/1)) (473 mg) as a colorless amorphous compound. The compound of Reference Example 1-6-1 (identified by TLC (silica gel plate) Rf value: 0.46 (hexane/ethyl acetate = 2/1)) was not isolated.

¹H-NMR (CDCl₃) δ: 7.35-7.27 (5H, m), 7.14 (1H, d, J = 8.5 Hz), 6.31 (1H, d, J = 8.5 Hz), 5.09 (2H, s), 4.92-4.86 (1H, m), 4.35-4.30 (2H, m), 4.06 (2H, dd, J = 9.8, 4.3 Hz), 3.93 (1H, d, J = 8.6 Hz), 2.16-2.08 (2H, m), 1.99 (2H, s), 1.94-1.90 (1H, m), 1.81 (1H, t, J = 5.5 Hz), 1.77-1.74 (1H, m), 1.66-1.62 (1H, m), 1.53 (9H, s), 1.52 (9H, s), 1.25 (3H, s), 1.18 (3H, s), 1.10-1.05 (1H, m), 1.02-0.98 (1H, m), 0.80 (1H, d, J = 10.4 Hz), 0.71 (3H, s), 0.39-0.32 (1H, m).

Reference Example 30: Synthesis of Reference Example 1-6-2 (4.5 g scale) 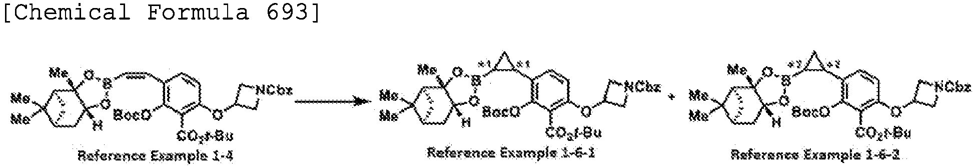 1 mol/L hexane (49.7 mL, 49.7 mmol) solution of diethylzinc was dissolved in dichloromethane (49.7 mL) under a nitrogen atmosphere at -20°C, and diiodomethane (6.88 mL, 85 mmol) was slowly added. After stirring the reaction mixture for 20 minutes at -20°C, a dichloromethane (5 mL) solution of the compound of Reference Example 1-4 (4.55 g, 6.47 mmol) was added dropwise. The reaction mixture was stirred for 30 minutes at -20°C and then stirred for 22.5 hours at -5°C. To the reaction mixture, a dichloromethane (15 mL) solution of di-tert-butyl dicarbonate (14.85 mL, 64.0 mmol), a dichloromethane (15 mL) solution of 4-dimethylaminopyridine (0.868 g, 7.11 mmol), and triethylamine (19.8 mL, 142 mmol) were added and stirred for 30 minutes at -5°C, and then the reaction mixture was warmed to room temperature and stirred for 30 minutes. At 0°C, an aqueous saturated ammonium chloride solution was added to the reaction mixture, which was extracted with chloroform. The organic layer was washed with saturated saline, then dried with anhydrous sodium sulfate, and filtered, and the filtrate was concentrated. The resulting residue was purified by silica gel column chromatography to obtain the compound of Reference Example 1-6-2 (identified by TLC (silica gel plate) Rf value: 0.52 (hexane/ethyl acetate = 2/1)) (2.537 g) as a colorless amorphous compound. The compound of Reference Example 1-6-1 (identified by TLC (silica gel plate) Rf value: 0.46 (hexane/ethyl acetate = 2/1)) was not isolated.

¹H-NMR (CDCl₃) δ: 7.35-7.27 (5H, m), 7.14 (1H, d, J = 8.5 Hz), 6.31 (1H, d, J = 8.5 Hz), 5.09 (2H, s), 4.92-4.86 (1H, m), 4.35-4.30 (2H, m), 4.06 (2H, dd, J = 9.8, 4.3 Hz), 3.93 (1H, d, J = 8.6 Hz), 2.16-2.08 (2H, m), 1.99 (2H, s), 1.94-1.90 (1H, m), 1.81 (1H, t, J = 5.5 Hz), 1.77-1.74 (1H, m), 1.66-1.62 (1H, m), 1.53 (9H, s), 1.52 (9H, s), 1.25 (3H, s), 1.18 (3H, s), 1.10-1.05 (1H, m), 1.02-0.98 (1H, m), 0.80 (1H, d, J = 10.4 Hz), 0.71 (3H, s), 0.39-0.32 (1H, m).

Reference Example 31: (2R)-2-(2-{3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidin-1-yl}-2-oxoethyl)-4,4-dimethylmorpholin-4-ium iodide

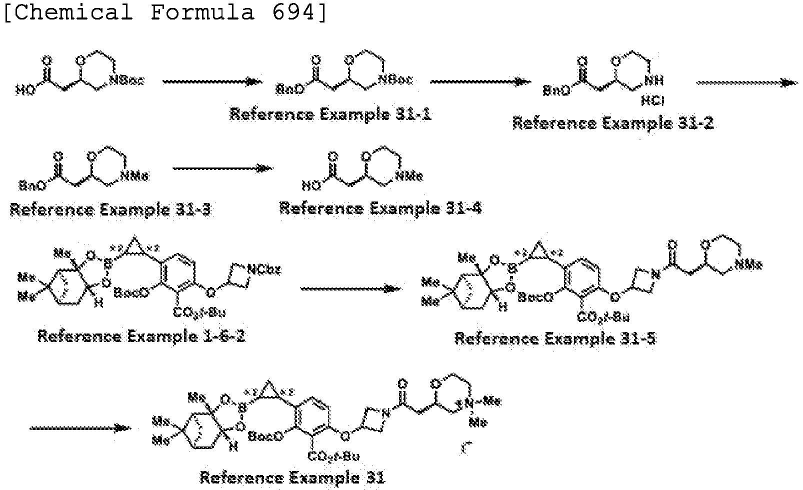

Reference Example 31-1: tert-butyl (2R)-2-[2-(benzyloxy)-2-oxoethyl]morpholine-4-carboxylate

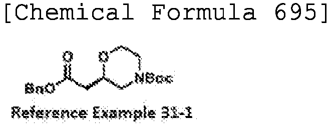

Benzylbromide (916 mg, 5.35 mmol) was added to a DMF (13.7 mL) mixture of [(2R)-4-(tert-butoxycarbonyl)morpholin-2-yl]acetic acid (1.01 g, 4.12 mmol) and cesium carbonate (2.01 g, 6.18 mmol). The reaction mixture was stirred for 3 hours at room temperature. An aqueous saturated ammonium chloride solution was added to the reaction mixture, which was extracted with ethyl acetate. The organic layer was washed twice with an aqueous saturated ammonium chloride solution and with saturated saline, then dried with anhydrous magnesium sulfate, filtered, and concentrated under reduced pressure. The resulting residue was purified by silica gel column chromatography (hexane/ethyl acetate) to obtain the title compound (1.33 g).

¹H-NMR (400 MHz, CDCl3) δ: 7.37-7.28 (5H, m), 5.16 (1H, d, J = 12.2 Hz), 5.11 (1H, d, J =12.2 Hz), 3.91-3.82 (4H, m), 3.54-3.48 (1H, m), 2.93-2.90 (1H, m), 2.65 (1H, brs), 2.58-2.52 (1H, m), 2.48-2.43 (1H, m), 1.44 (9H, s).

Reference Example 31-2: benzyl [(2R)-morpholin-2-yl]acetate hydrochloride

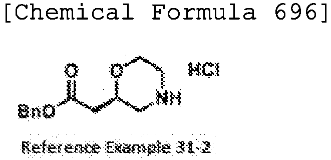

A mixture of the compound of Reference Example 31-1 (1.33 g, 3.95 mmol) and 4 mol/L hydrochloric acid/4-methyltetrahydropyran (19.8 mL, 79 mmol) was stirred at room temperature. After 2 hours, the reaction mixture was concentrated under reduced pressure to obtain the title compound (1.20 g).

¹H-NMR (400 MHz, DMSO-d₆) δ: 7.46-7.37 (5H, m), 5.20 (1H, d, J = 12.2 Hz), 5.16 (1H, d, J = 12.2 Hz), 4.23-4.16 (1H, m), 4.01-3.97 (1H, m), 3.83-3.76 (1H, m), 3.29 (1H, d, J = 12.8 Hz), 3.21 (1H, d, J = 12.8 Hz), 3.00-2.93 (1H, m), 2.90-2.77 (2H, m), 2.60-2.53 (1H, m) .

Reference Example 31-3: benzyl [(2R)-4-methylmorpholin-2-yl]acetate

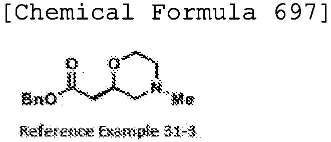

Diisopropylethylamine (4.14 mL, 23.70 mmol) and iodomethane (0.37 mL, 5.93 mmol) were added to a tetrahydrofuran (13 mL) mixture of the compound of Reference Example 31-2 (1.07 g, 3.95 mmol), and the reaction mixture was stirred for 6 hours at 60°C. In an ice bath, water was added to the reaction mixture, which was extracted with chloroform/ethanol (4:1). The organic layer was dried with anhydrous magnesium sulfate, filtered, and concentrated under reduced pressure. The resulting residue was purified by silica gel column chromatography (chloroform/methanol) to obtain the title compound (866.2 mg).

LCMS: [M+H]⁺/Rt = 250/1.433 min^{F}
¹H-NMR (400 MHz, CDCl3) δ: 7.36-7.27 (5H, m), 5.15 (1H, d, J = 12.2 Hz), 5.10 (1H, d, J = 12.2 Hz), 4.02-3.97 (1H, m), 3.86-3.83 (1H, m), 3.72-3.66 (1H, m), 2.76 (1H, d, J = 11.6 Hz), 2.65 (1H, d, J = 11.6 Hz), 2.59-2.53 (1H, m), 2.47-2.42 (1H, m), 2.28 (3H, s), 2.14-2.11 (1H, m), 1.91 -1.86 (1H, m).

Reference Example 31-4: [(2R)-4-methylmorpholin-2-yl]acetic acid

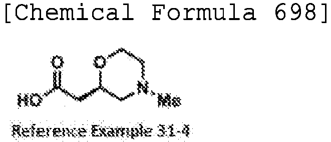

Palladium/carbon (Pd: 10%, wetted with ca. 55% water, 540 mg) was added to a methanol (17 mL) solution of the compound of Reference Example 31-3 (838 mg, 3.36 mmol), and the reaction mixture was stirred for 1.5 hours under a hydrogen atmosphere at room temperature. The reaction mixture was filtered through celite. The filtrate was concentrated under reduced pressure to obtain the title compound (568.6 mg).

¹H-NMR (400 MHz, CD₃OD) δ: 4.03-3.92 (2H, m), 3.76-3.69 (1H, m), 3.20 (1H, d, J = 12.2 Hz), 3.04 (1H, d, J = 12.2 Hz), 2.63-2.56 (4H, m), 2.48-2.33 (3H, m).

Reference Example 31-5: tert-butyl 2-[(tert-butoxycarbonyl)oxy]-6-[(1-{[(2R)-4-methylmorpholin-2-yl]acetyl}azetidin-3-yl)oxy]-3-{2-[(3aS,4S,6S,7aR)-3a,5,5-trimethylhexahydro-2H-4,6-methano-1,3,2-benzodioxaborol-2-yl]cyclopropyl}benzoate

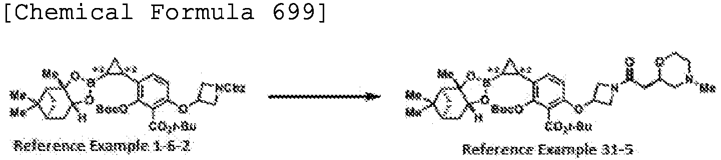

Palladium/carbon (Pd: 10%, wetted with ca. 55% water, 618 mg) was added to a methanol (10 mL) solution of the compound of Reference Example 1-6-2 (508.5 mg, 0.709 mmol), and the reaction mixture was stirred for 1.5 hours under a hydrogen atmosphere at room temperature. The reaction mixture was filtered through celite, and the filtrate was concentrated under reduced pressure. The resulting residue was dissolved in dichloromethane (7 mL) as solution A.

1-hydroxybenzotriazole (326 mg, 2.13 mmol) and 1-(3-dimethylaminopropyl)-3-ethylcarbodiimide hydrochloride (407 mg, 2.13 mmol) were added to a dichloromethane (3 mL) mixture of the compound of Reference Example 31-4 (568.6 mg, 3.57 mmol) and diisopropylethylamine (0.619 mL, 3.54 mmol), and the reaction mixture was stirred for 30 minutes at room temperature. The reaction mixture was added with solution A described above and stirred for 3 hours at room temperature. An aqueous saturated ammonium chloride solution was then added to the reaction mixture, which was extracted with chloroform/ethanol (4:1). The organic layer was washed with an aqueous saturated sodium hydrogen carbonate solution, then dried with anhydrous magnesium sulfate, filtered, and concentrated under reduced pressure. The resulting residue was purified by silica gel column chromatography (chloroform/methanol) to obtain the title compound (258 mg).

LCMS: [M+H]⁺/Rt = 725/1.692 min^{I}

Reference Example 31-5: (2R)-2-(2-{3-[2-(tert-butoxycarbonyl)-3-[(tert-butoxycarbonyl)oxy]-4-{2-[(3aS,4S,6S,7aR)-3a,5,5-trimethylhexahydro-2H-4,6-methano-1,3,2-benzodioxaborol-2-yl]cyclopropyl}phenoxy]azetidin-1-yl}-2-oxoethyl)-4,4-dimethylmorpholin-4-ium iodide

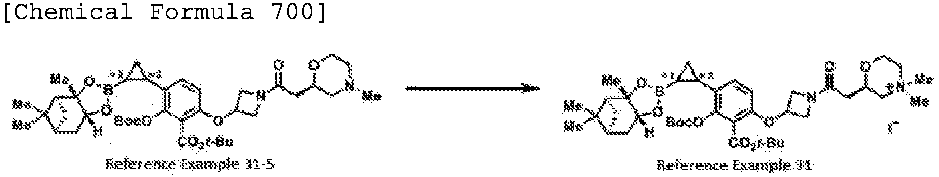

Iodomethane (0.019 mL, 0.304 mmol) was added to a dichloromethane (0.5 mL) solution of the compound of Reference Example 31-5 (142.6 mg, 0.197 mmol). After stirring overnight at room temperature, the reaction solution was concentrated under reduced pressure to obtain the title compound (170.6 mg).

LCMS: [M+H]⁺/Rt = 739/1.933 min^{D}

The compound of Reference Example 14 and amine corresponding to each of the following Reference Examples were used as a starting material, and a reaction, post-processing, and purification were performed in accordance with the same method as the method described in Reference Example 16 to obtain each of Reference Example compounds 32 and 33 shown in Table 6.

**[Table 6]**

| Reference Example | Structural formula | LCMS and/or NMR |
|---|---|---|
| 32 | 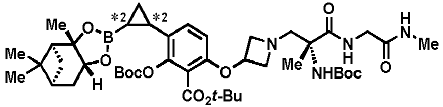 | LCMS: [M+H]⁺/Rt = 855/1.983 min^{D} |
| 33 | 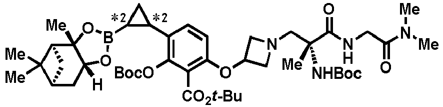 | LCMS: [M+H]⁺/Rt = 869/2.067 min^{D} |

Reference Example 34: tert-butyl 2-[(tert-butoxycarbonyl)oxy]-6-({1-[(3-hydroxy-2-methyl-4-oxopyridin-1(4H)-yl)acetyl]azetidin-3-yl}oxy)-3-{2-[(3aS,4S,6S,7aR)-3a,5,5-trimethylhexahydro-2H-4,6-methano-1,3,2-benzodioxaborol-2-yl]cyclopropyl}benzoate

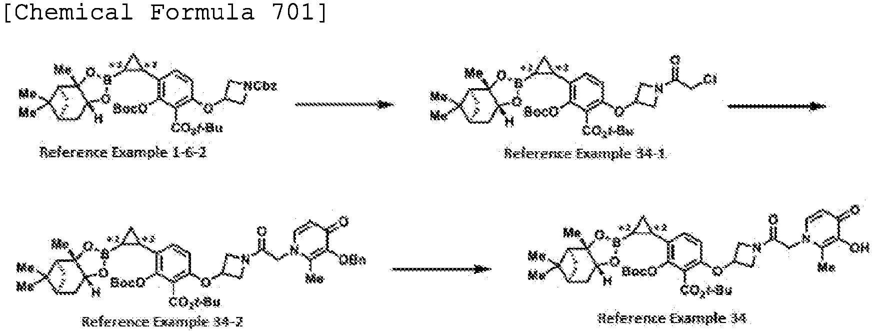

Reference Example 34-1: tert-butyl 2-[(tert-butoxycarbonyl)oxy]-6-{[1-(chloroacetyl)azetidin-3-yl]oxy}-3-{2-[(3aS,4S,6S,7aR)-3a,5,5-trimethylhexahydro-2H-4,6-methano-1,3,2-benzodioxaborol-2-yl]cyclopropyl}benzoate

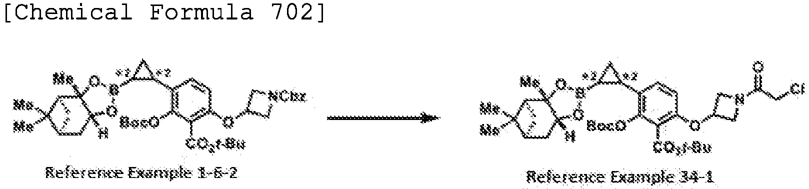

Palladium/carbon (Pd: 10%, wetted with ca. 55% water, 150 mg) was added to a methanol (25 mL) solution of the compound of Reference Example 1-6-2 (650.0 mg, 0.906 mmol), and the reaction mixture was stirred for 4 hours under a hydrogen atmosphere at room temperature. The reaction mixture was filtered through celite, and the filtrate was concentrated under reduced pressure. The resulting residue was dissolved in chloroform (18 mL) as solution A.

Solution A was cooled with ice, and chloroacetyl chloride (133.0 mg, 1.177 mmol) and triethylamine (275 mg, 2.72 mmol) were added. The reaction mixture was then stirred for 12 hours at room temperature. Water was added to the reaction mixture, which was extracted with chloroform/ethanol (4:1). The organic layer was dried with anhydrous magnesium sulfate, filtered, and concentrated under reduced pressure. The resulting residue was purified by silica gel column chromatography (hexane/ethyl acetate) to obtain the title compound (360 mg).

¹H-NMR (400 MHz, CDCl3) δ: 7.16-7.08 (1H, m), 6.34-6.24 (1H, m), 4.96-4.87 (1H, m), 4.60-4.50 (1H, m), 4.40-4.33 (1H, m), 4.30-4.21 (1H, m), 4.11-4.03 (1H, m), 3.89-3.84 (1H, m), 3.82 (2H, s), 2.14-2.01 (2H, m), 1.97-1.84 (1H, m), 1.80-1.68 (1H, m), 1.65-1.55 (1H, m), 1.50 (9H, s), 1.46 (9H, s), 1.23-1.19 (3H, m), 1.14 (3H, s), 1.08-1.00 (1H, m), 0.99-0.92 (1H, m), 0.90-0.84 (1H, m), 0.81-0.74 (1H, m), 0.68 (3H, s), 0.37-0.28 (1H, m)

Reference Example 34-2: tert-butyl 6-[(1-{[3-(benzyloxy)-2-methyl-4-oxopyridin-1(4H)-yl]acetyl}azetidin-3-yl)oxy]-2-[(tert-butoxycarbonyl)oxy]-3-{2-[(3aS,4S,6S,7aR)-3a,5,5-trimethylhexahydro-2H-4,6-methano-1,3,2-benzodioxaborol-2-yl]cyclopropyl}benzoate

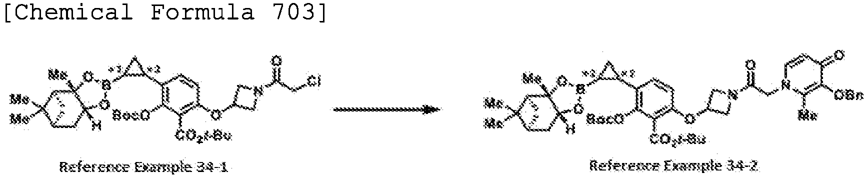

A THF (15 mL) solution of 3-benzyloxy-2-methylpyridin-4-one (137 mg, 0.363 mmol) was cooled with ice, and 60% sodium hydride (27.6 mg, 0.689 mmol) was added. The reaction mixture was stirred for 20 minutes. A THF (3 mL) solution of the compound of Reference Example 34-1 (350 mg, 0.530 mmol) was added, and the reaction mixture was then stirred for 14 hours at room temperature. The reaction mixture was further heated to 60°C and stirred for 2 hours. Water was added to the reaction mixture, which was extracted with chloroform/ethanol (4:1). The organic layer was dried with anhydrous sodium sulfate, filtered, and concentrated under reduced pressure. The resulting residue was purified by silica gel column chromatography (ethyl acetate/methanol) to obtain the title compound (150 mg).

LCMS: [M+H]⁺/Rt = 839/2.35 min^{D}

Reference Example 34: tert-butyl 2-[(tert-butoxycarbonyl)oxy]-6-({1-[(3-hydroxy-2-methyl-4-oxopyridin-1(4H)-yl)acetyl]azetidin-3-yl}oxy)-3-{2-[(3aS,4S,6S,7aR)-3a,5,5-trimethylhexahydro-2H-4,6-methano-1,3,2-benzodioxaborol-2-yl]cyclopropyl}benzoate

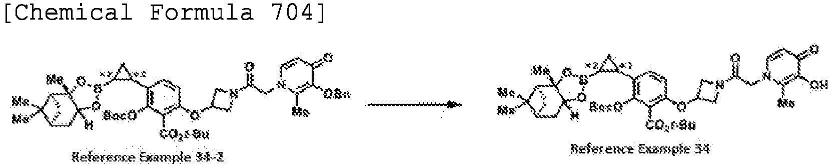

Palladium/carbon (Pd: 10%, wetted with ca. 55% water, 35 mg) was added to a methanol (8 mL) solution of the compound of Reference Example 34-2 (150.0 mg, 0.179 mmol), and the reaction mixture was stirred for 4 hours under a hydrogen atmosphere at room temperature. The reaction mixture was filtered through celite, and the filtrate was concentrated under reduced pressure to obtain the title compound (115 mg).

LCMS: [M+H]⁺/Rt = 749.54/1.289 min^{H}

Example 1: 5-({1-[amino(1H-imidazol-4-yl)acetyl]azetidin-3-yl}oxy)-2-hydroxy-1,1a,2,7b-tetrahydrocyclopropa[c][1,2]benzoxaborinine-4-carboxylic acid (derived from diastereomer 1-5-2; diastereo mixture)

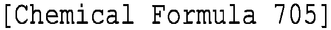

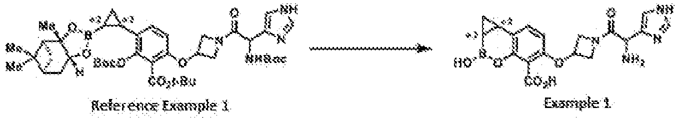

The compound of Reference Example 1 (diastereo mixture comprising two types of diastereomer) (73.7 mg, 0.091 mmol) was dissolved in 1 mol/L hydrochloric acid/acetic acid solution (0.91 mL), and phenylboronic acid (33.4 mg, 0.274 mmol) was added. The reaction mixture was stirred for 1 hour at room temperature. The reaction mixture was concentrated. The resulting residue was washed with acetonitrile, then dissolved in methanol/water and purified by reversed phase column chromatography (column: YMC-Actus Triart C18, eluent: solution A: water, solution B: acetonitrile) to obtain the title compound (23.6 mg) as a diastereo mixture comprising two types of diastereomers.

LCMS: [M+H]⁺/Rt = 399.3/0.405 min^{A}

¹H-NMR (400 MHZ, 0.02N HCl in CD₃OD) δ: 8.86 (0.5H, s), 8.84 (0.5H, s), 7.77 (0.5H, s), 7.75 (0.5H, s), 7.29 (1H, d, J = 8.5 Hz), 6.41 (0.5H, dd, J = 8.5, 1.8 Hz), 6.38 (0.5H, d, J = 8.5 Hz), 5.52 (0.5H, s), 5.46 (0.5H, d, J = 1.8 Hz), 5.13-5.09 (0.5H, m), 5.03-4.99 (0.5H, m), 4.70-4.65 (0.5H, m), 4.54 (0.5H, dd, J = 11.0, 6.7 Hz), 4.44 (0.5H, dd, J = 11.0, 6.7 Hz), 4.33-4.28 (0.5H, m), 4.25-4.20 (0.5H, m), 4.14-4.09 (0.5H, m), 4.07-4.02 (0.5H, m), 3.76-3.71 (0.5H, m), 2.26-2.21 (1H, m), 1.34-1.28 (1H, m), 0.58-0.52 (1H, m), 0.32-0.25 (1H, m).

The compound of Example 1, which is a diastereo mixture, was separated into the first peak (Rt = 3.465 min) and the second peak (Rt = 4.491 min) by the following chiral chromatography. Accordingly, each of two types of diastereomers contained in the compound of Example 1 can be further fractionated and identified.

Column: CROWNPAK CR-I(+) (0.30 cmI.D. x 15 cmL) (Daicel Corporation)
Mobile phase: aqueous perchloric acid solution (pH 1.0)/acetonitrile (85/15 <v/v>)
Flow rate: 0.5 mL/min
Temperature: 25°C

Example 2: 5-({1-[amino(1H-imidazol-4-yl)acetyl]azetidin-3-yl}oxy)-2-hydroxy-1,1a,2,7b-tetrahydrocyclopropa[c][1,2]benzoxaborinine-4-carboxylic acid (derived from diastereomer 1-5-1; diastereo mixture)

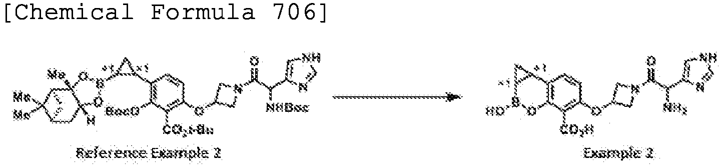

The compound of Reference Example 2 (diastereo mixture comprising two types of diastereomers) (82.5 mg, 0.102 mmol) was used as a raw material, and a reaction and post-processing were performed in accordance with the same method as the method described in Example 1 to obtain the title compound (25.1 mg) as a diastereo mixture comprising two types of diastereomers.

LCMS: [M+H]⁺/Rt = 399.14/0.413 min^{A}

¹H-NMR (400 MHZ, D₂O) δ: 7.49-7.47 (1H, m), 7.05 (0.5H, s), 7.03 (0.5H, s), 6.82 (0.51H, d, J = 8.5 Hz), 6.81 (0.5H, d, J = 8.5 Hz), 5.90 (0.5H, d, J = 8.5 Hz), 5.90 (0.5H, d, J = 8.5 Hz), 4.95 (0.5H, s), 4.94 (0.5H, s), 4.69-4.64 (0.5H, m), 4.58-4.53 (0.5H, m), 4.21-4.11 (1H, m), 4.08-4.04 (0.5H, m), 3.87-3.83 (0.5H, m), 3.79-3.71 (1H, m), 3.60-3.56 (0.5H, m), 3.29-3.25 (0.5H, m), 1.86-1.81 (1H, m), 0.91-0.86 (1H, m), 0.19-0.12 (1H, m), 0.02-0.02 (1H, m).

The compound of Example 2, which is a diastereomer mixture, was separated into the first peak (Rt = 3.446 min) and the second peak (Rt = 4.508 min) by the following chiral chromatography. Accordingly, each of two types of diastereomers contained in the compound of Example 2 can be further fractionated and identified.

Column: CROWNPAK CR-I(+) (0.30 cmI.D. x 15 cmL) (Daicel Corporation)
Mobile phase: aqueous perchloric acid solution (pH 1.0)/acetonitrile (85/15 <v/v>)
Flow rate: 0.5 mL/min
Temperature: 25°C

The compound of Example 1 and the compound of Example 2 are diastereo mixtures comprising two types of diastereomers that are different from each other, collectively consisting of four different diastereomers A1 to A4 shown in the following Table. They can be prepared separately or fractionated through chiral chromatography as described above. Specifically, the four types of diastereomers were substantially synthesized. A1 and A2 or A3 and A4 always form a pair to constitute a diastereo mixture of either Example 1 or Example 2. If Example 1 is a mixture consisting of one of the pairs, Example 2 is a mixture consisting of the other pair.

### [Chemical Formula 707]

| Serial number | Chemical structure of diastereomer [abbreviation based on configuration] | Chemical name |
|---|---|---|
| A1 | 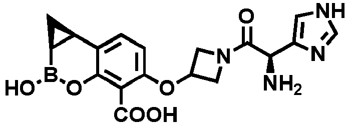 [(1aS,7bR,2R)-isomer] | (1aS,7bR)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid |
| A2 | 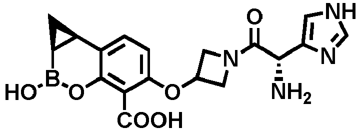 [(1aS, 7bR,2S)-isomer] | (1aS,7bR)-5-({1-[(2S)-2-amino-2-(1H-imidazol-4-yl)acetyl]azetidin-3-yl}oxy)-2-hydroxy-1,1a,2,7b-tetrahyd rocyclopropa[c] [1,2]benzoxa borinine-4-carboxylic acid |
| A3 | 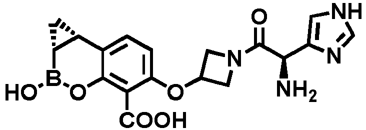 [(1aR,7bS,2R)-isomer] | (1aR,7bS)-5-({1-[(2R)-2-amino-2-(1H-imidazol-4-yl)acetyl]azetidin-3-yl}oxy)-2-hydroxy-1,1a,2,7b-tetrahyd rocyclopropa[c] [1,2]benzoxa borinine-4-carboxylic acid |
| A4 | 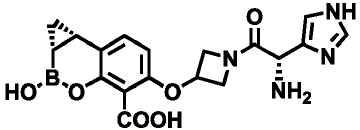 [(1aR,7bS,2S)-isomer] | (1aR,7bS)-5-({1-[(2S)-2-amino-2-(1H-imidazol-4-yl)acetyl]azetidin-3-yl}oxy)-2-hydroxy-1,1a,2,7b-tetrahyd rocyclopropa[c] [1,2]benzoxa borinine-4-carboxylic acid |

### Example 3: 9-[1-(propyl 2-amino-2-carboxylate)azetidin-3-yl]oxy-5,5-dihydroxy-6-oxa-5-boranuidatricyclo[5.4.0.0^{2,4}]undeca-1(7),8,10-triene-8-carboxylate trisodium salt (derived from diastereomer 1-5-1; single diastereomer)

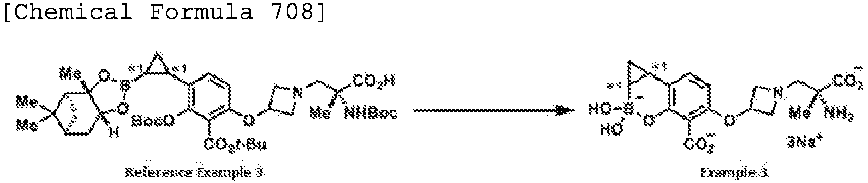

An aqueous 6 mol/L hydrochloric acid solution (0.085 mL) and phenylboronic acid (2.9 mg, 0.024 mmol) were added to a cyclopentyl methyl ether solution (0.127 mL) of the compound of Reference Example 3 (20 mg, 0.025 mmol), and the reaction mixture was stirred for 14 hours at room temperature. The supernatant solution of the reaction solution was removed, and the remaining solid was washed three times by decantation with cyclopentyl methyl ether. The resulting residue was added with an aqueous 2 mol/l sodium hydroxide solution (0.064 mL, 0.127 mmol) and purified by reversed phase column chromatography (Column: YMC-Actus Triart C18, eluent: solution A: water, solution B: acetonitrile) to obtain the title compound (4.9 mg) as a single diastereomer.

LCMS: [M+H]⁺/Rt = 377.15/0.369 min^{A}

¹H-NMR (400 MHZ, D₂O) δ: 7.00 (1H, d, J = 8.7 Hz), 6.10 (1H, d, J = 8.2 Hz), 4.73-4.67 (1H, m), 3.77 (2H, q, J = 7.3 Hz), 3.32-3.24 (2H, m), 2.90 (1H, d, J = 12.5 Hz), 2.65 (1H, d, J = 12.5 Hz), 1.80-1.75 (1H, m), 1.16 (3H, s), 0.81-0.76 (1H, m), 0.28-0.25 (1H, m), 0.20 (1H, td, J = 9.1, 6.4 Hz).

The compound of Example 3 is a single diastereomer, which is one of the two types of different diastereomers B1 and B2 shown in the following table. The other isomer can also be synthesized in the same manner as Reference Example 3 and Example 3 by using the compound of Reference Example 1-6-2 instead of the compound of Reference Example 1-6-1 in Reference Example 3-1.

### [Chemical Formula 709]

| Serial number | Chemical structure of diastereomer [abbreviation based on configuration] | Chemical name |
|---|---|---|
| B1 | 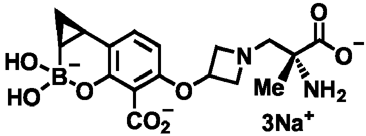 [(2R,4S,2R)-isomer] | (2R,4S)-9-[1-[propyl(2R)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate trisodium salt |
| B2 | 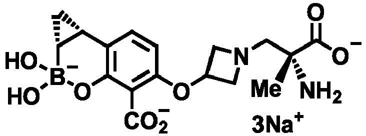 [(2S,4R,2R)-isomer] | (2S,4R)-9-[1-[propyl(2R)-2-amino-2-carboxylate]azetidin-3-yl]oxy-5,5-dihydroxy-6-oxa-5-boranuidatricyclo[5.4.0 .0^{2,4}] undeca-1(7),8,10-triene-8-carboxylate trisodium salt |

Example 4 : 5-({1-[2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid (derived from diastereomer 1-5-2; diastereo mixture)

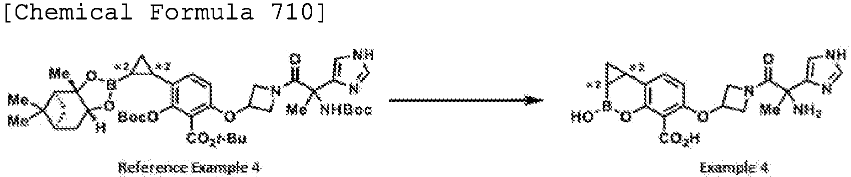

The compound of Reference Example 4 (diastereo mixture comprising two types of diastereomers) (13.5 mg, 0.016 mmol) was dissolved in a 1 mol/L hydrochloric acid/acetic acid solution (0.32 mL), and phenylboronic acid (6.2 mg, 0.051 mmol) was added. The reaction mixture was stirred for 3 hours at room temperature. The reaction mixture was concentrated. The resulting residue was washed with acetonitrile, then dissolved in triethylamine (0.01 mL)/water (0.5 mL) and purified by reversed phase column chromatography (Column: YMC-Actus Triart C18, eluent: solution A: water, solution B: acetonitrile). The eluent was lyophilized after adding triethylamine (0.01 mL) to obtain the title compound (6.5 mg) as a diastereo mixture comprising two types of diastereomers and a triethylamine containing substance.

LCMS: [M+H]⁺/Rt = 413.17/0.423 min^{A}

¹H-NMR (400 MHZ, CD₃OD) δ: 7.81 (0.5H, s), 7.78 (0.5H, s), 7.35 (0.5H, s) 7.33 (0.5H, s), 7.09 (1H, d, J = 8.5 Hz), 6.06-6.03 (1H, m), 4.79-4.76 (1H, m), 4.40-4.27 (2H, m), 4.10-3.98 (2H, m), 1.92-1.85 (1H, m), 1.84 (3H, s), 0.97-0.93 (1H, m), 0.37-0.32 (2H, m).

Example 5: 5-({1-[2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid (derived from diastereomer 1-5-1; diastereo mixture)

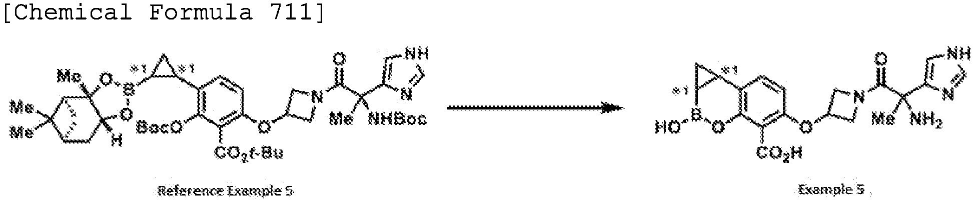

The compound of Reference Example 5 (diastereo mixture comprising two types of diastereomers) (11.8 mg, 0.014 mmol) was used as a raw material, and a reaction and post-processing were performed in accordance with the same method as the method described in Example 4 to obtain the title compound (4.7 mg) as a diastereo mixture comprising two types of diastereomers and a triethylamine containing substance (containing about 0.3 mol ratio of triethylamine with respect to the compound of Reference Example 5 according to NMR integration ratio).

LCMS: [M+H]⁺/Rt = 413.24/0.435 min^{A}

¹H-NMR (400 MHZ, CD₃OD) δ: 7.81 (0.5H, s), 7.78 (0.5H, s), 7.34 (0.5H, s), 7.31 (0.5H, s), 7.08 (1H, d, J = 8.5 Hz), 6.05-6.02 (1H, m), 4.80-4.75 (1H, m), 4.40-4.26 (2H, m), 4.07-4.00 (2H, m), 1.92-1.85 (1H, m), 1.83 (3H, s), 0.99-0.92 (1H, m), 0.38-0.31 (2H, m).

The compound of Example 4 and the compound of Example 5 are diastereo mixtures comprising two types of diastereomers that are different from each other, collectively consisting of four different diastereomers C1 to C4 shown in the following Table. They can be prepared separately or fractionated through chiral chromatography as described above. Specifically, the four types of diastereomers were substantially synthesized. C1 and C2 or C3 and C4 always form a pair to constitute a diastereo mixture of either Example 4 or Example 5. If Example 4 is a mixture consisting of one of the pairs, Example 5 is a mixture consisting of the other pair.

### [Chemical Formula 712]

| Serial number | Chemical structure of diastereomer [abbreviation based on configuration] | Chemical name |
|---|---|---|
| C1 | 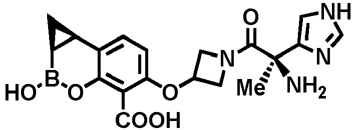 [(1aS, 7bR,2R)-isomer] | (1aS,7bR)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid |
| C2 | 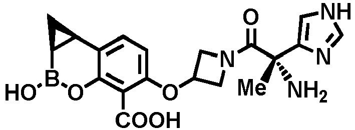 [(1aS, 7bR,2S)-isomer] | (1aS,7bR)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid |
| C3 | 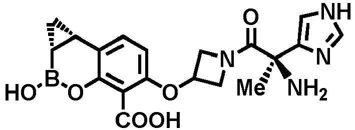 [(1aR,7bS,2R)-isomer] | (1aR,7bS)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid |
| C4 | 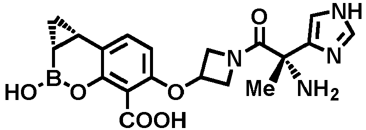 [(1aR,7bS,2S)-isomer] | (1aR,7bS)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid |

The compounds of Reference Examples (R)-6 and (S)-6 were used as a raw material, and reaction, post-processing, and purification were performed in accordance with the same method as the method described in Example 1 to obtain the following Example compounds 6 and 7.

**[Table 7]**

| Example | Raw Material | Structural formula | NMR and/or LCMS |
|---|---|---|---|
| 6 | Reference Example (R)-6 | 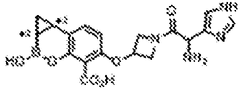 | ¹H-NMR (D₂O) δ: 7.50 (1H, br s), 7.02 (1H, br s), 6.75 (1H, d, J = 8.5 Hz), 5.77 (1H, d, J = 8.5 Hz), 4.74-4.69 (1H, m), 4.65-4.60 (1H, m), 4.34-4.25 (0.5H, m), 4.22-4.15 (0.5H, m), 4.14-4.08 (0.5H, m), 4.01-3.95 (0.5H, m), 3.88-3.83 (1H, m), 3.81-3.75 (0.5H, m), 3.60 -3.51 (0.5H, m), 1.56-1.51 (1H, m), 0.58-0.53 (1H, m), 0.01--0.07 (2H, m). (rotamer) |
| 7 | Reference Example (S)-6 | 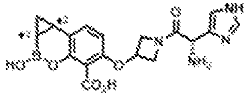 | ¹H-NMR (D₂O) δ: 7.50-7.47 (1H, m), 7.07-7.03 (1H, m), 6.81 (0.5 H, d, J = 8.5 Hz), 6.80 (0.5H, d, J = 8.5 Hz), 5.89 (0.5H, d, J = 8.5 H z), 5.88 (0.5H, d, J = 8.5 Hz), 4.96-4.94 (1H, m), 4.69-4.65 (0.5H, m), 4.59-4.55 (0.5H, m), 4.21-4.12 (1H, m), 4.09-4.04 (0.5H, m), 3.90-3.85 (0.5H, m), 3.80-3.73 (1H, m), 3.62-3.57 (0.5H, m), 3.31-3.27 (0.5H, m), 1.82-1.76 (1H, m), 0.86-0.80 (1H, m), 0.16-0.09 (1 H, m), 0.02--0.02 (1H, m). (rotamer) |

The column retention times of the compound of Example 6 and the compound of Example 7 in chiral chromatography were the following.
Column: CROWNPAK CR-I(-) (0.30 cmI.D. x 15 cmL) (Daicel Corporation)
Mobile phase: solution A/solution B = 85/15
Solution A: aqueous perchloric acid solution (70% perchloric acid/water = 1/100 VV)
Solution B: acetonitrile
Flow rate: 0.5 mL/min
Temperature: 25°C
Rt of compound of Example 6: 6.300 min
Rt of compound of Example 7: 4.235 min
Optical purity of Example 6 (computed by HPLC area percentage value): 99.3%ee
Optical purity of Example 7 (computed by HPLC area percentage value): 97.8%ee

The compound of Example 6 is a single diastereomer, which is one of the two types of different diastereomers 6A and 6B shown in the following table. The other isomer can also be synthesized in the same manner as Reference Example 1, Reference Example (R)-6, and Example 6 by using the compound of Reference Example 1-6-1 instead of the compound of Reference Example 1-6-2 in Reference Example 1-7.

### [Chemical Formula 713]

| Serial number | Chemical structure of diastereomer [abbreviation based on configuration] | Chemical name |
|---|---|---|
| 6A | 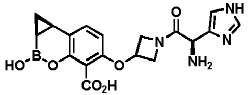 [(1aS, 7bR,2R)-isomer] | (1aS,7bR)-5-({1-[(2R)-2-amino-2-(1H-imidazol-4-yl)acetyl] azetid i n-3-yl}oxy)-2-hydroxy-1,1a,2,7b-tetrahydrocyclopropa[c][1,2]benzoxaborinine-4-carboxylic acid |
| 6B | 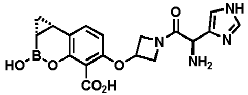 [(1aR,7bS,2S)-isomer] | (1aR,7bS)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid |

The compound of Example 7 is a single diastereomer, which is one of the two types of different diastereomers 7A and 7B shown in the following table. The other isomer can also be synthesized in the same manner as Reference Example 1, Reference Example (S)-6, and Example 7 by using the compound of Reference Example 1-6-1 instead of the compound of Reference Example 1-6-2 in Reference Example 1-7.

### [Chemical Formula 714]

| Serial number | Chemical structure of diastereomer [abbreviation based on configuration] | Chemical name |
|---|---|---|
| 7A | 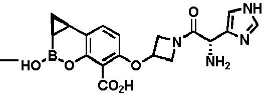 | (1aS,7bR)-5-({1-[(2S)-2-amino-2-(1H-imidazol-4-yl)acetyl]azetidin-3-yl)oxy)-2-hydroxy-1,1a,2,7b-tetrahydrocyclopropa[c][1,2]benzoxaborinine-4-carboxylic acid |
| | [(1aS, 7bR,2S)-isomer] | |
| 7B | 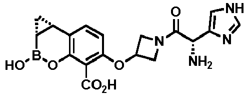 | (1aR,7bS)-5-({1-[(2S)-2-amino-2-(1H-imidazol-4-yl)acetyl]azetidin-3-yl)oxy)-2-hydroxy-1,1a,2,7b-tetrahydrocyclopropa[c][1,2]benzoxaborinine-4-carboxylic acid |
| | [(1aR,7bS,2S)-isomer] | |

Example 8: 5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid disodium salt

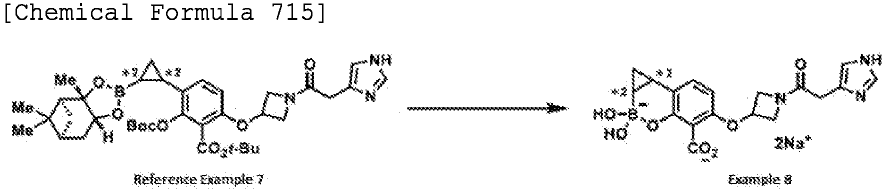

The compound of Reference Example 7 (110 mg, 0.159 mmol) was dissolved in a 1 mol/L hydrochloric acid/acetic acid solution (1.59 mL) while cooling with ice, and phenylboronic acid (58 mg, 0.477 mmol) was added. After stirring for 2 hours at room temperature, reaction mixture was concentrated. The resulting residue was washed with acetonitrile and then dissolved in water (2 mL). An aqueous 2 mol/L sodium hydroxide solution (0.318 mL) was added while cooling with ice. The reaction mixture was purified by reversed phase column chromatography (Column: YMC-Actus Triart C18, solution A: water, solution B: acetonitrile) and lyophilized to obtain the title compound (46 mg).

¹H-NMR (D₂O) δ: 7.73 (1H, s), 7.05 (1H, d, J = 8.6 Hz), 6.11 (1H, d, J = 8.6 Hz), 5.04-4.94 (1H, m), 4.63-4.59 (1H, m), 4.43-4.38 (1H, m), 4.33-4.28 (1H, m), 4.10-4.05 (1H, m), 3.36 (2H, s), 1.84-1.78 (1H, m), 0.84-0.79 (1H, m), 0.31-0.21 (2H, m)

The compound of Example 8 is a single diastereomer, which is one of the two types of different diastereomers 8A and 8B shown in the following table. The other isomer can also be synthesized in the same manner as Reference Example 7 and Example 8 by using the compound of Reference Example 1-6-1 instead of the compound of Reference Example 1-6-2 in Reference Example 7.

### [Chemical Formula 716]

| Serial number | Chemical structure of diastereomer [abbreviation based on configuration] | Chemical name |
|---|---|---|
| 8A | 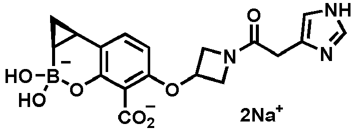 | (2R,4S)-5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid disodium salt |
| | [(2R,4S)-isomer] | |
| 8B | 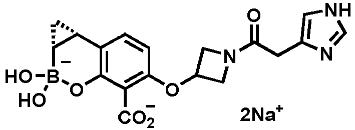 | (2S,4R)-5,5-dihydroxy-9-{1-[(1H-imidazol-4-yl)acetyl]azetidin-3-yl}oxy-6-oxa-5-boranuidatricyclo[5.4.0.0²⁻⁴]undeca-1(11),7,9-triene-8-carboxylic acid disodium salt |
| | [(2S,4R)-isomer] | |

The compounds of Reference Example 10 to 13 were used as a raw material, and a reaction, post-processing, and purification were performed in accordance with the same method as the method described in Example 8 to obtain the following Example compounds 9 to 12.

**[Table 8-1]**

| Example | Raw material | Structural formula | NMR and/or LCMS |
|---|---|---|---|
| 9 | Reference Example 10 | 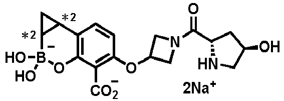 | ¹H-NMR (D₂O) δ: 7.73 (1H, brs), 7.05 (1H, d, J = 8.0 Hz), 6.12 (1H, d, J = 8.0 Hz), 5.09-5.00 (1H, m), 4.69--4.61 (1H, m), 4.47-4.29 (2H, m), 4.12-4.07 (1H, m), 3.36-3.29 (1H, m), 3.17-3.09 (1H, m), 2.3 4-2.26 (1H, m), 2.09-2.02 (1H, m), 1.91-1.79 (1H, m), 0.85-0.80 (1H, m), 0.31-0.20 (2H, m) |
| 10 | Reference Example 11 | 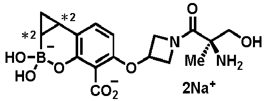 | LCMS: [M+H]⁺/RT=377.17/0.305min^{B 1}H-NMR (D₂O) δ: 6.81 (1H, d, J = 8.5 Hz), 5.87 (1 H, d, J = 8.5 Hz), 4.79-4.74 (1H, m), 4.67-4.61 (1H, m), 4.33-4.28 (1H, m), 4.20-4.14 (1H, m), 3.88-3. 82 (1H, m), 3.57 (1H, dd, J = 11.6, 7.3 Hz), 3.30 (1 H, dd, J = 11.9, 3.4 Hz), 1.57 (1H, td, J = 8.2, 3.9 H z), 1.09 (3H, s), 0.60-0.55 (1H, m), 0.07--0.04 (2H, m). |

**[Table 8-2]**

| | | | |
|---|---|---|---|
| 11 | Reference Example 12 | 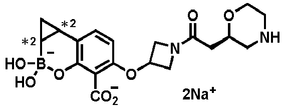 | LCMS: [M+H]⁺/RT=403.21/0.338min^{B 1}H-NMR (D₂O) δ: 6.81 (1H, d, J = 8.5 Hz), 5.88 (0. 5H, d, J = 8.5 Hz), 5.88 (0.5H, d, J = 8.5 Hz), 4.80-4.73 (1H, m), 4.40-4.34 (1H, m), 4.17-4.11 (1H, m), 4.09-4.04 (1H, m), 3.84-3.72 (3H, m), 3.54-3.4 7 (1H, m), 2.93-2.88 (1H, m), 2.86-2.81 (1H, m), 2. 73 (1H, t, J = 12.2 Hz), 2.55 (1H, t, J = 11.9 Hz), 2.2 7-2.12 (2H, m), 1.60-1.54 (1H, m), 0.60-0.55 (1H, m), 0.07--0.05 (2H, m). (rotamer) |
| 12 | Reference Example 13 | 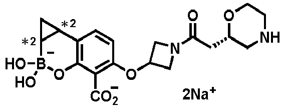 | ¹H-NMR (D₂O) δ: 6.82-6.80 (1H, m), 5.86 (1H, d, J=15.5), 4.81-4.72 (1H, m), 4.42-4.34 (1H, m), 4.19-4.01 (2H, m), 3.86-3.77 (1H, m), 3.73-3.60 (2H, m), 3.47-3.35 (1H, m), 2.76-2.67 (1H, m), 2.66-2.50 (2H, m), 2.41-2.31 (1H, m), 2.24-2.06 (2H, m), 1.61-1.53 (1H, m), 0.62-0.54 (1H, m), 0.08--0.05 (2H, m) |

The compounds of Examples 9 to 12 are single diastereomers, which are one of the two types of different diastereomers shown in the following table. The other isomer can also be synthesized in the same manner as the corresponding Reference Example and Example by using the compound of Reference Example 1-6-1 instead of the compound of Reference Example 1-6-2 in Reference Examples 10 to 13.

### [Chemical Formula 717-1]

| Example | Serial number | Chemical structure of diastereomer [abbreviation based on configuration] | Chemical name |
|---|---|---|---|
| 9 | 9A | 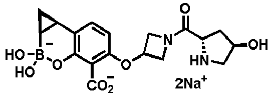 | (2R,4S)-5,5-dihydroxy-9-{1-[(4R)-4-hydroxy-L-prolyl]azetidin-3-yl}oxy-6-oxa-5-boranuidatricyclo[5.4.0.0^{2·4}]undeca-1(11),7,9-triene-8-carboxylic acid disodium salt |
| | | [(2R,4S)-isomer] | |
| | 9B | 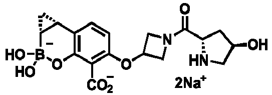 | (2S,4R)-5,5-dihydroxy-9-{1-[(4R)-4-hydroxy-L-prolyl]azetidin-3-yl}oxy-6-oxa-5-boranuidatricyclo[5.4.0.0^{2.4}]undeca-1(11),7,9-triene-8-carboxylic acid disodium salt |
| | | [(2S,4R)-isomer] | |
| 10 | 10A | 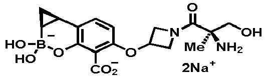 | (2R,4S)-5,5-dihydroxy-9-[1-(2-methyl-D-seryl)azetidin-3-yl]oxy-6-oxa-5-boranuidatricyclo[5.4.0.0^{2.4}]undeca-1(11),7,9-triene-8-carboxylic acid disodium salt |
| | | [(2R,4S)-isomer] | |
| | 10B | 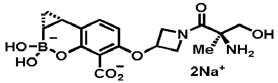 | (2S,4R)-5,5-dihydroxy-9-[1-(2-methyl-D-seryl)azetidin-3-yl]oxy-6-oxa-5-boranuidatricyclo[5.4.0.0^{2.4}]undeca-1(11),7,9-triene-8-carboxylic acid disodium salt |
| | | [(2S,4R)-isomer] | |

### [Chemical Formula 717-2]

| | | | |
|---|---|---|---|
| 11 | 11A | 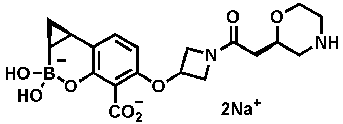 | (2R,4S)-5,5-dihydroxy-9-(1-{[(2R)-morpholin-2-yl]acetyl]azetidin-3-yl)oxy-5-boranuidatricyclo[5.4.0.0^{2,4}]undeca-1(11),7,9-triene-8-carboxylic acid disodium salt |
| | | [(2R,4S,2R)-isomer] | |
| | 11B | 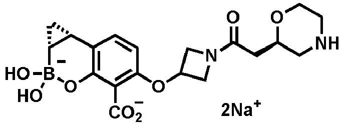 | (2S,4R)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid disodium salt |
| | | [(2S,4R,2R)-isomer] | |
| 12 | 12A | 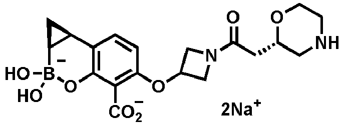 | (2R,4S)-5,5-dihydroxy-9-(1-{[(2S)-morpholin-2-yl]acetyl}azetidin-3-yl)oxy-5-boranuidatricyclo[5.4.0.0^{2.4}]undeca-1(11),7,9-triene-8-carboxylic acid disodium salt |
| | | [(2R,4S,2S)-isomer] | |
| | 12B | 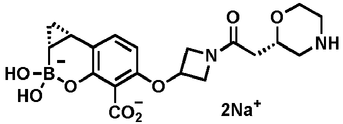 | (2S,4R)-5,5-dihydroxy-9-(1-{[(2S)-morpholin-2-yl]acetyl}azetidin-3-yl)oxy-5-boranuidatricyclo[5.4.0.0^{2·4}]undeca-1(11),7,9-triene-8-carboxylic acid disodium salt |
| | | [(2S,4R,2S)-isomer] | |

Example 13: 2-hydroxy-5-[(1-D-serylazetidin-3-yl)oxy]-1,1a,2,7b-tetrahydrocyclopropa[c][1,2]benzoxaborinine-4-carboxylic acid

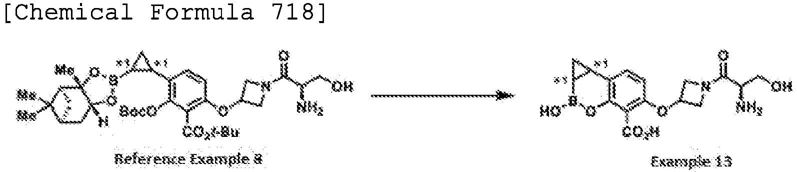

Phenylboronic acid (16.8 mg, 0.138 mmol), hexane (2 mL), and 4 mol/L hydrochloric acid/cyclopentyl methyl ether solution (1.09 mL) were added to an acetonitrile solution (1 mL) of the compound of Reference Example 8 (112 mg, 0.145 mmol), and the reaction mixture was stirred for 3.5 hours at room temperature. After leaving the reaction mixture standing, the supernatant (top layer) from the reaction mixture separated into two layers was removed, and the remaining bottom layer was washed with hexane (the washing step removes the supernatant after leaving the reaction mixture standing). The bottom layer was concentrated, and the resulting residual was purified by reversed phase column chromatography (Column: YMC-Actus Triart C18, solution A: water, solution B: acetonitrile) and lyophilized. Acetonitrile (1 mL) was added to the resulting residue. A solid was filtered out and dried under reduced pressure to obtain the title compound (7.0 mg) as a white solid.

LCMS: [M+H]⁺/Rt = 363.2/0.75 min^{G}
¹H-NMR (270 MHz, D₂O) δ: 7.00 (1H, d, J = 8.1 Hz), 6.07 (1H, d, J = 8.1 Hz), 5.00-4.59 (2H, m), 4.42-4.28 (2H, m), 4.06-4.00 (1H, m), 3.68-3.56 (3H, m), 1.80-1.70 (1H, m), 0.80-0.73 (1H, m), 0.21-0.16 (2H, m).

The compound of Example 13 is a single diastereomer, which is one of the two types of different diastereomers 13A and 13B shown in the following table. The other isomer can also be synthesized in the same manner as Reference Example 8 and Example 13 by using the compound of Reference Example 1-6-2 instead of the compound of Reference Example 1-6-1 in Reference Example 8.

### [Chemical Formula 719]

| Serial number | Chemical structure of diastereomer [abbreviation based on configuration] | Chemical name |
|---|---|---|
| 13A | 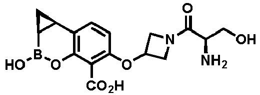 | (1aS,7bR)-2-hydroxy-5-[(1-D-serylazetidin-3-yl)oxyl-1,1a,2,7b-tetra hydrocyclopropa [c] [1,2]benzoxa borin i ne-4-carboxyl ic acid |
| | [(1aS, 7bR)-isomer] | |
| 13B | 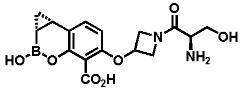 | (1aR,7bS)-2-hydroxy-5-[(1-D-serylazetidin-3-yl)oxyl-1,1a,2,7b-tetra hydrocyclopropa [c] [1,2]benzoxa borin i ne-4-carboxyl ic acid |
| | [(1aR,7bS)-isomer] | |

Example 14: 5,5-dihydroxy-9-{1-[(4R)-4-hydroxy-L-prolyl]azetidin-3-yl}oxy-6-oxa-5-boranuidatricyclo[5.4.0.0^{2,4}]undeca-1(11),7,9-triene-8-carboxylic acid disodium salt

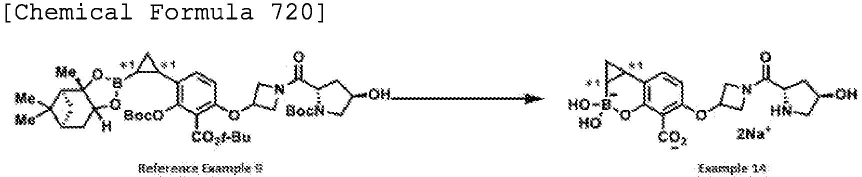

Phenylboronic acid (6.5 mg, 0.054 mmol) and a 4 mol/L hydrochloric acid/cyclopentyl methyl ether solution (0.424 mL) were added to an acetic acid solution (0.56 mL) of the compound of Reference Example 8 (45 mg, 0.056 mmol), and the reaction mixture was stirred for 2 hours at room temperature. After leaving the reaction mixture standing, the supernatant (top layer) from the reaction mixture separated into two layers was removed, and the remaining bottom layer was washed with hexane (the washing step removes the supernatant after leaving the reaction mixture standing). The bottom layer was concentrated. The resulting residue was washed with acetonitrile, and an aqueous 2 mol/L sodium hydroxide solution (0.1 mL) was added while cooling with ice. The reaction mixture was purified by reversed phase column chromatography (Column: YMC-Actus Triart C18, solution A: water, solution B: acetonitrile) and lyophilized to obtain the title compound (8.2 mg).

LCMS: [M+H]⁺/Rt = 389.4/0.88 min^{G}
¹H-NMR (270 MHz, D₂O) δ: 7.02 (1H, d, J = 8.1 Hz), 6.10-6.06 (1H, m), 5.08-4.96 (1H, m), 4.65-4.55 (2H, m), 4.44-4.02 (4H, m), 3.31-3.22 (1H, m), 3.08-2.99 (1H, m), 2.27-2.17 (1H, m), 2.04-1.93 (1H, m), 1.82-1.75 (1H, m), 0.82 (1H, t. J = 8.1 Hz), 0.28-0.15 (2H, m).

The compound of Example 14 is a single diastereomer, which is one of the two types of different diastereomers 14A and 14B shown in the following table. The other isomer can also be synthesized in the same manner as Reference Example 9 and Example 14 by using the compound of Reference Example 1-6-2 instead of the compound of Reference Example 1-6-1 in Reference Example 9.

### [Chemical Formula 721]

| Serial number | Chemical structure of diastereomer [abbreviation based on configuration] | Chemical name |
|---|---|---|
| 14A | 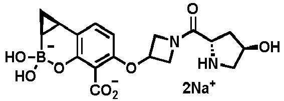 | (2R,4S)-5,5-dihydroxy-9-{1-[(4R)-4-hydroxy-L-prolyl]azetidin-3-yl}oxy-6-oxa-5-boranuidatricyclo[5.4.0.0^{2·4}]undeca-1(11),7,9-triene-8-carboxylic acid disodium salt |
| | [(2R,4S)-isomer] | |
| 14B | 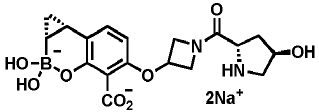 | (2S,4R)-5,5-dihydroxy-9-{1-[(4R)-4-hydroxy-L-prolyl]azetidin-3-yl}oxy-6-oxa-5-boranuidatricyclo[5.4.0.0^{2·4}]undeca-1(11),7,9-triene-8-carboxylic acid disodium salt |
| | [(2S,4R)-isomer] | |

Example 15: 9-[1-[propyl (2R)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylic acid trisodium salt

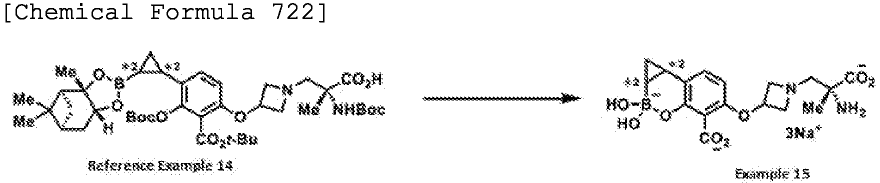

The compound of Reference Example 14 (108.8 mg, 0.139 mmol) was dissolved in 1 mol/L hydrochloric acid/acetic acid solution (2 mL), and phenylboronic acid (51 mg, 0.416 mmol) and water (0.2 mL) were added. The reaction mixture was stirred for 4 hours at room temperature. Cyclopentyl methyl ether was added to the reaction mixture. After leaving the reaction mixture standing, the supernatant (top layer) from the reaction mixture separated into two layers was removed, and the remaining bottom layer was washed with cyclopentyl methyl ether (the washing step removes the supernatant after leaving the reaction mixture standing). The bottom layer was concentrated. The resulting residue was washed with acetonitrile, and an aqueous 4 mol/L sodium hydroxide solution (0.174 mL) was added while cooling with ice. The reaction mixture was purified by reversed phase column chromatography (Column: YMC-Actus Triart C18, solution A: water, solution B: acetonitrile) and lyophilized to obtain the title compound (18.9 mg) .

¹H-NMR (D₂O) δ: 6.85 (1H, d, J = 8.5 Hz), 5.94 (1H, d, J = 8.5 Hz), 4.61-4.58 (1H, m), 3.70-3.66 (2H, m), 3.30-3.24 (2H, m), 2.93 (1H, d, J = 13.4 Hz), 2.69 (1H, d, J = 13.4 Hz), 1.65-1.60 (1H, m), 1.22 (3H, s), 0.66-0.61 (1H, m), 0.13-0.02 (2H, m).

The compound of Example 15 is a single diastereomer, which is one of the two types of different diastereomers 15A and 15B shown in the following table. The other isomer can also be synthesized in the same manner as Reference Example 14 and Example 15 by using the compound of Reference Example 1-6-1 instead of the compound of Reference Example 1-6-2 in Reference Example 14.

### [Chemical Formula 723]

| Serial number | Chemical structure of diastereomer [abbreviation based on configuration] | Chemical name |
|---|---|---|
| 15A | 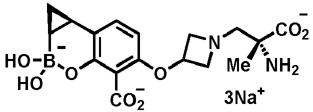 | (2R,4S)-9-[1-[propyl(2R)-2-amino-2-carboxylate]azetidin-3-yl]oxy-5,5-dihyd roxy-6-oxa-5-boranuidatricyclo[5.4.0.0^{2,4}]undeca-1(7),8,10-triene-8-carboxylic acid trisodium salt |
| | [(2R,4S)-isomer] | |
| 15B | 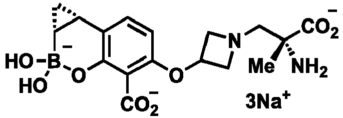 | (2S,4R)-9-[1-[propyl(2R)-2-amino-2-carboxylate]azetidin-3-yl]oxy-5,5-dihydroxy-6-oxa-5-boranuidatricyclo[5.4.0.0^{2.4}]undeca-1(7),8,10-triene-8-carboxylic acid trisodium salt |
| | [(2S,4R)-isomer] | |

Example 16: 9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylic acid disodium salt

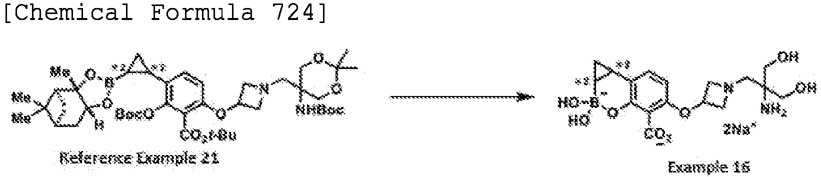

The compound of Reference Example 21 (81.4 mg, 0.098 mmol) was used as a raw material, and a reaction, post-processing, and purification were performed in accordance with the same method as the method described in Example 15 to obtain the title compound (21.8 mg) .

¹H-NMR (D₂O) δ: 6.86 (1H, d, J = 7.9 Hz), 5.95 (1H, d, J = 7.9 Hz), 4.67-4.63 (1H, m), 3.74 (2H, brs), 3.61-3.21 (8H, m), 1.65-1.61 (1H, m), 0.65-0.63 (1H, m), 0.32-0.00 (2H, m).

The compound of Example 16 is a single diastereomer, which is one of the two types of different diastereomers 16A and 16B shown in the following table. The other isomer can also be synthesized in the same manner as Reference Example 21 and Example 16 by using the compound of Reference Example 1-6-1 instead of the compound of Reference Example 1-6-2 in Reference Example 21.

### [Chemical Formula 725]

| Serial number | Chemical structure of diastereomer [abbreviation based on configuration] | Chemical name |
|---|---|---|
| 16A | 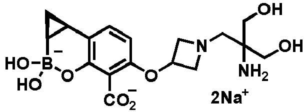 | (2R,4S)-9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylic acid disodium salt |
| | [(2R,4S)-isomer] | |
| 16B | 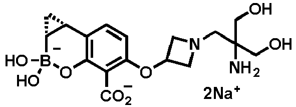 | (2S,4R)-9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylic acid disodium salt |
| | [(2S,4R)-isomer] | |

Example 17: 9-(1-{(2R)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylic acid disodium salt

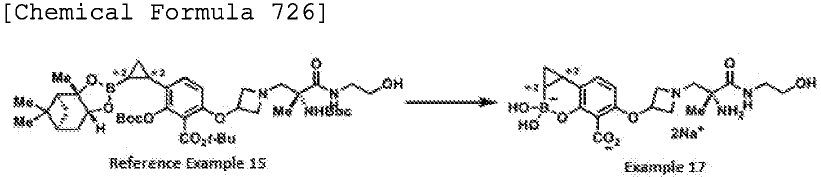

The compound of Reference Example 15 (37.6 mg, 0.045 mmol) was dissolved in a 1 mol/L hydrochloric acid/acetic acid solution (0.454 mL), and phenylboronic acid (5.7 mg, 0.047 mmol) and water (0.02 mL) were added. The reaction mixture was stirred for 1.5 hours at room temperature. The reaction mixture was concentrated. The resulting residue was washed with cyclopentyl methyl ether, and then an aqueous 2 mol/L sodium hydroxide solution (0.091 mL) was added while cooling with ice. The reaction mixture was purified by reversed phase column chromatography (Column: YMC-Actus Triart C18, solution A: water, solution B: acetonitrile) and lyophilized to obtain the title compound (6.4 mg).

LCMS: [M+H]⁺/RT = 420.20/0.425 min^{A}

¹H-NMR (D₂O) δ: 6.86 (1H, d, J = 7.9 Hz), 5.94 (1H, d, J = 7.9 Hz), 4.60-4.54 (1H, m), 3.64-3.58 (2H, m), 3.53 (2H, t, J = 5.5 Hz), 3.23-3.15 (4H, m), 2.83 (1H, d, J = 12.9 Hz), 2.55 (1H, d, J = 12.9 Hz), 1.63 (1H, td, J = 8.2, 3.7 Hz), 1.09 (3H, s), 0.67-0.62 (1H, m), 0.14-0.03 (2H, m).

The compound of Example 17 is a single diastereomer, which is one of the two types of different diastereomers 17A and 17B shown in the following table. The other isomer can also be synthesized in the same manner as Reference Examples 14 and 15 and Example 17 by using the compound of Reference Example 1-6-1 instead of the compound of Reference Example 1-6-2 in Reference Example 14.

### [Chemical Formula 727]

| Serial number | Chemical structure of diastereomer [abbreviation based on configuration] | Chemical name |
|---|---|---|
| 17A | 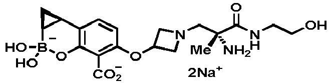 | (2R,4S)-9-(1-{(2R)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2.4}]undeca-1(7),8,10-triene-8-carboxylic acid disodium salt |
| | [(2R,4S,2R)-isomer] | |
| 17B | 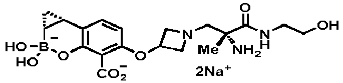 | (2S,4R)-9-(1-{(2R)- 2-a mino-3-[(2-hydroxyethyl)a mino]- 2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylic acid disodium salt |
| | [(2S,4R,2R)-isomer] | |

Example 18: 9-(1-{(2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylic acid disodium salt

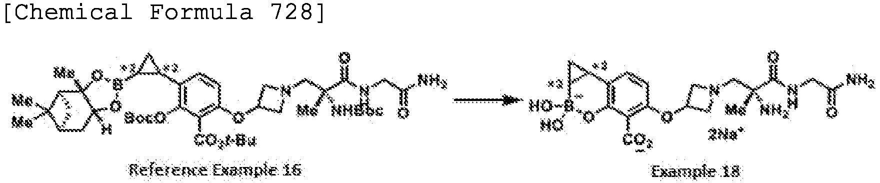

Water (0.678 mL), trifluoroacetic acid (6.8 mL, 89 mmol), and triethylsilane (0.341 mL, 2.13 mmol) were added to a mixture of the compound of Reference Example 16 (299.1 mg, 0.356 mmol) and phenylboronic acid (130 mg, 1.067 mmol) while cooling with ice, and the reaction mixture was stirred for 2 hours at room temperature. Cyclopentyl methyl ether and water were added to the reaction mixture while cooling with ice. After leaving the reaction mixture, the supernatant (top layer) from the reaction mixture separated into two layers was removed, and the remaining bottom layer was washed with cyclopentyl methyl ether (the washing step removes the supernatant after leaving the reaction mixture standing). The bottom layer was concentrated, and the resulting residual was washed with acetonitrile, and then an aqueous 2 mol/L sodium hydroxide solution (0.89 mL) was added while cooling with ice. The reaction mixture was purified by reversed phase column chromatography (Column: YMC-Actus Triart C18, solution A: water, solution B: acetonitrile) and lyophilized to obtain the title compound (6.4 mg).

¹H-NMR (D₂O) δ: 6.87 (1H, d, J = 8.5 Hz), 5.94 (1H, d, J = 8.5 Hz), 4.61-4.57 (1H, m), 3.79 (2H, s), 3.67-3.59 (2H, m), 3.26-3.22 (2H, m), 2.84 (1H, d, J = 12.8 Hz), 2.60 (1H, d, J = 12.8 Hz), 1.67-1.62 (1H, m), 1.13 (3H, s), 0.69-0.63 (1H, m), 0.15-0.02 (2H, m).

The compound of Example 18 is a single diastereomer, which is one of the two types of different diastereomers 18A and 18B shown in the following table. The other isomer can also be synthesized in the same manner as Reference Examples 14 and 16 and Example 18 by using the compound of Reference Example 1-6-1 instead of the compound of Reference Example 1-6-2 in Reference Example 14.

### [Chemical Formula 729]

| Serial number | Chemical structure of diastereomer [abbreviation based on configuration] | Chemical name |
|---|---|---|
| 18A | 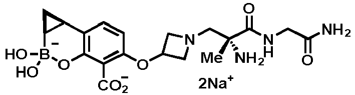 | (2R,4S)-9-(1-{(2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylic acid disodium salt |
| | [(2R,4S,2R)-isomer] | |
| 18B | 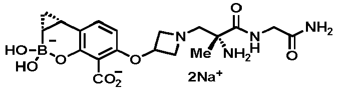 | (2S,4R)-9-(1-{(2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2.4}]undeca-1(7),8,10-triene-8-carboxylic aciddisodiumsalt |
| | [(2S,4R,2R)-isomer] | |

The compounds of Reference Examples 17, 18, and 22 to 25 were used as a raw material, and a reaction, post-processing, and purification were performed in accordance with the same method as the method described in Example 18 to obtain the following Example compounds 19 to 24.

**[Table 9-1]**

| Example | Raw Material | Structural formula | NMR and/or LCMS |
|---|---|---|---|
| 19 | Reference Example 17 | 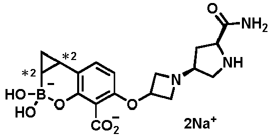 | ¹H-NMR (D20) δ: 7.01 (1H, d, J = 8.5 Hz), 6.12 (1H, d, J = 8.5 Hz), 4.83-4.73 (1H, m), 3.82-3.70 (3H, m), 3.37-3.24 (3H, m), 3.08-3.02 (1H, m), 2.76-2.72 (1H, m), 2.53-2.39 (1H, m), 1.81-1.75 (1H, m), 1.71-1.50 (1H, m), 0.82-0.77 (1H, m), 0.29-0.17 (2H, m). |
| 20 | Reference Example 18 | 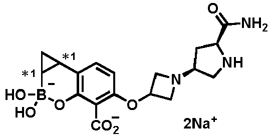 | LCMS: [M+H]⁺/Rt=388.3/0.38min^{G 1}H-NMR (270 MHz, D20) δ: 6.99 (1H, d, J = 8.1 Hz), 6.00 (1H, d, J = 8.1 Hz), 4.89-4.71 (1H, m), 3.82-3.76 (3H, m), 3.39-3.25 (3H, m), 3.06-3.00 (1H, m), 2.76-2.70 (1H, m), 2.46-2.35 (1H, m), 1.80-1.72 (1H, m), 1.58-1.47 (1H, m), 0.76 (1H, t. J = 8.1 Hz), 0.27-0.14 (2H, m). |
| 21 | Reference Example 22 | 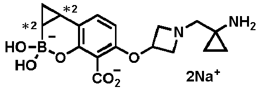 | ¹H-NMR (D20) δ: 7.05 (1H, d, J = 7.9 Hz), 6.13 (1H, d, J = 7.9 Hz), 4.95-4.88 (1H, m), 4.10-4.06 (2H, m), 3.69-3.64 (2H, m), 2.87 (2H, s), 1.83-1.78 (1H, m), 0.84-0.79 (1H, m), 0.69-0.62 (4H, m), 0.32-0.18 (2H, m). |
| 22 | Reference Example 23 | 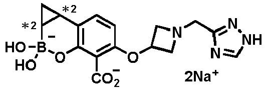 | ¹H-NMR (D20) δ: 8.36 (1H, s), 7.04 (1H, d, J = 8.5 Hz), 6.13 (1H, d, J = 8.5 Hz), 4.88-4.83 (2H, m), 4.06 (2H, s), 4.02-3.98 (2H, m), 3.63-3.62 (2H, m), 1.86-1.81 (1H, m), 0.86-0.83 (1H, m), 0.30-0.26 (2H, m). |

**[Table 9-2]**

| | | | |
|---|---|---|---|
| 23 | Reference Example 24 | 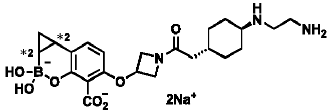 | ¹H-NMR (D20) δ: 7.04 (1H, d, J = 8.5 Hz), 6.10 (1H, d, J = 8.5 Hz), 5.00-4.96 (1H, m), 4.61-4.57 (1H, m), 4.39-4.35 (1H, m), 4.27-4.25 (1H, m), 4.04-3.99 (1H, m), 3.33-2.87 (5H, m), 2.17-2.03 (4H, m), 1.87-1.78 (3H, m), 1.71-1.65 (1H, m), 1.42-1.24 (2H, m), 1.17-1.04 (2H, m), 0.84-0.79 (1H, m), 0.29-0.19 (2H, m). |
| 24 | Reference Example 25 | 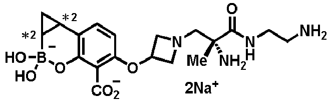 | LCMS: [M+H]⁺/RT=419.10/1.167min^{F 1}H-NMR (400 MHz, D20) δ: 7.06 (1H, d, J = 8.5 Hz), 6.12 (1H, d, J = 8.5 Hz), 3.79 (2H, m), 3.65 (1H, m), 3.55 (3H, m), 3.21 (2H, m), 3.04 (1H, d, J = 12.8 Hz), 2.75 (1H, d, J = 12.8 Hz), 1.84 (1H, td, J = 8.0, 4.4 Hz), 1.35 (3H, s), 0.86 (1H, td, J = 8.0, 2.4 Hz). 0.34 (3H, m) |

The compounds of Examples 19 to 24 are single diastereomers, which are one of the two types of different diastereomers shown in the following table. The other isomer can also be synthesized in the same manner as a corresponding Reference Example and an Example by using the compound of Reference Example 1-6-1 instead of the compound of Reference Example 1-6-2 in Reference Examples 17 and 22 to 25, or by using the compound of Reference Example 1-6-2 instead of the compound of Reference Example 1-6-1 in Reference Example 18.

### [Chemical Formula 730-1]

| Example | Serial number | Chemical structure of diastereomer [abbreviation based on configuration] | Chemical name |
|---|---|---|---|
| 19 and 20 | D1 | 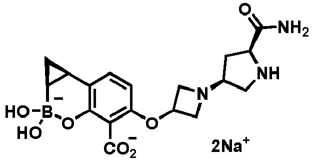 | (2R,4S)-9-{1-[(3S,5S)-5-carbamoyipyrrolidin-3-yl]azetidin-3-yl}oxy-5,5-dihydroxy-6-oxa-5-boranuidatricyclo[5.4.0.0^{2.4}]undeca-1(7),8,10-triene-8-carboxylic acid disodium salt |
| | | [(2R,4S,3S,5S)-isomer] | |
| | D2 | 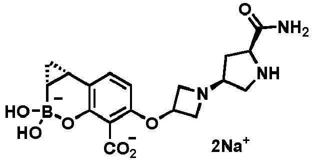 | (2S,4R)-9-{1-[(3S,5S)-5-carbamoylpyrrolidin-3-yl]azetidin-3-yl}oxy-5,5-dihydroxy-6-oxa-5-boranuidatricyclo[5.4.0.0^{2·4}]undeca-1(7),8,10-triene-8-carboxylic acid disodium salt |
| | | [(2S,4R,3S,5S)-isomer] | |
| 21 | 21A | 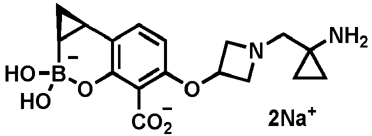 | (2R,4S)-9-{1-[(1-aminocyclopropyl)methyl]azetidin-3-yl}oxy-5,5-dihydroxy-6-oxa-5-boranuidatricyclo[5.4.0.0^{2·4}]undeca-1(7),8,10-triene-8-carboxylic acid disodium salt |
| | | [(2R,4S)-isomer] | |
| | 21B | 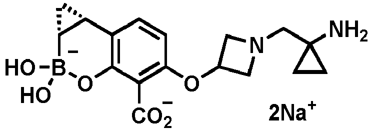 | (2S,4R)-9-{1-[(1-aminocyclopropyl)methyl]azetidin-3-yl}oxy-5,5-dihydroxy-6-oxa-5-boranuidatricyclo[5.4.0.0^{2.4}]undeca-1(7),8,10-triene-8-carboxylic acid disodium salt |
| | | [(2S,4R)-isomer] | |

### [Chemical Formula 730-2]

| | | | |
|---|---|---|---|
| 22 | 22A | 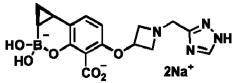 | (2R,4S)-5,5-dihydroxy-9-{1-[(1H-1,2,4-triazol-3-yl)methyl]azetidin-3-yl}oxy-5-boranuidatricyclo[5.4.0.0^{2.4}]undeca-1(11),7,9-triene-8-carboxylic acid disodium salt |
| | | [(2R,4S)-isomer] | |
| | 22B | 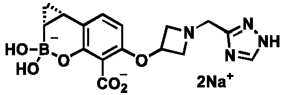 | (2S,4R)-5,5-dihydroxy-9-{1-[(1H-1,2,4-triazol-3-yl)methyl]azetidin-3-yl}oxy-5-boranuidatricyclo[5.4.0.0^{2.4}]undeca-1(11),7,9-triene-8-carboxylic acid disodium salt |
| | | [(2S,4R)-isomer] | |
| 23 | 23A | 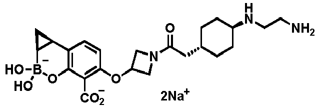 | (2R,4S)-9-[1-({(1r,4r)-4-[(2-aminoethyl)amino]cyclohexyl}acetyl)azetidin-3-yl]oxy-5,5-dihydroxy-6-oxa-5-boranuidatricyclo[5.4.0.0^{2.4}]undeca-1(7),8,10-triene-8-carboxylic acid disodium salt |
| | | [(2R,4S)-isomer] | |
| | 23B | 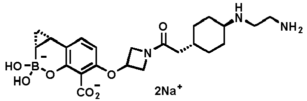 | (2S,4R)-9-[1-({(1r,4r)-4-[(2-aminoethyl)amino]cyclohexyl}acetyl)azetidin-3-yl]oxy-5,5-dihydroxy-6-oxa-5-boranuidatricyclo[5.4.0.0^{2·4}]undeca-1(7),8,10-triene-8-carboxylic acid disodium salt |
| | | [(2S,4R)-isomer] | |

### [Chemical Formula 730-3]

| | | | |
|---|---|---|---|
| 24 | 24A | 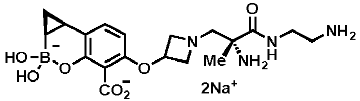 | (2R,4S)-9-(1-{(2R)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2.4}]undeca-1(7),8,10-triene-8-carboxylic acid disodium salt |
| | | [(2R,4S,2R)-isomer] | |
| | 24B | 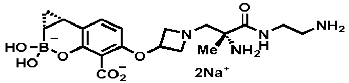 | (2S,4R)-9-(1-{(2R)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylic acid disodium salt |
| | | [(2S,4R,2R)-isomer] | |

The compounds of Reference Examples 19, 20, and 26 to 28 were used as a raw material, and a reaction, post-processing, and purification were performed in accordance with the same method as the method described in Example 13 to obtain the following Example compounds 25 to 29.

**[Table 10-1]**

| Example | Raw Material | Structural formula | NMR and/or LCMS |
|---|---|---|---|
| 25 | Reference Example 19 | 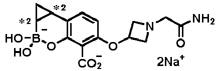 | LCMS: [M+H]⁺/RT=333.22/0.356 min^{H} |
| 26 | Reference Example 20 | 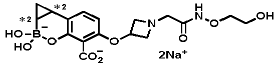 | ¹H-NMR (D₂O) δ: 6.95 (1H, d, J = 8.5 Hz), 6.04 (1H, d, J = 8.5 Hz), 4.77 (1H, t, J = 5.5 Hz), 3.94-3.90 (2H, m), 3.87 (2H, t, J = 4.6 Hz), 3.70-3.68 (2H, m), 3.52-3.46 (2H, m), 3.22 (2H, s), 1.74-1.69 (1H, m), 0.75-0.70 (1H, m), 0.22-0.19 (1H, m), 0.17-0.09 (1H, m). |
| 27 | Reference Example 26 | 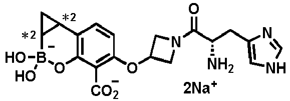 | LCMS: [M+H]⁺/Rt=413.6/0.31min^{G 1}H-NMR (270 MHz, D₂O) δ: 7.71-7.45 (1H, m), 7.03-6.99 (1H, m), 6.95-6.91 (1H, m), 6.03-5.88 (1H, m), 4.89-4.70 (1H, m), 4.43-4.14 (2H, m), 4.04-2.99 (3H, m), 2.91-2.72 (2H, m), 1.83-1.76 (1H, m), 0.84-0.78 (1H, m), 0.31-0.17 (2H, m). |
| 28 | Reference Example 27 | 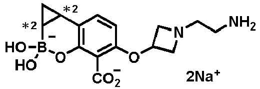 | LCMS: [M+H]+/Rt = 319.4/0.30min^{G 1}H-NMR (270 MHz, D₂O) δ: 7.02 (1H, d, J = 8.1 Hz), 6.14 (1H, d, J = 8.1 Hz), 4.90-4.70 (1H, m), 3.76-3.71 (2H, m), 3.29-3.23 (2H, m), 2.69-2.58 (4H, m), 1.83-1.75 (1H, m), 0.80 (1H, t, J = 8.1 Hz), 0.30-0.17 (2H, m). |

**[Table 10-2]**

| | | | |
|---|---|---|---|
| 29 | Reference Example 28 | 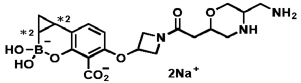 | LCMS: [M+H]+/Rt = 432.5/0.43min^{G} 1H-NMR (270 MHz, D₂O) δ: 7.05 (1H, d, J = 8.1 Hz), 6.11 (1H, d, J = 8.1 Hz), 5.04-4.96 (1H, m), 4.65-4.58 (1H, m), 4.41-4.27 (2H, m), 4.06-3.24 (4H, m), 2.98-2.30 (7H, m), 1.84-1.76 (1H, m), 0.81 (1H, t, J = 8.1 H z), 0.31-0.13 (2H, m). |

The compounds of Examples 25 to 29 are single diastereomers, which are one of the two types of different diastereomers shown in the following table. The other isomer can also be synthesized in the same manner as a corresponding Reference Example and an Example by using the compound of Reference Example 1-6-1 instead of the compound of Reference Example 1-6-2 in Reference Examples 19, 20, and 26 to 28.

### [Chemical Formula 731-1]

| Example | Serial number | Chemical structure of diastereomer [abbreviation based on configuration] | Chemical name |
|---|---|---|---|
| 25 | 25A | 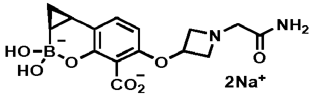 | (2R,4S)-9-[1-(2-amino-2-oxoethyl)azetidin-3-yl]oxy-5,5-dihydroxy-6-oxa-5-boranuidatricyclo[5.4.0.0^{2·4}]undeca-1(7),8,10-triene-8-carboxylic acid disodium salt |
| | | [(2R,4S)-isomer] | |
| | 25B | 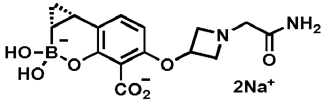 | (2S,4R)-9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylic acid disodium salt |
| | | [(2S,4R)-isomer] | |
| 26 | 26A | 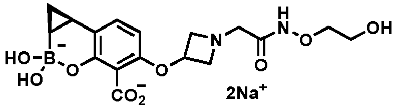 | (2R,4S)-5,5-dihydroxy-9-(1-{2-[(2-hydroxyethoxy)amino]-2-oxoethyl}azetidin-3-yl)oxy-5-boranuidatricyclo[5.4.0.0^{2A}]undeca-1(11),7,9-triene-8-carboxylic acid disodium salt |
| | | [(2R,4S)-isomer] | |
| | 26B | 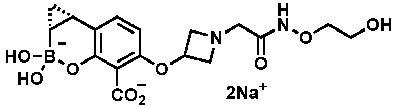 | (2S,4R)-5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid disodium salt |
| | | [(2S,4R)-isomer] | |

### [Chemical Formula 731-2]

| | | | |
|---|---|---|---|
| 27 | 27A | 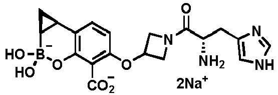 | (2R,4S)-9-(1-L-histidylazetidin-3-yl)oxy-5,5-dihydroxy-6-oxa-5-boranuidatricyclo[5.4.0.0^{2,4}]undeca-1(7),8,10-triene-8-carboxylic acid disodium salt |
| | | [(2R,4S)-isomer] | |
| | 27B | 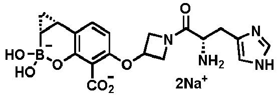 | (2S,4R)-9-(1-L-histidylazetidin-3-yl)oxy-5,5-dihydroxy-6-oxa-5-boranuidatricyclo[5.4.0.0^{2,4}]undeca-1(7),8,10-triene-8-carboxylic acid disodium salt |
| | | [(2S,4R)-isomer] | |
| 28 | 28A | 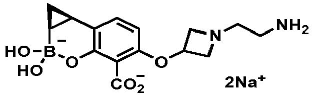 | (2R,4S)-9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid disodium salt |
| | | [(2R,4S)-isomer] | |
| | 28B | 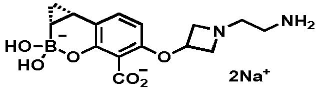 | (2S,4R)-9-[1-(2-aminoethyl)azetidin-3-yl] oxy-5,5-dihydroxy-6-oxa-5-boranuidatricyclo[5.4.0.0^{2,4}]undeca-1(7),8,10-triene-8-carboxylic acid disodium salt |
| | | [(2S,4R)-isomer] | |

### [Chemical Formula 731-3]

| | | | |
|---|---|---|---|
| 29 | 29A | 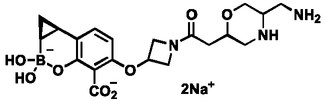 | (2R,4S)-9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid disodium salt |
| | | [(2R,4S)-isomer] | |
| | 29B | 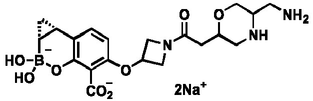 | (2S,4R)-9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid disodium salt |
| | | [(2S,4R)-isomer] | |

The compounds of Reference Examples 31 to 34 were used as a raw material, and a reaction, post-processing, and purification were performed in accordance with the same method as the method described in Example 18 to obtain the following Example compounds 30 to 34.

**[Table 11-1]**

| Example | Raw Material | Structural formula | NMR and/or LCMS |
|---|---|---|---|
| 30 | Reference Example 31 | 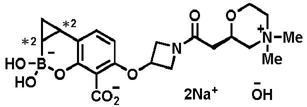 | ¹H-NMR (400 MHz, D₂O) δ: 6.89 (1H, d, J = 8.5 Hz), 5.97-5.94 (1H, m), 4.87-4.82 (1H, m), 4.47-4.42 (1H, m), 4.28-4.21 (2H, m), 4.17-4.11 (1H, m), 3.97-3.87 (3H, m), 3.41-3.33 (3H, m), 3.20-3.16 (1H, m), 3.13 (3H, s), 3.10 (3H, s), 2.41-2.24 (2H, m), 1.67-1.62 (1H, m), 0.68-0.63 (1H, m), 0.14-0.01 (2H, m). |
| 31 | Reference Example 32 | 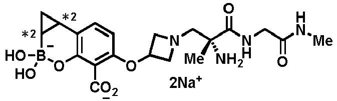 | ¹H-NMR (400 MHz, D₂O) δ: 6.85 (1H, d, J = 8.5 Hz), 5.92 (1H, d, *J* = 8.5 Hz), 4.62-4.56 (1H, m), 3.73 (2H, s), 3.65-3.58 (2H, m), 3.21-3.17 (2H, m), 2.83 (1H, d, J = 13.4 Hz), 2.59-2.55 (4H, m), 1.65-1.60 (1H, m), 1.10 (3H, s), 0.66-0.61 (1H, m), 0.13-0.02 (2H, m). |
| 32 | Reference Example 33 | 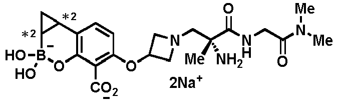 | ¹H-NMR (400 MHz, D₂O) δ: 6.85 (1H, d, J = 8.5 Hz), 5.94 (1H, d, J = 8.5 Hz), 4.60-4.55 (1H, m), 4.00-3.90 (2H, m), 3.65-3.58 (2H, m), 3.18-3.15 (2H, m), 2.91 (3H, s), 2.83 (1H, d, J = 13.4 Hz), 2.80 (3H, s), 2.57 (1H, d, J = 13.4 Hz), 1.65-1.60 (1H, m), 1.11 (3H, s), 0.66-0.61 (1H, m), 0.13-0.00 (2H, m). |

**[Table 11-2]**

| | | | |
|---|---|---|---|
| 33 | Reference Example 31-5 | 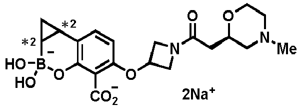 | ¹H-NMR (400 MHz, D₂O) δ: 6.82 (1H, d, J = 8.0 Hz), 5.88 (1H, d, J = 8.4 Hz), 4.78-4.71 (1H, m), 4.38 (1H, m), 4.14 (1H, m), 4.07 (1H, m), 3.81 (1H, m), 3.68 (2H, m), 3.40 (1H, m), 2.06 (1H, d, J = 13.6 Hz), 2.52 (1H,d, J = 11.2 Hz), 2.19 (1H, m), 2.03 (3H, s), 1.78(2H, m), 1.68 (1H, m), 0.57 (1H, m), 0.05-0.01 (2H, m). |
| 34 | | 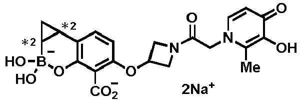 | LCMS: [M+H]⁺/Rt=441.3/0.584min^{H 1}H-NMR (400 MHz, D₂O) δ: 7.38-7.27 (1H, m), 6.87-6.78 (1H, m), 6.33-6.24 (1H, m), 5.93-5.84 (1H, m), 4.90-4.81 (1H, m), 4.74-4.66 (2H, s), 4.49-4.37 (1H, m), 4.29-4.21 (1H, m), 4.20-4.08 (1H, m), 3.97-3.86 (1H, m), 2.12-2.02 (3H, s), 1.62-1.51 (1H, m), 0.62-0.52 (1H, m), 0.09-0.04 (2H, m) |

The compounds of Examples 30 to 34 are single diastereomers, which are one of the two types of different diastereomers shown in the following table. The other isomer can also be synthesized in the same manner as a corresponding Reference Example and an Example by using the compound of Reference Example 1-6-1 instead of the compound of Reference Example 1-6-2 in Reference Examples 31 to 34.

### [Chemical Formula 732-1]

| Example | Serial number | Chemical structure of diastereomer [abbreviation based on configuration] | Chemical name |
|---|---|---|---|
| 30 | 30A | 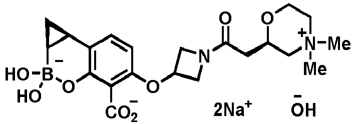 | (2R,4S)-9-[1-[2-[(2R)-4,4-dimethylmorpholin-4-ium-2-yl]acetyl]azetidin-3-yl]oxy-5,5-dihydroxy-6-oxa-5-boranuidatricyclo[5.4.0.0^{2,4}]undeca-1(7),8,10-triene-8-carboxylate hydroxide disodium salt |
| | | [(2R,4S,2R)-isomer] | |
| | 30B | 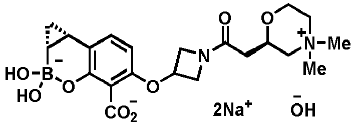 | (2S,4R)-9-[1-[2-[(2R)-4,4-dimethylmorpholin-4-ium-2-yl]acetyl]azetidin-3-yl]oxy-5,5-dihydroxy-6-oxa-5-boranuidatricyclo[5.4.0.0^{2,4}]undeca-1(7),8,10-triene-8-carboxylate hydroxide disodium salt |
| | | [(2S,4R,2R)-isomer] | |
| 31 | 31A | 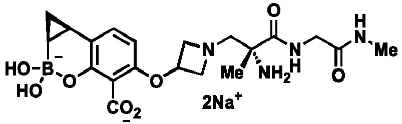 | (2R,4S)-9-[1-[(2R)-2-amino-2-methyl-3-[[2-(methylamino)-2-oxoethyl]amino]-3-oxopropyl]azetidin-3-yl]oxy-5,5-dihydroxy-6-oxa-5-boranuidatricyclo[5.4.0.0^{2,4}]undeca-1(7),8,10-triene-8-carboxylate disodium salt |
| | | [(2R,4S,2R)-isomer] | |

### [Chemical Formula 732-2]

| | | | |
|---|---|---|---|
| | 31B | 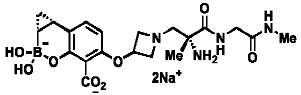 | (2S,4R)-9-[1-[(2R)-2-a mino-2-methyl-3-[[2-(methylamino)-2-oxoethyl]amino]-3-oxopropyl]azetidin-3-yl]oxy-5,5-dihydroxy-6-oxa-5-boranuidatricyclo[5.4.0.0^{2,4}]undeca-1(7),8,10-triene-8-carboxylate disodium salt |
| | | [(2S,4R,2R)-isomer] | |
| 32 | 32A | 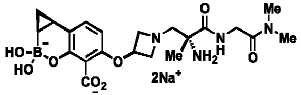 | (2R,4S)-9-[1-[(2R)-2-amino-3-[[2-(dimethylamino)-2-oxoethyl]amino]-2-methyl-3-oxopropyl]azetidin-3-yl]oxy-5,5-dihydroxy-6-oxa-5-boranuidatricyclo[5.4.0.0^{2,4}]undeca-1(7),8,10-triene-8-carboxylate disodium salt |
| | | [(2R,4S,2R)-isomer] | |
| | 32B | 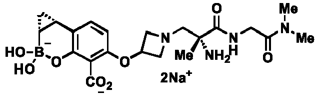 | (2S,4R)-9-[1-[(2R)-2-amino-3-[[2-(dimethylamino)-2-oxoethyl]amino]-2-methyl-3-oxopropyl]azetidin-3-yl]oxy-5,5-dihydroxy-6-oxa-5-boranuidatricyclo[5.4.0.0^{2,4}]undeca-1(7),8,10-triene-8-carboxylate disodium salt |
| | | [(2S,4R,2R)-isomer] | |

### [Chemical Formula 732-3]

| | | | |
|---|---|---|---|
| 33 | 33A | 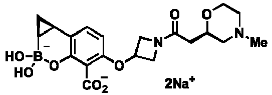 | (2R,4S)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid disodium salt |
| | | [(2R,4S,2R)-isomer] | |
| | 33B | 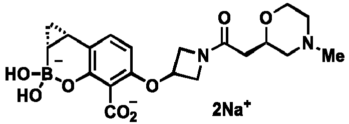 | (2S,4R)-5,5-dihydroxy-9-[(1-{[(2R)-4-methylmorpholin-2-yl]acetyl}azetidin-3-yi) oxyl-5-bo ra nu id atricycl o[5.4.0.0^{2,4}] u ndeca-1(11),7,9-triene-8-carboxylic acid disodium salt |
| | | [(2S,4R,2R)-isomer] | |
| 34 | 34A | 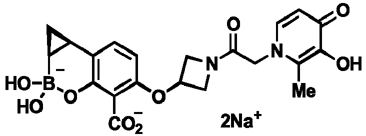 | (2R,4S)-5,5-dihydroxy-9-({1-[(3-hydroxy-2-methyl-4-oxopyridin-1(4H)-yl)acetyl]azetid in-3-yl}oxy)-5-boranuidatricyclo[5.4.0.0^{2,4}]undeca-1(11),7,9-triene-8-carboxylic acid disodium salt |
| | | [(2R,4S)-isomer] | |
| | 34B | 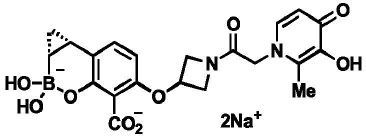 | (2S,4R)-5,5-dihydroxy-9-({1-[(3-hydroxy-2-methyl-4-oxopyridin-1(4H)-yl)acetyl]azetidin-3-yl}oxy)-5-boranuidatricyclo[5.4.0.0^{2,4}]undeca-1(11),7,9-triene-8-carboxylic acid disodium salt |
| | | [(2S,4R)-isomer] | |

Test Example 1: To evaluate the β-lactamase inhibitory activity of test compounds evaluated for the minimum inhibitory concentration (MIC) of MEPM against β-lactamase producing bacteria, the effect of concomitant use of the test compound and a β-lactam agent against β-lactamase producing bacteria was evaluated. Meropenem (MEPM) was used as a β-lactam antimicrobial agent. The minimum inhibitory concentration (MIC) of MEPM against β-lactamase producing bacteria when a test compound was added at a fixed concentration (4 µg/ mL) was measured by broth microdilution method (common ratio: 2).

The numerical values of (MIC of MEPM in combination with a test compound)/(MIC of MEPM alone) are shown below.

Pharmacological testing methods and results thereof for representative compounds of the invention are shown hereinafter, but the present invention is not limited to the Test Examples.

### Test Example 1: Evaluation of minimum inhibitory concentration (MIC) of MEPM against β-lactamase producing bacteria

To evaluate the β-lactamase inhibitory activity of test compounds, the effect of combination of a test compound and a β-lactam agent against β-lactamase producing bacteria was evaluated. Meropenem (MEPM) was used as a β-lactam antimicrobial agent. The minimum inhibitory concentration (MIC) of MEPM against β-lactamase producing bacteria when a test compound was added at a fixed concentration (4 µg/ mL) was measured by broth microdilution method (common ratio: 2).

The numerical values of (MIC of MEPM in combination with a test compound)/(MIC of MEPM alone) are shown below.

**[Table 3-1]**

| Example number | K. pneumoniae ATCC BAA-2344 (KPC) | K. pneumoniae ATCC BAA-2524 (OXA-48) |
|---|---|---|
| 1 | ≤ 0.063/32 | ≤ 0.063/2 |
| 2 | 0.125/32 | ≤ 0.063/2 |
| 3 | ≤ 0.063/32 | ≤ 0.063/2 |
| 4 | ≤ 0.063/32 | ≤ 0.063/2 |
| 5 | ≤ 0.063/32 | ≤ 0.063/2 |

**[Table 3-2]**

| **Example number** | **K. pneumoniae ATCC BAA-2344 (KPC)** | **K. pneumoniae ATCC BAA-2524 (OXA-48)** |
|---|---|---|
| 6 | 0.031/32 | 0.063/2 |
| 7 | 0.031/32 | 0.063/2 |
| 8 | 0.031/32 | 0.031/2 |
| 9 | 0.031/32 | 0.063/2 |
| 10 | 0.031/32 | 0.063/2 |
| 11 | 0.031/32 | 0.063/2 |
| 12 | 0.125/32 | 0.031/1 |
| 13 | 0.031/32 | 0.031/2 |
| 14 | 0.031/32 | 0.031/2 |
| 15 | 0.031/32 | 0.031/2 |
| 16 | 0.031/32 | 0.031/2 |
| 17 | 0.031/32 | 0.031/1 |
| 18 | 0.063/32 | 0.063/2 |
| 19 | 0.031/32 | 0.031/2 |
| 20 | 0.031/32 | 0.031/2 |
| 21 | 0.031/32 | 0.031/2 |
| 22 | 0.031/32 | 0.031/2 |
| 23 | 0.25/32 | 0.031/2 |
| 24 | 0.031/32 | 0.063/1 |
| 25 | 0.063/32 | 0.063/2 |
| 26 | 0.031/32 | 0.063/2 |
| 27 | 0.031/32 | 0.031/2 |
| 28 | 0.031/32 | 0.031/1 |
| 29 | 0.063/32 | 0.031/2 |
| 30 | 0.031/32 | 0.031/2 |
| 31 | 0.031/32 | 0.031/2 |
| 32 | 0.031/32 | 0.031/2 |
| 33 | 0.031/32 | 0.031/2 |

### Test Example 2: Evaluation of minimum inhibitory concentration (MIC) of MEPM against β-lactamase producing bacteria

In the same manner as Test Example 1, E. coli ATCC BAA-2340 (KPC), E. coli ATCC BAA-2469 (NDM-1), K. pneumomiae ATCC BAA-2470 (NDM-1), K. pneumomiae NCTC 13439 (VIM-1), K. pneumomiae NCTC 13440 (VIM-1), E. coli NCTC 13476 (IMP), and the like can be used to evaluate metallo-p-lactamase inhibitory activity of test compounds.

As disclosed above, the present invention is exemplified by the use of its preferred embodiments. However, it is understood that the scope of the present invention should be interpreted based solely on the Claims. The present application claims priority to Japanese Patent Application No. 2019-194753 (filed on October 25, 2019). The entire content thereof is incorporated herein by reference. It is also understood that any patent, any patent application, and any other references cited herein should be incorporated herein by reference in the same manner as the contents are specifically described herein.

### [Industrial Applicability]

The compound of the invention exhibits a potent inhibitory action against β-lactamase and is useful as a therapeutic agent and/or prophylactic agent for sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, deep skin infection, lymphangitis/lymphadenitis, secondary infection of trauma, burn injury, surgical wound, or the like, urinary tract infection, genital infection, eye infection, or odontogenic infection.

## Claims

1. A compound represented by formula (1a) or (1b): 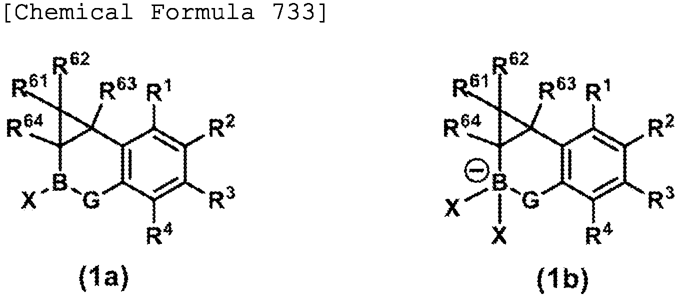 or a pharmaceutically acceptable salt thereof
wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 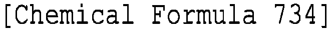 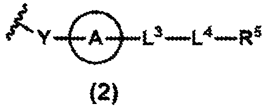 wherein
Y is an oxygen atom, a sulfur atom, or -NR^{j}-, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, optionally substituted -NH-, optionally substituted -NH-SO₂-, -S(=O)-, or -S(=O)₂-,
L⁴ is a single bond, an optionally substituted hydrocarbylene group, an optionally substituted heterohydrocarbylene group, or - C (=N-OR^{h1})-,
R^{h1} is
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted),
R⁵ is a hydrogen atom, a hydroxyl group, an optionally substituted hydrocarbyl group, an optionally substituted heterohydrocarbyl group, optionally substituted -NHOH, a sulfo group (sulfonic acid group), optionally substituted -SO₂H, optionally substituted -SO₂-NH₂, optionally substituted - S(=O)(=NH)H, optionally substituted -NH-SO₂-H, optionally substituted -NH-SO₂-NH₂, optionally substituted -N=S(=O)H₂, or optionally substituted -NH₂,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
1) -C (=O) R⁸,
2) -SO₂-L⁶-R⁸,
(wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene group),
3) -NR^{a4}R^{b3},
4) -B (OR^{m1})₂,
5) -PO(OR^{m1}) (OR^{m2}),
6) optionally substituted heteroaryl,
7) an optionally substituted non-aryl heterocycle, or
8) a bioisostere of one of 1) to 7),
(wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

2. A compound represented by formula (1a) or (1b): 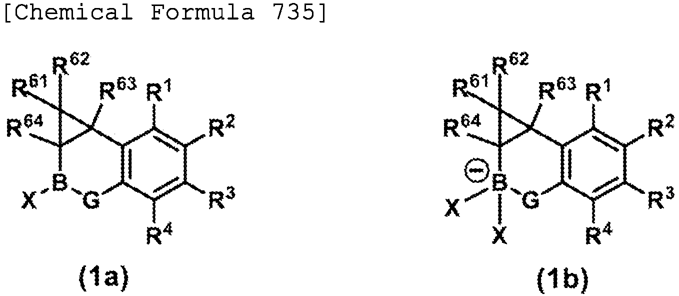 or a pharmaceutically acceptable salt thereof wherein
G is an oxygen atom, a sulfur atom, or -NR^{a1}-,
X is a hydroxyl group, an optionally substituted alkoxy group, or -NR^{a2}R^{b1},
R^{a1}, R^{a2}, and R^{b1} are the same or different, each independently a hydrogen atom, a hydroxyl group, an optionally substituted hydrocarbyl group, or an optionally substituted heterohydrocarbyl group,
wherein R^{a2} and R^{b1} together may form an optionally substituted nitrogen-containing non-aryl heterocycle,
L¹ is a single bond, an oxygen atom, a sulfur atom, -SO-, - SO₂-, an optionally substituted hydrocarbylene group, or an optionally substituted heterohydrocarbylene group,
L² is a single bond or an optionally substituted hydrocarbylene group,
Z is a hydrogen atom, a hydroxyl group, an optionally substituted hydrocarbyl group, or an optionally substituted heterohydrocarbyl group,
one of R¹, R², and R³ is represented by formula (2) : 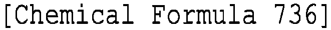 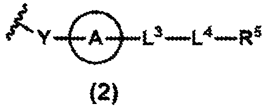 wherein
Y is an oxygen atom, a sulfur atom, or -NR^{j}-, and R^{j} is a hydrogen atom, a hydroxyl group, or an optionally substituted hydrocarbyl group,
ring A is an optionally substituted non-aryl heterocycle,
L³ is an oxygen atom, a sulfur atom, an optionally substituted hydrocarbylene group, an optionally substituted heterohydrocarbylene group, -S(=O)-, or -S(=O)₂-,
L⁴ is a single bond, an optionally substituted hydrocarbylene group, or -C(=N-OR^{h1})-,
R^{h1} is
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted),
R⁵ is a hydrogen atom, a hydroxyl group, an optionally substituted hydrocarbyl group, or an optionally substituted heterohydrocarbyl group,
the remaining two (without the structure of formula (2) among R¹, R², and R³) are the same or different, each independently a hydrogen atom, halogen, a hydroxyl group, an optionally substituted hydrocarbyl group, or an optionally substituted heterohydrocarbyl group,
R⁴ is
1) -C (=O) R⁸,
2) -SO₂-L⁶-R⁸,
(wherein R⁸ in 1) and 2) is a hydroxyl group, an optionally substituted hydrocarbyl group, or an optionally substituted heterohydrocarbyl group, and L⁶ is a single bond or an optionally substituted hydrocarbylene,
3) -NR^{a4}R^{b3},
4) -B(OR^{m1})₂,
5) -PO(OR^{m1}) (OR^{m2}),
6) optionally substituted heteroaryl,
7) an optionally substituted non-aryl heterocycle, or
8) a bioisostere of one of 1) to 7),
(wherein the formulas of 1), 2), 4), 5), and 6) include a carboxylic acid isostere, and 8) may include them in duplicates),
R^{a4} and R^{b3} are the same or different, each independently having the same definition as R^{a1}, R^{a2}, and R^{b1}, wherein a combination of R^{a4} and R^{b3}, when attached to the same nitrogen atom, together may form an optionally substituted nitrogen-containing non-aryl heterocycle,
R^{m1} is a hydrogen atom or an optionally substituted hydrocarbyl group,
wherein if R^{m1} is attached to a boron atom via an oxygen atom, two R^{m1}, as alkylene, together with the boron atom and two oxygen atoms, may form a non-aryl heterocycle (wherein an alkylene moiety is optionally substituted in the non-aryl heterocycle),
R^{m2} is a hydrogen atom or an optionally substituted hydrocarbyl group, and
R⁶¹, R⁶², R⁶³, and R⁶⁴ are each independently a hydrogen atom, halogen, an optionally substituted alkyl group, or -L¹-L²-Z.

3. The compound or the pharmaceutically acceptable salt thereof according to claim 1 or 2, represented by formula (1a) or (1b): 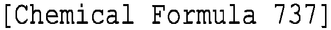 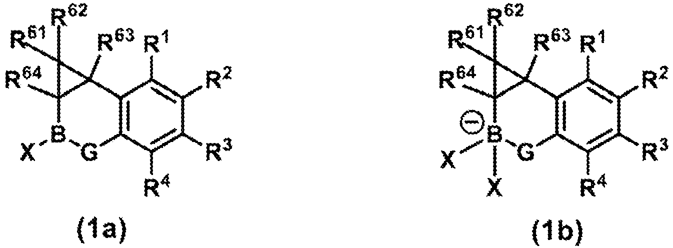 wherein
G is an oxygen atom, a sulfur atom, or -NR^{a1}-,
X is a hydroxyl group, an optionally substituted C₁₋₆ alkoxy group, or -NR^{a2}R^{b1},
R^{a1}, R^{a2}, and R^{b1} are the same or different, each independently
1) a hydrogen atom,
2) a C₁₋₆ alkyl group,
3) a C₃₋₁₀ alicyclic group,
4) C₆₋₁₀ aryl,
5) 5- or 6-membered heteroaryl,
6) a 4- to 10-membered non-aryl heterocycle,
7) a C₁₋₆ alkylcarbonyl group,
8) a C₃₋₁₀ alicyclic carbonyl group,
9) a C₆₋₁₀ arylcarbonyl group,
10) a 5- or 6-membered heteroarylcarbonyl group,
11) a C₁₋₆ alkylsulfonyl group,
12) a C₃₋₁₀ alicyclic sulfonyl group,
13) a C₆₋₁₀ arylsulfonyl group,
14) a 5- or 6-membered heteroarylsulfonyl group, or
15) -OR^{c1},
(wherein each substituent from 2) to 14) is optionally substituted),
wherein R^{a2} and R^{b1} together may form an optionally substituted 4- to 10-membered nitrogen-containing non-aryl heterocycle,
R^{c1} is
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted),
L¹ is a single bond, an oxygen atom, a sulfur atom, -SO-, - SO₂-, -NR^{d1}-, -NR^{d1}C(=O) -, or -NR^{d1}SO₂-,
L² is a single bond or an optionally substituted C₁₋₆ alkylene group,
Z is
1) a hydrogen atom,
2) a hydroxyl group,
3) a cyano group,
4) a carboxyl group,
5) a C₃₋₁₀ alicyclic group,
6) C₆₋₁₀ aryl,
7) 5- or 6-membered heteroaryl,
8) a 4- to 10-membered non-aryl heterocycle,
9) a C₁₋₆ alkoxy group,
10) a C₃₋₁₀ alicyclic oxy group,
11) a C₆₋₁₀ aryloxy group,
12) a 5- or 6-membered heteroaryloxy group,
13) a 4- to 10-membered non-aryl heterocyclyl oxy group,
14) a C₁₋₆ alkylthio group,
15) a C₃₋₁₀ alicyclic thio group,
16) a C₆₋₁₀ arylthio group,
17) a 5- or 6-membered heteroarylthio group,
18) a 4- to 10-membered non-aryl heterocyclyl thio group,
(wherein each substituent from 5) to 18) is optionally substituted),
19) -SO₂-NR^{e1}R^{f1},
20) -NR^{e1-}C(=O) OR^{f1},
21) -NR^{g1}-C(=O) NR^{e1}R^{f1},
22) -NR^{e1-}C (=S)R^{f1},
23) -NR^{e1-}C (=S)OR^{f1},
24) -NR^{g1}-C(=S)NR^{e1}R^{f1},
25) -NR^{g1}-CR^{e1}(=NR^{f1}),
26) -NR^{g1}-CR^{e1}(=N-OR^{f1}),
27) -NR^{h1}-C(=NR^{g1})NR^{e1}R^{f1},
28) -NR^{h1}-C(=N-OR^{g1})NR^{e1}R^{f1},
29) -NRⁱ¹-C(=NR^{h1})NR^{g1}-NR^{e1}R^{f1},
30) -NRⁱ¹-C(=N-OR^{h1})NR^{g1}-NR^{e1}R^{f1},
31) -NR^{e1}-SO₂-R^{f1},
32) -NR^{g1}-SO₂-NR^{e1}R^{f1},
33) -C(=O)OR^{e1},
34) -C(=S)OR^{e1},
35) -C(=S)NR^{e1}R^{f1},
36) -C(=S)NR^{e1}OR^{f1},
37) -C (=S)NR^{g1}-NR^{e1}R^{f1},
38) -C(=NR^{e1})R^{f1},
39) -C(=N-OR^{e1})R^{f1},
40) -C(=NR^{h1})NR^{g1}-NR^{e1}R^{f1},
41) -C(=N-OR^{h1})NR^{g1}-NR^{e1}R^{f1},
42) -NR^{e1}R^{f1},
43) -NR^{g1}-NR^{e1}R^{f1},
44) -NR^{e1}OR^{f1},
45) -NR^{e1-}C (=O) R^{f1},
46) -C(=O) NR^{e1}R^{f1},
47) -C(=O)NR^{e1}OR^{f1},
48) -C(=O)NR^{g1}-NR^{e1}R^{f1},
49) -C(=O) R^{e1},
50) -C(=NR^{g1})NR^{e1}R^{f1}, or
51) -C(=N-OR^{h1})NR^{e1}R^{f1},
one of R¹, R², and R³ is a group represented by formula (2) : 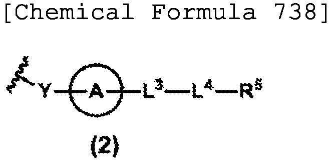 wherein
Y is an oxygen atom, a sulfur atom, or -NR^{j}-,
ring A is an optionally substituted 4- to 20-membered non-aryl heterocycle,
L³ is
1) an oxygen atom,
2) a sulfur atom,
3) -NR^{d2}-,
4) -NR^{d2}C(=O) -,
5) -NR^{d2}SO₂-,
6) a C₁₋₆ alkylene group,
7) a C₃₋₁₀ cycloalkylene group,
8) a 4- to 10-membered non-aryl heterocyclylene group, (wherein each substituent from 6) to 8) is optionally substituted),
9) -C(=O)-,
10) -S(=O)-, or
11) -S(=O)₂-,
L⁴ is
1) a single bond,
2) a C₁₋₆ alkylene group,
3) a C₃₋₁₀ cycloalkylene group,
4) a C₆₋₁₀ arylene group,
5) a 5- or 6-membered heteroarylene group,
6) a 4- to 10-membered non-aryl heterocyclylene group, (wherein each substituent from 2) to 6) is optionally substituted), or
7) -C(=N-OR^{h1})-,
R⁵ is
1) a hydrogen atom,
2) a C₁₋₆ alkyl group,
3) a C₃₋₁₀ alicyclic group,
4) a 4- to 10-membered non-aryl heterocycle,
5) C₆₋₁₀ aryl,
6) 5- to 10-membered heteroaryl,
7) a C₁₋₆ alkylthio group,
(wherein each substituent from 2) to 7) is optionally substituted, and if two substituents further substituted with the substituent of 1), 2), or 3) are each substituted on adjacent atoms within a ring, the two substituents together may further form a condensed ring structure),
8) -NR^{e1}OH,
9) a carboxyl group (-C(=O)OH),
10) a carboxylic acid isostere (wherein the carboxylic acid isostere comprises an ester group-C(=O)OR^{20a}),
11) a sulfo group (sulfonic acid group),
12) -SO₂R^{e1},
13) -SO₂-NR^{e1}R^{f1},
14) -S(=O) (=NR^{f1})R^{e1},
15) -NR^{e1-}C (=O)R^{f1},
16) -NR^{e1-}C (=O)OR^{f1},
17) -NR^{g1}-C(=O) NR^{e1}R^{f1},
18) -NR^{e1}-SO₂-R^{f1},
19) -NR^{g1}-SO₂-NR^{e1}R^{f1},
20) -N=S(=O)R^{e1}R^{f1},
21) -C(=O)NR⁵⁰R⁵¹, or
22) -NR^{e1}R^{f1} (wherein if R⁵ is the substituent of 22), -L³-L⁴-R⁵ is not - (CH₂)₁₋₄NR^{e1}R^{f1} (wherein R^{e1} and R^{f1} are a hydrogen atom, optionally substituted C₁₋₄ alkyl, an optionally substituted C₃₋₇ alicyclic group, an optionally substituted 4- to 10-membered non-aryl heterocyclic group, optionally substituted C₆₋₁₀ aryl, or optionally substituted 5- to 10-membered heteroaryl)),
R20a is
1) a C₁₋₆ alkyl group,
2) a C₃₋₁₀ alicyclic group,
3) C₆₋₁₀ aryl,
4) 5- or 6-membered heteroaryl, or
5) a 4- to 10-membered non-aryl heterocycle,
(wherein each substituent from 1) to 5) is optionally substituted),
R⁵⁰ represents
1) a hydrogen atom,
2) a C₁₋₆ alkyl group,
3) a hydroxyl group,
4) C₁₋₆ alkoxy group,
5) a C₃₋₆ cycloalkoxy group,
6) a C₃₋₆ alicyclic group,
7) a 4- to 6-membered non-aryl heterocycle,
8) C₆₋₁₀ aryl,
9) 5- to 10-membered heteroaryl,
10) 4- to 6-membered non-aryl heterocyclyl oxy,
11) C₆₋₁₀ aryloxy,
12) 5- to 10-membered heteroaryloxy,
13) a C₁₋₆ alkylsulfonyl group,
14) a C₃₋₆ cycloalkoxysulfonyl group, or
15) a 4- to 6-membered non-aryl heterocyclyl sulfonyl group, (wherein each substituent of 2) and 4) to 15) is optionally substituted),
R⁵¹ represents
1) a hydrogen atom,
2) a C₁₋₆ alkyl group,
3) a C₃₋₆ alicyclic group,
4) a 4- to 6-membered non-aryl heterocycle,
5) C₆₋₁₀ aryl, or
6) 5- to 10-membered heteroaryl,
(wherein each substituent from 2) to 6) is optionally substituted), or
R⁵⁰ and R⁵¹ together may form optionally substituted 4- to 7-membered nitrogen-containing non-aryl heterocycle,
the remaining two (without the structure of formula (2) among R¹, R², and R³) are the same or different, each independently a hydrogen atom, a halogen atom, an optionally substituted C₁₋₆ alkyl group, an optionally substituted C₁₋₆ alkoxy group, an optionally substituted C₁₋₆ alkylthio group, optionally substituted 5- or 6-membered heteroaryl, or -NR^{a3}R^{b2},
R^{d1}, R^{d2}, R^{e1}, R^{f1}, R^{g1}, R^{h1}, Rⁱ¹, and R^{j} are the same or different, each independently
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted),
a combination of R^{e1} and R^{f1}, when attached to the same nitrogen atom, together may form an optionally substituted 4- to 10-membered nitrogen-containing non-aryl heterocycle,
R⁴ is
1) -C (=O) R⁸,
2) -SO₂-L⁶-R⁸,
(wherein R⁸ in 1) and 2) is -NR^{a5}R^{b4}, -NR^{a5}-L⁷-B(OR^{m1}) ₂, -OR^{m1}, or an optionally substituted C₁₋₆ alkyl group, and L⁶ is a single bond or -NR^{a6}-),
3) -NR^{a4}R^{b3},
4) -B(OR^{m1})₂,
5) -PO(OR^{m1}) (OR^{m2}),
6) optionally substituted 5-membered heteroaryl,
7) an optionally substituted 5-membered non-aryl heterocycle, or
8) a bioisostere of one of 1) to 7),
(wherein the formulas of 2), 4), 5), and 6) include a carboxylic acid isostere, and 8) may include them in duplicates),
R^{a3}, R^{a4}, R^{a5}, R^{a6}, R^{b2}, R^{b3}, and R^{b4} are the same or different, each independently having the same definition as R^{a1}, R^{a2}, and R^{b1}, wherein a combination of R^{a3} and R^{b2}, R^{a4} and R^{b3}, or R^{a5} and R^{b4}, when attached to the same nitrogen atom, together may form an optionally substituted 4- to 10-membered nitrogen-containing non-aryl heterocycle,
R^{m1} is
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle, (wherein each substituent from 2) to 6) is optionally substituted),
wherein if R^{m1} is attached to a boron atom via an oxygen atom, two R^{m1}, as C₂₋₄ alkylene, together with the boron atom and two oxygen atoms, may form a 5- to 7-membered non-aryl heterocycle (wherein an alkylene moiety is optionally substituted in the non-aryl heterocycle),
R^{m2} is a hydrogen atom, an optionally substituted C₁₋₆ alkyl group, or an optionally substituted C₃₋₁₀ alicyclic group,
L⁷ is an optionally substituted C₁₋₃ alkylene group,
R⁶¹, R⁶², and R⁶³ are each independently a hydrogen atom, halogen, or an optionally substituted C₁₋₆ alkyl group, and
R⁶⁴ is a hydrogen atom, halogen, an optionally substituted C₁₋₆ alkyl group, or -L¹-L²-Z.

4. The compound or the pharmaceutically acceptable salt thereof according to any one of claims 1 to 3, wherein L³ is
1) a C₁₋₆ alkylene group,
2) a C₃₋₁₀ cycloalkylene group, or
3) a 4- to 10-membered non-aryl heterocyclylene group,
(wherein each substituent from 1) to 3) is optionally substituted), and
L⁴ is a single bond or an optionally substituted C₁₋₅ alkylene group.

5. The compound or the pharmaceutically acceptable salt thereof according to any one of claims 1 to 4, wherein
L³ is an optionally substituted C₁₋₄ alkylene group, and
L⁴ is a single bond or an optionally substituted C₁₋₃ alkylene group.

6. The compound or the pharmaceutically acceptable salt thereof according to any one of claims 1 to 5, wherein
L³ is -(CR³⁰R³¹)ₙ₁-,
R³⁰ and R³¹ are each independently, or if there are multiple instances of each of them, all of them independently represent
1) a hydrogen atom,
2) -NR^{a7}R^{a8},
3) a C₁₋₄ alkyl group,
4) C₆₋₁₀ aryl,
5) 5- to 10-membered heteroaryl,
6) a C₃₋₆ alicyclic group,
7) a 4- to 10-membered non-aryl heterocycle,
(wherein each substituent from 3) to 7) is optionally substituted), or
8) -OR^{c2}, or
R³⁰ and R³¹, together with the carbon atom to which they are attached, form a C₃₋₆ alicyclic group or a 4- to 6-membered non-aryl heterocycle,
R^{a7} and R^{a8} are the same or different, each independently
1) a hydrogen atom,
2) a C₁₋₆ alkyl group,
3) a C₃₋₁₀ alicyclic group,
4) C₆₋₁₀ aryl,
5) 5- or 6-membered heteroaryl,
6) a 4- to 10-membered non-aryl heterocycle,
7) a C₁₋₆ alkylcarbonyl group,
8) a C₃₋₁₀ alicyclic carbonyl group,
9) a C₆₋₁₀ arylcarbonyl group,
10) a 5- or 6-membered heteroarylcarbonyl group,
11) a C₁₋₆ alkylsulfonyl group,
12) a C₃₋₁₀ alicyclic sulfonyl group,
13) a C₆₋₁₀ arylsulfonyl group,
14) a 5- or 6-membered heteroarylsulfonyl group, or
15) -OR^{c3},
(wherein each substituent from 2) to 14) is optionally substituted),
wherein R^{a7} and R^{a8} together may form an optionally substituted 4- to 10-membered nitrogen-containing non-aryl heterocycle,
R^{c2} and R^{c3} are each independently
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted),
n1 is an integer 1, 2, 3, or 4,
L⁴ is -(CR⁴⁰R⁴¹)ₙ₂-,
R⁴⁰ and R⁴¹ each independently, or if there are multiple instances of each of them, all of them independently represent
1) a hydrogen atom,
2) -NR^{a9}R^{a10},
3) a C₁₋₄ alkyl group,
4) C₆₋₁₀ aryl,
5) 5- to 10-membered heteroaryl,
6) a C₃₋₆ alicyclic group,
7) a 4- to 10-membered non-aryl heterocycle,
(wherein each substituent from 3) to 7) is optionally substituted), or
8) -OR^{c4}, or
R⁴⁰ and R⁴¹, together with the carbon atom to which they are attached, form a C₃₋₆ alicyclic group or a 4- to 6-membered non-aryl heterocycle,
R^{a9} and R^{a10} are the same or different, each independently
1) a hydrogen atom,
2) a C₁₋₆ alkyl group,
3) a C₃₋₁₀ alicyclic group,
4) C₆₋₁₀ aryl,
5) 5- or 6-membered heteroaryl,
6) a 4- to 10-membered non-aryl heterocycle,
7) a C₁₋₆ alkylcarbonyl group,
8) a C₃₋₁₀ alicyclic carbonyl group,
9) a C₆₋₁₀ arylcarbonyl group,
10) a 5- or 6-membered heteroarylcarbonyl group,
11) a C₁₋₆ alkylsulfonyl group,
12) a C₃₋₁₀ alicyclic sulfonyl group,
13) a C₆₋₁₀ arylsulfonyl group,
14) a 5- or 6-membered heteroarylsulfonyl group,
(wherein each substituent from 2) to 14) is optionally substituted), or
15) -OR^{c5},
wherein R^{a9} and R^{a10} together may form an optionally substituted 4- to 10-membered nitrogen-containing non-aryl heterocycle,
R^{c4} and R^{c5} are each independently defined the same as R^{c2} and R^{c3}, and
n2 is an integer 0 (i.e., when L⁴ is a single bond), 1, 2, or 3.

7. The compound or the pharmaceutically acceptable salt thereof according to any one of claims 1 to 6, wherein
-L³-L⁴- is an optionally substituted C₁₋₂ alkylene group.

8. The compound or the pharmaceutically acceptable salt thereof according to any one of claims 1 to 7, wherein -L³-L⁴- is a C₁₋₂ alkylene group optionally substituted with a C₁₋₃ alkyl group, an amino group, or a hydroxymethyl group, or a plurality of the same or different groups thereamong (wherein two C₁₋₃ alkyl groups, when attached to the same carbon atom, together with the carbon atom to which they are attached, may form a C₃₋₆ alicyclic group).

9. The compound or the pharmaceutically acceptable salt thereof according to any one of claims 1 to 8, wherein
R⁵ is
1) a C₃₋₁₀ alicyclic group,
2) C₆₋₁₀ aryl,
3) 5- to 10-membered heteroaryl,
4) a C₁₋₆ alkylthio group,
(wherein each substituent from 1) to 4) is optionally substituted, and if two substituents further substituted with the substituent of 2) or 3) are each substituted on adjacent atoms within a ring, the two substituents together may further form a condensed ring structure),
5) -NR^{e1}OH,
6) -C(=O)NR⁵⁰R⁵¹,
7) -SO₂-NR^{e1}R^{f1},
8) -NR^{e1}-SO₂-R^{f1},
9) -C (=O) OR²⁰, or
10) -NR^{e1}R^{f1} (wherein if R⁵ is the substituent of 10), L³ and/or L⁴ is a C₁₋₆ alkylene group that is necessarily substituted with one or more groups other than a hydrogen atom (wherein L³ or L⁴, together with said substituent, may form a C₃₋₁₀ alicyclic group or a 4- to 10-membered non-aryl heterocycle)), and
R²⁰ is
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted).

10. The compound or the pharmaceutically acceptable salt thereof according to any one of claims 1 to 9, wherein
R⁵ is
1) C₆₋₁₀ aryl,
2) 5- to 10-membered heteroaryl,
3) -C(=O)NR⁵⁰R⁵¹,
4) -C(=O) OR²⁰, or
5) -NR^{e1}R^{f1} (wherein if R⁵ is the substituent of 5), L³ and/or L⁴ is a C₁₋₆ alkylene group that is necessarily substituted with one or more groups other than a hydrogen atom, and together with said substituent forms at least one C₃₋₁₀ alicyclic group or 4- to 10-membered non-aryl heterocycle),
(wherein each substituent from 1) to 2) is optionally substituted, and any two groups thereof, when each is attached to adjacent atoms within a ring, together may further form a condensed ring structure).

11. The compound or the pharmaceutically acceptable salt thereof according to any one of claims 1 to 10, wherein Z-L²-L¹ is an optionally substituted C₁₋₆ alkylthio group.

12. The compound or the pharmaceutically acceptable salt thereof according to any one of claims 1 to 11, wherein R⁶¹, R⁶², R⁶³, and R⁶⁴ are each independently a hydrogen atom, a fluorine atom, or a C₁₋₃ alkyl group optionally substituted with a fluorine atom.

13. The compound or the pharmaceutically acceptable salt thereof according to any one of claims 1 to 12, wherein R⁶¹, R⁶², R⁶³, and R⁶⁴ are each independently a hydrogen atom or a fluorine atom.

14. The compound or the pharmaceutically acceptable salt thereof according to any one of claims 1 to 13, wherein G is an oxygen atom.

15. The compound or the pharmaceutically acceptable salt thereof according to any one of claims 1 to 14, wherein X is a hydroxyl group or an optionally substituted C₁₋₆ alkoxy group.

16. The compound or the pharmaceutically acceptable salt thereof according to any one of claims 1 to 15, wherein X is a hydroxyl group.

17. The compound or the pharmaceutically acceptable salt thereof according to any one of claims 1 to 16, wherein the compounds of formulas (1a) and (1b) are represented by formulas (3a) and (3b), respectively: 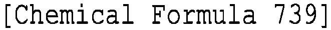 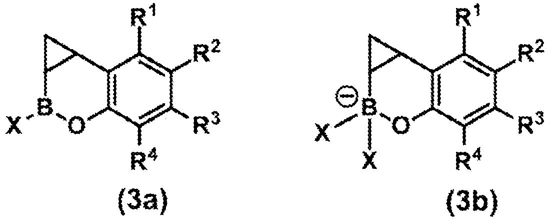 wherein X, R¹, R², and R³ are defined the same as any one of claims 1 to 16, and
R⁴ is selected from the group consisting of
1) -C(=O)OR^{m1} (wherein R^{m1} is a hydrogen atom, a C₁₋₆ alkyl group, a C₃₋₁₀ alicyclic group, C₆₋₁₀ aryl, 5- or 6-membered heteroaryl, or a 4- to 10-membered non-aryl heterocycle, wherein the C₁₋₆ alkyl group, the C₃₋₁₀ alicyclic group, the C₆₋₁₀ aryl, the 5- or 6-membered heteroaryl, and the 4- to 10-membered non-aryl heterocycle are each optionally substituted), and
2) a bioisostere of 1).

18. The compound or the pharmaceutically acceptable salt thereof according to any one of claims 1 to 17, wherein R⁴ is
1) -C(=O)OH (i.e., a carboxyl group), or
2) a carboxylic acid isostere.

19. The compound or the pharmaceutically acceptable salt thereof according to any one of claims 1 to 18, wherein the compounds of formulas (1a) and (1b) or the compounds of formulas (3a) and (3b) are represented by formulas (4a) and (4b), respectively: 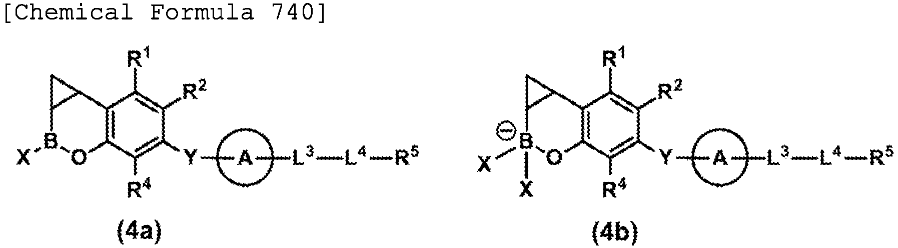 wherein X, R⁴, Y, ring A, L³, L⁴, and R⁵ are defined the same as any one of claims 1 to 18, and
R¹ and R² are the same or different, each independently a hydrogen atom, a halogen atom, a C₁₋₆ alkyl group, a C₁₋₆ alkoxy group, or a C₁₋₆ alkylthio group (wherein the C₁₋₆ alkyl group, C₁₋₆ alkoxy group, and C₁₋₆ alkylthio group are optionally substituted).

20. The compound or the pharmaceutically acceptable salt thereof according to any one of claims 1 to 19, wherein ring A is an optionally substituted 4- to 10-membered non-aryl heterocycle.

21. The compound or the pharmaceutically acceptable salt thereof according to any one of claims 1 to 20, wherein ring A is an optionally substituted 4- to 7-membered non-aryl heterocycle.

22. The compound or the pharmaceutically acceptable salt thereof according to any one of claims 1 to 21, wherein Y is an oxygen atom or a sulfur atom.

23. The compound or the pharmaceutically acceptable salt thereof according to any one of claims 1 to 22, wherein Y is an oxygen atom.

24. The compound or the pharmaceutically acceptable salt thereof according to any one of claims 1 to 23, wherein the compounds of formulas (1a) and (1b), the compounds of formulas (3a) and (3b), or the compounds of formulas (4a) and (4b) are represented by formulas (5a) and (5b), respectively: 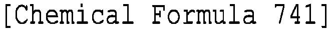 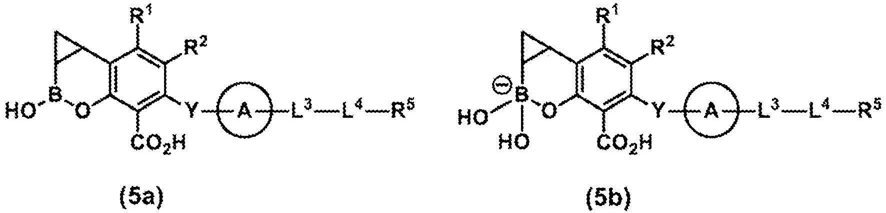 wherein R¹, R², Y, L³, L⁴, and R⁵ are defined the same as any one of claims 1 to 23, and ring A is an optionally substituted 4- to 6-membered nitrogen-containing non-aryl heterocycle.

25. The compound or the pharmaceutically acceptable salt thereof according to any one of claims 1 to 3 and 11 to 24, wherein L³ is -C(=O)- or -S(=O)₂-.

26. The compound or the pharmaceutically acceptable salt thereof according to any one of claims 1 to 3 and 11 to 25, wherein L³ is -C(=O)-.

27. The compound or the pharmaceutically acceptable salt thereof according to any one of claims 1 to 3 and 11 to 26, wherein L⁴ is a single bond, -C(=N-OR^{h1})-, or an optionally substituted C₁₋₆ alkylene group, wherein R^{h1} is an optionally substituted C₁₋₆ alkyl group.

28. The compound or the pharmaceutically acceptable salt thereof according to any one of claims 1 to 27, wherein R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
(wherein each substituent from 3) to 5) is optionally substituted).

29. The compound or the pharmaceutically acceptable salt thereof according to any one of claims 1 to 28, wherein R¹ and R² are the same or different, each independently selected from the group consisting of
1) a hydrogen atom,
2) a halogen atom,
3) a C₁₋₆ alkyl group,
4) a C₁₋₆ alkoxy group, and
5) a C₁₋₆ alkylthio group,
(wherein each substituent from 3) to 5) is optionally substituted with a halogen atom).

30. The compound or the pharmaceutically acceptable salt thereof according to any one of claims 1 to 29, wherein R¹ and R² are the same or different, each independently selected from the group consisting of
1) a hydrogen atom,
2) a halogen atom, and
3) a C₁₋₆ alkyl group optionally substituted with a halogen atom.

31. The compound or the pharmaceutically acceptable salt thereof according to any one of claims 1 to 30, wherein R¹ and R² are the same or different, each independently selected from the group consisting of
1) a hydrogen atom,
2) a fluorine atom, and
3) a C₁₋₃ alkyl group optionally substituted with a fluorine atom.

32. The compound or the pharmaceutically acceptable salt thereof according to any one of claims 1 to 31, wherein R¹ and R² are both hydrogen atoms.

33. The compound or the pharmaceutically acceptable salt thereof according to any one of claims 1 to 32, wherein the compounds of formulas (1a) and (1b), the compounds of formulas (3a) and (3b), the compounds of formulas (4a) and (4b), or the compounds of formulas (5a) and (5b) are represented by formulas (6a) and (6b), respectively: 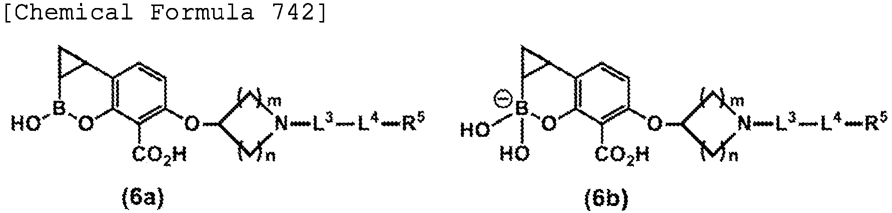 wherein
L³, L⁴, and R⁵ are defined the same as any one of claims 1 to 32,
m is an integer 1, 2, or 3,
n is an integer 1, 2, or 3, and
m + n is 2, 3, or 4.

34. The compound or the pharmaceutically acceptable salt thereof according to claim 33, wherein the compounds of formulas (6a) and (6b) are enantiomers represented by
formulas (7a) and (7b): 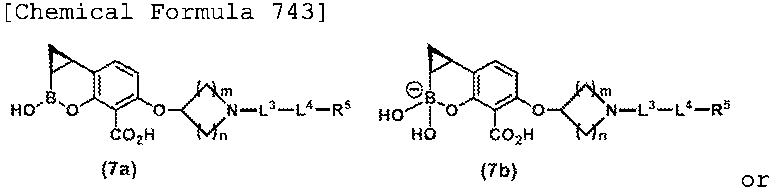
formulas (8a) and (8b): 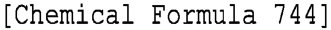 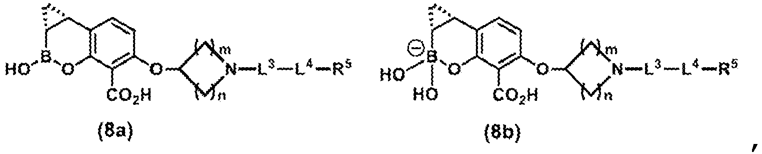 respectively, wherein
L³, L⁴, and R⁵ are defined the same as any one of claims 1 to 33, and
m and n are defined the same as claim 33.

35. The compound or the pharmaceutically acceptable salt thereof according to any one of claims 1 to 34, wherein m is 1 or 2, n is 1 or 2, and m + n is 2 or 3.

36. The compound or the pharmaceutically acceptable salt thereof according to any one of claims 1 to 35, wherein m is 1, and n is 1.

37. The compound or the pharmaceutically acceptable salt thereof according to any one of claims 1 to 6, 9 to 24, and 28 to 36, wherein
L³ is - (CR³⁰R³¹)ₙ₁- or 4- to 10-membered non-aryl heterocyclylene,
R³⁰ and R³¹ each independently, or if there are multiple instances of each of them, all of them independently represent
1) a hydrogen atom,
2) an optionally substituted C₁₋₄ alkyl group, or
3) optionally substituted C₆₋₁₀ aryl,
n1 is 1, 2, or 3,
L⁴ is a single bond or - (CR⁴⁰R⁴¹)ₙ₂-,
R⁴⁰ and R⁴¹ each independently, or if there are multiple instances of each of them, all of them independently represent
1) a hydrogen atom,
2) -NR^{a9}R^{a10},
3) an optionally substituted C₁₋₄ alkyl group, or
4) -OR^{c4} or
R⁴⁰ and R⁴¹, together with the carbon atom to which they are attached, form a C₃₋₆ alicyclic group or a 4- to 6-membered non-aryl heterocycle,
R^{a9}, R^{a10}, and R^{c4} are the same or different, each independently
1) a hydrogen atom,
2) an optionally substituted C₁₋₆ alkyl group, or
3) an optionally substituted C₃₋₁₀ alicyclic group,
wherein R^{a9} and R^{a10} together may form an optionally substituted 4-to 10-membered nitrogen-containing non-aryl heterocycle, and
n2 is 0, 1, or 2.

38. The compound or the pharmaceutically acceptable salt thereof according to claim 37, wherein n1 is 1 or 2, and n2 is 0 or 1.

39. The compound or the pharmaceutically acceptable salt thereof according to any one of claims 1 to 6, 9 to 24, and 28 to 38, wherein
L³ is -CR³⁰R³¹- or 4- to 10-membered non-aryl heterocyclylene,
R³⁰ and R³¹ each independently represent,
1) a hydrogen atom,
2) a C₁₋₄ alkyl group (wherein the group is optionally substituted with halogen, -NR^{a11}R^{a12}, or -OR^{c6}), or
3) C₆ aryl (wherein the group is optionally substituted with halogen, -NR^{e13}R^{e14}, -OR^{c7}, or a C₁₋₃ alkyl group (wherein the group is optionally substituted with halogen, -NR^{a15}R^{a16}, or -OR^{c8})),
L⁴ is a single bond or - (CR⁴⁰R⁴¹)ₙ₂-,
R⁴⁰ and R⁴¹ each independently represent
1) a hydrogen atom,
2) -NR^{a9}R^{a10},
3) a C₁₋₄ alkyl group (wherein the group is optionally substituted with halogen, -NR^{a17}R^{a18}, or -OR^{c9}), or
4) -OR^{c4}, or
R⁴⁰ and R⁴¹, together with the carbon atom to which they are attached, form a C₃₋₆ alicyclic group,
R^{a9}, R^{a10}, R^{a11}, **R^{a12}, R^{a13},** R^{a14}, **R^{a15}, R^{a16}, R^{a17}, R^{a18}, R^{c4}, R^{c6},** R^{c7}, R^{c8}, and R^{c9} are the same or different, each independently representing
1) a hydrogen atom, or
2) a C₁₋₄ alkyl group (wherein the group is optionally substituted with halogen, -NR^{a19}R^{a20}, or -OR^{c10}),
R^{a19}, R^{a20}, and R^{c10} are the same or different, each independently representing
1) a hydrogen atom, or
2) a C₁₋₄ alkyl group (wherein the group is optionally substituted with halogen),
wherein each combination of R^{a9} and R^{a10}, R^{a11} and R^{a12}, R^{a13} and R^{a14}, R^{a15} and R^{a16}, R^{a17} and R^{a18}, or R^{a19} and R^{a20} together may form an optionally substituted 4- to 7-membered nitrogen-containing non-aryl heterocycle, and
n2 is 0 or 1.

40. The compound or the pharmaceutically acceptable salt thereof according to any one of claims 1 to 6, 9 to 24, and 28 to 39, wherein
L³ is -CH₂-, -CH(CH₂NH₂)-, or -CH(CH₂OH)-, and
L⁴ is a single bond, -CH₂-, -CH(NH₂)-, -CMe(NH₂)-, -CEt(NH₂)-, -C(iso-Pr)(NH₂)-, -CH (CH₂NH₂)-, -CH(OH)-, -CH(CH₂OH)-, -C(CH₂OH)₂-, or

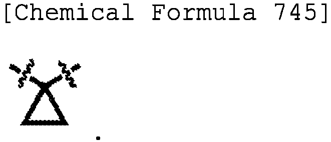

41. The compound or the pharmaceutically acceptable salt thereof according to any one of claims 1 to 6, 9 to 24, and 28 to 39, wherein L³ is 4- to 10-membered non-aryl heterocyclylene.

42. The compound or the pharmaceutically acceptable salt thereof according to any one of claims 1 to 6, 9 to 24, 28 to 39, and 41, wherein L³ is 5-membered non-aryl heterocyclylene.

43. The compound or the pharmaceutically acceptable salt thereof according to any one of claims 1 to 6, 9 to 24, 28 to 39, and 41 to 42, wherein L³ is 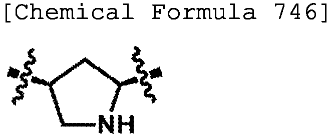 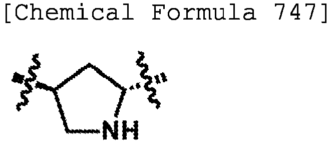 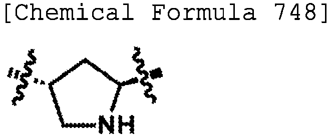 or

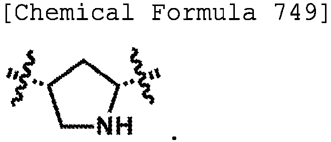

44. The compound or the pharmaceutically acceptable salt thereof according to any one of claims 1 to 6, 9 to 24, 28 to 39, and 41 to 43, wherein L⁴ is a single bond.

45. The compound or the pharmaceutically acceptable salt thereof according to any one of claims 1 to 24 and 28 to 40, wherein R⁵ is
1) C₆₋₁₀ aryl, or
2) 5- to 10-membered heteroaryl,
(wherein each substituent from 1) to 2) is optionally substituted, and if two substituents further substituted with the substituent of 1) or 2) are each substituted on adjacent atoms within a ring, the two substituents together may further form a condensed ring structure).

46. The compound or the pharmaceutically acceptable salt thereof according to any one of claims 1 to 24 and 28 to 45, wherein
R⁵ is C₆ aryl (i.e., phenyl) or 5-membered, 6-membered, 9-membered, or 10-membered heteroaryl,
each group of R⁵ is optionally substituted with each of R^{6a} or R^{6b} at all chemically substitutable positions on a carbon atom or a nitrogen atom within a ring thereof,
wherein R^{6a}, which are substituents on the carbon atom, if there are multiple instances on the same ring, are all independently selected from the group consisting of
1) a hydrogen atom,
2) a hydroxyl group,
3) a cyano group,
4) halogen,
5) a C₁₋₄ alkyl group,
6) a C₃₋₁₀ alicyclic group,
7) a C₁₋₄ alkoxy group,
8) a C₃₋₁₀ alicyclic oxy group,
9) a C₆₋₁₀ aryloxy group,
10) a 5- or 6-membered heteroaryloxy group,
11) a 4- to 10-membered non-aryl heterocyclyl oxy group,
(wherein each substituent from 5) and 11) is optionally substituted),
12) -SO₂-NR^{e2}R^{f2},
13) -NR^{g2}-CR^{e2} (=NR^{f2}),
14) -NR^{g2}-CR^{e2}(=N-OR^{f2}),
15) -NR^{h2}-C(=NR^{g2})NR^{e2}R^{f2},
16) -NR^{h2}-C(=N-OR^{g2})NR^{e2}R^{f2},
17) -NRⁱ²-C(=NR^{h2})NR^{g2}-NR^{e2}R^{f2},
18) -NRⁱ²-C(=N-OR^{h2})NR^{g2}-NR^{e2}R^{f2},
19) -C (=NR^{e2})R^{f2},
20) -C (=N-OR^{e2})R^{f2},
21) -C(=NR^{h2})-NR^{e2}R^{f2},
22) -C (=NR^{h2})NR^{g2}-NR^{e2}R^{f2},
23) -C(=N-OR^{h2})NR^{g2}NR^{e2}R^{f2},
24) -NR^{e2}R^{f2},
25) -NR^{g2}-NR^{e2}R^{f2},
26) -NR^{e2}OR^{f2},
27) -NR^{e2}-C (=O) R^{f2},
28) -C(=O) NR^{e2}R^{f2},
29) -C(=O) NR^{e2}OR^{f2},
30) -C(=O) NR^{g2}-NR^{e2}R^{f2},
31) -C (=O) R^{e2},
32) -C (=O) OR^{e2}, and
33) -C(=N-OR^{h2})NR^{e2}R^{f2},
R^{6b}, which are substituents on the nitrogen atom, if there are multiple instances on the same ring, are all independently selected from the group consisting of
1) a hydrogen atom,
2) a hydroxyl group,
3) a C₁₋₄ alkyl group
(wherein the alkyl group is optionally substituted),
4) a C₃₋₁₀ alicyclic group
(wherein the alicyclic group is optionally substituted),
5) -C(=NR^{e2})R^{f2},
6) -C(=N-OR^{e2})R^{f2},
7) -SO₂-NR^{e2}R^{f2},
8) -C(=NR^{h2})-NR^{e2}R^{f2},
9) -C(=NR^{h2})NR^{g2}-NR^{e2}R^{f2},
10) -C(=N-OR^{h2})NR^{g2}-NR^{e2}R^{f2},
11) -C(=O) NR^{e2}R^{f2},
12) -C(=O)NR^{e2}OR^{f2},
13) -C (=O) NR^{g2}-NR^{e2}R^{f2},
14) -C(=O)R^{e2}, and
15) -C (=N-OR^{h2})NR^{e2}R^{f2}, or
if a combination of two R^{6a} or R^{6a} and R^{6b} are each substituted on adjacent atoms within a ring, the two substituents together may form an optionally substituted 5- to 6-membered heteroaryl ring or an optionally substituted 5- to 7-membered non-aryl heterocycle, which further fuses to an attachment moiety between the adjacent atoms within the ring,
R^{e2}, R^{f2}, R^{g2}, R^{h2}, and Rⁱ² are the same or different, each independently
1) a hydrogen atom,
2) a C₁₋₆ alkyl group,
3) a C₃₋₁₀ alicyclic group,
4) C₆₋₁₀ aryl,
5) 5- or 6-membered heteroaryl, or
6) a 4- to 10-membered non-aryl heterocycle,
(wherein each substituent from 2) to 6) is optionally substituted), and
a combination of R^{e2} and R^{f2}, when attached to the same nitrogen atom, together may form an optionally substituted 4- to 10-membered nitrogen-containing non-aryl heterocycle.

47. The compound or the pharmaceutically acceptable salt thereof according to any one of claims 1 to 24 and 28 to 46, wherein
R⁵ is C₆ aryl or 5-membered, 6-membered, 9-membered, or 10-membered heteroaryl selected from the group consisting of 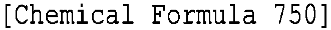 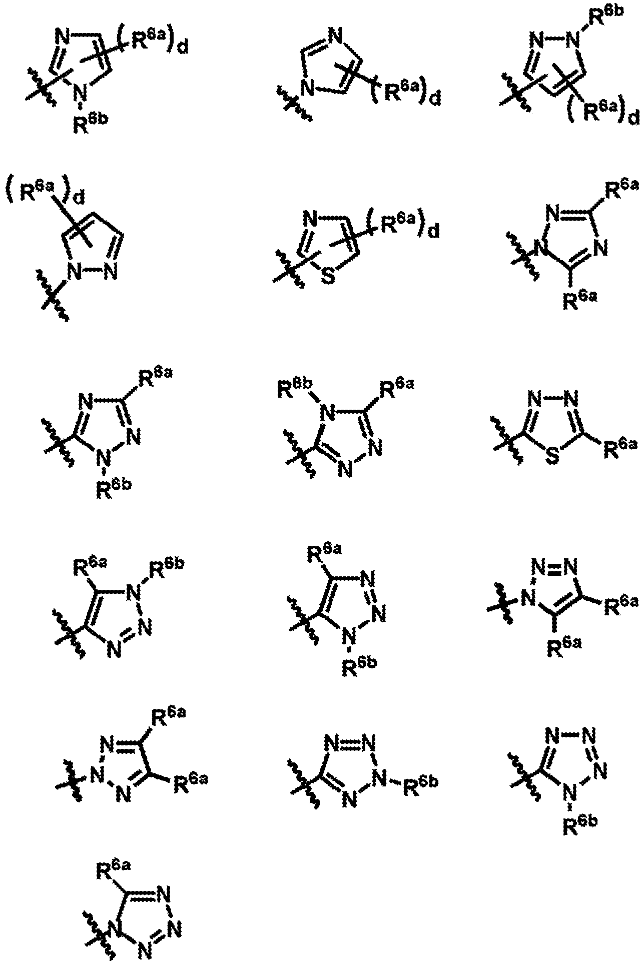 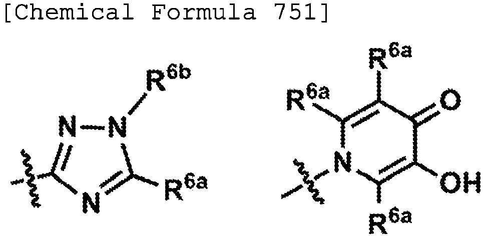 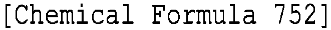 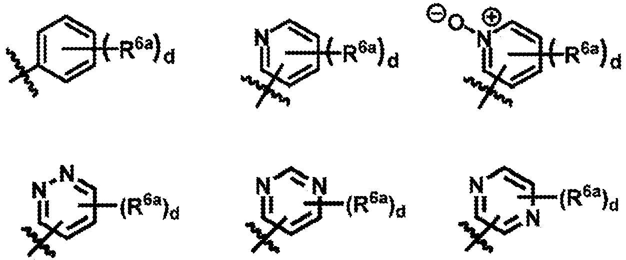 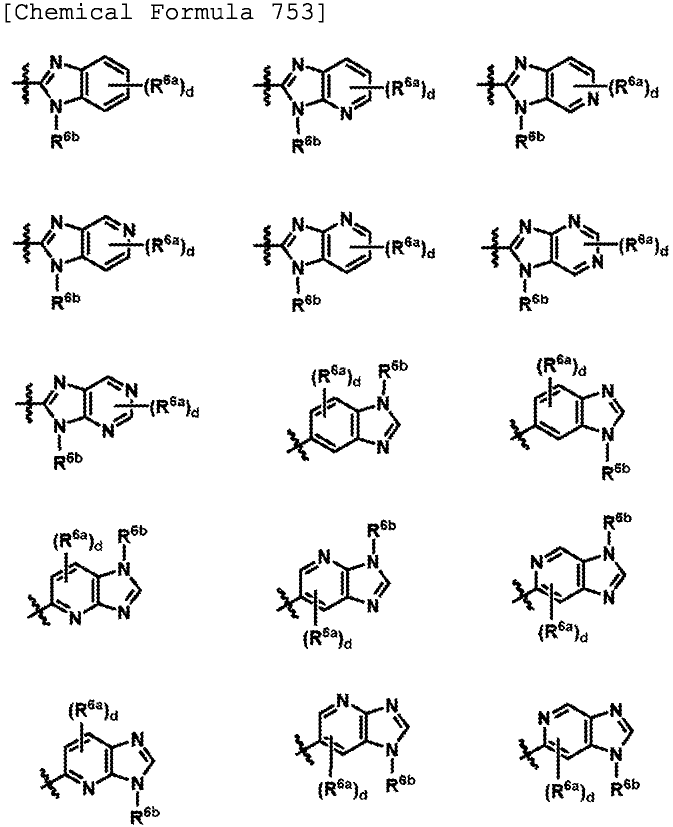 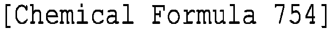 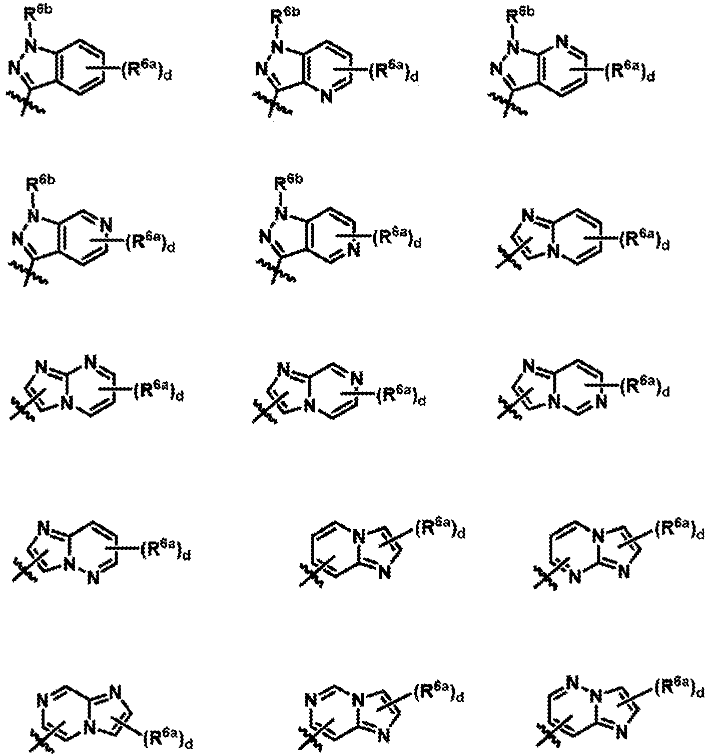 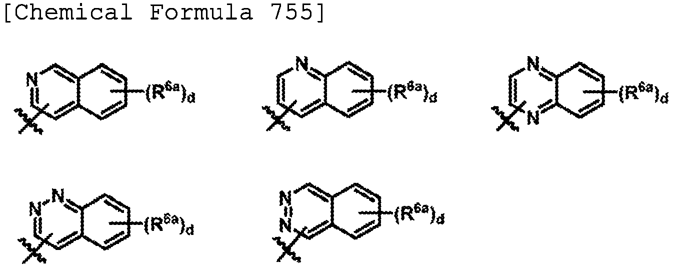
d is the number of chemically substitutable positions on a ring of each group by R^{6a}, and
each R^{6a} and each R^{6b} are defined the same as claim 46.

48. The compound or the pharmaceutically acceptable salt thereof according to claim 46 or 47, wherein
R^{6a}, if there are multiple instances on the same ring, are all independently selected from the group consisting of
1) a hydrogen atom,
2) a hydroxyl group,
3) halogen,
4) a C₁₋₄ alkyl group,
(wherein the alkyl group is optionally substituted with NR^{e2}R^{f2}, - C(=O)OR^{f2}, -C (=O)NR^{e2}R^{f2}, -C (=O)NR^{e2}(OR^{f2}), or a hydroxyl group),
5) a C₁₋₄ alkoxy group,
6) -NR^{e2}R^{f2},
7) -C (=O)NR^{e2}R^{f2}, and
8) -C (=O)OR^{e2}, and
each of R^{6b}, if there are multiple instances on the same ring, are all independently selected from the group consisting of
1) a hydrogen atom,
2) a hydroxyl group, and
3) a C₁₋₄ alkyl group,
(wherein the alkyl group is optionally substituted with NR^{e2}R^{f2}, - C(=O)OR^{f2}, -C (=O)NR^{e2}R^{f2}, -C (=O)NR^{e2}(OR^{f2}), or a hydroxyl group).

49. The compound or the pharmaceutically acceptable salt thereof according to any one of claims 1 to 24 and 28 to 48, wherein R^{e2} and R^{f2} are the same or different, each independently a hydrogen atom, an optionally substituted C₁₋₆ alkyl group, or an optionally substituted C₃₋₁₀ alicyclic group.

50. The compound or the pharmaceutically acceptable salt thereof according to any one of claims 1 to 24 and 28 to 49, wherein R^{e2} and R^{f2} are the same or different, each independently a hydrogen atom or an optionally substituted C₁₋₆ alkyl group.

51. The compound or the pharmaceutically acceptable salt thereof according to any one of claims 1 to 24 and 28 to 50, wherein R^{e2} and R^{f2} are hydrogen atoms.

52. The compound or the pharmaceutically acceptable salt thereof according to any one of claims 46 to 51, wherein R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

53. The compound or the pharmaceutically acceptable salt thereof according to any one of claims 1 to 24 and 28 to 44, wherein R⁵ is -C (=O)NR⁵⁰R⁵¹.

54. The compound or the pharmaceutically acceptable salt thereof according to any one of claims 1 to 24, 28 to 40, and 53, wherein
R⁵⁰ represents
1) a hydrogen atom,
2) an optionally substituted C₁₋₄ alkyl group,
3) a hydroxyl group,
4) an optionally substituted C₁₋₄ alkoxy group, or
5) an optionally substituted C₁₋₆ alkylsulfonyl group,
R⁵¹ represents
1) a hydrogen atom, or
2) an optionally substituted C₁₋₄ alkyl group, or
R⁵⁰ and R⁵¹ together may form a 4- to 6-membered nitrogen-containing non-aryl heterocycle.

55. The compound or the pharmaceutically acceptable salt thereof according to any one of claims 1 to 24, 28 to 40, and 53 to 54, wherein
R⁵⁰ represents
1) a hydrogen atom,
2) a C₁₋₄ alkyl group (wherein the group is optionally substituted with halogen, a C₃₋₆ alicyclic group, -NR^{a21}R^{a22}, or -OR^{c11}),
3) a hydroxyl group,
4) a C₁₋₄ alkoxy group (wherein the group is optionally substituted with halogen, a C₃₋₆ alicyclic group, -NR^{a23}R^{a24}, or -OR^{c12}), or
5) a C₁₋₄ alkylsulfonyl group (wherein the group is optionally substituted with halogen, a C₃₋₆ alicyclic group, -NR^{a21}R^{a22}, or - OR^{c11}),
R^{a21}, R^{a22}, R^{a23}, R^{a24}, R^{c11}, and R^{c12} are the same or different, each independently representing
1) a hydrogen atom, or
2) a C₁₋₄ alkyl group (wherein the group is optionally substituted with halogen),
wherein each combination of R^{a21} and R^{a22} or R^{a23} and R^{a24} together may form an optionally substituted 4- to 7-membered nitrogen-containing non-aryl heterocycle, and
R⁵¹ is a hydrogen atom or C₁₋₄ alkyl (wherein the group is optionally substituted with halogen).

56. The compound or the pharmaceutically acceptable salt thereof according to any one of claims 1 to 24, 28 to 40, and 53 to 55, wherein
R⁵⁰ represents
1) a hydrogen atom,
2) a C₁₋₄ alkyl group (wherein the group is optionally substituted with a cyclopropyl group, -NH₂, -NHMe, -C(=O)NH₂, -C(=O)NHMe, - C(=O)NMe₂, or a -hydroxyl group),
3) a hydroxyl group,
4) a C₁₋₄ alkoxy group (wherein the group is optionally substituted with a cyclopropyl group, -NH₂, -NHMe, or a -hydroxyl group), or
5) a C₁₋₄ alkylsulfonyl group, and
R⁵¹ is a hydrogen atom.

57. The compound or the pharmaceutically acceptable salt thereof according to any one of claims 1 to 24 and 28 to 40, wherein R⁵ is -C(=O)OR²⁰.

58. The compound or the pharmaceutically acceptable salt thereof according to claim 57, wherein R²⁰ is
1) a hydrogen atom, or
2) an optionally substituted C₁₋₄ alkyl group.

59. The compound or the pharmaceutically acceptable salt thereof according to claim 56 or 58, wherein
L³ is -CH₂-, and
L⁴ is a single bond, -CH(NH₂)-, or -CMe(NH₂)-.

60. The compound or the pharmaceutically acceptable salt thereof according to claim 59, wherein L⁴ is 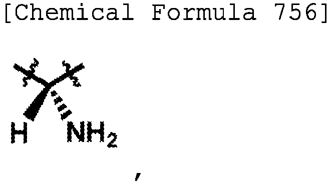 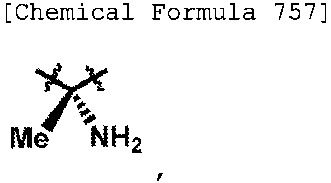 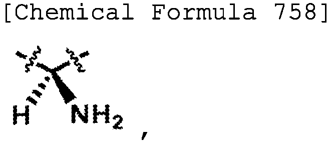 or

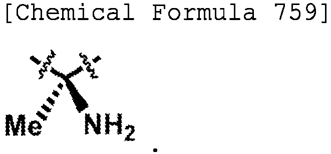

61. The compound or the pharmaceutically acceptable salt thereof according to claim 59 or 60, wherein L⁴ is 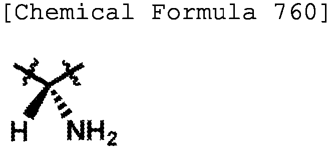 or

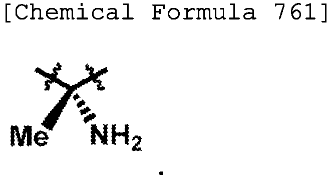

62. The compound or the pharmaceutically acceptable salt thereof according to any one of claims 57 to 61, wherein R²⁰ is a hydrogen atom.

63. The compound or the pharmaceutically acceptable salt thereof according to any one of claims 1 to 24 and 28 to 40, wherein
R⁵ is -NR^{e1}R^{f1},
L³ is -CH₂-,
L⁴ is -CR⁴⁰R⁴¹-, and
R⁴⁰ and R⁴¹ are each independently a C₁₋₄ alkyl group substituted with a hydroxyl group, or together with the carbon atom to which they are attached, form a C₃₋₆ alicyclic group.

64. The compound or the pharmaceutically acceptable salt thereof according to claim 63, wherein
R^{e1} and R^{f1} are the same or different, each independently
1) a hydrogen atom, or
2) an optionally substituted C₁₋₃ alkyl group, and
L⁴ is

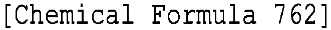

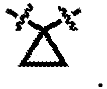

65. The compound or the pharmaceutically acceptable salt thereof according to claim 63, wherein
R^{e1} and R^{f1} are the same or different, each independently
1) a hydrogen atom, or
2) an optionally substituted C₁₋₃ alkyl group, and
L⁴ is a C₁₋₄ alkylene group substituted with a hydroxyl group.

66. The compound or the pharmaceutically acceptable salt thereof according to any one of claims 1 to 3 and 11 to 36, wherein L⁴ is a single bond, or a C₁₋₆ alkylene group optionally substituted with -NR²¹R²² or =NOR²³, wherein R²¹, R²², and R²³ are each independently a hydrogen atom, an optionally substituted C₁₋₆ alkyl group, or an optionally substituted 4- to 10-membered non-aryl heterocyclyl carbonyl group.

67. The compound or the pharmaceutically acceptable salt thereof according to any one of claims 1 to 3, 11 to 36, and 66, wherein L⁴ is a single bond, -CH₂-, -CH(NH₂)-, -CMe(NH₂)-, -CH(NHMe)-, - CD (NH₂)- (wherein D represents a heavy hydrogen atom), -CH₂CH₂-, or -CH(NH₂)-CH₂-, wherein if an amino group is present in L⁴, carbon that attaches to the amino group attaches to L³.

68. The compound or the pharmaceutically acceptable salt thereof according to claim 67, wherein
L³ is -C(=O)-, and
L⁴ is a single bond, -CH₂-, -CH(NH₂)-, -CMe(NH₂)-, or - CH (NH₂)-CH₂-.

69. The compound or the pharmaceutically acceptable salt thereof according to claim 68, wherein L⁴ is one of the following isomeric structures: 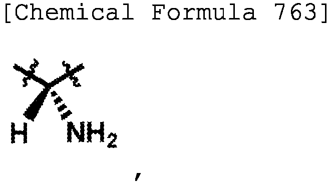 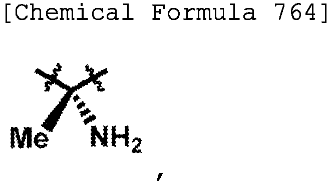 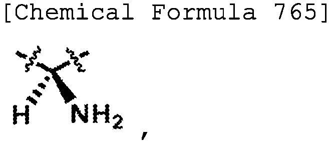 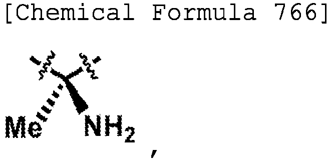 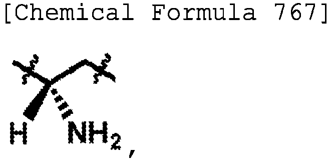 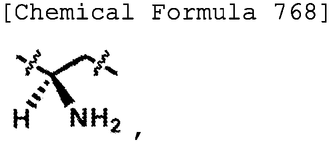 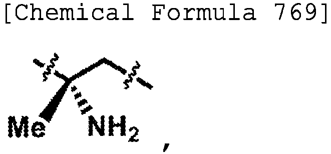 or

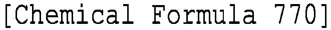

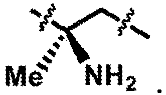

70. The compound or the pharmaceutically acceptable salt thereof according to claim 68 or 69, wherein L⁴ is 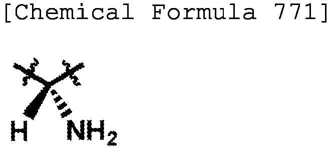 or

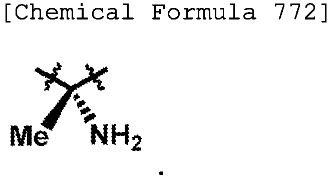

71. The compound or the pharmaceutically acceptable salt thereof according to claim 68 or 69, wherein L⁴ is 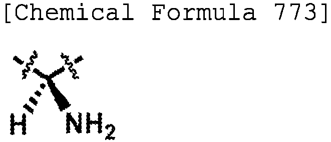 or

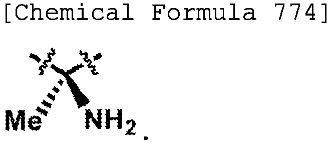

72. The compound or the pharmaceutically acceptable salt thereof according to claim 68 or 69, wherein L⁴ is 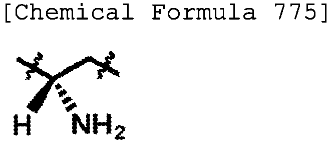 or

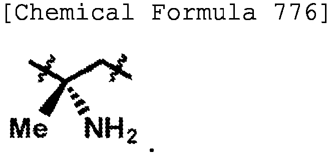

73. The compound or the pharmaceutically acceptable salt thereof according to claim 68 or 69, wherein L⁴ is 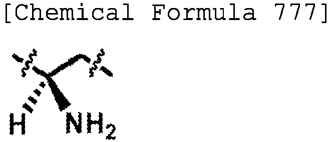 or

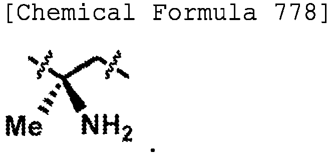

74. The compound or the pharmaceutically acceptable salt thereof according to any one of claims 1 to 3, 11 to 36, and 66 to 73, wherein R⁵ is a hydrogen atom, an optionally substituted C₁₋₆ alkyl group, an optionally substituted C₃₋₁₀ alicyclic group, an optionally substituted 4- to 10-membered non-aryl heterocycle, optionally substituted C₆₋₁₀ aryl, optionally substituted 5- or 6-membered heteroaryl, an optionally substituted C₁₋₆ alkylthio group, or -NR^{e1}OH, wherein R^{e1} is a hydrogen atom or an optionally substituted C₁₋₆ alkyl group.

75. The compound or the pharmaceutically acceptable salt thereof according to any one of claims 1 to 3, 11 to 36, and 66 to 74, wherein R⁵ is optionally substituted 5- or 6-membered heteroaryl or optionally substituted C₆₋₁₀ aryl.

76. The compound or the pharmaceutically acceptable salt thereof according to any one of claims 1 to 3, 11 to 36, and 66 to 75, wherein R⁵ is optionally substituted 5- or 6-membered heteroaryl.

77. The compound or the pharmaceutically acceptable salt thereof according to any one of claims 1 to 3, 11 to 36, and 66 to 76, wherein R⁵ is an optionally substituted C₄₋₁₀ alicyclic group or an optionally substituted 4- to 10-membered non-aryl heterocycle.

78. The compound or the pharmaceutically acceptable salt thereof according to any one of claims 1 to 3, 11 to 36, and 66 to 74, wherein L⁴ is a single bond and R⁵ is -NR^{e1}OH, wherein R^{e1} is a hydrogen atom or an optionally substituted C₁₋₆ alkyl group.

79. The compound or the pharmaceutically acceptable salt thereof according to any one of claims 1 to 3 and 11 to 36, wherein
L⁴ is
1) -(CH₂)p-CR¹⁰(NHR¹¹)-,
2) -(CH₂)_{q}-CR¹²R¹³-, or
3) - (CH₂)ₚ-CR¹⁰(NHR¹¹)- (CH₂)_{q}-CR¹²R¹³- (wherein p and q are independently 0 or 1),
R¹⁰ is
1) a hydrogen atom,
2) a carboxyl group, or
3) -C(=O)NR^{10a}R^{10b},
R¹¹ is
1) a hydrogen atom,
2) -C (=O)R^{11a}, or
3) an optionally substituted 5- or 6-membered non-aryl heterocyclyl carbonyl group,
wherein if R¹⁰ is -C (=O)NR^{10a}R^{10b}, R^{10b} and R¹¹ together may form - CH₂CH₂-,
R¹² is
1) a hydrogen atom, or
2) an optionally substituted C₁₋₄ alkyl group,
R¹³ is
1) a hydrogen atom,
2) a hydroxyl group,
3) an optionally substituted C₁₋₄ alkyl group,
4) a sulfanyl group,
5) a carboxyl group,
6) an optionally substituted C₁₋₄ alkylthio group,
7) -NR^{13a}R^{13b},
8) -NR^{13a}-C(=O)R^{13b},
9) an optionally substituted 5- or 6-membered non-aryl heterocyclyl carbonylamino group,
10) -NR^{13a}-C(=O)NR^{13b}R^{13c},
11) -C(=O)NR^{13a}R^{13b},
12) -C(=O)NR^{13a}OR^{13b},
13) -S(=O)₂-R^{13a},
14) -S(=O)₂-NR^{13a}R^{13b},
15) -C (=O)NR^{13a}-S(=O)₂-R^{13b}, or
16) -C (=O)NR^{13a}-S(=O)₂-NR^{13b}R^{13c}, and
R^{10a}, R^{10b}, R^{11a}, R^{13a}, R^{13b}, and R^{13c} are each independently a hydrogen atom or an optionally substituted C₁₋₄ alkyl group.

80. The compound or the pharmaceutically acceptable salt thereof according to claim 79, wherein R⁵ is a hydrogen atom or an optionally substituted C₁₋₄ alkyl group.

81. The compound or the pharmaceutically acceptable salt thereof according to any one of claims 1 to 3, 11 to 36, and 66 to 76, wherein
R⁵ is selected from the group consisting of 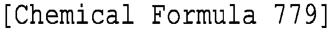 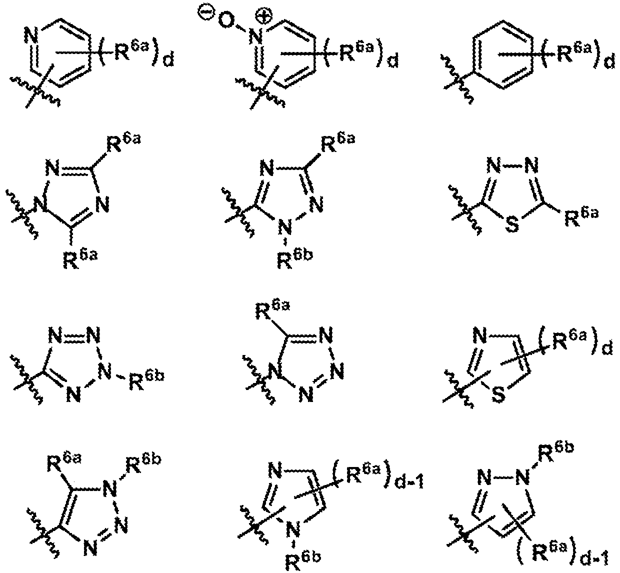 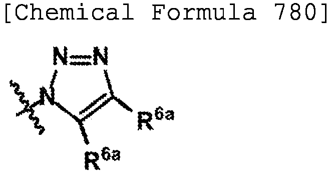 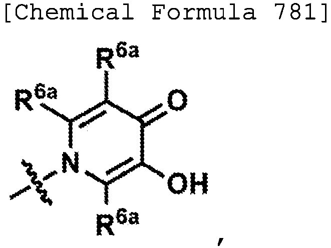
subscript d is the number of substitutable positions on a ring of R⁵,
each R^{6a} is independently selected from the group consisting of
1) a hydrogen atom,
2) a hydroxyl group,
3) a cyano group,
4) a nitro group,
5) halogen,
6) a C₁₋₄ alkyl group,
7) a C₃₋₁₀ alicyclic group,
8) a C₁₋₄ alkoxy group,
9) a C₃₋₁₀ alicyclic oxy group,
10) a C₆₋₁₀ aryloxy group,
11) a 5- or 6-membered heteroaryloxy group,
12) a 4- to 10-membered non-aryl heterocyclyl oxy group,
(wherein each substituent from 6) to 12) is optionally substituted),
13) -SO₂-NR^{e2}R^{f2},
14) -NR^{g2}-CR^{e2}(=NR^{f2}),
15) -NR^{g2}-CR^{e2}(=N-OR^{f2}),
16) -NR^{h2}-C(=NR^{g2})NR^{e2}R^{f2},
17) -NR^{h2}-C(=N-OR^{g2})NR^{e2}R^{f2},
18) -NRⁱ²-C(=NR^{h2})NR^{g2}-NR^{e2}R^{f2},
19) -NRⁱ²-C(=N-OR^{h2})NR^{g2}-NR^{e2}R^{f2},
20) -C (=NR^{e2})R^{f2},
21) -C(=N-OR^{e2})R^{f2},
22) -C(=NR^{h2})-NR^{e2}R^{f2},
23) -C(=NR^{h2})NR^{g2}-NR^{e2}R^{f2},
24) -C(=N-OR^{h2})NR^{g2}-NR^{e2}R^{f2},
25) -NR^{e2}R^{f2},
26) -NR^{g2}-NR^{e2}R^{f2},
27) -NR^{e2}OR^{f2},
28) -NR^{e2}-C(=O)R^{f2},
29) -C(=O)NR^{e2}R^{f2},
30) -C(=O)NR^{e2}OR^{f2},
31) -C(=O)NR^{g2}-NR^{e2}R^{f2},
32) -C(=O)R^{e2},
33) -C (=O)OR^{e2}, and
34) -C(=N-OR^{h2})NR^{e2}R^{f2}, and
each R^{6b} is independently selected from the group consisting of
1) a hydrogen atom,
2) a hydroxyl group,
3) a C₁₋₄ alkyl group
(wherein the alkyl group is optionally substituted),
4) a C₃₋₁₀ alicyclic group
(wherein the alicyclic group is optionally substituted),
5) -C (=NR^{e2})R^{f2},
6) -C(=N-OR^{e2})R^{f2},
7) -SO₂-NR^{e2}R^{f2},
8) -C (=NR^{h2})-NR^{e2}R^{f2},
9) -C (=NR^{h2})NR^{g2}-NR^{e2}R^{f2},
10) -C(=N-OR^{h2})NR^{g2}-NR^{e2}R^{f2},
11) -C(=O)NR^{e2}R^{f2},
12) -C(=O)NR^{e2}OR^{f2},
13) -C (=O)NR^{g2}-NR^{e2}R^{f2},
14) -C (=O)R^{e2}, and
15) -C(=N-OR^{h2})NR^{e2}R^{f2}.

82. The compound or the pharmaceutically acceptable salt thereof according to any one of claims 1 to 3, 11 to 36, 66 to 76, and 81, wherein
R⁵ is 5- or 6-membered aryl or heteroaryl selected from the group consisting of 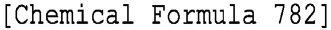 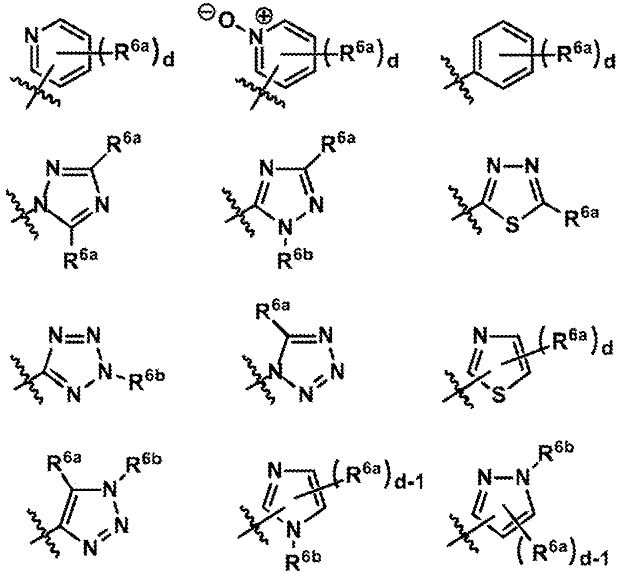 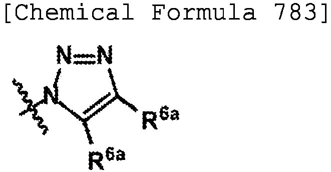 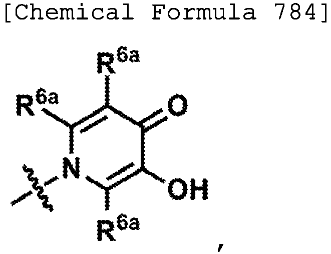
subscript d is the number of substitutable positions on a ring of R⁵,
each R^{6a} is independently selected from the group consisting of
1) a hydrogen atom,
2) a hydroxyl group,
3) halogen,
4) a C₁₋₄ alkyl group
(wherein the alkyl group is optionally substituted with NR^{e2}R^{f2}, a 5- or 6-membered non-aryl heterocycle, -C(=O)OR^{f2}, or a hydroxyl group),
5) a C₁₋₄ alkoxy group,
6) -NR^{e2}R^{f2}, and
7) -C(=O)OR^{e2}, and
each R^{6b} is independently selected from the group consisting of
1) a hydrogen atom,
2) a hydroxyl group, and
3) a C₁₋₄ alkyl group
(wherein the alkyl group is optionally substituted with NR^{e2}R^{f2}, - C (=O)NR^{e2}R^{f2}, -C(=O)OR^{f2}, or a hydroxyl group).

83. The compound or the pharmaceutically acceptable salt thereof according to any one of claims 1 to 3, 11 to 36, 66 to 76, and 81 to 82, wherein R⁵ is

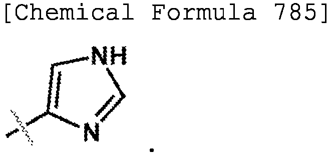

84. The compound or the pharmaceutically acceptable salt thereof according to any one of claims 1 to 3, 11 to 36, 66 to 76, and 81 to 82, wherein R^{e2} and R^{f2} are the same or different, each independently a hydrogen atom, an optionally substituted C₁₋₆ alkyl group, or an optionally substituted C₃₋₁₀ alicyclic group.

85. The compound or the pharmaceutically acceptable salt thereof according to any one of claims 1 to 3, 11 to 36, 66 to 76, 81 to 82, and 84, wherein R^{e2} and R^{f2} are the same or different, each independently a hydrogen atom or an optionally substituted C₁₋₆ alkyl group.

86. The compound or the pharmaceutically acceptable salt thereof according to any one of claims 1 to 3, 11 to 36, 66 to 76, 81 to 82, and 84 to 85, wherein R^{e2} and R^{f2} are hydrogen atoms.

87. The compound or the pharmaceutically acceptable salt thereof according to any one of claims 81 to 82 and 84 to 85, wherein R^{6a} is -NR^{e2}R^{f2}, and one of R^{e2} and R^{f2} is a hydrogen atom and the other is a C₁₋₄ alkyl group (wherein the alkyl group is optionally substituted with an amino group or a hydroxyl group).

88. The compound or the pharmaceutically acceptable salt thereof according to any one of claims 1 to 3, 11 to 36, 66 to 74, and 77, wherein
R⁵ is a 4- to 6-membered non-aryl heterocycle selected from the group consisting of 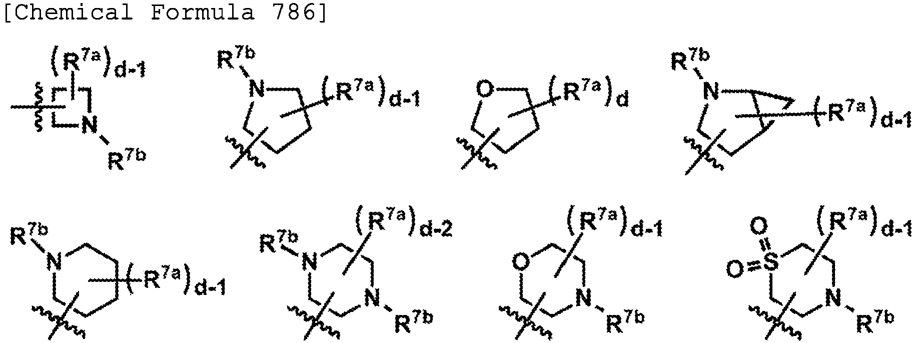 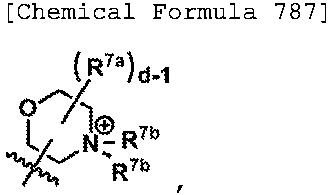
subscript d is the number of substitutable positions on a ring of R⁵,
each R^{7a} is independently selected from the group consisting of
1) a hydrogen atom,
2) a hydroxyl group,
3) a cyano group,
4) halogen,
5) a C₁₋₄ alkyl group,
6) a C₃₋₁₀ alicyclic group,
7) a C₁₋₄ alkoxy group,
8) a C₃₋₁₀ alicyclic oxy group,
9) a C₆₋₁₀ aryloxy group,
10) a 5- or 6-membered heteroaryloxy group,
11) a 4- to 10-membered non-aryl heterocyclyl oxy group,
(wherein each substituent from 5) and 11) is optionally substituted),
12) -SO₂-NR^{e3}R^{f3},
13) -NR^{g2}-CR^{e3}(=NR^{f3}),
14) -NR^{g2}-CR^{e3}(=N-OR^{f3}),
15) -NR^{h2}-C(=NR^{g2})NR^{e3}R^{f3},
16) -NR^{h2}-C(=N-OR^{g2})NR^{e3}R^{f3},
17) -NRⁱ²-C(=NR^{h2})NR^{g2}-NR^{e3}R^{f3},
18) -NRⁱ²-C(=N-OR^{h2})NR^{g2}-NR^{e3}R^{f3},
19) -C(=NR^{e3})R^{f3},
20) -C(=N-OR^{e3})R^{f3},
21) -C(=NR^{h2})-NR^{e3}R^{f3},
22) -C(=NR^{h2})NR^{g2}-NR^{e3}R^{f3},
23) -C(=N-OR^{h2})NR^{g2}-NR^{e3}R^{f3},
24) -NR^{e3}R^{f3},
25) -NR^{g2}-NR^{e3}R^{f3},
26) -NR^{e3}OR^{f3},
27) -NR^{e3}-C(=O)R^{f3},
28) -C(=O)NR^{e3}R^{f3},
29) -C(=O)NR^{e3}OR^{f3},
30) -C(=O)NR⁹²-NR^{e3}R^{f3},
31) -C(=O)R^{e3},
32) -C(=O)OR^{e3}, and
33) -C (=N-OR^{h2})NR^{e3}R^{f3},
each R^{7b} is independently selected from the group consisting of
1) a hydrogen atom,
2) a hydroxyl group,
3) a C₁₋₄ alkyl group (wherein the alkyl group is optionally substituted),
4) a C₃₋₁₀ alicyclic group (wherein the alicyclic group is optionally substituted),
5) -C (=NR^{e3})R^{f3},
6) -C(=N-OR^{e3})R^{f3},
7) -SO₂-NR^{e3}R^{f3},
8) -C(=NR^{h2})-NR^{e3}R^{f3},
9) -C(=NR^{h2})NR^{g2}-NR^{e3}R^{f3},
10) -C (=N-OR^{h2})NR^{g2}-NR^{e3}R^{f3},
11) -C(=O)NR^{e3}R^{f3},
12) -C(=O)NR^{e3}OR^{f3},
13) -C(=O)NR^{g2}-NR^{e3}R^{f3},
14) -C (=O)R^{e3}, and
15) -C(=N-OR^{h2})NR^{e3}R^{f3}, and
R^{e3}, R^{f3}, R^{g2}, R^{h2}, and Rⁱ² are defined the same as R^{e2}, R^{f2}, R^{g2}, R^{h2}, and Rⁱ² according to claim 3.

89. The compound or the pharmaceutically acceptable salt thereof according to any one of claims 1 to 3, 11 to 36, 66 to 74, 77, and 88, wherein
R⁵ is a 4- to 6-membered non-aryl heterocycle selected from the group consisting of 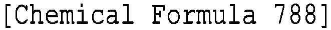 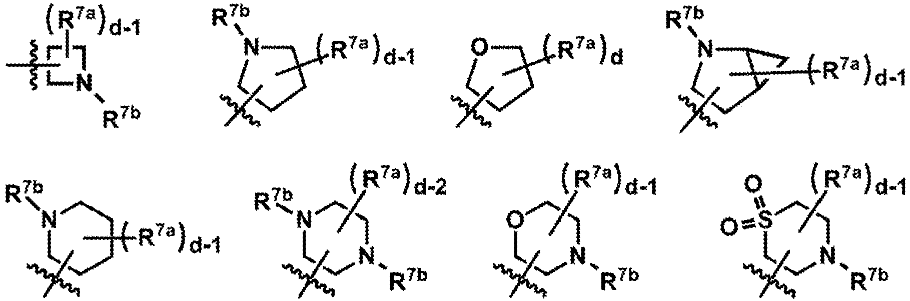 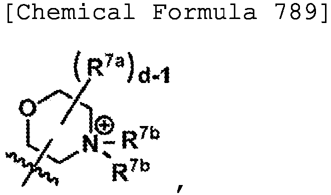
subscript d is the number of substitutable positions on a ring of R⁵,
each R^{7a} is independently selected from the group consisting of
1) a hydrogen atom,
2) a hydroxyl group,
3) halogen,
4) a C₁₋₄ alkyl group
(wherein the alkyl group is optionally substituted with NR^{e3}R^{f3}, a 5- or 6-membered non-aryl heterocycle, -C(=O)OR^{f3}, or a hydroxyl group),
5) a C₁₋₄ alkoxy group,
6) -NR^{e3}R^{f3},
7) -C(=O)OR^{e3},
8) C₆₋₁₀ aryl, and
9) -C(=O)NR^{e3}R^{f3},
each R^{7b} is independently selected from the group consisting of
1) a hydrogen atom,
2) a hydroxyl group, and
3) a C₁₋₄ alkyl group
(wherein the alkyl group is optionally substituted with NR^{e3}R^{f3}, - C(=O)OR^{f3}, or a hydroxyl group), and
R^{e3} and R^{f3} are defined the same as R^{e2} and R^{f2} according to any one of claims 84 to 86.

90. The compound or the pharmaceutically acceptable salt thereof according to any one of claims 1 to 3, 11 to 36, 66 to 74, and 77, wherein
R⁵ is C₄₋₆ cycloalkyl selected from the group consisting of 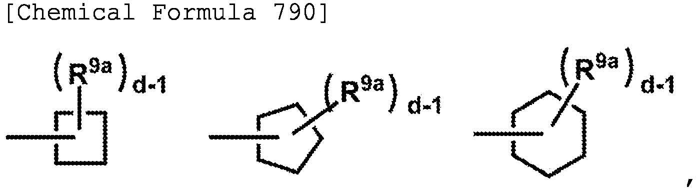
subscript d is the number of substitutable positions on a ring of R⁵,
each R^{9a} is independently selected from the group consisting of
1) a hydrogen atom,
2) a hydroxyl group,
3) a cyano group,
4) halogen,
5) a C₁₋₄ alkyl group,
6) a C₃₋₁₀ alicyclic group,
7) a C₁₋₄ alkoxy group,
8) a C₃₋₁₀ alicyclic oxy group,
9) a C₆₋₁₀ aryloxy group,
10) a 5- or 6-membered heteroaryloxy group,
11) a 4- to 10-membered non-aryl heterocyclyl oxy group,
(wherein each substituent from 5) and 11) is optionally substituted),
12) -SO₂-NR^{e3}R^{f3},
13) -NR^{g2}-CR^{e3}(=NR^{f3}),
14) -NR^{g2}-CR^{e3}(=N-OR^{f3}),
15) -NR^{h2}-C(=NR^{g2})NR^{e3}R^{f3},
16) -NR^{h2}-C(=N-OR^{g2})NR^{e3}R^{f3},
17) -NRⁱ²-C(=NR^{h2})NR^{g2}-NR^{e3}R^{f3},
18) -NRⁱ²-C(=N-OR^{h2})NR^{g2}NR^{e3}R^{f3},
19) -C (=NR^{e3})R^{f3},
20) -C(=N-OR^{e3})R^{f3},
21) -C (=NR^{h2})-NR^{e3}R^{f3},
22) -C(=NR^{h2})NR^{g2}NR^{e3}R^{f3},
23) -C (=N-OR^{h2})NR^{g2}NR^{e3}R^{f3},
24) -NR^{e3}R^{f3},
25) -NR^{g2}-NR^{e3}R^{f3},
26) -NR^{e3}OR^{f3},
27) -NR^{e3}-C(=O)R^{f3},
28) -C(=O)NR^{e3}R^{f3},
29) -C(=O)NR^{e3}OR^{f3},
30) -C (=O)NR^{g2}-NR^{e3}R^{f3},
31) -C(=O)R^{e3},
32) -C (=O)OR^{e3}, and
33) -C(=N-OR^{h2})NR^{e3}R^{f3}, and
R^{e3}, R^{f3}, R^{g2}, R^{h2}, and Rⁱ² are defined the same as R^{e2} and R^{f2} according to claim 3.

91. The compound or the pharmaceutically acceptable salt thereof according to any one of claims 1 to 3, 11 to 36, 66 to 74, 77, and 90, wherein
R⁵ is C₄₋₆ cycloalkyl selected from the group consisting of 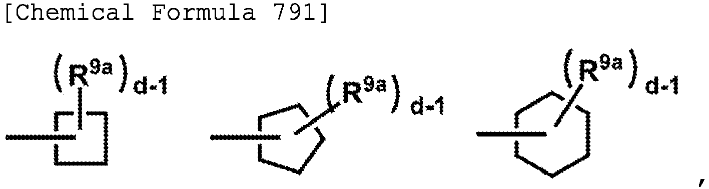
subscript d is the number of substitutable positions on a ring of R⁵,
each R^{9a} is independently selected from the group consisting of
1) a hydrogen atom,
2) a hydroxyl group,
3) halogen,
4) a C₁₋₄ alkyl group (wherein the alkyl group is optionally substituted with NR^{e3}R^{f3}, a 5- or 6-membered non-aryl heterocycle, -C(=O)OR^{f3}, or a hydroxyl group),
5) a C₁₋₄ alkoxy group,
6) -NR^{e3}R^{f3},
7) -C(=O)OR^{e3},
8) C₆₋₁₀ aryl, and
9) -C (=O)NR^{e3}R^{f3}, and
R^{e3} and R^{f3} are defined the same as R^{e2} and R^{f2} according to any one of claims 75 to 77.

92. The compound or the pharmaceutically acceptable salt thereof according to any one of claims 1 to 3, 11 to 36, and 79 to 80, wherein
L⁴ is -CH(NH₂)-CHR¹³-, wherein carbon that attaches to the NH₂ attaches to L³,
R⁵ is a hydrogen atom, and
R¹³ is
1) -NH-C (=O)CH₃,
2) -NH-C (=O)NH₂,
3) -NH-C(=O)CH(NH₂)-CH₂C(=O)NH₂,
4) -NH-C (=O)CH₂-NH₂,
5) -NH-C (=O)CH (NH₂)-CH₂OH, or
6) a pyrrolidin-2-ylcarbonylamino group.

93. The compound or the pharmaceutically acceptable salt thereof according to any one of claims 1 to 3, 11 to 36, and 79 to 80, wherein L⁴ is -CH (NH₂)-CR¹²R¹³-, wherein carbon that attaches to the NH₂ attaches to L³,
R⁵ is a hydrogen atom or methyl,
R¹² is a hydrogen atom or methyl, and
R¹³ is a benzylthio group or a sulfanyl group.

94. The compound or the pharmaceutically acceptable salt thereof according to any one of claims 1 to 3, 11 to 36, and 79 to 80, wherein
L⁴ is -CH(NH₂)-(CH₂)_{q}-CHR¹³-, wherein q is 0 or 1, and carbon that attaches to the NH₂ attaches to L³,
R⁵ is a hydrogen atom, and
R¹³ is
1) a carboxyl group,
2) -C(=O)NH₂,
3) -C(=O)NH(CH₃),
4) -C(=O)N(CH₃)₂,
5) -C(=O)NH-(CH₂)₂-OH,
6) -C(=O)NH- (CH₂)₂-NH₂,
7) -C(=O)NH-S (=O)₂-CH₃,
8) -C(=O)NHOH,
9) -S (=O)₂-NH₂,
10) -S(=O)₂-CH₃, or
11) a hydroxyl group.

95. The compound or the pharmaceutically acceptable salt thereof according to any one of claims 1 to 3, 11 to 36, and 79 to 80, wherein
L⁴ is -CH(NHR¹¹)-CH₂-, wherein carbon that attaches to the NHR¹¹ attaches to L³,
R⁵ is hydrogen, and
R¹¹ is
1) -C(=O)CH(NH₂)-CH₂C(=O)NH₂,
2) -C(=O)CH₂-NH₂,
3) -C(=O)CH(CH₃)-NH₂,
4) -C(=O)CH(NH₂)-CH₂OH, or
5) pyrrolidin-2-ylcarbonyl.

96. The compound or the pharmaceutically acceptable salt thereof according to any one of claims 1 to 3, 11 to 36, and 79 to 80, wherein
L⁴ is -CH (NHR¹¹)-CH (COOH) -, wherein carbon that attaches to the NHR¹¹ attaches to L³,
R⁵ is hydrogen, and
R¹¹ is
1) -C(=O)CH(NH₂)-CH₂C(=O)NH₂,
2) -C(=O)CH₂-NH₂,
3) -C(=O)CH(CH₃)-NH₂,
4) -C(=O)CH(NH₂)-CH₂OH, or
5) pyrrolidin-2-ylcarbonyl.

97. The compound or the pharmaceutically acceptable salt thereof according to any one of claims 1 to 3, 11 to 36, and 79 to 80, wherein
L⁴ is -CHR¹³- or -CH₂-CHR¹³-,
R⁵ is hydrogen, and
R¹³ is -C(=O)NH₂ or -C(=O)NHOH.

98. The compound or the pharmaceutically acceptable salt thereof according to any one of claims 1 to 3, 11 to 36, and 79 to 80, wherein
L⁴ is -CH₂-CR¹⁰(NH₂)-, wherein the CH₂ group attaches to L³,
R⁵ is hydrogen, and
R¹⁰ is a carboxy group or -C(=O)NH₂.

99. The compound or the pharmaceutically acceptable salt thereof according to any one of claims 1 to 3, 11 to 36, and 79 to 80, wherein
L⁴ is - (CH₂)ₚ-CR¹⁰(NHR¹¹)- (CH₂)_{q}-CHR¹³- or -CHR¹³-(CH₂)_{q}-CR¹⁰(NHR¹¹)- (CH₂)ₚ-, wherein q is 0 or 1,
R⁵ is hydrogen,
(1) if L⁴ is -CHR¹³-(CH₂)_{q}-CR¹⁰(NHR¹¹)-(CH₂)ₚ-, carbon of the -CHR¹³- group attaches to L³,
p is 0,
R¹⁰ is a hydrogen atom, a carboxyl group, or -C(=O)NHR^{10b},
R¹¹ is a hydrogen atom,
R^{10b} is a hydrogen atom,
wherein if R¹⁰ is -C (=O)NHR^{10b}, R^{10b} and R¹¹ together may form - CH₂CH₂-, and
R¹³ is a hydrogen atom,
(2) if L⁴ is -(CH₂)ₚ-CR¹⁰(NHR¹¹)-(CH₂)_{q}-CHR¹³-,
carbon of the -(CH₂)ₚ- group attaches to L³,
p is 1,
R¹⁰ and R¹¹ are both hydrogen atoms,
R¹³ is a carboxyl group or -C (=O)NR^{13a}R^{13b}, and
R^{13a} and R^{13b} are each independently a hydrogen atom or an optionally substituted C₁₋₄ alkyl group.

100. The compound or the pharmaceutically acceptable salt thereof according to any one of claims 1 to 3, 11 to 36, and 79 to 80, wherein
L⁴ is -CR¹²(NH₂)-,
R¹² is a hydrogen atom or a methyl group, and
R⁵ is a C₁₋₄ alkyl group optionally substituted with a hydroxyl group.

101. The compound or the pharmaceutically acceptable salt thereof according to claim 1 or 2, selected from the group consisting of the compounds represented by the following names or structural formulas:
5-({1-[amino(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 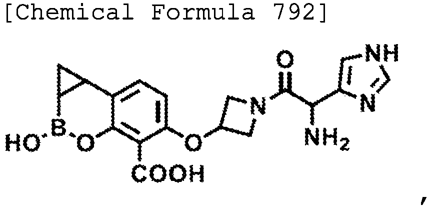
9-[1-[2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 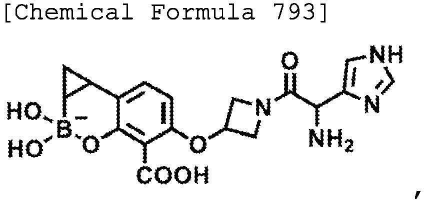
(1aS,7bR)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 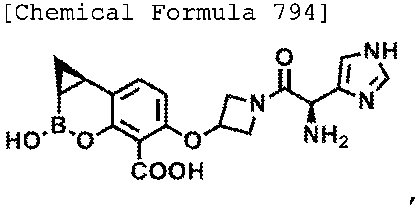
(2R,4S)-9-[1-[(2R)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 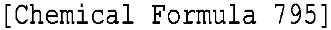 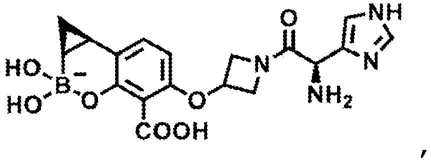
(1aS,7bR)-5-({1-[(2S)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 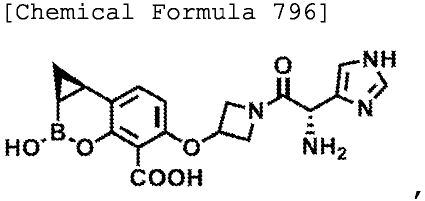
(2R,4S)-9-[1-[(2S)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 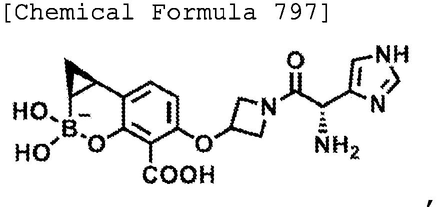
(1aR,7bS)-5-({1-[(2R)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 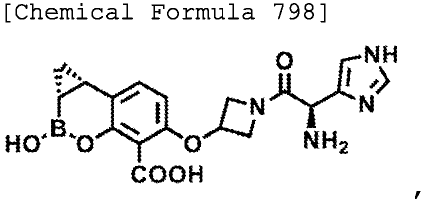
(2S,4R)-9-[1-[(2R)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 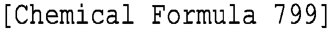 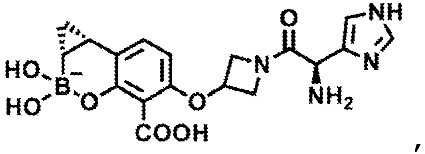
(1aR,7bS)-5-({1-[(2S)-2-amino-2-(1H-imidazol-4-yl)acetyl]azetidin-3-yl}oxy)-2-hydroxy-1,1a,2,7b-tetrahydrocyclopropa[c][1,2]benzoxaborinine-4-carboxylic acid 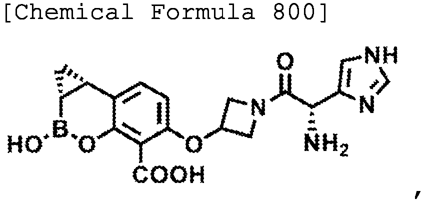
(2S,4R)-9-[1-[(2S)-2-amino-2-(1H-imidazol-4-yl)acetyl]azetidin-3-yl]oxy-5,5-dihydroxy-6-oxa-5-boranuidatricyclo[5.4.0.0^{2,4}]undeca-1(11),7,9-triene-8-carboxylic acid 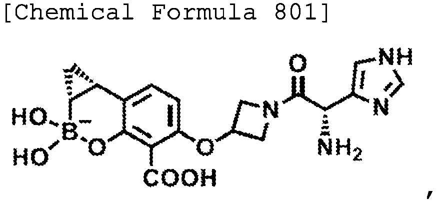
5-({1-(2-amino-2-carboxypropyl)azetidin-3-yl}oxy)-2-hydroxy-1,1a,2,7b-tetrahydrocyclopropa[c][1,2]benzoxaborinine-4-carboxylic acid 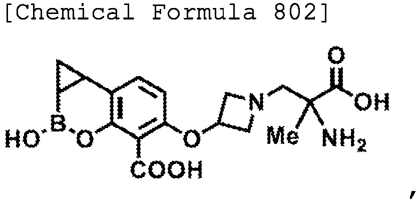
9-[1-(propyl 2-amino-2-carboxylate)azetidin-3-yl]oxy-5,5-dihydroxy-6-oxa-5-boranuidatricyclo[5.4.0.0^{2,4}]undeca-1(7),8,10-triene-8-carboxylate 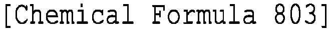 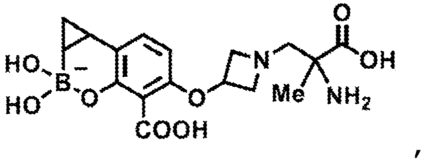
(1aS,7bR)-5-({1-[(2R)-2-amino-2-carboxypropyl]azetidin-3-yl}oxy)-2-hydroxy-1,1a,2,7b-tetrahydrocyclopropa[c][1,2]benzoxaborinine-4-carboxylic acid 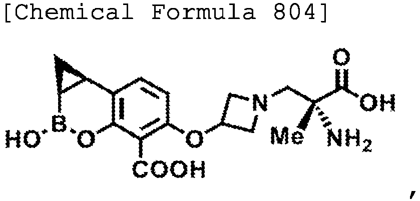
(2R,4S)-9-[1-[propyl (2R)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 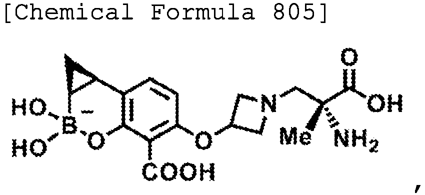
(1aR,7bS)-5-({1-[(2R)-2-amino-2-carboxypropyl]azetidin-3-yl}oxy)-2-hydroxy-1,1a,2,7b-tetrahydrocyclopropa[c][1,2]benzoxaborinine-4-carboxylic acid 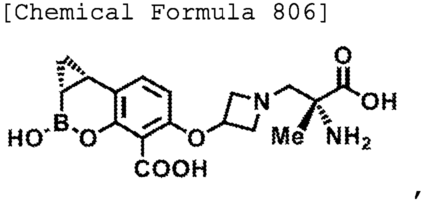
(2S,4R)-9-[1-[propyl (2R)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 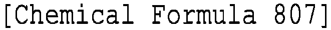 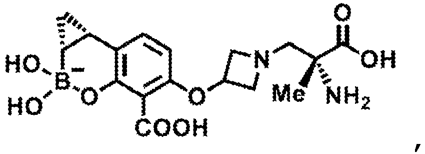
5-({1-[2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 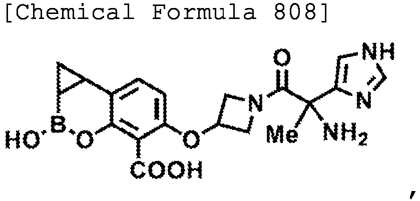
9-[1-[2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 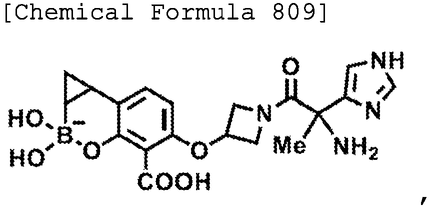
(1aS,7bR)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 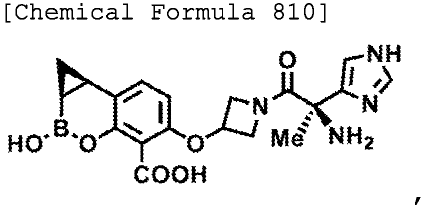
(2R,4S)-9-[1-[(2R)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 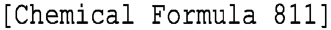 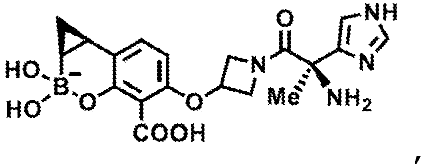
(1aS,7bR)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 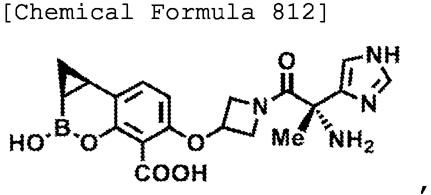
(2R,4S)-9-[1-[(2S)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 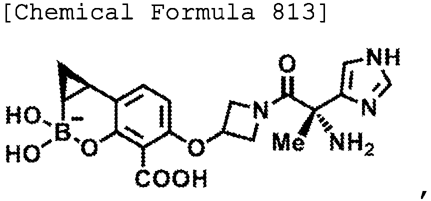
(1aR,7bS)-5-({1-[(2R)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 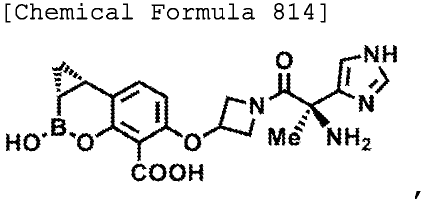
(2S,4R)-9-[1-[(2R)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid 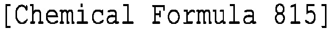 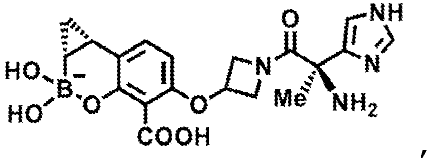
(1aR,7bS)-5-({1-[(2S)-2-amino-2-(1H-imidazol-4-yl)propanoyl]azetidin-3-yl}oxy)-2-hydroxy-1,1a,2,7b-tetrahydrocyclopropa[c][1,2]benzoxaborinine-4-carboxylic acid 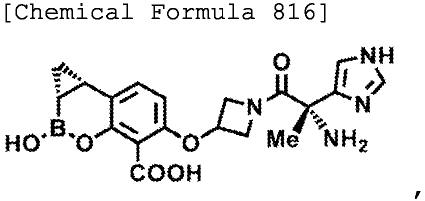 and
(2S,4R)-9-[1-[(2S)-2-amino-2-(1H-imidazol-4-yl)propanoyl]azetidin-3-yl]oxy-5,5-dihydroxy-6-oxa-5-boranuidatricyclo[5.4.0.0^{2,4}]undeca-1(11),7,9-triene-8-carboxylic acid

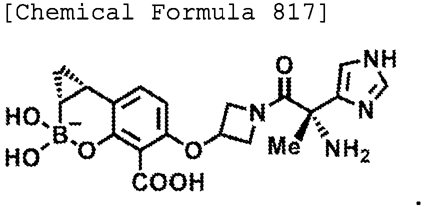

102. The compound or the pharmaceutically acceptable salt thereof according to claim 1 or 2, selected from the group consisting of the compounds represented by the following names or structural formulas:
2-hydroxy-5-({1-[(1H-imidazol-4-yl)acetyl]azetidin-3-yl}oxy)-1,1a,2,7b-tetrahydrocyclopropa[c][1,2]benzoxaborinine-4-carboxylic acid 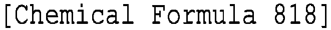 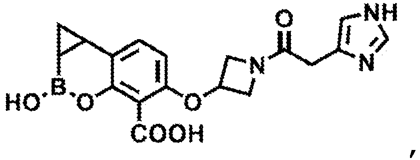
5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid 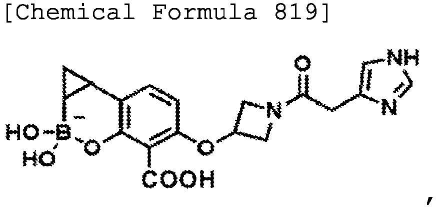
(1aS,7bR)-2-hydroxy-5-({1-[(1H-imidazol-4-yl)acetyl]azetidin-3-yl}oxy)-1,1a,2,7b-tetrahydrocyclopropa[c][1,2]benzoxaborinine-4-carboxylic acid 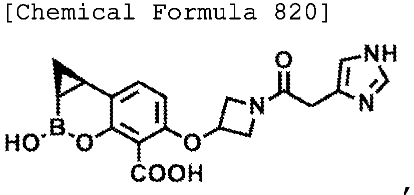
(2R,4S)-5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid 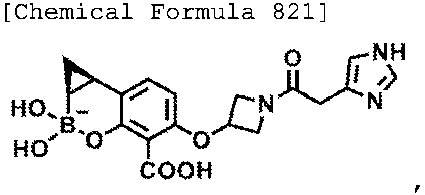
(1aR,7bS)-2-hydroxy-5-({1-[(1H-imidazol-4-yl)acetyl]azetidin-3-yl}oxy)-1,1a,2,7b-tetrahydrocyclopropa[c][1,2]benzoxaborinine-4-carboxylic acid 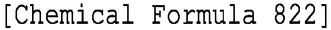 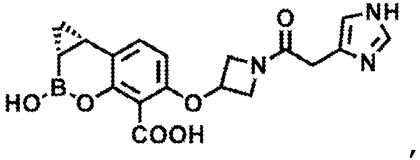
(2S,4R)-5,5-dihydroxy-9-{1-[(1H-imidazol-4-yl)acetyl]azetidin-3-yl}oxy-6-oxa-5-boranuidatricyclo[5.4.0.0^{2,4}]undeca-1(11),7,9-triene-8-carboxylic acid 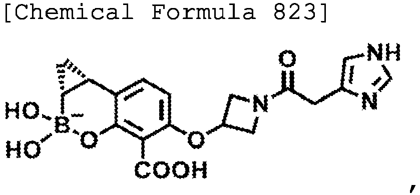
2-hydroxy-5-[(1-serylazetidin-3-yl)oxy]-1,1a,2,7b-tetrahydrocyclopropa[c][1,2]benzoxaborinine-4-carboxylic acid 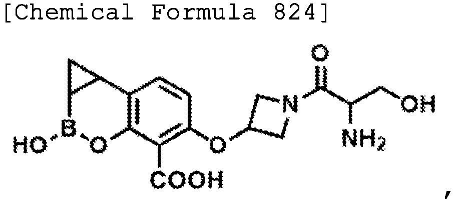
5,5-dihydroxy-9-(1-serylazetidin-3-yl)oxy-6-oxa-5-boranuidatricyclo[5.4.0.0^{2,4}]undeca-1(11),7,9-triene-8-carboxylic acid 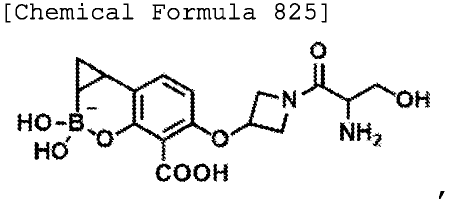
(1aS,7bR)-2-hydroxy-5-[(1-D-serylazetidin-3-yl)oxy]-1,1a,2,7b-tetrahydrocyclopropa[c][1,2]benzoxaborinine-4-carboxylic acid 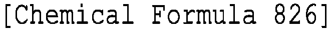 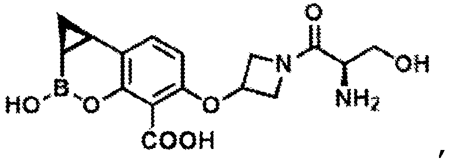
(2R,4S)-5,5-dihydroxy-9-(1-D-serylazetidin-3-yl)oxy-6-oxa-5-boranuidatricyclo[5.4.0.0^{2,4}]undeca-1(11),7,9-triene-8-carboxylic acid 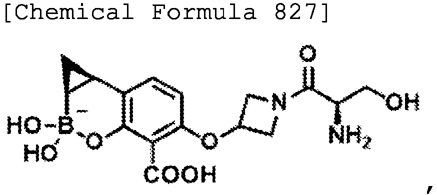
(1aS,7bR)-2-hydroxy-5-[(1-L-serylazetidin-3-yl)oxy]-1,1a,2,7b-tetrahydrocyclopropa[c][1,2]benzoxaborinine-4-carboxylic acid 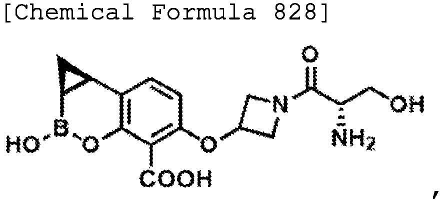
(2R,4S)-5,5-dihydroxy-9-(1-L-serylazetidin-3-yl)oxy-6-oxa-5-boranuidatricyclo[5.4.0.0^{2,4}]undeca-1(11),7,9-triene-8-carboxylic acid 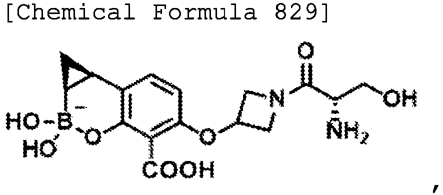
(1aR,7bS)-2-hydroxy-5-[(1-D-serylazetidin-3-yl)oxy]-1,1a,2,7b-tetrahydrocyclopropa[c][1,2]benzoxaborinine-4-carboxylic acid 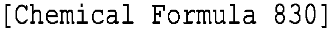 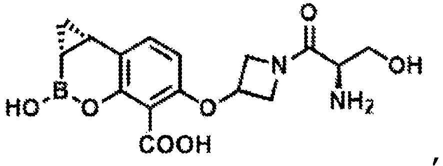
(2S,4R)-5,5-dihydroxy-9-(1-D-serylazetidin-3-yl)oxy-6-oxa-5-boranuidatricyclo[5.4.0.0^{2,4}]undeca-1(11),7,9-triene-8-carboxylic acid 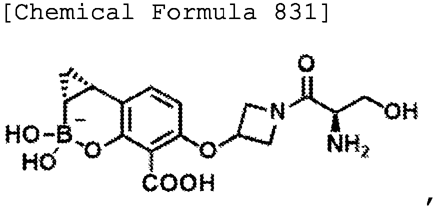
(1aR,7bS)-2-hydroxy-5-[(1-L-serylazetidin-3-yl)oxy]-1,1a,2,7b-tetrahydrocyclopropa[c][1,2]benzoxaborinine-4-carboxylic acid 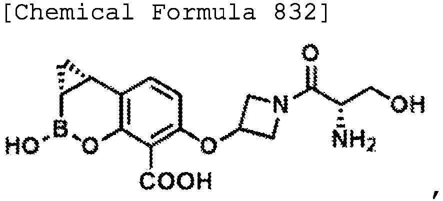
(2S,4R)-5,5-dihydroxy-9-(1-L-serylazetidin-3-yl)oxy-6-oxa-5-boranuidatricyclo[5.4.0.0^{2,4}]undeca-1(11),7,9-triene-8-carboxylic acid 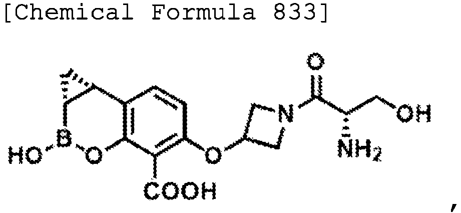
2-hydroxy-5-({1-[4-hydroxy-prolyl]azetidin-3-yl}oxy)-1,1a,2,7b-tetrahydrocyclopropa[c][1,2]benzoxaborinine-4-carboxylic acid 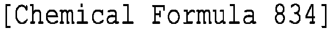 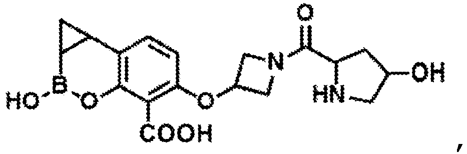
5,5-dihydroxy-9-{1-[4-hydroxy-prolyl]azetidin-3-yl}oxy-6-oxa-5-boranuidatricyclo[5.4.0.0^{2,4}]undeca-1(11),7,9-triene-8-carboxylic acid 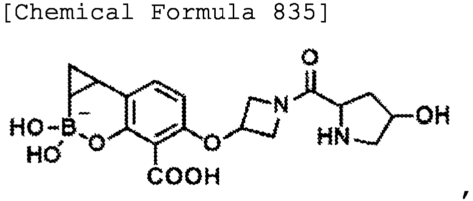
(1aS,7bR)-2-hydroxy-5-({1-[(4R)-4-hydroxy-L-prolyl]azetidin-3-yl}oxy)-1,1a,2,7b-tetrahydrocyclopropa[c][1,2]benzoxaborinine-4-carboxylic acid 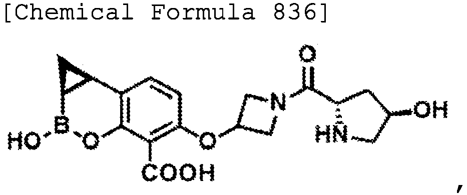
(2R,4S)-5,5-dihydroxy-9-{1-[(4R)-4-hydroxy-L-prolyl]azetidin-3-yl}oxy-6-oxa-5-boranuidatricyclo[5.4.0.0^{2,4}]undeca-1(11),7,9-triene-8-carboxylic acid 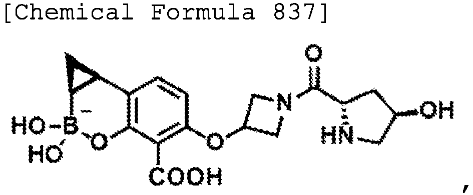
(1aS,7bR)-2-hydroxy-5-({1-[(4S)-4-hydroxy-L-prolyl]azetidin-3-yl}oxy)-1,1a,2,7b-tetrahydrocyclopropa[c][1,2]benzoxaborinine-4-carboxylic acid 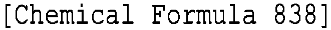 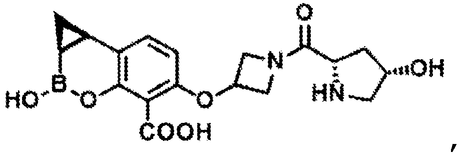
(2R,4S)-5,5-dihydroxy-9-{1-[(4S)-4-hydroxy-L-prolyl]azetidin-3-yl}oxy-6-oxa-5-boranuidatricyclo[5.4.0.0^{2,4}]undeca-1(11),7,9-triene-8-carboxylic acid 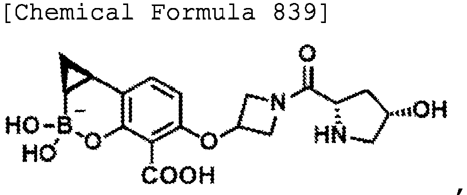
(1aS,7bR)-2-hydroxy-5-({1-[(4R)-4-hydroxy-D-prolyl]azetidin-3-yl}oxy)-1,1a,2,7b-tetrahydrocyclopropa[c][1,2]benzoxaborinine-4-carboxylic acid 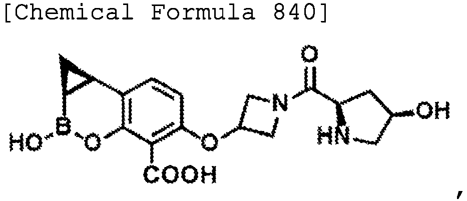
(2R,4S)-5,5-dihydroxy-9-{1-[(4R)-4-hydroxy-D-prolyl]azetidin-3-yl}oxy-6-oxa-5-boranuidatricyclo[5.4.0.0^{2,4}]undeca-1(11),7,9-triene-8-carboxylic acid 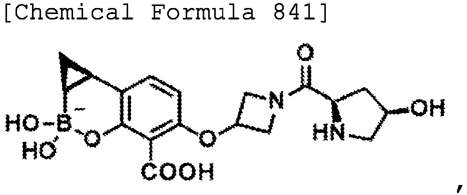
(1aS,7bR)-2-hydroxy-5-({1-[(4S)-4-hydroxy-D-prolyl]azetidin-3-yl}oxy)-1,1a,2,7b-tetrahydrocyclopropa[c][1,2]benzoxaborinine-4-carboxylic acid 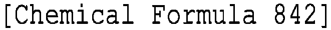 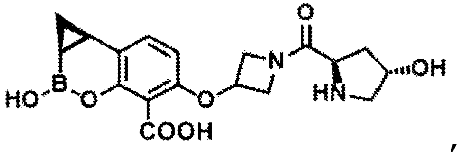
(2R,4S)-5,5-dihydroxy-9-{1-[(4S)-4-hydroxy-D-prolyl]azetidin-3-yl}oxy-6-oxa-5-boranuidatricyclo[5.4.0.0^{2,4}]undeca-1(11),7,9-triene-8-carboxylic acid 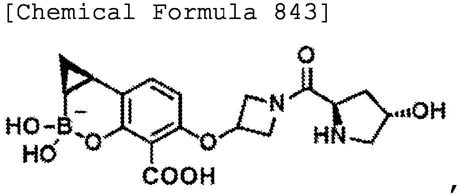
(1aR,7bS)-2-hydroxy-5-({1-[(4R)-4-hydroxy-L-prolyl]azetidin-3-yl}oxy)-1,1a,2,7b-tetrahydrocyclopropa[c][1,2]benzoxaborinine-4-carboxylic acid 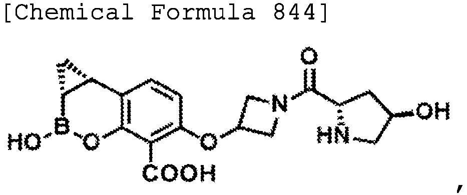
(2S,4R)-5,5-dihydroxy-9-{1-[(4R)-4-hydroxy-L-prolyl]azetidin-3-yl}oxy-6-oxa-5-boranuidatricyclo[5.4.0.0^{2,4}]undeca-1(11),7,9-triene-8-carboxylic acid 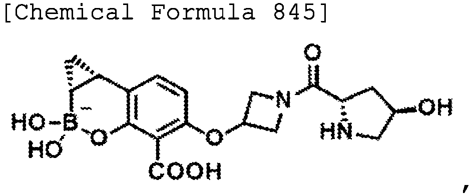
(1aR,7bS)-2-hydroxy-5-({1-[(4S)-4-hydroxy-L-prolyl]azetidin-3-yl}oxy)-1,1a,2,7b-tetrahydrocyclopropa[c][1,2]benzoxaborinine-4-carboxylic acid 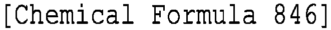 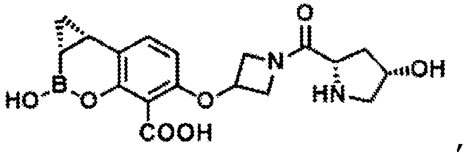
(2S,4R)-5,5-dihydroxy-9-{1-[(4S)-4-hydroxy-L-prolyl]azetidin-3-yl}oxy-6-oxa-5-boranuidatricyclo[5.4.0.0^{2,4}]undeca-1(11),7,9-triene-8-carboxylic acid 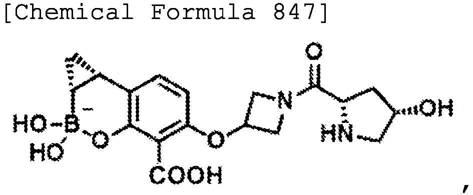
(1aS,7bR)-2-hydroxy-5-({1-[(4R)-4-hydroxy-D-prolyl]azetidin-3-yl}oxy)-1,1a,2,7b-tetrahydrocyclopropa[c][1,2]benzoxaborinine-4-carboxylic acid 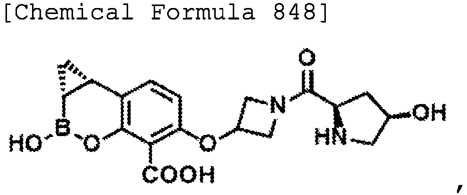
(2S,4R)-5,5-dihydroxy-9-{1-[(4R)-4-hydroxy-D-prolyl]azetidin-3-yl}oxy-6-oxa-5-boranuidatricyclo[5.4.0.0^{2,4}]undeca-1(11),7,9-triene-8-carboxylic acid 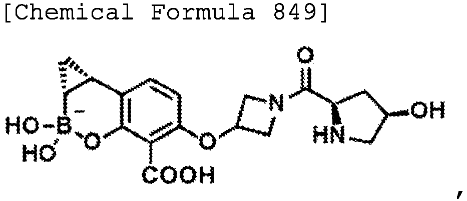
(1aR,7bS)-2-hydroxy-5-({1-[(4S)-4-hydroxy-D-prolyl]azetidin-3-yl}oxy)-1,1a,2,7b-tetrahydrocyclopropa[c][1,2]benzoxaborinine-4-carboxylic acid 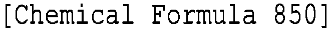 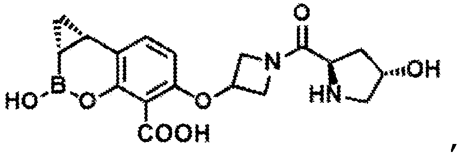
(2S,4R)-5,5-dihydroxy-9-{1-[(4S)-4-hydroxy-D-prolyl]azetidin-3-yl}oxy-6-oxa-5-boranuidatricyclo[5.4.0.0^{2,4}]undeca-1(11),7,9-triene-8-carboxylic acid 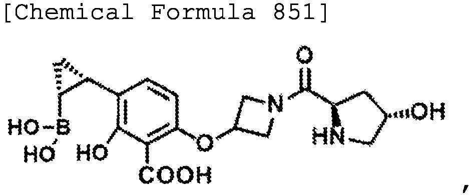
2-hydroxy-5-{[1-(2-methyl-seryl)azetidin-3-yl]oxy}-1,1a,2,7b-tetrahydrocyclopropa[c][1,2]benzoxaborinine-4-carboxylic acid 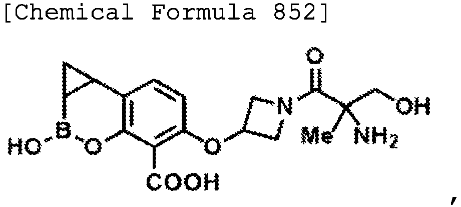
5,5-dihydroxy-9-[1-(2-methyl-seryl)azetidin-3-yl]oxy-6-oxa-5-boranuidatricyclo[5.4.0.0^{2,4}]undeca-1(11),7,9-triene-8-carboxylic acid 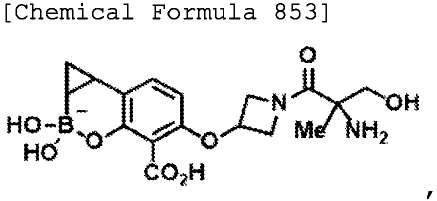
(1aS,7bR)-2-hydroxy-5-{[1-(2-methyl-D-seryl)azetidin-3-yl]oxy}-1,1a,2,7b-tetrahydrocyclopropa[c][1,2]benzoxaborinine-4-carboxylic acid 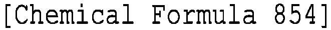 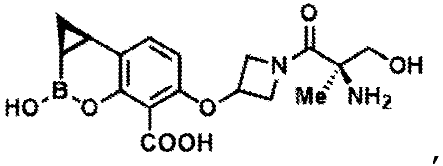
(2R,4S)-5,5-dihydroxy-9-[1-(2-methyl-D-seryl)azetidin-3-yl]oxy-6-oxa-5-boranuidatricyclo[5.4.0.0^{2,4}]undeca-1(11),7,9-triene-8-carboxylic acid 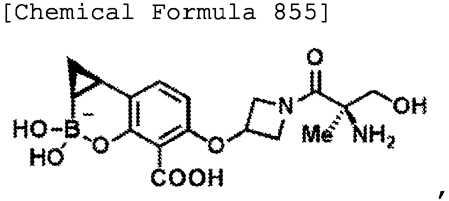
(1aS,7bR)-2-hydroxy-5-{[1-(2-methyl-L-seryl)azetidin-3-yl]oxy}-1,1a,2,7b-tetrahydrocyclopropa[c][1,2]benzoxaborinine-4-carboxylic acid 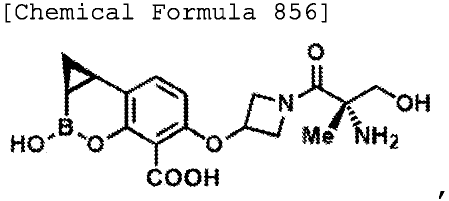
(2R,4S)-5,5-dihydroxy-9-[1-(2-methyl-L-seryl)azetidin-3-yl]oxy-6-oxa-5-boranuidatricyclo[5.4.0.0^{2,4}]undeca-1(11),7,9-triene-8-carboxylic acid 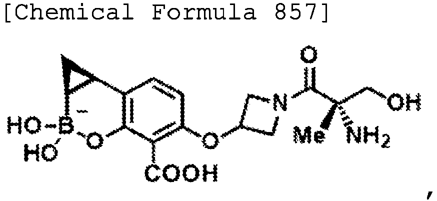
(1aR,7bS)-2-hydroxy-5-{[1-(2-methyl-D-seryl)azetidin-3-yl]oxy}-1,1a,2,7b-tetrahydrocyclopropa[c][1,2]benzoxaborinine-4-carboxylic acid 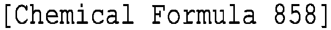 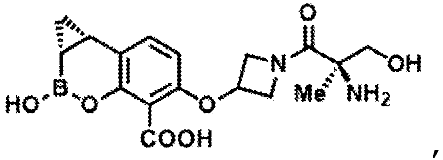
(2S,4R)-5,5-dihydroxy-9-[1-(2-methyl-D-seryl)azetidin-3-yl]oxy-6-oxa-5-boranuidatricyclo[5.4.0.0^{2,4}]undeca-1(11),7,9-triene-8-carboxylic acid 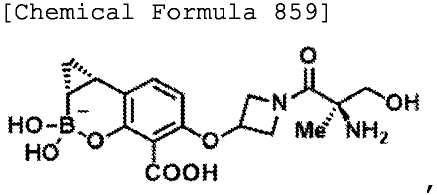
(1aR,7bS)-2-hydroxy-5-{[1-(2-methyl-L-seryl)azetidin-3-yl]oxy}-1,1a,2,7b-tetrahydrocyclopropa[c][1,2]benzoxaborinine-4-carboxylic acid 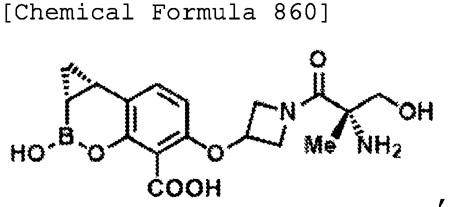
(2S,4R)-5,5-dihydroxy-9-[1-(2-methyl-L-seryl)azetidin-3-yl]oxy-6-oxa-5-boranuidatricyclo[5.4.0.0^{2,4}]undeca-1(11),7,9-triene-8-carboxylic acid 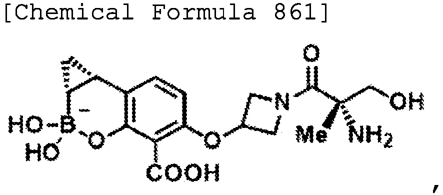
2-hydroxy-5-[(1-{[morpholin-2-yl]acetyl}azetidin-3-yl)oxy]-1,1a,2,7b-tetrahydrocyclopropa[c][1,2]benzoxaborinine-4-carboxylic acid 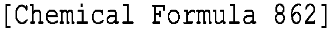 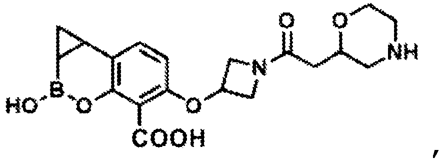
5,5-dihydroxy-9-(1-{[morpholin-2-yl]acetyl}azetidin-3-yl)oxy-5-boranuidatricyclo[5.4.0.0^{2,4}]undeca-1(11),7,9-triene-8-carboxylic acid 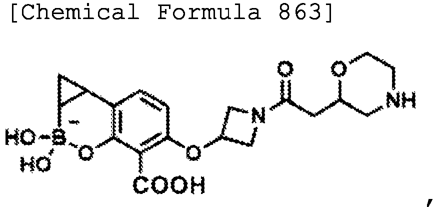
(1aS,7bR)-2-hydroxy-5-[(1-{[(2R)-morpholin-2-yl]acetyl}azetidin-3-yl)oxy]-1,1a,2,7b-tetrahydrocyclopropa[c][1,2]benzoxaborinine-4-carboxylic acid 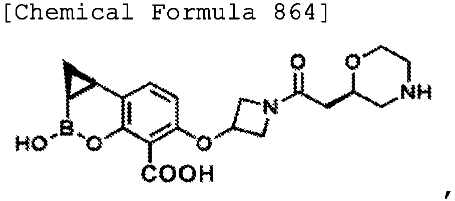
(2R,4S)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid 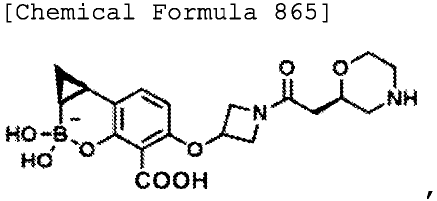
(1aS,7bR)-2-hydroxy-5-[(1-{[(2S)-morpholin-2-yl]acetyl}azetidin-3-yl)oxy]-1,1a,2,7b-tetrahydrocyclopropa[c][1,2]benzoxaborinine-4-carboxylic acid 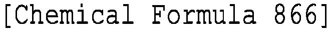 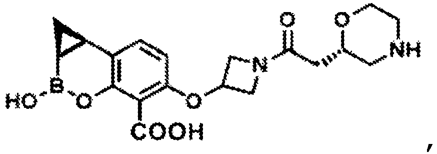
(2S,4R)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid 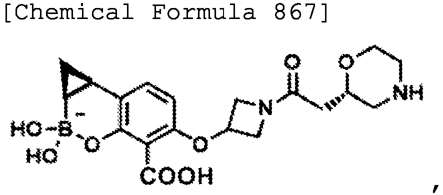
(1aR,7bS)-2-hydroxy-5-[(1-{[(2R)-morpholin-2-yl]acetyl}azetidin-3-yl)oxy]-1,1a,2,7b-tetrahydrocyclopropa[c][1,2]benzoxaborinine-4-carboxylic acid 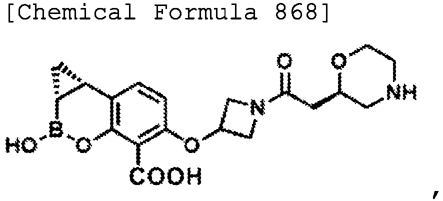
(2S,4R)-5,5-dihydroxy-9-(1-{[(2R)-morpholin-2-yl]acetyl}azetidin-3-yl)oxy-5-boranuidatricyclo[5.4.0.0^{2,4}]undeca-1(11),7,9-triene-8-carboxylic acid 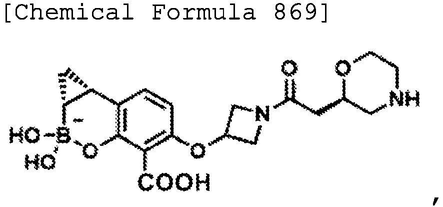
(1aR,7bS)-2-hydroxy-5-[(1-{[(2S)-morpholin-2-yl]acetyl}azetidin-3-yl)oxy]-1,1a,2,7b-tetrahydrocyclopropa[c][1,2]benzoxaborinine-4-carboxylic acid 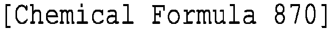 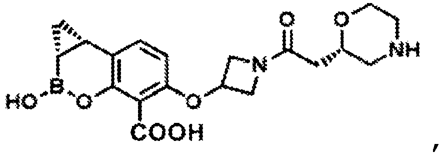
(2S,4R)-5,5-dihydroxy-9-(1-{[(2S)-morpholin-2-yl]acetyl}azetidin-3-yl)oxy-5-boranuidatricyclo[5.4.0.0^{2,4}]undeca-1(11),7,9-triene-8-carboxylic acid 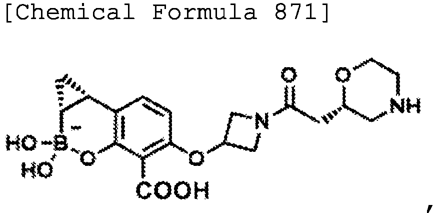
(1aS,7bR)-5-({1-[(2S)-2-amino-2-carboxypropyl]azetidin-3-yl}oxy)-2-hydroxy-1,1a,2,7b-tetrahydrocyclopropa[c][1,2]benzoxaborinine-4-carboxylic acid 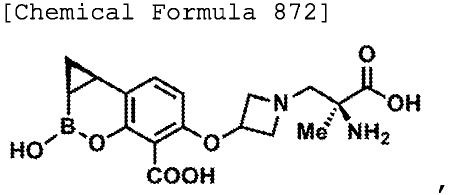
(2R,4S)-9-[1-[propyl (2S)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 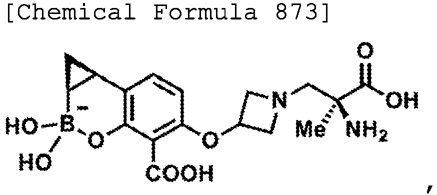
(1aR,7bS)-5-({1-[(2S)-2-amino-2-carboxypropyl]azetidin-3-yl}oxy)-2-hydroxy-1,1a,2,7b-tetrahydrocyclopropa[c][1,2]benzoxaborinine-4-carboxylic acid 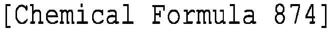 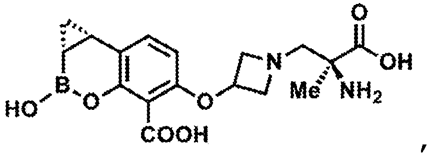
(2S,4R)-9-[1-[propyl (2S)-2-amino-2-carboxylate]azetidin-3-yl]oxy-5,5-dihydroxy-6-oxa-5-boranuidatricyclo[5.4.0.0^{2,4}]undeca-1(7),8,10-triene-8-carboxylate 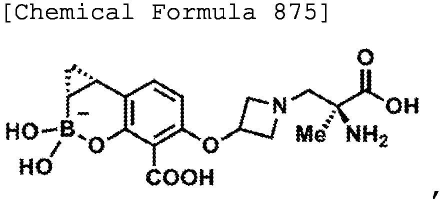
5-({1-[2-amino-2-carboxypropyl]azetidin-3-yl}oxy)-2-hydroxy-1,1a,2,7b-tetrahydrocyclopropa[c][1,2]benzoxaborinine-4-carboxylic acid 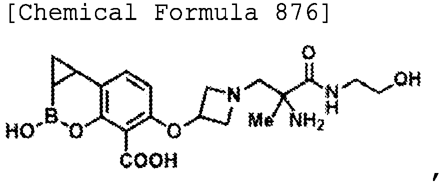
9-(1-{2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 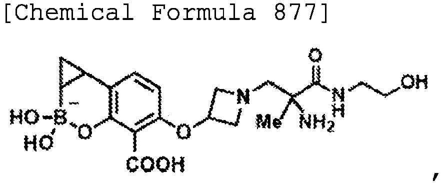
(1aS,7bR)-5-[(1-{(2R)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 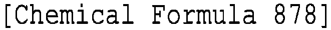 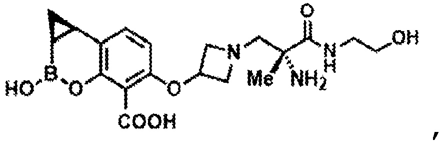
(2R,4S)-9-(1-{(2R)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 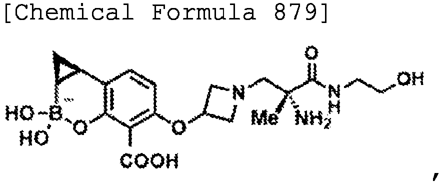
(1aS,7bR)-5-[(1-{(2S)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 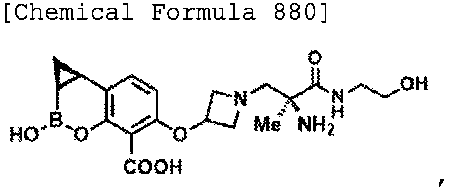
(2R,4S)-9-(1-{(2S)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 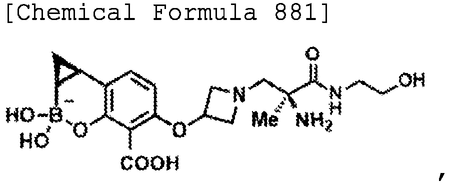
(1aR,7bS)-5-[(1-{(2R)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 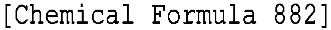 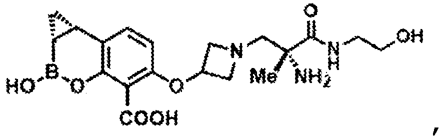
(2S,4R)-9-(1-{(2R)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 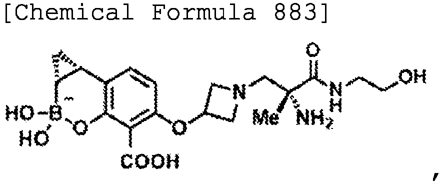
(1aR,7bS)-5-[(1-{(2S)-2-amino-3-[(2-hydroxyethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 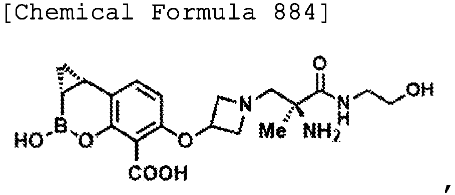
(2S,4R)-9-(1-{(2S)-2-amino-3-[(2-hydroxyethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 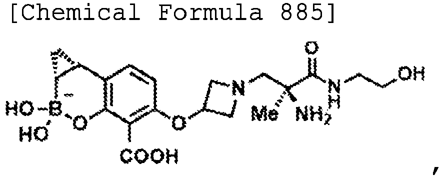
5-[(1-{2-amino-3-[(2-amino-2-oxoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 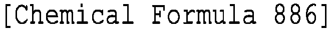 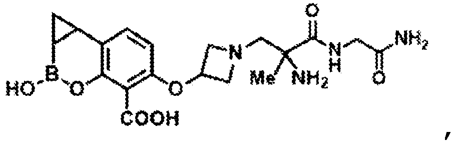
9-(1-{2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 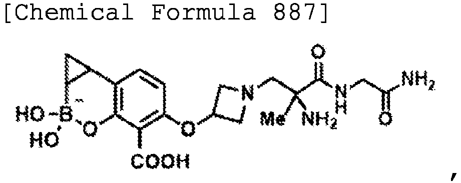
(1aS,7bR)-5-[(1-{(2R)-2-amino-3-[(2-amino-2-oxoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 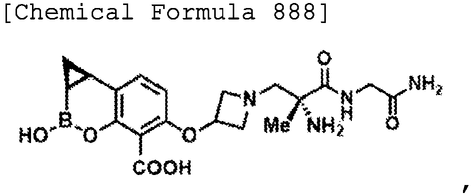
(2R,4S)-9-(1-{(2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 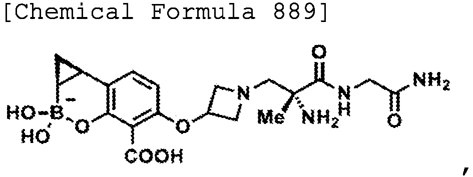
(1aS,7bR)-5-[(1-{(2S)-2-amino-3-[(2-amino-2-oxoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 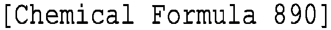 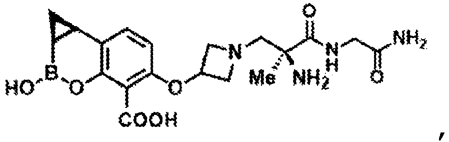
(2R,4S)-9-(1-{(2S)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 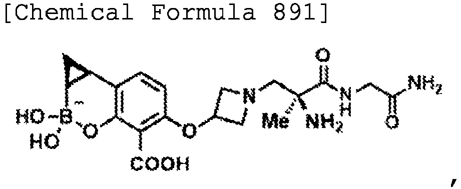
(1aR,7bS)-5-[(1-{(2R)-2-amino-3-[(2-amino-2-oxoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 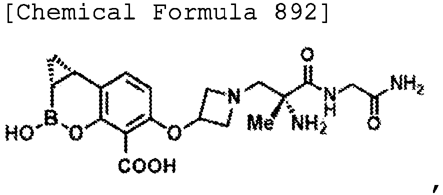
(2S,4R)-9-(1-{(2R)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 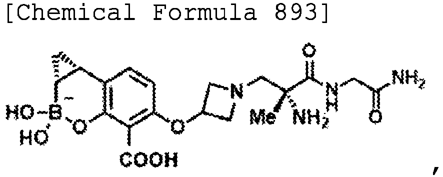
(1aR,7bS)-5-[(1-{(2S)-2-amino-3-[(2-amino-2-oxoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 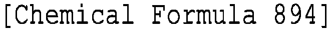 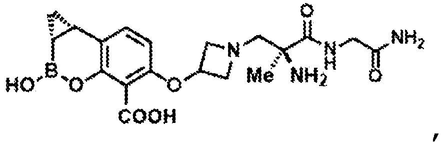
(2S,4R)-9-(1-{(2S)-2-amino-3-[(2-amino-2-oxoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 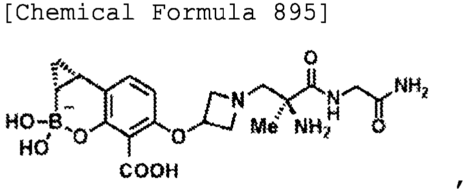
5-({1-[5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 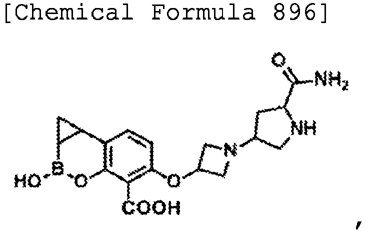
9-{1-[5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 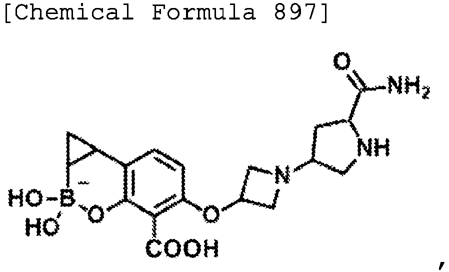
(1aS,7bR)-5-({1-[(3S,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 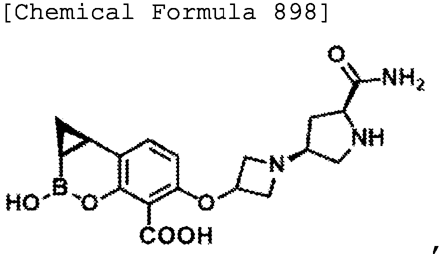
(2R,4S)-9-{1-[(3S,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 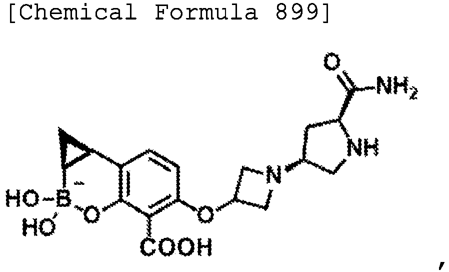
(1aS,7bR)-5-({1-[(3R,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 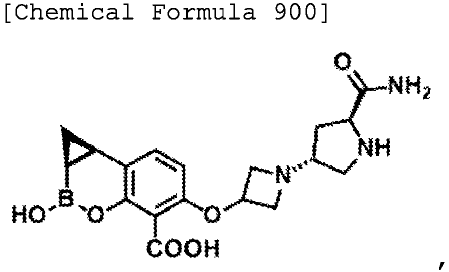
(2R,4S)-9-{1-[(3R,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 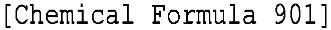 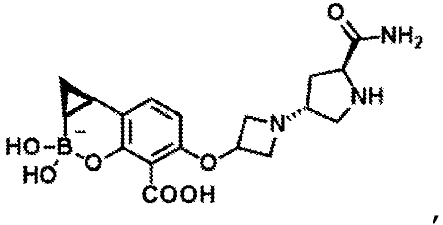
(1aS,7bR)-5-({1-[(3S,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 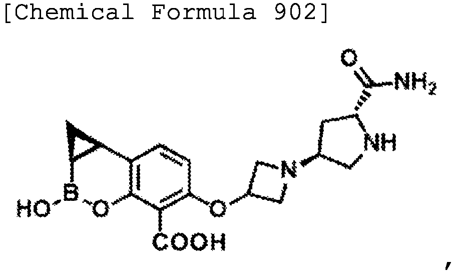
(2R,4S)-9-{1-[(3S,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 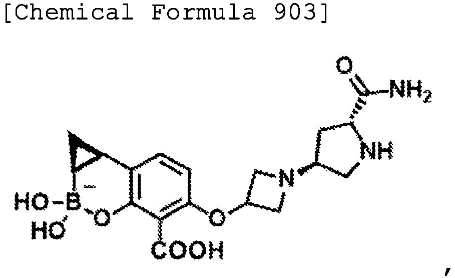
(1aS,7bR)-5-({1-[(3R,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 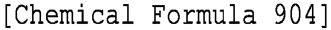 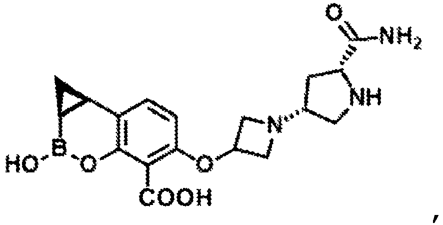
(2R,4S)-9-{1-[(3R,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 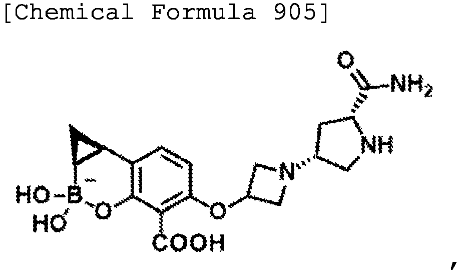
(1aR,7bS)-5-({1-[(3S,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 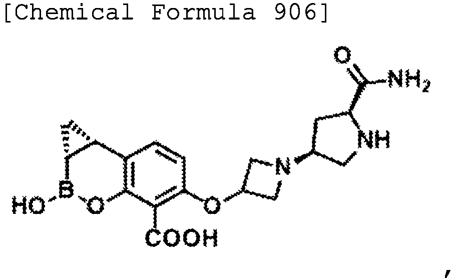
(2S,4R)-9-{1-[(3S,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 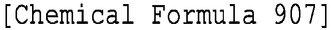 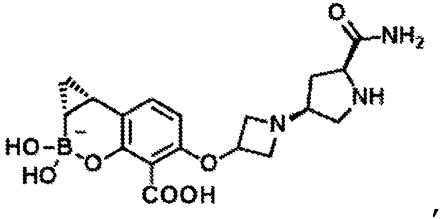
(1aR,7bS)-5-({1-[(3R,5S)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 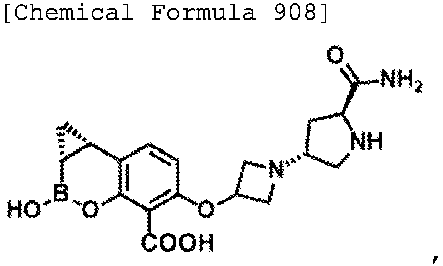
(2S,4R)-9-{1-[(3R,5S)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 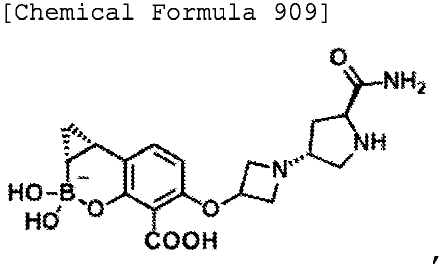
(1aR,7bS)-5-({1-[(3S,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 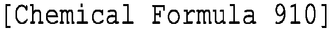 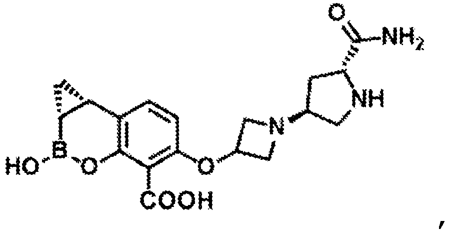
(2S,4R)-9-{1-[(3S,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 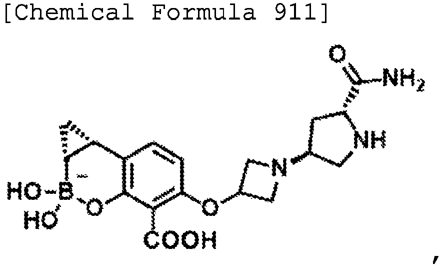
(1aR,7bS)-5-({1-[(3R,5R)-5-carbamoylpyrrolidin-3-yl]azetidin-3-yl}oxy)-2-hydroxy-1,1a,2,7b-tetrahydrocyclopropa[c][1,2]benzoxaborinine-4-carboxylic acid 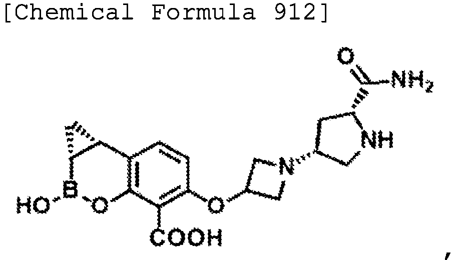
(2S,4R)-9-{1-[(3R,5R)-5-carbamoylpyrrolidin-3-yl]azetidin-3-yl}oxy-5,5-dihydroxy-6-oxa-5-boranuidatricyclo[5.4.0.0^{2,4}]undeca-1(7),8,10-triene-8-carboxylate 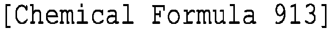 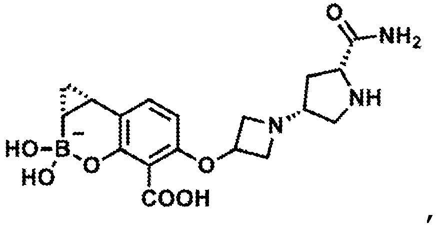
5-{[1-(2-amino-2-oxoethyl)azetidin-3-yl]oxy}-2-hydroxy-1,1a,2,7b-tetrahydrocyclopropa[c][1,2]benzoxaborinine-4-carboxylic acid 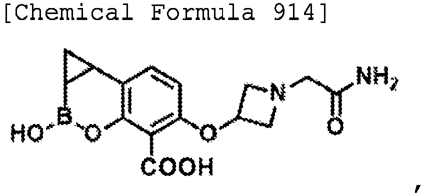
9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylate 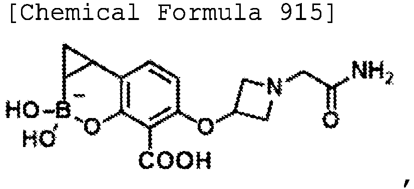
(1aS,7bR)-5-{[1-(2-amino-2-oxoethyl)azetidin-3-yl]oxy}-2-hydroxy-1,1a,2,7b-tetrahydrocyclopropa[c][1,2]benzoxaborinine-4-carboxylic acid 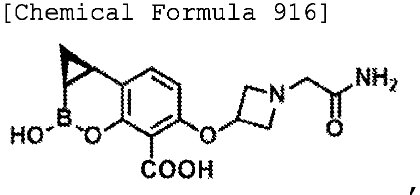
(2R,4S)-9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylate 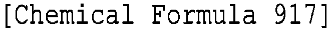 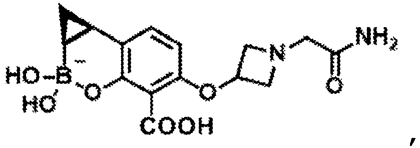
(1aR,7bS)-5-{[1-(2-amino-2-oxoethyl)azetidin-3-yl]oxy}-2-hydroxy-1,1a,2,7b-tetrahydrocyclopropa[c][1,2]benzoxaborinine-4-carboxylic acid 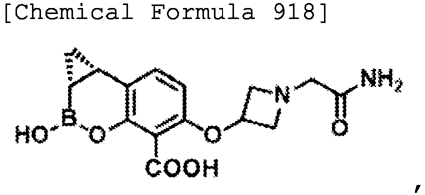
(2S,4R)-9-[1-(2-amino-2-oxoethyl)azetidin-3-yl]oxy-5,5-dihydroxy-6-oxa-5-boranuidatricyclo[5.4.0.0^{2,4}]undeca-1(7),8,10-triene-8-carboxylate 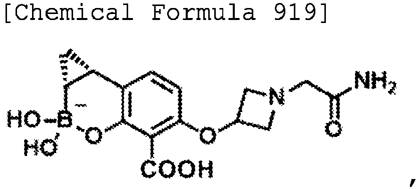
2-hydroxy-5-[(1-{2-[(2-hydroxyethoxy)amino]-2-oxoethyl}azetidin-3-yl)oxy]-1,1a,2,7b-tetrahydrocyclopropa[c][1,2]benzoxaborinine-4-carboxylic acid 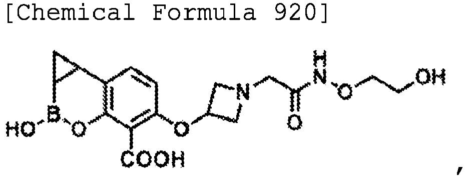
5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid 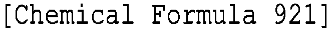 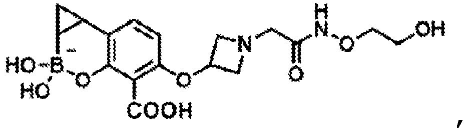
(1aS,7bR)-2-hydroxy-5-[(1-{2-[(2-hydroxyethoxy)amino]-2-oxoethyl}azetidin-3-yl)oxy]-1,1a,2,7b-tetrahydrocyclopropa[c][1,2]benzoxaborinine-4-carboxylic acid 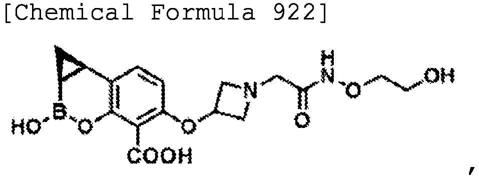
(2R,4S)-5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid 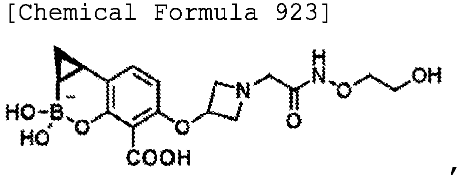
(1aR,7bS)-2-hydroxy-5-[(1-{2-[(2-hydroxyethoxy)amino]-2-oxoethyl}azetidin-3-yl)oxy]-1,1a,2,7b-tetrahydrocyclopropa[c][1,2]benzoxaborinine-4-carboxylic acid 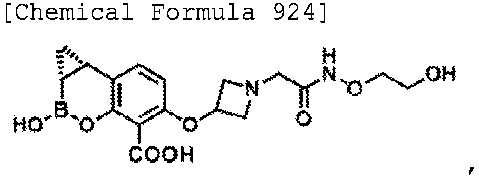
(2S,4R)-5,5-dihydroxy-9-(1-{2-[(2-hydroxyethoxy)amino]-2-oxoethyl}azetidin-3-yl)oxy-5-boranuidatricyclo[5.4.0.0^{2,4}]undeca-1(11),7,9-triene-8-carboxylic acid 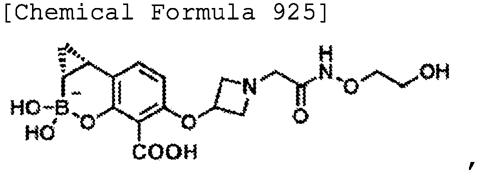
5-({1-[2-amino-3-hydroxy-2-(hydroxymethyl)propyl]azetidin-3-yl}oxy)-2-hydroxy-1,1a,2,7b-tetrahydrocyclopropa[c][1,2]benzoxaborinine-4-carboxylic acid 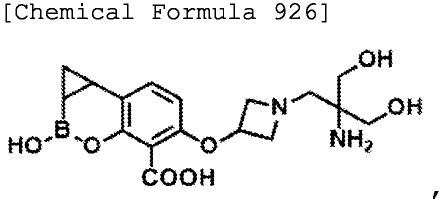
9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylate 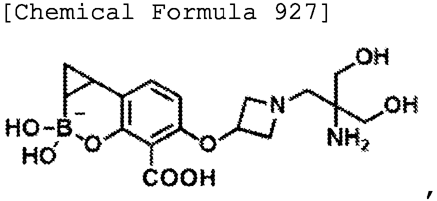
(1aS,7bR)-5-({1-[2-amino-3-hydroxy-2-(hydroxymethyl)propyl]azetidin-3-yl}oxy)-2-hydroxy-1,1a,2,7b-tetrahydrocyclopropa[c][1,2]benzoxaborinine-4-carboxylic acid 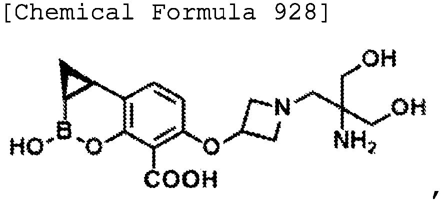
(2R,4S)-9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylate 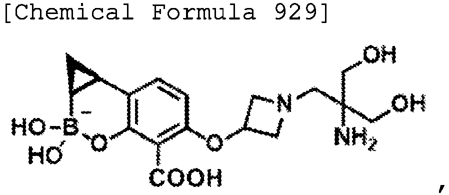
(1aR,7bS)-5-({1-[2-amino-3-hydroxy-2-(hydroxymethyl)propyl]azetidin-3-yl}oxy)-2-hydroxy-1,1a,2,7b-tetrahydrocyclopropa[c][1,2]benzoxaborinine-4-carboxylic acid 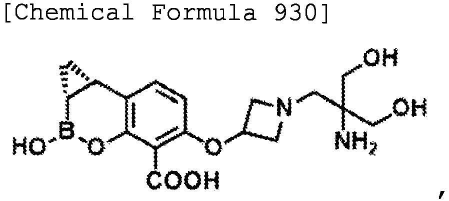
(2S,4R)-9-{1-[2-amino-3-hydroxy-2-(hydroxymethyl)propyl]azetidin-3-yl}oxy-5,5-dihydroxy-6-oxa-5-boranuidatricyclo[5.4.0.0^{2,4}]undeca-1(7),8,10-triene-8-carboxylate 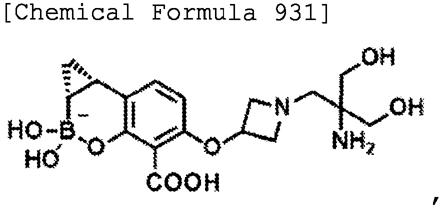
5-({1-[(1-aminocyclopropyl)methyl]azetidin-3-yl}oxy)-2-hydroxy-1,1a,2,7b-tetrahydrocyclopropa[c][1,2]benzoxaborinine-4-carboxylic acid 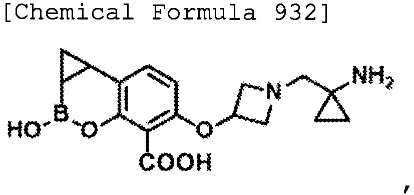
9-{1-[(1-aminocyclopropyl)methyl]azetidin-3-yl}oxy-5,5-dihydroxy-6-oxa-5-boranuidatricyclo[5.4.0.0^{2,4}]undeca-1(7),8,10-triene-8-carboxylate 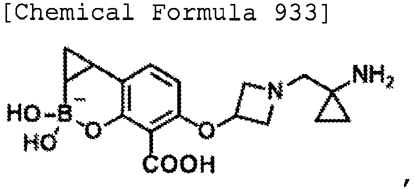
(1aS,7bR)-5-({1-[(1-aminocyclopropyl)methyl]azetidin-3-yl}oxy)-2-hydroxy-1,1a,2,7b-tetrahydrocyclopropa[c][1,2]benzoxaborinine-4-carboxylic acid 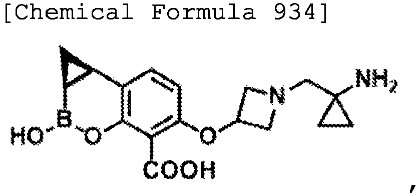
(2R,4S)-9-{1-[(1-aminocyclopropyl)methyl]azetidin-3-yl}oxy-5,5-dihydroxy-6-oxa-5-boranuidatricyclo[5.4.0.0^{2,4}]undeca-1(7),8,10-triene-8-carboxylate 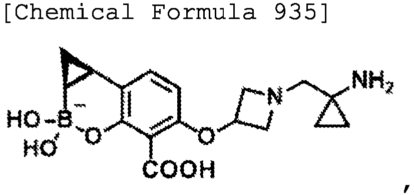
(1aR,7bS)-5-({1-[(1-aminocyclopropyl)methyl]azetidin-3-yl}oxy)-2-hydroxy-1,1a,2,7b-tetrahydrocyclopropa[c][1,2]benzoxaborinine-4-carboxylic acid 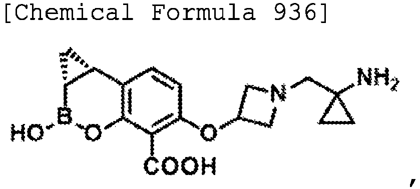
(2S,4R)-9-{1-[(1-aminocyclopropyl)methyl]azetidin-3-yl}oxy-5,5-dihydroxy-6-oxa-5-boranuidatricyclo[5.4.0.0^{2,4}]undeca-1(7),8,10-triene-8-carboxylate 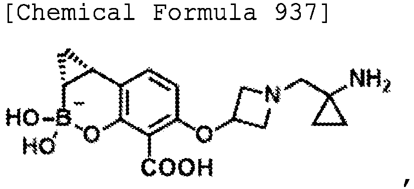
2-hydroxy-5-({1-[(1H-1,2,4-triazol-3-yl)methyl]azetidin-3-yl}oxy)-1,1a,2,7b-tetrahydrocyclopropa[c][1,2]benzoxaborinine-4-carboxylic acid 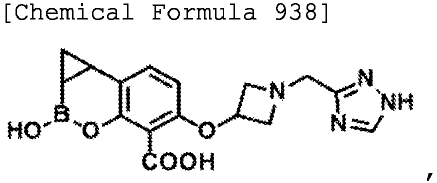
5,5-dihydroxy-9-{1-[(1H-1,2,4-triazol-3-yl)methyl]azetidin-3-yl}oxy-5-boranuidatricyclo[5.4.0.0^{2,4}]undeca-1(11),7,9-triene-8-carboxylic acid 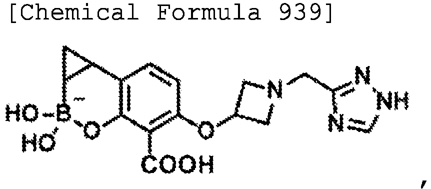
(1aS,7bR)-2-hydroxy-5-({1-[(1H-1,2,4-triazol-3-yl)methyl]azetidin-3-yl}oxy)-1,1a,2,7b-tetrahydrocyclopropa[c][1,2]benzoxaborinine-4-carboxylic acid 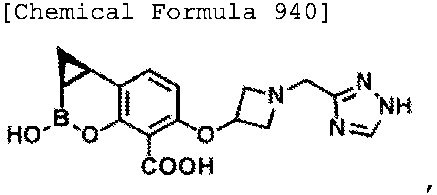
(2R,4S)-5,5-dihydroxy-9-{1-[(1H-1,2,4-triazol-3-yl)methyl]azetidin-3-yl}oxy-5-boranuidatricyclo[5.4.0.0^{2,4}]undeca-1(11),7,9-triene-8-carboxylic acid 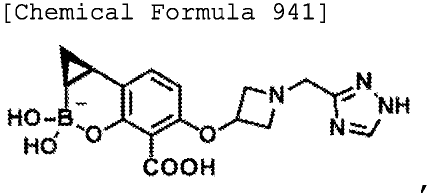
(1aR,7bS)-2-hydroxy-5-({1-[(1H-1,2,4-triazol-3-yl)methyl]azetidin-3-yl}oxy)-1,1a,2,7b-tetrahydrocyclopropa[c][1,2]benzoxaborinine-4-carboxylic acid 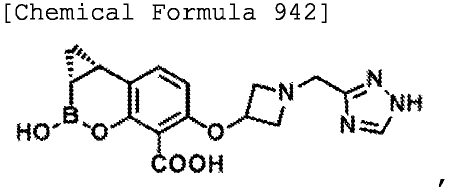
(2S,4R)-5,5-dihydroxy-9-{1-[(1H-1,2,4-triazol-3-yl)methyl]azetidin-3-yl}oxy-5-boranuidatricyclo[5.4.0.0^{2,4}]undeca-1(11),7,9-triene-8-carboxylic acid 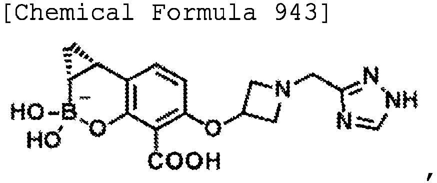
5-{[1-({4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 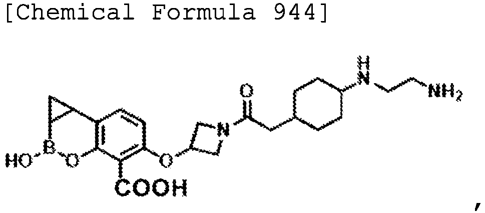
9-[1-({4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 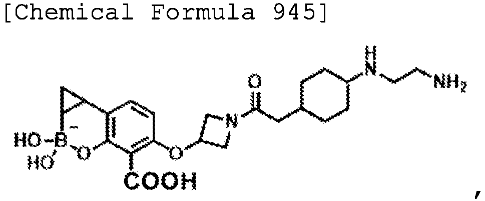
(1aS,7bR)-5-1[1-({(1r,4r)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 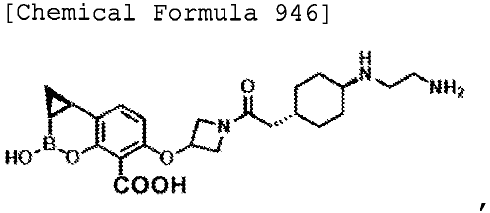
(2R,4S)-9-[1-({(1r,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 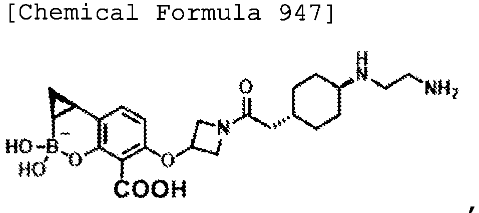
(1aS,7bR)-5-{[1-({(1s,4r)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 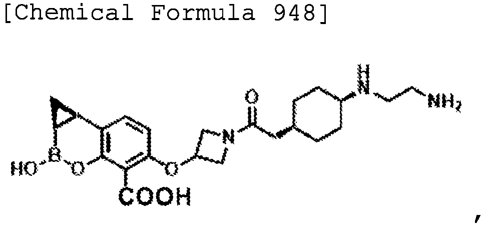
(2R,4S)-9-[1-({(1s,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 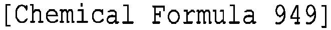 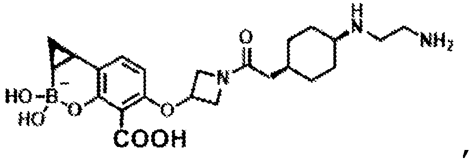
(1aS,7bR)-5-{[1-({(1r,4s)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 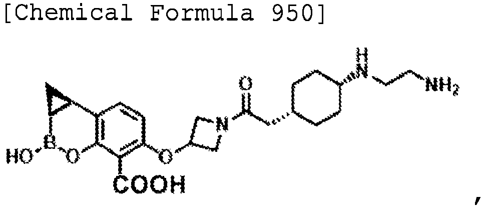
(2R,4S)-9-[1-({(1r,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 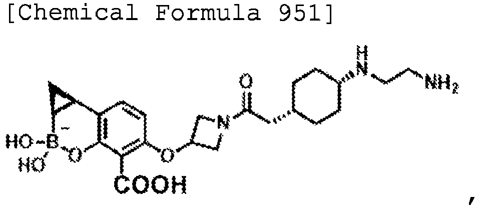
(1aS,7bR)-5-{[1-({(1s,4s)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 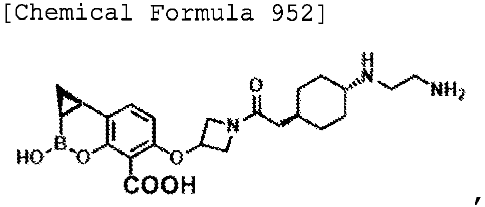
(2R,4S)-9-[1-({(1s,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 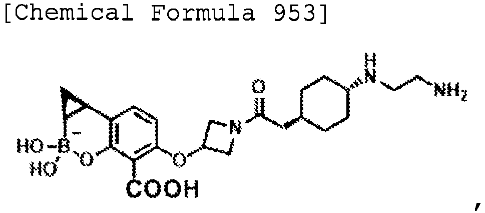
(1aR,7bS)-5-{[1-({(1r,4r)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 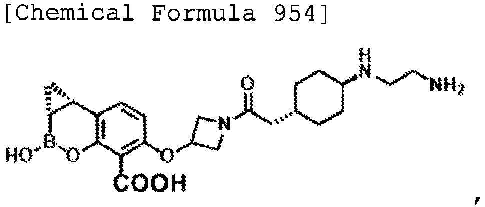
(2S,4R)-9-[1-({(1r,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 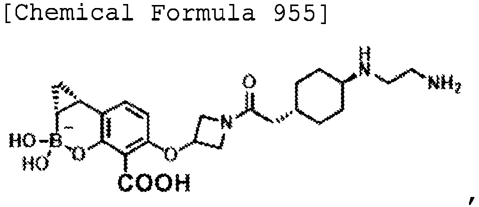
(1aR,7bS)-5-{[1-({(1s,4r)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 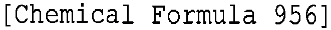 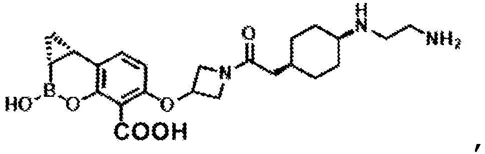
(2S,4R)-9-[1-({(1s,4r)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 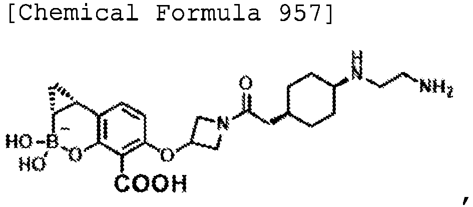
(1aR,7bS)-5-{[1-({(1r,4s)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 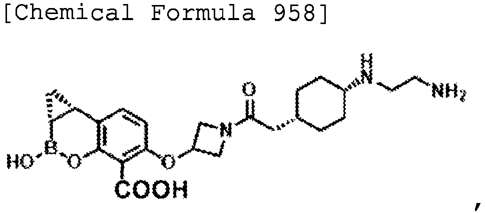
(2S,4R)-9-[1-({(1r,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 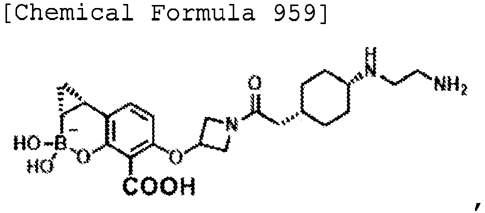
(1aR,7bS)-5-{[1-({(1s,4s)-4-[(2-aminoethyl)amino]cyclohexyl}acetyl)azetidin-3-yl]oxy}-2-hydroxy-1,1a,2,7b-tetrahydrocyclopropa[c][1,2]benzoxaborinine-4-carboxylic acid 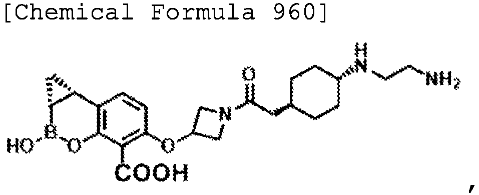
(2S,4R)-9-[1-({(1s,4s)-4-[(2-aminoethyl)amino]cyclohexyl}acetyl)azetidin-3-yl]oxy-5,5-dihydroxy-6-oxa-5-boranuidatricyclo[5.4.0.0^{2,4}]undeca-1(7),8,10-triene-8-carboxylate 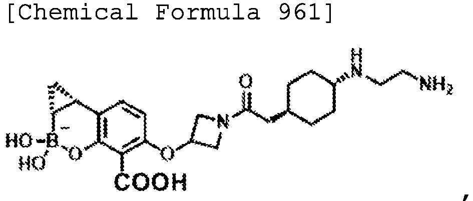
5-[(1-{2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 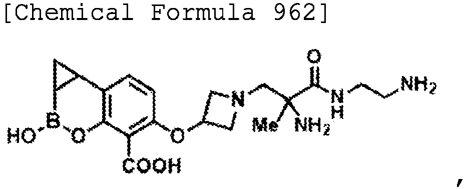
9-(1-{2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 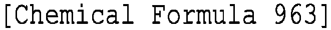 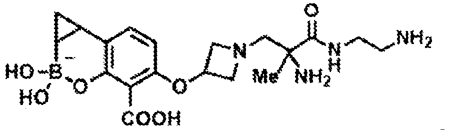
(1aS,7bR)-5-[(1-{(2R)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 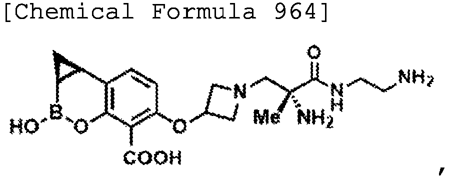
(2R,4S)-9-(1-{(2R)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 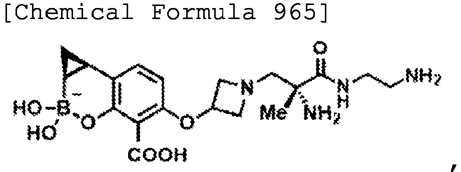
(1aS,7bR)-5-[(1-{(2S)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 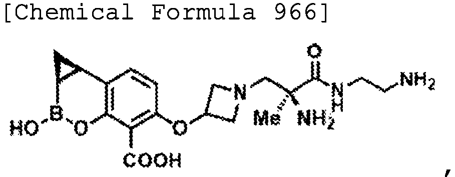
(2R,4S)-9-(1-{(2S)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 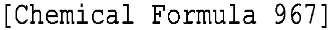 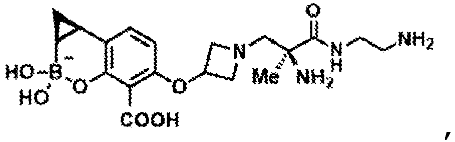
(1aR,7bS)-5-[(1-{(2R)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 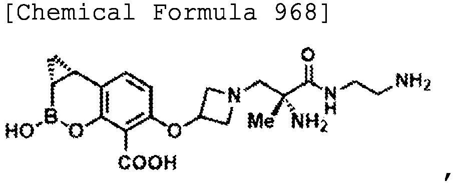
(2S,4R)-9-(1-1(2R)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 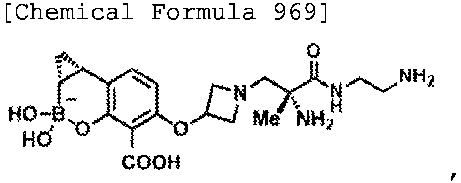
(1aR,7bS)-5-[(1-{(2S)-2-amino-3-[(2-aminoethyl)amino]-2-methyl-3-oxopropyl}azetidin-3-yl)oxy]-2-hydroxy-1,1a,2,7b-tetrahydrocyclopropa[c][1,2]benzoxaborinine-4-carboxylic acid 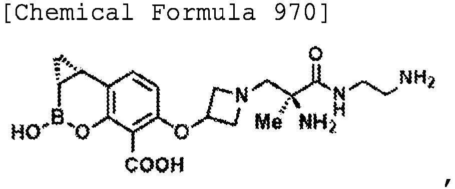
(2S,4R)-9-(1-{(2S)-2-amino-3-[(2-aminoethyl)amino]-2-methyl-3-oxopropyl}azetidin-3-yl)oxy-5,5-dihydroxy-6-oxa-5-boranuidatricyclo[5.4.0.0^{2,4}]undeca-1(7),8,10-triene-8-carboxylate 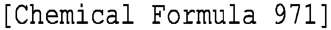 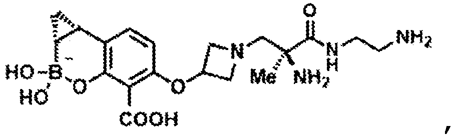
5-[(1-histidylazetidin-3-yl)oxy]-2-hydroxy-1,1a,2,7b-tetrahydrocyclopropa[c][1,2]benzoxaborinine-4-carboxylic acid 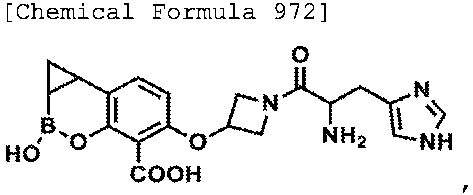
9-(1-histidylazetidin-3-yl)oxy-5,5-dihydroxy-6-oxa-5-boranuidatricyclo[5.4.0.0^{2,4}]undeca-1(7),8,10-triene-8-carboxylic acid 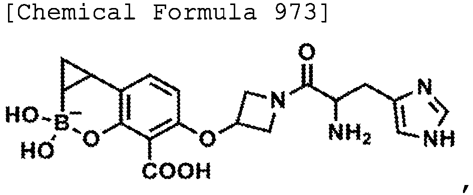
(1aS,7bR)-5-[(1-D-histidylazetidin-3-yl)oxy]-2-hydroxy-1,1a,2,7b-tetrahydrocyclopropa[c][1,2]benzoxaborinine-4-carboxylic acid 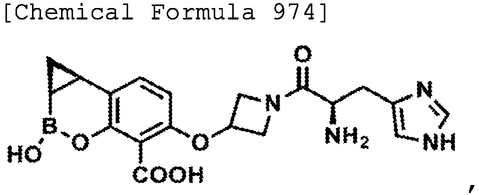
(2R,4S)-9-(1-D-histidylazetidin-3-yl)oxy-5,5-dihydroxy-6-oxa-5-boranuidatricyclo[5.4.0.0^{2,4}]undeca-1(7),8,10-triene-8-carboxylic acid 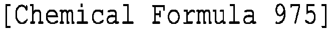 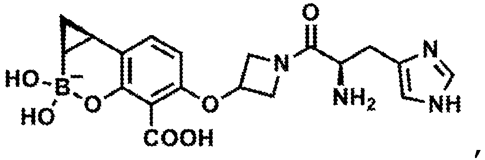
(1aS,7bR)-5-[(1-L-histidylazetidin-3-yl)oxy]-2-hydroxy-1,1a,2,7b-tetrahydrocyclopropa[c][1,2]benzoxaborinine-4-carboxylic acid 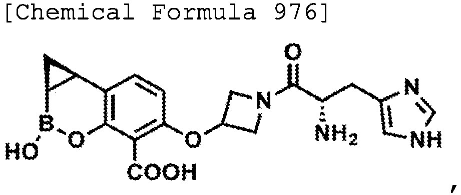
(2R,4S)-9-(1-L-histidylazetidin-3-yl)oxy-5,5-dihydroxy-6-oxa-5-boranuidatricyclo[5.4.0.0^{2,4}]undeca-1(7),8,10-triene-8-carboxylic acid 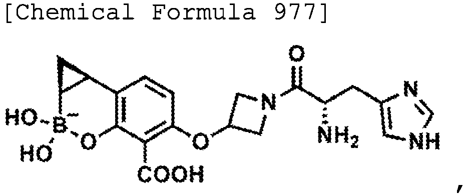
(1aR,7bS)-5-[(1-D-histidylazetidin-3-yl)oxy]-2-hydroxy-1,1a,2,7b-tetrahydrocyclopropa[c][1,2]benzoxaborinine-4-carboxylic acid 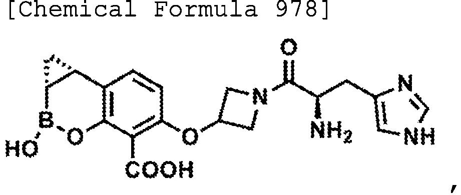
(2S,4R)-9-(1-D-histidylazetidin-3-yl)oxy-5,5-dihydroxy-6-oxa-5-boranuidatricyclo[5.4.0.0^{2,4}]undeca-1(7),8,10-triene-8-carboxylic acid 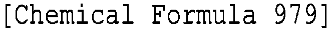 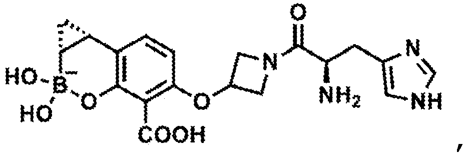
(1aR,7bS)-5-[(1-L-histidylazetidin-3-yl)oxy]-2-hydroxy-1,1a,2,7b-tetrahydrocyclopropa[c][1,2]benzoxaborinine-4-carboxylic acid 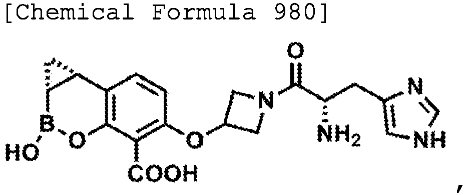
(2S,4R)-9-(1-L-histidylazetidin-3-yl)oxy-5,5-dihydroxy-6-oxa-5-boranuidatricyclo[5.4.0.0^{2,4}]undeca-1(7),8,10-triene-8-carboxylic acid 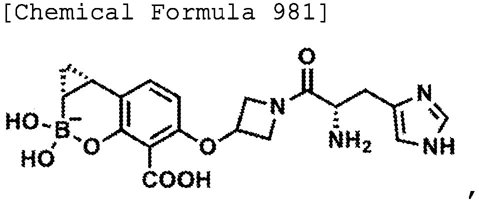
5-{[1-(2-aminoethyl)azetidin-3-yl]oxy}-2-hydroxy-1,1a,2,7b-tetrahydrocyclopropa[c][1,2]benzoxaborinine-4-carboxylic acid 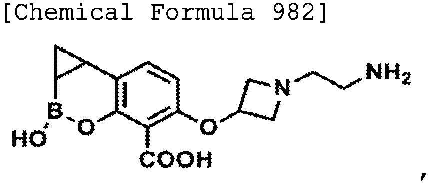
9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid 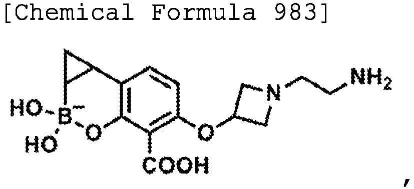
(1aS,7bR)-5-{[1-(2-aminoethyl)azetidin-3-yl]oxy}-2-hydroxy-1,1a,2,7b-tetrahydrocyclopropa[c][1,2]benzoxaborinine-4-carboxylic acid 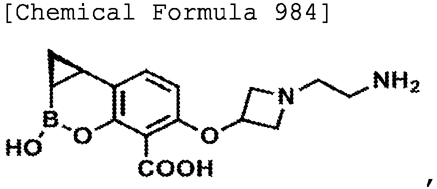
(2R,4S)-9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid 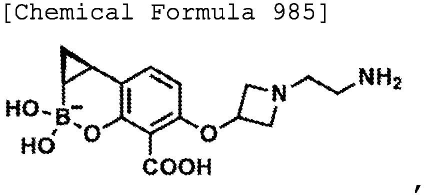
(1aR,7bS)-5-{[1-(2-aminoethyl)azetidin-3-yl]oxy}-2-hydroxy-1,1a,2,7b-tetrahydrocyclopropa[c][1,2]benzoxaborinine-4-carboxylic acid 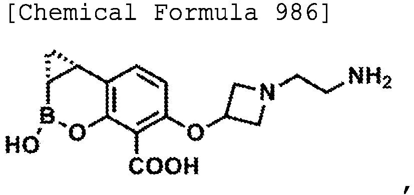
(2S,4R)-9-[1-(2-aminoethyl)azetidin-3-yl]oxy-5,5-dihydroxy-6-oxa-5-boranuidatricyclo[5.4.0.0^{2,4}]undeca-1(7),8,10-triene-8-carboxylic acid 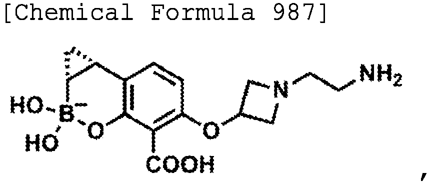
5-[(1-{[5-(aminomethyl)morpholin-2-yl]acetyl}azetidin-3-yl)oxy]-2-hydroxy-1,1a,2,7b-tetrahydrocyclopropa[c][1,2]benzoxaborinine-4-carboxylic acid 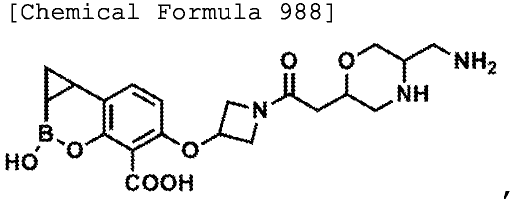
9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid 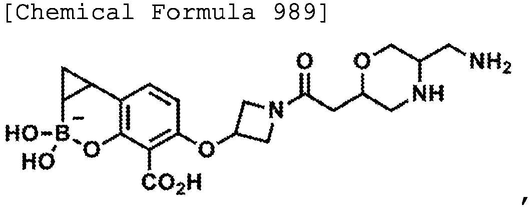
(1aS,7bR)-5-[(1-{[5-(aminomethyl)morpholin-2-yl]acetyl}azetidin-3-yl)oxy]-2-hydroxy-1,1a,2,7b-tetrahydrocyclopropa[c][1,2]benzoxaborinine-4-carboxylic acid 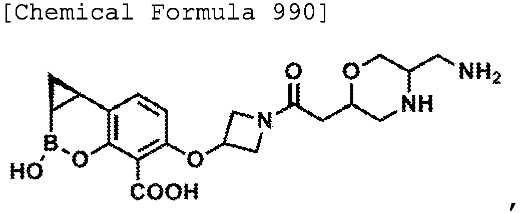
(2R,4S)-9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid 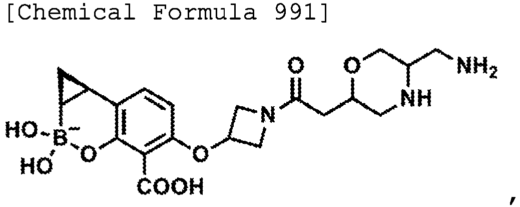
(1aR,7bS)-5-[(1-{[5-(aminomethyl)morpholin-2-yl]acetyl}azetidin-3-yl)oxy]-2-hydroxy-1,1a,2,7b-tetrahydrocyclopropa[c][1,2]benzoxaborinine-4-carboxylic acid 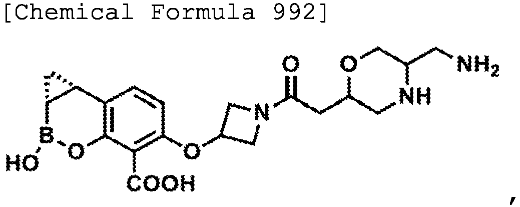
(2S,4R)-9-(1-{[5-(aminomethyl)morpholin-2-yl]acetyl}azetidin-3-yl)oxy-5,5-dihydroxy-6-oxa-5-boranuidatricyclo[5.4.0.0^{2,4}]undeca-1(7),8,10-triene-8-carboxylic acid 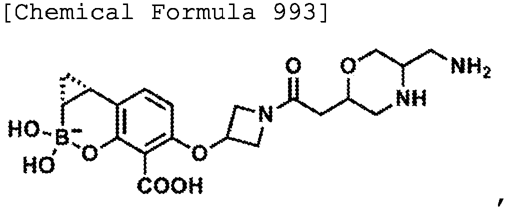
5-({1-[2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c][1,2]benzoxaborinine-4-carboxylic acid 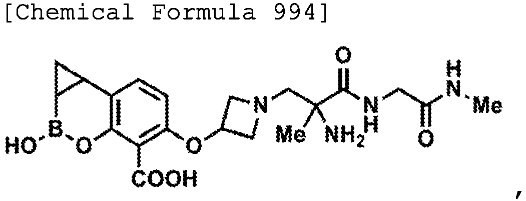
9-({1-[2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 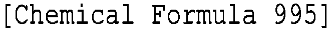 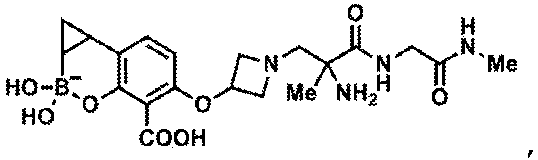
(1aS,7bR)-5-({1-[(2R)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c][1,2]benzoxaborinine-4-carboxylic acid 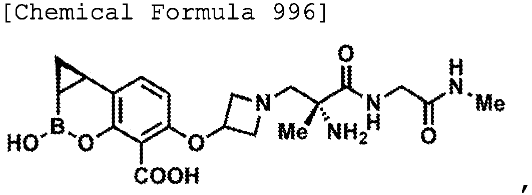
(2R,4S)-9-({1-[(2R)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 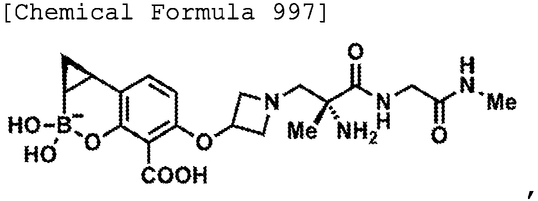
(1aS,7bR)-5-({1-[(2S)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c][1,2]benzoxaborinine-4-carboxylic acid 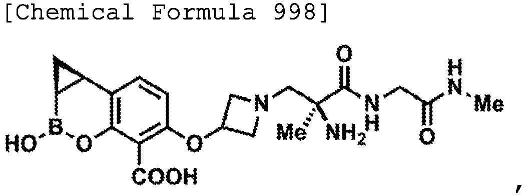
(2R,4S)-9-({1-[(2S)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 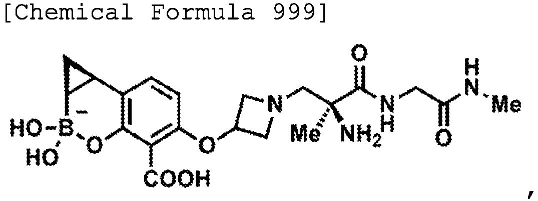
(1aR,7bS)-5-({1-[(2R)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c][1,2]benzoxaborinine-4-carboxylic acid 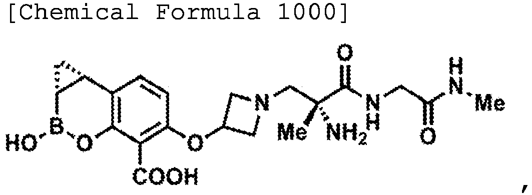
(2S,4R)-9-({1-[(2R)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 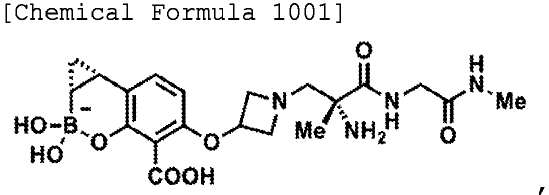
(1aR,7bS)-5-({1-[(2S)-2-amino-2-methyl-3-{[2-(methylamino)-2-oxoethyl]amino}-3-oxopropyl]azetidin-3-yl}oxy)-2-hydroxy-1,1a,2,7b-tetrahydrocyclopropa[c][1,2]benzoxaborinine-4-carboxylic acid 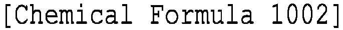 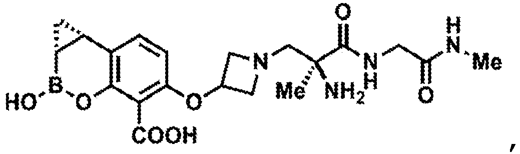
(2S,4R)-9-({1-[(2S)-2-amino-2-methyl-3-{[2-(methylamino)-2-oxoethyl]amino}-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 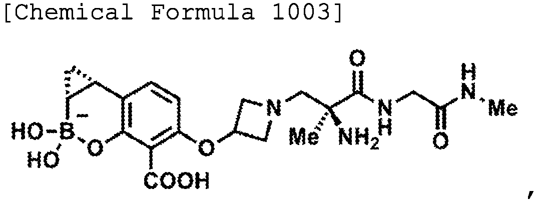
5-({1-[2-amino-3-{[2-(dimethylamino)-2-oxoethyl]amino}-2-methyl-3-oxopropyl]azetidin-3-yl}oxy)-2-hydroxy-1,1a,2,7b-tetrahydrocyclopropa[c][1,2]benzoxaborinine-4-carboxylic acid 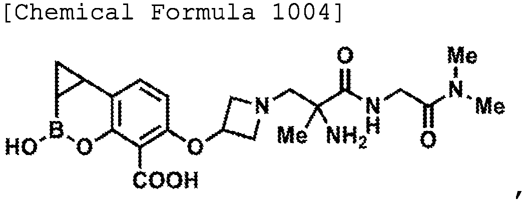
9-({1-[2-amino-3-{[2-(dimethylamino)-2-oxoethyl]amino}-2-methyl-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 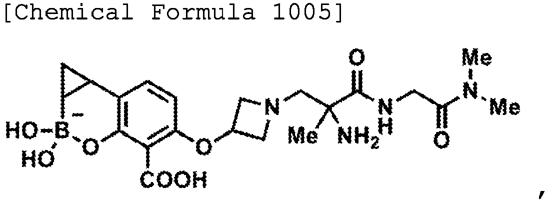
(1aS,7bR)-5-({1-[(2R)-2-amino-3-{[2-(dimethylamino)-2-oxoethyl]amino}-2-methyl-3-oxopropyl]azetidin-3-yl}oxy)-2-hydroxy-1,1a,2,7b-tetrahydrocyclopropa[c][1,2]benzoxaborinine-4-carboxylic acid 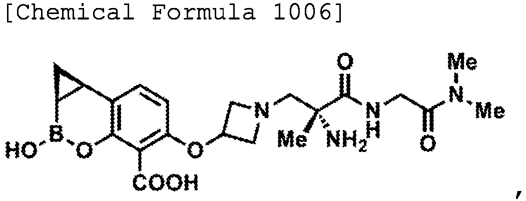
(2R,4S)-9-({1-[(2R)-2-amino-3-{[2-(dimethylamino)-2-oxoethyl]amino}-2-methyl-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 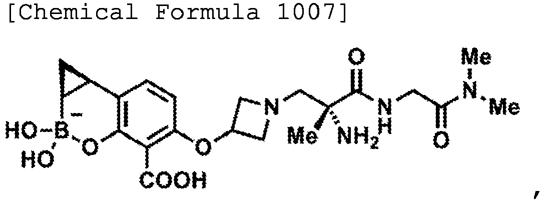
(1aS,7bR)-5-({1-[(2S)-2-amino-3-{[2-(dimethylamino)-2-oxoethyl]amino}-2-methyl-3-oxopropyl]azetidin-3-yl}oxy)-2-hydroxy-1,1a,2,7b-tetrahydrocyclopropa[c][1,2]benzoxaborinine-4-carboxylic acid 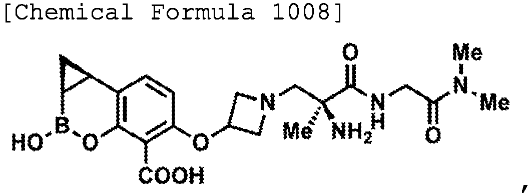
(2R,4S)-9-({1-[(2S)-2-amino-3-{[2-(dimethylamino)-2-oxoethyl]amino}-2-methyl-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 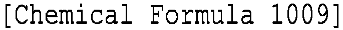 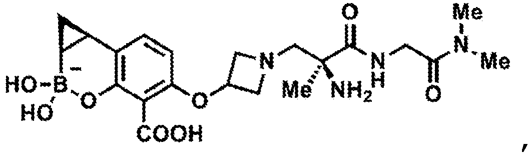
(1aR,7bS)-5-({1-[(2R)-2-amino-3-{[2-(dimethylamino)-2-oxoethyl]amino}-2-methyl-3-oxopropyl]azetidin-3-yl}oxy)-2-hydroxy-1,1a,2,7b-tetrahydrocyclopropa[c][1,2]benzoxaborinine-4-carboxylic acid 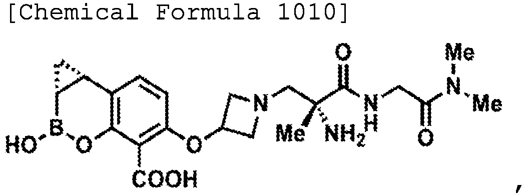
(2S,4R)-9-({1-[(2R)-2-amino-3-{[2-(dimethylamino)-2-oxoethyl]amino}-2-methyl-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 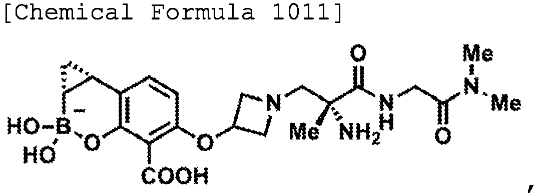
(1aR,7bS)-5-({1-[(2S)-2-amino-3-{[2-(dimethylamino)-2-oxoethyl]amino}-2-methyl-3-oxopropyl]azetidin-3-yl}oxy)-2-hydroxy-1,1a,2,7b-tetrahydrocyclopropa[c][1,2]benzoxaborinine-4-carboxylic acid 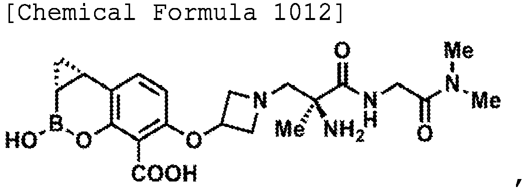
(2S,4R)-9-({1-[(2S)-2-amino-3-{[2-(dimethylamino)-2-oxoethyl]amino}-2-methyl-3-oxopropyl]azetidin-3-yl}oxy)-5,5-dihydroxy-6-oxa-5-boranuidatricyclo[5.4.0.0^{2,4}]undeca-1(7),8,10-triene-8-carboxylic acid 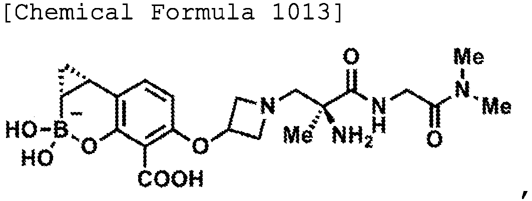
2-hydroxy-5-[(1-{[4-methylmorpholin-2-yl]acetyl}azetidin-3-yl)oxy]-1,1a,2,7b-tetrahydrocyclopropa[c][1,2]benzoxaborinine-4-carboxylic acid 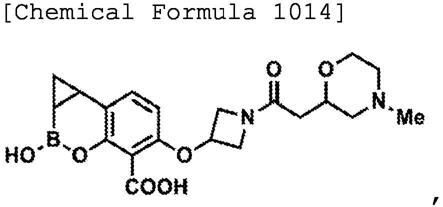
5,5-dihydroxy-9-[(1-{[(4-methylmorpholin-2-yl]acetyl}azetidin-3-yl)oxy]-5-boranuidatricyclo[5.4.0.0^{2,4}]undeca-1(11),7,9-triene-8-carboxylic acid 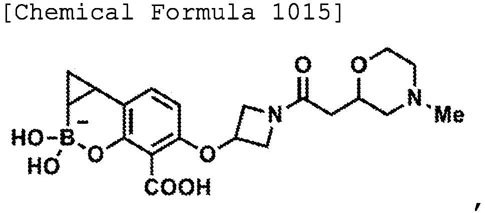
(1aS,7bR)-2-hydroxy-5-[(1-{[(2R)-4-methylmorpholin-2-yl]acetyl}azetidin-3-yl)oxy]-1,1a,2,7b-tetrahydrocyclopropa[c][1,2]benzoxaborinine-4-carboxylic acid 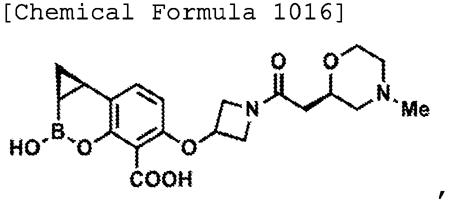
(2R,4S)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid 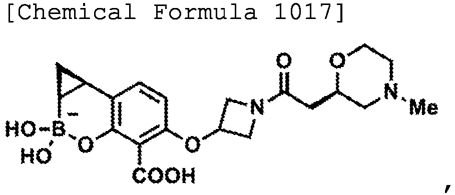
(1aS,7bR)-2-hydroxy-5-[(1-{[(2S)-4-methylmorpholin-2-yl]acetyl}azetidin-3-yl)oxy]-1,1a,2,7b-tetrahydrocyclopropa[c][1,2]benzoxaborinine-4-carboxylic acid 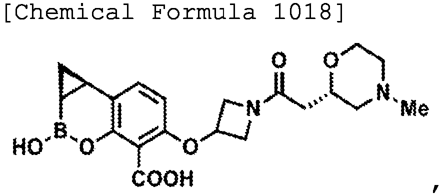
(2R,4S)-5,5-dihydroxy-9-[(1-{[(2S)-4-methylmorpholin-2-yl]acetyl}azetidin-3-yl)oxy]-5-boranuidatricyclo[5.4.0.0^{2,4}]undeca-1(11),7,9-triene-8-carboxylic acid 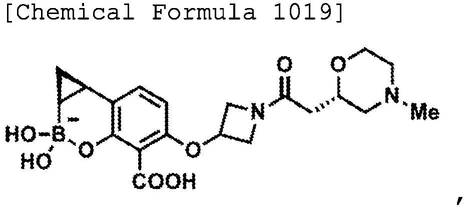
(1aR,7bS)-2-hydroxy-5-[(1-{[(2R)-4-methylmorpholin-2-yl]acetyl}azetidin-3-yl)oxy]-1,1a,2,7b-tetrahydrocyclopropa[c][1,2]benzoxaborinine-4-carboxylic acid 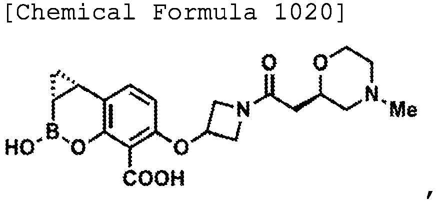
(2S,4R)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid 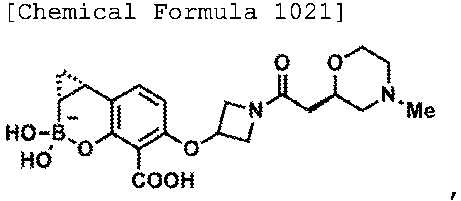
(1aR,7bS)-2-hydroxy-5-[(1-{[(2S)-4-methylmorpholin-2-yl]acetyl}azetidin-3-yl)oxy]-1,1a,2,7b-tetrahydrocyclopropa[c][1,2]benzoxaborinine-4-carboxylic acid 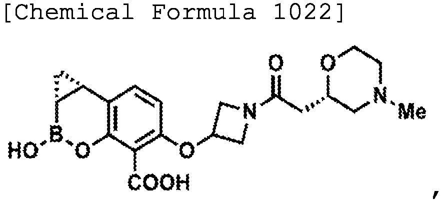
(2S,4R)-5,5-dihydroxy-9-[(1-{[(2R)-4-methylmorpholin-2-yl]acetyl}azetidin-3-yl)oxy]-5-boranuidatricyclo[5.4.0.0^{2,4}]undeca-1(11),7,9-triene-8-carboxylic acid 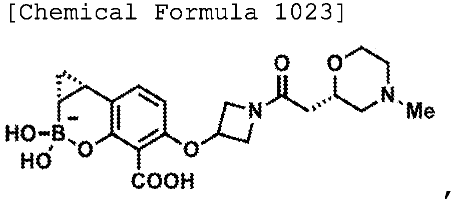
5-[(1-{[4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 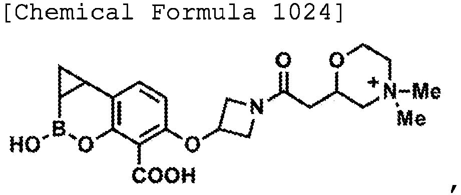
9-[(1-{[4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 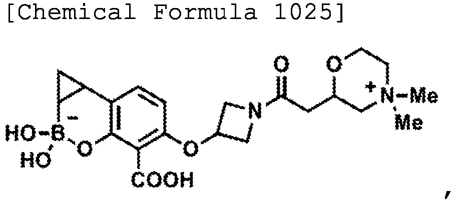
(1aS,7bR)-5-[(1-{[(2R)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 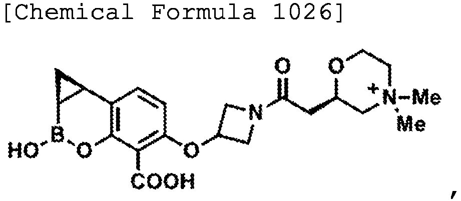
(2R,4S)-9-[(1-{[(2R)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 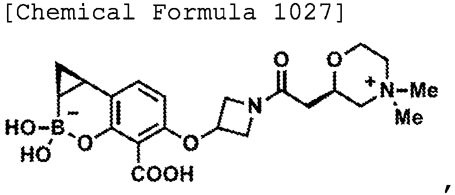
(1aS,7bR)-5-[(1-{[(2S)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 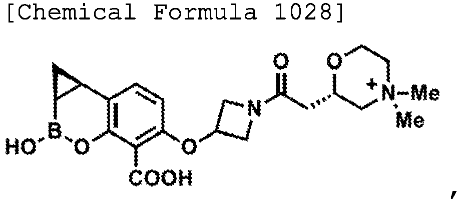
(2R,4S)-9-[(1-{[(2S)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 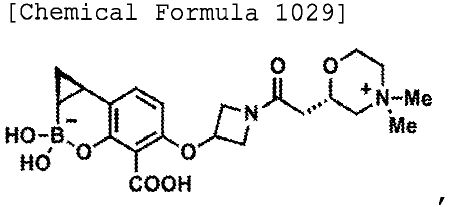
(1aR,7bS)-5-[(1-{[(2R)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 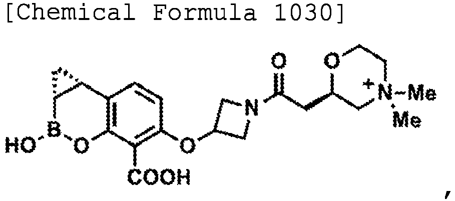
(2S,4R)-9-[(1-{[(2R)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 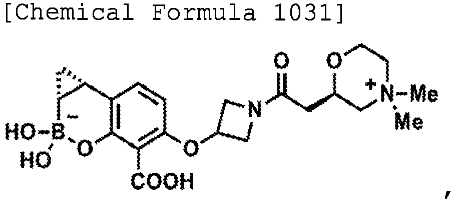
(1aR,7bS)-5-[(1-{[(2S)-4,4-dimethylmorpholin-4-ium-2-yl]acetyl}azetidin-3-yl)oxy]-2-hydroxy-1,1a,2,7b-tetrahydrocyclopropa[c][1,2]benzoxaborinine-4-carboxylate 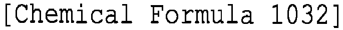 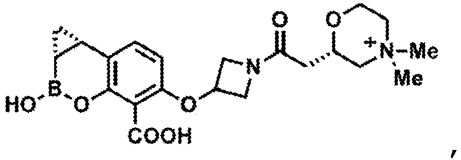
(2S,4R)-9-[(1-{[(2S)-4,4-dimethylmorpholin-4-ium-2-yl]acetyl}azetidin-3-yl)oxy]-5,5-dihydroxy-6-oxa-5-boranuidatricyclo[5.4.0.0^{2,4}]undeca-1(7),8,10-triene-8-carboxylic acid 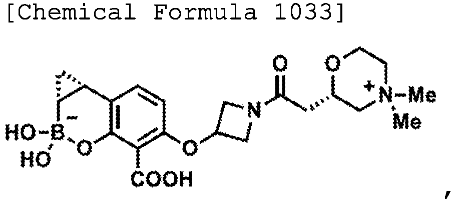
(1aR,7bS)-2-hydroxy-5-({1-[(3-hydroxy-2-methyl-4-oxopyridin-1(4H)-yl)acetyl]azetidin-3-yl}oxy)-1,1a,2,7b-tetrahydrocyclopropa[c][1,2]benzoxaborinine-4-carboxylic acid 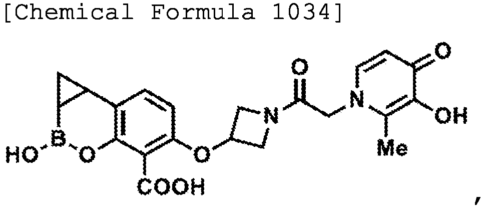
5,5-dihydroxy-9-({1-[(3-hydroxy-2-methyl-4-oxopyridin-1(4H)-yl)acetyl]azetidin-3-yl}oxy)-5-boranuidatricyclo[5.4.0.0^{2,4}]undeca-1(11),7,9-triene-8-carboxylic acid 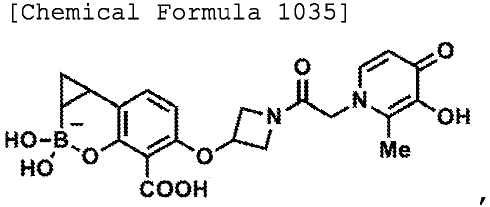
(1aR,7bS)-2-hydroxy-5-({1-[(3-hydroxy-2-methyl-4-oxopyridin-1(4H)-yl)acetyl]azetidin-3-yl}oxy)-1,1a,2,7b-tetrahydrocyclopropa[c][1,2]benzoxaborinine-4-carboxylic acid 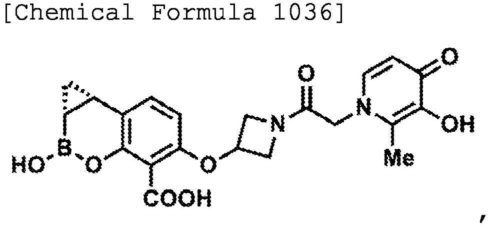
(2S,4R)-5,5-dihydroxy-9-({1-[(3-hydroxy-2-methyl-4-oxopyridin-1(4H)-yl)acetyl]azetidin-3-yl}oxy)-5-boranuidatricyclo[5.4.0.0^{2,4}]undeca-1(11),7,9-triene-8-carboxylic acid 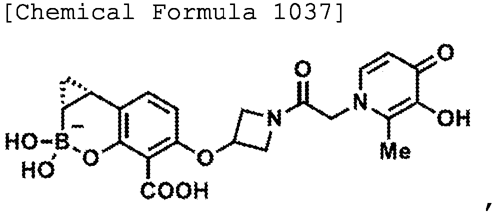
(1aS,7bR)-2-hydroxy-5-({1-[(3-hydroxy-2-methyl-4-oxopyridin-1(4H)-yl)acetyl]azetidin-3-yl}oxy)-1,1a,2,7b-tetrahydrocyclopropa[c][1,2]benzoxaborinine-4-carboxylic acid 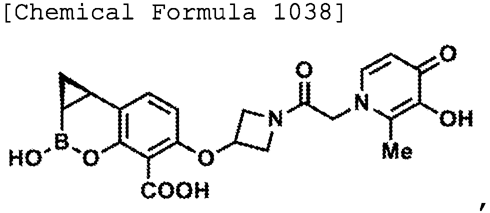 and
(2R,4S)-5,5-dihydroxy-9-({1-[(3-hydroxy-2-methyl-4-oxopyridin-1(4H)-yl)acetyl]azetidin-3-yl}oxy)-5-boranuidatricyclo[5.4.0.0^{2,4}]undeca-1(11),7,9-triene-8-carboxylic acid

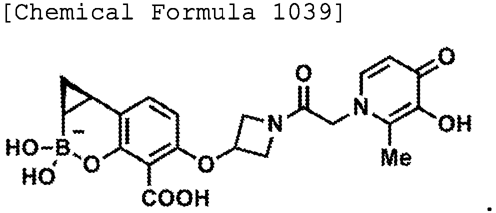

103. A compound represented by formula (11): 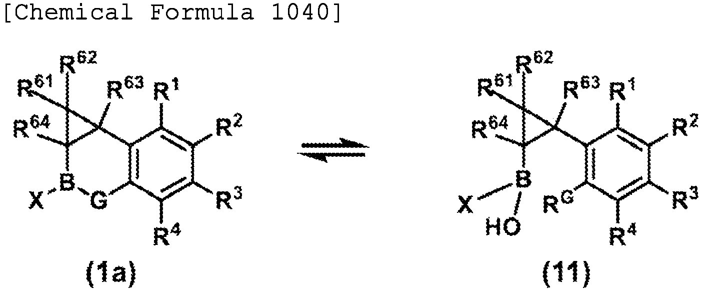 or a pharmaceutically acceptable salt thereof,
wherein R^{G} is a hydroxyl group, a thiol group, or -NHR^{a1}, R^{a1}, X, R¹, R², R³, R⁴, R⁶¹, R⁶², R⁶³, and R⁶⁴ are defined the same as the definition according to any of claims 1 to 16, and formula (1a) is defined the same as claim 1 or 2.

104. The compound or the pharmaceutically acceptable salt thereof according to claim 103, wherein the compound of formula (11) is represented by formula (12): 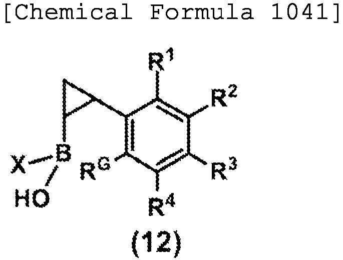 wherein X, R¹, R², R³, and R⁴ are defined the same as the definition according to claim 17 or 18.

105. The compound or the pharmaceutically acceptable salt thereof according to claim 103 or 104, wherein the compound of formula (11) or formula (12) is represented by formula (13): 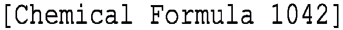 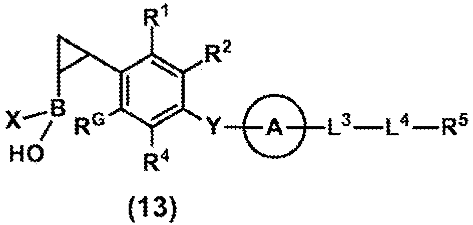 wherein X, Y, ring A, L³, L⁴, R¹, R², R⁴, and R⁵ are defined the same as the definition according to any of claims 19 to 23 and 29 to 32.

106. The compound or the pharmaceutically acceptable salt thereof according to claim 105, wherein X and R^{G} are hydroxyl groups, R⁴ is a carboxyl group, and ring A is an optionally substituted 4- to 6-membered nitrogen-containing non-aryl heterocycle.

107. The compound or the pharmaceutically acceptable salt thereof according to any one of claims 103 to 106, wherein the compound of formula (11), formula (12), or formula (13) is represented by formula (14): 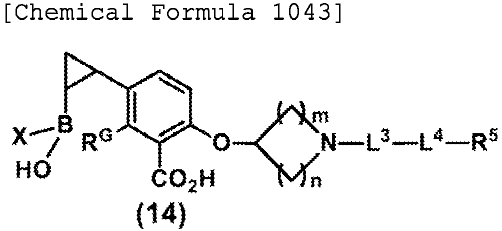 wherein X, L³, L⁴, m, n, and R⁵ are defined the same as the definition according to any one of claims 33 to 64.

108. The compound or the pharmaceutically acceptable salt thereof according to claim 107, wherein the compound of formula (14) is one of the enantiomers represented by formula (15): 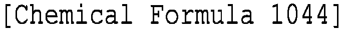 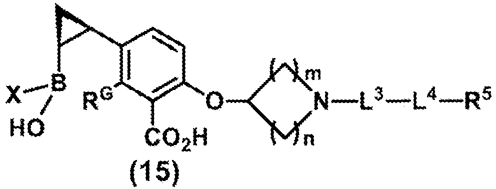 and
formula (16): 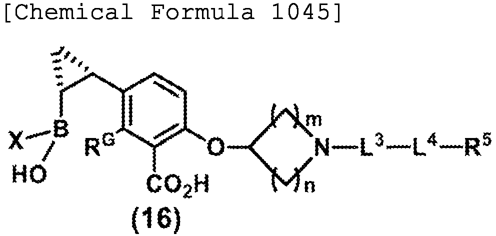 wherein X, L³, L⁴, m, n, and R⁵ are defined the same as the definition according to any one of claims 33 to 64.

109. The compound or the pharmaceutically acceptable salt thereof according to any one of claims 103 to 108, wherein R^{G} is a hydroxyl group or a thiol group.

110. The compound or the pharmaceutically acceptable salt thereof according to any one of claims 103 to 109, wherein R^{G} is a hydroxyl group.

111. The compound or the pharmaceutically acceptable salt thereof according to any one of claims 103 to 110, wherein X is a hydroxyl group or a C₁₋₆ alkoxy group.

112. The compound or the pharmaceutically acceptable salt thereof according to any one of claims 103 to 111, wherein X is a hydroxyl group.

113. The compound or the pharmaceutically acceptable salt thereof according to any one of claims 107 to 112, wherein m is 1 or 2, n is 1 or 2, and m + n is 2 or 3.

114. The compound or the pharmaceutically acceptable salt thereof according to any one of claims 107 to 113, wherein m is 1, and n is 1.

115. The compound or the pharmaceutically acceptable salt thereof according to any one of claims 103 to 114, wherein L³ is defined the same as the definition according to claim 25 or 26.

116. The compound or the pharmaceutically acceptable salt thereof according to any one of claims 103 to 115, wherein L⁴ and R⁵ are defined the same as the definition according to any one of claims 27 and 66 to 100.

117. The compound or the pharmaceutically acceptable salt thereof according to claim 104, selected from the group consisting of the compounds represented by the following names or structural formulas:
6-({1-[amino(1H-imidazol-4-yl)acetyl]azetidin-3-yl}oxy)-3-(2-boronocyclopropyl)-2-hydroxybenzoic acid 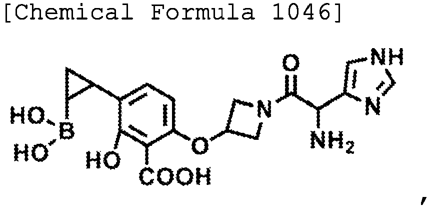
6-({1-[(2R)-2-amino-2-(1H-imidazol-4-yl)acetyl]azetidin-3-yl}oxy)-3-[(1R,2S)-2-boronocyclopropyl]-2-hydroxybenzoic acid 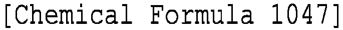 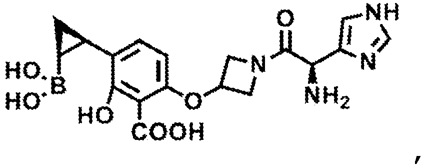
6-({1-[(2S)-2-amino-2-(1H-imidazol-4-yl)acetyl]azetidin-3-yl}oxy)-3-[(1R,2S)-2-boronocyclopropyl]-2-hydroxybenzoic acid 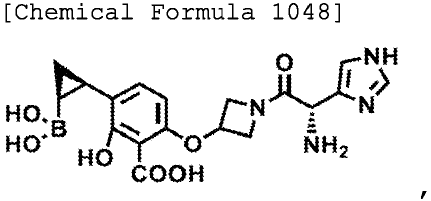
6-({1-[(2R)-2-amino-2-(1H-imidazol-4-yl)acetyl]azetidin-3-yl}oxy)-3-[(1S,2R)-2-boronocyclopropyl]-2-hydroxybenzoic acid 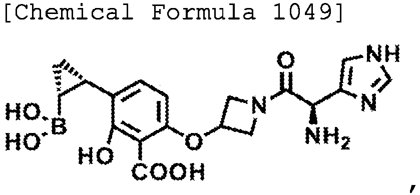
6-({1-[(2S)-2-amino-2-(1H-imidazol-4-yl)acetyl]azetidin-3-yl}oxy)-3-[(1S,2R)-2-boronocyclopropyl]-2-hydroxybenzoic acid 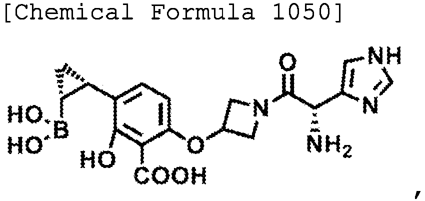
6-({1-(2-amino-2-carboxypropyl)azetidin-3-yl}oxy)-3-(2-boronocyclopropyl)-2-hydroxybenzoic acid 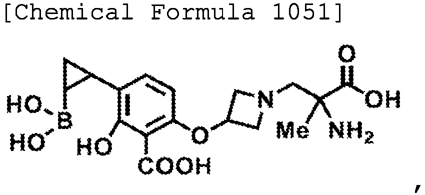
6-({1-[(2R)-2-amino-2-carboxypropyl]azetidin-3-yl}oxy)-3-[(1R,2S)-2-boronocyclopropyl]-2-hydroxybenzoic acid 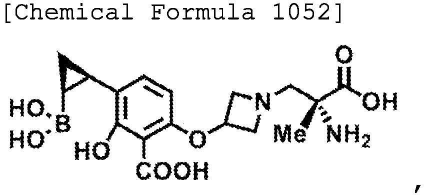
6-({1-[(2R)-2-amino-2-carboxypropyl]azetidin-3-yl}oxy)-3-[(IS, 2R) - 2-boronocyclopropyl]-2-hydroxybenzoic acid 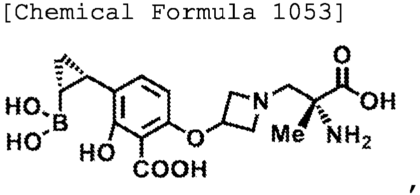
6-({1-[2-amino-2-(1H-imidazol-4-yl)propanoyl]azetidin-3-yl}oxy)-3-(2-boronocyclopropyl)-2-hydroxybenzoic acid 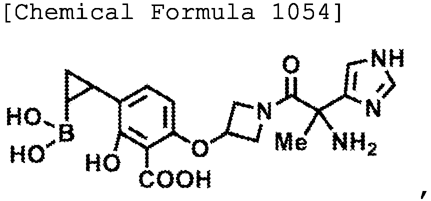
6-({1-[(2R)-2-amino-2-(1H-imidazol-4-yl)propanoyl]azetidin-3-yl}oxy)-3-[(1R,2S)-2-boronocyclopropyl]-2-hydroxybenzoic acid 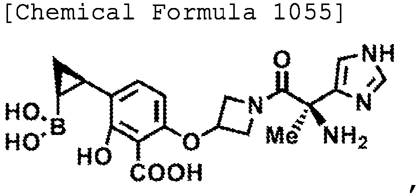
6-({1-[(2S)-2-amino-2-(1H-imidazol-4-yl)propanoyl]azetidin-3-yl}oxy)-3-[(1R,2S)-2-boronocyclopropyl]-2-hydroxybenzoic acid 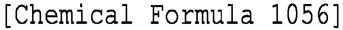 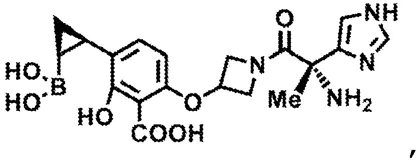
6-({1-[(2R)-2-amino-2-(1H-imidazol-4-yl)propanoyl]azetidin-3-yl}oxy)-3-[(1S,2R)-2-boronocyclopropyl]-2-hydroxybenzoic acid 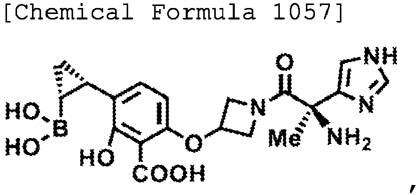 and
6-({1-[(2S)-2-amino-2-(1H-imidazol-4-yl)propanoyl]azetidin-3-yl}oxy)-3-[(1S,2R)-2-boronocyclopropyl]-2-hydroxybenzoic acid

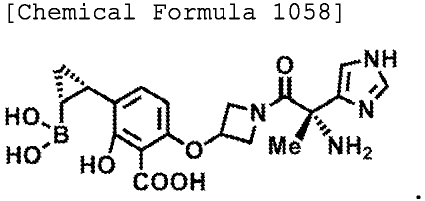

118. The compound or the pharmaceutically acceptable salt thereof according to claim 104, selected from the group consisting of the compounds represented by the following names or structural formulas:
2-boronocyclopropyl]-2-hydroxy-6-({1-[(1H-imidazol-4-yl)acetyl]azetidin-3-yl}oxy)benzoic acid 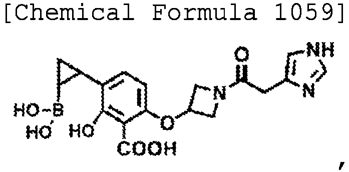
3-[(1R,2S)-2-boronocyclopropyl]-2-hydroxy-6-({1-[(1H-imidazol-4-yl)acetyl]azetidin-3-yl}oxy)benzoic acid 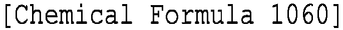 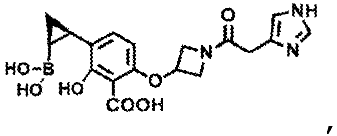
3-[(1S,2R)-2-boronocyclopropyl]-2-hydroxy-6-({1-[(1H-imidazol-4-yl)acetyl]azetidin-3-yl}oxy)benzoic acid 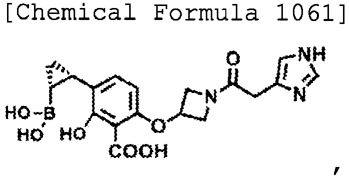
3-[2-boronocyclopropyl]-2-hydroxy-6-[(1-serylazetidin-3-yl)oxy]benzoic acid 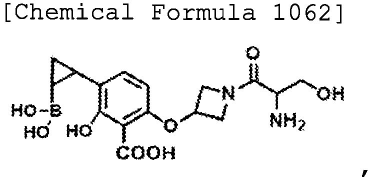
3-[(1R,2S)-2-boronocyclopropyl]-2-hydroxy-6-[(1-D-serylazetidin-3-yl)oxy]benzoic acid 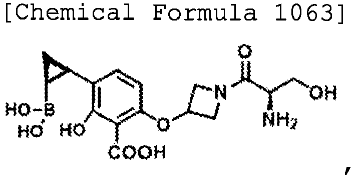
3-[(1R,2S)-2-boronocyclopropyl]-2-hydroxy-6-[(1-L-serylazetidin-3-yl)oxy]benzoic acid 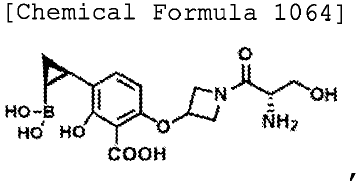
3-[(1S,2R)-2-boronocyclopropyl]-2-hydroxy-6-[(1-D-serylazetidin-3-yl)oxy]benzoic acid 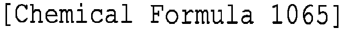 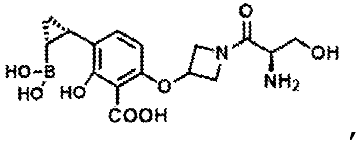
3-[(1S,2R)-2-boronocyclopropyl]-2-hydroxy-6-[(1-L-serylazetidin-3-yl)oxy]benzoic acid 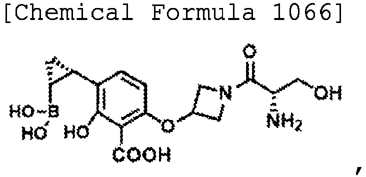
3-[2-boronocyclopropyl]-2-hydroxy-6-({1-[4-hydroxy-L-prolyl]azetidin-3-yl}oxy)benzoic acid 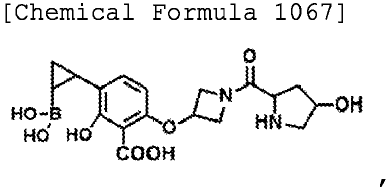
3-[(1R,2S)-2-boronocyclopropyl]-2-hydroxy-6-({1-[(4R)-4-hydroxy-L-prolyl]azetidin-3-yl}oxy)benzoic acid 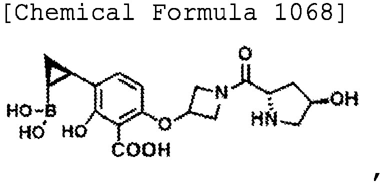
3-[(1R,2S)-2-boronocyclopropyl]-2-hydroxy-6-({1-[(4S)-4-hydroxy-L-prolyl]azetidin-3-yl}oxy)benzoic acid 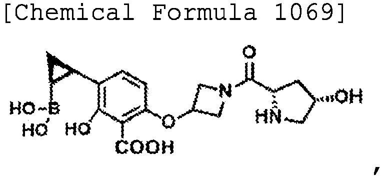
3-[(1R,2S)-2-boronocyclopropyl]-2-hydroxy-6-({1-[(4R)-4-hydroxy-D-prolyl]azetidin-3-yl}oxy)benzoic acid 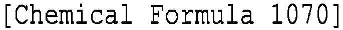 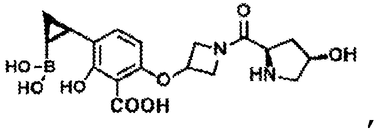
3-[(1R,2S)-2-boronocyclopropyl]-2-hydroxy-6-({1-[(4S)-4-hydroxy-D-prolyl]azetidin-3-yl}oxy)benzoic acid 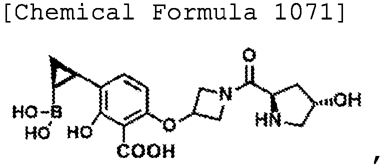
3-[(1S,2R)-2-boronocyclopropyl]-2-hydroxy-6-({1-[(4R)-4-hydroxy-L-prolyl]azetidin-3-yl}oxy)benzoic acid 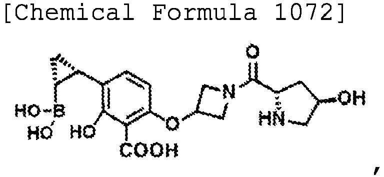
3-[(1S,2R)-2-boronocyclopropyl]-2-hydroxy-6-({1-[(4S)-4-hydroxy-L-prolyl]azetidin-3-yl}oxy)benzoic acid 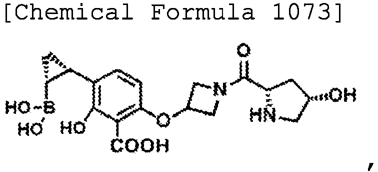
3-[(1S,2R)-2-boronocyclopropyl]-2-hydroxy-6-({1-[(4R)-4-hydroxy-D-prolyl]azetidin-3-yl}oxy)benzoic acid 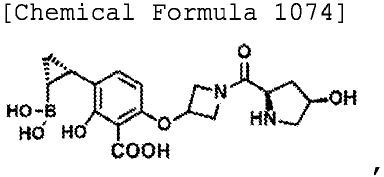
3-[(1S,2R)-2-boronocyclopropyl]-2-hydroxy-6-({1-[(4S)-4-hydroxy-D-prolyl]azetidin-3-yl}oxy)benzoic acid 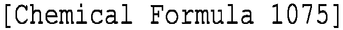 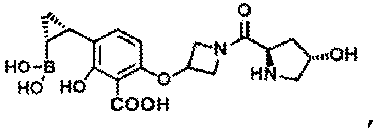
3-[2-boronocyclopropyl]-2-hydroxy-6-{[1-(2-methyl-seryl)azetidin-3-yl]oxy}benzoic acid 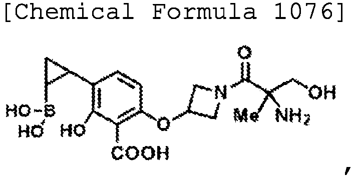
3-[(1R,2S)-2-boronocyclopropyl]-2-hydroxy-6-{[1-(2-methyl-D-seryl)azetidin-3-yl]oxy}benzoic acid 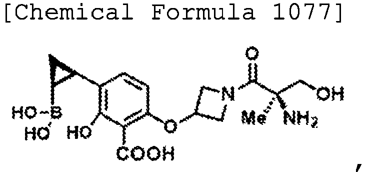
3-[(1R,2S)-2-boronocyclopropyl]-2-hydroxy-6-{[1-(2-methyl-L-seryl)azetidin-3-yl]oxy}benzoic acid 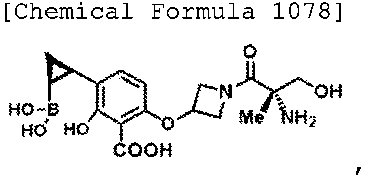
3-[(1S,2R)-2-boronocyclopropyl]-2-hydroxy-6-{[1-(2-methyl-D-seryl)azetidin-3-yl]oxy}benzoic acid 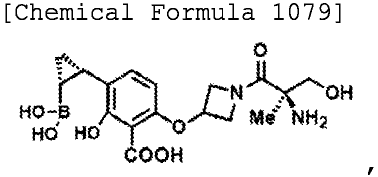
3-[(1S,2R)-2-boronocyclopropyl]-2-hydroxy-6-{[1-(2-methyl-L-seryl)azetidin-3-yl]oxy}benzoic acid 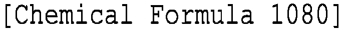 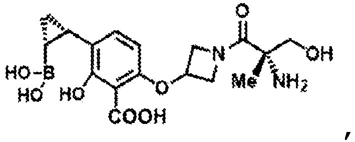
3-[(2-boronocyclopropyl]-2-hydroxy-6-[(1-{[morpholin-2-yl]acetyl}azetidin-3-yl)oxy]benzoic acid 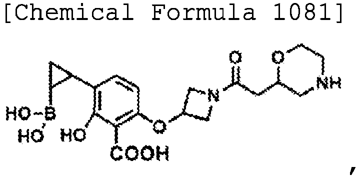
3-[(1R,2S)-2-boronocyclopropyl]-2-hydroxy-6-[(1-{[(2R)-morpholin-2-yl]acetyl}azetidin-3-yl)oxy]benzoic acid 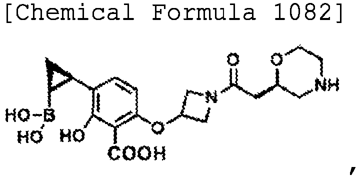
3-[(1R,2S)-2-boronocyclopropyl]-2-hydroxy-6-[(1-{[(2S)-morpholin-2-yl]acetyl}azetidin-3-yl)oxy]benzoic acid 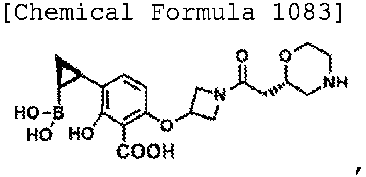
3-[(1S,2R)-2-boronocyclopropyl]-2-hydroxy-6-[(1-{[(2R)-morpholin-2-yl]acetyl}azetidin-3-yl)oxy]benzoic acid 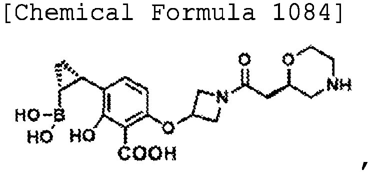
3-[(1S,2R)-2-boronocyclopropyl]-2-hydroxy-6-[(1-{[(2S)-morpholin-2-yl]acetyl}azetidin-3-yl)oxy]benzoic acid 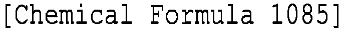 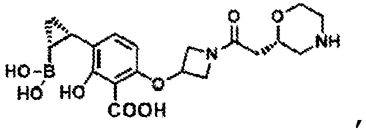
6-({1-[(2S)-2-amino-2-carboxypropyl]azetidin-3-yl}oxy)-3-[(1R,2S)-2-boronocyclopropyl]-2-hydroxybenzoic acid 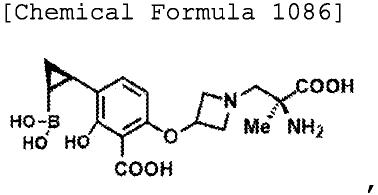
6-({1-[(2S)-2-amino-2-carboxypropyl]azetidin-3-yl}oxy)-3-[(1S,2R)-2-boronocyclopropyl]-2-hydroxybenzoic acid 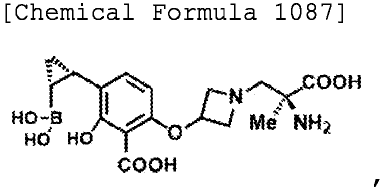
6-[(1-{2-amino-3-[(2-hydroxyethyl)amino]-2-methyl-3-oxopropyl}azetidin-3-yl)oxy]-3-[2-boronocyclopropyl]-2-hydroxybenzoic acid 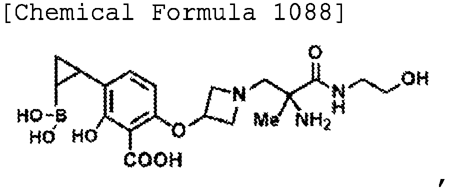
6-[(1-{(2R)-2-amino-3-[(2-hydroxyethyl)amino]-2-methyl-3-oxopropyl}azetidin-3-yl)oxy]-3-[(1R,2S)-2-boronocyclopropyl]-2-hydroxybenzoic acid 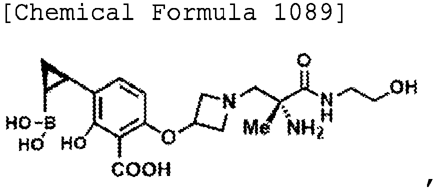
6-[(1-{(2S)-2-amino-3-[(2-hydroxyethyl)amino]-2-methyl-3-oxopropyl}azetidin-3-yl)oxy]-3-[(1R,2S)-2-boronocyclopropyl]-2-hydroxybenzoic acid 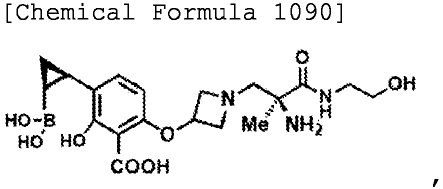
6-[(1-{(2R)-2-amino-3-[(2-hydroxyethyl)amino]-2-methyl-3-oxopropyl}azetidin-3-yl)oxy]-3-[(1S,2R)-2-boronocyclopropyl]-2-hydroxybenzoic acid 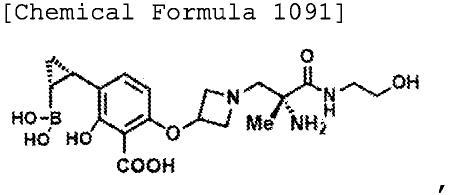
6-[(1-{(2S)-2-amino-3-[(2-hydroxyethyl)amino]-2-methyl-3-oxopropyl}azetidin-3-yl)oxy]-3-[(1S,2R)-2-boronocyclopropyl]-2-hydroxybenzoic acid 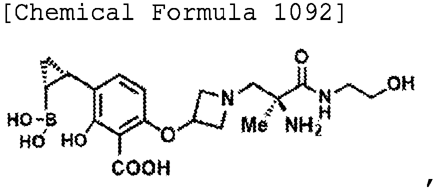
6-[(1-{2-amino-3-[(2-amino-2-oxoethyl)amino]-2-methyl-3-oxopropyl}azetidin-3-yl)oxy]-3-[2-boronocyclopropyl]-2-hydroxybenzoic acid 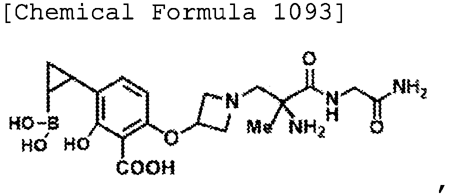
6-[(1-{(2R)-2-amino-3-[(2-amino-2-oxoethyl)amino]-2-methyl-3-oxopropyl}azetidin-3-yl)oxy]-3-[(1R,2S)-2-boronocyclopropyl]-2-hydroxybenzoic acid 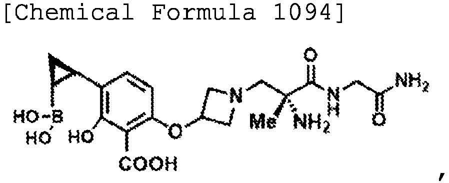
6-[(1-{(2S)-2-amino-3-[(2-amino-2-oxoethyl)amino]-2-methyl-3-oxopropyl}azetidin-3-yl)oxy]-3-[(1R,2S)-2-boronocyclopropyl]-2-hydroxybenzoic acid 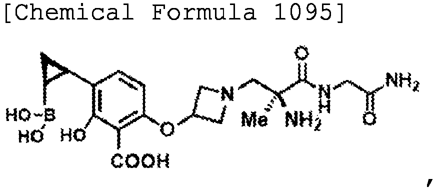
6-[(1-{(2R)-2-amino-3-[(2-amino-2-oxoethyl)amino]-2-methyl-3-oxopropyl}azetidin-3-yl)oxy]-3-[(1S,2R)-2-boronocyclopropyl]-2-hydroxybenzoic acid 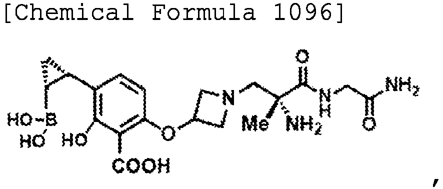
6-[(1-{(2S)-2-amino-3-[(2-amino-2-oxoethyl)amino]-2-methyl-3-oxopropyl}azetidin-3-yl)oxy]-3-[(1S,2R)-2-boronocyclopropyl]-2-hydroxybenzoic acid 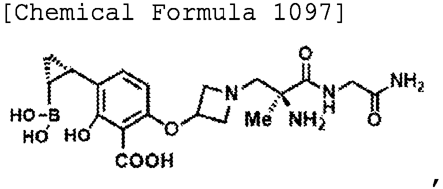
3-[2-boronocyclopropyl]-6-({1-[(5-carbamoylpyrrolidin-3-yl]azetidin-3-yl}oxy)-2-hydroxybenzoic acid 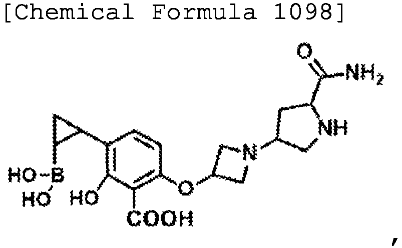
3-[(1R,2S)-2-boronocyclopropyl]-6-({1-[(3S,5S)-5-carbamoylpyrrolidin-3-yl]azetidin-3-yl}oxy)-2-hydroxybenzoic acid 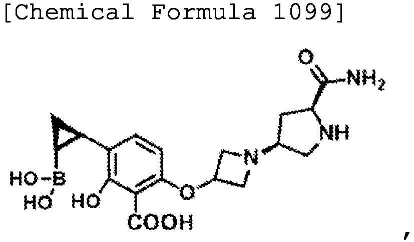
3-[(1R,2S)-2-boronocyclopropyl]-6-({1-[(3R,5S)-5-carbamoylpyrrolidin-3-yl]azetidin-3-yl}oxy)-2-hydroxybenzoic acid 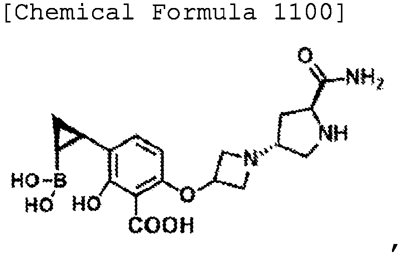
3-[(1R,2S)-2-boronocyclopropyl]-6-({1-[(3S,5R)-5-carbamoylpyrrolidin-3-yl]azetidin-3-yl}oxy)-2-hydroxybenzoic acid 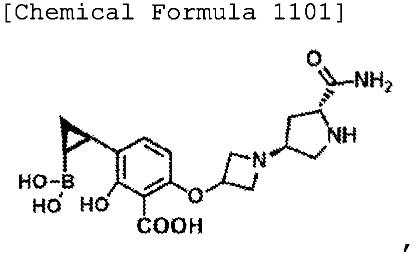
3-[(1R,2S)-2-boronocyclopropyl]-6-({1-[(3R,5R)-5-carbamoylpyrrolidin-3-yl]azetidin-3-yl}oxy)-2-hydroxybenzoic acid 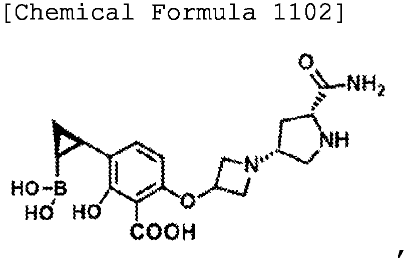
3-[(1S,2R)-2-boronocyclopropyl]-6-({1-[(3S,5S)-5-carbamoylpyrrolidin-3-yl]azetidin-3-yl}oxy)-2-hydroxybenzoic acid 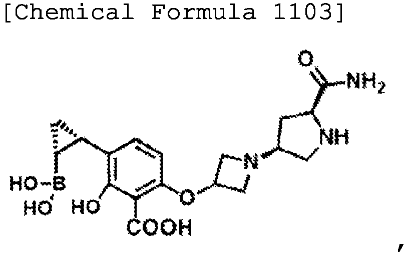
3-[(1S,2R)-2-boronocyclopropyl]-6-({1-[(3R,5S)-5-carbamoylpyrrolidin-3-yl]azetidin-3-yl}oxy)-2-hydroxybenzoic acid 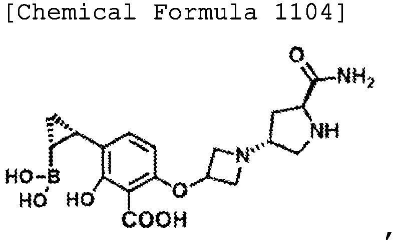
3-[(1S,2R)-2-boronocyclopropyl]-6-({1-[(3S,5R)-5-carbamoylpyrrolidin-3-yl]azetidin-3-yl}oxy)-2-hydroxybenzoic acid 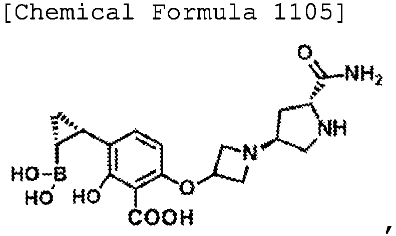
3-[(1S,2R)-2-boronocyclopropyl]-6-({1-[(3R,5R)-5-carbamoylpyrrolidin-3-yl]azetidin-3-yl}oxy)-2-hydroxybenzoic acid 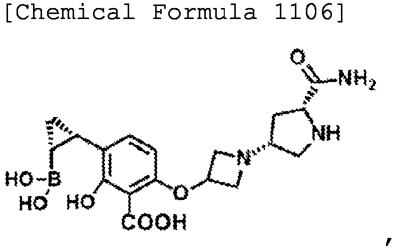
6-{[1-(2-amino-2-oxoethyl)azetidin-3-yl]oxy}-3-[2-boronocyclopropyl]-2-hydroxybenzoic acid 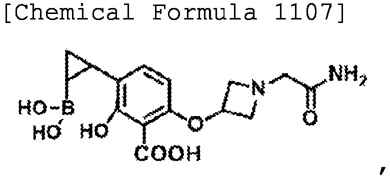
6-{[1-(2-amino-2-oxoethyl)azetidin-3-yl]oxy}-3-[(1R,2S)-2-boronocyclopropyl]-2-hydroxybenzoic acid 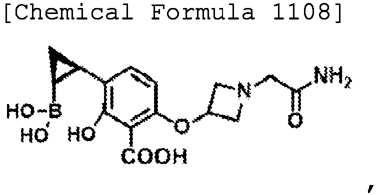
6-{[1-(2-amino-2-oxoethyl)azetidin-3-yl]oxy}-3-[(1S,2R)-2-boronocyclopropyl]-2-hydroxybenzoic acid 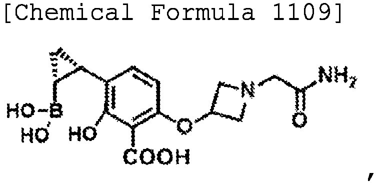
3-[(2-boronocyclopropyl]-2-hydroxy-6-[(1-{2-[(2-hydroxyethoxy)amino]-2-oxoethyl}azetidin-3-yl)oxy]benzoic acid 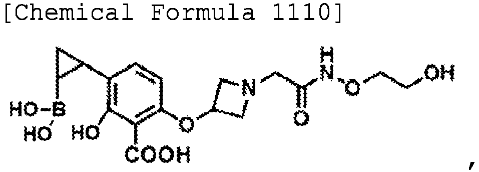
3-[(1R,2S)-2-boronocyclopropyl]-2-hydroxy-6-[(1-{2-[(2-hydroxyethoxy)amino]-2-oxoethyl}azetidin-3-yl)oxy]benzoic acid 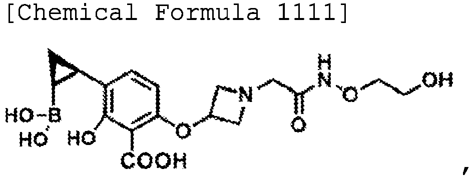
3-[(1S,2R)-2-boronocyclopropyl]-2-hydroxy-6-[(1-{2-[(2-hydroxyethoxy)amino]-2-oxoethyl}azetidin-3-yl)oxy]benzoic acid 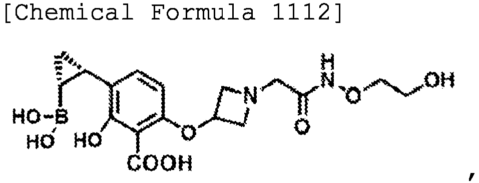
6-({1-[2-amino-3-hydroxy-2-(hydroxymethyl)propyl]azetidin-3-yl}oxy)-3-[2-boronocyclopropyl]-2-hydroxybenzoic acid 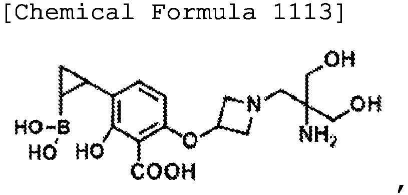
6-({1-[2-amino-3-hydroxy-2-(hydroxymethyl)propyl]azetidin-3-yl}oxy)-3-[(1R,2S)-2-boronocyclopropyl]-2-hydroxybenzoic acid 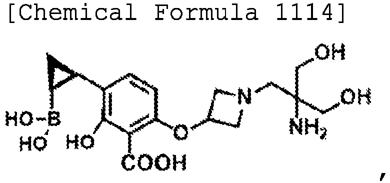
6-({1-[2-amino-3-hydroxy-2-(hydroxymethyl)propyl]azetidin-3-yl}oxy)-3-[(1S,2R)-2-boronocyclopropyl]-2-hydroxybenzoic acid 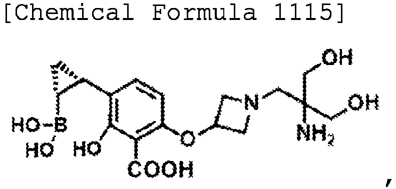
6-({1-[(1-aminocyclopropyl)methyl]azetidin-3-yl}oxy)-3-[2-boronocyclopropyl]-2-hydroxybenzoic acid 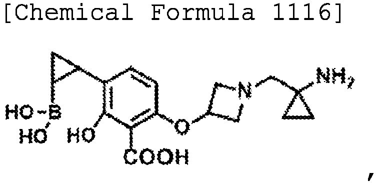
6-({1-[(1-aminocyclopropyl)methyl]azetidin-3-yl}oxy)-3-[(1R,2S)-2-boronocyclopropyl]-2-hydroxybenzoic acid 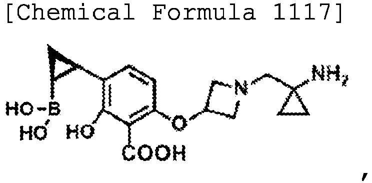
6-({1-[(1-aminocyclopropyl)methyl]azetidin-3-yl}oxy)-3-[(1S,2R)-2-boronocyclopropyl]-2-hydroxybenzoic acid 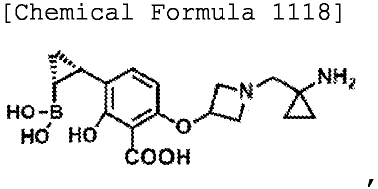
3-[2-boronocyclopropyl]-2-hydroxy-6-({1-[(1H-1,2,4-triazol-3-yl)methyl]azetidin-3-yl}oxy)benzoic acid 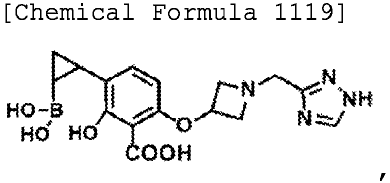
3-[(1R,2S)-2-boronocyclopropyl]-2-hydroxy-6-({1-[(1H-1,2,4-triazol-3-yl)methyl]azetidin-3-yl}oxy)benzoic acid 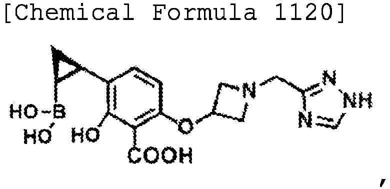
3-[(1S,2R)-2-boronocyclopropyl]-2-hydroxy-6-({1-[(1H-1,2,4-triazol-3-yl)methyl]azetidin-3-yl}oxy)benzoic acid 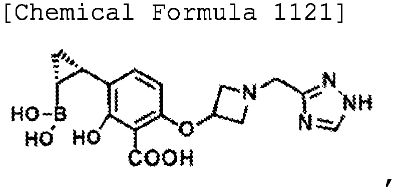
6-{[1-({4-[(2-aminoethyl)amino]cyclohexyl}acetyl)azetidin-3-yl]oxy}-3-[2-boronocyclopropyl]-2-hydroxybenzoic acid 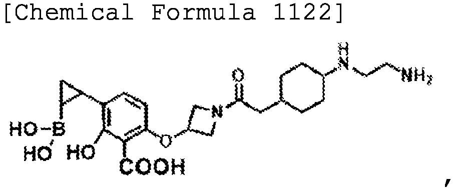
6-{[1-({(1r,4r)-4-[(2-aminoethyl)amino]cyclohexyl}acetyl)azetidin-3-yl]oxy}-3-[(1R,2S)-2-boronocyclopropyl]-2-hydroxybenzoic acid 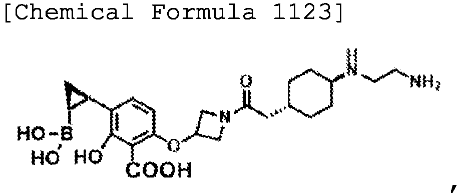
6-{[1-({(1s,4r)-4-[(2-aminoethyl)amino]cyclohexyl}acetyl)azetidin-3-yl]oxy}-3-[(1R,2S)-2-boronocyclopropyl]-2-hydroxybenzoic acid 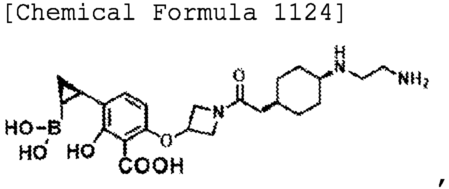
6-{[1-({(1r,4s)-4-[(2-aminoethyl)amino]cyclohexyl}acetyl)azetidin-3-yl]oxy}-3-[(1R,2S)-2-boronocyclopropyl]-2-hydroxybenzoic acid 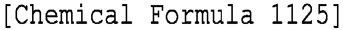 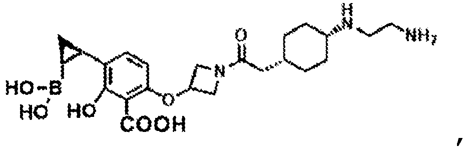
6-{[1-({(1s,4s)-4-[(2-aminoethyl)amino]cyclohexyl}acetyl)azetidin-3-yl]oxy}-3-[(1R,2S)-2-boronocyclopropyl]-2-hydroxybenzoic acid 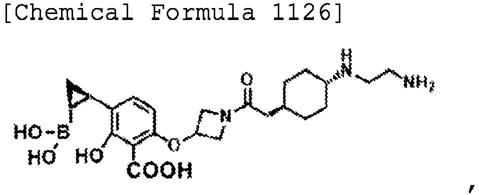
6-{[1-({(1r,4r)-4-[(2-aminoethyl)amino]cyclohexyl}acetyl)azetidin-3-yl]oxy}-3-[(1S,2R)-2-boronocyclopropyl]-2-hydroxybenzoic acid 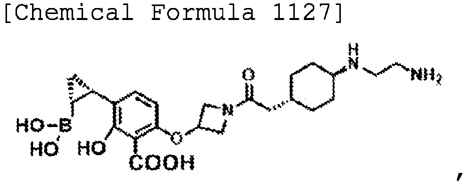
6-{[1-({(1s,4r)-4-[(2-aminoethyl)amino]cyclohexyl}acetyl)azetidin-3-yl]oxy}-3-[(1S,2R)-2-boronocyclopropyl]-2-hydroxybenzoic acid 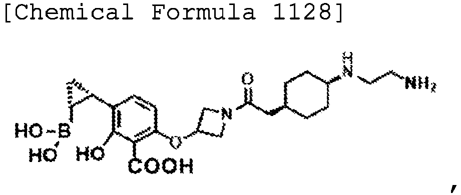
6-{[1-({(1r,4s)-4-[(2-aminoethyl)amino]cyclohexyl}acetyl)azetidin-3-yl]oxy}-3-[(1S,2R)-2-boronocyclopropyl]-2-hydroxybenzoic acid 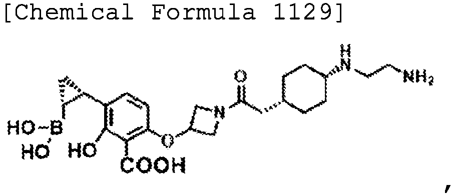
6-{[1-({(1s,4s)-4-[(2-aminoethyl)amino]cyclohexyl}acetyl)azetidin-3-yl]oxy}-3-[(1S,2R)-2-boronocyclopropyl]-2-hydroxybenzoic acid 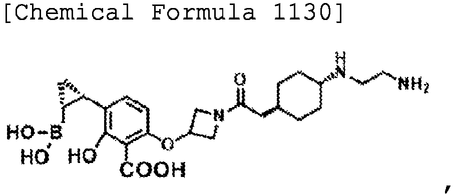
6-[(1-{2-amino-3-[(2-aminoethyl)amino]-2-methyl-3-oxopropyl}azetidin-3-yl)oxy]-3-[2-boronocyclopropyl]-2-hydroxybenzoic acid 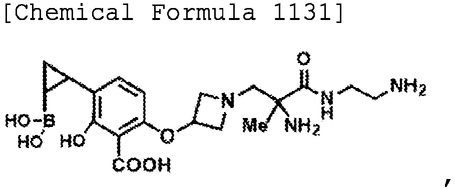
6-[(1-{(2R)-2-amino-3-[(2-aminoethyl)amino]-2-methyl-3-oxopropyl}azetidin-3-yl)oxy]-3-[(1R,2S)-2-boronocyclopropyl]-2-hydroxybenzoic acid 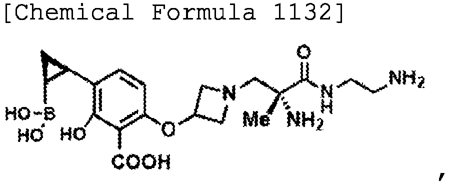
6-[(1-{(2S)-2-amino-3-[(2-aminoethyl)amino]-2-methyl-3-oxopropyl}azetidin-3-yl)oxy]-3-[(1R,2S)-2-boronocyclopropyl]-2-hydroxybenzoic acid 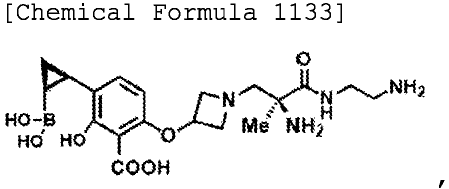
6-[(1-{(2R)-2-amino-3-[(2-aminoethyl)amino]-2-methyl-3-oxopropyl}azetidin-3-yl)oxy]-3-[(1S,2R)-2-boronocyclopropyl]-2-hydroxybenzoic acid 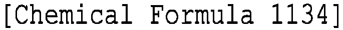 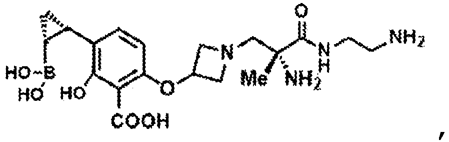
6-[(1-{(2S)-2-amino-3-[(2-aminoethyl)amino]-2-methyl-3-oxopropyl}azetidin-3-yl)oxy]-3-[(1S,2R)-2-boronocyclopropyl]-2-hydroxybenzoic acid 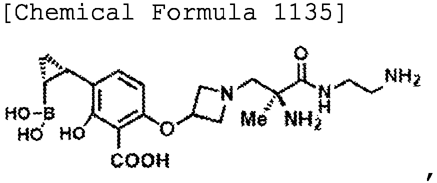
3-(2-boronocyclopropyl)-6-[(1-histidylazetidin-3-yl)oxy]-2-hydroxybenzoic acid 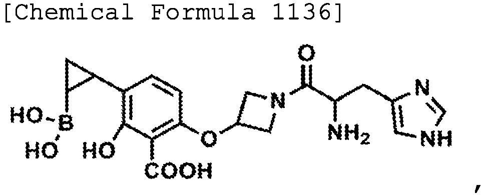
3-[(1R,2S)-2-boronocyclopropyl]-6-[(1-D-histidylazetidin-3-yl)oxy]-2-hydroxybenzoic acid 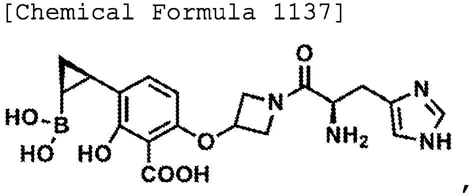
3-[(1R,2S)-2-boronocyclopropyl]-6-[(1-L-histidylazetidin-3-yl)oxy]-2-hydroxybenzoic acid 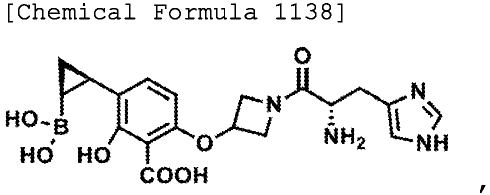
3-[(1S,2R)-2-boronocyclopropyl]-6-[(1-D-histidylazetidin-3-yl)oxy]-2-hydroxybenzoic acid 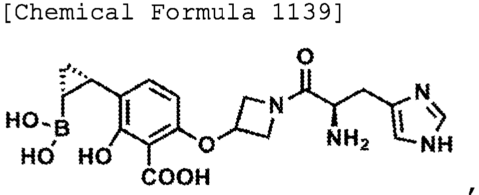
3-[(1S,2R)-2-boronocyclopropyl]-6-[(1-L-histidylazetidin-3-yl)oxy]-2-hydroxybenzoic acid 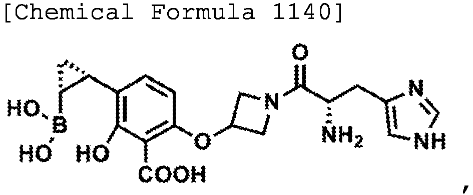
6-{[1-(2-aminoethyl)azetidin-3-yl]oxy}-3-(2-boronocyclopropyl)-2-hydroxybenzoic acid 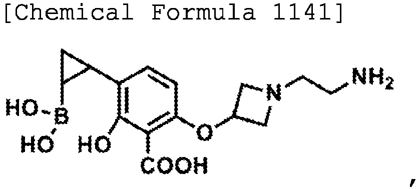
6-{[1-(2-aminoethyl)azetidin-3-yl]oxy}-3-[(1R,2S)-2-boronocyclopropyl]-2-hydroxybenzoic acid 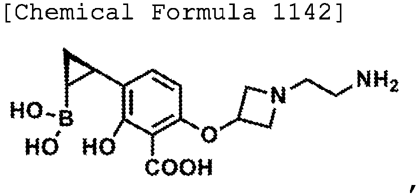
6-{[1-(2-aminoethyl)azetidin-3-yl]oxy}-3-[(1S,2R)-2-boronocyclopropyl]-2-hydroxybenzoic acid 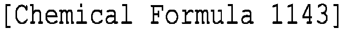 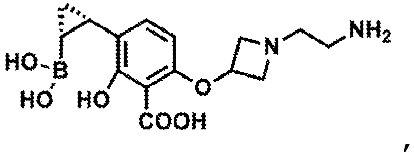
6-[(1-{[5-(aminomethyl)morpholin-2-yl]acetyl}azetidin-3-yl)oxy]-3-(2-boronocyclopropyl)-2-hydroxybenzoic acid 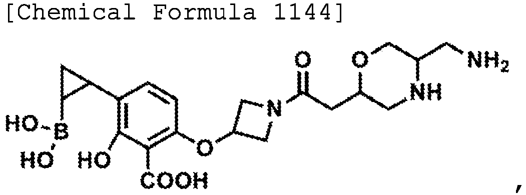
6-[(1-{[5-(aminomethyl)morpholin-2-yl]acetyl}azetidin-3-yl)oxy]-3-[(1R,2S)-2-boronocyclopropyl]-2-hydroxybenzoic acid 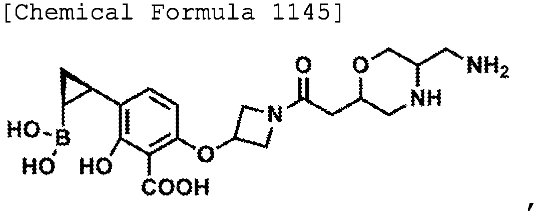
6-[(1-{[5-(aminomethyl)morpholin-2-yl]acetyl}azetidin-3-yl)oxy]-3-[(1S,2R)-2-boronocyclopropyl]-2-hydroxybenzoic acid 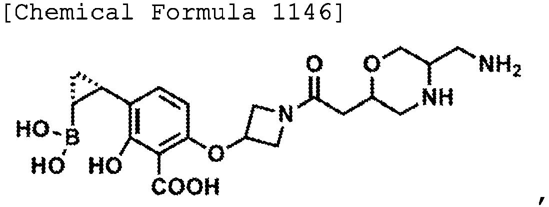
6-({1-[2-amino-2-methyl-3-{[2-(methylamino)-2-oxoethyl]amino}-3-oxopropyl]azetidin-3-yl}oxy)-3-[2-boronocyclopropyl]-2-hydroxybenzoic acid 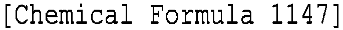 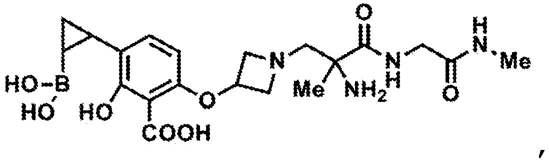
6-({1-[(2R)-2-amino-2-methyl-3-{[2-(methylamino)-2-oxoethyl]amino}-3-oxopropyl]azetidin-3-yl}oxy)-3-[(1R,2S)-2-boronocyclopropyl]-2-hydroxybenzoic acid 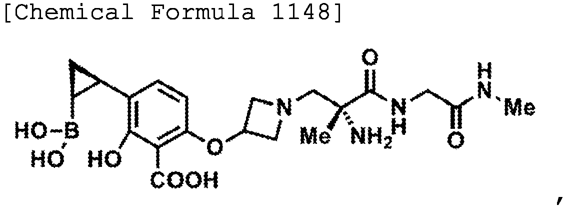
6-({1-[(2S)-2-amino-2-methyl-3-{[2-(methylamino)-2-oxoethyl]amino}-3-oxopropyl]azetidin-3-yl}oxy)-3-[(1R,2S)-2-boronocyclopropyl]-2-hydroxybenzoic acid 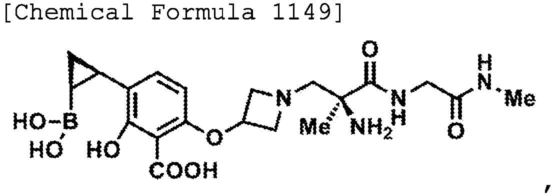
6-({1-[(2R)-2-amino-2-methyl-3-{[2-(methylamino)-2-oxoethyl]amino}-3-oxopropyl]azetidin-3-yl}oxy)-3-[(1S,2R)-2-boronocyclopropyl]-2-hydroxybenzoic acid 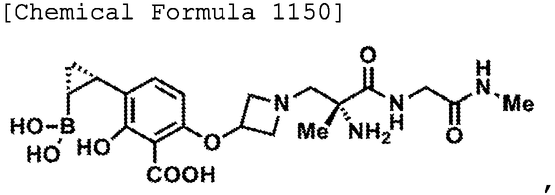
6-({1-[(2S)-2-amino-2-methyl-3-{[2-(methylamino)-2-oxoethyl]amino}-3-oxopropyl]azetidin-3-yl}oxy)-3-[(1S,2R)-2-boronocyclopropyl]-2-hydroxybenzoic acid 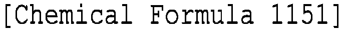 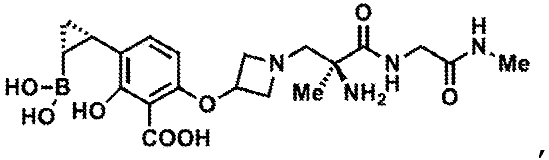
6-({1-[2-amino-3-{[2-(dimethylamino)-2-oxoethyl]amino}-2-methyl-3-oxopropyl]azetidin-3-yl}oxy)-3-[2-boronocyclopropyl]-2-hydroxybenzoic acid 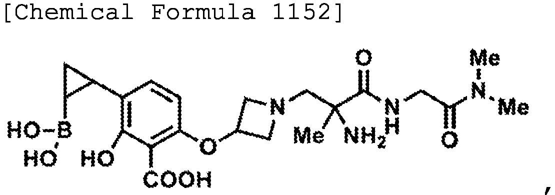
6-({1-[(2R)-2-amino-3-{[2-(dimethylamino)-2-oxoethyl]amino}-2-methyl-3-oxopropyl]azetidin-3-yl}oxy)-3-[(1R,2S)-2-boronocyclopropyl]-2-hydroxybenzoic acid 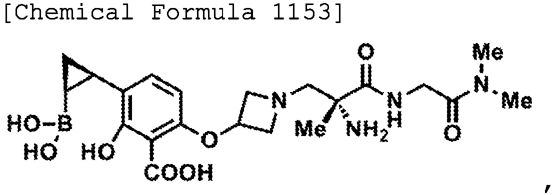
6-({1-[(2S)-2-amino-3-{[2-(dimethylamino)-2-oxoethyl]aminol-2-methyl-3-oxopropyl]azetidin-3-yl}oxy)-3-[(1R,2S)-2-boronocyclopropyl]-2-hydroxybenzoic acid 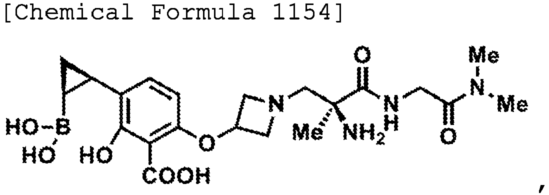
6-({1-[(2R)-2-amino-3-{[2-(dimethylamino)-2-oxoethyl]amino}-2-methyl-3-oxopropyl]azetidin-3-yl}oxy)-3-[(1S,2R)-2-boronocyclopropyl]-2-hydroxybenzoic acid 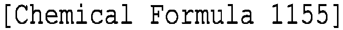 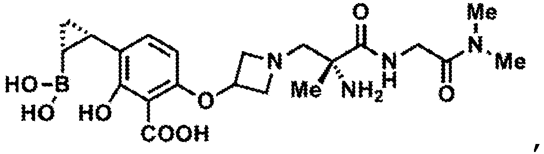
6-({1-[(2S)-2-amino-3-{[2-(dimethylamino)-2-oxoethyl]aminol-2-methyl-3-oxopropyl]azetidin-3-yl}oxy)-3-[(1S,2R)-2-boronocyclopropyl]-2-hydroxybenzoic acid 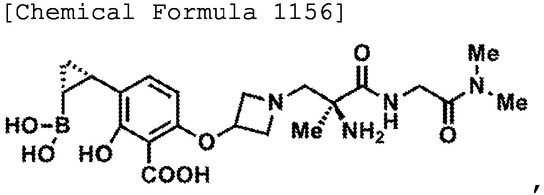
3-[2-boronocyclopropyl]-2-hydroxy-6-[(1-{[4-methylmorpholin-2-yl]acetyl}azetidin-3-yl)oxy]benzoic acid 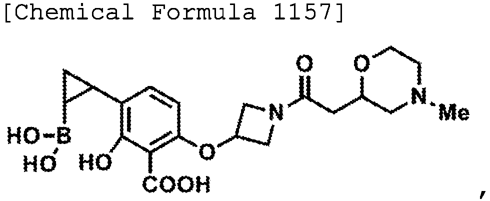
3-[(1R,2S)-2-boronocyclopropyl]-2-hydroxy-6-[(1-{[(2R)-4-methylmorpholin-2-yl]acetyl}azetidin-3-yl)oxy]benzoic acid 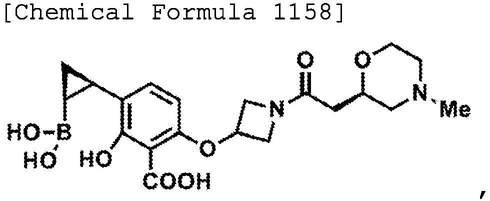
3-[(1R,2S)-2-boronocyclopropyl]-2-hydroxy-6-[(1-{[(2S)-4-methylmorpholin-2-yl]acetyl}azetidin-3-yl)oxy]benzoic acid 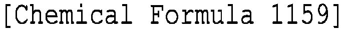 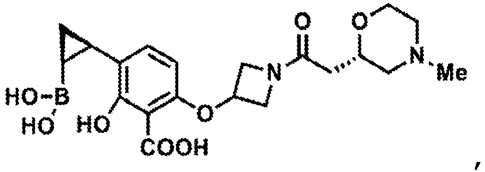
3-[(1S,2R)-2-boronocyclopropyl]-2-hydroxy-6-[(1-{[(2R)-4-methylmorpholin-2-yl]acetyl}azetidin-3-yl)oxy]benzoic acid 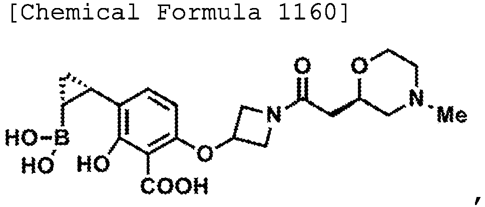
3-[(1S,2R)-2-boronocyclopropyl]-2-hydroxy-6-[(1-{[(2S)-4-methylmorpholin-2-yl]acetyl}azetidin-3-yl)oxy]benzoic acid 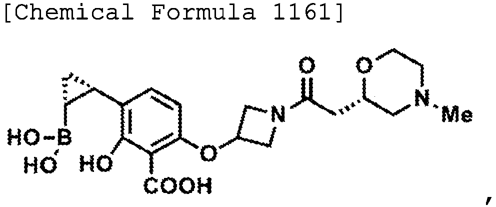
3-[2-boronocyclopropyl]-6-[(1-{[4,4-dimethylmorpholin-4-ium-2-yl]acetyl}azetidin-3-yl)oxy]-2-hydroxybenzoate 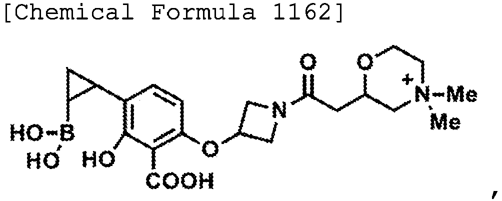
3-[(1R,2S)-2-boronocyclopropyl]-6-[(1-{[(2R)-4,4-dimethylmorpholin-4-ium-2-yl]acetyl}azetidin-3-yl)oxy]-2-hydroxybenzoate 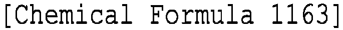 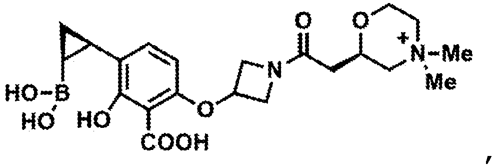
3-[(1R,2S)-2-boronocyclopropyl]-6-[(1-{[(2S)-4,4-dimethylmorpholin-4-ium-2-yl]acetyl}azetidin-3-yl)oxy]-2-hydroxybenzoate 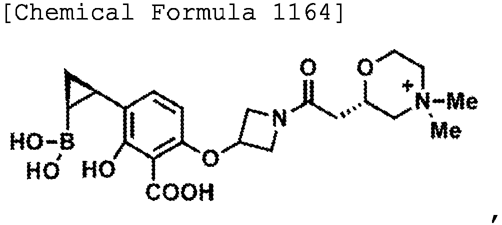
3-[(1S,2R)-2-boronocyclopropyl]-6-[(1-{[(2R)-4,4-dimethylmorpholin-4-ium-2-yl]acetyl}azetidin-3-yl)oxy]-2-hydroxybenzoate 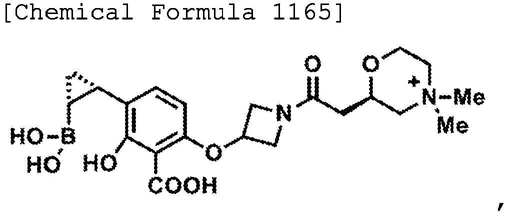
3-[(1S,2R)-2-boronocyclopropyl]-6-[(1-{[(2S)-4,4-dimethylmorpholin-4-ium-2-yl]acetyl}azetidin-3-yl)oxy]-2-hydroxybenzoate 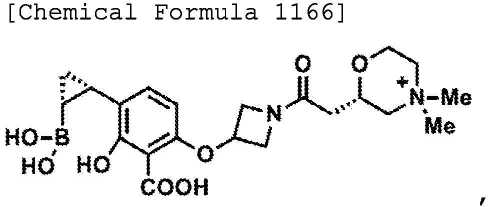
3-(2-boronocyclopropyl)-2-hydroxy-6-({1-[(3-hydroxy-2-methyl-4-oxopyridin-1(4H)-yl)acetyl]azetidin-3-yl}oxy)benzoic acid 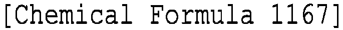 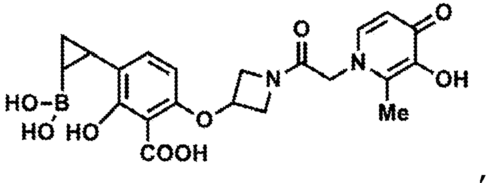
3-[(1S,2R)-2-boronocyclopropyl]-2-hydroxy-6-({1-[(3-hydroxy-2-methyl-4-oxopyridin-1(4H)-yl)acetyl]azetidin-3-yl}oxy)benzoic acid 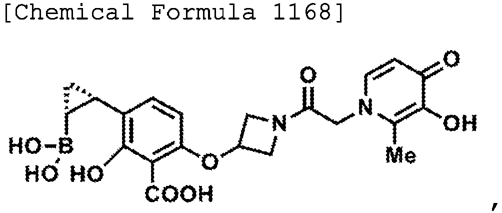 and
3-[(1R,2S)-2-boronocyclopropyl]-2-hydroxy-6-({1-[(3-hydroxy-2-methyl-4-oxopyridin-1(4H)-yl)acetyl]azetidin-3-yl}oxy)benzoic acid

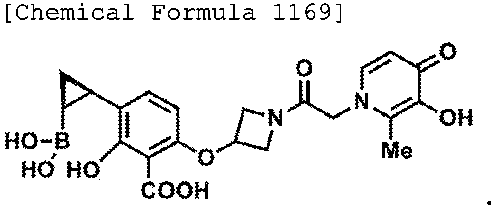

119. A medicament comprising the compound or the pharmaceutically acceptable salt thereof according to any one of claims 1 to claim 118.

120. The medicament according to claim 119, which is a therapeutic drug or a prophylactic drug for a bacterial infection.

121. An agent for inhibiting β-lactamase, comprising the compound or the pharmaceutically acceptable salt thereof according to any one of claims 1 to 118 as an active ingredient.

122. A pharmaceutical composition comprising the compound or the pharmaceutically acceptable salt thereof according to any one of claims 1 to 118 and a pharmaceutically acceptable carrier.

123. The pharmaceutical composition according to claim 122, further comprising an additional agent.

124. The pharmaceutical composition according to claim 123, wherein the additional agent is selected from the group consisting of an antibacterial agent, an antifungal agent, an antiviral agent, an anti-inflammatory agent, and an anti-allergic agent.

125. The pharmaceutical composition according to claim 123 or 124, wherein the additional agent is a β-lactam agent.

126. The pharmaceutical composition according to claim 124 or 125, wherein a β-lactam agent, which is the additional agent, is selected from the group consisting of amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442.

127. The pharmaceutical composition according to claim 125 or 126, wherein the β-lactam agent is selected from ceftazidime, biapenem, doripenem, ertapenem, imipenem, meropenem, or panipenem.

128. The pharmaceutical composition according to claim 125 or 126, wherein the β-lactam agent is selected from aztreonam, tigemonam, BAL30072, SYN2416, or carumonam.

129. The pharmaceutical composition according to claim 122, **characterized in that** an additional agent is concomitantly administered.

130. The pharmaceutical composition according to claim 129, wherein the additional agent is selected from an antibacterial agent, an antifungal agent, an antiviral agent, an anti-inflammatory agent, or an anti-allergic agent.

131. The pharmaceutical composition according to claim 129 or 130, wherein the additional agent is a β-lactam agent.

132. The pharmaceutical composition according to claim 130 or 131, wherein a β-lactam agent, which is the additional agent, is selected from the group consisting of amoxicillin, ampicillin (pivampicillin, hetacillin, bacampicillin, metampicillin, and talampicillin), epicillin, carbenicillin (carindacillin), ticarcillin, temocillin, azlocillin, piperacillin, mezlocillin, mecillinam (pivmecillinam), sulbenicillin, benzylpenicillin (G), clometocillin, benzathine benzylpenicillin, procaine benzylpenicillin, azidocillin, penamecillin, phenoxymethyl penicillin (V), propicillin, benzathine phenoxymethylpenicillin, phenethicillin, cloxacillin (dicloxacillin and flucloxacillin), oxacillin, methicillin, nafcillin, faropenem, biapenem, doripenem, ertapenem, imipenem, meropenem, panipenem, tomopenem, razupenem, cefazolin, cefacetrile, cefadroxil, cephalexin, cefaloglycin, cefalonium, cefaloridine, cephalothin, cephapirin, cefatrizine, cefazedone, cefazaflur, cefradine, cefroxadine, ceftezole, cefaclor, cefamandole, cefminox, cefonicide, ceforanide, cefotiam, cefprozil, cefbuperazone, cefuroxime, cefuzonam, cefoxitin, cefotetan, cefmetazole, loracarbef, cefixime, ceftazidime, ceftriaxone, cefcapene, cefdaloxime, cefdinir, cefditoren, cefetamet, cefmenoxime, cefodizime, cefoperazone, cefotaxime, cefpimizole, cefpiramide, cefpodoxime, cefsulodin, cefteram, ceftibuten, ceftiolene, ceftizoxime, flomoxef, latamoxef, cefepime, cefozopran, cefpirome, cefquinome, ceftobiprole, ceftaroline, CXA-101, RWJ-54428, MC-04546, ME1036, BAL30072, SYN2416, ceftiofur, cefquinome, cefovecin, aztreonam, tigemonam, carumonam, RWJ-442831, RWJ-333441, and RWJ-333442.

133. The pharmaceutical composition according to claim 131 or 132, wherein the β-lactam agent is selected from the group consisting of ceftazidime, biapenem, doripenem, ertapenem, imipenem, meropenem, and panipenem.

134. The pharmaceutical composition according to claim 131 or 132, wherein the β-lactam agent is selected from the group consisting of aztreonam, tigemonam, BAL30072, SYN2416, and carumonam.

135. The compound or the pharmaceutically acceptable salt thereof according to any one of claims 1 to 118 for use in treating a bacterial infection.

136. The compound or the pharmaceutically acceptable salt thereof according to claim 135, wherein the bacterial infection is a bacterial infection in which a bacteria that can have a β-lactamase is involved.

137. The compound or the pharmaceutically acceptable salt thereof according to claim 135 or 136, wherein the bacterial infection is sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

138. A medicament comprised of a combination of the compound or the pharmaceutically acceptable salt thereof according to any one of claims 1 to 118 and at least one agent selected from the group consisting of therapeutic agents for sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, and an odontogenic infection.

139. A pharmaceutical composition comprising a β-lactam agent, wherein the pharmaceutical composition is administered with the compound or the pharmaceutically acceptable salt thereof according to any one of claims 1 to 118.

140. A method for treating a bacterial infection, **characterized in that** a therapeutically effective amount of the compound or the pharmaceutically acceptable salt thereof according to any one of claims 1 to 118 is administered to a patient in need thereof.

141. The method according to claim 140, wherein the bacterial infection is a bacterial infection in which a bacteria that can have a β-lactamase is involved.

142. The method according to claim 140 or 141, wherein the bacterial infection is sepsis, febrile neutropenia, bacterial meningitis, bacterial endocarditis, otitis media, sinusitis, pneumonia, lung abscess, empyema, secondary infection of a chronic respiratory disease, pharyngolaryngitis, tonsillitis, osteomyelitis, arthritis, peritonitis, intraperitoneal abscess, cholecystitis, cholangitis, liver abscess, a deep skin infection, lymphangitis/lymphadenitis, secondary infection of trauma, burn injury, surgical wound, or the like, a urinary tract infection, a genital infection, an eye infection, or an odontogenic infection.

143. The method according to any one of claims 140 to 142, **characterized in that** an additional agent is concomitantly administered.
